# EUROPEAN PATENT APPLICATION

(11) **EP 4 442 683 A1**
(43) Date of publication of application: **09.10.2024**
(21) Application number: 22900619.2
(22) Date of filing: 01.12.2022
(51) Int. Cl.: C07D 307/81, C07D 401/14, C07D 403/04, C07D 405/04, C07D 413/04, A61K 31/343, A61K 31/443, A61P 35/00

(54) **AROMATIC COMPOUND, PHARMACEUTICAL COMPOSITION, AND APPLICATION THEREOF**

(30) Priority: 02.12.2021 CN 202111458624; 20.07.2022 CN 202210864227; 28.09.2022 CN 202211195846
(71) Applicant: Shanghai Kygent Pharmaceutical Co., Ltd, Shanghai 201204 (CN)
(72) Inventor: CHEN, Jian, Shanghai 201204 (CN); SHI, Yufang, Shanghai 201204 (CN); ZHANG, Yong, Shanghai 201204 (CN)
(74) Representative: dompatent von Kreisler Selting Werner - Partnerschaft von Patent- und Rechtsanwälten mbB
(86) International application number: PCT/CN2022/135909
(87) International publication number: WO 2023/098815

(57) **Abstract**

An aromatic compound, a pharmaceutical composition, and an application thereof. Specifically disclosed are the compound represented by formula (I), a pharmaceutically acceptable salt thereof or a solvate of the pharmaceutically acceptable salt. The compound has good inhibitory activity on proliferation of tumor cells.

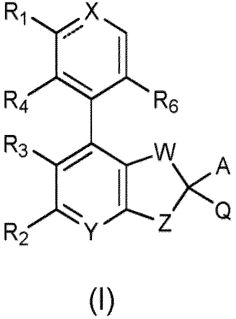

## Description

The present application claims the right of the priorities of Chinese patent application 2021114586244 filed on December 2, 2021, Chinese patent application 2022108642275 filed on July 20, 2022, and Chinese patent application 2022111958466 filed on September 28, 2022. The contents of the above Chinese patent applications are incorporated herein by reference in their entireties.

### TECHNICAL FIELD

The present disclosure relates to an aromatic compound, a pharmaceutical composition thereof, and a use thereof.

### BACKGROUND

The normal growth, tissue repair, and remodeling of tissue cells are precisely regulated and balanced by transcriptional activity. Transcriptional activity is regulated by many signaling pathways, one of which is the Hippo signaling pathway. Genetic studies in fruit flies and mammals have shown that the Hippo signaling pathway is a conservative and important signal transduction pathway. This pathway is composed of a series of kinases, such as MST1/2 and LATS1/2, which are responsible for regulating the transcription factors YAP/TAZ.

When the Hippo signaling pathway is in an active and excited state, YAP/TAZ are phosphorylated and exist in the cytoplasm, or are degraded. When the Hippo signaling pathway is in an inactive and unexcited state, YAP/TAZ will translocate to the nucleus and bind with other transcription factors, namely the so-called TEAD family proteins, TEAD1, TEAD2, TEAD3, and TEAD4. The YAP/TAZ-TEAD complex promotes the transcription of downstream genes and regulates cell proliferation, death, and differentiation. YAP and TAZ can also bind to other factors, and TEAD is widely considered to be the most important regulator of cell growth and tumor growth promotion (Holder and Cunningham 2018).

The high activity of the YAP/TAZ-TEAD complex resulting from the high activation of YAP/TAZ has been found in many human cancers. In these cancers, the expression and protein levels of YAP/TAZ in the nucleus are increased, for example, breast cancer, lung cancer, ovarian cancer, rectal cancer, pancreatic cancer, prostate cancer, gastric cancer, esophageal cancer, and liver cancer (Steinhardt et al., 2008; Harvey et al., 2015). Gene mutations in the Hippo signaling pathway are rare in normal tissue cells, but a large number of mutations have been found in cancer cells. For example, mutations in NF2 and LATS1/2 cause these proteins to no longer function normally, leading to an increase in the activity of the YAP-TEAD complex, especially in malignant pleural mesothelioma (MPM). The growth of cells in malignant pleural mesothelioma depends on the activity of YAP/TAZ-TEAD.

Furthermore, the activation of YAP/TAZ-TEAD in tumor cells leads to the development of drug resistance to chemotherapy drugs and targeted drugs, such as BRAF, MEK, EGFR, KRAS small molecules (Lin L et al., 2015). These findings suggest that by using combination therapy to inhibit the activity of YAP/TAZ-TEAD can enhance efficacy and reduce tumor drug resistance. Combination therapy with small molecule targeted drugs can prevent tumor cells from escaping immune surveillance, and inhibiting the activity of YAP can also activate the immune system (Kim et al., 2018).

Disruption of the YAP/TAZ-TEAD protein-protein interaction can block the Hippo signaling pathway.

### CONTENT OF THE PRESENT INVENTION

The technical problem to be solved by the present disclosure is that there are fewer types of existing compounds that inhibit YAP/TAZ-TEAD. Therefore, the present disclosure provides an aromatic compound, a pharmaceutical composition thereof, and a use thereof. This class of compounds have good inhibitory activity on the proliferation of tumor cells.

The present disclosure provides a compound of formula (I), a pharmaceutically acceptable salt thereof, or a solvate of the pharmaceutically acceptable salt: 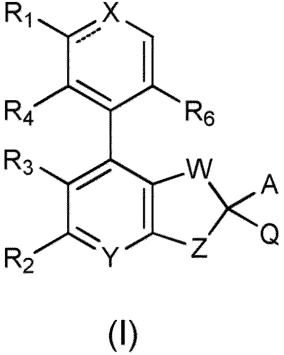 wherein
"-----" is a single bond or a double bond;
Y is CH or N;
W is O or CH-R_{W}; R_{W} is H, OH, C₁-C₆ alkoxy, -C₁-C₆ alkyl-OR_{W-1}, or C₁-C₆ alkyl; R_{W-1} is H or C₁-C₆ alkyl;
Z is CH₂, O, S, or NH;
R₂ is H or halogen;
R₆ is H, CN, -CONHR₆₋₁, -NHR₆₋₂. or C₁-C₆ alkoxy substituted by 1, 2, or 3 NH₂ or OH groups;
R₆₋₁ is H, C₁-C₆ alkyl, C₃-C₆ cycloalkyl, "5- to 6-membered monocyclic heteroaryl with 1, 2, or 3 heteroatoms selected from 1, 2, or 3 of N, O, and S", or "5- to 6-membered monocyclic heteroaryl with 1, 2, or 3 heteroatoms selected from 1, 2, or 3 of N, O, and S" substituted by 1, 2, or 3 C₁-C₆ alkyl groups;
R₆₋₂ is C₁-C₆ alkyl substituted by 1, 2, or 3 NH₂ or OH groups;
R₁, R₃, R₄, X, A, and Q are as defined in any one of the following schemes:
   scheme 1:
      when the "-----" connected to X is a double bond, X is N, CH, or C-R_{X1};
      when X is C-R_{X1}, R_{X1}, R₁ together with the atom to which they are attached form a ring B, that is, 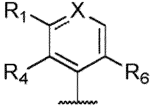 is 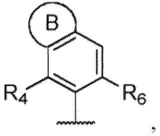 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "-----" connected to X is a single bond, X is N-R_{X2}, and R_{X2}, R₁ together with the atom to which they are attached form a ring C, that is, 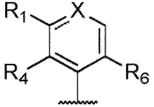 is 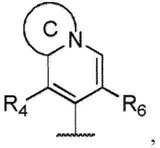 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, C₁-C₆ alkoxy substituted by 1, 2, or 3 R₁₋₁₋₂ groups, -(CH₂)ₙ₁-O-(CH₂)ₙ₂-O-C₃-C₆ cycloalkyl, n1 is 0, 1, or 2, n2 is 1 or 2; R₁₋₁₋₁ and R₁₋₁₋₂ are each independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      R₃ is halogen, CN, C₂-C₆ alkynyl, NH₂, OH, or C₁-C₆ alkoxy;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
      when X is N or CH, R₁ is -(CH₂)ₘ₁-O-(CH₂)ₘ₂-O-R₁₋₆, m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
   scheme 2:
      Q is "3- to 15-membered monocyclic or bicyclic heteroaryl with 1, 2, or 3 heteroatoms selected from 1, 2, or 3 of N, O, and S", "3- to 15-membered monocyclic or bicyclic heteroaryl with 1, 2, or 3 heteroatoms selected from 1, 2, or 3 of N, O, and S" substituted by 1, 2, or 3 R_{Q-2} groups, "3- to 6-membered heterocycloalkyl with 1, 2, or 3 heteroatoms selected from 1, 2, or 3 of N, O, and S" substituted by 1, 2, or 3 R_{Q-5} groups, or 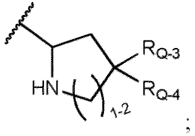
      wherein R_{Q-3}, R_{Q-4} together with the atom to which they are attached form a 3- to 15-membered monocyclic or bicyclic saturated or unsaturated carbocyclic ring, a 3- to 15-membered monocyclic or bicyclic saturated or unsaturated carbocyclic ring substituted by 1, 2, or 3 R_{Q-1} groups, "a 3- to 15-membered monocyclic or bicyclic saturated or unsaturated heterocyclic ring with 1, 2, or 3 heteroatoms selected from 1, 2, or 3 of N, O, and S", or "a 3- to 15-membered monocyclic or bicyclic saturated or unsaturated heterocyclic ring with 1, 2, or 3 heteroatoms selected from 1, 2, or 3 of N, O, and S" substituted by 1, 2, or 3 R_{Q-2} groups;
      R_{Q-1} and R_{Q-2} are independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
      R_{Q-5} is independently oxo (=O), methylene (=CH₂), or C₂-C₆ alkenyl;
      when the "-----" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 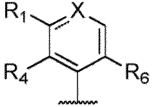 is 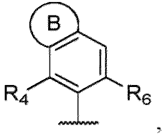 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "-----" connected to X is a single bond, X is N-R_{X2}, and R_{X2}, R₁ together with the atom to which they are attached form a ring C, that is, 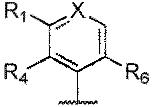 is 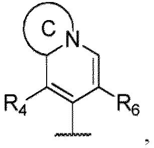 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, C₁-C₆ alkoxy substituted by 1, 2, or 3 R₁₋₁₋₂ groups, -(CH₂)ₙ₁-O-(CH₂)ₙ₂-O-C₃-C₆ cycloalkyl, n1 is 0, 1, or 2, n2 is 1 or 2; R₁₋₁₋₁ and R₁₋₁₋₂ are each independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      R₃ is halogen, CN, C₂-C₆ alkynyl, NH₂, OH, or C₁-C₆ alkoxy;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
   scheme 3:
      A is C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups, A is not 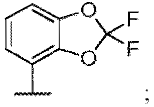
      R_{A-1} is independently halogen, C₁-C₆ alkoxy, NH₂, CN, C₃-C₆ cycloalkyl, C₁-C₆ alkyl substituted by 1 hydroxyl group, or oxo (=O), and at least 1 R_{A-1} is C₁-C₆ alkoxy, NH₂, CN, C₃-C₆ cycloalkyl, C₁-C₆ alkyl substituted by 1 hydroxyl group, or oxo (=O);
      R_{A-2} is independently halogen, C₁-C₆ alkoxy, NH₂, CN, C₃-C₆ cycloalkyl, C₁-C₆ alkyl substituted by 1 hydroxyl group, or oxo (=O);
      when the "-----" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 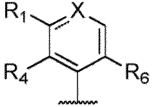 is 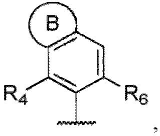 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "-----" connected to X is a single bond, X is N-R_{X2}, and R_{X2}, R₁ together with the atom to which they are attached form a ring C, that is, 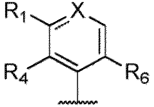 is 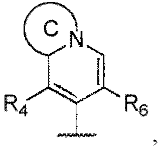 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, C₁-C₆ alkoxy substituted by 1, 2, or 3 R₁₋₁₋₂ groups, -(CH₂)ₙ₁-O-(CH₂)ₙ₂-O-C₃-C₆ cycloalkyl, n1 is 0, 1, or 2, n2 is 1 or 2; R₁₋₁₋₁ and R₁₋₁₋₂ are each independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      R₃ is halogen, CN, C₂-C₆ alkynyl, NH₂, OH, or C₁-C₆ alkoxy;
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
   scheme 4:
      R₄ is NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      when the "-----" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 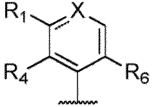 is 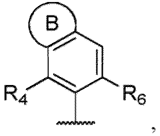 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "-----" connected to X is a single bond, X is N-R_{X2}, and R_{X2}, R₁ together with the atom to which they are attached form a ring C, that is, 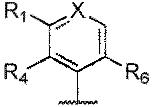 is 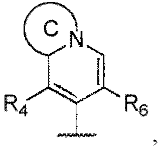 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, C₁-C₆ alkoxy substituted by 1, 2, or 3 R₁₋₁₋₂ groups, -(CH₂)ₙ₁-O-(CH₂)ₙ₂-O-C₃-C₆ cycloalkyl, n1 is 0, 1, or 2, n2 is 1 or 2; R₁₋₁₋₁ and R₁₋₁₋₂ are independently OH, NH₂, or halogen;
      R₃ is halogen, CN, C₂-C₆ alkynyl, NH₂, OH, or C₁-C₆ alkoxy;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
   scheme 5:
      R₃ is C₂-C₆ alkynyl, NH₂, OH, or C₁-C₆ alkoxy;
      when the "-----" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 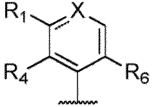 is 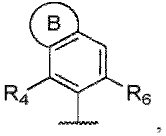 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "-----" connected to X is a single bond, X is N-R_{X2}, and R_{X2}, R₁ together with the atom to which they are attached form a ring C, that is, 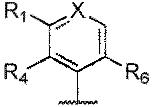 is 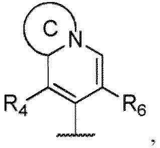 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, C₁-C₆ alkoxy substituted by 1, 2, or 3 R₁₋₁₋₂ groups, -(CH₂)ₙ₁-O-(CH₂)ₙ₂-O-C₃-C₆ cycloalkyl, n1 is 0, 1, or 2, n2 is 1 or 2; R₁₋₁₋₁ and R₁₋₁₋₂ are each independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂.

In a certain embodiment,
"-----" is a single bond or a double bond;
Y is CH or N;
W is O or CH-R_{W}; R_{W} is H, OH, C₁-C₆ alkoxy, -C₁-C₆ alkyl-OR_{W-1}, or C₁-C₆ alkyl; R_{W-1} is H or C₁-C₆ alkyl;
Z is CH₂, O, S, or NH;
R₂ is H or halogen;
R₆ is H, CN, -CONHR₆₋₁, -NHR₆₋₂, or C₁-C₆ alkoxy substituted by 1, 2, or 3 NH₂ or OH groups;
R₆₋₁ is H, C₁-C₆ alkyl, C₃-C₆ cycloalkyl, "5- to 6-membered monocyclic heteroaryl with 1, 2, or 3 heteroatoms selected from 1, 2, or 3 of N, O, and S", or "5- to 6-membered monocyclic heteroaryl with 1, 2, or 3 heteroatoms selected from 1, 2, or 3 of N, O, and S" substituted by 1, 2, or 3 C₁-C₆ alkyl groups;
R₆₋₂ is C₁-C₆ alkyl substituted by 1, 2, or 3 NH₂ or OH groups;
R₁, R₃, R₄, X, A, and Q are as defined in any one of the following schemes:
   scheme 1:
      when the "-----" connected to X is a double bond, X is N, CH, or C-R_{X1};
      when X is N or CH, R₁ is -(CH₂)ₘ₁-O-(CH₂)ₘ₂-O-R₁₋₆, m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 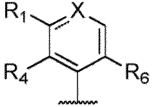 is 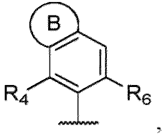 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "-----" connected to X is a single bond, X is N-R_{X2}, and R_{X2}, R₁ together with the atom to which they are attached form a ring C, that is, 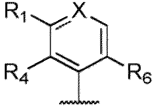 is 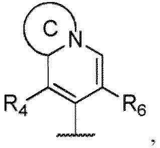 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      R₃ is halogen, CN, C₂-C₆ alkynyl, NH₂, OH, or C₁-C₆ alkoxy;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
   scheme 2:
      Q is "3- to 15-membered monocyclic or bicyclic heteroaryl with 1, 2, or 3 heteroatoms selected from 1, 2, or 3 of N, O, and S", "3- to 15-membered monocyclic or bicyclic heteroaryl with 1, 2, or 3 heteroatoms selected from 1, 2, or 3 of N, O, and S" substituted by 1, 2, or 3 R_{Q-2} groups, "3- to 6-membered heterocycloalkyl with 1, 2, or 3 heteroatoms selected from 1, 2, or 3 of N, O, and S" substituted by 1, 2, or 3 R_{Q-5} groups, or 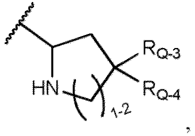
      wherein R_{Q-3}, R_{Q-4} together with the atom to which they are attached form a 3- to 15-membered monocyclic or bicyclic saturated or unsaturated carbocyclic ring, a 3- to 15-membered monocyclic or bicyclic saturated or unsaturated carbocyclic ring substituted by 1, 2, or 3 R_{Q-1} groups, "a 3- to 15-membered monocyclic or bicyclic saturated or unsaturated heterocyclic ring with 1, 2, or 3 heteroatoms selected from 1, 2, or 3 of N, O, and S", or "a 3- to 15-membered monocyclic or bicyclic saturated or unsaturated heterocyclic ring with 1, 2, or 3 heteroatoms selected from 1, 2, or 3 of N, O, and S" substituted by 1, 2, or 3 R_{Q-2} groups;
      R_{Q-1} and R_{Q-2} are independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
      R_{Q-5} is independently oxo (=O), methylene (=CH₂), or C₂-C₆ alkenyl;
      when the "-----" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 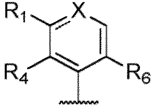 is 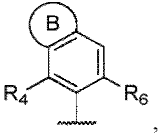 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "-----" connected to X is a single bond, X is N-R_{X2}, and R_{X2}, R₁ together with the atom to which they are attached form a ring C, that is, 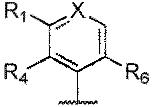 is 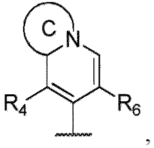 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      R₃ is halogen, CN, C₂-C₆ alkynyl, NH₂, OH, or C₁-C₆ alkoxy;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
   scheme 3:
      A is C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups, A is not 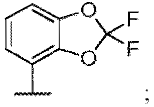
      R_{A-1} is independently halogen, C₁-C₆ alkoxy, NH₂, CN, C₃-C₆ cycloalkyl, C₁-C₆ alkyl substituted by 1 hydroxyl group, or oxo (=O), and at least 1 R_{A-1} is C₁-C₆ alkoxy, NH₂, CN, C₃-C₆ cycloalkyl, C₁-C₆ alkyl substituted by 1 hydroxyl group, or oxo (=O);
      R_{A-2} is independently halogen, C₁-C₆ alkoxy, NH₂, CN, C₃-C₆ cycloalkyl, C₁-C₆ alkyl substituted by 1 hydroxyl group, or oxo (=O);
      when the "-----" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 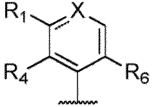 is 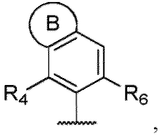 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "-----" connected to X is a single bond, X is N-R_{X2}, and R_{X2}, R₁ together with the atom to which they are attached form a ring C, that is, 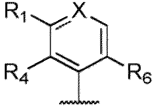 is 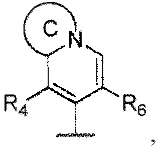 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      R₃ is halogen, CN, C₂-C₆ alkynyl, NH₂, OH, or C₁-C₆ alkoxy;
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
   scheme 4:
      R₄ is NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      when the "-----" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 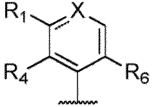 is 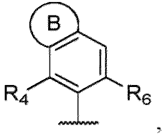 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "-----" connected to X is a single bond, X is N-R_{X2}, and R_{X2}, R₁ together with the atom to which they are attached form a ring C, that is, 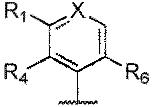 is 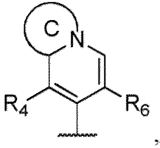 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
      R₃ is halogen, CN, C₂-C₆ alkynyl, NH₂, OH, or C₁-C₆ alkoxy;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
   scheme 5:
      R₃ is C₂-C₆ alkynyl, NH₂, OH, or C₁-C₆ alkoxy;
      when the "-----" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 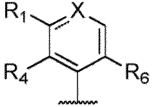 is 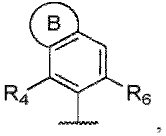 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "-----" connected to X is a single bond, X is N-R_{X2}, and R_{X2}, R₁ together with the atom to which they are attached form a ring C, that is 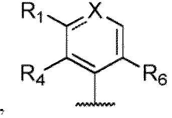 is 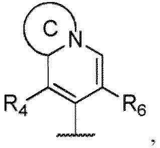 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂.

In a certain embodiment, "-" is a single bond or a double bond;
Y is CH or N;
W is O or CH-R_{W}; R_{W} is H, OH, C₁-C₆ alkoxy, -C₁-C₆ alkyl-OR_{W-1}, or C₁-C₆ alkyl; R_{W-1} is H or C₁-C₆ alkyl;
Z is CH₂, O, S, or NH;
R₂ is H or halogen;
R₆ is H, CN, -CONHR₆₋₁, -NHR₆₋₂, or C₁-C₆ alkoxy substituted by 1, 2, or 3 NH₂ or OH groups;
R₆₋₁ is H, C₁-C₆ alkyl, C₃-C₆ cycloalkyl, "5- to 6-membered monocyclic heteroaryl with 1, 2, or 3 heteroatoms selected from 1, 2, or 3 of N, O, and S", or "5- to 6-membered monocyclic heteroaryl with 1, 2, or 3 heteroatoms selected from 1, 2, or 3 of N, O, and S" substituted by 1, 2, or 3 C₁-C₆ alkyl groups;
R₆₋₂ is C₁-C₆ alkyl substituted by 1, 2, or 3 NH₂ or OH groups;
R₁, R₃, R₄, X, A, and Q are as defined in any one of the following schemes:
   scheme 1:
      when the "- - - - -" connected to X is a double bond, X is N, CH, or C-R_{X1};
      when X is N or CH, R₁ is -(CH₂)ₘ₁-O-(CH₂)ₘ₂-O-R₁₋₆, m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 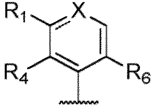 is 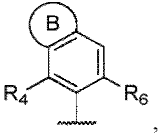 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "- - - - -" connected to X is a single bond, X is N-R_{X2}, and R_{X2}, R₁ together with the atom to which they are attached form a ring C, that is, 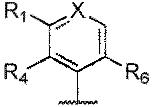 is 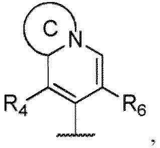 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      R₃ is halogen, CN, C₂-C₆ alkynyl, NH₂, OH, or C₁-C₆ alkoxy;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", or "a 3-to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
   scheme 2:
      Q is "3- to 15-membered monocyclic or bicyclic heteroaryl with 1, 2, or 3 heteroatoms selected from 1, 2, or 3 of N, O, and S", "3- to 15-membered monocyclic or bicyclic heteroaryl with 1, 2, or 3 heteroatoms selected from 1, 2, or 3 of N, O, and S" substituted by 1, 2, or 3 R_{Q-2} groups, "3- to 6-membered heterocycloalkyl with 1, 2, or 3 heteroatoms selected from 1, 2, or 3 of N, O, and S" substituted by 1, 2, or 3 R_{Q-5} groups, or 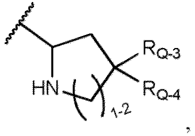
      wherein R_{Q-3}, R_{Q-4} together with the atom to which they are attached form a 3- to 15-membered monocyclic or bicyclic saturated or unsaturated carbocyclic ring, a 3- to 15-membered monocyclic or bicyclic saturated or unsaturated carbocyclic ring substituted by 1, 2, or 3 R_{Q-1} groups, "a 3- to 15-membered monocyclic or bicyclic saturated or unsaturated heterocyclic ring with 1, 2, or 3 heteroatoms selected from 1, 2, or 3 of N, O, and S", or "a 3- to 15-membered monocyclic or bicyclic saturated or unsaturated heterocyclic ring with 1, 2, or 3 heteroatoms selected from 1, 2, or 3 of N, O, and S" substituted by 1, 2, or 3 R_{Q-2} groups;
      R_{Q-1} and R_{Q-2} are independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
      R_{Q-5} is independently oxo (=O), methylene (=CH₂), or C₂-C₆ alkenyl;
      when the "- - - - -" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 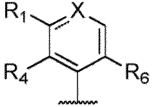 is 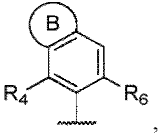 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "- - - - -" connected to X is a single bond, X is N-R_{X2}, and R_{X2}, R₁ together with the atom to which they are attached form a ring C, that is, 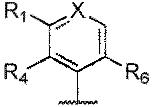 is 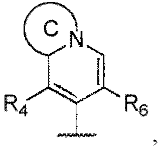 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      R₃ is halogen, CN, C₂-C₆ alkynyl, NH₂, OH, or C₁-C₆ alkoxy;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
   scheme 3:
      A is C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups, A is not

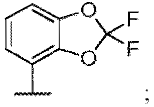

      R_{A-1} is independently halogen, C₁-C₆ alkoxy, NH₂, CN, C₃-C₆ cycloalkyl, C₁-C₆ alkyl substituted by 1 hydroxyl group, or oxo (=O), and at least 1 R_{A-1} is C₁-C₆ alkoxy, NH₂, CN, C₃-C₆ cycloalkyl, C₁-C₆ alkyl substituted by 1 hydroxyl group, or oxo (=O);
      R_{A-2} is independently halogen, C₁-C₆ alkoxy, NH₂, CN, C₃-C₆ cycloalkyl, C₁-C₆ alkyl substituted by 1 hydroxyl group, or oxo (=O);
      when the "- - - - -" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 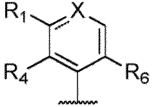 is 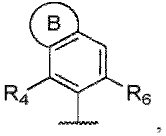 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "- - - - -" connected to X is a single bond, X is N-R_{X2}, and R_{X2}, R₁ together with the atom to which they are attached form a ring C, that is, 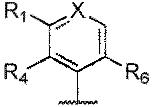 is 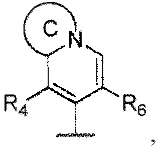 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      R₃ is halogen, CN, C₂-C₆ alkynyl, NH₂, OH, or C₁-C₆ alkoxy;
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", or "a 3-to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
   scheme 4:
      R₄ is NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      when the "- - - - -" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 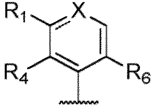 is 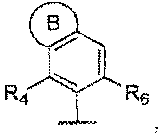 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "- - - - -" connected to X is a single bond, X is N-R_{X2}, and R_{X2}, R₁ together with the atom to which they are attached form a ring C, that is, 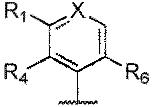 is 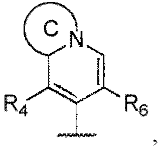 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
      R₃ is halogen, CN, C₂-C₆ alkynyl, NH₂, OH, or C₁-C₆ alkoxy;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", or "a 3-to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂;
   scheme 5:
      R₃ is C₂-C₆ alkynyl, NH₂, OH, or C₁-C₆ alkoxy;
      when the "- - - - -" connected to X is a double bond, X is N, CH, or C-R_{X1},
      when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
      L₁ is absent, -O-, -NH-, -S-, or -S(O)₂-;
      R₁₋₄ is C₁-C₆ alkyl or C₁-C₆ alkyl substituted by 1, 2, or 3 R₁₋₅ groups;
      R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
      m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
      when X is C-R_{X1}, R_{X1}, R₁ together with the atom to which they are attached form a ring B, that is, 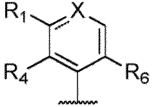 is 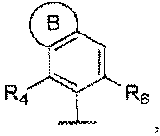 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
      when the "- - - - -" connected to X is a single bond, X is N-R_{X2}, and R_{X2}, R₁ together with the atom to which they are attached form a ring C, that is, 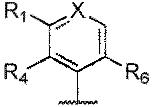 is 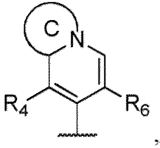 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
      R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
      R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
      A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
      R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
      Q is -CR₇R₈-NR₉-R₁₀ or R₁₁;
      R₇, R₈, and R₉ are independently H or C₁-C₆ alkyl;
      R₁₀ is H, C₁-C₆ alkyl, or -(CH₂)₀₋₂-R₁₀₋₁;
      R₁₀₋₁ is C₁-C₆ alkyl substituted by 1, 2, or 3 OH groups, C₁-C₆ alkoxy, or R₁₀₋₂;
      R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", or "a 3-to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups;
      R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
      R_{Q-1-1} and R_{Q-1-2} are independently H or C₁-C₆ alkyl;
      R_{Q-1-3} and R_{Q-1-4} are independently halogen, OH, or NH₂.

In some preferred embodiments of the present disclosure, some groups in the compound of formula (I), the pharmaceutically acceptable salt thereof, or the solvate of the pharmaceutically acceptable salt are defined as follows, and the unmentioned groups are the same as groups described in any embodiment of the present disclosure (referred to as "in a certain embodiment of the present disclosure"), each C₁-C₆ alkyl is independently methyl, ethyl, n-propyl, isopropyl, n-butyl, isobutyl, sec-butyl, or tert-butyl, such as methyl or ethyl.

In a certain embodiment of the present disclosure, each C₁-C₆ alkoxy is independently methoxy, ethoxy, n-propoxy, isopropoxy, n-butoxy, isobutoxy, sec-butoxy, or tert-butoxy, such as methoxy or ethoxy.

In a certain embodiment of the present disclosure, each halogen is independently F, Cl, Br, or I, such as F or Cl.

In a certain embodiment of the present disclosure, each C₃-C₆ cycloalkyl is independently cyclopropyl, cyclobutyl, cyclopentyl, or cyclohexyl, such as cyclopropyl.

In a certain embodiment of the present disclosure, each "5- to 6-membered monocyclic heteroaryl with 1, 2, or 3 heteroatoms selected from 1, 2, or 3 of N, O, and S" is independently "5- to 6-membered monocyclic heteroaryl with 1, 2, or 3 heteroatoms selected from 1 or 2 of N and O", such as 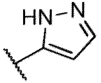 or

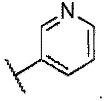

In a certain embodiment of the present disclosure, each "3- to 6-membered heterocycloalkyl with 1, 2, or 3 heteroatoms selected from 1, 2, or 3 of N, O, and S" is independently "5- to 6-membered heterocycloalkyl with 1, 2, or 3 heteroatoms selected from 1 or 2 of N and O", such as

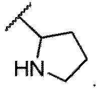

In a certain embodiment of the present disclosure, each C₂-C₆ alkynyl is independently 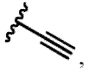 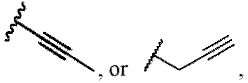 such as

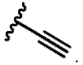

In a certain embodiment of the present disclosure, each C₂-C₆ alkenyl is independently 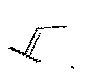 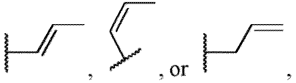 such as

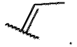

In a certain embodiment of the present disclosure, each "C₆-C₁₀ monocyclic or bicyclic aryl" is independently phenyl or naphthyl, such as phenyl.

In a certain embodiment of the present disclosure, each "8- to 12-membered bicyclic heteroaryl with 1, 2, or 3 heteroatoms selected from 1, 2, or 3 of N, O, and S" is independently "9- or 10-membered bicyclic heteroaryl with 1, 2, or 3 heteroatoms selected from 1, 2, or 3 of N, O, and S", such as

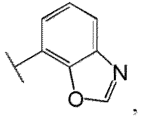

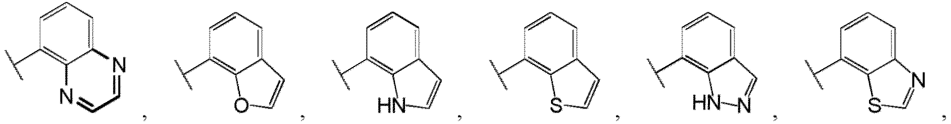

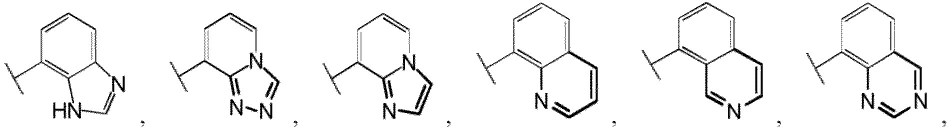

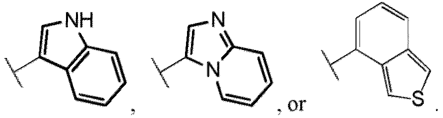

In a certain embodiment of the present disclosure, each "3- to 15-membered monocyclic or bicyclic heteroaryl with 1, 2, or 3 heteroatoms selected from 1, 2, or 3 of N, O, and S" is independently "5- to 10-membered monocyclic or bicyclic heteroaryl with 1, 2, or 3 heteroatoms selected from 1, 2 or 3 of N O, and S", such as

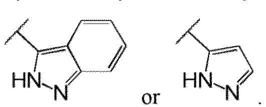

In a certain embodiment of the present disclosure, each 5- to 15-membered monocyclic, bicyclic, or tricyclic saturated or unsaturated carbocyclic ring is independently a 5- to 6-membered monocyclic, 6- to 10-membered bicyclic, or 8- to 15-membered tricyclic saturated or unsaturated carbocyclic ring, preferably a 5- to 6-membered monocyclic, 6- to 10-membered bicyclic, or 8- to 15-membered tricyclic saturated carbocyclic ring.

In a certain embodiment of the present disclosure, each "5- to 15-membered monocyclic, bicyclic, or tricyclic saturated or unsaturated heterocyclic ring with 1, 2, or 3 heteroatoms selected from 1, 2, or 3 of N, O, and S" is independently "5- to 6-membered monocyclic, 6- to 10-membered bicyclic, or 8- to 15-membered tricyclic saturated or unsaturated heterocyclic ring with 1, 2, or 3 heteroatoms selected from 1, 2, or 3 of N, O, and S".

In a certain embodiment of the present disclosure, each 3- to 15-membered monocyclic or bicyclic saturated or unsaturated carbocyclic ring is independently a 3- to 6-membered monocyclic or 6- to 10-membered bicyclic saturated or unsaturated carbocyclic ring, preferably a 3- to 6-membered monocyclic saturated carbocyclic ring or a 6- to 10-membered bicyclic saturated or unsaturated carbocyclic ring.

In a certain embodiment of the present disclosure, each "3- to 15-membered monocyclic or bicyclic saturated or unsaturated heterocyclic ring with 1, 2, or 3 heteroatoms selected from 1, 2, or 3 of N, O, and S" is independently "3- to 6-membered monocyclic saturated heterocyclic ring or 6- to 10-membered bicyclic saturated or unsaturated heterocyclic ring with 1 or 2 heteroatoms selected from 1 or 2 of N and O".

In a certain embodiment of the present disclosure, Y is CH.

In a certain embodiment of the present disclosure, R_{W} is H, OH, or C₁-C₆ alkyl.

In a certain embodiment of the present disclosure, W is CH-R_{W}, and R_{W} is H, OH, or C₁-C₆ alkyl, such as CH₂, CH-OH, or CH-CH₃.

In a certain embodiment of the present disclosure, W is CH₂.

In a certain embodiment of the present disclosure, Z is O.

In a certain embodiment of the present disclosure, R₂ is halogen, preferably F.

In a certain embodiment of the present disclosure, R₆₋₁ is H or C₁-C₆ alkyl.

In a certain embodiment of the present disclosure, R₆ is -CONHR₆₋₁, such as 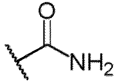 or

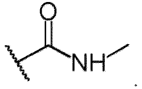

In a certain embodiment of the present disclosure, R₆ is H, CN,

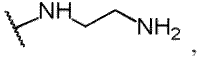

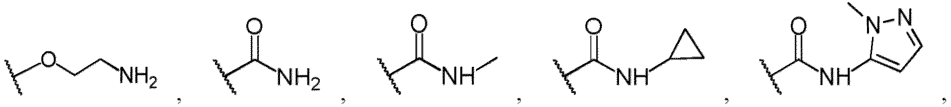

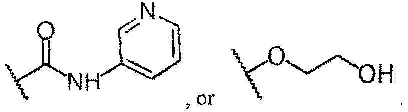

In a certain embodiment of the present disclosure, R₁₋₆ is C₃-C₆ cycloalkyl.

In a certain embodiment of the present disclosure, R₁₋₁ is oxo (=O), OH, NH₂, CN, halogen, - S(O)₂-C₁-C₆ alkyl, C₁-C₆ alkyl, or C₁-C₆ alkoxy substituted by 1, 2, or 3 R₁₋₁₋₂ groups.

In a certain embodiment of the present disclosure, R₁₋₁₋₂ is OH.

In a certain embodiment of the present disclosure, R₁₋₁ is oxo (=O), OH, NH₂, CN, halogen, - S(O)₂-C₁-C₆ alkyl, C₁-C₆ alkyl, or C₁-C₆ alkoxy substituted by 1, 2, or 3 R₁₋₁₋₂ groups, and R₁₋₁₋₂ is OH.

In a certain embodiment of the present disclosure, R₁₋₂ is OH or halogen.

In a certain embodiment of the present disclosure, in scheme 1, 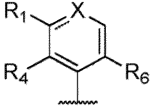 is 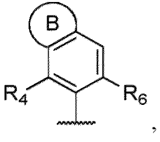 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5-to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
R₁₋₁ is oxo (=O), OH, NH₂, CN, halogen, -S(O)₂-C₁-C₆ alkyl, C₁-C₆ alkyl, or C₁-C₆ alkoxy substituted by 1, 2, or 3 R₁₋₁₋₂ groups;
R₁₋₂ is OH or halogen;
R₁₋₁₋₂ is OH;
such as

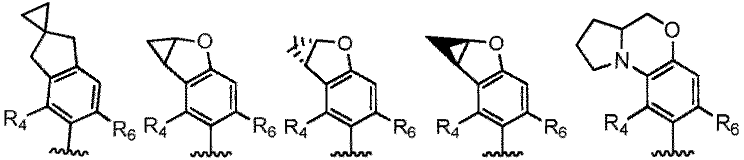

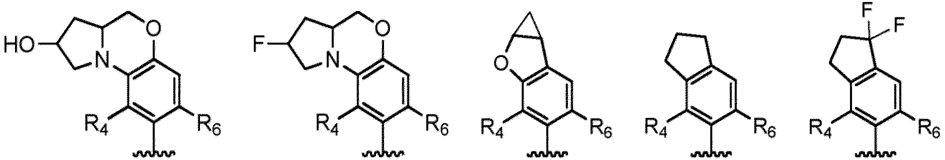

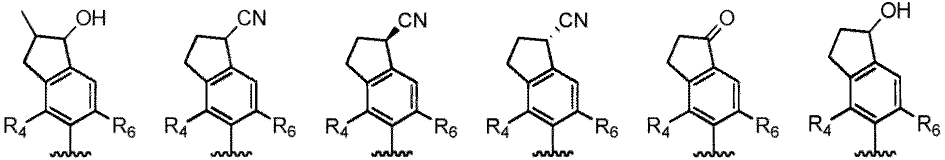

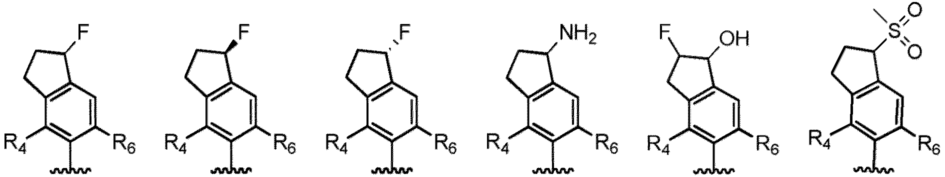

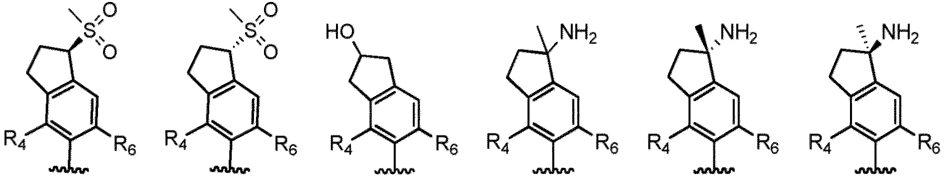

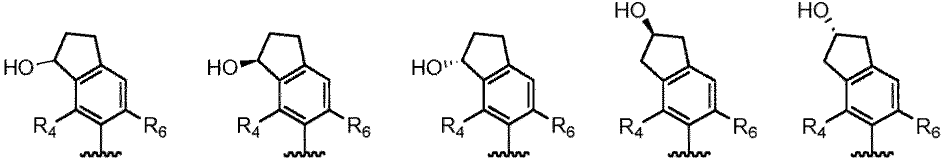

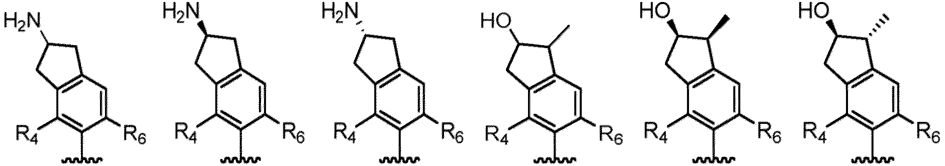

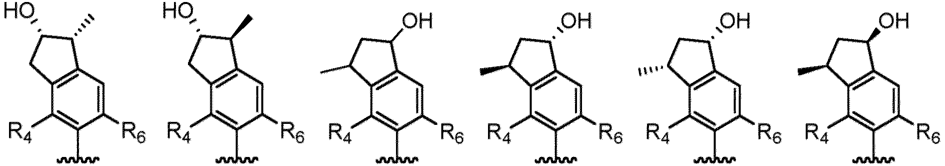

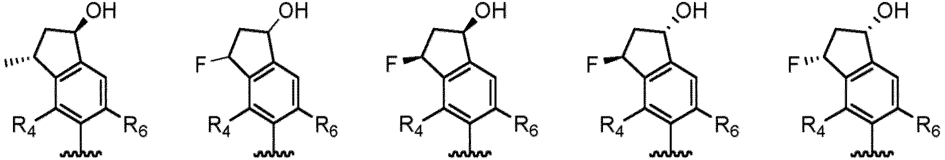

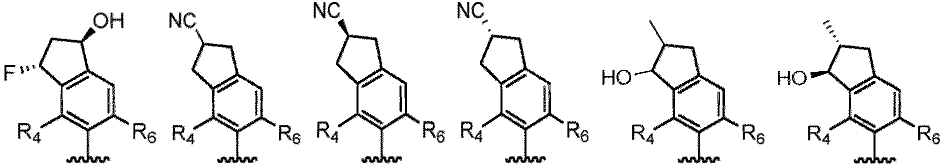

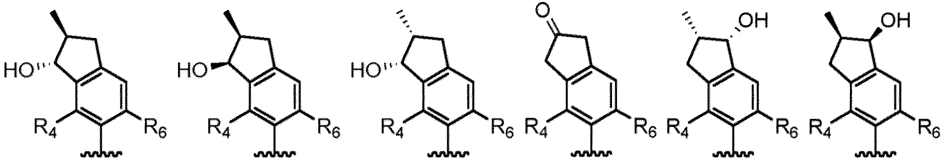

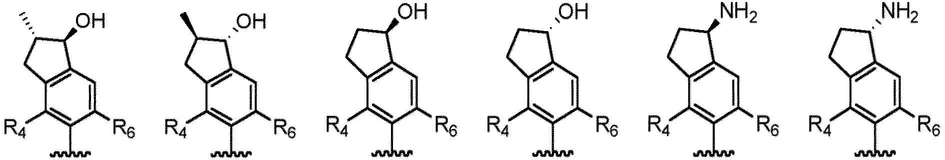

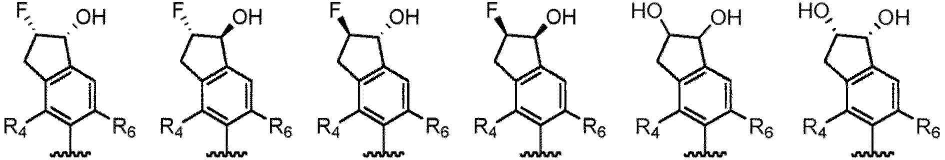

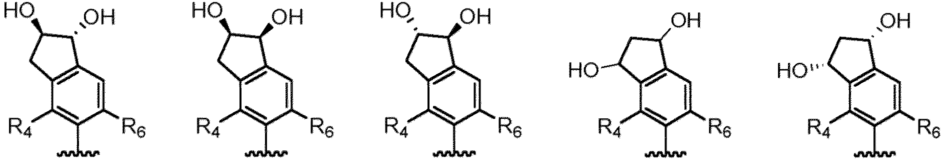

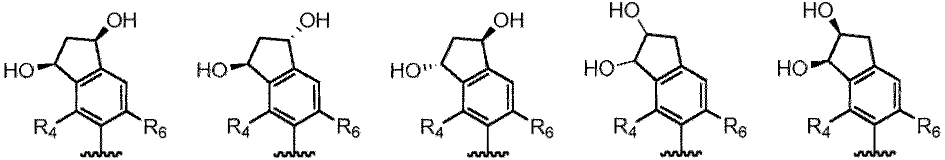

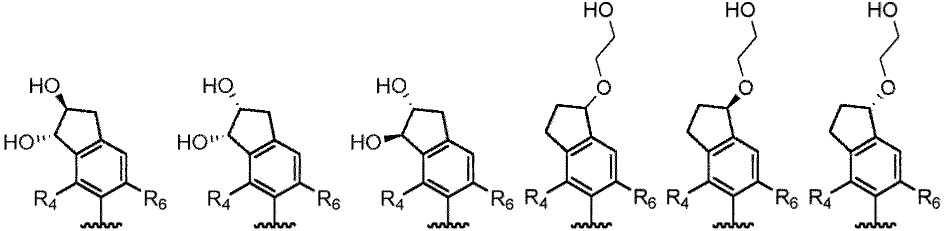

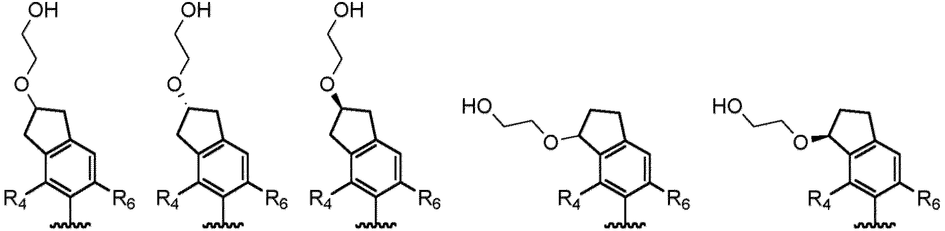

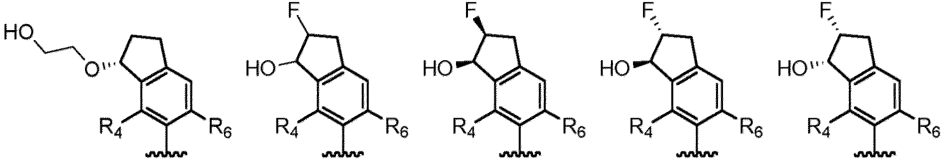

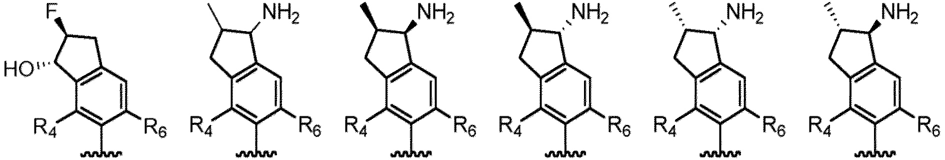

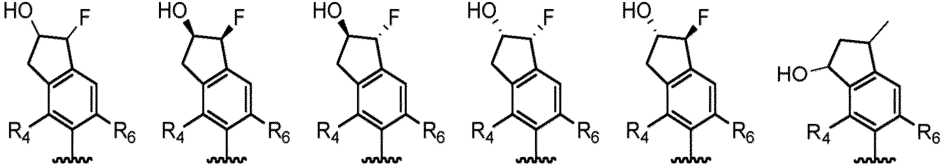

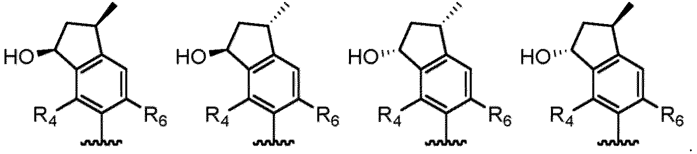

In a certain embodiment of the present disclosure, in scheme 1, 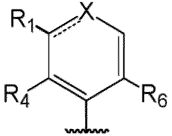 is 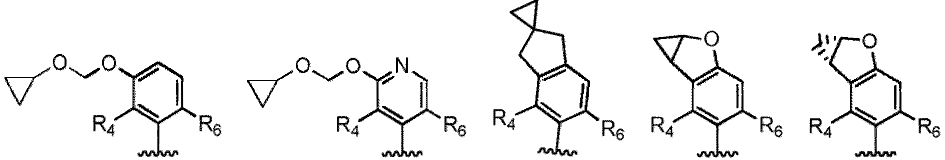 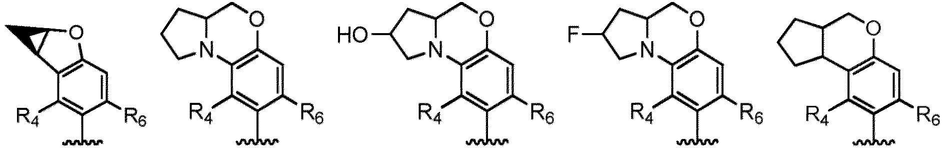 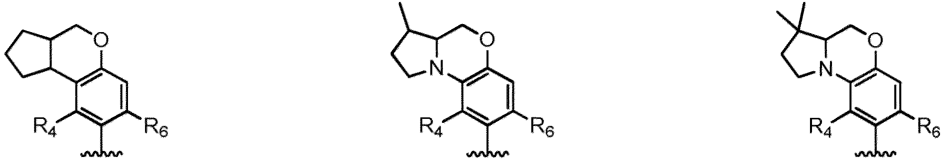 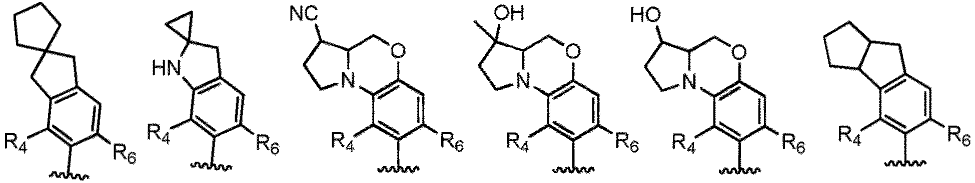 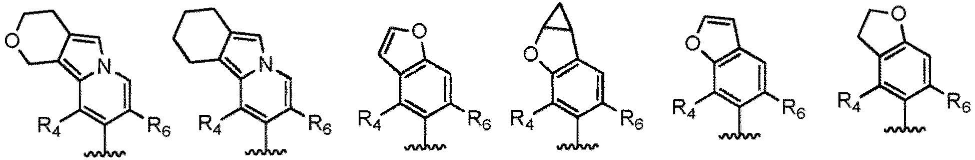 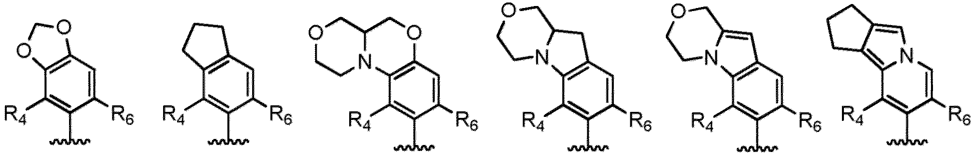 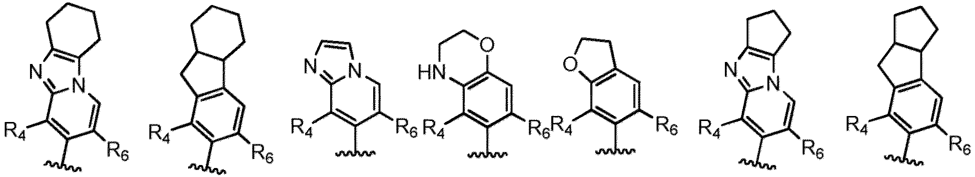 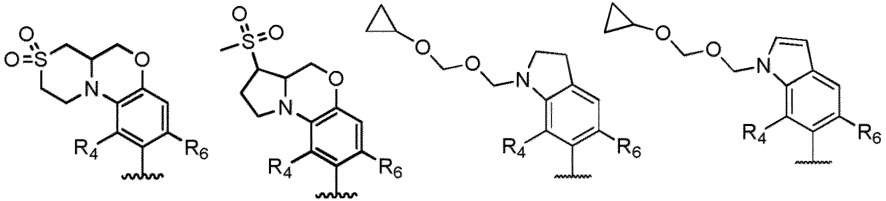 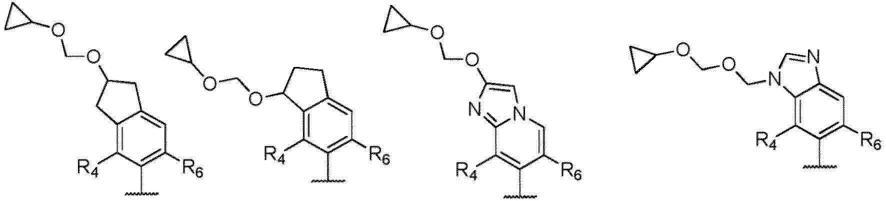 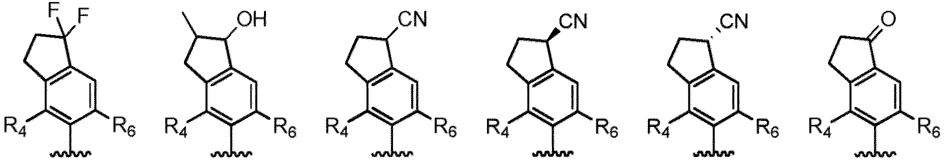 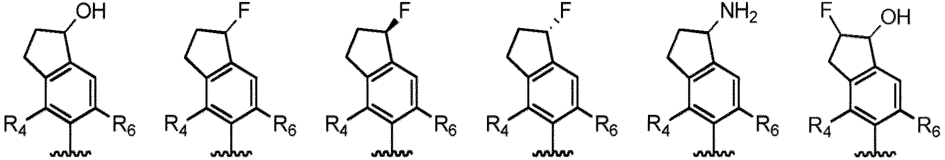 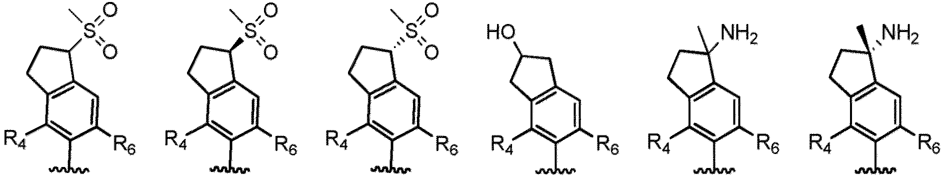 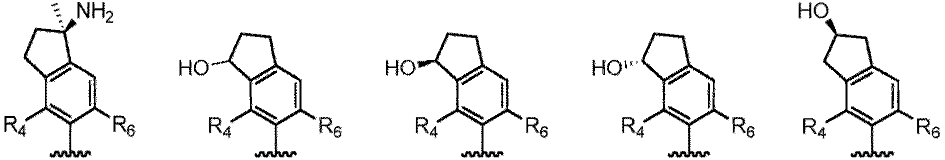 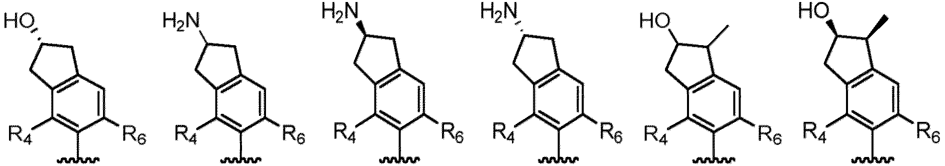 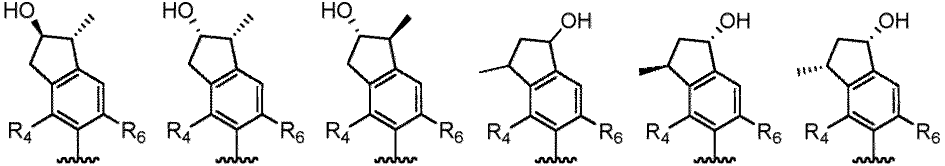 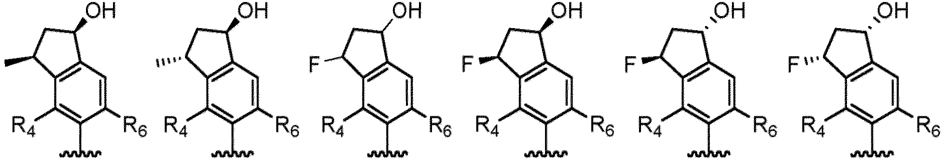 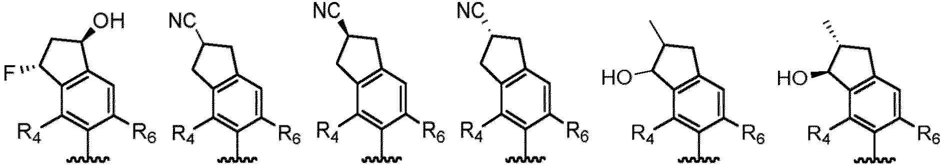 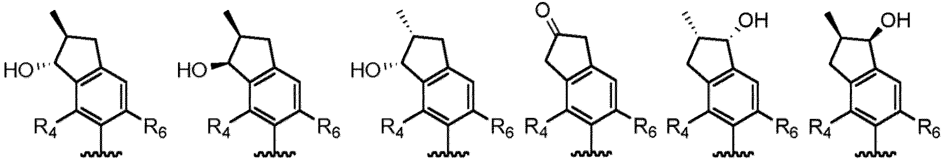 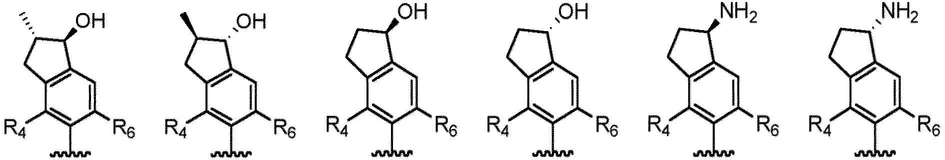 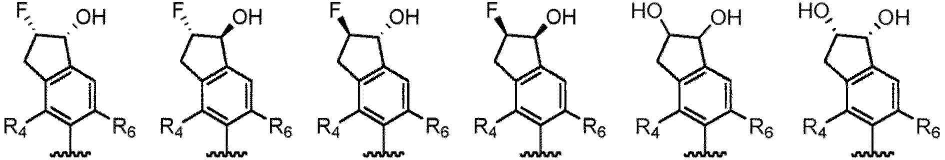 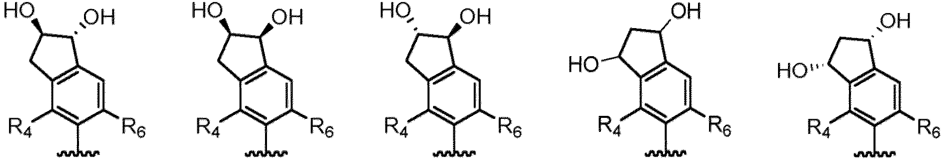 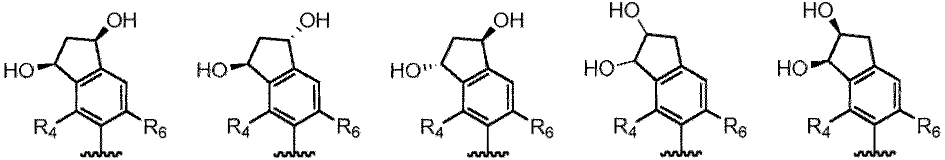 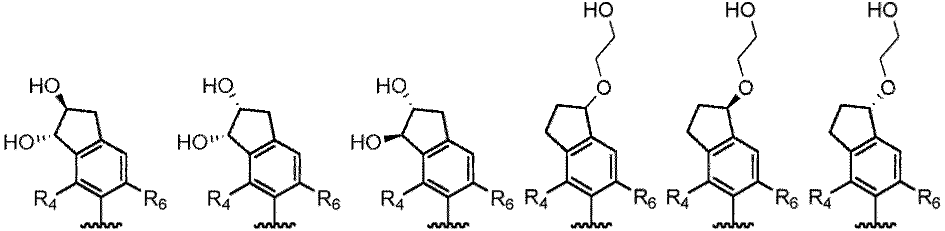 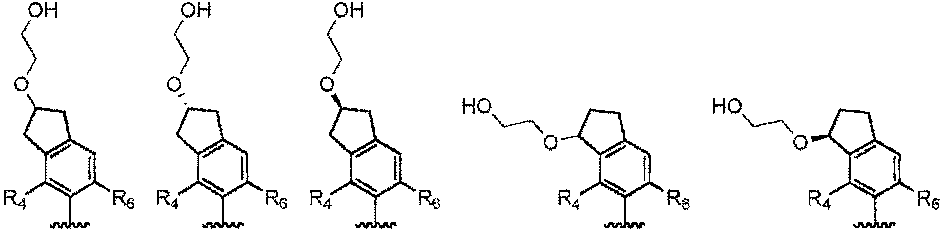 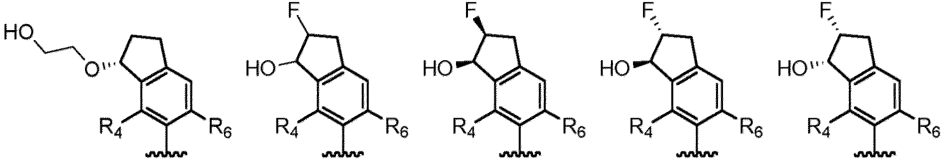 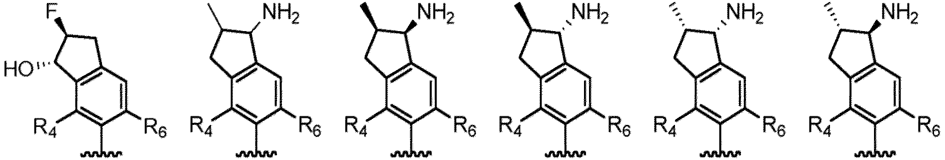 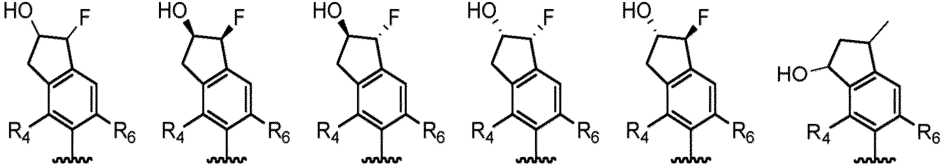 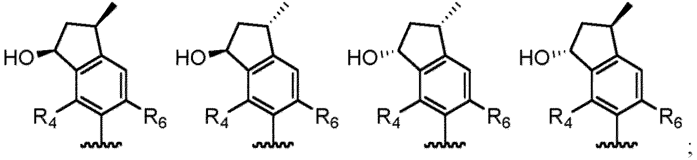
preferably 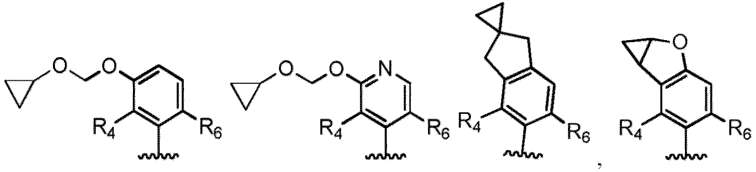 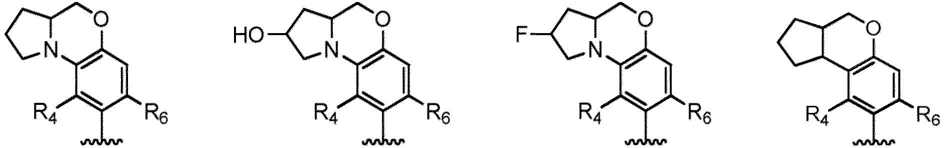 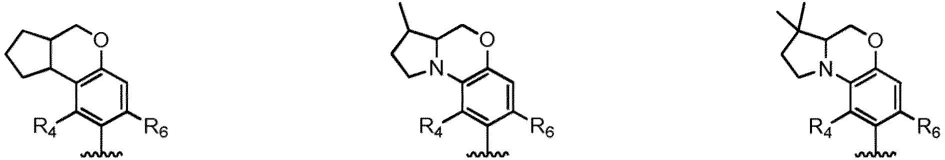 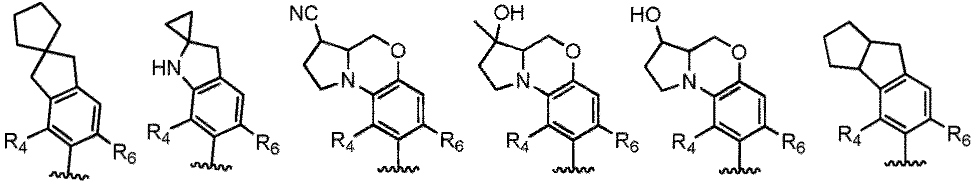 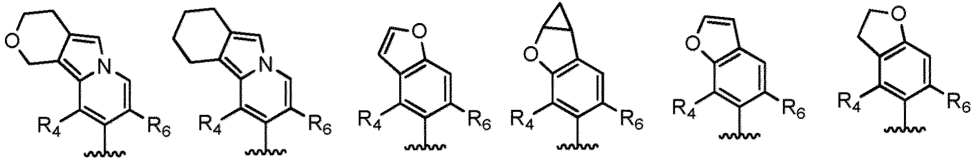 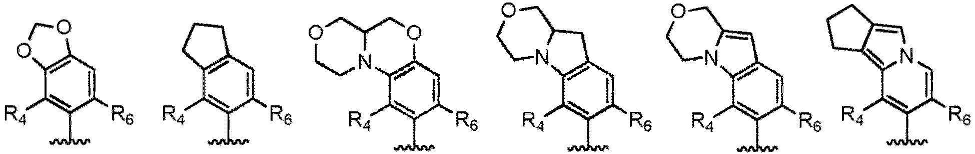 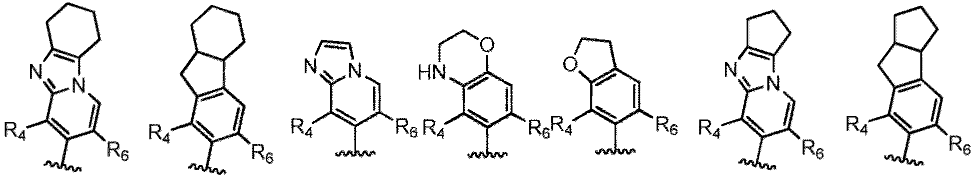 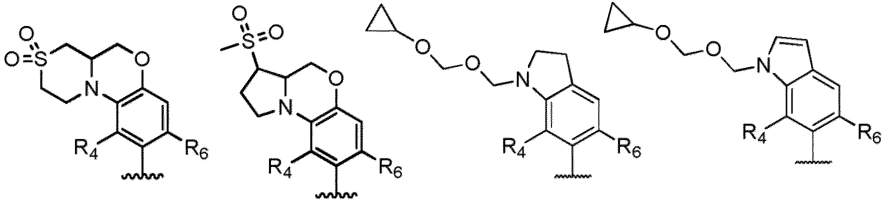 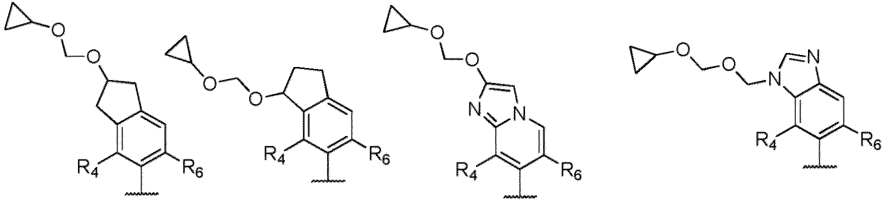 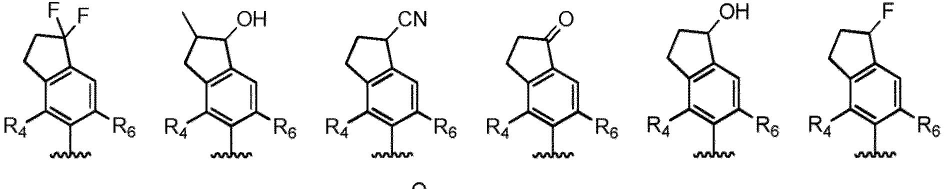 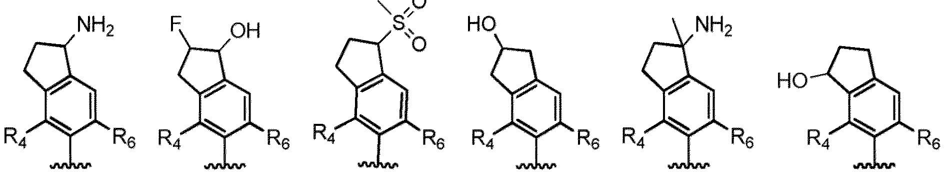 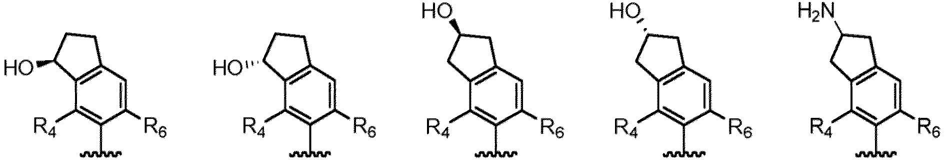 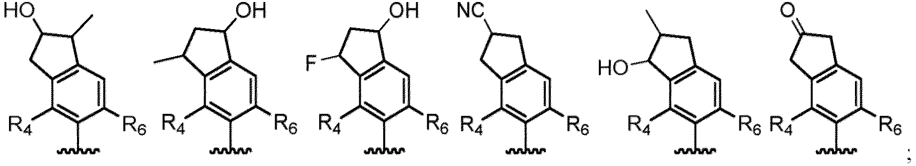
more preferably 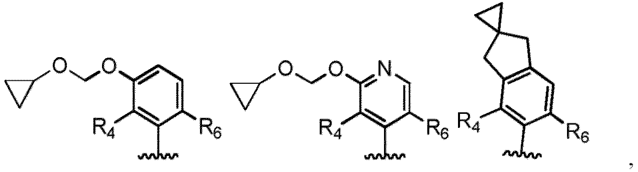 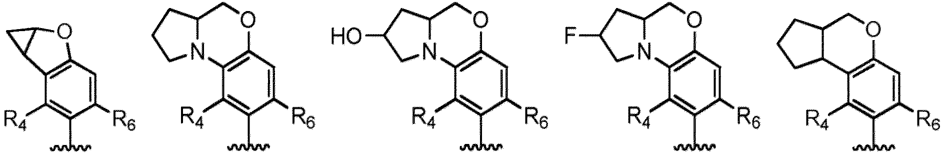 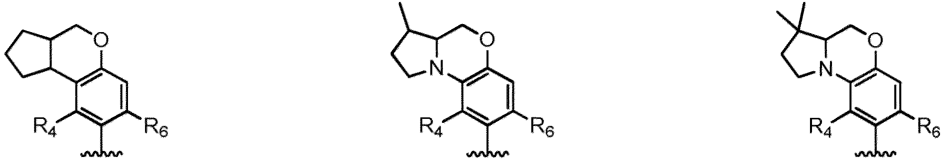 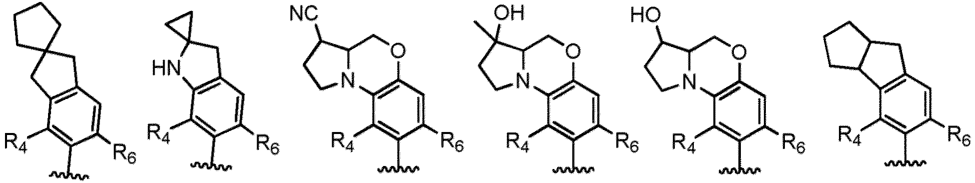 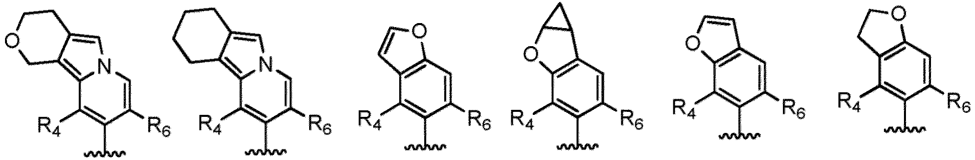 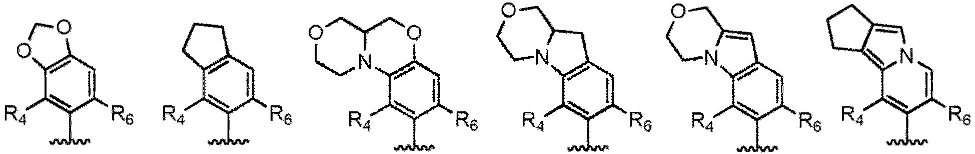 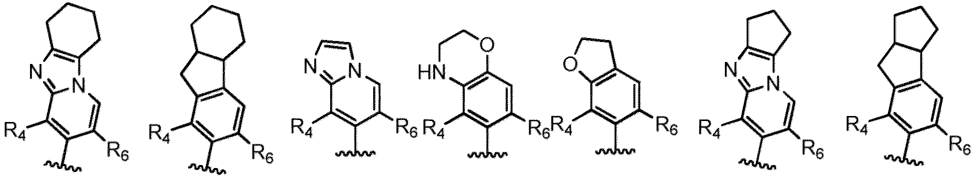 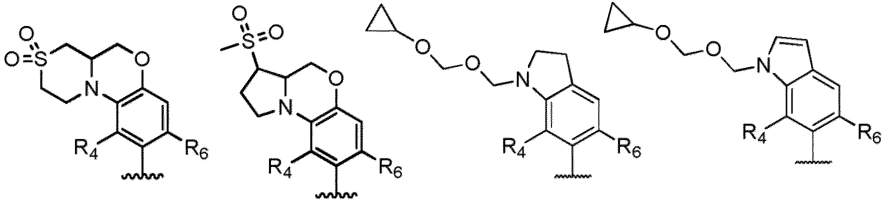 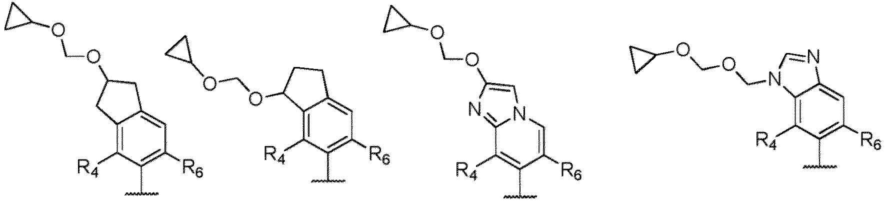 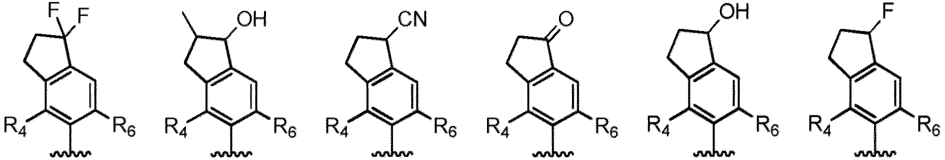 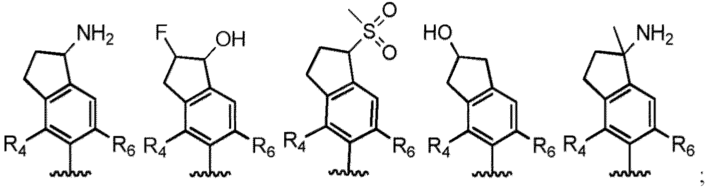
further more preferably:

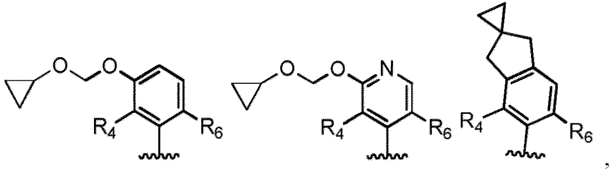

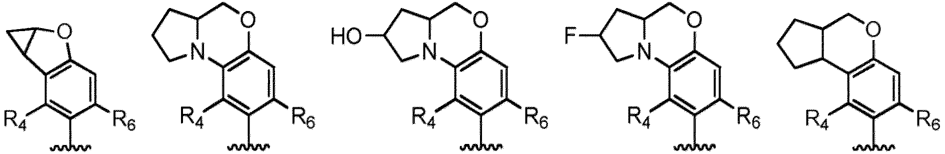

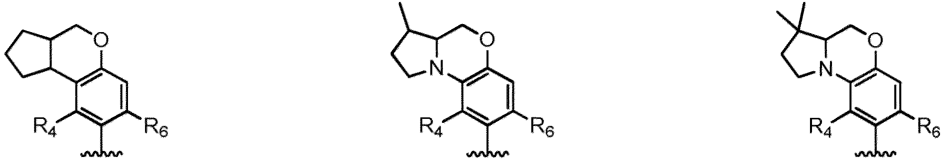

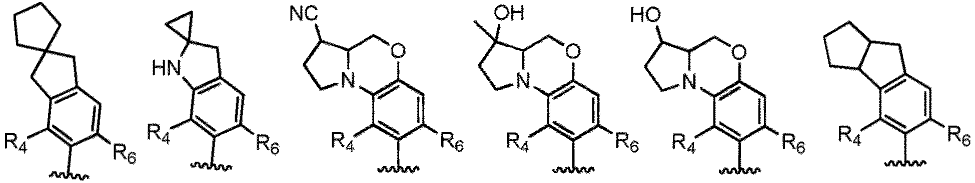

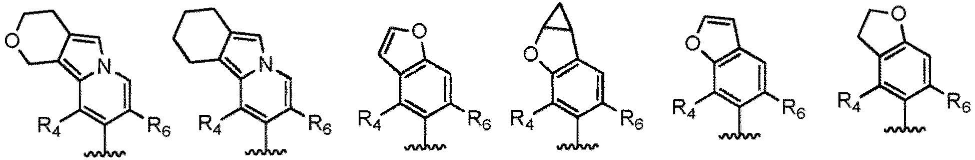

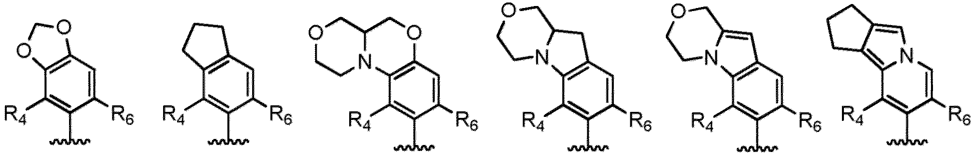

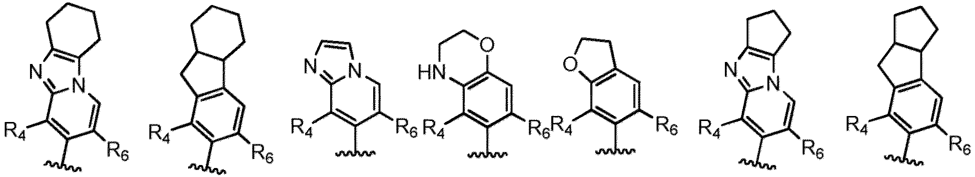

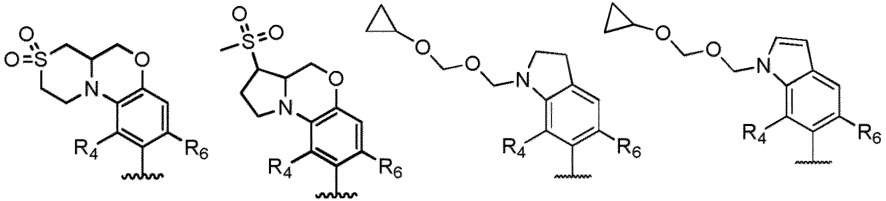

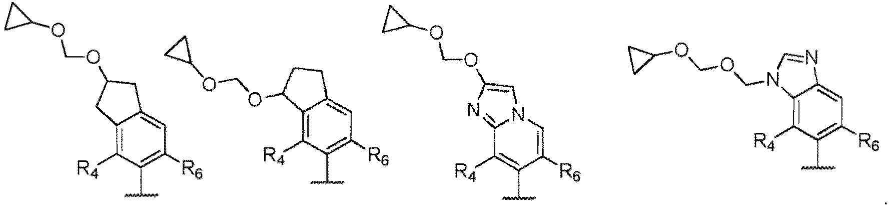

In a certain embodiment of the present disclosure, in schemes 2 to 5, 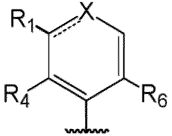 is 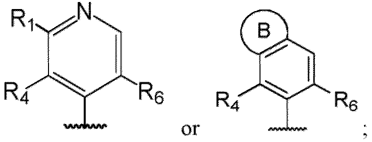
R₁ is -L₁-R₁₋₄;
L₁ is -O-;
R₁₋₄ is C₁-C₆ alkyl substituted by 1, 2, or 3 R₁₋₅ groups; R₁₋₅ is independently OH;
ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5-to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
R₁₋₁ is oxo (=O), OH, NH₂, CN, halogen, -S(O)₂-C₁-C₆ alkyl, C₁-C₆ alkyl, or C₁-C₆ alkoxy substituted by 1, 2, or 3 R₁₋₁₋₂ groups;
R₁₋₂ is OH or halogen;
R₁₋₁₋₂ is OH;
such as

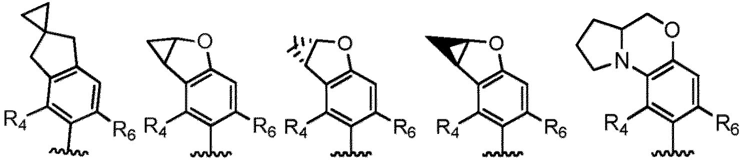

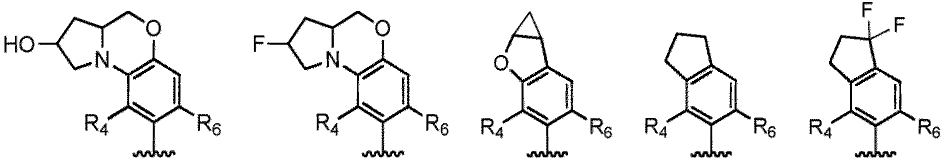

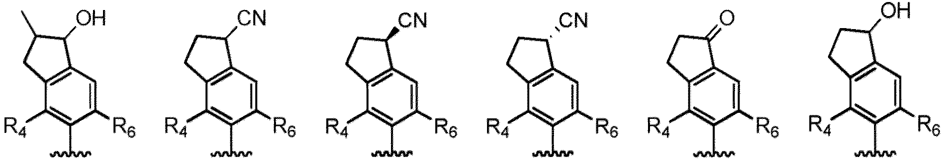

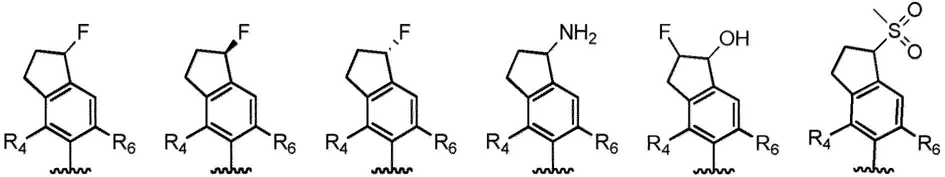

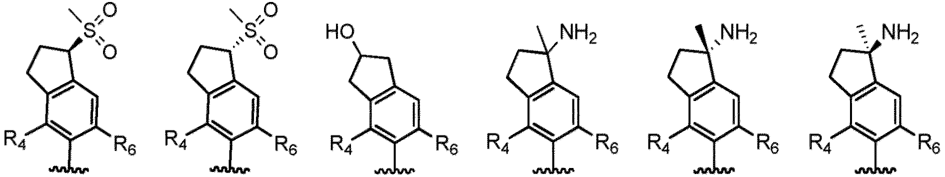

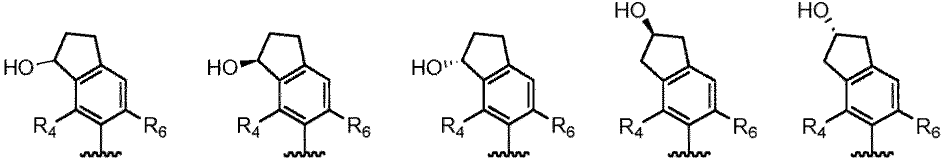

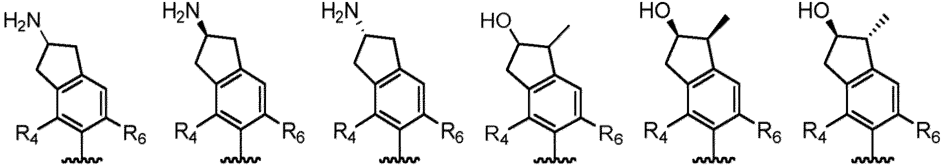

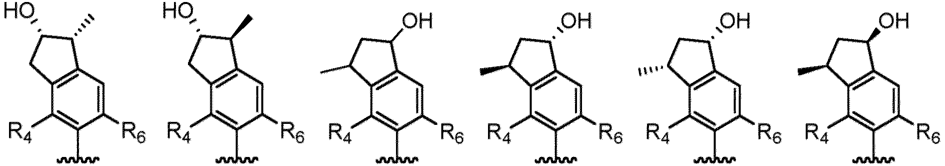

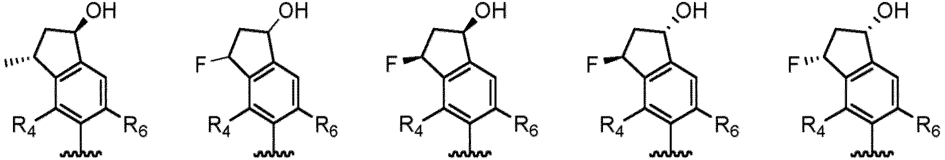

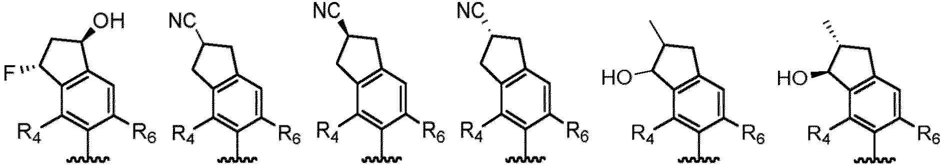

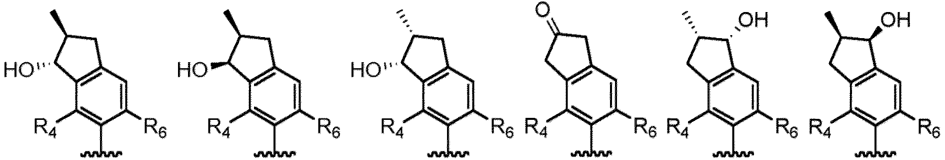

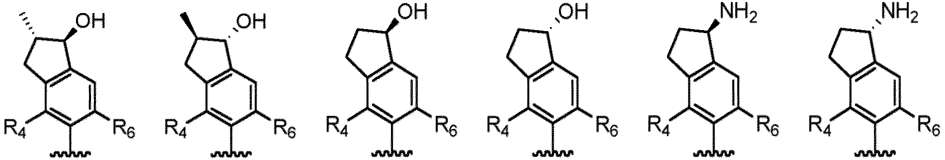

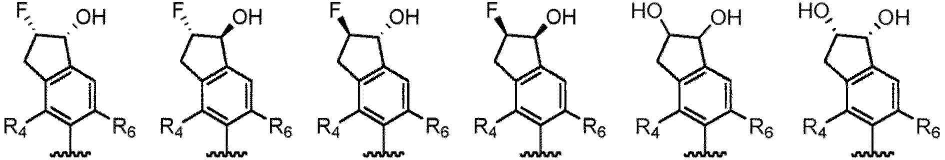

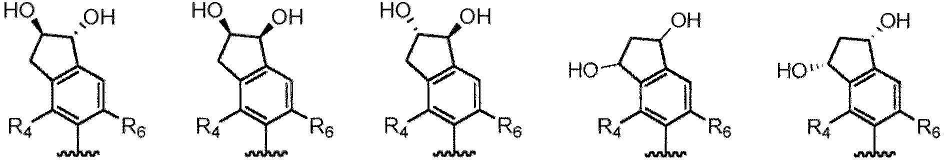

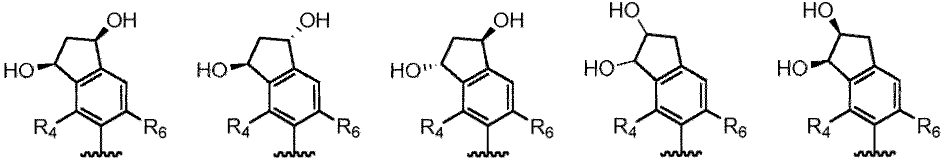

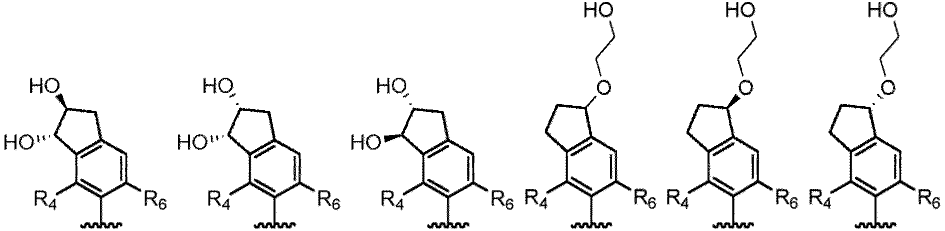

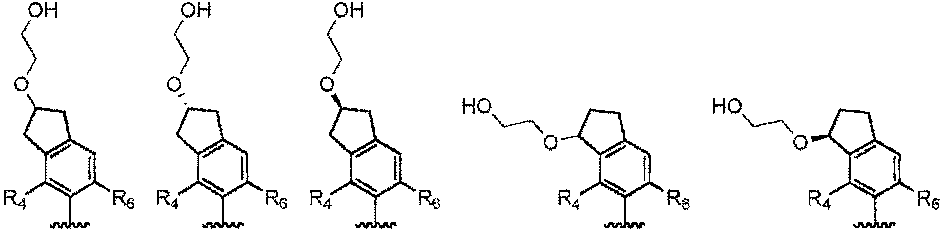

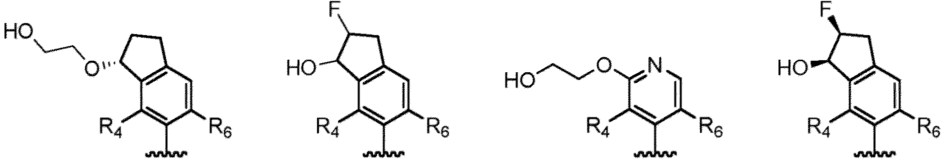

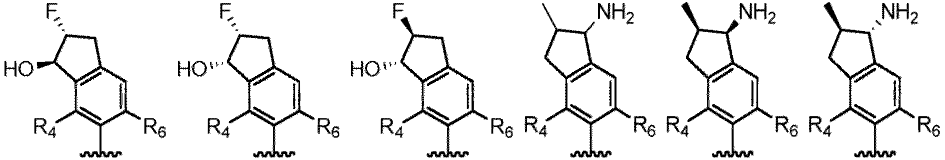

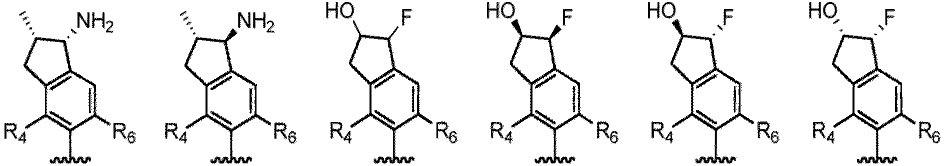

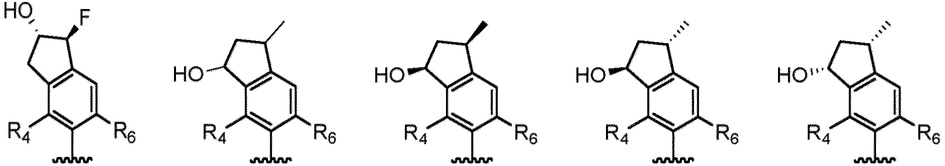

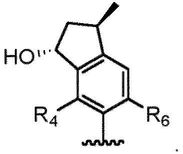

In a certain embodiment of the present disclosure, in schemes 2 to 5, 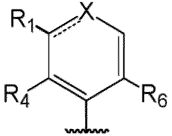 is independently 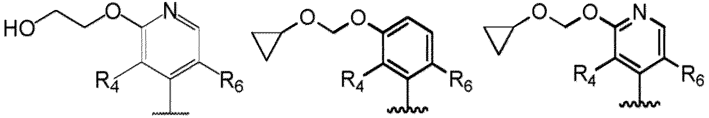 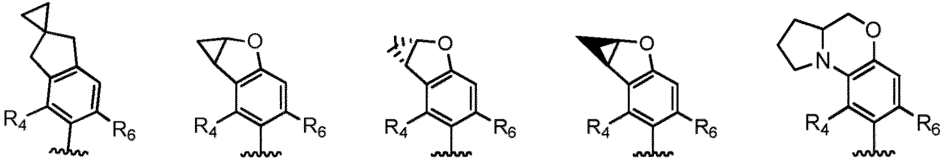 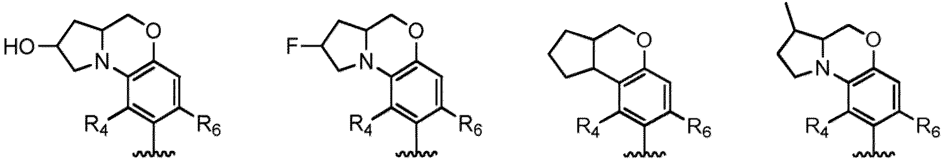 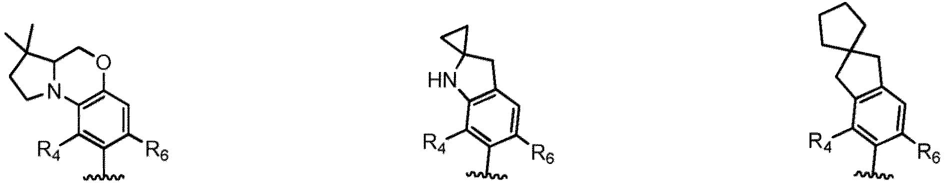 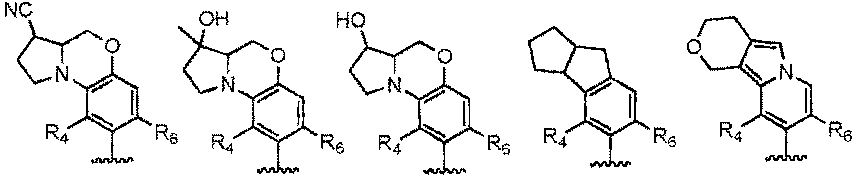 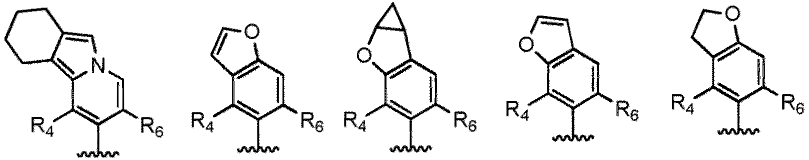 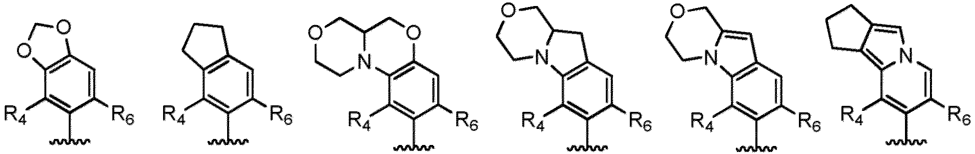 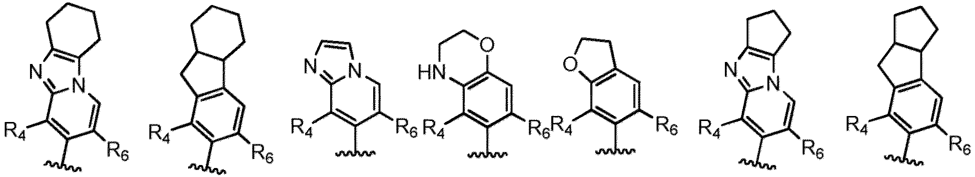 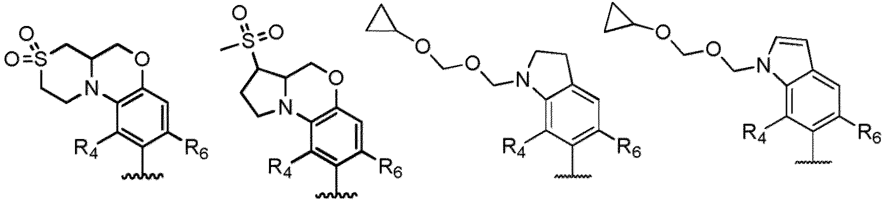 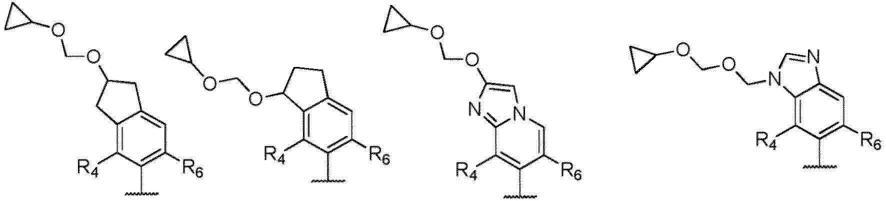 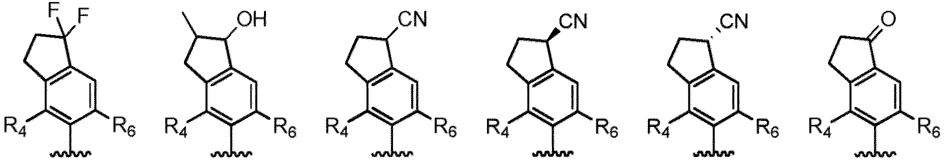 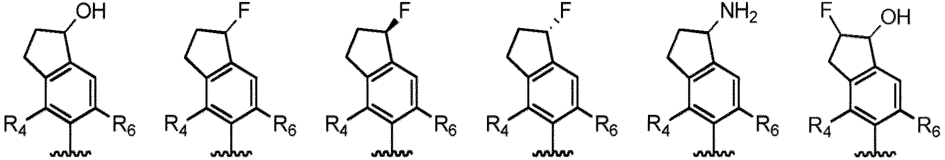 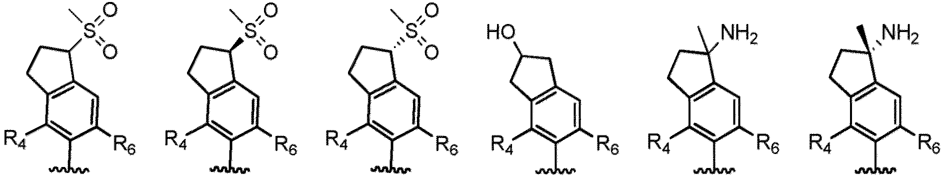 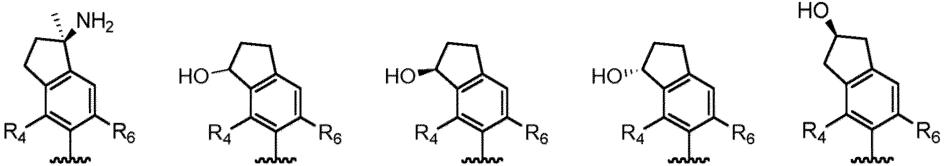 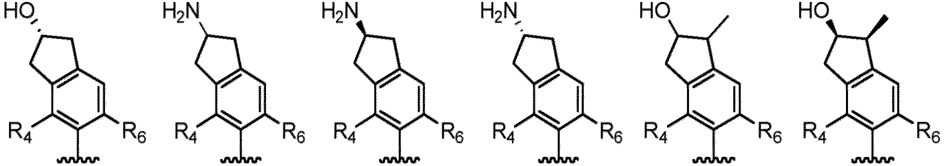 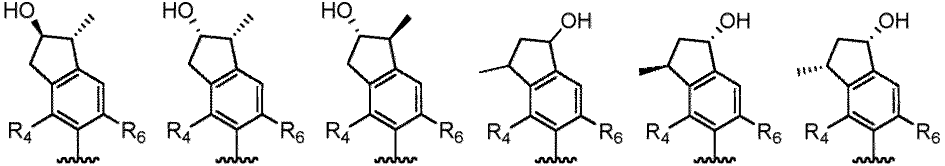 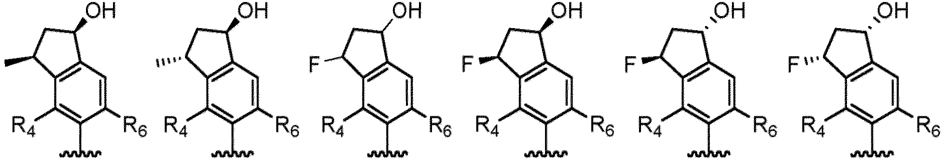 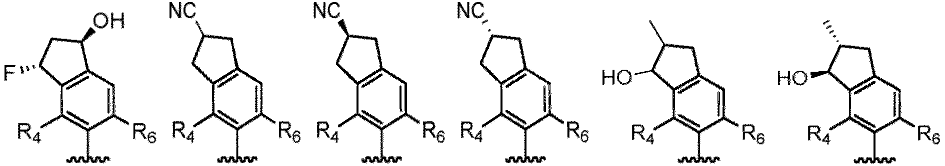 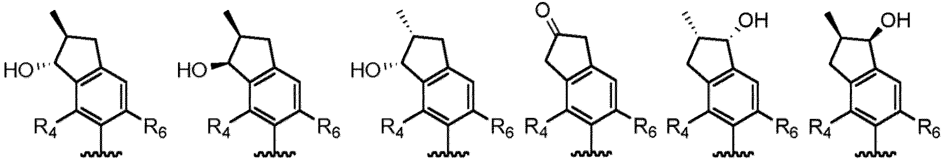 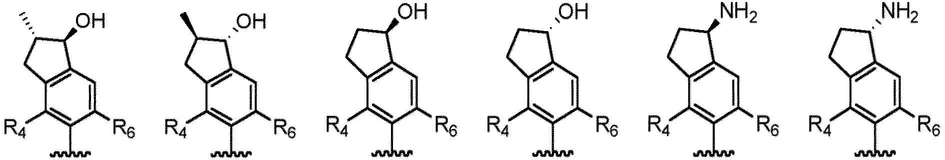 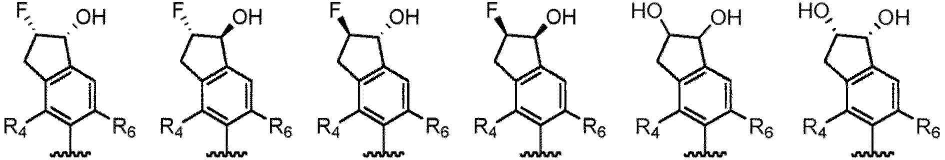 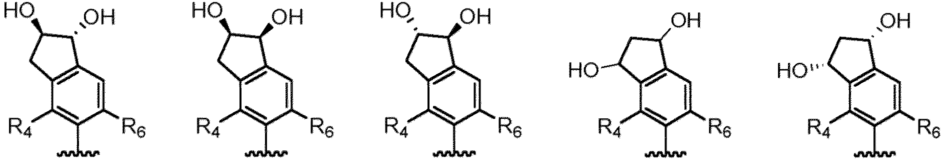 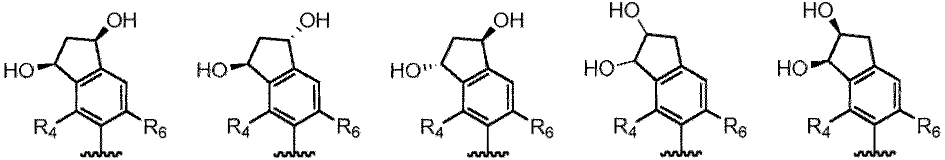 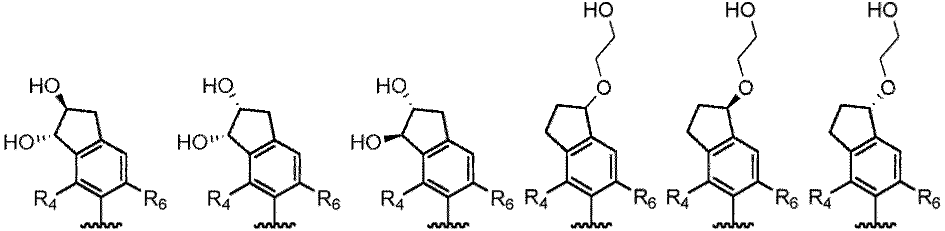 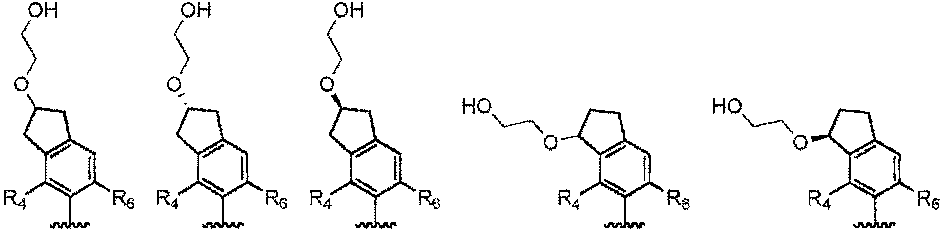 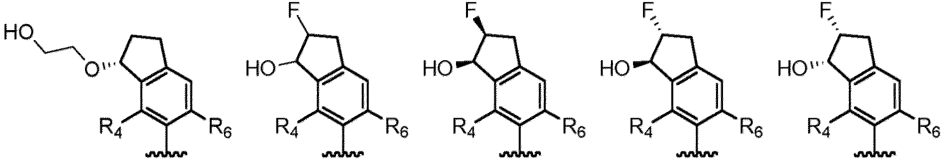 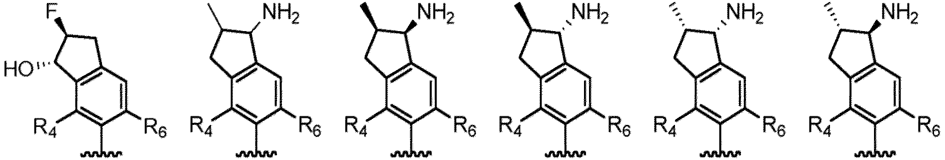 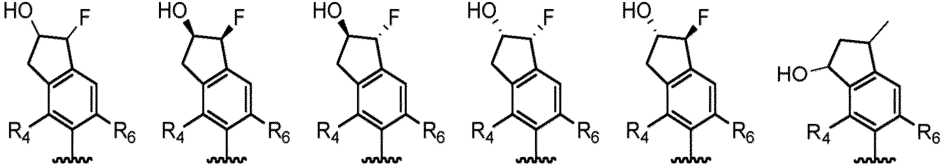 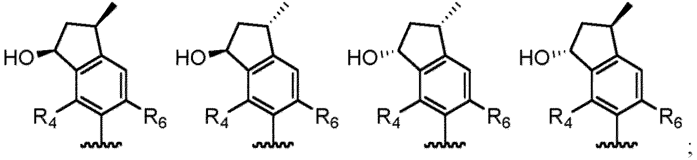
preferably 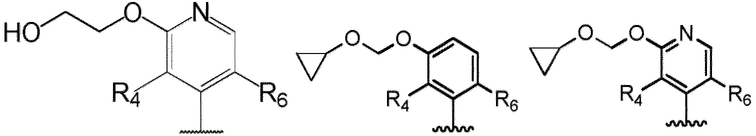 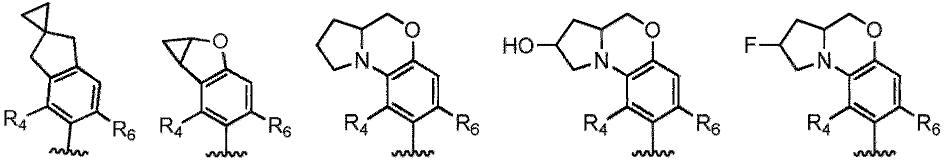 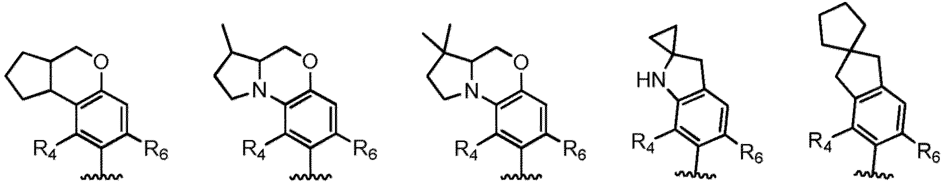 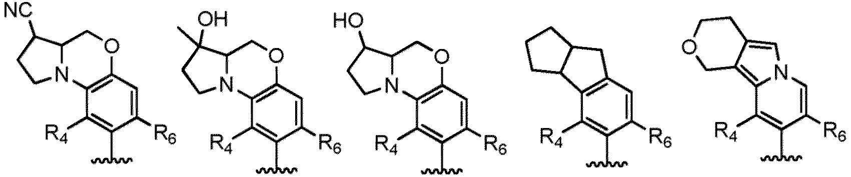 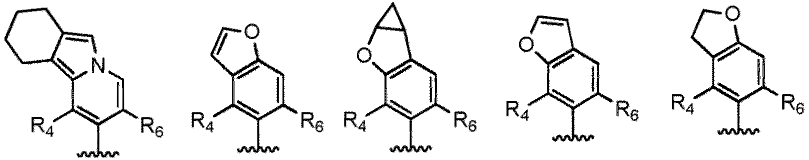 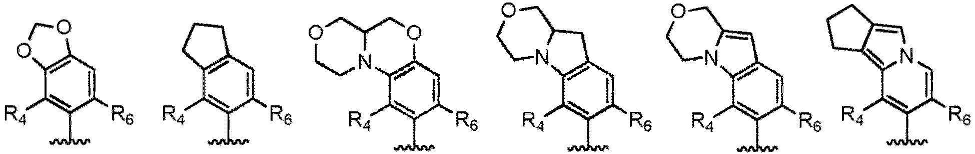 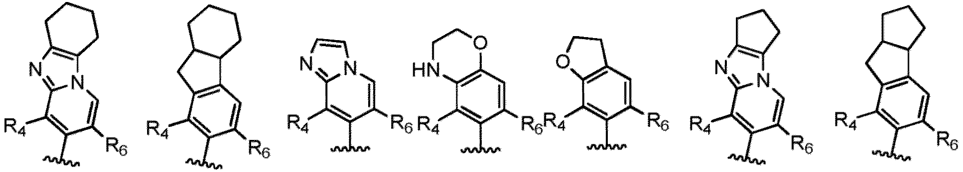 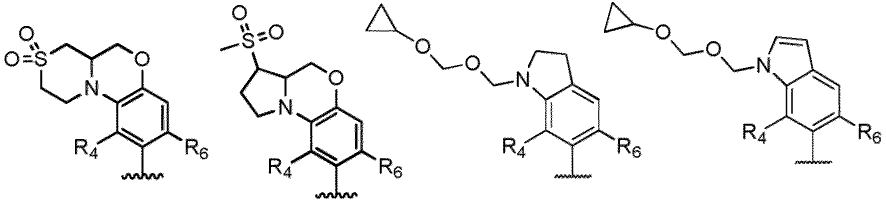 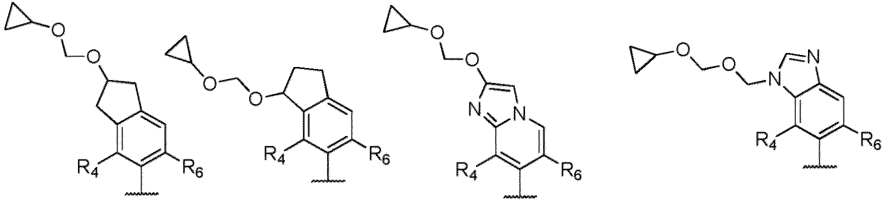 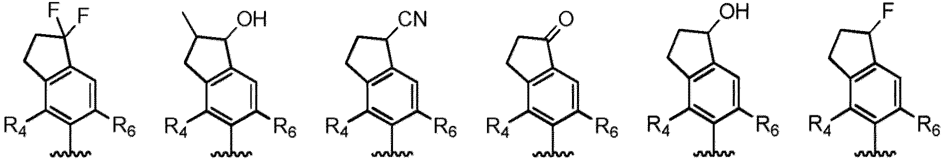 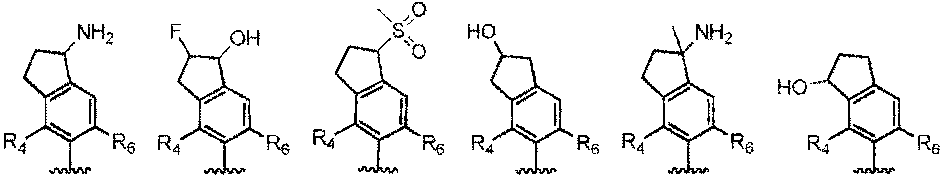 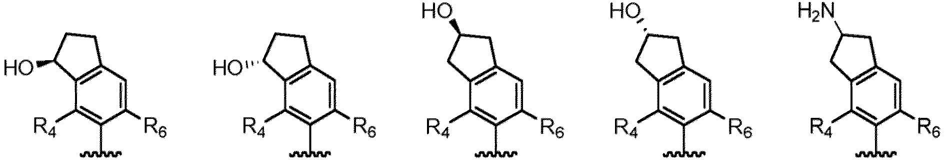 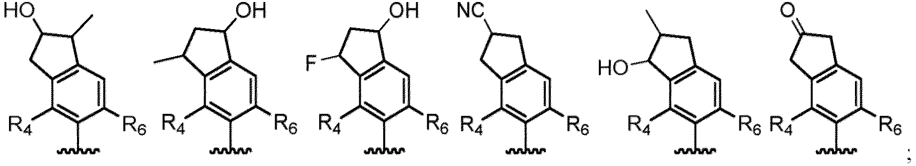
more preferably 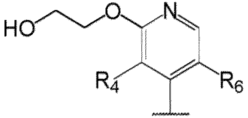 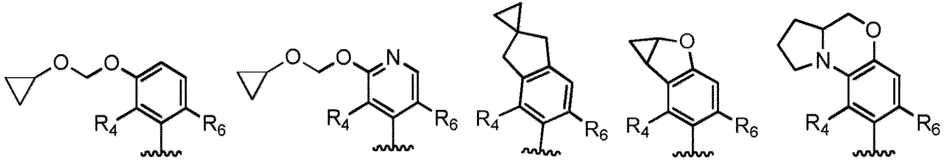 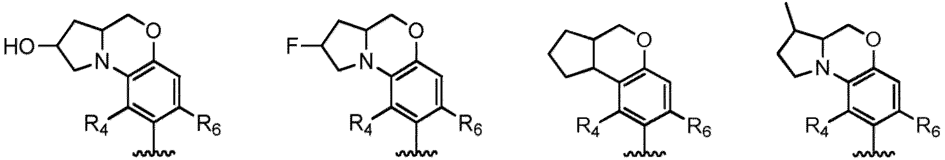 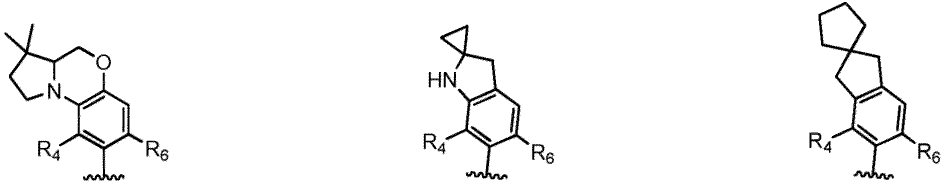 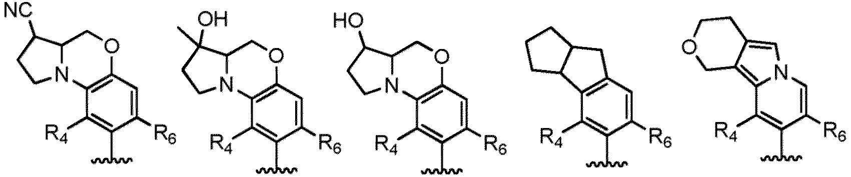 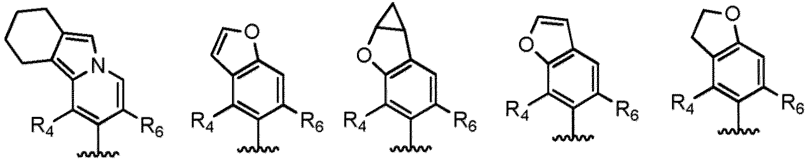 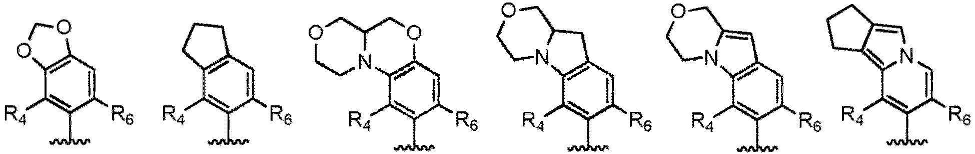 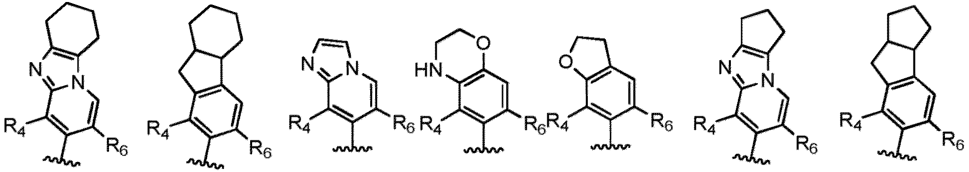 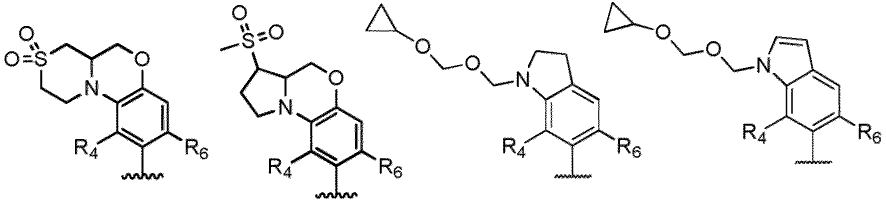 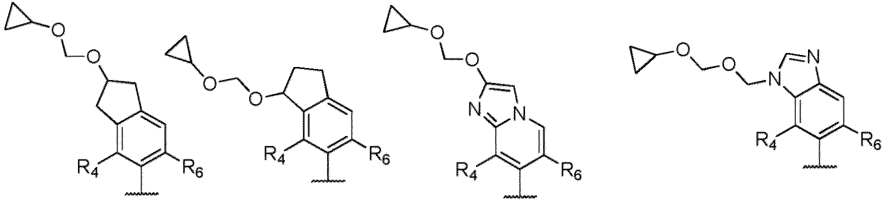 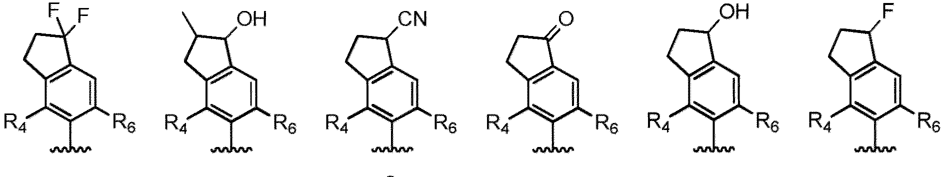 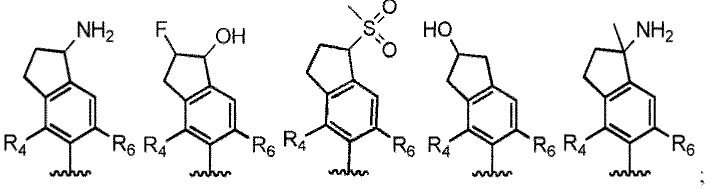
further more preferably:

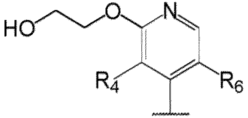

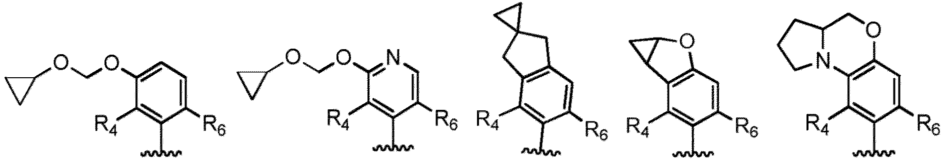

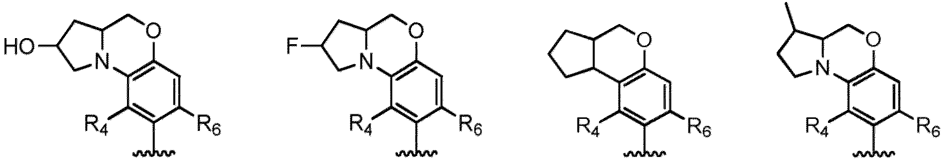

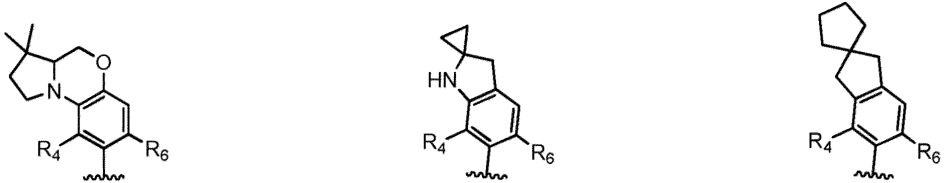

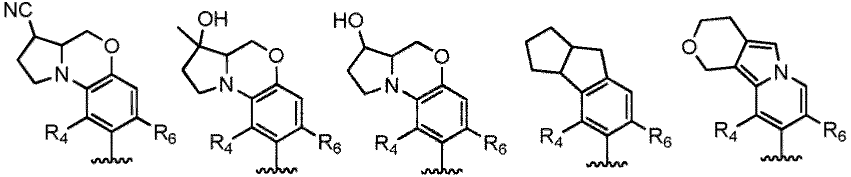

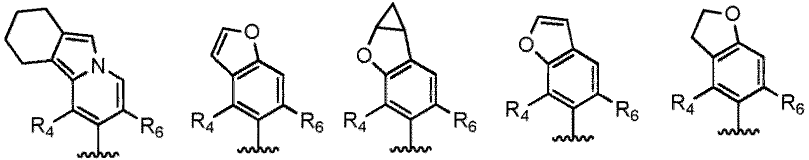

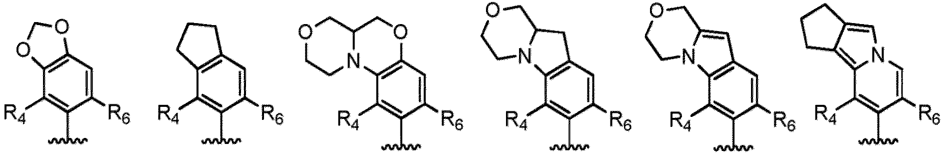

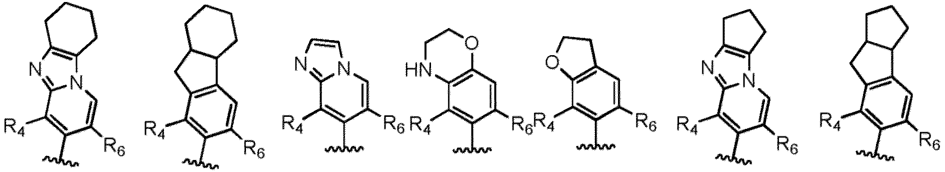

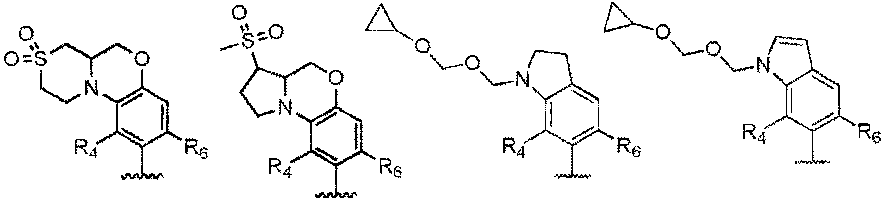

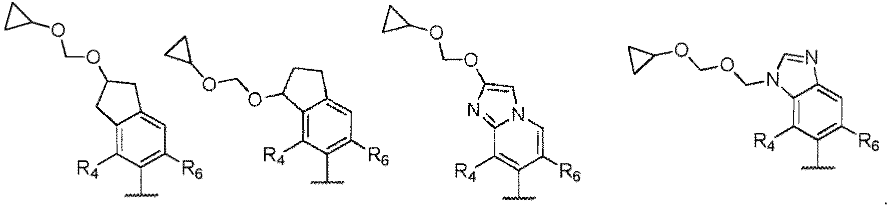

In a certain embodiment of the present disclosure, in scheme 4, R₄ is OH, NH₂, C₁-C₆ alkoxy, or C₂-C₆ alkynyl, such as

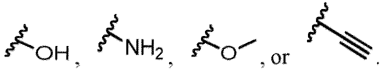

In a certain embodiment of the present disclosure, in scheme 4, R₄ is

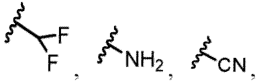

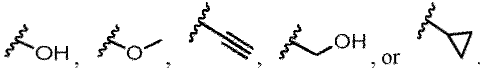

In a certain embodiment of the present disclosure, in schemes 1 to 3 and 5, R₄ is independently halogen, OH, NH₂, C₁-C₆ alkoxy, or C₂-C₆ alkynyl, such as F,

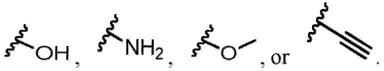

In a certain embodiment of the present disclosure, in schemes 1 to 3 and 5, R₄ is independently

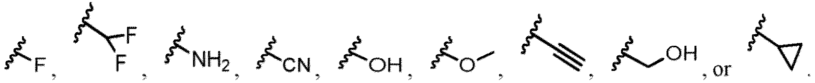

In a certain embodiment of the present disclosure, in scheme 5, R₃ is

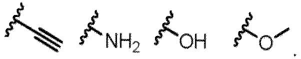

In a certain embodiment of the present disclosure, in schemes 1 to 4, R₃ is independently

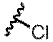

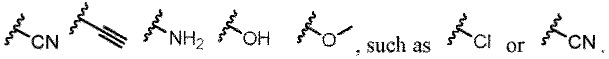

In a certain embodiment of the present disclosure, in scheme 3, A is

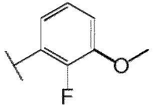

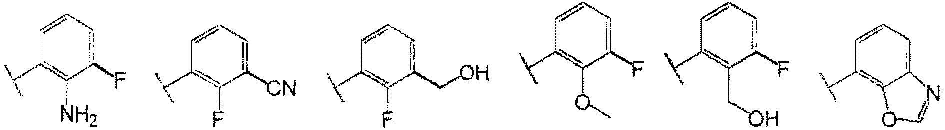

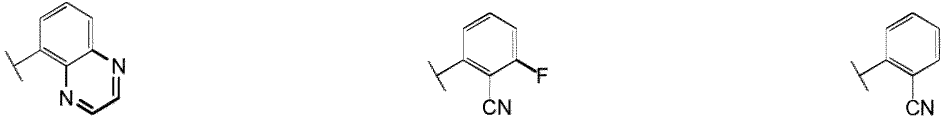

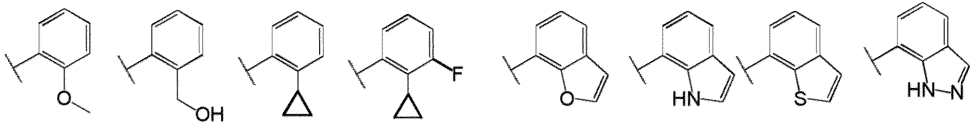

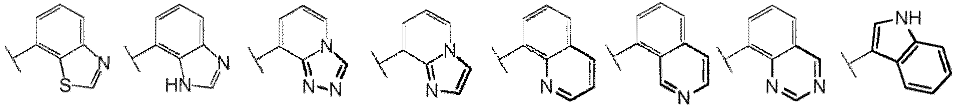

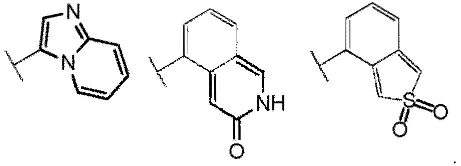

In a certain embodiment of the present disclosure, in schemes 1 to 2 and 4 to 5, A is independently

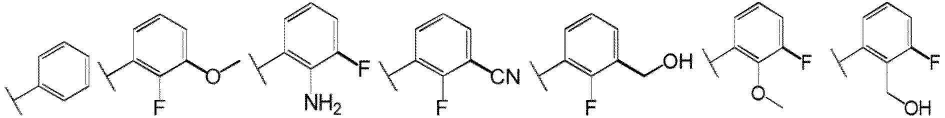

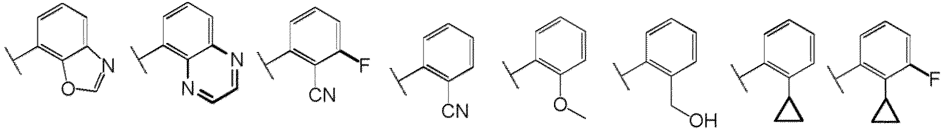

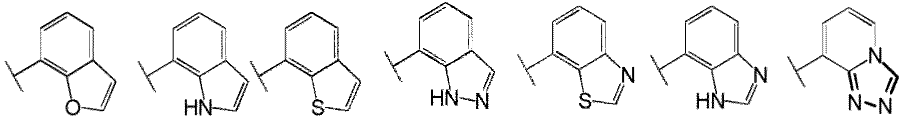

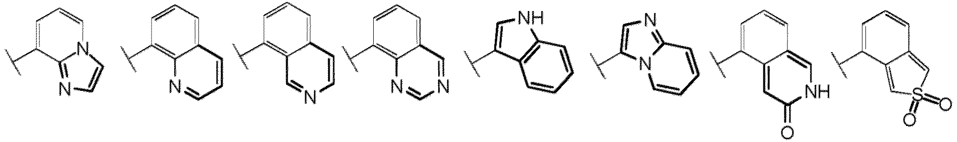

In a certain embodiment of the present disclosure, in scheme 2, Q is

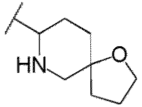

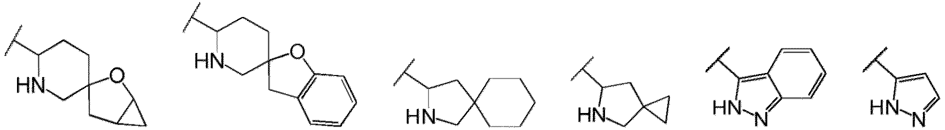

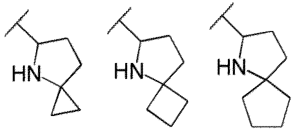

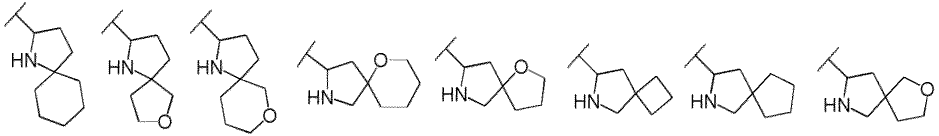

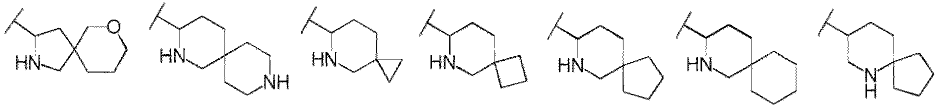

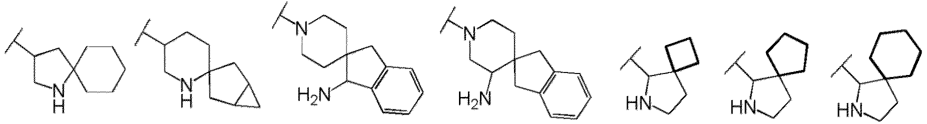

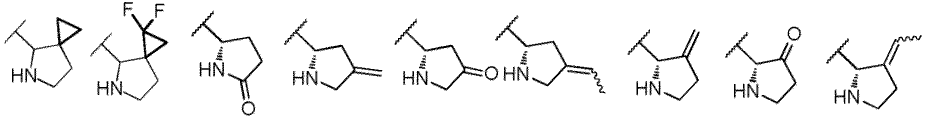

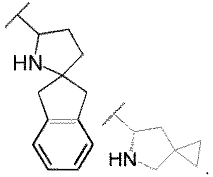

In a certain embodiment of the present disclosure, in schemes 1 and 3 to 5, Q is

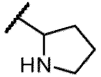

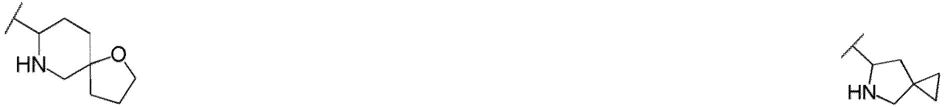

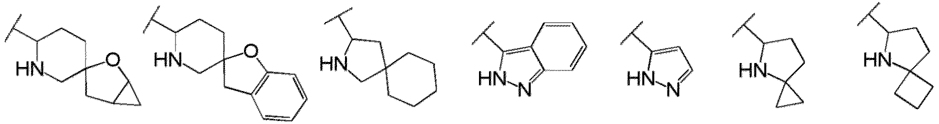

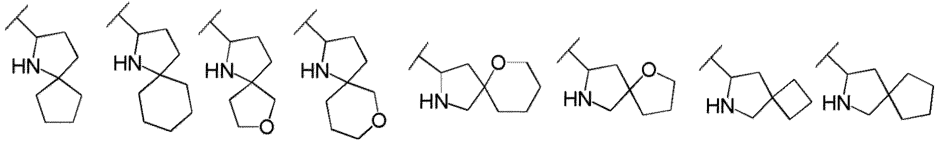

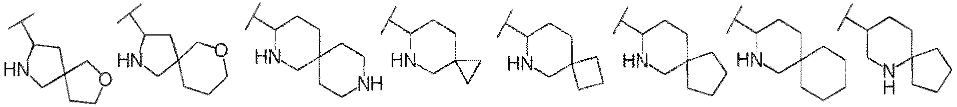

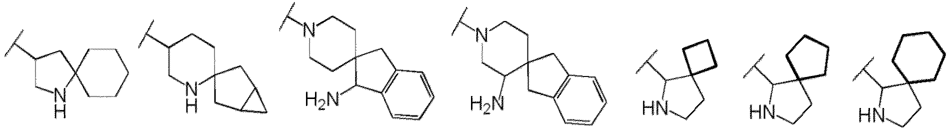

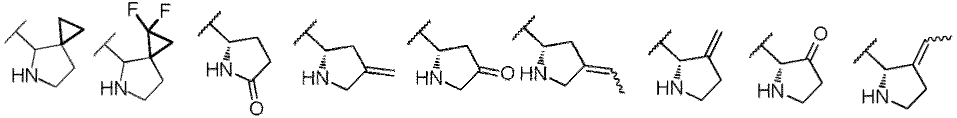

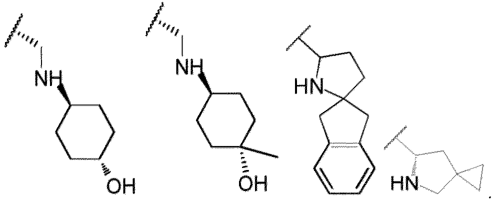

In a certain embodiment of the present disclosure, the compound of formula (I) is as follows: 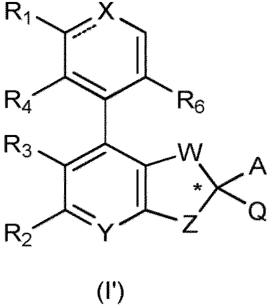
wherein a carbon atom with "*" indicates that when it is a chiral carbon atom, it is in S configuration, R configuration, or a mixture thereof;
R₁, R₂, R₃, R₄, R₆, X, Y, W, Z, A, and Q are as defined in any one of the present disclosure.

In a certain embodiment of the present disclosure, the compound of formula (I) is as follows: 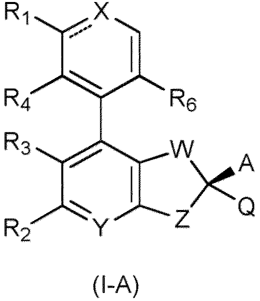 wherein R₁, R₂, R₃, R₄, R₆, X, Y, W, Z, A, and Q are as defined in any one of the present disclosure.

In a certain embodiment of the present disclosure, the compound of formula (I) is as follows: 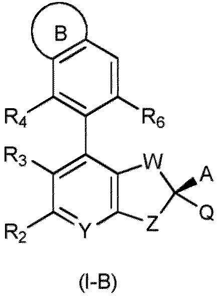 wherein ring B, R₂, R₃, R₄, R₆, Y, W, Z, A, and Q are as defined in any one of the present disclosure.

In a certain embodiment of the present disclosure, the compound of formula (I) is as follows: 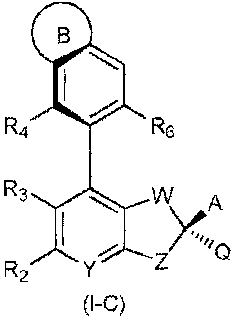 wherein ring B, R₂, R₃, R₄, R₆, Y, W, Z, A, and Q are as defined in any one of the present disclosure.

In a certain embodiment of the present disclosure, the compound of formula (I) is a compound of formula (I-D) or a compound of formula (I-E): 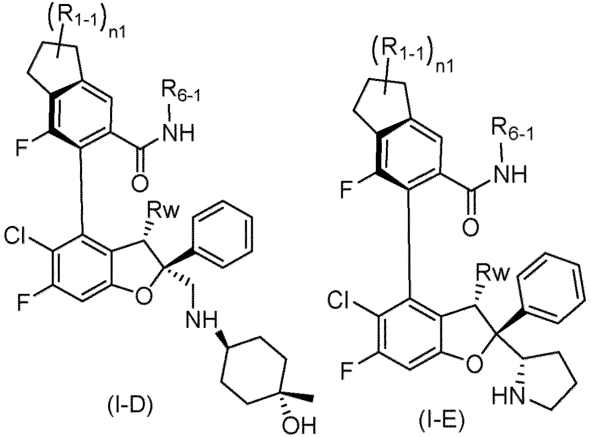 wherein n1 is 0, 1, 2, or 3; R₁₋₁, R₆₋₁, and R_{w} are as defined in any one of the present disclosure.

In a certain embodiment of the present disclosure, the compound of formula (I) is any one of the following compounds:

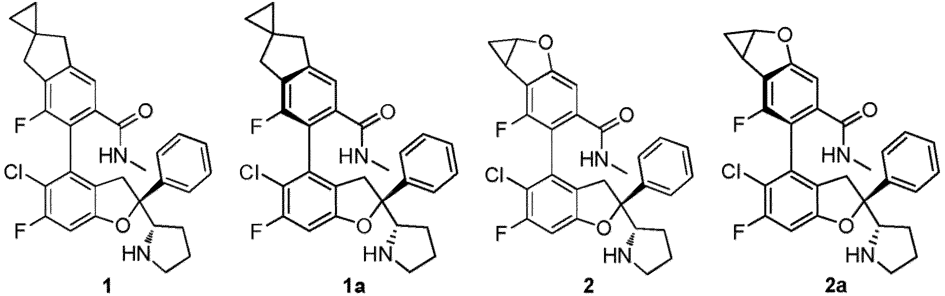

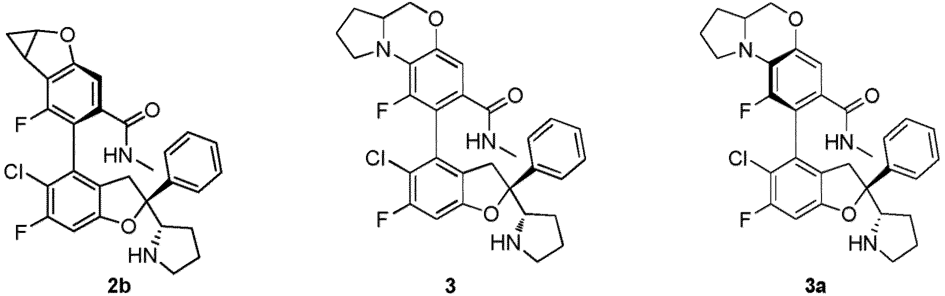

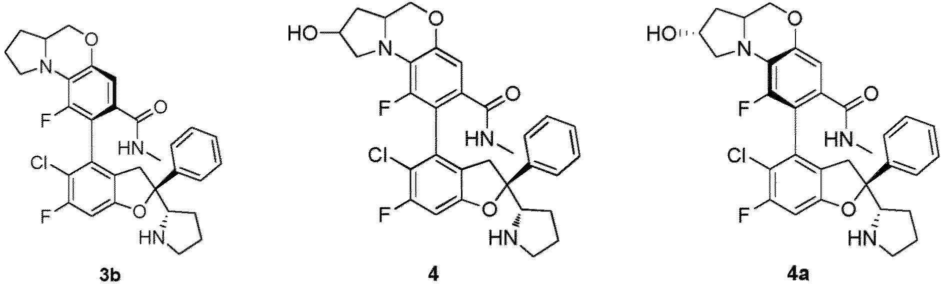

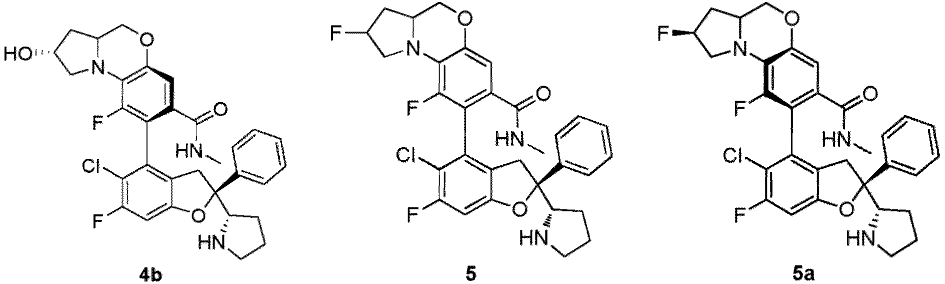

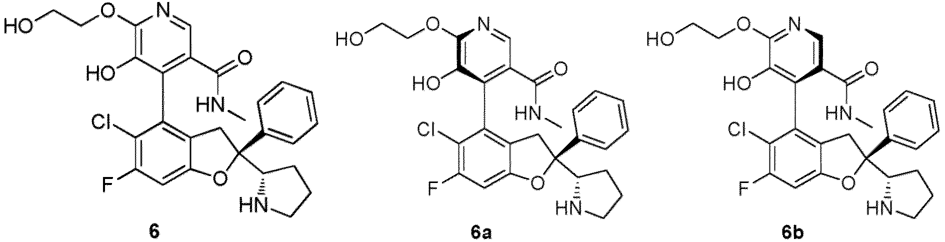

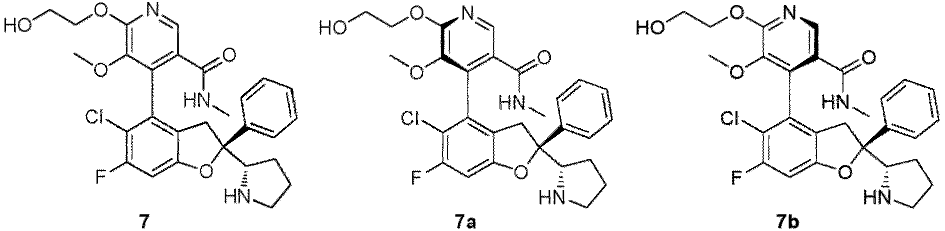

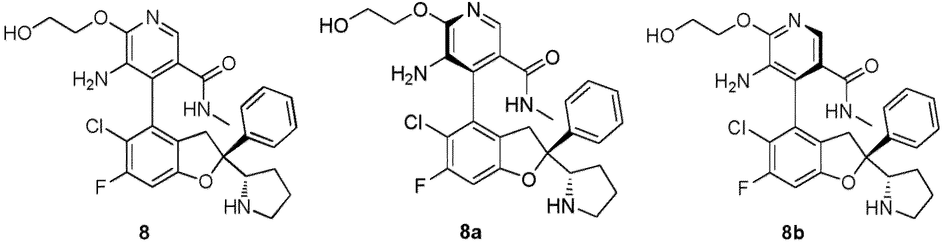

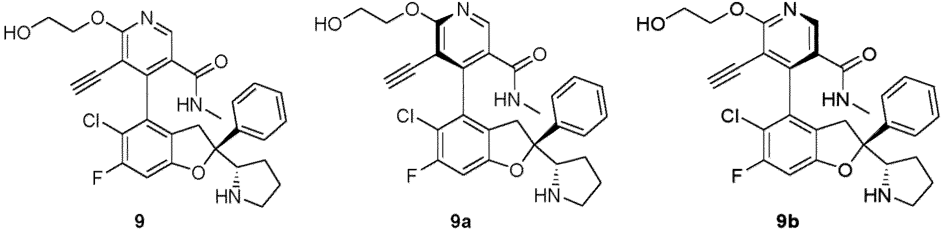

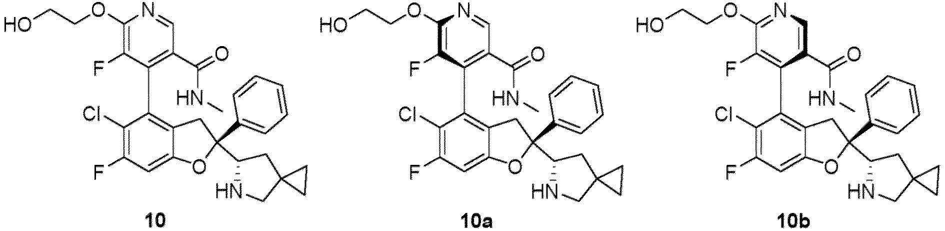

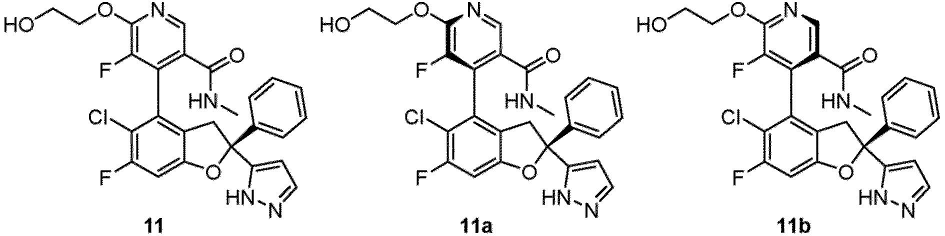

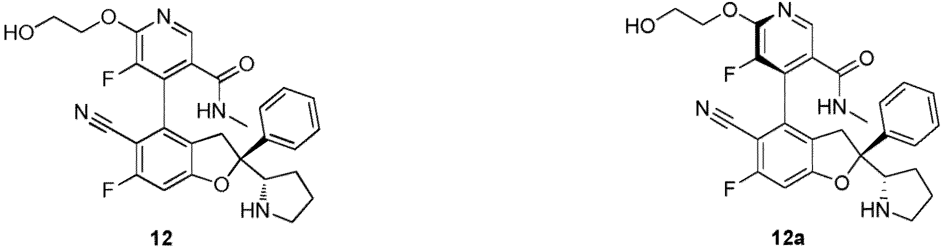

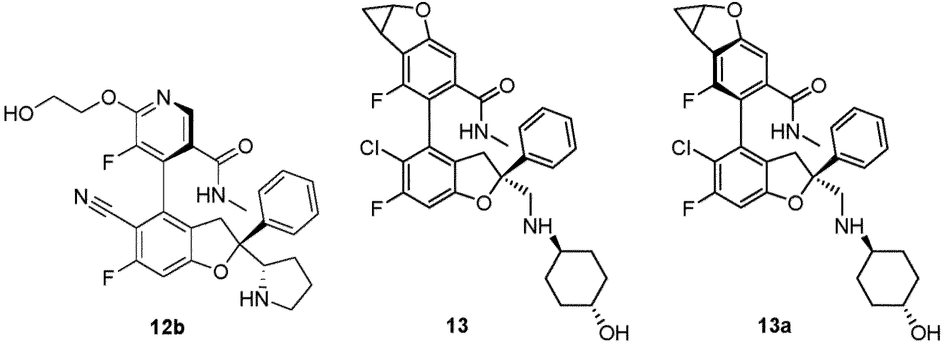

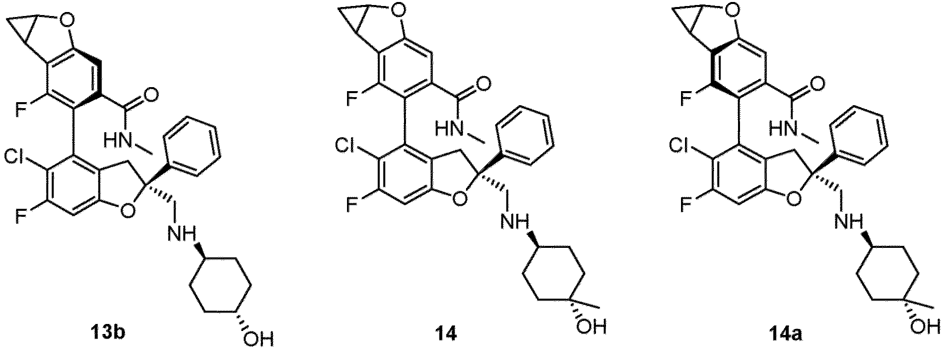

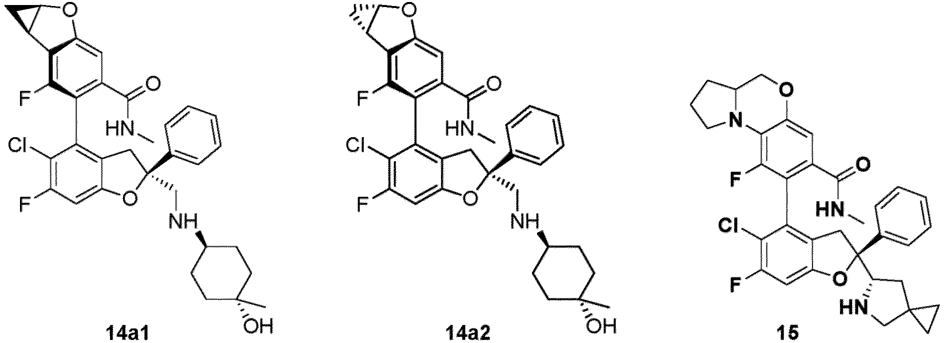

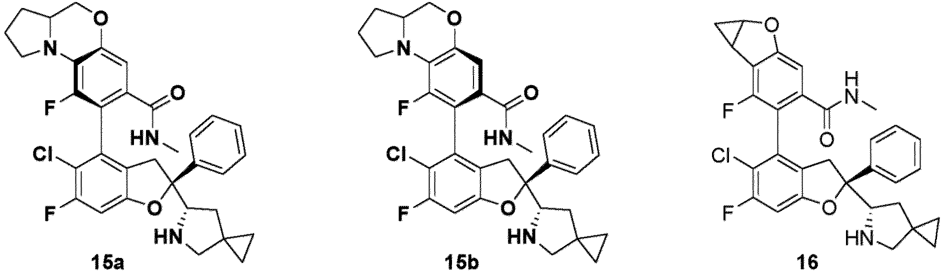

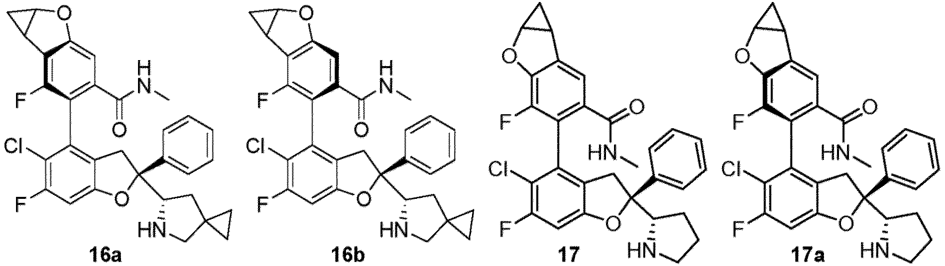

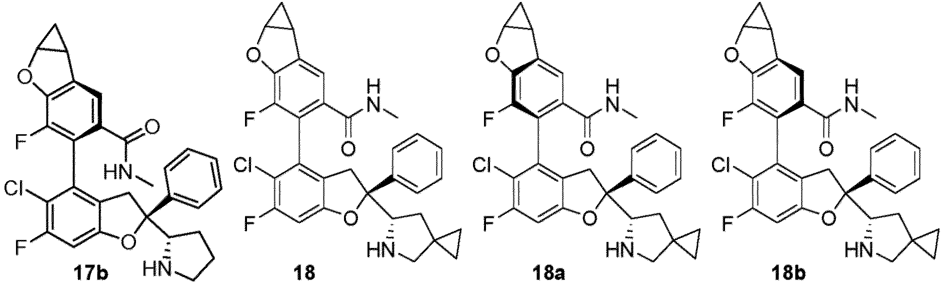

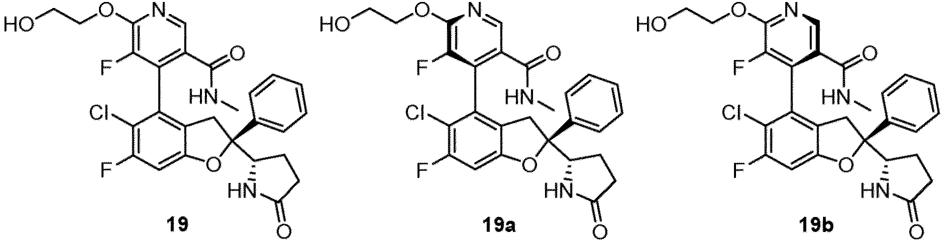

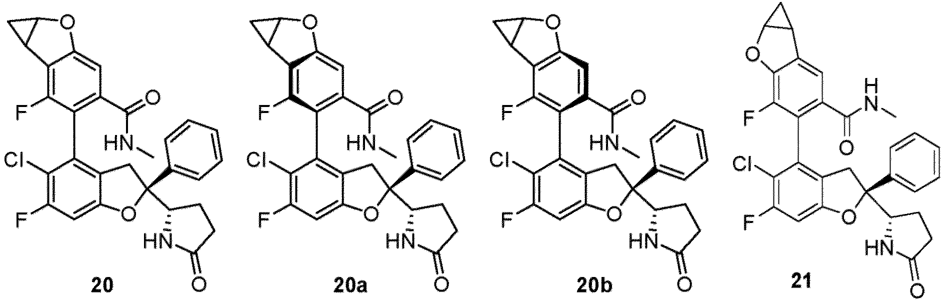

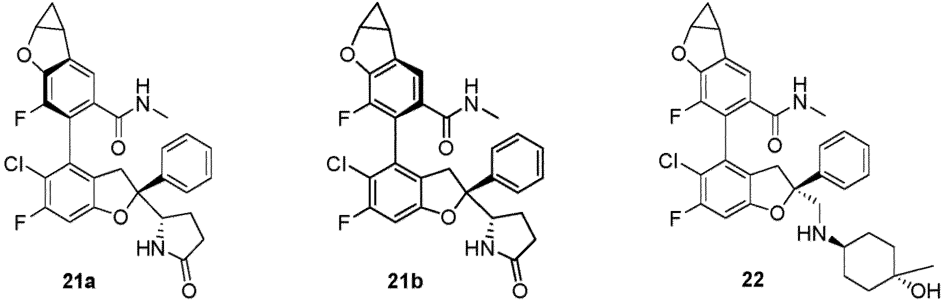

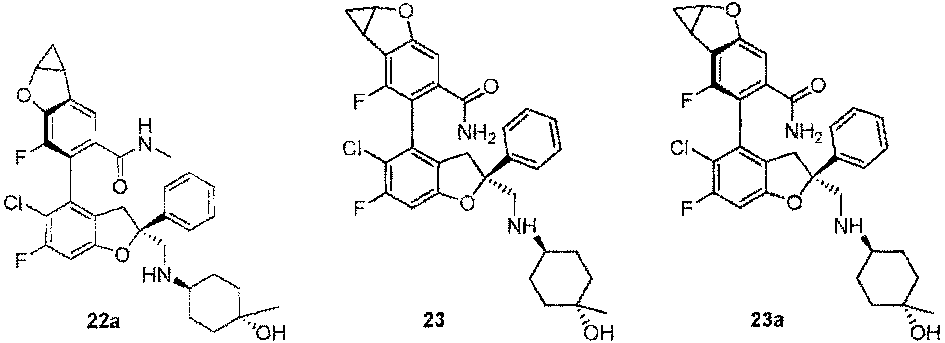

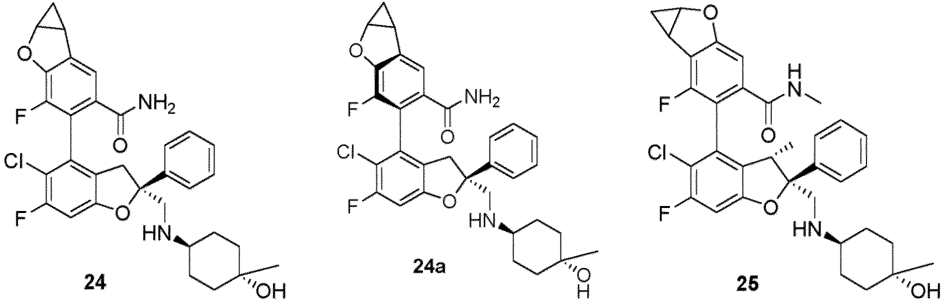

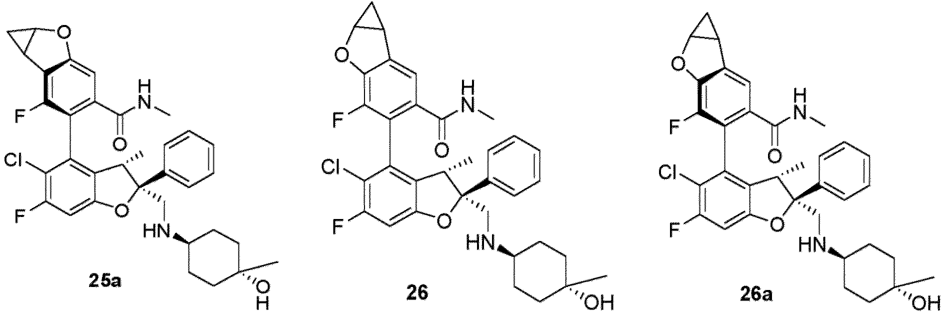

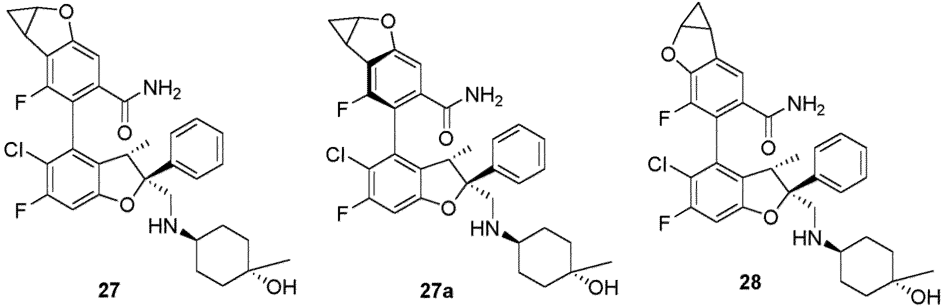

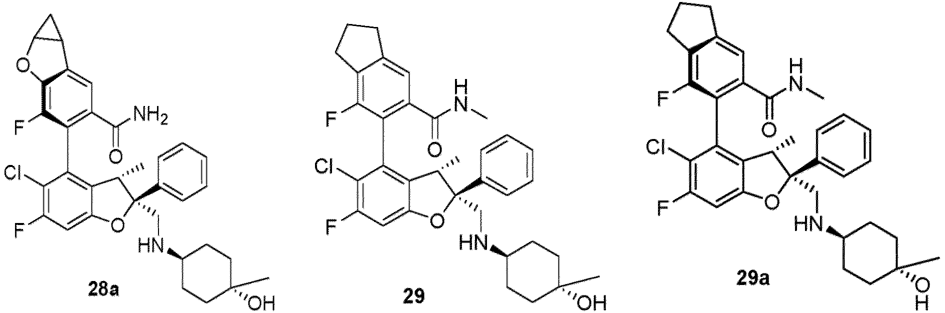

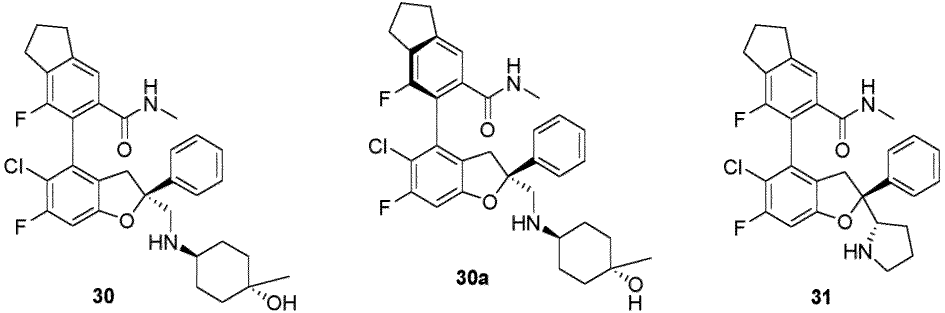

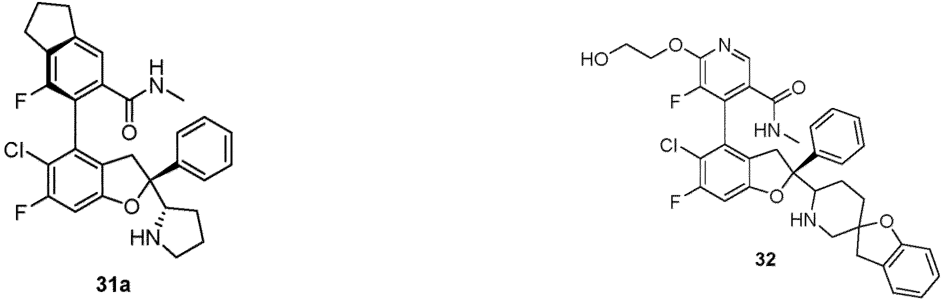

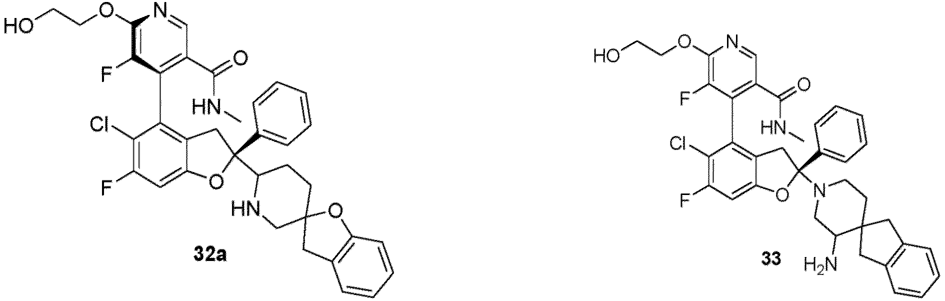

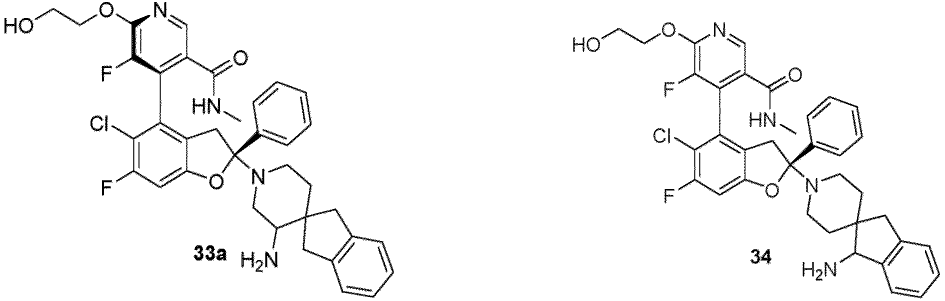

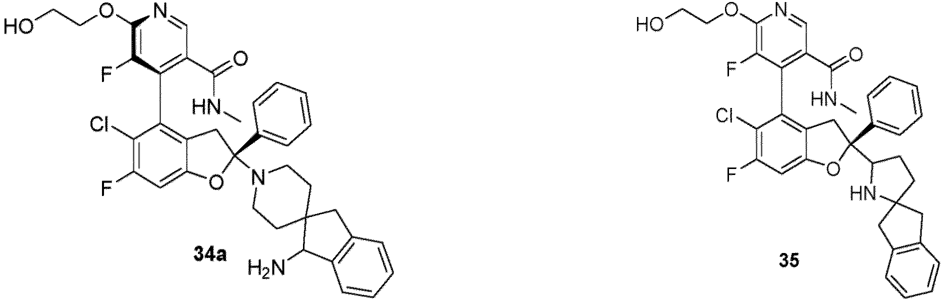

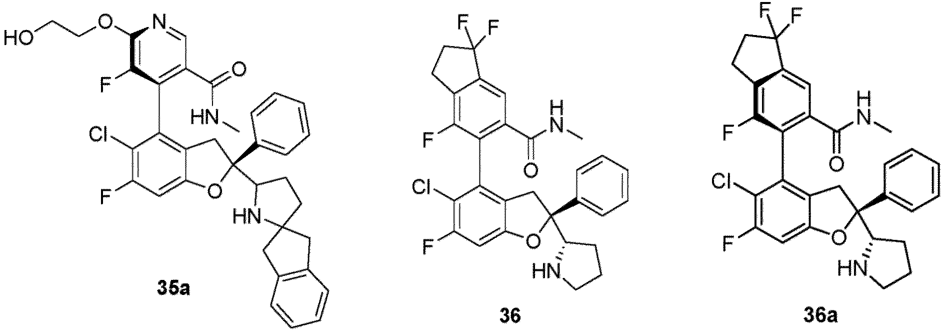

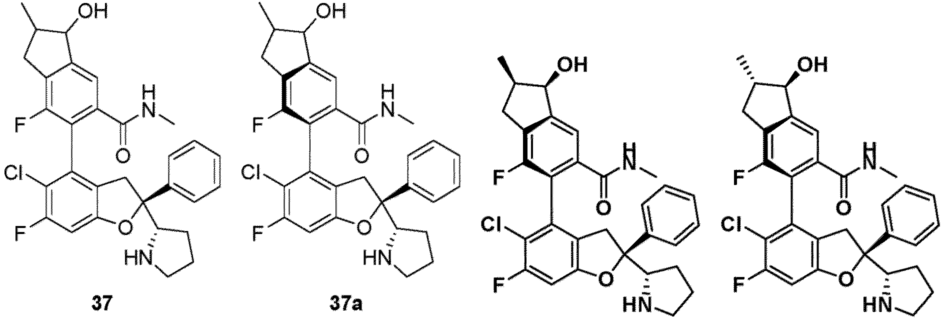

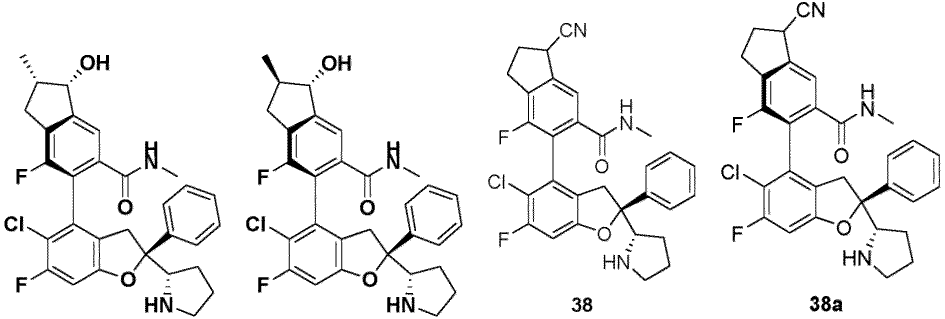

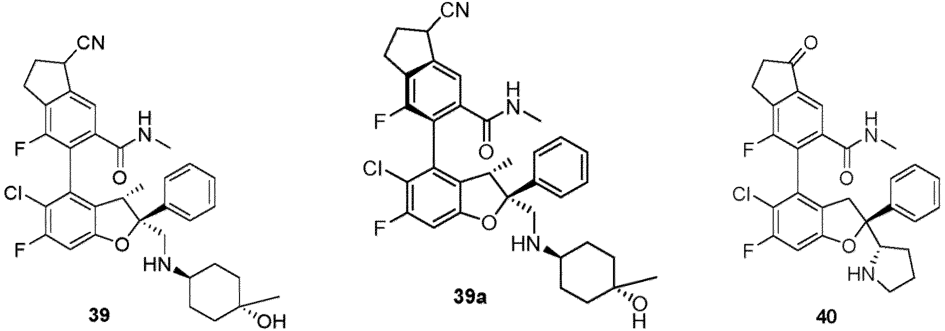

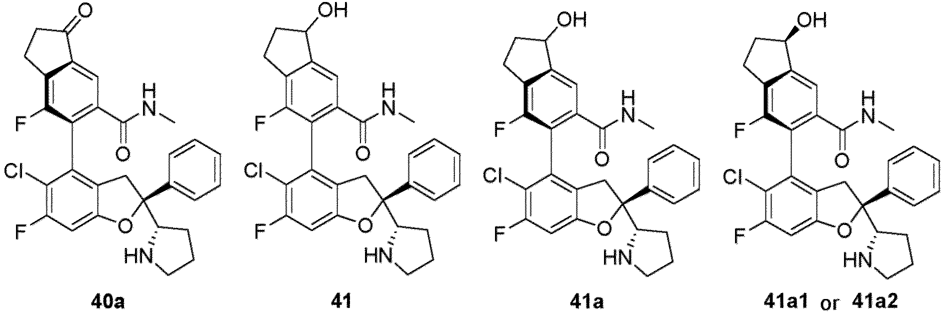

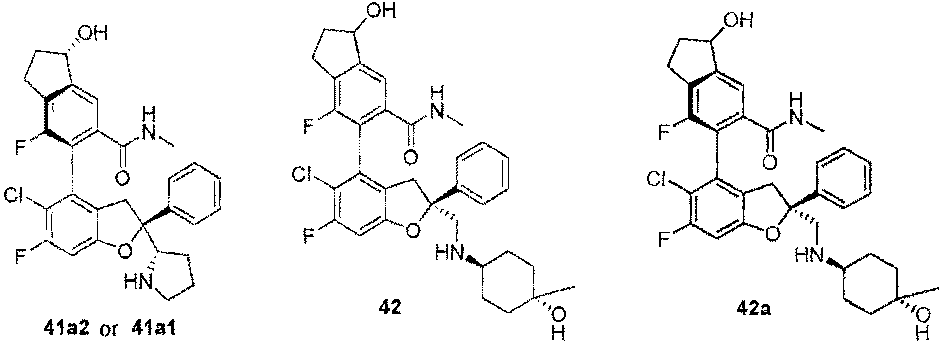

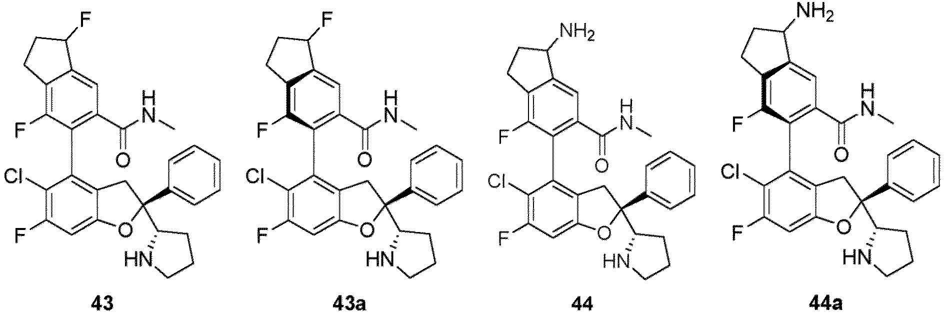

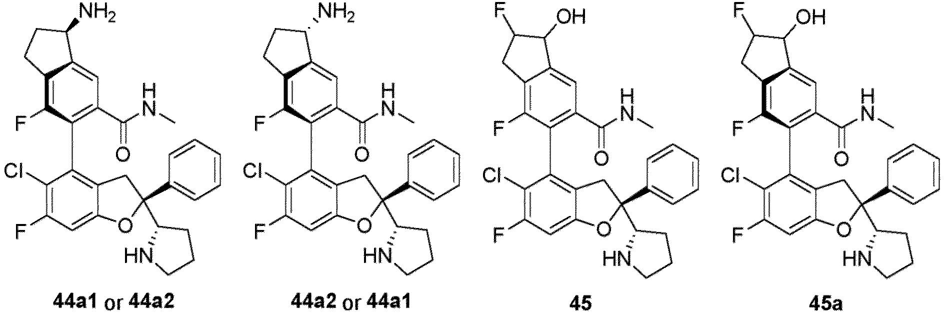

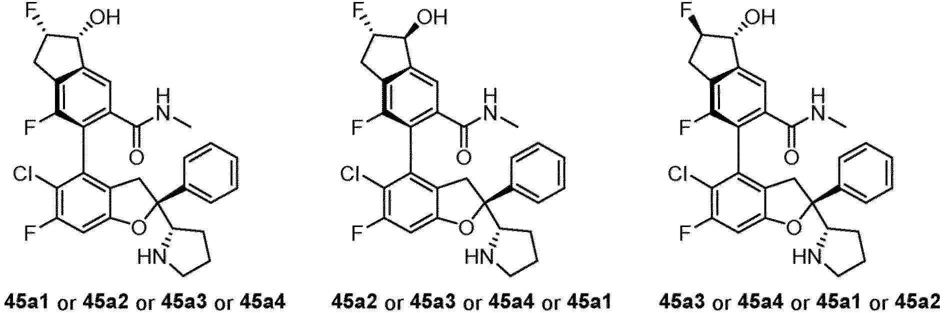

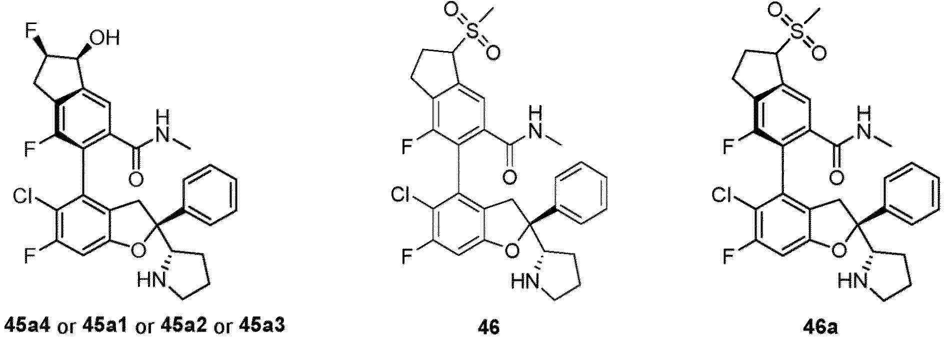

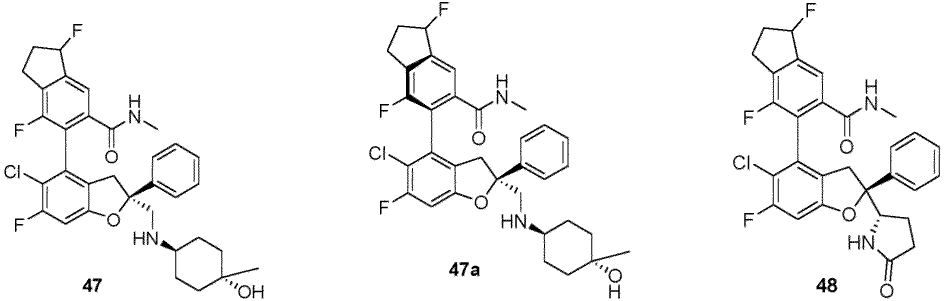

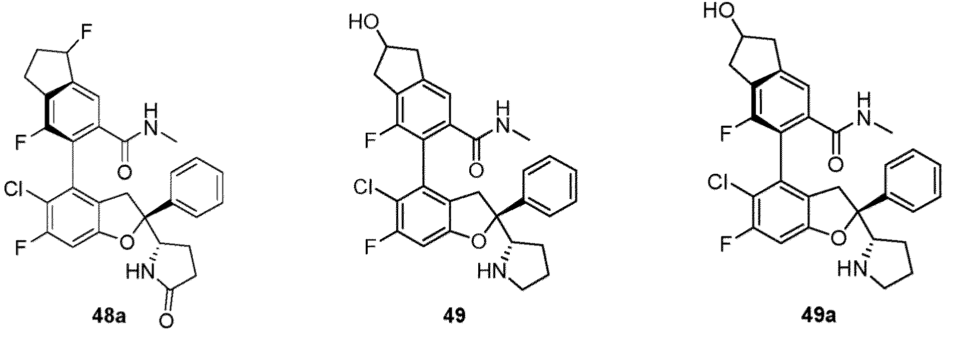

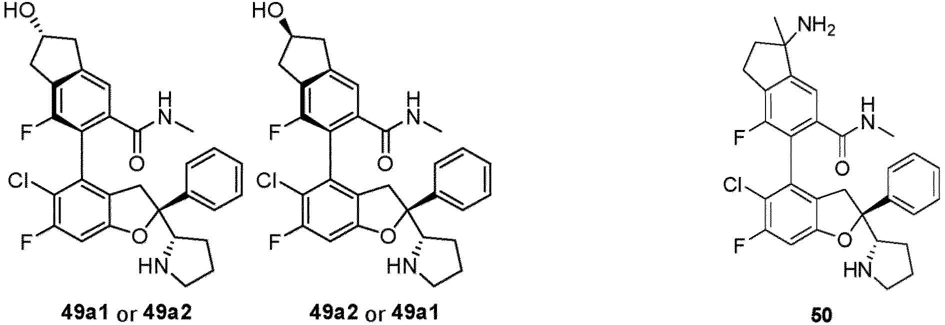

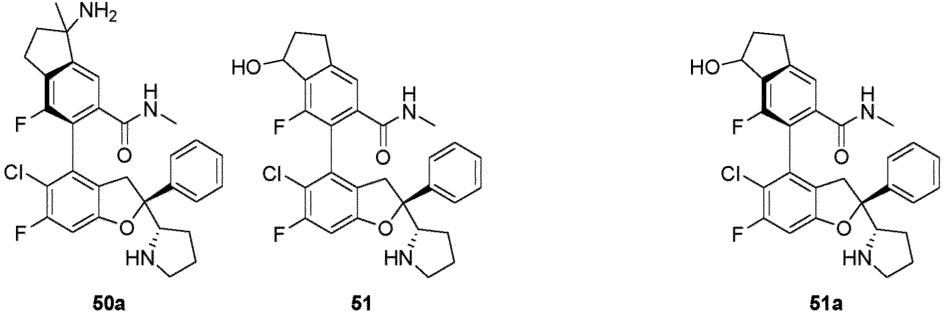

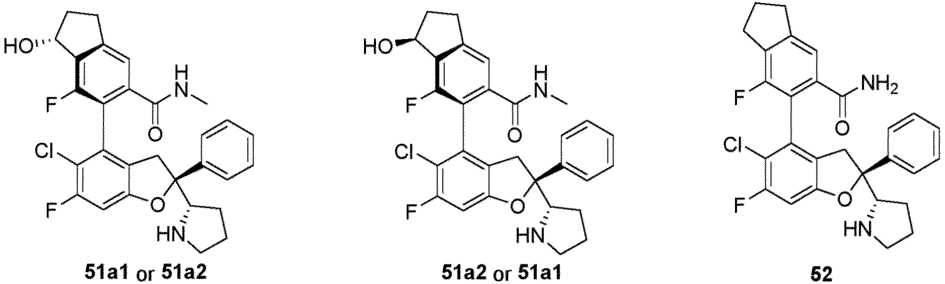

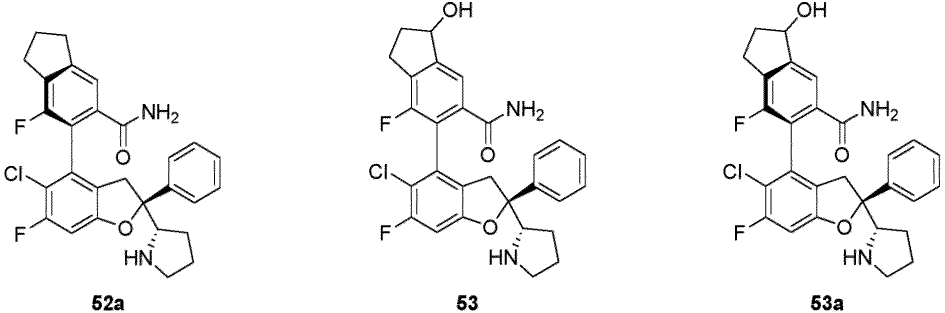

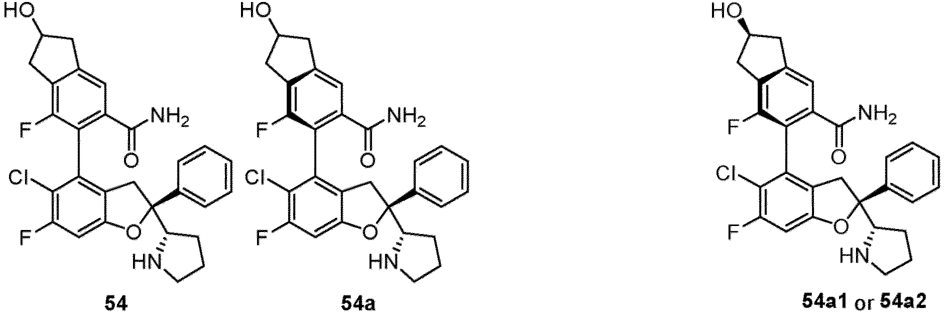

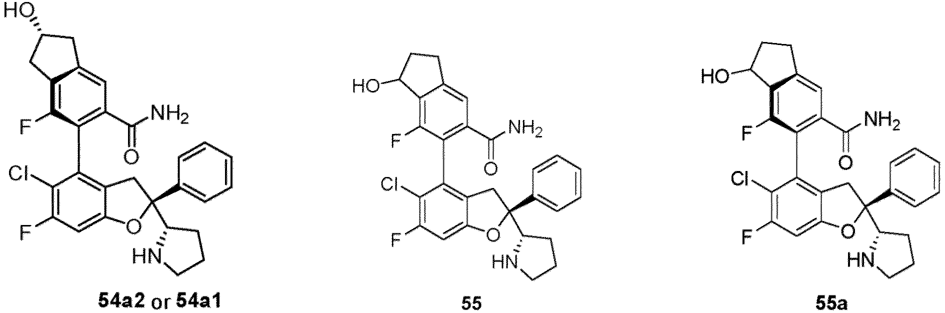

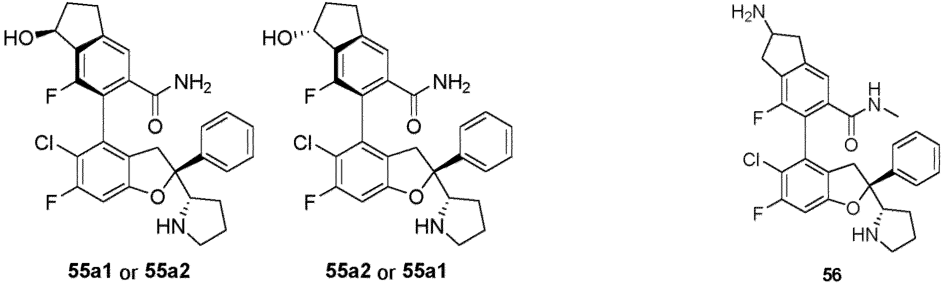

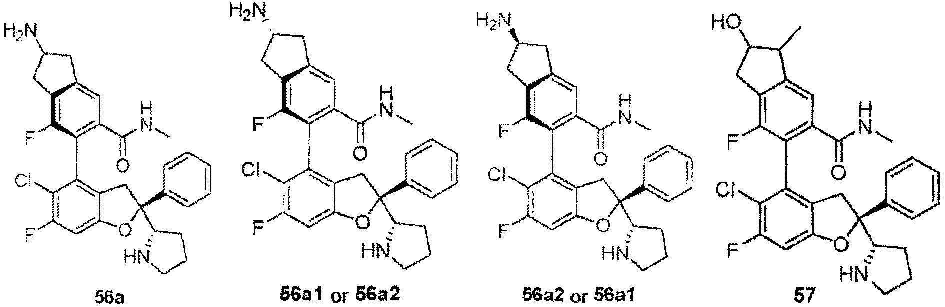

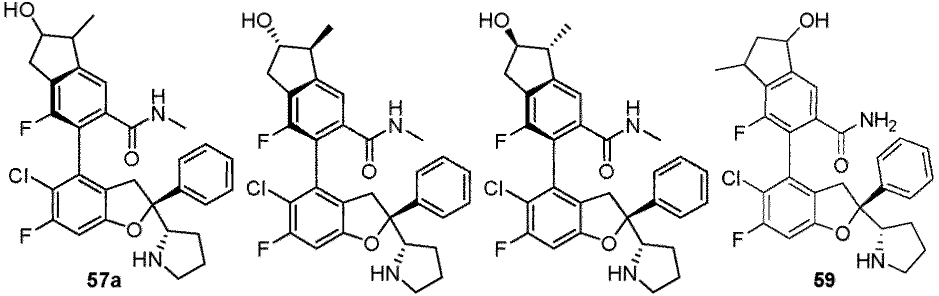

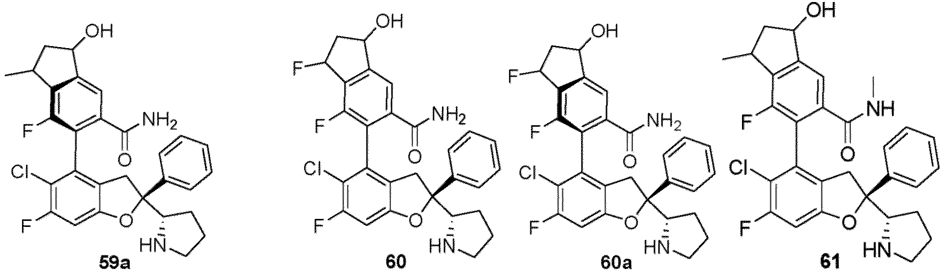

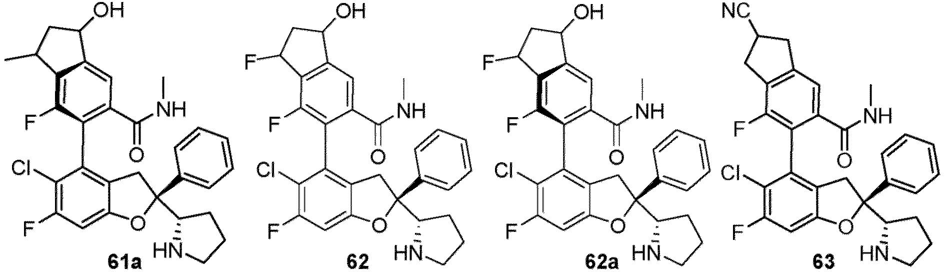

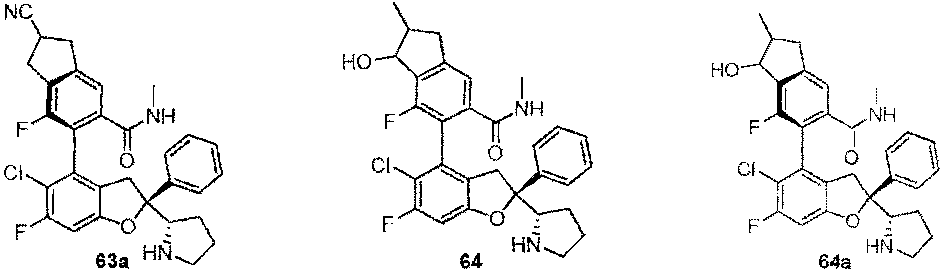

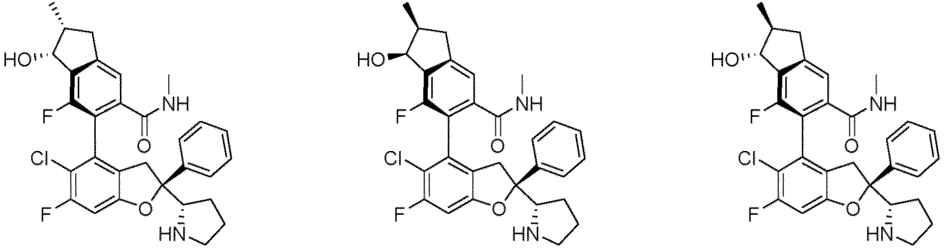

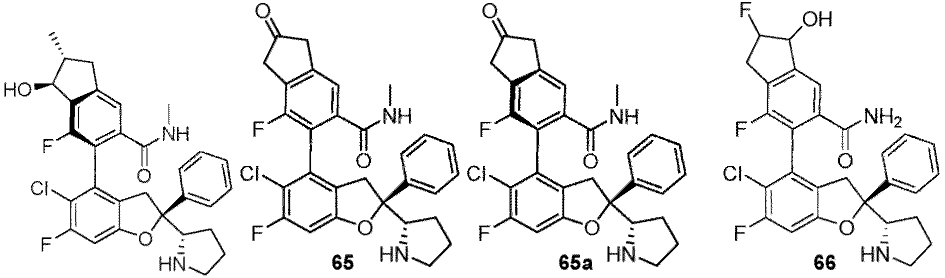

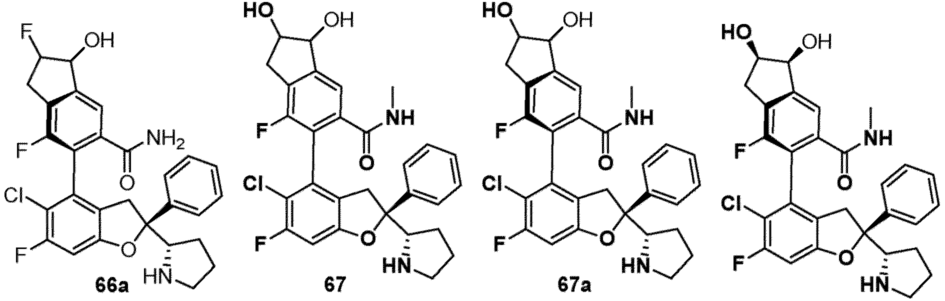

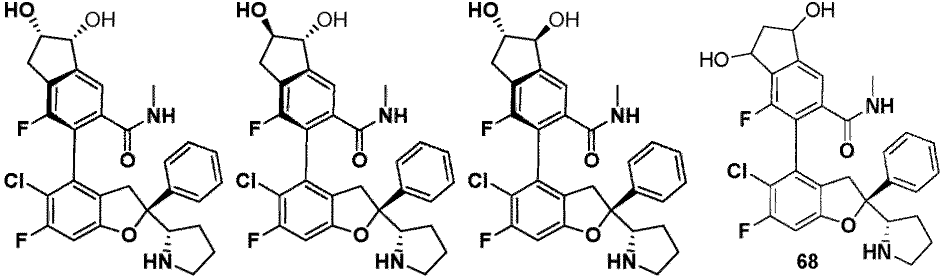

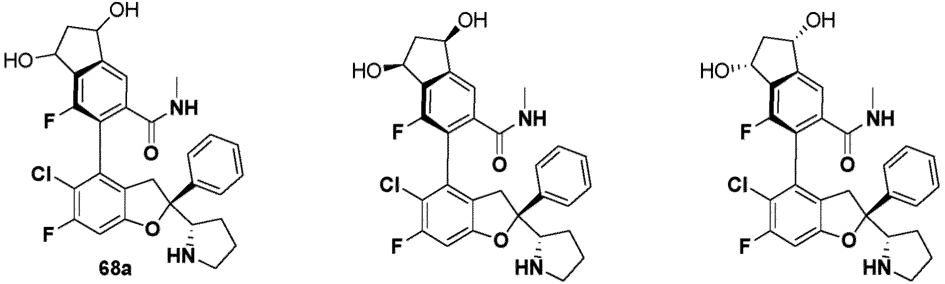

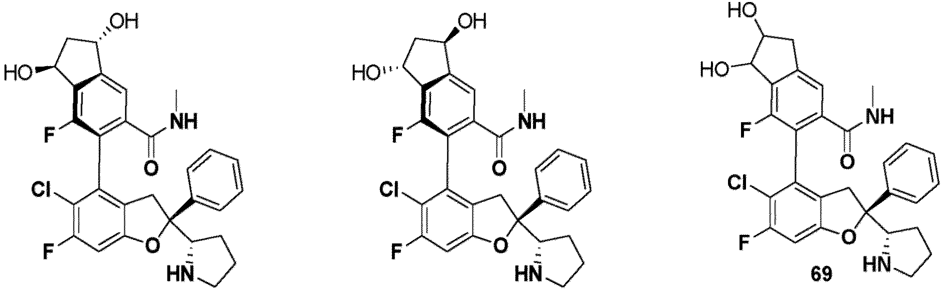

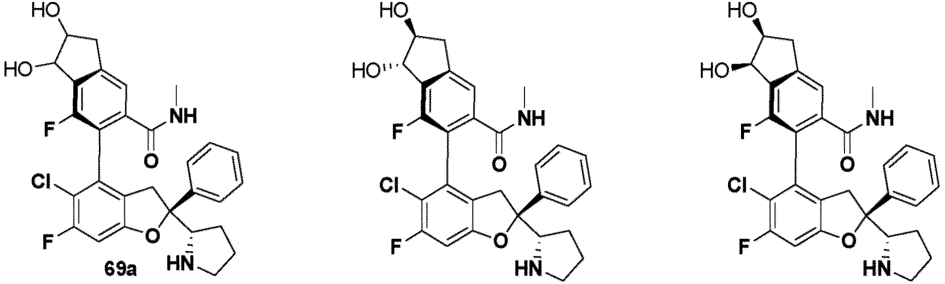

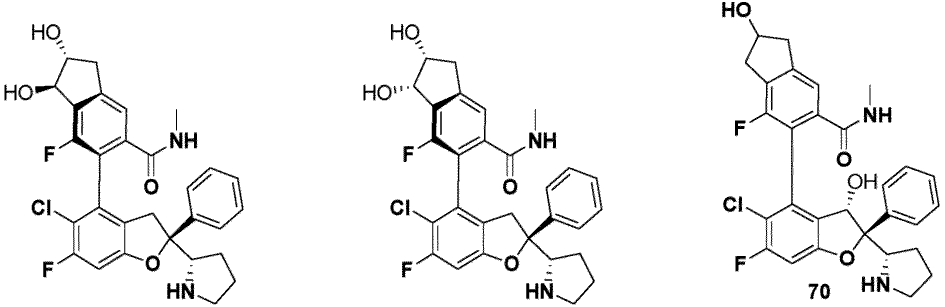

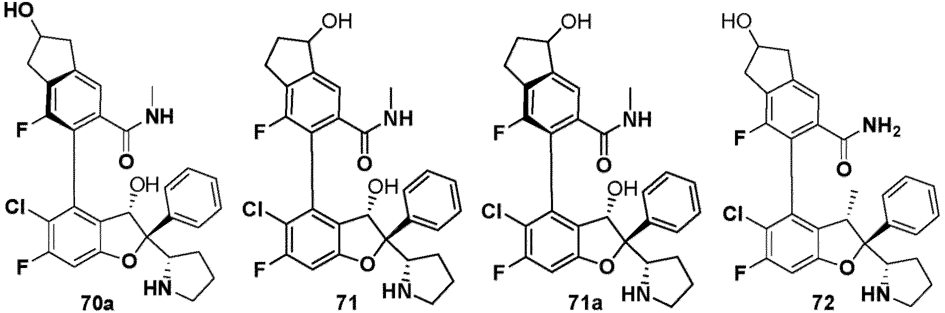

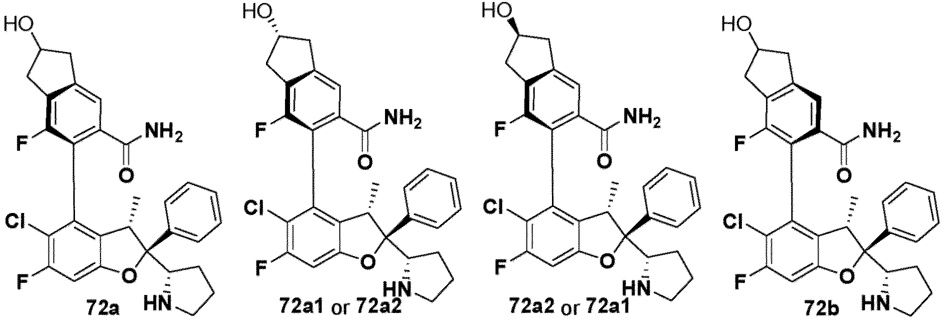

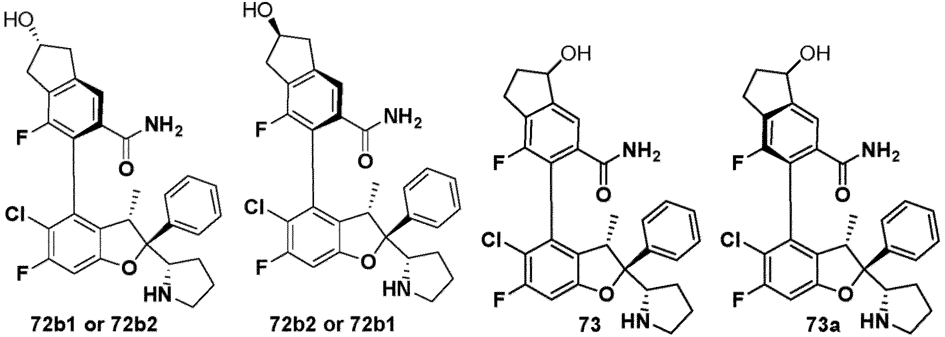

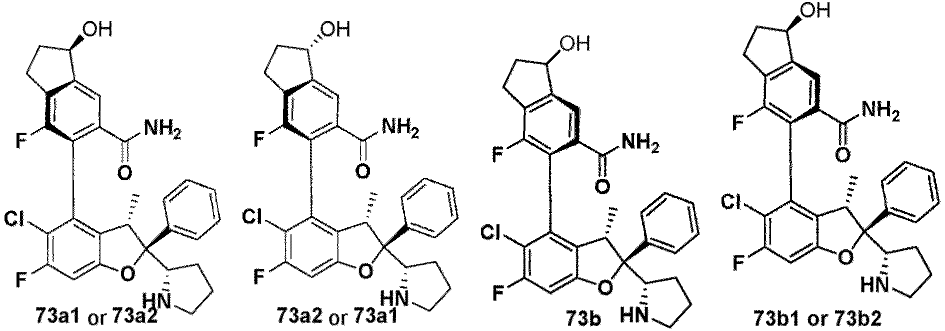

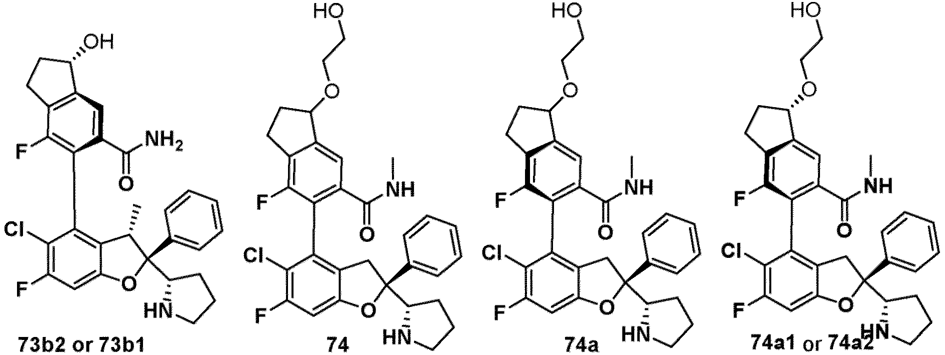

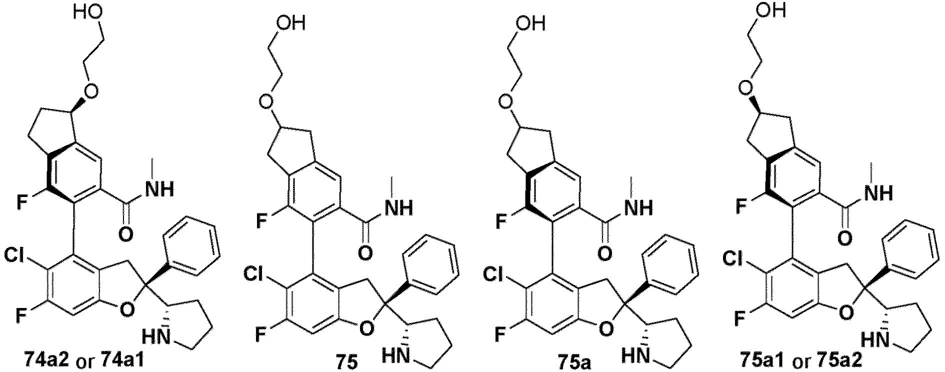

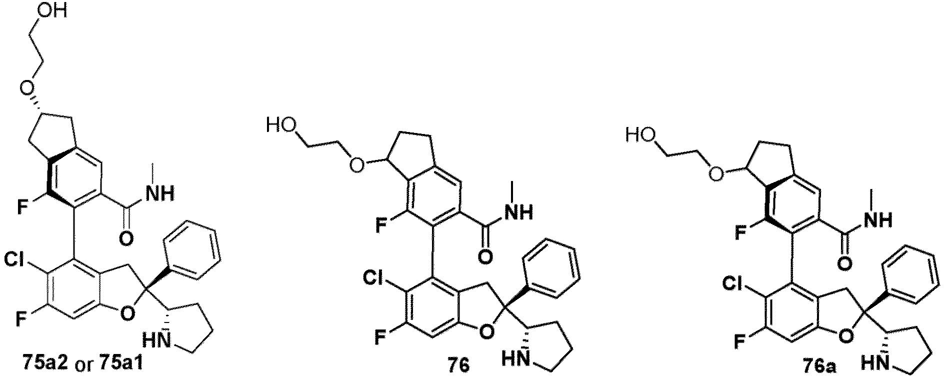

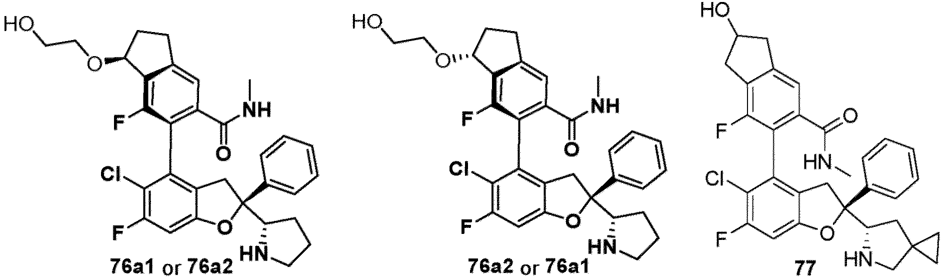

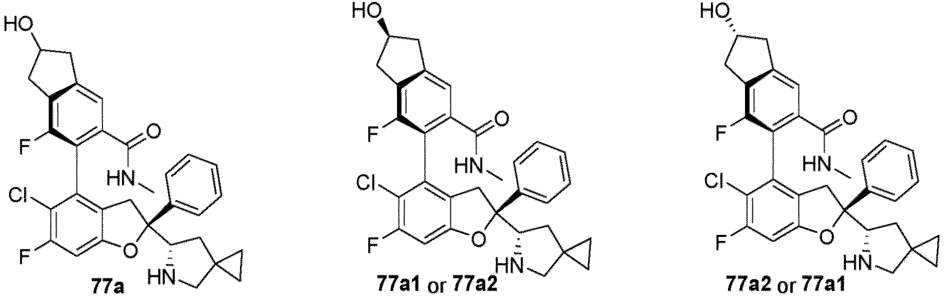

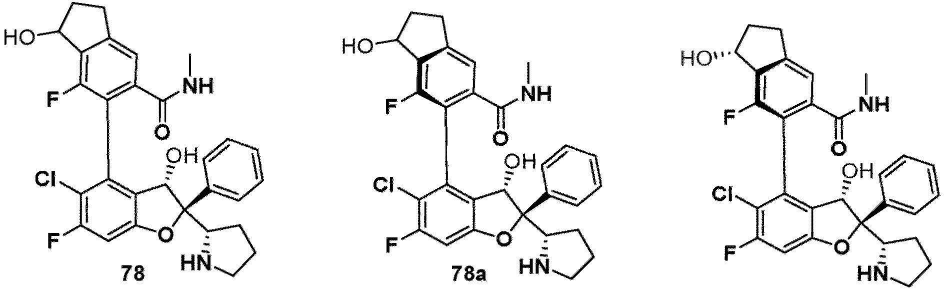

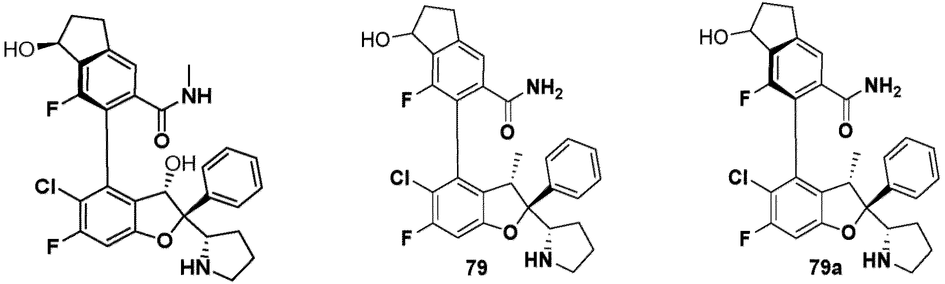

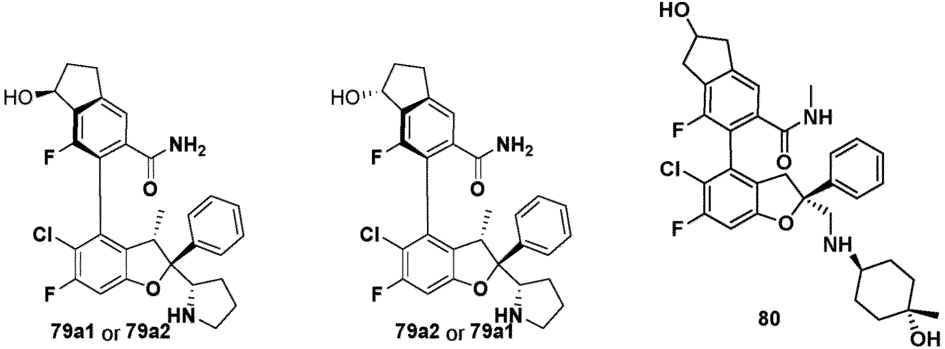

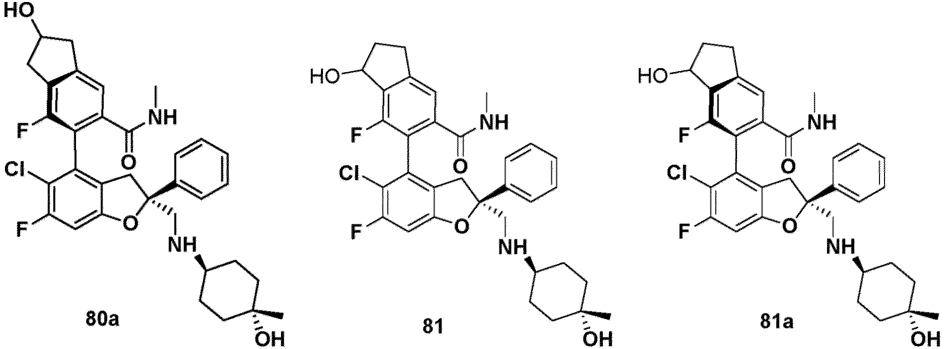

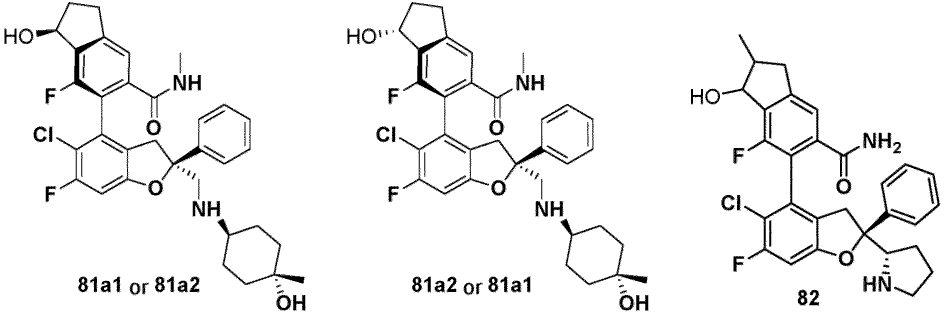

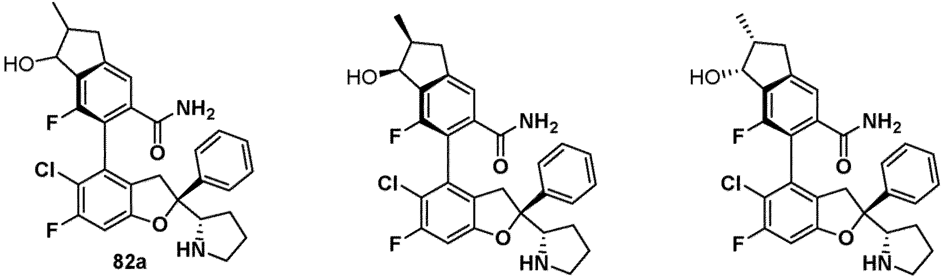

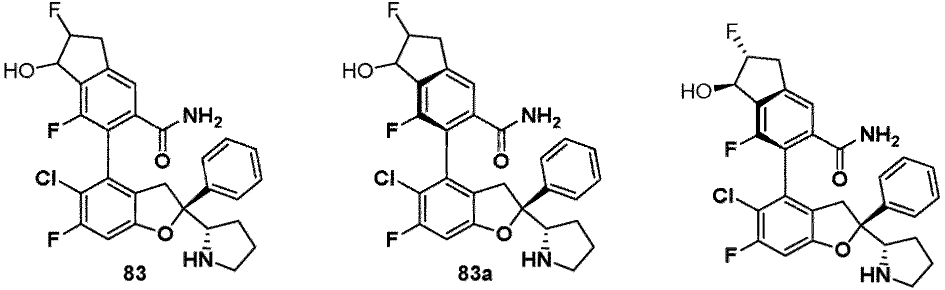

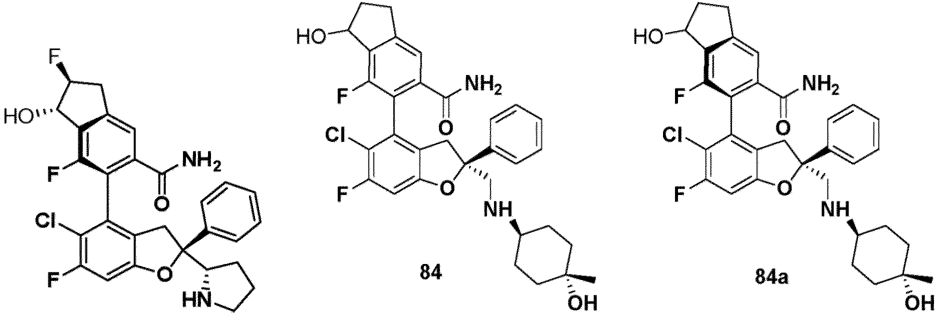

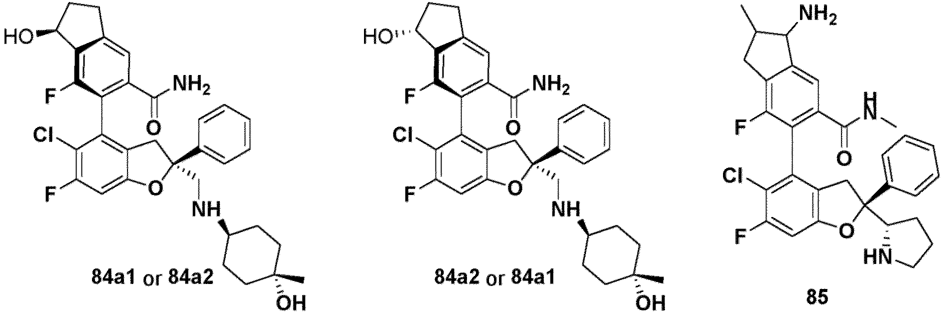

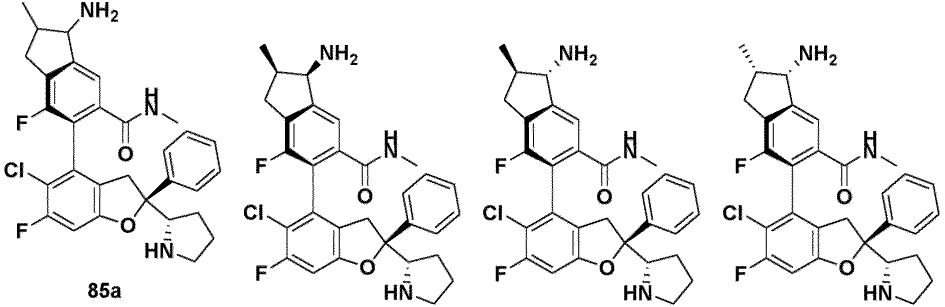

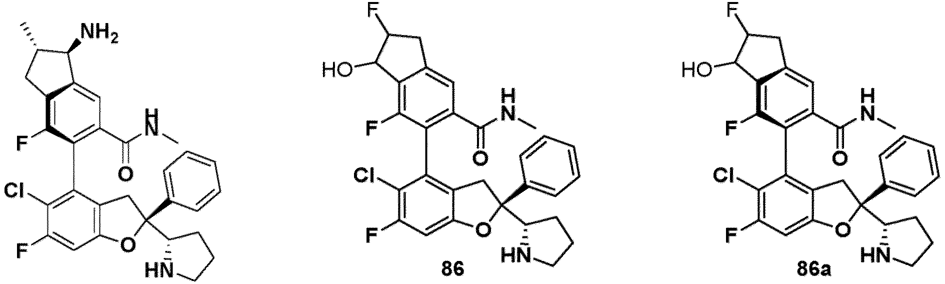

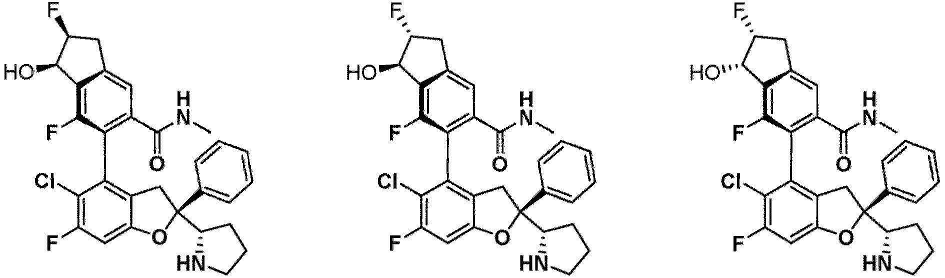

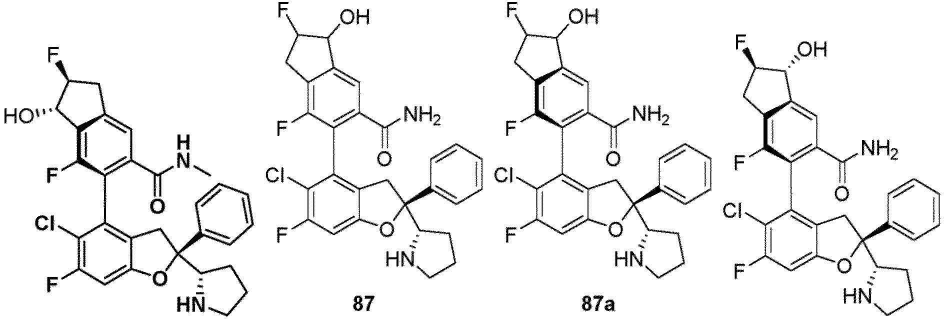

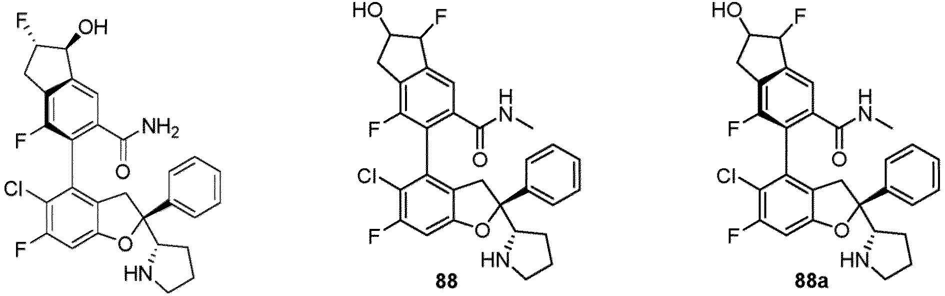

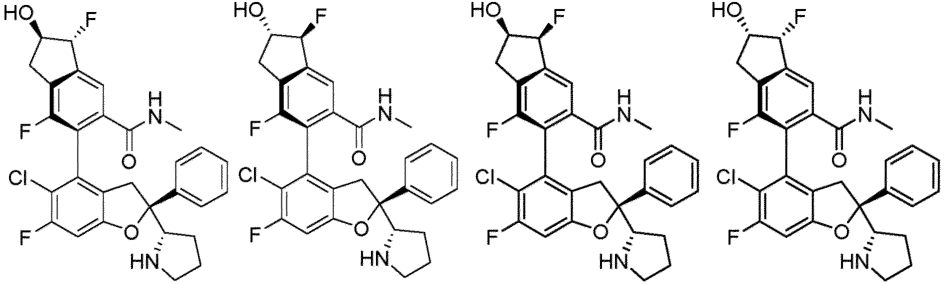

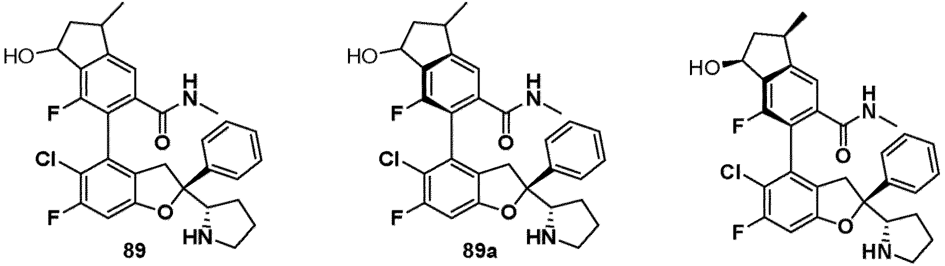

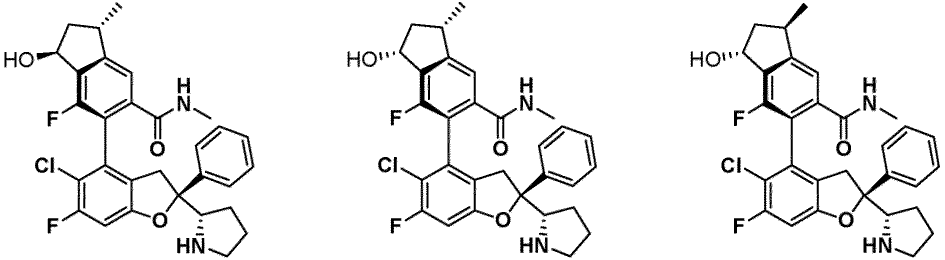

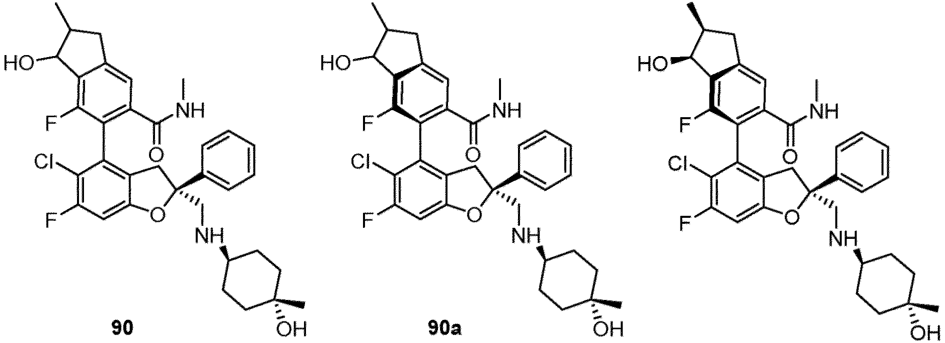

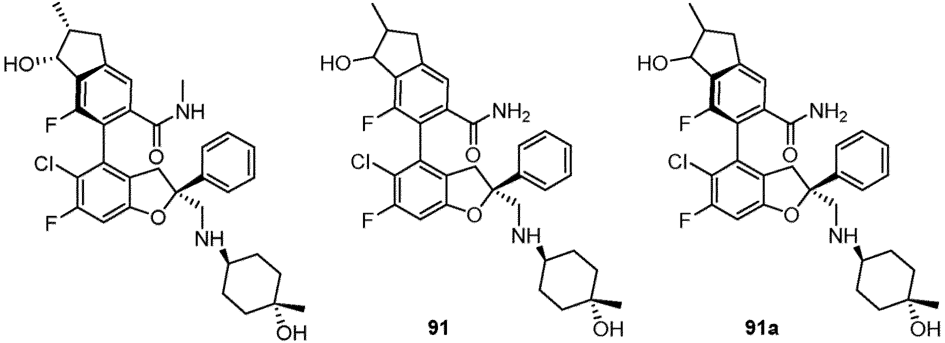

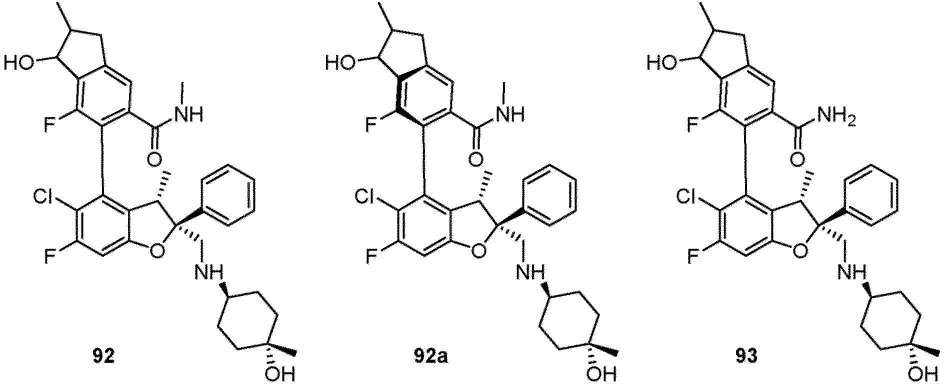

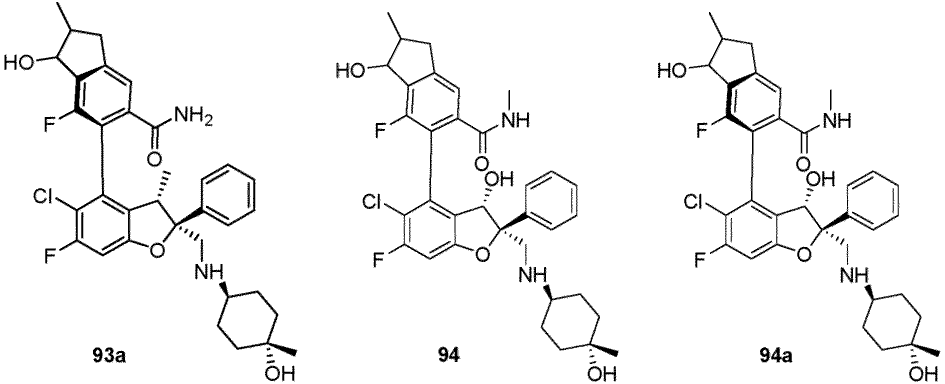

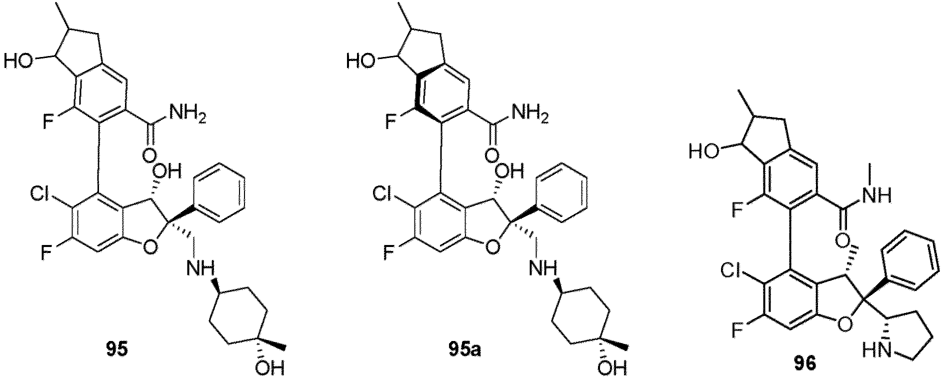

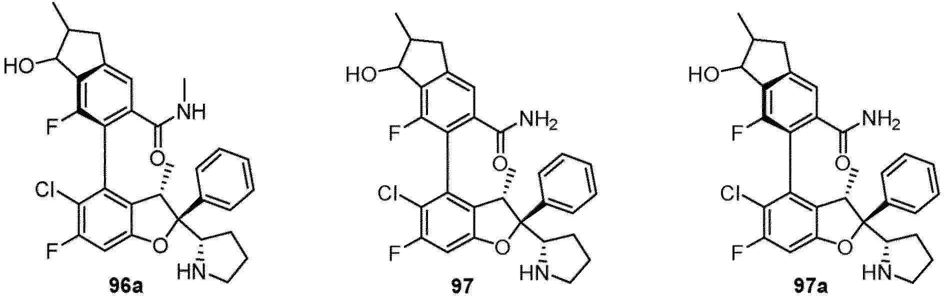

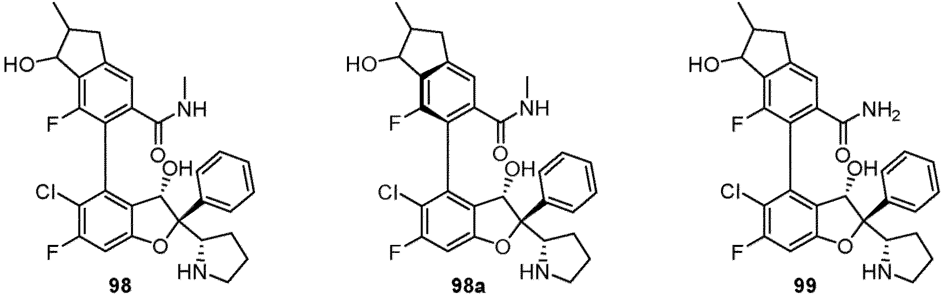

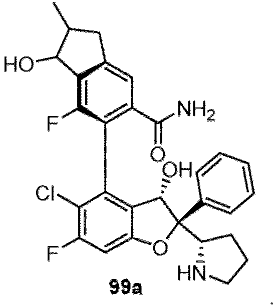

In a certain embodiment, the compound of formula (I) is any one of the following compounds:
a compound with a retention time of 1.89 min under the following conditions, which is a stereoisomer in 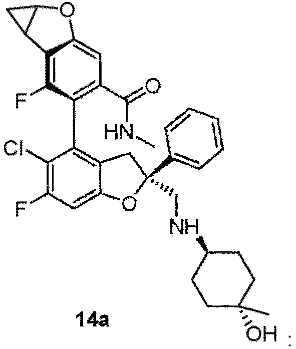 chiral column model: Cellulose-SB; eluent: carbon dioxide/(methanol + 20 mM NH₃); gradient: 10%; flow rate: 3.0 mL/min;
a compound with a retention time of 2.12 min under the following conditions, which is a stereoisomer in 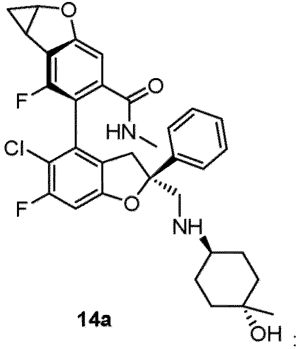 chiral column model: Cellulose-SB; eluent: carbon dioxide/(methanol + 20 mM NH₃); gradient: 10%; flow rate: 3.0 mL/min;
a compound with a retention time of 6.50 min under the following conditions, which is a stereoisomer in 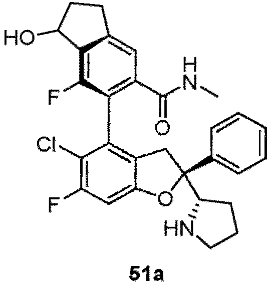 : column model: Celluiose-SB, 100 * 4.6 mm, 5 µm; eluent: hexane (0.1% DEA)/IPA; gradient: isocratic 9/1, flow rate: 1 mL/min;
a compound with a retention time of 7.62 min under the following conditions, which is a stereoisomer in 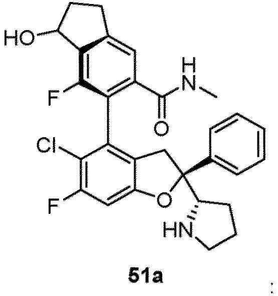 column model: Celluiose-SB, 100 * 4.6 mm, 5 µm; eluent: hexane (0.1% DEA)/IPA; gradient: isocratic 9/1, flow rate: 1 mL/min;
a compound with a retention time of 4.68 min under the following conditions, which is a stereoisomer in 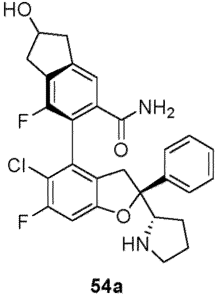 : column model: Celluiose-SB, 100 * 4.6 mm, 5 µm; eluent: hexane (0.1% DEA)/IPA; gradient: isocratic 4/1, flow rate: 1 mL/min;
a compound with a retention time of 4.20 min under the following conditions, which is a stereoisomer in 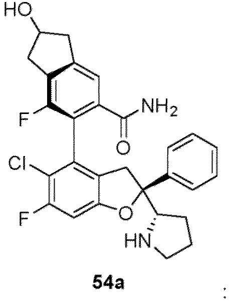 column model: Celluiose-SB, 100 * 4.6 mm, 5 µm; eluent: hexane (0.1% DEA)/IPA; gradient: isocratic 4/1, flow rate: 1 mL/min;
a mixture with retention times of 4.21 min and 4.71 min under the following conditions, which is a pair of diastereomers in 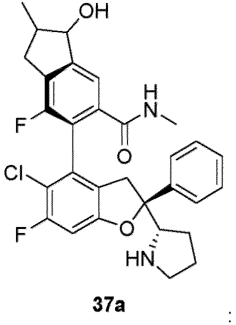 column model: Amylose-SA, 100 * 4.6 mm, 5 µm; eluent: hexane (0.1% DEA)-IPA; gradient: isocratic 9/1, flow rate: 1 mL/min, dr = 54 (4.21 min):46 (4.71 min);
a mixture with retention times of 4.60 min and 6.10 min under the following conditions, which is a pair of diastereomers in 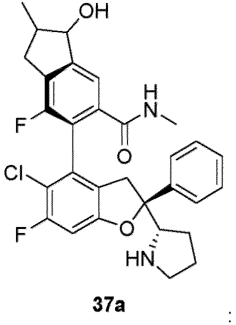 column model: Amylose-SA, 100 * 4.6 mm, 5 µm; eluent: hexane (0.1% DEA)-IPA; gradient: isocratic 9/1, flow rate: 1 mL/min, dr = 48 (4.60 min):52 (6.10 min);
a mixture with retention times of 620 min and 6.81 min under the following conditions, which is a pair of diastereomers in 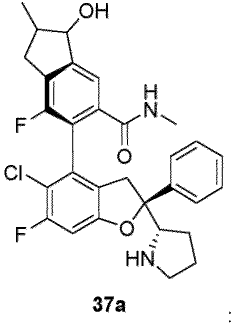 column model: Amylose-SA, 100 * 4.6 mm, 5 µm; eluent: hexane (0.1% DEA)-IPA; gradient: isocratic 9/1, flow rate: 1 mL/min, dr = 8 (6.20 min):92 (6.81 min);
a compound with a retention time of 2.43 min under the following conditions, which is a stereoisomer in 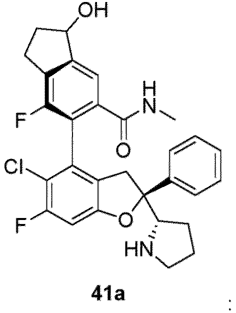 column model: Celluiose-SB, 100 * 4.6 mm, 5 µm; eluent: hexane/IPA; gradient: isocratic 7/3, flow rate: 1 mL/min;
a compound with a retention time of 3337 min under the following conditions, which is a stereoisomer in 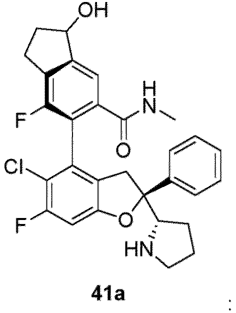 column model: Celluiose-SB, 100 * 4.6 mm 5 µm; eluent: hexane/IPA; gradient: isocratic 7/3, flow rate: 1 mL/min;
a mixture with retention times of 2.86 min and 3.75 min under the following conditions, which is a pair of diastereomers in 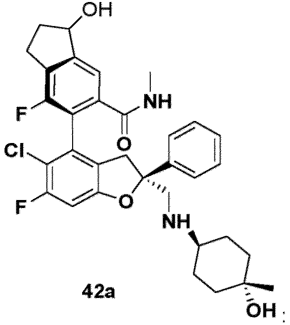 column model: Amylose-C 100 * 4.6 mm 3 µm; eluent: hexane (0.1% DEA)/IPA; gradient: isocratic 8/2, flow rate: 1 mL/min, dr = 52 (2.86 min):48 (3.75 min);
a compound with a retention time of 8.87 min under the following conditions, which is a stereoisomer in 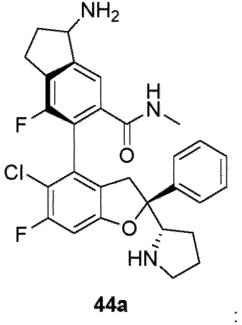 column model: Celluiose-SC, 100 * 4.6 mm, 5 µm; eluent: hexane (0.1% DEA)/IPA; gradient: isocratic 7/3, flow rate: 1 mL/min;
a compound with a retention time of 9.60 min under the following conditions, which is a stereoisomer in 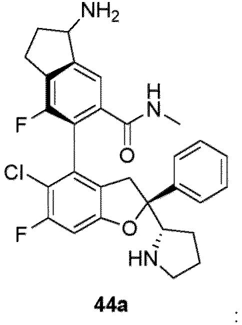 column model: Celluiose-SB, 100 * 4.6 mm, 5 µm; eluent: hexane/IPA; gradient: isocratic 7/3, flow rate: 1 mL/min;
a compound with a retention time of 4.16 min under the following conditions, which is a stereoisomer in 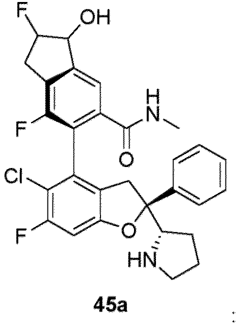 column model: Celluiose-C, 100 * 4.6 mm, 5 µm; eluent: hexane (0.1% DEA)-IPA; gradient: isocratic 4/1, flow rate: 1 mL/min;
a compound with a retention time of 2.68 min under the following conditions, which is a stereoisomer in 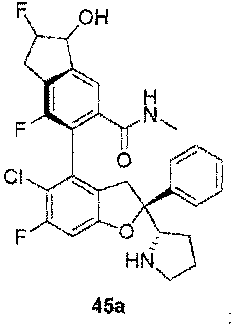 column model: Celluiose-C, 100 * 4.6 mm, 5 µm; eluent: hexane (0.1% DEA)-IPA; gradient: isocratic 4/1, flow rate: 1 mL/min;
a compound with a retention time of 4.92 min under the following conditions, which is a stereoisomer in 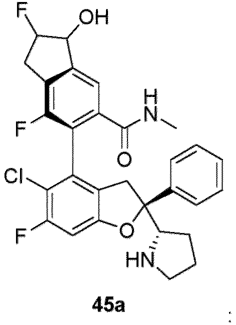 column model: Celluiose-C, 100 * 4.6 mm, 5 µm; eluent: hexane (0.1% DEA)-IPA; gradient: isocratic 4/1, flow rate: 1 mL/min;
a compound with a retention time of 4.87 min under the following conditions, which is a stereoisomer in 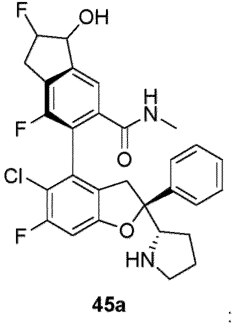 column model: Celluiose-C, 100 * 4.6 mm, 5 µm; eluent: hexane (0.1% DEA)-IPA; gradient: isocratic 4/1, flow rate: 1 mL/min;
a compound with a retention time of 2.197 min under the following conditions, which is a stereoisomer in 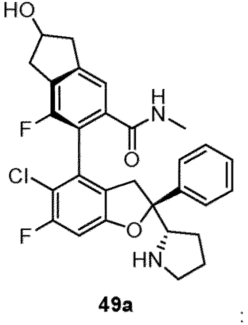 column model: Amylose-C, 100 * 4.6 mm, 5 µm; eluent: hexane-IPA; gradient: isocratic 7/3, flow rate: 1 mL/min;
a compound with a retention time of 2.646 min under the following conditions, which is a stereoisomer in 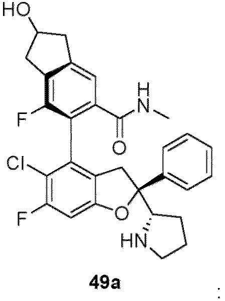 column model: Amylose-C, 100 * 4.6 mm, 5 µm; eluent: hexane-IPA; gradient: isocratic 7/3, flow rate: 1 mL/min;
a compound with a retention time of 3.14 min under the following conditions, which is a stereoisomer in 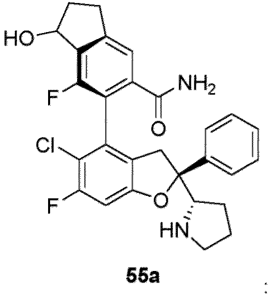 column model: Amylose-SA, 100 * 4.6 mm, 5 µm; eluent: hexane (0.1% DEA)-IPA; gradient: isocratic 4/1, flow rate: 1 mL/min;
a compound with a retention time of 3.04 min under the following conditions, which is a stereoisomer in 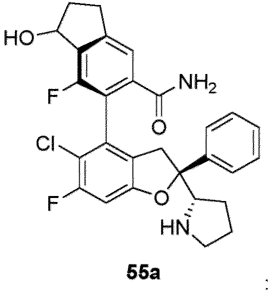 column model: Amylose-SA, 100 * 4.6 mm, 5 µm; eluent: hexane (0.1% DEA)-IPA; gradient: isocratic 4/1, flow rate: 1 mL/min;
a compound with a retention time of 3.89 min under the following conditions, which is a stereoisomer in 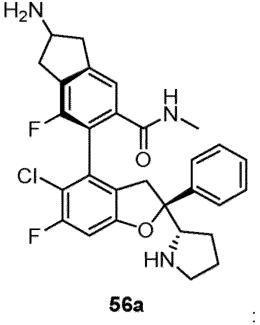 column model: Celluiose-SB, 100 * 4.6 mm, 5 µm; eluent: hexane (0.1% DEA)-IPA; gradient: isocratic 6/4, flow rate: 1 mL/min;
a compound with a retention time of 3.53 min under the following conditions, which is a stereoisomer in 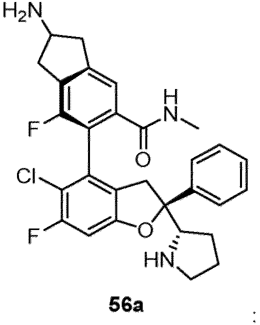 column model: Celluiose-SB, 100 * 4.6 mm, 5 µm; eluent: hexane (0.1% DEA)-IPA; gradient: isocratic 6/4, flow rate: 1 mL/min;
a mixture with retention times of 1.95 min and 2.88 min under the following conditions, which is a pair of diastereomers in 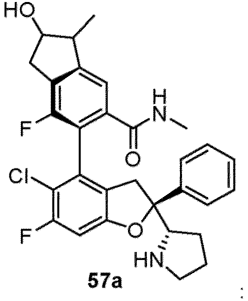 column model: Amylose-C 100 * 4.6 mm, 5 µm; eluent: hexane (0.1% DEA)/IPA; gradient: isocratic 8/2, flow rate: 1 mL/min, dr = 65 (1.95 min):35 (2.88 min);
a compound with a retention time of 5.75 min under the following conditions, which is a stereoisomer in 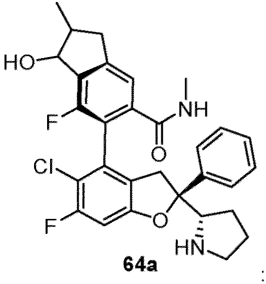 column model: Celluiose-SB, 100 * 4.6 mm, 5 µm; eluent: hexane (0.1% DEA)-IPA; gradient: isocratic 9/1, flow rate: 1 mL/min;
a compound with a retention time of 4.76 min under the following conditions, which is a stereoisomer in 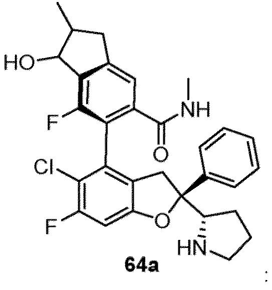 column model: Celluiose-SB, 100 * 4.6 mm, 5 µm; eluent: hexane (0.1% DEA)-IPA; gradient: isocratic 9/1, flow rate: 1 mL/min;
a compound with a retention time of 3.06 min under the following conditions, which is a stereoisomer in 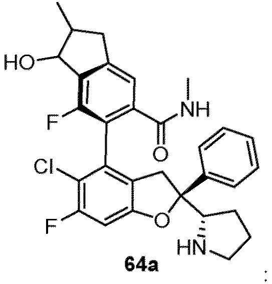 column model: Celluiose-SB, 100 * 4.6 mm, 5 µm; eluent: hexane (0.1% DEA)-IPA; gradient: isocratic 4/1, flow rate: 1 mL/min;
a compound with a retention time of 3.32 min under the following conditions, which is a stereoisomer in 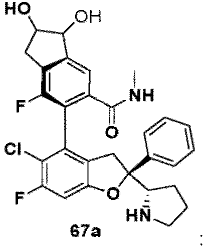 column model: Celluiose-SB, 100 * 4.6 mm, 3 µm; eluent: hexane (0.1% DEA)/EtOH; gradient: isocratic 8/2, flow rate: 1 mL/min;
a compound with a retention time of 3.12 min under the following conditions, which is a stereoisomer in 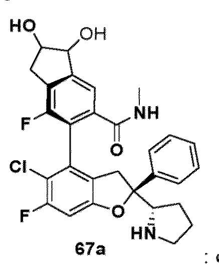 column model: Celluiose-SB, 100 * 4.6 mm, 3 µm; eluent: hexane (0.1% DEA)/EtOH; gradient: isocratic 8/2, flow rate: 1 mL/min;
a compound with a retention time of 2.67 min under the following conditions, which is a stereoisomer in 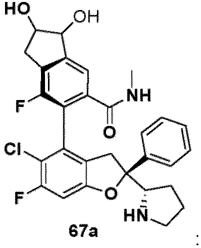 column model: Celluiose-SB, 100 * 4.6 mm, 3 µm; eluent: hexane (0.1% DEA)/EtOH; gradient: isocratic 8/2, flow rate: 1 mL/min;
a compound with a retention time of 4.10 min under the following conditions, which is a stereoisomer in 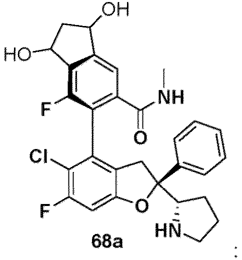 column model: Amylose-C, 100 * 4.6 mm, 3 µm; eluent: hexane/IPA; gradient: isocratic 85/15; flow rate: 1 mL/min;
a compound with a retention time of 4.47 min under the following conditions, which is a stereoisomer in 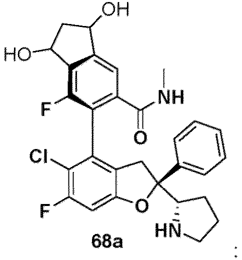 column model: Amylose-C, 100 * 4.6 mm, 3 µm; eluent: hexane/IPA; gradient: isocratic 85/15; flow rate: 1 mL/min;
a compound with a retention time of 5.59 min under the following conditions, which is a stereoisomer in 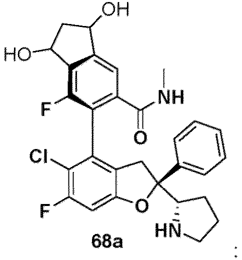 column model: Amylose-C, 100 * 4.6 mm, 3 µm; eluent: hexane/IPA; gradient: isocratic 85/15; flow rate: 1 mL/min;
a compound with a retention time of 4.29 min under the following conditions, which is a stereoisomer in 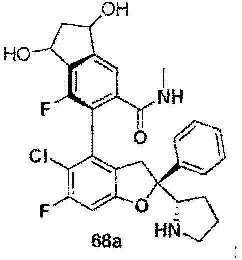 column model: Amylose-C, 100 * 4.6 mm, 3 µm; eluent: hexane/IPA; gradient: isocratic 85/15; flow rate: 1 mL/min;
a compound with a retention time of 6.65 min under the following conditions, which is a stereoisomer in 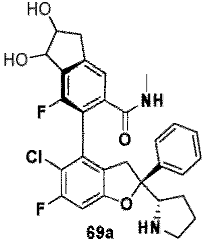 : column model: Celluiose-SC, 100 * 4.6 mm, 3 µm; eluent: hexane (0.1% DEA)-IPA; gradient: isocratic 4/1, flow rate: 1 mL/min;
a compound with a retention time of 11.46 min under the following conditions, which is a stereoisomer in 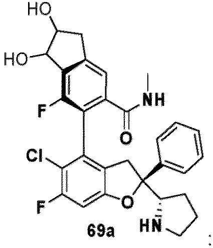 column model: Celluiose-SC, 100 * 4.6 mm, 3 µm; eluent: hexane (0.1% DEA)-IPA; gradient: isocratic 4/1, flow rate: 1 mL/min;
a compound with a retention time of 5.68 min under the following conditions, which is a stereoisomer in 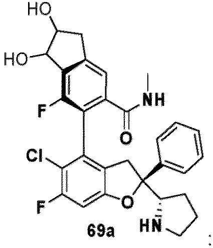 column model: Celluiose-SC, 100 * 4.6 mm, 3 µm; eluent: hexane (0.1% DEA)-IPA; gradient: isocratic 4/1, flow rate: 1 mL/min;
a compound with a retention time of 6.71 min under the following conditions, which is a stereoisomer in 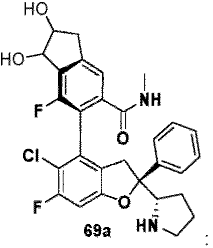 column model: Celluiose-SC, 100 * 4.6 mm, 3 µm; eluent: hexane (0.1% DEA)-IPA; gradient: isocratic 4/1, flow rate: 1 mL/min;
a compound with a retention time of 6.21 min under the following conditions, which is a stereoisomer in 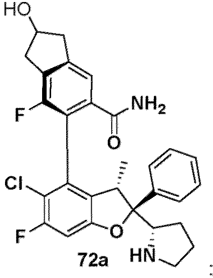 column model: Celluiose-SB, 100 * 4.6 mm, 3 µm; eluent: hexane/IPA; gradient: isocratic 8/2, flow rate: 1 mL/min;
a compound with a retention time of 4.71 min under the following conditions, which is a stereoisomer in 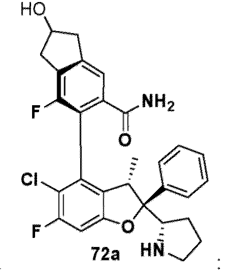 column model: Celluiose-SB, 100 * 4.6 mm, 3 µm; eluent: hexane/IPA; gradient: isocratic 8/2, flow rate: 1 mL/min;
a compound with a retention time of 8.1 min under the following conditions, which is a stereoisomer in 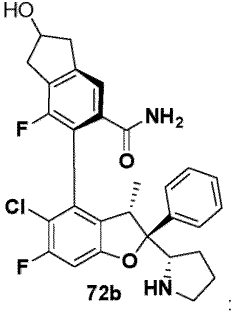 Xselect CSH Prep Fluoro-Phenyl, 150 * 19 mm, 5 µm; eluent: (0.1% FA) H₂O-ACN; gradient: 66% to 86%; flow rate: 20 mL/min;
a compound with a retention time of 6.5 min under the following conditions, which is a stereoisomer in 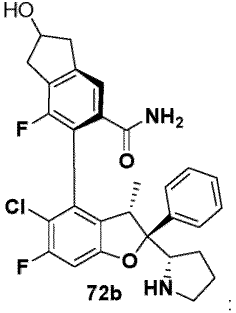 Xselect CSH Prep Fluoro-Phenyl, 150 * 19 mm, 5 µm; eluent: (0.1% FA) H₂O-ACN; gradient: 66% to 86%; flow rate: 20 mL/min;
a compound with a retention time of 3.90 min under the following conditions, which is a stereoisomer in 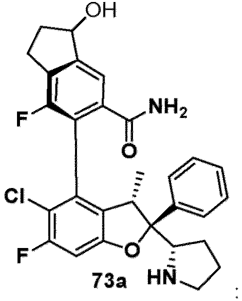 column model: Amylose-SA, 100 * 4.6 mm, 3 µm; eluent: hexane (0.1% DEA)/EtOH; gradient: isocratic 8/2, flow rate: 1 mL/min;
a compound with a retention time of 3.02 min under the following conditions, which is a stereoisomer in 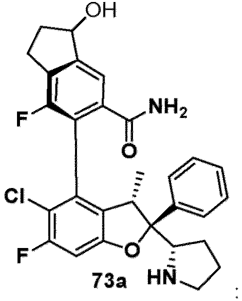 column model: Amylose-SA, 100 * 4.6 mm, 3 µm; eluent: hexane (0.1% DEA)/EtOH; gradient: isocratic 8/2, flow rate: 1 mL/min;
a compound with a retention time of 3.35 min under the following conditions, which is a stereoisomer in 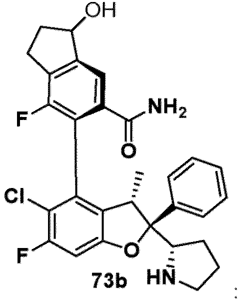 column model: Amylose-SA, 100 * 4.6 mm, 3 µm; eluent: hexane (0.1% DEA)/EtOH; gradient: isocratic 8/2, flow rate: 1 mL/min;
a compound with a retention time of 4.57 min under the following conditions, which is a stereoisomer in 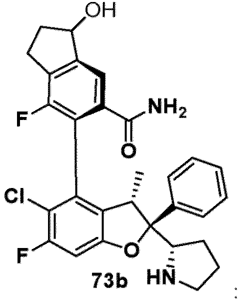 column model: Amylose-SA, 100 * 4.6 mm, 3 µm; eluent: hexane (0.1% DEA)/EtOH; gradient: isocratic 8/2, flow rate: 1 mL/min;
a compound with a retention time of 4.97 min under the following conditions, which is a stereoisomer in 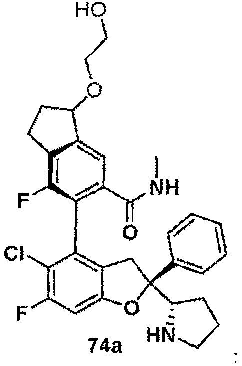 column model: Amylose-SA, 100 * 4.6 mm, 3 µm; eluent: hexane (0.1% DEA)/EtOH; gradient: isocratic 9/1, flow rate: 1 mL/min;
a compound with a retention time of 5.22 min under the following conditions, which is a stereoisomer in 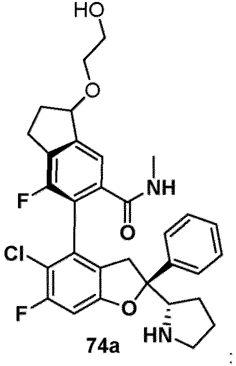 column model: Amylose-SA, 100 * 4.6 mm, 3 µm; eluent: hexane (0.1% DEA)/EtOH; gradient: isocratic 9/1, flow rate: 1 mL/min;
a compound with a retention time of 7.21 min under the following conditions, which is a stereoisomer in 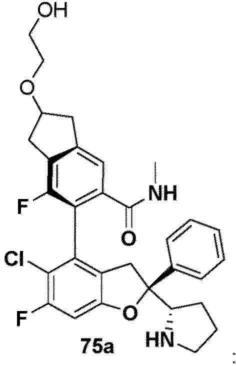 column model: Celluiose-SB, 100 * 4.6 mm, 5 µm; eluent: hexane (0.1% DEA)-IPA; gradient: isocratic 85/15, flow rate: 1 mL/min;
a compound with a retention time of 8.17 min under the following conditions, which is a stereoisomer in 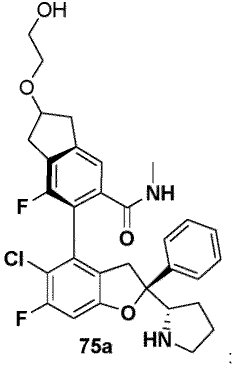 column model: Celluiose-SB, 100 * 4.6 mm, 5 µm; eluent: hexane (0.1% DEA)-IPA; gradient: isocratic 85/15, flow rate: 1 mL/min;
a compound with a retention time of 9.45 min under the following conditions, which is a stereoisomer in 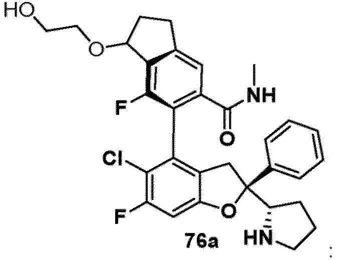 column model: Celluiose-SC, 100 * 4.6 mm, 3 µm; eluent: hexane (0.1% DEA)-IPA; gradient: isocratic 4/1, flow rate: 1 mL/min;
a compound with a retention time of 6.63 min under the following conditions, which is a stereoisomer in 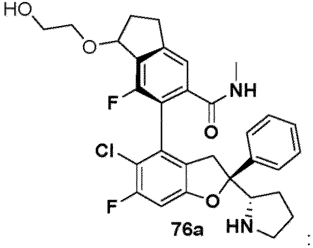 column model: Celluiose-SC, 100 * 4.6 mm, 3 µm; eluent: hexane (0.1% DEA)-IPA; gradient: isocratic 4/1, flow rate: 1 mL/min;
a compound with a retention time of 3.21 min under the following conditions, which is a stereoisomer in 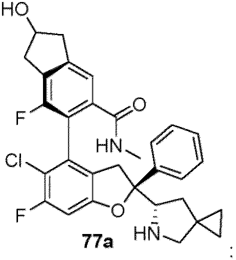 column model: Amylose-SA, 100 * 4.6 mm, 3 µm; eluent: hexane (0.1% DEA)-IPA; gradient: isocratic 85/15, flow rate: 1 mL/min;
a compound with a retention time of 7.29 min under the following conditions, which is a stereoisomer in 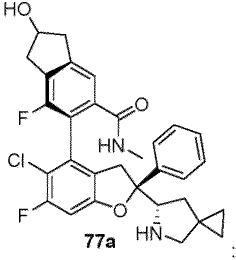 column model: Amylose-SA, 100 * 4.6 mm, 3 µm; eluent: hexane (0.1% DEA)-IPA; gradient: isocratic 85/15, flow rate: 1 mL/min;
a compound with a retention time of 5.46 min under the following conditions, which is a stereoisomer in 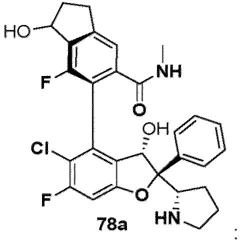 Celluiose-C, 100 * 4.6 mm, 3 µm; eluent: hexane (0.1% DEA)/IPA; gradient: isocratic 9/1, flow rate: 1 mL/min;
a compound with a retention time of 6.42 min under the following conditions, which is a stereoisomer in 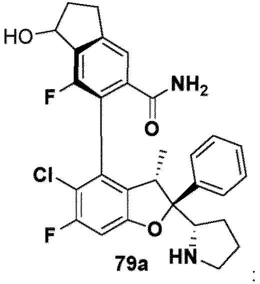 column model: Celluiose-SB, 100 * 4.6 mm, 3 µm; eluent: hexane (0.1% DEA)/IPA; gradient: isocratic 85/15, flow rate: 1 mL/min;
a compound with a retention time of 4.47 min under the following conditions, which is a stereoisomer in 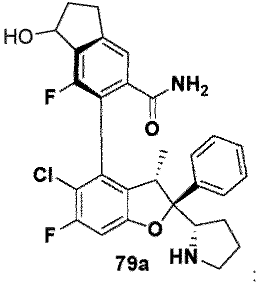 column model: Celluiose-SB, 100 * 4.6 mm, 3 µm; eluent: hexane (0.1% DEA)/IPA; gradient: isocratic 85/15, flow rate: 1 mL/min;
a mixture with retention times of 4.97 min and 6.09 min under the following conditions, which is a pair of diastereomers in 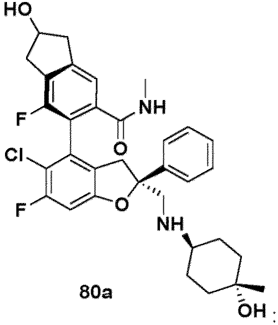 column model: Celluiose-SC 100 * 4.6 mm 3 µm; eluent: hexane/IPA; gradient: isocratic 8/2, flow rate: 1 mL/min, dr = 49 (4.97 min):51 (6.09 min);
a compound with a retention time of 3.88 min under the following conditions, which is a stereoisomer in 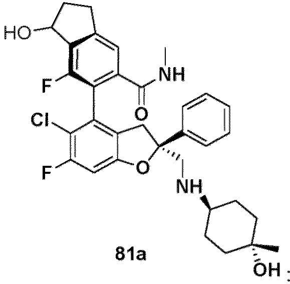 column model: Celluiose-C, 100 * 4.6 mm, 3 µm; eluent: hexane (0.1% DEA)/IPA; gradient: isocratic 4/1, flow rate: 1 mL/min;
a compound with a retention time of 3.52 min under the following conditions, which is a stereoisomer in 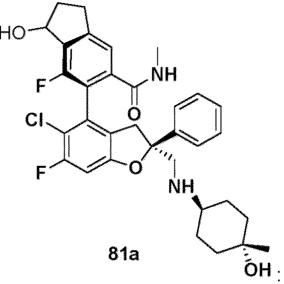 column model: Celluiose-C, 100 * 4.6 mm, 3 µm; eluent: hexane (0.1% DEA)/IPA; gradient: isocratic 4/1, flow rate: 1 mL/min;
a compound with a retention time of 4.91 min under the following conditions, which is a stereoisomer in 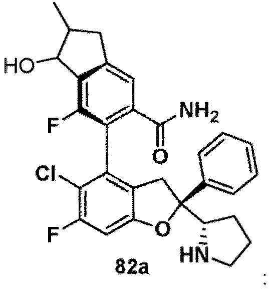 column model: Amylose-SA, 100 * 4.6 mm, 3 µm; eluent: hexane (0.1% DEA)-IPA; gradient: isocratic 9/1, flow rate: 1 mL/min;
a compound with a retention time of 5.05 min under the following conditions, which is a stereoisomer in 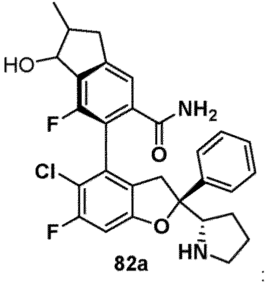 column model: Amylose-SA, 100 * 4.6 mm, 3 µm; eluent: hexane (0.1% DEA)-IPA; gradient: isocratic 9/1, flow rate: 1 mL/min;
a compound with a retention time of 7.00 min under the following conditions, which is a stereoisomer in 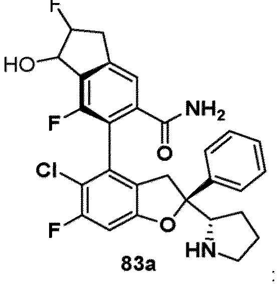 column model: Celluiose-SC, 100 * 4.6 mm, 5 µm; eluent: hexane (0.1% DEA)-IPA; gradient: isocratic 4/1, flow rate: 1 mL/min;
a compound with a retention time of 3.56 min under the following conditions, which is a stereoisomer in 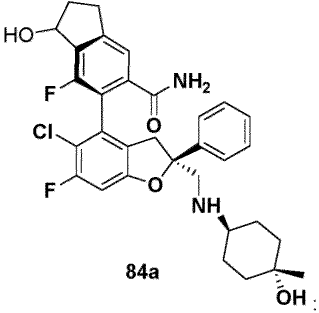 column model: Amylose-SA, 100 * 4.6 mm, 5 µm; eluent: hexane (0.1% DEA)-IPA; gradient: isocratic 4/1, flow rate: 1 mL/min;
a compound with a retention time of 3.40 min under the following conditions, which is a stereoisomer in 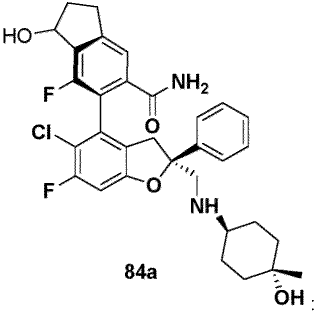 column model: Amylose-SA, 100 * 4.6 mm, 5 µm; eluent: hexane (0.1% DEA)-IPA; gradient: isocratic 4/1, flow rate: 1 mL/min;
a compound with a retention time of 3.45 min under the following conditions, which is a stereoisomer in 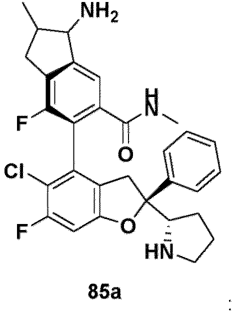 column model: Amylose-SA, 100 * 4.6 mm, 5 µm; eluent: hexane (0.1% DEA)-IPA; gradient: isocratic 4/1, flow rate: 1 mL/min;
a compound with a retention time of 3.53 min under the following conditions, which is a stereoisomer in 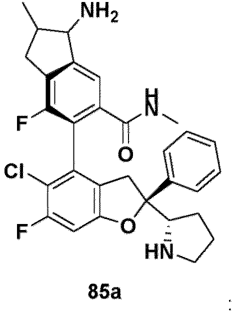 column model: Amylose-SA, 100 * 4.6 mm, 5 µm; eluent: hexane (0.1% DEA)-IPA; gradient: isocratic 4/1, flow rate: 1 mL/min;
a compound with a retention time of 7.48 min under the following conditions, which is a stereoisomer in 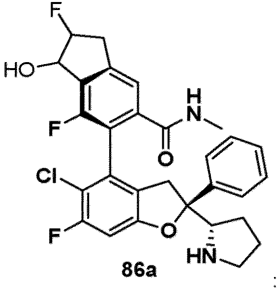 column model: Celluiose-SC, 100 * 4.6 mm, 5 µm; eluent: hexane (0.1% DEA)-IPA; gradient: isocratic 4/1, flow rate: 1 mL/min;
a compound with a retention time of 7.48 min under the following conditions, which is a stereoisomer in 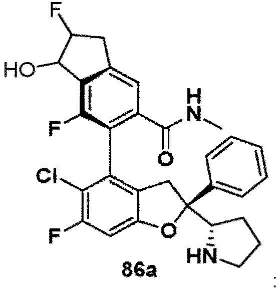 column model: Celluiose-SC, 100 * 4.6 mm, 5 µm; eluent: hexane (0.1% DEA)-IPA; gradient: isocratic 4/1, flow rate: 1 mL/min;
a compound with a retention time of 5.91 min under the following conditions, which is a stereoisomer in 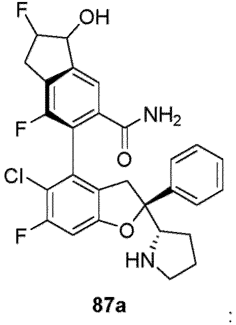 column model: Amylose-C, 100 * 4.6 mm, 5 µm; eluent: hexane (0.1% DEA)-IPA; gradient: isocratic 4/1, flow rate: 1 mL/min;
a compound with a retention time of 4.72 min under the following conditions, which is a stereoisomer in 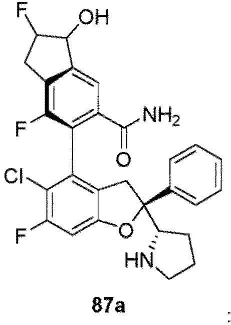 column model: Amylose-C, 100 * 4.6 mm, 5 µm; eluent: hexane (0.1% DEA)-IPA; gradient: isocratic 4/1, flow rate: 1 mL/min;
a compound with a retention time of 2.09 min under the following conditions, which is a stereoisomer in 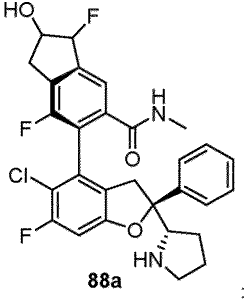 column model: Amylose-C, 100 * 4.6 mm, 3 µm; eluent: hexane (0.1% DEA)/IPA; gradient: isocratic 8/2, flow rate: 1 mL/min;
a compound with a retention time of 4.03 min under the following conditions, which is a stereoisomer in 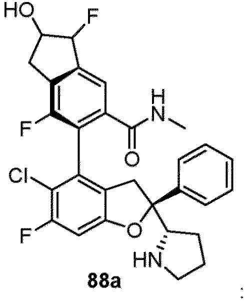 column model: Amylose-C, 100 * 4.6 mm, 3 µm; eluent: hexane (0.1% DEA)/IPA; gradient: isocratic 8/2, flow rate: 1 mL/min;
a compound with a retention time of 2.92 min under the following conditions, which is a stereoisomer in 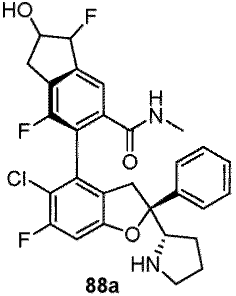 : column model: Amylose-C, 100 * 4.6 mm, 3 µm; eluent: hexane (0.1% DEA)/IPA; gradient: isocratic 8/2, flow rate: 1 mL/min;
a compound with a retention time of 4.62 min under the following conditions, which is a stereoisomer in 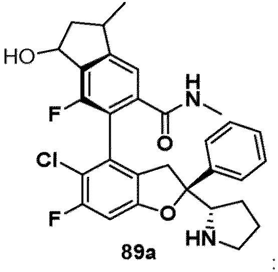 column model: Amylose-SA, 100 * 4.6 mm, 5 µm; eluent: hexane (0.1% DEA)-IPA; gradient: isocratic 9/1, flow rate: 1 mL/min;
a compound with a retention time of 5.32 min under the following conditions, which is a stereoisomer in 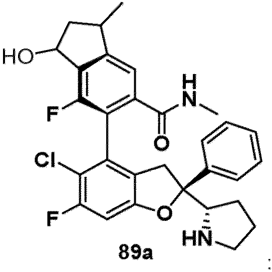 column model: Amylose-SA, 100 * 4.6 mm, 3 µm; eluent: hexane (0.1% DEA)-IPA; gradient: isocratic 9/1, flow rate: 1 mL/min;
a compound with a retention time of 3.26 min under the following conditions, which is a stereoisomer in 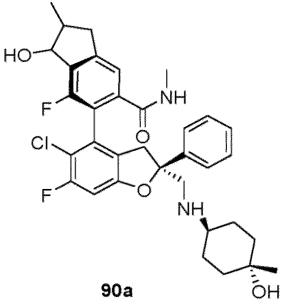 : column model: Celluiose-C, 100 * 4.6 mm, 3 µm; eluent: hexane (0.1% DEA)-IPA; gradient: isocratic 4/1, flow rate: 1 mL/min;
a compound with a retention time of 2.90 min under the following conditions, which is a stereoisomer in 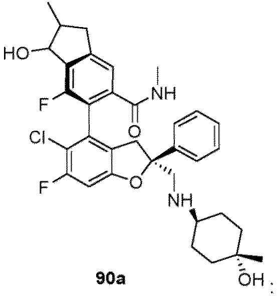 column model: Celluiose-C, 100 * 4.6 mm, 3 µm; eluent: hexane (0.1% DEA)-IPA; gradient: isocratic 4/1, flow rate: 1 mL/min.

The above retention time test conditions are not a limitation for the compound. As long as the above test conditions are used for measurement, and the obtained retention time is the same as or within the error range of the retention time recorded above, and the compound is a stereoisomer in the compounds defined by the above retention time, it falls within the scope of protection of the present disclosure.

The present disclosure also provides a pharmaceutical composition, which comprises:
(1) the compound of formula (I), the pharmaceutically acceptable salt thereof, or the solvate of the pharmaceutically acceptable salt according to any one of the present disclosure;
(2) a pharmaceutically acceptable excipient.

The present disclosure also provides a use of the compound of formula (I), the pharmaceutically acceptable salt thereof, or the solvate of the pharmaceutically acceptable salt described in any one of the above, or the above pharmaceutical composition in the manufacture of a medicament for preventing and/or treating cancer.

The present disclosure also provides a method for preventing and/or treating cancer, which comprises administering to a patient a therapeutically effective amount of the compound of formula (I), the pharmaceutically acceptable salt thereof, or the solvate of the pharmaceutically acceptable salt according to any one of the present disclosure.

On the basis of not violating the common sense in the art, the preferred conditions above can be arbitrarily combined to obtain the preferred examples of the present disclosure.

The reagents and raw materials used in the present disclosure are commercially available.

Unless otherwise specified, the terms used in the present disclosure have the following meanings:

It can be understood by those skilled in the art that according to the conventions used in the art, the " 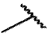 " used in the structural formula of the group described in the present disclosure means that the corresponding group is connected with other moieties and groups in the compound through this site.

The substituent used herein may be preceded by a single dash "-" to indicate that the named substituent is connected to the parent moiety through a single bond.

If a linking group is expressed as "absent", the structures on both sides of the linking group are directly connected by a single bond, for example, -A-B-C-. When B is absent, -A-B-C- is -A-C-.

The term "pharmaceutically acceptable" means that salts, solvents, excipients, etc. are generally non-toxic, safe, and suitable for use by patients. The "patient" is preferably a mammal, more preferably a human.

The term "pharmaceutically acceptable salt" refers to the salt prepared by the compound of the present disclosure and a relatively nontoxic and pharmaceutically acceptable acid or base. When the compound of the present disclosure contains a relatively acidic functional group, a base addition salt can be obtained by bringing the neutral form of the compound into contact with a sufficient amount of the pharmaceutically acceptable base in a pure solution or a suitable inert solvent. When the compound of the present disclosure contains a relatively basic functional group, an acid addition salt can be obtained by bringing the neutral form of the compound into contact with a sufficient amount of the pharmaceutically acceptable acid in a pure solution or a suitable inert solvent. The pharmaceutically acceptable acid includes an inorganic acid. The pharmaceutically acceptable acid includes an organic acid. When the compounds of the present disclosure contain relatively acidic and relatively basic functional groups, they can be converted into base addition salts or acid addition salts. For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to a substance formed by combining a compound of the present disclosure with a stoichiometric or non-stoichiometric solvent. Solvent molecules in the solvate can exist in an ordered or unordered arrangement. The solvent includes, but is not limited to, water, methanol, ethanol, etc.

The "pharmaceutically acceptable salt" and "solvate" in the term "solvate of pharmaceutically acceptable salt" are defined as above, and the solvate of pharmaceutically acceptable salt refers to a substance formed by combining the compound of the present disclosure: 1, with a relatively nontoxic and pharmaceutically acceptable acid or base, and, 2, with a stoichiometric or non-stoichiometric solvent. The "solvate of pharmaceutically acceptable salt" includes, but is not limited to, the hydrochloride monohydrate of the compound of the present disclosure.

The terms "compound", "pharmaceutically acceptable salt", "solvate", and "solvate of pharmaceutically acceptable salt", if stereoisomers exist, may exist in the form of a single stereoisomer or a mixture thereof (e.g., racemate). The term "stereoisomer" refers to a cis-trans isomer or an optical isomer. These stereoisomers can be separated, purified, and enriched by asymmetric synthesis methods or chiral separation methods (including but not limited to thin-layer chromatography, rotary chromatography, column chromatography, gas chromatography, high-pressure liquid chromatography, etc.), and can also be obtained by chiral resolution through bonding (such as chemical bonding) with other chiral compounds or salt formation (such as physical bonding), etc. The term "single stereoisomer" refers to a stereoisomer of the compound of the present disclosure, with a mass content of not less than 95% relative to all stereoisomers of the compound.

The terms "compound", "pharmaceutically acceptable salt", "solvate", and "solvate of pharmaceutically acceptable salt", if tautomers exist, may exist in the form of a single tautomer or a mixture thereof, preferably in the form of a relatively stable tautomer.

When an arbitrary variable (e.g., R₁₋₁) appears many times in the definition of a compound, the definition of each occurrence of the variable has nothing to do with the definitions of other occurrences, and their meanings are independent of each other and have no influence on each other. Therefore, if a group is substituted by 1, 2, or 3 R₁₋₁ groups, that is, the group may be substituted by up to 3 R₁₋₁ groups, and the definition of R₁₋₁ groups of this position and the definition of R₁₋₁ groups of the remaining positions are independent of each other. Additionally, combinations of substituents and/or variables are permissible only if such combinations result in stable compounds.

The term "alkyl" refers to a linear or branched alkyl group with a specified number of carbon atoms. Examples of alkyl include methyl, ethyl, n-propyl, isopropyl, n-butyl, tert-butyl, isobutyl, sec-butyl, n-pentyl, n-hexyl, and similar alkyl groups.

The term "alkoxy" refers to the group -O-R_{X}, wherein R_{X} is the alkyl as defined above.

The term "alkenyl" refers to a linear or branched alkene containing one or more than one carbon-carbon double bond and has no carbon-carbon triple bonds with a specific number of carbon atoms, and the one or more than one carbon-carbon double bond may be internal or terminal. Examples of alkene include vinyl, allyl, methylvinyl, propenyl, butenyl, pentenyl, 1,1-dimethyl-2-propenyl, hexenyl, etc.

The term "alkynyl" refers to a linear or branched hydrocarbon group having one or more than one triple bond with a specific number of carbon atoms (such as C₂-C₆ alkynyl, also such as C₂-C₄ alkynyl). The one or more than one carbon-carbon triple bond may be internal or terminal, such as propynyl 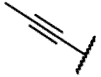 with an internal triple bond or propynyl 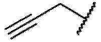 with a terminal triple bond.

The term "cycloalkyl" refers to a saturated monocyclic ring, a bridged ring, or a spiro ring group with a specified number of ring carbon atoms (for example, C₃-C₆) and the ring atoms are composed of only carbon atoms. Monocyclic cycloalkyl includes, but is not limited to, cyclopropyl, cyclobutyl, cyclopentyl, cyclohexyl, etc.

The term "heterocycloalkyl" refers to a cyclic group with a specified number of ring atoms (e.g. 3- to 6-membered), a specified number of heteroatoms (e.g. 1, 2, or 3), and a specified type of heteroatoms (1, 2, or 3 of N, O, and S), which is a monocyclic ring, a bridged ring, or a spiro ring, and each ring is saturated. Heterocycloalkyl includes, but is not limited to, azetidinyl, tetrahydropyrrolyl, tetrahydrofuryl, morpholinyl, piperidinyl, etc.

The term "heteroaryl" refers to a cyclic aromatic group with a specified number of ring atoms (e.g., 3- to 15-membered), a specified number of heteroatoms (e.g., 1, 2, or 3), and a specified type of heteroatoms (1, 2, or 3 of N, O, and S), which is monocyclic or bicyclic. When the cyclic aromatic group is bicyclic, each ring is aromatic, such as furyl, pyridyl, pyridazinyl, pyrimidinyl, pyrazinyl, thienyl, isoxazolyl, oxazolyl, diazolyl, imidazolyl, pyrrolyl, pyrazolyl , triazolyl, tetrazolyl, thiazolyl, isothiazolyl, thiadiazolyl, benzimidazolyl, indolyl, indazolyl, benzothiazolyl, benzisothiazolyl, quinolyl, isoquinolyl.

The term "carbocyclic ring" refers to a saturated or unsaturated monocyclic, bicyclic, or tricyclic cyclic group with a specified number of ring atoms (for example, 5- to 15-membered), and the ring atoms are composed of only carbon atoms. When the carbocyclic ring is bicyclic or tricyclic, it can be connected by fused or spiro linkage, such as,

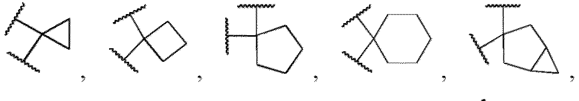

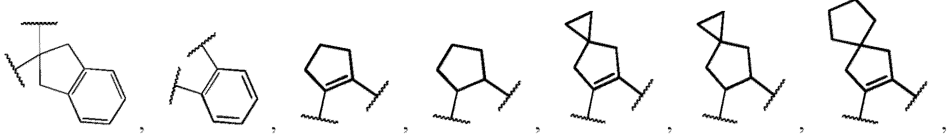

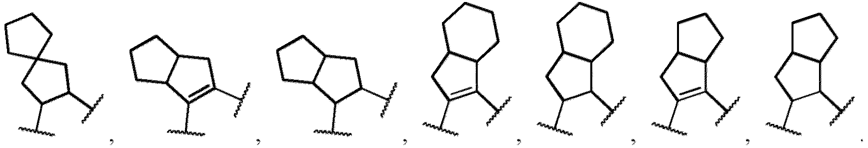

The term "heterocyclic ring" refers to a saturated or unsaturated cyclic group with a specified number of ring atoms (e.g. 5- to 15-membered), a specified number of heteroatoms (e.g. 1, 2, or 3), and a specified type of heteroatoms (1, 2, or 3 of N, O, and S), which is monocyclic, bicyclic, or tricyclic. When the heterocyclic ring is bicyclic or tricyclic, it can be connected by fused or spiro linkage, such as,

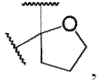

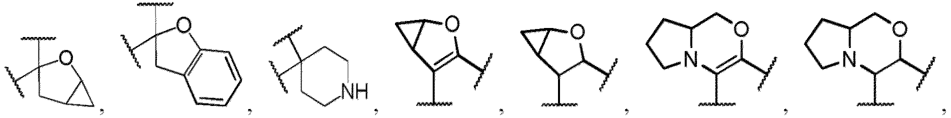

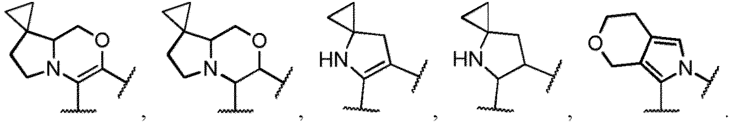

The term "pharmaceutically acceptable excipients" refers to the excipients and additives used in the manufacture of drugs and the formulation of prescriptions, which are all substances contained in pharmaceutical preparations except active ingredients. See the Pharmacopoeia of the People's Republic of China (2015 Edition) Part IV, or, Handbook of Pharmaceutical Excipients (Raymond C Rowe, 2009 Sixth Edition).

The term "treatment" refers to therapeutic therapy. When referring to a specific disorder, treatment refers to: (1) ameliorating one or more biological manifestations of the disease or disorder, (2) interfering with (a) one or more points in the biological cascade leading to or causing the disorder or (b) one or more biological manifestations of the disorder, (3) ameliorating one or more symptoms, effects, or side effects associated with the disorder, or one or more symptoms, effects or side effects associated with the disorder or its treatment, or (4) slowing the progression of the disorder or one or more biological manifestations of the disorder.

The term "prevention" refers to the reduction of the risk of acquiring or developing diseases or disorders.

The term "therapeutically effective amount" refers to the amount of a compound that is sufficient to effectively treat the diseases or disorders described herein when administered to a patient. The "therapeutically effective amount" will vary according to the compound, the disease and its severity, and the age of the patient to be treated, but it can be adjusted by those skilled in the art as needed.

The term "patient" refers to any animal that will or has received the administration of the compound or composition according to the example of the present disclosure, preferably a mammal, most preferably a human. The term "mammal" includes any mammal. Examples of mammals include, but are not limited to, cows, horses, sheep, pigs, cats, dogs, mice, rats, rabbits, guinea pigs, monkeys, humans, etc., most preferably humans.

The positive progressive effect of the present disclosure lies in that the compound of the present disclosure can effectively inhibit the proliferation of tumor cells and has good inhibitory activity, thereby treating related diseases.

### DETAILED DESCRIPTION OF THE PREFERRED EMBODIMENT

The present disclosure is further described below by the way of examples, but the present disclosure is not thereby limited to the scope of the described examples. The experimental methods for which the specific conditions are not specified in the following examples are selected according to the conventional methods and conditions, or according to the commodity instructions.

### Example 1

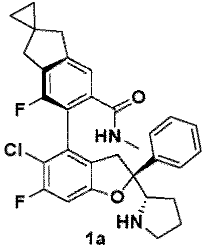

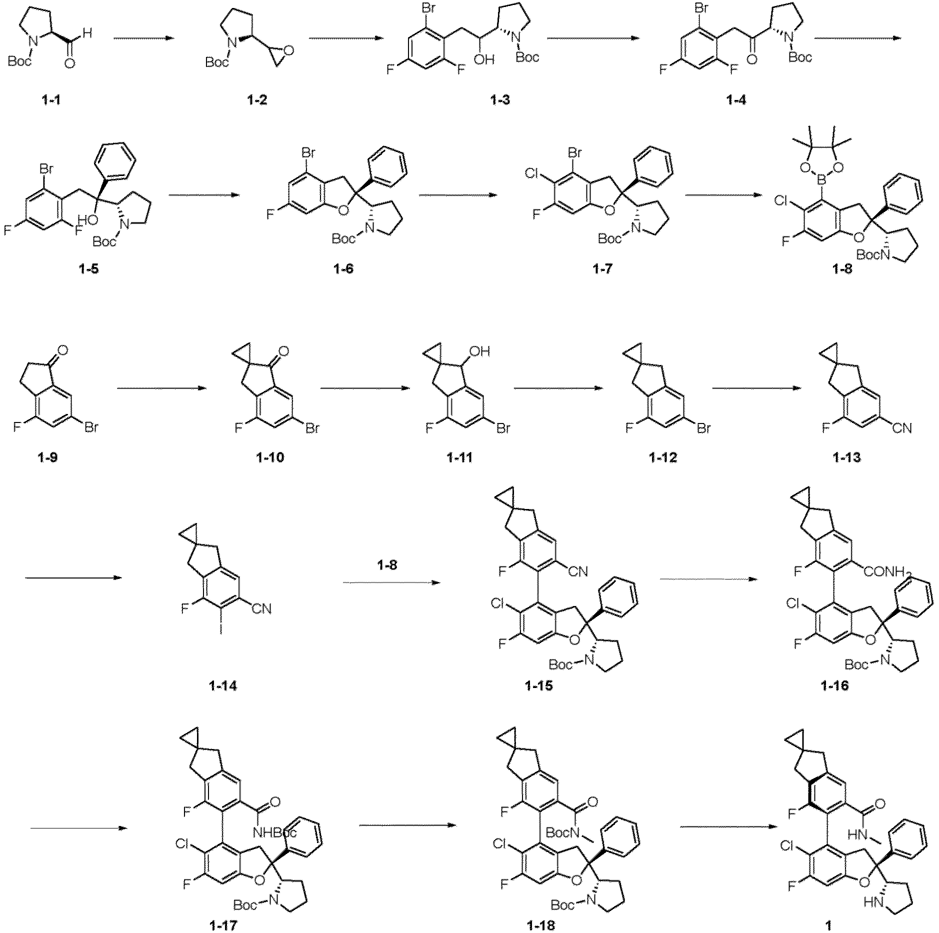

Step 1: Sodium hydride (36.1 g, 903 mmol, 60%) was dissolved in dimethyl sulfoxide (700 mL), and the reaction mixture was heated to 70°C and reacted for 1 hour. The reaction mixture was cooled to room temperature, diluted with THF (350 mL), added with trimethylsulfoxonium iodide (298 g, 1.36 mol) in batches at 5°C, warmed to room temperature, and reacted for 30 minutes. Compound 1-1 (90.0 g, 451 mmol) was dissolved in tetrahydrofuran (350 mL), and quickly added dropwise to the reaction mixture. After the dropwise addition was completed, the reaction mixture was reacted at room temperature overnight. After the reaction was completed, the reaction mixture was cooled to 5°C, slowly poured into ice water (2 L) to quench, and extracted with ethyl acetate (1000 mL × 2). The organic phases were combined, washed with saturated brine (1000 mL × 2), dried over anhydrous sodium sulfate, concentrated under reduced pressure, and purified by silica gel column chromatography (PE: EA= 10:1) to obtain compound **1-2** (32.1 g, Y: 33%). LC-MS m/z (ESI): 214.1 [M+H]⁺.

Step 2: 1-Bromo-3,5-difluoro-2-iodobenzene (62.4 g, 196 mmol) was dissolved in tetrahydrofuran (624 mL) under nitrogen atmosphere. The reaction mixture was cooled to -70°C, added dropwise with a solution of isopropylmagnesium chloride in tetrahydrofuran (97.8 mL, 196 mmol, 2.0 mol/L), heated to - 40°C, and reacted for 1 hour. Cuprous iodide (8.60 g, 45.2 mmol) was added thereto, and the reaction mixture was reacted for another 10 minutes. The reaction system was cooled to -70°C. Compound 1-2 (32.1 g, 150 mmol) was dissolved in tetrahydrofuran (300 mL), and added dropwise to the reaction system. The reaction mixture was reacted for 30 minutes, warmed to room temperature, and reacted for 12 hours. After the reaction was completed as detected by TLC (PE: EA = 5:1), the reaction mixture was cooled to room temperature in an ice water bath, and added with saturated ammonium chloride solution (200 mL) to quench. The phases were separated, and the organic phase was collected, dried over anhydrous sodium sulfate, concentrated under reduced pressure, and purified by silica gel column chromatography (PE: EA = 10:1) to obtain compound 1-3 (27.5 g, Y: 45%). LC-MS m/z (ESI): 428.1, 430.1 [M+Na]⁺.

Step 3: Compound 1-3 (27.5 g, 67.7 mmol) was dissolved in dichloromethane (200 mL) under nitrogen atmosphere, then Dess-Martin periodinane (43.1 g, 101 mmol) was added thereto in batches at room temperature, and the reaction mixture was reacted for 2 hours. The reaction mixture was cooled in an ice water bath, and added with saturated sodium bicarbonate solution to quench. The phases were separated, and the organic phase was collected, dried over anhydrous sodium sulfate, concentrated under reduced pressure, and purified by silica gel column chromatography (PE: EA = 8:1) to obtain compound 1-4 (21.9 g, Y: 80%). LC-MS m/z (ESI): 426.1, 428.1 [M+Na]⁺.

Step 4: Compound 1-4 (21.9 g, 54.2 mmol) was dissolved in dichloromethane (220 mL) and n-heptane (220 mL) under nitrogen atmosphere. The reaction mixture was cooled to 0°C, added dropwise with phenylmagnesium bromide (48.4 mL, 135 mmol, 2.8 mol/L), warmed to room temperature, and reacted for 12 hours. After the reaction was completed, the reaction mixture was cooled to 0°C, and added with saturated ammonium chloride (20 mL) to quench. The phases were separated, and the organic phase was collected, dried over anhydrous sodium sulfate, concentrated under reduced pressure, and purified by silica gel column chromatography (PE: EA = 5:1) to obtain compound 1-5 (17.5 g, Y: 67%). LC-MS m/z (ESI): 482.2, 484.2 [M+H]⁺.

Step 5: Compound 1-5 (17.5 g, 36.3 mmol) was dissolved in tetrahydrofuran (200 mL) under nitrogen atmosphere. The reaction mixture was cooled to -5°C, and added dropwise with a solution of potassium tert-butoxide in tetrahydrofuran (54.4 mL, 54.4 mmol, 1.0 mol/L). After the dropwise addition was completed, the reaction mixture was kept at -5 to 0°C and reacted for 2 hours. After the reaction was completed, the reaction mixture was concentrated under reduced pressure, and purified by silica gel column chromatography (PE: EA = 5:1) to obtain compound 1-6 (13.2 g, Y: 78%). LC-MS m/z (ESI): 406.1, 408.2 [M-tBu]⁺.

Step 6: Compound 1-6 (13.2 g, 28.6 mmol) was dissolved in tetrahydrofuran (70 mL) and acetonitrile (140 mL). The reaction mixture was cooled to 0°C, added with p-toluenesulfonic acid (6.39 g, 37.1 mmol) and N-chlorosuccinimide (4.96 g, 37.1 mmol), warmed to room temperature, and reacted for 12 hours. After the reaction was completed, the reaction mixture was added with saturated sodium bicarbonate aqueous solution (200 mL) to quench, and extracted with ethyl acetate (100 mL × 2). The organic phases were combined, washed with saturated brine (100 mL × 2), dried over anhydrous sodium sulfate, concentrated under reduced pressure, and purified by silica gel column chromatography (PE: EA = 10:1) to obtain a product (8.50 g, ee: 82%), which was separated by chiral resolution (CHIRALPAK IA, CO₂: IPA = 70:30) to obtain compound 1-7 (7.0 g, ee > 98%, Y: 49%). LC-MS m/z (ESI): 518.1, 520.1 [M+Na]⁺.

Step 7: Compound 1-7 (7.00 g, 14.1 mmol) was dissolved in toluene (70 mL), and bis(neopentyl glycolato)diboron (9.05 g, 35.4 mmol), potassium acetate (4.15 g, 42.4 mmol), and [1,1'-bis(diphenylphosphino)ferrocene]palladium(II) dichloride (1.46 g, 1.41 mmol) were sequentially added thereto. The reaction mixture was replaced with nitrogen, heated to 110°C, and reacted for 12 hours. After the reaction was completed, the reaction mixture was concentrated under reduced pressure, and subjected to silica gel column chromatography (PE: EA = 5:1) to obtain 9.80 g of a crude product, which was purified by preparative reversed-phase chromatography to obtain compound 1-8 (4.20 g, Y: 55%). LC-MS m/z (ESI): 566.3, 568.3 [M+Na]⁺.

Step 8: Compound 1-9 (5.0 g, 21.8 mmol) was dissolved in N-methylpyrrolidone (50 mL). The reaction mixture was cooled to 0°C under nitrogen atmosphere, sequentially added with sodium hydride (5.24 g, 87.3 mmol) and 1,2-dibromoethane (140 mg, 1.98 mmol), gradually warmed to 25°C, and reacted for 12 hours. TLC (PE: EtOAc = 5:1) showed that the starting material was completely reacted. The reaction mixture was poured into ice-cold 0.5 M hydrochloric acid (200 mL) to quench, and extracted with ethyl acetate (100 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:10) to obtain compound 1-10 (3.0 g, Y: 53%). ¹H NMR (400 MHz, DMSO-*d₆*) δ 7.91-7.85 (m, 1H), 7.68 (s, 1H), 3.22 (s, 2H), 1.37-1.25 (m, 4H).

Step 9: Compound 1-10 (3.0 g, 11.7 mmol) was dissolved in methanol (30 mL). The reaction mixture was cooled to 0°C under nitrogen atmosphere, added with sodium borohydride (489 mg, 12.9 mmol) in batches, and reacted at 0°C for 2 hours. TLC (PE: EA = 5:1) showed that the starting material was completely reacted. The reaction mixture was poured into 15% sodium hydroxide solution (20 mL) at 0°C to quench, and extracted with ethyl acetate (20 mL × 3). The organic phases were combined, washed with saturated brine (50 mL), dried over anhydrous sodium sulfate, filtered, and the filtrate was concentrated under reduced pressure to obtain compound 1-11 (3.0 g, crude).

Step 10: Compound 1-11 (3.0 g, 11.7 mmol) was dissolved in triethoxysilane (9 mL). The reaction mixture was cooled to 0°C under nitrogen atmosphere, added with trifluoroacetic acid (6 mL), gradually warmed to 25°C, and reacted for 12 hours. TLC (PE: EA= 20:1) showed that the starting material was completely reacted. The reaction mixture was poured into saturated sodium bicarbonate (50 mL) to quench, and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:10) to obtain compound 1-12 (1.1 g, Y: 38%). ¹H NMR (400 MHz, DMSO-*d₆*) δ 7.31-7.25 (m, 2H), 2.90 (s, 2H), 2.83 (s, 2H), 0.65-0.60 (m, 4H).

Step 11: Compound 1-12 (1.1 g, 4.98 mmol) was dissolved in N,N-dimethylformamide (10 mL). The reaction mixture was sequentially added with zinc cyanide (584 mg, 4.98 mmol), tris(dibenzylideneacetone)dipalladium (227 mg, 248 µmol), and 1,1'-bis(diphenylphosphino)ferrocene (380 mg, 496 µmol) under nitrogen atmosphere, gradually heated to 100°C, and reacted for 12 hours. TLC (PE: EA = 20:1) showed that the starting material was completely reacted. The reaction mixture was cooled to 25°C, and filtered through diatomite. The filtrate was added with water (30 mL), and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 10:1) to obtain compound **1-13** (600 mg, Y: 64%). ¹H NMR (400 MHz, DMSO-*d₆*) δ 7.66-7.60 (m, 1H), 7.57 (s, 1H), 2.95 (s, 4H), 0.65 (d, *J* = 4.2 Hz, 4H).

Step 12: Compound 1-13 (600 mg, 3.59 mmol) was dissolved in tetrahydrofuran (6 mL). The reaction mixture was cooled to -70°C under nitrogen atmosphere, added with lithium diisopropylamide (2 M, 1.8 mL) and stirred for 30 minutes, then added with iodine (1.03 g, 4.05 mmol), warmed to 25°C, and reacted for 1 hour. TLC (PE: EA = 20:1) showed that the starting material was completely reacted. The reaction mixture was poured into saturated ammonium chloride (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (EA: PE = 1:10) to obtain compound 1-14 (600 mg, Y: 68%). ¹H NMR (400 MHz, DMSO-*d₆*) δ 7.60 (s, 1H), 2.97 (s, 2H), 2.91 (s, 2H), 0.64 (d, *J* = 4.7 Hz, 4H).

Step 13: Compound 1-14 (200 mg, 638 µmol) and compound **1-8** (382 mg, 702 µmol) were dissolved in toluene (2 mL) and water (0.4 mL). The reaction mixture was sequentially added with tris(dibenzylideneacetone)dipalladium (58.4 mg, 63.8 µmol), 4,5-bis(diphenylphosphino)-9,9-dimethylxanthene (73.8 mg, 127 µmol), and potassium phosphate (406 mg, 1.92 µmol) under nitrogen atmosphere, gradually heated to 110°C, and reacted for 12 hours. LCMS showed that the starting material was completely reacted. The reaction mixture was cooled to 25°C, poured into saturated ammonium chloride (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (EA: PE = 1:10) to obtain compound 1-15 (180 mg, Y: 46%). LC-MS m/z (ESI): 625 [M+Na]⁺.

Step 14: Compound 1-15 (180 mg, 289 µmol) and sodium hydroxide (92.5 mg, 2.31 mmol) were dissolved in ethanol (2 mL) and water (1 mL), and the reaction mixture was heated to 110°C and reacted for 24 hours. LCMS showed that the starting material was completely reacted. The reaction mixture was cooled to 0°C, poured into 1 M hydrochloric acid (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound 1-16 (110 mg, crude). LC-MS m/z (ESI): 619 [M-H]⁻.

Step 15: Compound 1-16 (110 mg, 117 µmol) was dissolved in tetrahydrofuran (2 mL). The reaction mixture was added with sodium hydride (9.85 mg, 164.19 µmol) and di-tert-butyl dicarbonate (35.8 mg, 164 µmol) in batches at 0°C, returned to room temperature, and stirred and reacted for 2 hours. TLC (PE: EA = 2:1) showed that the starting material was completely reacted. The reaction mixture was cooled to 0°C, poured into 1 M hydrochloric acid (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound 1-17 (110 mg, crude).

Step 16: Compound 1-17 (110 mg, crude) was dissolved in tetrahydrofuran (2 mL). The reaction mixture was sequentially added with sodium hydride (9.85 mg, 164 µmol) and iodomethane (35.8 mg, 164 µmol) at 0°C, and stirred and reacted at room temperature for 2 hours. TLC (PE: EA = 2:1) showed that the starting material was completely reacted. The reaction mixture was cooled to 0°C, poured into 1 M hydrochloric acid (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 10:1) to obtain compound 1-18 (100 mg, Y: 98%). LC-MS m/z (ESI): 735 [M+H]⁺.

Step 17: Compound 1-18 (110 mg, 117 µmol) was dissolved in a solution of 20% trifluoroacetic acid in dichloromethane (2 mL), and the reaction mixture was reacted at 25°C for 0.5 hours. TLC (DCM: MeOH = 10:1) showed that the starting material was completely reacted. The reaction mixture was cooled to 0°C, poured into saturated sodium carbonate (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by preparative high-pressure liquid chromatography (column model: Xselect CSH Prep Fluoro-Phenyl, 150 * 19 mm, 5 µm; eluent: 0.05% FA/CH₃CN; gradient: 41% to 61%; flow rate: 20 mL/min; run time: 11 min; retention time: 7.2 min) to obtain compound **1a** (10.5 mg, Y: 14%). LC-MS m/z (ESI): 535.30 [M+H]⁺. ¹H NMR (400 MHz, DMSO-*d₆*) δ 8.98 (br, 1H), 8.35-8.28 (m, 1H), 7.58-7.51 (m, 2H), 7.48-7.43 (m, 2H), 7.40-7.36 (m, 2H), 7.12 (d, *J* = 9.2 Hz, 1H), 4.30-4.20 (m, 1H), 3.60-3.50 (m, 1H), 3.22-3.02 (m, 2H), 3.00-2.88 (m, 4H), 2.63 (d, *J =* 4.4 Hz, 3H), 2.48-2.40 (m, 1H), 2.18-2.10 (m, 1H), 1.96-1.70 (m, 3H), 0.66 (s, 4H).

### Example 2

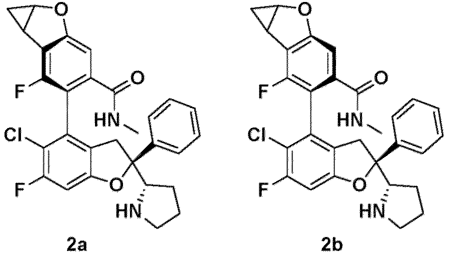

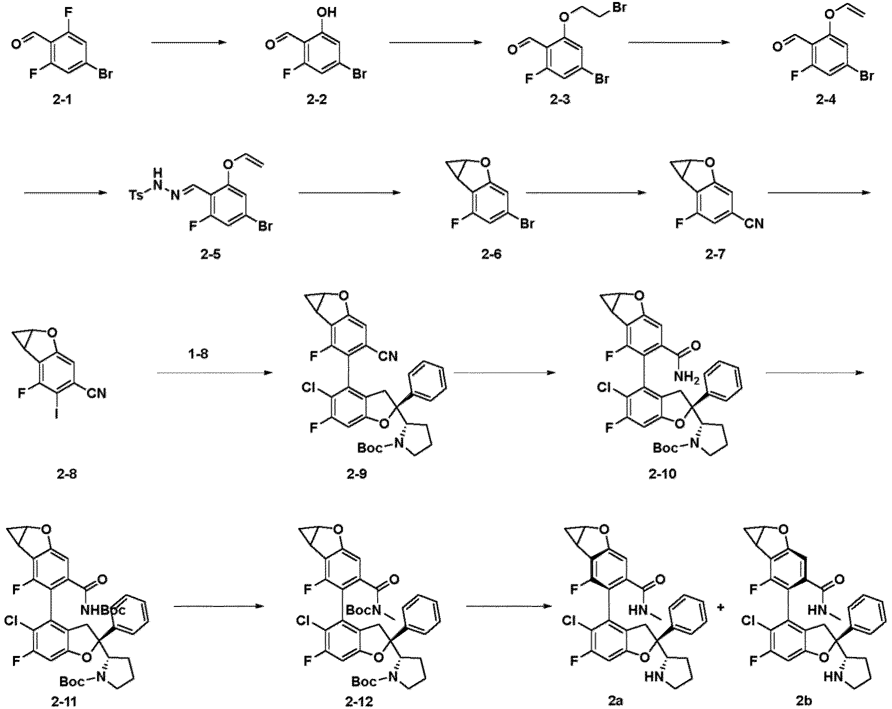

Step 1: Compound 2-1 (50 g, 226 mmol) was dissolved in dimethyl sulfoxide (250 mL). The reaction mixture was cooled to 0°C under nitrogen atmosphere, and added with potassium hydroxide (25.3 g, 452 mmol) in batches. After the addition was completed, the reaction mixture was heated to 60°C and reacted for 4 hours. TLC (PE: EA = 5:1) showed that the starting material was completely reacted. The reaction mixture was cooled to room temperature, poured into ice-cold 0.5 M hydrochloric acid (500 mL) to quench, and extracted with ethyl acetate (500 mL × 3). The organic phases were combined, washed with saturated brine (500 mL), dried over anhydrous sodium sulfate, filtered, and the filtrate was concentrated under reduced pressure to obtain compound 2-2 (35.0 g, crude). LC-MS m/z (ESI): 219, 221 [M+H]⁺.

Step 2: Compound 2-2 (35.0 g, 159 mmol) was dissolved in acetonitrile (350 mL). The reaction mixture was cooled to 0°C under nitrogen atmosphere, sequentially added with potassium carbonate (22.0 g, 159 mmol), 1,2-dibromoethane (150 g, 799 mmol), and potassium iodide (2.65 g, 15.9 mmol), heated to 80°C, and reacted for 12 hours. TLC (PE: EA = 5:1) showed that the starting material was completely reacted. The reaction mixture was poured into ice-cold 0.5 M hydrochloric acid (500 mL) at 0°C to quench, and extracted with ethyl acetate (500 mL × 3). The organic phases were combined, washed with saturated brine (500 mL), dried over anhydrous sodium sulfate, and filtered. The filtrate was concentrated under reduced pressure and then purified by silica gel column chromatography (EA: PE = 1:10) to obtain compound 2-3 (26.0 g, Y: 49%). ¹H NMR (400 MHz, DMSO-*d₆*) δ 10.29 (s, 1H), 7.40 (d, *J* = 1.2 Hz, 1H), 7.35-7.29 (m, 1H), 4.58-4.52 (m, 2H), 3.89-3.82 (m, 2H).

Step 3: Compound 2-3 (3.5 g, 10.7 mmol) was dissolved in a mixed solvent of toluene (30.0 mL) and sodium hydroxide (50%, 10 mL). The reaction mixture was cooled to 0°C under nitrogen atmosphere, added with tetrabutylammonium hydrogen sulfate (3.83 g, 11.2 mmol), warmed to 10°C, and reacted for 1 hour. TLC (PE: EA = 20:1) showed that the starting material was completely reacted. The reaction mixture was poured into ice-cold 0.5 M hydrochloric acid (50 mL) at 0°C to quench, and extracted with methyl tert-butyl ether (50 mL × 3). The organic phases were combined, washed with saturated brine (50 mL), dried over anhydrous sodium sulfate, filtered, and the filtrate was concentrated under reduced pressure to obtain compound 2-4 (2.63 g, crude).

Step 4: Compound 2-4 (400 mg, 1.63 mmol) was dissolved in tetrahydrofuran (4 mL), then 4-methylbenzenesulfonhydrazide (455 mg, 2.45 mmol) was added thereto under nitrogen atmosphere, and the reaction mixture was reacted at 25°C for 2 hours. TLC (PE: EA = 20:1) showed that the starting material was completely reacted. The reaction mixture was directly purified by reversed-phase chromatography to obtain compound 2-5 (50 mg, Y: 7%). ¹H NMR (400 MHz, DMSO-*d*₆) δ 11.62 (s, 1H), 7.95 (s, 1H), 7.72 (d, J= 8.3 Hz, 2H), 7.39 (d, J= 8.0 Hz, 2H), 7.38-7.33 (m, 1H), 7.19 (s, 1H), 6.93-6.86 (m, 1H), 4.83-4.77 (m, 1H), 4.67-4.61 (m, 1H), 2.36 (s, 3H). LC-MS m/z (ESI): 413, 415 [M+H]⁺.

Step 5: Compound 2-5 (500 mg, 1.21 mmol) was dissolved in toluene (5.0 mL), then rhodium(II) acetate dimer (53.4 mg, 120 µmol) and sodium hydride (79.8 mg, 1.33 mmol) were sequentially added thereto under nitrogen atmosphere, and the reaction mixture was heated to 100°C and reacted for 3 hours. TLC (PE: EA = 20:1) showed that the starting material was completely reacted. The reaction mixture was poured into saturated ammonium chloride (20 mL) at 0°C to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 10:1) to obtain compound 2-6 (100 mg, Y: 36%). ¹H NMR (400 MHz, CDCl₃) δ 7.20-7.14 (m, 2H), 4.89-4.83 (m, 1H), 2.74-2.67 (m, 1H), 1.12-1.05 (m, 1H), 0.42-0.35 (m, 1 H).

Step 6: Compound 2-6 (0.6 g, 2.62 mmol) was dissolved in N,N-dimethylformamide (10 mL). The reaction mixture was sequentially added with zinc cyanide (307 mg, 2.62 mmol), tris(dibenzylideneacetone)dipalladium (119 mg, 130 µmol), and 1,1'-bis(diphenylphosphino)ferrocene (200 mg, 261 µmol) under nitrogen atmosphere, gradually heated to 100°C, and reacted for 12 hours. TLC (PE: EA = 20:1) showed that the starting material was completely reacted. The reaction mixture was cooled to 25°C, filtered through diatomite, and the filtrate was added with water (30 mL) and ethyl acetate (50 mL). The organic phases were combined, washed with saturated brine (50 mL), dried over anhydrous sodium sulfate, and filtered. The filtrate was concentrated under reduced pressure and then purified by silica gel column chromatography (EA: PE = 1:10) to obtain compound 2-7 (350 mg, Y: 76%). ¹H NMR (400 MHz, CDCl₃) δ 6.96-6.90 (m, 1H), 6.89 (s, 1H), 4.99-4.93 (m, 1H), 2.83-2.77 (m, 1H), 1.26-1.20 (m, 1H), 0.47-0.41 (m, 1H).

Step 7: Compound 2-7 (350 mg, 2.00 mmol) was dissolved in tetrahydrofuran (6 mL). The reaction mixture was cooled to -70°C under nitrogen atmosphere, added with lithium diisopropylamide (2 M, 1.8 mL) and stirred for 30 minutes, then added with iodine (557 mg, 2.20 mmol), warmed to 25°C with dry ice bath removed, and reacted for 1 hour. TLC (PE: EA = 20:1) showed that the starting material was completely reacted. The reaction mixture was poured into saturated ammonium chloride (10 mL) at 0°C to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 10:1) to obtain compound 2-8 (600 mg, Y: 64%). ¹H NMR (400 MHz, CDCl₃) δ 6.94 (s, 1H), 5.02-4.95 (m, 1H), 2.86-2.80 (m, 1H), 1.28-1.23 (m, 1H), 0.48-0.43 (m, 1H).

Step 8: Compound 2-8 (250 mg, 664 µmol) and compound 1-8 (361 mg, 664 µmol) were dissolved in toluene (2 mL) and water (0.4 mL). The reaction mixture was sequentially added with tris(dibenzylideneacetone)dipalladium (60.7 mg, 66.4 µmol), 4,5-bis(diphenylphosphino)-9,9-dimethylxanthene (776 mg, 132 µmol), and potassium phosphate (422 mg, 1.99 mmol) under nitrogen atmosphere, gradually heated to 110°C, and reacted for 12 hours. LCMS showed that the starting material was completely reacted. The reaction mixture was cooled to 25°C, poured into saturated ammonium chloride (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (EA: PE = 1:10) to obtain compound 2-9 (260 mg, Y: 66%). LC-MS m/z (ESI): 613 [M+Na]⁺.

Step 9: Compound 2-9 (50.0 mg, 84.6 µmol) and sodium hydroxide (27.0 mg, 676 mmol) were dissolved in ethanol (2 mL) and water (1 mL), and the reaction mixture was heated to 110°C and reacted for 24 hours. LCMS showed that the starting material was completely reacted. The reaction mixture was cooled to 0°C, poured into 1 M HCl (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound 2-10 (50 mg, crude). LC-MS m/z (ESI): 609 [M+H]⁺

Step 10: Compound 2-10 (50 mg, 82.0 µmol) was dissolved in tetrahydrofuran (2 mL), then sodium hydride (9.85 mg, 164 µmol) and di-tert-butyl dicarbonate (35.8 mg, 164 µmol) were sequentially added thereto at 0°C, and the reaction mixture was stirred and reacted at room temperature for 2 hours. TLC (PE: EA = 2:1) showed that the starting material was completely reacted. The reaction mixture was cooled to 0°C, poured into 1 M hydrochloric acid (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound 2-11 (50 mg, crude).

Step 11: Compound 2-11 (50 mg, 82.0 µmol) was dissolved in tetrahydrofuran (2 mL), then sodium hydride (9.85 mg, 164 µmol) and iodomethane (35.8 mg, 164 µmol) were sequentially added thereto at 0°C, and the reaction mixture was stirred and reacted at room temperature for 2 hours. TLC (PE: EA = 2:1) showed that the starting material was completely reacted. The reaction mixture was cooled to 0°C, poured into 1 M hydrochloric acid (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (EA: PE = 1:10) to obtain compound 2-12 (30 mg, Y: 50%). LC-MS m/z (ESI): 646 [M+Na]⁺.

Step 12: Compound 2-12 (150 mg, 240 µmol) was dissolved in a solution of 20% trifluoroacetic acid in dichloromethane (2 mL), and the reaction mixture was reacted at 25°C for 0.5 hours. TLC (DCM: MeOH = 10:1) showed that the starting material was completely reacted. The reaction mixture was cooled to 0°C, poured into saturated sodium carbonate (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a crude product, which was then purified by preparative high-pressure liquid chromatography (column model: Xselect CSH Prep Fluoro-Phenyl, 150 * 19 mm, 5 µm; eluent: 0.05% FA/CH₃CN; gradient: 40% to 60%; flow rate: 20 mL/min; run time: 13 min; retention time: 8.9 min for 2a and 7.6 min for 2b) to obtain compound 2a (9.5 mg, Y: 7%) and compound 2b (11.0 mg, Y: 8%). 2a: ¹H NMR (400 MHz, DMSO-*d₆*) δ 8.19 (s, 1H), 7.50-7.33 (m, 5H), 7.05 (d, *J* = 11.5 Hz, 1H), 6.98 (s, 1H), 5.18-5.13 (m, 1H), 3.52-3.46 (m, 1H), 2.95-2.78 (m, 4H), 2.68-2.50 (m, 3H), 1.87-1.21 (m, 6H), 0.38-0.33 (m, 1H). LC-MS m/z (ESI): 523.3 [M+H]⁺. 2b: ¹H NMR (400 MHz, DMSO-*d*₆) δ 8.23 (s, 1H), 8.16-8.10 (m, 1H), 7.49-7.43 (m, 2H), 7.33-7.22 (m, 3H), 6.97-6.90 (m, 2H), 5.19-5.14 (m, 1H), 3.53-3.47 (m, 1H), 3.19-3.13 (m, 2H), 2.94-2.88 (m, 1H), 2.80-2.70 (m, 1H), 2.61-2.50 (m, 1H), 2.24-2.17 (m, 3H), 1.52-1.23 (m, 6H), 0.42-0.37 (m, 1H). LC-MS m/z (ESI): 523.3 [M+H]⁺.

### Example 3

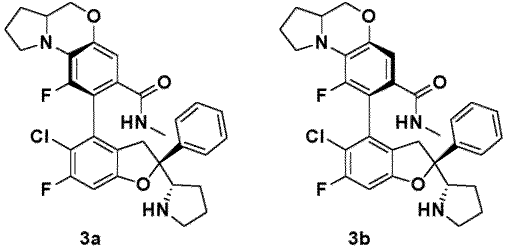

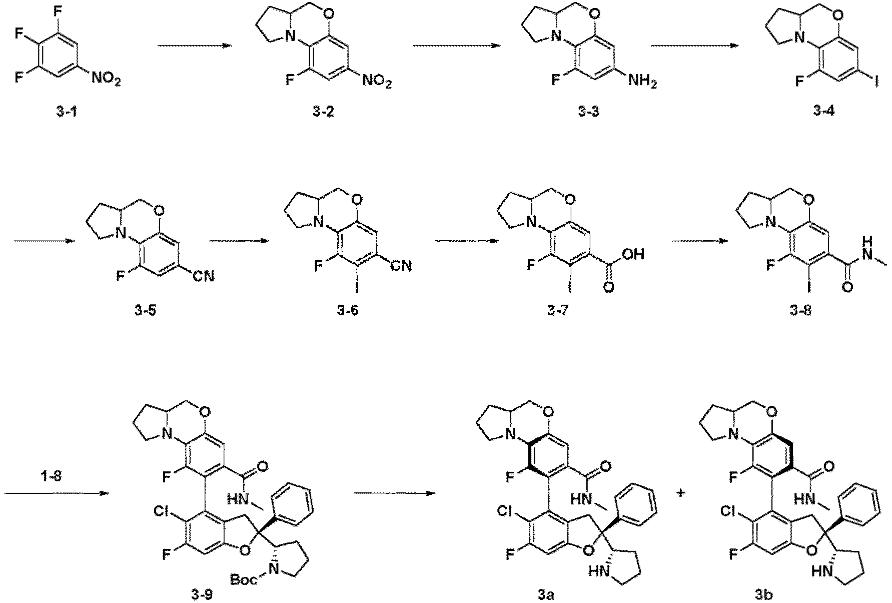

Step 1: Compound 3-1 (8.5 g, 48.0 mmol) was dissolved in dimethyl sulfoxide (85 mL). The reaction mixture was sequentially added with starting materials 2-(hydroxymethyl)pyrrolidine (4.9 g, 48.0 mmol) and potassium hydroxide (8.1 g, 144.0 mmol), reacted at 25°C for 2 hours, gradually heated to 80°C, and reacted for 2 hours. LCMS showed that the starting materials were completely reacted. The reaction mixture was cooled to 25°C, poured into ice water (400 mL) to quench, and extracted with ethyl acetate (100 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:2) to obtain compound 3-2 (4.8 g, 41%). LC-MS m/z (ESI): 239.1 [M+H]⁺.

Step 2: Compound 3-2 (4.7 g, 19.7 mmol) was dissolved in ethanol (50 mL), then water (10 mL), iron powder (1.8 g, 98.5 mmol), and ammonium chloride (5.2 g, 98.5 mmol) were sequentially added thereto, and the reaction mixture was gradually heated to 80°C and reacted for 2 hours. LCMS showed that the starting material was completely reacted. The reaction mixture was cooled to 25°C, and filtered through diatomite. The filtrate was added with water (100 mL), and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:1) to obtain compound 3-3 (3.1 g, Y: 65%). LC-MS m/z (ESI): 209.2 [M+H]⁺.

Step 3: Compound 3-3 (3.1 g, 14.9 mmol) was dissolved in acetonitrile (40 mL). The reaction mixture was added with cuprous iodide (8.5 g, 44.7 mmol), cooled to 0°C, added dropwise with tert-butyl nitrite (1.9 g, 22.3 mmol), gradually heated to 80°C, and reacted for 16 hours. LCMS showed that the starting material was completely reacted. The reaction mixture was cooled to 25°C, and filtered through diatomite. The filtrate was added with water (50 mL), and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:5) to obtain compound 3-4 (1.1 g, Y: 23%). ¹H NMR (400 MHz, CDCl₃): δ 7.00-6.95 (m, 1H), 6.69-6.63 (m, 1H), 4.25-4.19 (m, 1H), 3.93-3.88 (m, 1H), 3.50-3.45 (m, 1H), 3.35-3.28 (m, 1H), 3.08-3.01 (m, 1H), 2.21-2.15 (m, 1H), 1.98-1.90(m, 2H), 1.53-1.45(m, 1H).

Step 4: Compound 3-4 (1.1 g, 3.45 mmol) was dissolved in N-methylpyrrolidone (11 mL), then cuprous cyanide (1.2 g, 13.8 mmol) was added thereto, and the reaction mixture was gradually heated to 100°C and reacted for 16 hours. LCMS showed that the starting material was completely reacted. The reaction mixture was cooled to 25°C, and filtered through diatomite. The filtrate was added with water (30 mL), and extracted with ethyl acetate (30 mL × 3). The organic phases were combined, washed with saturated brine (30 mL), dried over anhydrous sodium sulfate, and filtered. The filtrate was concentrated under reduced pressure and then purified by silica gel column chromatography (EA: PE = 1:5) to obtain compound 3-5 (0.6 g, Y: 45%). LC-MS m/z (ESI): 219.1 [M+H]⁺.

Step 5: Compound 3-5 was dissolved in tetrahydrofuran (5 mL). The reaction mixture was cooled to -70°C under nitrogen atmosphere, added dropwise with lithium diisopropylamide (2 M in THF, 1.38 mL, 2.77 mmol), kept at -70°C and stirred for 0.5 hours, added with iodine (704 mg, 2.77 mmol) in batches, kept at -70°C and stirred for 1 hour, gradually warmed to 25°C, and reacted for 1 hour. TLC (PE: EA = 10:1) showed that the starting material was completely reacted. The reaction mixture was added with saturated ammonium chloride (20 mL) to quench, and extracted with ethyl acetate (30 mL × 3). The organic phases were combined, washed with saturated brine (30 mL), dried over anhydrous sodium sulfate, and filtered. The filtrate was concentrated under reduced pressure and then purified by silica gel column chromatography (EA: PE = 1:10) to obtain compound 3-6 (610 mg, Y: 70%). LC-MS m/z (ESI): 345.1 [M+H]⁺.

Step 6: Compound 3-6 (610 mg, 1.77 mmol) was dissolved in ethanol (8 mL) and water (8 mL), then sodium hydroxide (708 mg, 17.70 mmol) was added thereto, and the reaction mixture was gradually heated to 100°C and reacted for 16 hours. TLC (PE: EA = 5:1) showed that the starting material was completely reacted. The reaction mixture was added with water (40 mL) to quench, and extracted with ethyl acetate (40 mL × 3). The organic phases were combined, washed with saturated brine (40 mL), dried over anhydrous sodium sulfate, filtered, and the filtrate was concentrated under reduced pressure to obtain compound 3-7 (300 mg, Y: 46%). LC-MS m/z (ESI): 364.1 [M+H]⁺.

Step 7: Compound 3-7 (300 mg, 0.82 mmol) was dissolved in N,N-dimethylformamide (5 mL), then 2-(7-azabenzotriazol-1-yl)-N,N,N',N'-tetramethyluronium hexafluorophosphate (471 mg, 1.24 mmol), methylamine hydrochloride (110 mg, 1.65 mmol), and triethylamine (250 mg, 2.48 mmol) were sequentially added thereto, and the reaction mixture was reacted at 25°C for 2 hours. LCMS showed that the starting material was completely reacted. The reaction mixture was added with water (30 mL), and extracted with ethyl acetate (20 mL × 3). The organic phases were combined, washed with saturated brine (30 mL), dried over anhydrous sodium sulfate, and filtered. The filtrate was concentrated under reduced pressure and then purified by silica gel column chromatography (EA: PE = 1:1) to obtain compound 3-8 (201 mg, Y: 64%). LC-MS m/z (ESI): 376.9 [M+H]⁺.

Step 8: Compound **3-7** (201 mg, 0.53 mmol) was dissolved in toluene (5 mL), then compound 1-**8** (290 mg, 0.53 mmol), potassium phosphate (226 mg, 1.06 mmol), water (1 mL), 4,5-bis(diphenylphosphino)-9,9-dimethylxanthene (61 mg, 0.11 mmol), and tris(dibenzylideneacetone)dipalladium (48 mg, 0.05 mmol) were sequentially added thereto, and the reaction mixture was reacted at 80°C for 16 hours under nitrogen atmosphere. LCMS showed that the starting material was completely reacted. The reaction mixture was cooled to 25°C, concentrated under reduced pressure, and then purified by silica gel column chromatography (EA: PE = 1:2) to obtain compound **3-8** (110 mg, Y: 30%). LC-MS m/z (ESI): 666.1 [M+H]⁺.

Step 9: Compound **3-8** (110 mg, 0.16 mol) was dissolved in dichloromethane (5 mL), then trifluoroacetic acid (1 mL) was added thereto, and the reaction mixture was reacted at 25°C for 0.5 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: Xselect CSH Prep Fluoro-Phenyl, 150 * 19 mm, 5 µm; eluent: 0.05% FA/CH₃CN+MeOH = 1:1; gradient: 38% to 58%; flow rate: 20 mL/min; run time: 11 min; retention time: 8.9 min for **3a** and 7.8 min for **3b**) to obtain compound **3a** (18.0 mg, Y: 11%) and compound **3b** (38.0 mg, Y: 24%). **3a:** ¹H NMR (400 MHz, DMSO-*d*₆): δ 8.18-8.09 (m, 1H), 7.55-7.32 (m, 5H), 7.06 (s, 1H), 7.04 (d, *J* = 6.4 Hz, 1H), 4.49-4.40 (m, 1H), 4.08-3.95 (m, 1H), 3.73-3.62 (m, 1H), 3.51-3.28 (m, 3H), 3.27-2.87 (m, 4H), 2.61 (d, *J* = 4.4 Hz, 3H), 1.97-1.45 (m, 8H). LC-MS m/z (ESI): 556.4 [M+H]⁺. **3b:** ¹H NMR (400 MHz, DMSO-*d*₆): δ 8.14-7.90 (m, 1H), 7.49-7.31 (m, 5H), 7.06-6.92 (m, 2H), 4.48-4.32 (m, 1H), 4.00-3.76 (m, 2H), 3.50-3.40 (m, 4H), 3.09-2.90 (m, 3H), 2.37-2.26 (m, 3H), 2.18-1.45 (m, 8H). LC-MS m/z (ESI): 556.4 [M+H]⁺.

### Example 4

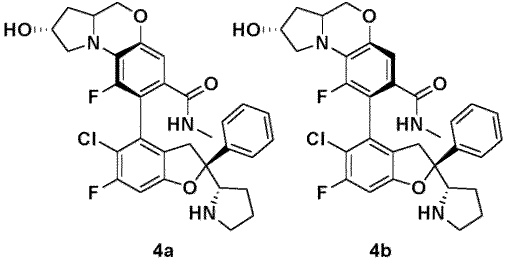

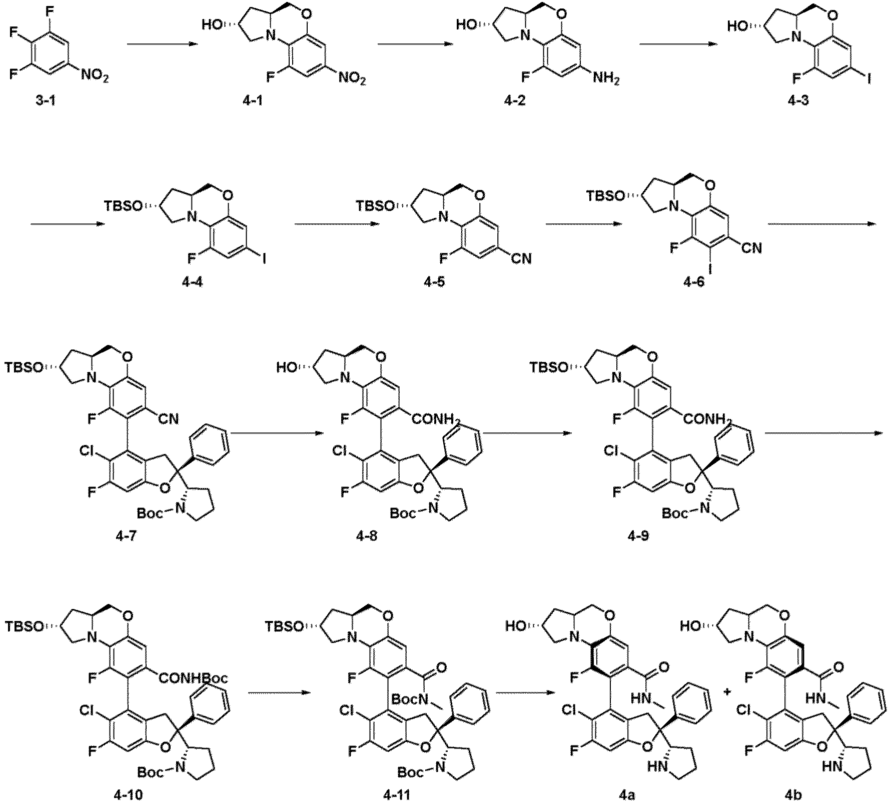

Step 1: Compound **3-1** (40.0 g, 22.6 mmol) was dissolved in dimethyl sulfoxide (320 mL). The reaction mixture was added with starting material (3R,5S)-5-(hydroxymethyl)pyrrolidin-3-ol hydrochloride (34.0 g, 22.6 mmol), then added with potassium hydroxide (50.6 g, 90.4 mmol) in batches under nitrogen atmosphere, reacted at 25°C for 2 hours, and heated to 60°C and reacted for 2 hours. LCMS showed that the starting material was completely reacted. The reaction mixture was cooled to 25°C, filtered through diatomite, and the filter cake was rinsed with ethyl acetate (200 mL). The filtrate was added with 1 M hydrochloric acid to adjust the pH to 5 to 6, and extracted with ethyl acetate (600 mL × 3). The organic phases were combined, washed with saturated brine (300 mL), dried over anhydrous sodium sulfate, and filtered. The filtrate was concentrated under reduced pressure and then purified by silica gel column chromatography (PE: EA= 1:2) to obtain compound **4-1** (13.0 g, Y: 22%). ¹H NMR (400 MHz, DMSO-*d₆*) δ 7.69 (d, *J* = 10.4 Hz, 1H), 7.52 (s, 1H), 5.19 (s, 1H), 4.65-4.59 (m, 1H), 4.39 (s, 1H), 3.84-3.70 (m, 3H), 3.48-3.42 (m, 1H), 2.04-1.95 (m, 1H), 1.62-1.52 (m, 1H).

Step 2: Compound **4-1** (15.2 g, 59.8 mmol) was dissolved in ethanol (150 mL). The reaction mixture was added with a solution of ammonium chloride (16.4 g, 299 mmol) in water (150 mL), heated to 80°C, added with iron powder (16.7 g, 299 mmol) in batches, and reacted at 80°C for 1.5 hours. LCMS showed that the starting material was completely reacted. The reaction mixture was cooled to 25°C, and filtered through diatomite. The filtrate was collected, and extracted with ethyl acetate (100 mL × 3). The organic phases were combined, washed with saturated brine (100 mL), dried over anhydrous sodium sulfate, and filtered. The filtrate was concentrated under reduced pressure and then purified by column chromatography (PE: EA = 2:3) to obtain compound **4-2** (8.7 g, Y: 64%). ¹H NMR (400 MHz, DMSO-*d₆*) δ 6.05-5.95 (m, 1H), 5.91 (s, 1H), 4.92-4.75 (m, 3H), 4.27-4.22 (m, 1H), 4.07-4.01 (m, 1H), 3.50-3.42 (m, 1H), 3.30-3.20 (m, 1H), 2.90-2.80 (m, 1H), 1.98-1.90 (m, 1H), 1.74-1.64 (m, 1H). LC-MS m/z (ESI): 225 [M+H]⁺.

Step 3: Compound **4-2** (8.7 g, 38.8 mmol) was dissolved in acetonitrile (100 mL). The reaction mixture was cooled to 0°C under nitrogen atmosphere, added dropwise with tert-butyl nitrite (4.8 g, 45.6 mmol), reacted at 0°C for 0.5 hours, then sequentially added with cuprous iodide (7.3 g, 38.8 mmol) and potassium iodide (19.3 g, 116.4 mmol), heated to 60°C, and reacted for 12 hours. TLC (PE: EA = 2:1) showed that the starting material was completely reacted. The reaction mixture was cooled to 0°C, poured into saturated sodium bicarbonate (100 mL) to quench, and extracted with ethyl acetate (100 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 3:1) to obtain compound **4-3** (2.2 g, Y: 16%).

Step 4: Compound **4-3** (1.5 g, 4.5 mmol) was dissolved in dichloromethane (20 mL). The reaction mixture was added with imidazole (913 mg, 13.4 mmol) under nitrogen atmosphere, stirred for 0.5 hours, then added with tert-butyldimethylsilyl chloride (1.0 g, 6.7 mmol), and reacted at 25°C for 2 hours. LCMS showed that the starting material was completely reacted. The reaction mixture was diluted with dichloromethane (20 mL), and washed with water (20 mL). The organic phase was dried over anhydrous sodium sulfate, filtered, and the filtrate was concentrated under reduced pressure and then purified by silica gel column chromatography (PE: EA = 1:10) to obtain compound **4-4** (1.1 g, Y: 37%). ¹H NMR (400 MHz, DMSO-*d₆*) δ 7.06 (d, *J =* 13.2 Hz, 1H), 6.98 (s, 1H), 4.54-4.46 (m, 1H), 4.36-4.31 (m, 1H), 3.59-3.50 (m, 2H), 3.37-3.28 (m, 2H), 1.98-1.90 (m, 2H), 1.72-1.68 (m, 2H) 0.86 (s, 9H), 0.07 (s, 6H). LC-MS m/z (ESI): 450 [M+1]⁺.

Step 5: Compound **4-4** (1.1 g, 2.5 mmol) was dissolved in N-methylpyrrolidone (20 mL), then cuprous cyanide (1.8 g, 4.1 mmol) was added thereto under nitrogen atmosphere, and the reaction mixture was heated to 110°C and reacted for 16 hours. LCMS showed that the starting material was completely reacted. The reaction mixture was cooled to 80°C, slowly poured into ethyl acetate (100 mL), stirred for 0.5 hours, filtered through diatomite, and the filtrate was washed with saturated brine (20 mL). The organic phase was dried over anhydrous sodium sulfate, filtered, and the filtrate was concentrated under reduced pressure and then purified by silica gel column chromatography (EA: PE = 5:1) to obtain compound **4-5** (500 mg, Y: 58%). LC-MS m/z (ESI): 349 [M+H]⁺.

Step 6: Compound **4-5** (500 mg, 1.4 mmol) was dissolved in tetrahydrofuran (10 mL). The reaction mixture was cooled to -75°C under nitrogen atmosphere, and added dropwise with lithium diisopropylamide (0.86 mL, 1.72 mol, 2.0 M). After the dropwise addition was completed, the reaction mixture was stirred at -75°C for 0.5 hours, and added dropwise with iodine in tetrahydrofuran (435 mg, 1.72 mmol/2 mL). After the dropwise addition was completed, the reaction mixture was reacted at -75°C for another 0.5 hours. LCMS showed that the starting material was completely reacted. The reaction mixture was poured into saturated ammonium chloride (10 mL) at 0°C to quench, and extracted with ethyl acetate (50 mL × 3). The organic phases were combined, washed with saturated brine (20 mL), dried over anhydrous sodium sulfate, and filtered. The filtrate was concentrated under reduced pressure and then purified by silica gel column chromatography (PE: THF = 1:3) to obtain compound **4-6** (410 mg, Y: 60%). ¹H NMR (400 MHz, DMSO-*d₆*) δ 7.19 (s, 1H), 4.58-4.52 (m, 2H), 3.78-3.64 (m, 3H), 3.45-3.40 (m, 1H), 1.99-1.92 (m, 1H), 1.69-1.59 (m, 1H), 0.87 (s, 9H), 0.09 (s, 6H). LC-MS m/z (ESI): 475 [M+H]⁺.

Step 7: Compound **4-6** (280 mg, 591 µmol) was dissolved in toluene (5 mL) and water (1 mL). The reaction mixture was sequentially added with compound **1-8** (336 mg, 621 µmol), tris(dibenzylideneacetone)dipalladium (108 mg, 118 µmol), 4,5-bis(diphenylphosphino)-9,9-dimethylxanthene (137 mg, 236 µmol), and potassium phosphate (376 mg, 1.77 mmol) under nitrogen atmosphere, heated to 110°C, and reacted for 12 hours. LCMS showed that the starting material was completely reacted. The reaction mixture was cooled to 25°C, poured into saturated ammonium chloride (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:10) to obtain compound **4-7** (270 mg, Y: 39%). LC-MS m/z (ESI): 664 [M+Na]⁺.

Step 8: Compound **4-7** (270 mg, 354 µmol) was dissolved in ethanol (2 mL) and water (1 mL), then sodium hydroxide (65 mg, 2.83 mmol) was added thereto, and the reaction mixture was heated to 120°C and reacted for 16 hours. LCMS showed that the starting material was completely reacted. The reaction mixture was cooled to 0°C, poured into 0.3 M hydrochloric acid (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **4-8** (190 mg, Y: 72%). LC-MS m/z (ESI): 668 [M+H]⁺.

Step 9: Compound **4-8** (190 mg, 284 µmol) was dissolved in dichloromethane (10 mL). The reaction mixture was added with imidazole (58 mg, 854 µmol) under nitrogen atmosphere, reacted at 25°C for 0.5 hours, then added with tert-butyldimethylsilyl chloride (85 mg, 569 µmol), and reacted at 25°C for another 2 hours. LCMS showed that the starting material was completely reacted. The reaction mixture was diluted with dichloromethane (10 mL), washed with water (10 mL × 3), dried over anhydrous sodium sulfate, and filtered. The filtrate was concentrated under reduced pressure and then purified by column chromatography (PE: EA= 1:2) to obtain compound **4-9** (110 mg, Y: 49%). LC-MS m/z (ESI): 782 [M+H]⁺.

Step 10: Compound **4-9** (110 mg, 141 µmol) was dissolved in tetrahydrofuran (2 mL). The reaction mixture was added with sodium hydride (22 mg, 423 µmol) in batches at 0°C, reacted for 0.5 hours, then added with di-tert-butyl dicarbonate (87 mg, 400 µmol), and reacted at 25°C for 2 hours. LCMS showed that the starting material was completely reacted. The reaction mixture was cooled to 0°C, poured into 0.5 M hydrochloric acid (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **4-10** (100 mg, crude). LC-MS m/z (ESI): 882 [M+H]⁺.

Step 11: Compound **4-10** (100 mg, 113 µmol) was dissolved in tetrahydrofuran (2 mL). The reaction mixture was added with sodium hydride (6 mg, 135 µmol) at 0°C, reacted at 0°C for 0.5 hours, then added dropwise with iodomethane (20 mg, 135 µmol), and reacted at 25°C for 0.5 hours. TLC (PE: EA = 2:1) showed that the starting material was completely reacted. The reaction mixture was cooled to 0°C, poured into 0.1 M hydrochloric acid (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:10) to obtain compound **4-11** (54 mg, Y: 42%). LC-MS m/z (ESI): 796 [M+H]⁺.

Step 12: Compound **4-11** (54 mg, 60 µmol) was dissolved in a solution of trifluoroacetic acid in dichloromethane (2 mL/4 mL), and the reaction mixture was reacted at 25°C for 0.5 hours. LCMS showed that the starting material was completely reacted. The reaction mixture was cooled to 0°C, poured into saturated sodium carbonate (5 mL) to quench and to adjust the pH to 8 to 9, and extracted with ethyl acetate (10 mL × 3). The organic phases were combined, washed with saturated brine (10 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O/CH₃CN; gradient: 64% to 84%; flow rate: 20 mL/min; run time: 10 min; retention time: 8.0 min for **4a** and 6.8 min for **4b**) to obtain compound **4a** (3.6 mg, Y: 10%) and compound **4b** (1.8 mg, Y: 5%). **4a:** ¹H NMR (400 MHz, DMSO-*d₆*) δ 7.89 (br, 1H), 7.48-7.45 (m, 2H), 7.42-7.22 (m, 3H), 7.01 (s, 1H), 6.99-6.90 (m, 1H), 5.03 (br, 1H), 4.49-4.41 (m, 1H), 4.34-4.23 (m, 2H), 4.68-4.41 (m, 5H), 2.99-2.88 (m, 1H), 2.85-2.59 (m, 5H), 2.04-1.92 (m, 1H), 1.64-1.26 (m, 4H). LC-MS m/z (ESI): 582 [M+H]⁺. **4b:** ¹H NMR (400 MHz, DMSO-*d₆*) δ 7.95 (br, 1H), 7.46-7.41 (m, 2H), 7.36-7.25 (m, 3H), 6.96 (s, 1H), 6.94-6.88 (m, 1H), 5.12-5.05 (m, 1H), 4.51-4.31 (m, 3H), 3.74-3.38 (m, 5H), 3.11-3.02 (m, 1H), 2.78-2.55 (m, 2H), 2.28 (d, *J* = 4.4 Hz, 3H), 2.05-1.95 (m, 1H), 1.70-1.62 (m, 1H), 1.52-1.40 (m, 3H). LC-MS m/z (ESI): 582 [M+H]⁺.

### Example 5

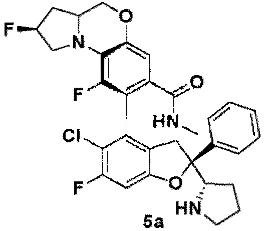

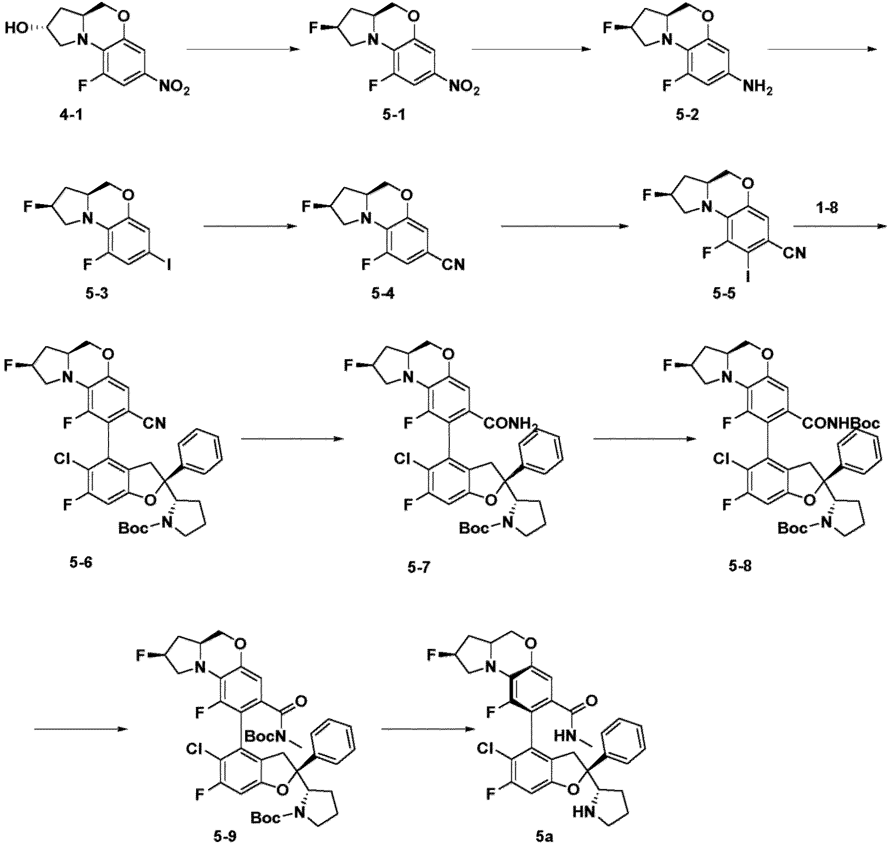

Step 1: Compound **4-1** (4.2 g, 16.5 mmol) was dissolved in dichloromethane (40 mL), then diethylaminosulfur trifluoride (5.3 g, 33.1 mmol) was added dropwise thereto at 25°C, and the reaction mixture was reacted at 25°C for 2 hours. LCMS monitored that the reaction was completed. The reaction mixture was added with saturated sodium bicarbonate (50 mL) to quench, and extracted with ethyl acetate (20 mL × 3). The organic phases were combined, washed with saturated brine (30 mL), dried over anhydrous sodium sulfate, and filtered. The filtrate was concentrated under reduced pressure and then purified by silica gel column chromatography (PE: THF = 4:1) to obtain compound **5-1** (2.1 g, Y: 50%). LC-MS m/z (ESI): 257 [M+H]⁺.

Step 2: Compound **5-1** (2.1 g, 8.2 mmol), ammonium chloride (2.2 g, 41.0 mmol), and iron powder (2.3 g, 41.0 mmol) were mixed with ethanol (20 mL) and water (20 mL), and the reaction mixture was reacted at 80°C for 2 hours. LCMS monitored that the reaction was completed. The reaction mixture was filtered, and the filtrate was concentrated under reduced pressure and extracted with ethyl acetate (10 mL × 3). The organic phases were combined, washed with saturated brine (30 mL), dried over anhydrous sodium sulfate, and filtered. The filtrate was concentrated under reduced pressure and then purified by silica gel column chromatography (PE: THF = 3:2) to obtain compound **5-2** (1.2 g, Y: 66%). LC-MS m/z (ESI): 227 [M+H]⁺.

Step 3: Compound **5-2** (3.5 g, 14.16 mmol) was dissolved in acetonitrile (40 mL). The reaction mixture was added dropwise with tert-butyl nitrite (4.4 g, 42.48 mmol) at 0°C, then added with potassium iodide (7.0 g, 42.48 mmol) and cuprous iodide (2.7 g, 14.16 mmol), and reacted at 60°C for 2 hours. LCMS monitored that the reaction was completed. The reaction mixture was added with water (20 mL), and extracted with ethyl acetate (20 mL × 3). The organic phases were combined, washed with saturated brine (30 mL), dried over anhydrous sodium sulfate, and filtered. The filtrate was concentrated under reduced pressure and then purified by silica gel column chromatography (PE: EA = 19:1) to obtain compound **5-3** (2.0 g, Y: 38%). LC-MS m/z (ESI): 338 [M+H]⁺.

Step 4: Compound **5-3** (2.0 g, 5.93 mmol) was dissolved in N-methylpyrrolidone (20 mL), then cuprous hydride (4.4 g 47.46 mmol) was added thereto, and the reaction mixture was heated to 100°C and reacted for 12 hours. LCMS monitored that the reaction was completed. The reaction mixture was cooled to 40°C and filtered. The filtrate was added with water (20 mL) and extracted with ethyl acetate (15 mL × 3). The organic phases were combined, washed with saturated brine (15 mL × 3), dried over anhydrous sodium sulfate, and filtered. The filtrate was concentrated under reduced pressure and then purified by silica gel column chromatography (PE: EA = 9:1) to obtain compound **5-4** (0.9 g, Y: 64%). LC-MS m/z (ESI): 237 [M+H]⁺.

Step 5: Compound **5-4** (450 mg, 1.91 mmol) was dissolved in anhydrous tetrahydrofuran (10 mL). The reaction mixture was added dropwise with lithium diisopropylamide (244 mg, 2.29 mmol) at -70°C, reacted at -70°C for 0.5 hours, added dropwise with a solution of iodine (581 mg, 2.29 mmol) in tetrahydrofuran, reacted at -70°C for 0.5 hours, then naturally warmed to room temperature, and reacted at room temperature for 2 hours. LCMS monitored that the reaction was completed. The reaction mixture was cooled to 0°C, added with saturated ammonium chloride solution (5 mL), then added with saturated sodium sulfite solution (5 mL) to quench, and extracted with ethyl acetate (10 mL × 3). The organic phases were combined, washed with saturated brine (10 mL), dried over anhydrous sodium sulfate, and filtered. The filtrate was concentrated under reduced pressure and then purified by reversed-phase liquid chromatography to obtain compound **5-5** (550 mg, Y: 79%). ¹H NMR (400 MHz, CDCl₃): δ 7.00 (s, 1H), 5.36-5.21 (m, 1H), 4.34-4.28 (m, 1H), 4.08-3.81 (m, 2H), 3.65-3.58 (m, 2H), 2.57-2.35 (m, 1H), 2.20-2.11 (m, 1H). LC-MS m/z (ESI): 363 [M+H]⁺.

Step 6: Compound **5-5** (214 mg, 0.59 mmol), compound **1-8** (337 mg, 0.62 mmol), tris(dibenzylideneacetone)dipalladium (54 mg, 0.06 mmol), 4,5-bis(diphenylphosphino)-9,9-dimethylxanthene (34 mg, 0.06 mmol), and potassium phosphate (377 mg, 1.78 mmol) were dissolved in toluene (5 mL) and water (1 mL), and the reaction mixture was reacted at 100°C for 24 hours under nitrogen atmosphere. LCMS monitored that the reaction was completed. The reaction mixture was added with water (5 mL), and extracted with ethyl acetate (5 mL × 3). The organic phases were combined, washed with saturated brine (10 mL × 3), dried over anhydrous sodium sulfate, and filtered. The filtrate was concentrated under reduced pressure and then purified by silica gel column chromatography (PE: EA = 17:3) to obtain compound **5-6** (110 mg, Y: 28%). LC-MS m/z (ESI): 552 [M+H-Boc]⁺.

Step 7: Compound **5-6** (110 mg, 0.17 mmol) and sodium hydroxide (121 mg, 3 mmol) were dissolved in ethanol (2 mL) and water (1 mL), and the reaction mixture was reacted at 120°C for 24 hours. LCMS monitored that the reaction was completed. The reaction mixture was added with water (5 mL), and extracted with ethyl acetate (10 mL × 3). The organic phases were combined, washed with saturated brine (10 mL), dried over anhydrous sodium sulfate, filtered, and the filtrate was concentrated under reduced pressure to obtain compound **5-7** (95 mg, crude). LC-MS m/z (ESI): 670 [M+H]⁺.

Step 8: Compound **5-7** (95 mg, 0.14 mmol), sodium hydride (17 mg, 0.71 mmol), and di-tert-butyl dicarbonate (124 mg, 0.57 mmol) were mixed, and the reaction mixture was reacted at room temperature for 48 hours. LCMS monitored that the reaction was completed. The reaction mixture was added dropwise with water (5 mL) to quench, and extracted with ethyl acetate (5 mL × 3). The organic phases were combined, washed with saturated brine (5 mL), dried over anhydrous sodium sulfate, filtered, and the filtrate was concentrated under reduced pressure to obtain compound **5-8** (95 mg, crude). LC-MS m/z (ESI): 770 [M+H]⁺.

Step 9: Compound **5-8** (95 mg, 0.14 mmol), sodium hydride (17 mg, 0.71 mmol), and iodomethane (80 mg, 0.57 mmol) were mixed, and the reaction mixture was reacted at room temperature for 24 hours. LCMS monitored that the reaction was completed. The reaction mixture was added dropwise with water (5 mL) to quench, and extracted with ethyl acetate (5 mL × 3). The organic phases were combined, washed with saturated brine (5 mL), dried over anhydrous sodium sulfate, and filtered. The filtrate was concentrated under reduced pressure and then purified by silica gel column chromatography (PE: EA = 4:1) to obtain compound **5-9** (50 mg, 45%). LC-MS m/z (ESI): 784 [M+H]⁺.

Step 10: Compound **5-9** (48 mg, 0.061 mmol) was dissolved in dichloromethane (5 mL) and trifluoroacetic acid (5 mL), and the reaction mixture was reacted at room temperature for 1 hour. LCMS monitored that the reaction was completed. The reaction mixture was added with water (5 mL), then added with saturated sodium bicarbonate to adjust the pH to 8, and extracted with ethyl acetate (5 mL × 3). The organic phases were combined, washed with saturated brine (10 mL), dried over anhydrous sodium sulfate, and filtered. The filtrate was concentrated under reduced pressure, then purified by preparative high-pressure liquid chromatography (column model: Xselect CSH Prep Fluoro-Phenyl, 150 * 19 mm, 5 µm; eluent: 0.05% FA/CH₃CN; gradient: 40% to 60%; flow rate: 20 mL/min; run time: 12 min; retention time: 7.8 min), and lyophilized to obtain compound **5a** (7.4 mg, 20%). ¹H NMR (400 MHz, DMSO-*d₆*) δ 88.25 (s, 1H), 7.96-7.92 (m, 1H), 7.49-7.43 (m, 2H), 7.38-7.24 (m, 3H), 7.02 (s, 1H), 6.96 (d, *J =* 9.6 Hz, 1H), 5.41-5.24 (m, 1H), 4.48-4.38 (m, 1H), 3.93-3.77 (m, 1H), 3.62-3.38 (m, 5H), 3.02-2.96 (m, 1H), 2.77-2.58 (m, 5H), 2.45-2.39 (m, 1H), 2.15-2.03 (m, 1H), 1.58-1.33 (m, 4H). LC-MS m/z (ESI): 584 [M+H]⁺.

### Example 6

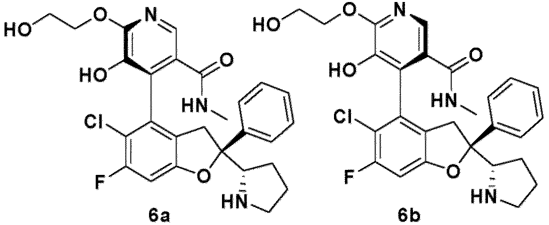

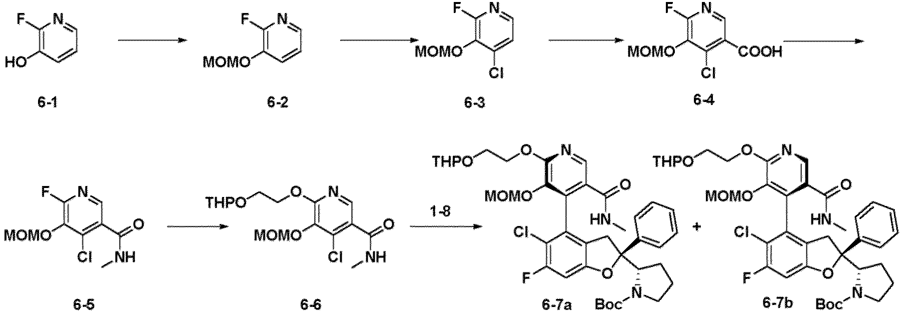

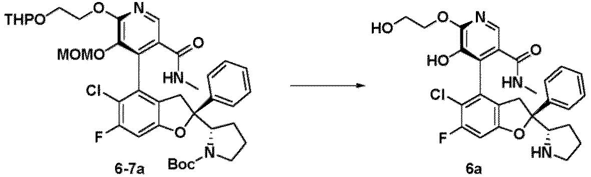

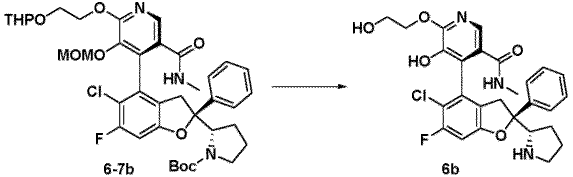

Step 1: Compound **6-1** (12.5 g, 110.6 mmol) was dissolved in N,N-dimethylformamide (250 mL). The reaction mixture was cooled to 0°C under nitrogen atmosphere, added with sodium hydride (5.2 g, 331.8 mmol) in batches, stirred for 20 minutes, added dropwise with bromomethyl methyl ether (13.43 g, 452 mmol), and reacted at 0°C for 2 hours. TLC (PE: EA = 5:1) showed that the starting material was completely reacted. The reaction mixture was poured into saturated ammonium chloride aqueous solution (20 mL) to quench, and extracted with ethyl acetate (400 mL × 3). The organic phases were combined, washed with saturated brine (300 mL), dried over anhydrous sodium sulfate, filtered, and the filtrate was concentrated under reduced pressure to obtain compound **6-2** (14.0 g, crude). ¹H NMR (400 MHz, DMSO-*d₆*) δ 7.83-7.77 (m, 1H), 7.56-7.50 (m, 1H), 7.12-7.07 (m, 1H), 5.22 (s, 2H), 3.51 (s, 3H).

Step 2: Compound **6-2** (9.6 g, 61.1 mmol) was dissolved in tetrahydrofuran (90 mL). The reaction mixture was cooled to -75°C under nitrogen atmosphere, and added dropwise with n-butyllithium solution (26.9 mL, 67.2 mmol, 2.5 mol/L). After the dropwise addition was completed, the reaction mixture was stirred at -75°C for 40 minutes, then added dropwise with hexachloroethane (28.95 g, 122.2 mmol), and reacted at -75°C for 3 hours. TLC (PE: EA= 5:1) showed that the starting material was completely reacted. The reaction mixture was added dropwise with saturated ammonium chloride aqueous solution (10 mL) to quench, and extracted with ethyl acetate (300 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:5) to obtain compound 6-3 (5.0 g, Y: 49%). ¹H NMR (400 MHz, DMSO-*d₆*) δ 7.87-7.81 (m, 1H), 7.23 (d, J= 4.0 Hz, 1H), 5.22 (s, 2H), 3.62 (s, 3H).

Step 3: Compound **6-3** (9.0 g, 47.1 mmol) was dissolved in tetrahydrofuran (90.0 mL). The reaction mixture was cooled to -75°C under nitrogen atmosphere, added dropwise with n-butyllithium solution (26.9 mL, 51.8 mmol, 2.5 M), stirred at for 40 minutes, and then introduced with carbon dioxide gas. TLC (PE: EA = 7:1) showed that the starting material was completely reacted. The reaction mixture was added dropwise with saturated ammonium chloride aqueous solution (20 mL) at -75°C to quench, then added with 1 N hydrochloric acid to adjust the pH to 5 to 6, and extracted with ethyl acetate (200 mL × 3). The organic phases were combined, washed with saturated brine (150 mL), dried over anhydrous sodium sulfate, and filtered. The filtrate was concentrated under reduced pressure and then subjected to silica gel column chromatography (EA: PE = 1:6) to obtain compound **6-4** (3.0 g, crude).

Step 4: Compound **6-4** (2.4 g, 19.15 mmol) was dissolved in N,N-dimethylformamide (30 mL). The reaction mixture was added with diisopropylethylamine (7.4 g, 57.45 mmol) and methylamine hydrochloride (1.93 g, 28.72 mmol), then added with 2-(7-azabenzotriazol-1-yl)-N,N,N',N'-tetramethyluronium hexafluorophosphate (8.0 g, 21.06 mmol) in batches in an ice bath, and reacted for 2 hours. TLC (MeOH: DCM = 1:10) showed that the starting material was completely reacted. The reaction mixture was added with water to quench, then added with ethyl acetate (100 mL), and washed with water (80 mL × 2). The organic phases were combined, washed with saturated brine (150 mL), and filtered. The filtrate was concentrated under reduced pressure and then subjected to silica gel column chromatography (EA: PE = 1:1) to obtain compound **6-5** (2.4 g, Y: 51%). LC-MS m/z (ESI): 413, 415 [M+H]⁺.

Step 5: 2-(Tetrahydro-2H-pyran-2-yloxy)ethanol (1.55 g, 10.64 mmol) was dissolved in tetrahydrofuran (30.0 mL). The reaction mixture was added with sodium hydride (425 mg, 10.64 mmol) in batches at 0°C, stirred for 20 minutes, then added dropwise with compound **6-5** (2.4 g, 9.67 mmol) dissolved in tetrahydrofuran at 0°C, and reacted for 3 hours. TLC (PE: EA = 1:1) showed that the starting material was completely reacted. The reaction mixture was added dropwise with water (10 mL) at 0°C to quench, and extracted with ethyl acetate (60 mL × 3). The organic phases were combined, washed with saturated brine (20 mL), dried over anhydrous sodium sulfate, and filtered. The filtrate was concentrated under reduced pressure and then purified by reversed-phase chromatography to obtain compound **6-6** (1.0 g, Y: 27%).

Step 6: Compound **1-8** (500 mg, 0.92 mmol) and compound **6-6** (344 mg, 0.92 mmol) were dissolved in toluene (8 mL) and water (0.8 mL). The reaction mixture was sequentially added with tris(dibenzylideneacetone)dipalladium (95.3 mg, 0.092 mmol), 4,5-bis(diphenylphosphino)-9,9-dimethylxanthene (106 mg, 0.184 mmol), and potassium carbonate (318 mg, 2.3 mmol) under nitrogen atmosphere, heated to 110°C, and reacted for 16 hours. LCMS showed that the starting material was completely reacted. The reaction mixture was cooled to room temperature, concentrated under reduced pressure, and then purified by a preparative silica gel plate (EA: PE = 1:1) to obtain compound **6-7a** (40 mg, Y: 5%, Rf = 0.3) and compound **6-7b** (50 mg, Y: 7%, Rf = 0.35). LC-MS m/z (ESI): 756.3 [M+H]⁺.

Step 7: Compound **6-7a** (40 mg, 0.05 mmol) was dissolved in a solution of 20% trifluoroacetic acid/dichloromethane (2 mL), and the reaction mixture was reacted at 5°C for 1 hour. TLC (DCM: MeOH = 10:1) showed that the starting material was completely reacted. The reaction mixture was cooled to 0°C, poured into saturated sodium carbonate (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O+10 mmol/L NH₄HCO₃/CH₃CN; gradient: 43% to 63%; flow rate: 20 mL/min; run time: 11 min; retention time: 7.9 min) to obtain compound **6a** (12.1 mg, Y: 43%). ¹H NMR (400 MHz, DMSO-*d₆*) δ 7.80-7.50 (m, 1H), 7.46-7.18 (m, 6H), 6.88-6.76 (m, 1H), 4.48-4.12 (m, 3H), 3.85-3.60 (m, 2H), 3.51-3.43 (m, 1H), 3.33-3.25 (m, 1H), 3.10-2.88 (m, 1H), 2.75-2.55 (m, 3H), 2.48-2.40 (m, 2H), 1.99 (s, 1H), 1.77-1.63 (m, 1H), 1.43-1.37 (m, 2H), 1.25-1.15 (m, 1H). LC-MS m/z (ESI): 528.2/530.3 [M+H]⁺.

Step 8: Compound **6-7b** (50 mg, 0.07 mmol) was dissolved in a solution of 20% trifluoroacetic acid/dichloromethane (2 mL), and the reaction mixture was reacted at 5°C for 1 hour. TLC (DCM: MeOH = 10:1) showed that the starting material was completely reacted. The reaction mixture was cooled to 0°C, poured into saturated sodium carbonate (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O+10 mmol/L NH₄HCO₃/CH₃CN; gradient: 43% to 63%; flow rate: 20 mL/min; run time: 10 min; retention time: 7.8 min) to obtain compound 6b (17.8 mg, Y: 51%). ¹H NMR (400 MHz, DMSO-*d₆*) δ 8.04-7.98 (m, 1H), 7.59-7.23 (m, 6H), 6.86 (d, *J* = 10.4 Hz, 1H), 4.35-4.28 (m, 2H), 3.75 (s, 2H), 3.51-3.45 (m, 2H), 3.04-2.98 (m, 1H), 2.75-2.66 (m, 1H), 2.31 (d, *J* = 4.4 Hz, 3H), 2.05 (s, 1H), 1.52-1.14 (m, 4H). LC-MS m/z (ESI): 528.2/530.3 [M+H]⁺.

### Example 7

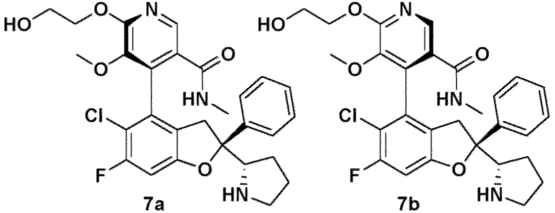

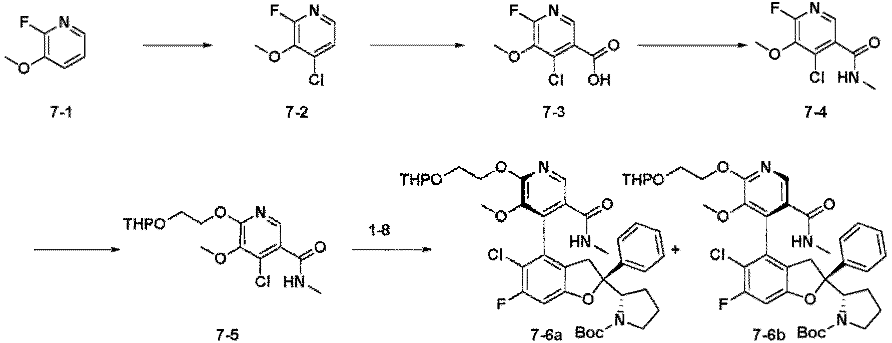

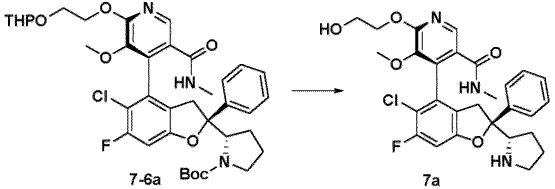

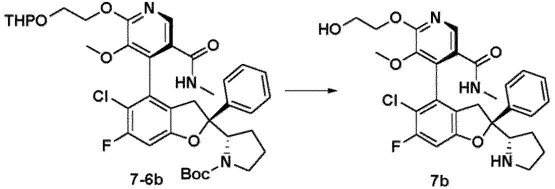

Step 1: Compound **7-1** (25 g, 196.67 mmol) was dissolved in tetrahydrofuran (250 mL). The reaction mixture was cooled to -75°C under nitrogen atmosphere, and added dropwise with n-butyllithium (86.61 mL, 216.34 mmol, 2.5 M). After the dropwise addition was completed, the reaction mixture was reacted for 0.5 hours, then added with hexachloroethane (69.90 g, 295.01 mmol) in batches at -75°C, and reacted at -75°C for 4 hours. TLC (PE: EA= 5:1) showed that the starting material was completely reacted. The reaction mixture was poured into saturated ammonium chloride solution (50 mL) to quench, and extracted with ethyl acetate (50 mL × 3). The organic phases were combined, washed with saturated brine (100 mL), dried over anhydrous sodium sulfate, and filtered. The filtrate was concentrated under reduced pressure and then purified by silica gel column chromatography (EA: PE = 1:5) to obtain compound **7-2** (7.65 g, Y: 24%). LC-MS m/z (ESI): 162.10 [M+H]⁺.

Step 2: Compound **7-2** (7.65 g, 47.35 mmol) was dissolved in tetrahydrofuran (80 mL). The reaction mixture was cooled to -75°C under nitrogen atmosphere, added dropwise with n-butyllithium (21.71 mL, 52.09 mmol), and reacted for 40 minutes. Carbon dioxide was introduced thereto at -75°C, and the reaction mixture was reacted at -75°C for 2 hours. TLC (PE: EA = 5:1) showed that the starting material was completely reacted. The reaction mixture was poured into saturated ammonium chloride aqueous solution (20 mL) at 0°C to quench, and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:5) to obtain compound **7-3** (3.90 g, Y: 40%). ¹H NMR (400 MHz, DMSO-*d₆*) δ 8.38 (s, 1H), 3.95 (s, 3H). LC-MS m/z (ESI): 206.1, 208.1 [M+H]⁺.

Step 3: Compound **7-3** (1.35 g, 6.57 mmol) was dissolved in N,N-dimethylformamide (15.0 mL). The reaction mixture was cooled to 0 to 5°C, added with 2-(7-azabenzotriazol-1-yl)-N,N,N',N'-tetramethyluronium hexafluorophosphate (2.5 g, 6.57 mmol) and diisopropylethylamine (2.55 g, 19.70 mmol), reacted at 0 to 5°C for 5 minutes, then added with methylamine hydrochloride (0.89 g, 13.13 mmol), and reacted at room temperature for 1 hour. TLC (PE: EA = 1:1) showed that the starting material was completely reacted. The reaction mixture was poured into water (10 mL) at 0°C to quench, and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:1) to obtain compound **7-4** (1.00 g, Y: 69%).

Step 4: 2-(Tetrahydro-2H-pyran-2-yloxy)ethanol (1.55 g, 10.64 mmol) was dissolved in tetrahydrofuran (30.0 mL). The reaction mixture was added with sodium hydride (425 mg, 10.64 mmol) in batches at 0°C, stirred for 20 minutes, then added dropwise with compound **7-4** (2.4 g, 9.67 mmol) dissolved in tetrahydrofuran at 0°C, and reacted at room temperature for 1 hour. TLC (PE: EA = 1:1) showed that the starting material was completely reacted. The reaction mixture was added with ice water (10 mL) at 0°C to quench, and extracted with ethyl acetate (60 mL × 3). The organic phases were combined, washed with saturated brine (20 mL), dried over anhydrous sodium sulfate, and filtered. The filtrate was concentrated under reduced pressure and then purified by reversed-phase chromatography to obtain compound **7-5** (1.0 g, Y: 27%).

Step 5: Compound **1-8** (500 mg, 0.92 mmol) and compound **7-5** (317 mg, 0.92 mmol) were dissolved in toluene (8 mL) and water (0.8 mL). The reaction mixture was sequentially added with tris(dibenzylideneacetone)dipalladium (95.3 mg, 0.092 mmol), 4,5-bis(diphenylphosphino)-9,9-dimethylxanthene (106 mg, 0.184 mmol), and potassium carbonate (318 mg, 2.3 mmol) under nitrogen atmosphere, heated to 100°C, and reacted for 16 hours. LCMS showed that the starting material was completely reacted. The reaction mixture was cooled to room temperature, concentrated under reduced pressure, and then purified by a preparative silica gel plate (EA: PE = 1:1) to obtain compound **7-6a** (23 mg, Y: 3%, Rf = 0.3) and compound **7-6b** (32 mg, Y: 4%, Rf = 0.35). LC-MS m/z (ESI): 726.2 [M+H]⁺.

Step 6: Compound **7-6a** (23 mg, 0.032 mmol) was dissolved in a solution of 20% trifluoroacetic acid/dichloromethane (2 mL), and the reaction mixture was reacted at 5°C for 1 hour. TLC (DCM: MeOH = 10:1) showed that the starting material was completely reacted. The reaction mixture was cooled to 0°C, poured into saturated sodium carbonate (15 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oily crude product, which was purified by a preparative silica gel plate (DCM: MeOH = 5:1), and lyophilized to obtain compound **7a** (9.4 mg, Y: 54%). ¹H NMR (400 MHz, DMSO-*d₆*) δ 8.23 (br, 1H), 8.15 (s, 1H), 7.49-7.42 (m, 2H), 7.38-7.25 (m, 3H), 7.02 (d, J= 9.6 Hz, 1H), 4.87 (t, J= 5.2 Hz, 1H), 4.43-4.33 (m, 2H), 3.78-3.73 (m, 2H), 3.68-3.61 (m, 1H), 3.45-3.35 (m, 4H), 3.29 (s, 1H), 2.90-2.73 (m, 2H), 2.63-2.52 (m, 3H), 1.70-1.42 (m, 4H). LC-MS m/z (ESI): 542.3/544.3 [M+H]⁺.

Step 7: Compound **7-6b** (32 mg, 0.044 mmol) was dissolved in a solution of 20% trifluoroacetic acid/dichloromethane (2 mL), and the reaction mixture was reacted at 5°C for 1 hour. TLC (DCM: MeOH = 10:1) showed that the starting material was completely reacted. The reaction mixture was cooled to 0°C, poured into saturated sodium carbonate (15 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was purified by a preparative silica gel plate (DCM: MeOH = 5:1), and lyophilized to obtain compound **7b** (12.3 mg, Y: 51%). ¹H NMR (400 MHz, DMSO-*d₆*) δ 8.23 (br, 1H), 8.10 (d, *J* = 3.6 Hz, 1H), 7.47-7.23 (m, 5H), 6.96 (d, *J* = 10.4 Hz, 1H), 4.90 (t, *J* = 3.2 Hz, 1H), 4.45-4.38 (m, 2H), 3.85-3.72 (m, 2H), 3.68 (s, 3H), 3.65-3.60 (m, 1H), 3.31-3.26 (m, 1H), 3.08 (d, *J* = 10.4 Hz, 1H), 2.80-2.60 (m, 3H), 2.33 (s, 3H), 1.60-1.30 (m, 4H). LC-MS m/z (ESI): 542.3/544.3 [M+H]⁺.

### Example 8

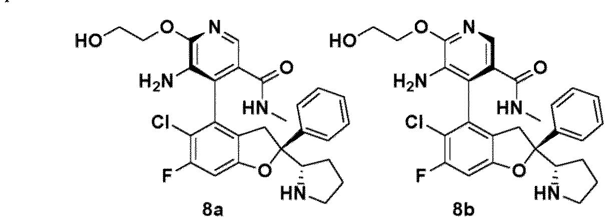

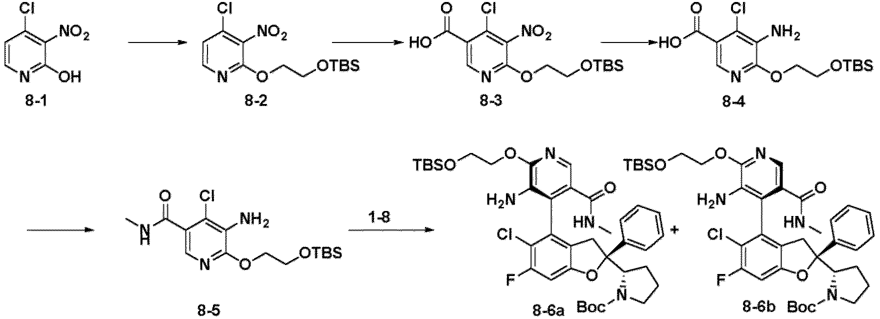

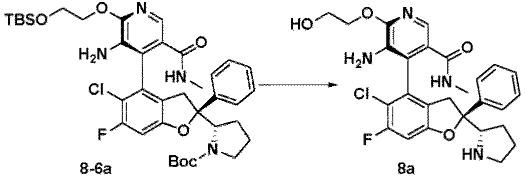

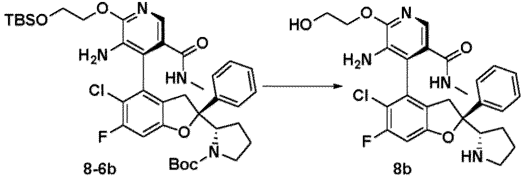

Step 1: Compound **8-1** (10.0 g, 69.2 mmol), tert-butyl-(2-iodoethoxy)dimethylsilane (21.8 g, 76.1 mmol), and silver carbonate (21.0 g, 76.1 mmol) were dissolved in toluene (150 mL), and the reaction mixture was reacted at 100°C for 12 hours under nitrogen atmosphere. LCMS monitored that the starting material was completely reacted. The reaction mixture was cooled to 25°C, filtered through diatomite, and the filter cake was rinsed with ethyl acetate (30 mL). The filtrate was dried over anhydrous sodium sulfate, filtered, concentrated under reduced pressure, and then purified by silica gel column chromatography (PE: EA = 19:1) to obtain compound **8-2** (11.0 g, Y: 52%). ¹H NMR (400 MHz, DMSO-*d₆*) δ 8.37 (d, *J* = 5.2 Hz, 1H), 7.47 (d, *J* = 6.0 Hz, 1H), 4.50 (t, J= 4.8 Hz, 2H), 3.88 (t, *J* = 4.8 Hz, 2H), 0.81 (s, 9H), -0.01 (s, 6H).

Step 2: Compound **8-2** (10.0 g, 30.0 mmol) was dissolved in anhydrous tetrahydrofuran (100 mL). The reaction mixture was cooled to -70°C under nitrogen atmosphere, and added dropwise with lithium diisopropylamide (2 M, 18 mL) at -70°C. After the dropwise addition was completed, the reaction mixture was reacted at -70°C for 0.5 hours, then added with dry ice, reacted at -70°C for 0.5 hours, naturally warmed to room temperature, and reacted at room temperature for 2 hours. LCMS monitored that the starting material was completely reacted. The reaction mixture was cooled to 0°C, added with saturated ammonium chloride solution (50 mL) to quench, and extracted with ethyl acetate (50 mL × 3). The organic phases were combined, washed with saturated brine (100 mL), dried over anhydrous sodium sulfate, filtered, and the filtrate was concentrated under reduced pressure to obtain compound **8-3** (3.8 g, crude). LC-MS m/z (ESI): 377 [M+H]⁺.

Step 3: Compound **8-3** (3.8 g, 10.1 mmol), ammonium chloride (2.7 g, 50.4 mmol), iron powder (2.8 g, 50.4 mmol), ethanol (20 mL), and water (20 mL) were mixed, and the reaction mixture was reacted at 60°C for 2 hours. LCMS monitored that the starting material was completely reacted. The reaction mixture was filtered, and the filtrate was concentrated under reduced pressure and extracted with ethyl acetate (20 mL × 3). The organic phases were combined, washed with saturated brine (30 mL), dried over anhydrous sodium sulfate, and filtered. The filtrate was concentrated under reduced pressure and then purified by preparative reversed-phase chromatography to obtain compound **8-4** (900 mg, Y: 25%). LC-MS m/z (ESI): 347 [M+H]⁺.

Step 4: Compound **8-4** (400 mg, 1.2 mmol), 2-(7-azabenzotriazol-1-yl)-N,N,N',N'-tetramethyluronium hexafluorophosphate (876 mg, 2.3 mmol), diisopropylethylamine (1.2 g, 9.2 mmol), and dichloromethane (10 mL) were mixed, then methylamine hydrochloride (196 mg, 2.9 mmol) was added thereto, and the reaction mixture was reacted at 25°C for 1 hour. LCMS monitored that the starting material was completely reacted. The reaction mixture was added with pure water (10 mL), and extracted with ethyl acetate (10 mL × 3). The organic phases were combined, washed with saturated brine (10 mL), dried over anhydrous sodium sulfate, and filtered. The filtrate was concentrated under reduced pressure and then purified by silica gel column chromatography (PE: EA = 1:1) to obtain compound **8-5** (250 mg, 60%). ¹H NMR (400 MHz, DMSO-*d₆*) δ 8.25 (br, 1H), 7.41 (s, 1H), 5.20 (br, 1H), 4.37 (t, *J* = 4.8 Hz, 2H), 3.94 (t, *J* = 4.8 Hz, 2H), 2.72 (d, *J* = 4.4 Hz, 3H), 0.86 (s, 9H), 0.05 (s, 6H).

Step 5: Compound **8-5** (460 mg, 1.3 mmol), compound **1-8** (730 mg, 1.3 mmol), tris(dibenzylideneacetone)dipalladium (234 mg, 0.26 mmol), 4,5-bis(diphenylphosphino)-9,9-dimethylxanthene (296 mg, 0.51 mmol), and potassium carbonate (529 mg, 3.8 mmol) were dissolved in toluene (5 mL) and water (1 mL), and the reaction mixture was reacted at 120°C for 12 hours under nitrogen atmosphere. LCMS monitored that the starting material was completely reacted. The reaction mixture was added with pure water (5 mL), and extracted with ethyl acetate (5 mL × 3). The organic phases were combined, washed with saturated brine (10 mL), dried over anhydrous sodium sulfate, and filtered. The filtrate was concentrated under reduced pressure and then purified by silica gel column chromatography (PE: EA= 1:1) to obtain compound **8-6a** (100 mg, Y: 10%, Rf = 0.4) and compound **8-6b** (110 mg, Y: 11%, Rf = 0.5). LC-MS m/z (ESI): 741 [M+H]⁺.

Step 6: Compound **8-6a** (100 mg, 0.13 mmol) was dissolved in dichloromethane (5 mL), then trifluoroacetic acid (1 mL) was added dropwise thereto at 0°C, and the reaction mixture was reacted at 25°C for 1 hour. LCMS monitored that the starting material was completely reacted. The reaction mixture was added with water (5 mL), then added with saturated sodium bicarbonate to adjust the pH to 8, and extracted with ethyl acetate (5 mL × 3). The organic phases were combined, washed with saturated brine (10 mL), dried over anhydrous sodium sulfate, and filtered. The filtrate was concentrated under reduced pressure and then purified by preparative high-performance liquid chromatography (column model: Xbridge Prep Phenyl OBD, 150 * 19 mm, 5 µm; eluent: 0.05% FA/CH₃CN; gradient: 39% to 59%; flow rate: 20 mL/min; run time: 11 min; retention time: 8.3 min) to obtain compound **8a** (8.1 mg, Y: 6%). ¹H NMR (400 MHz, DMSO-*d₆*) δ 8.23 (br, 1H), 7.93 (d, *J* = 4.8 Hz, 1H), 7.61 (s, 1H), 7.50-7.40 (m, 2H), 7.36-7.21 (m, 3H), 6.97 (d, *J=* 10.0 Hz, 1H), 4.72 (br, 2H), 4.33-4.21 (m, 2H), 3.71-3.66 (m, 2H), 3.55-3.45 (m, 2H), 2.95 (d, *J* = 16.0 Hz, 1H), 2.78-2.58 (m, 5H), 1.58-1.31 (m, 4H). LC-MS m/z (ESI): 527 [M+H]⁺.

Step 7: Compound **8-6b** (110 mg, 0.15 mmol) was dissolved in dichloromethane (5 mL), then trifluoroacetic acid (1 mL) was added dropwise thereto at 0°C, and the reaction mixture was reacted at 25°C for 1 hour. LCMS monitored that the starting material was completely reacted. The reaction mixture was added with water (5 mL), then added with saturated sodium bicarbonate to adjust the pH to 8, and extracted with ethyl acetate (5 mL × 3). The organic phases were combined, washed with saturated brine (10 mL), dried over anhydrous sodium sulfate, and filtered. The filtrate was concentrated under reduced pressure and then purified by preparative high-performance liquid chromatography (column model: Xbridge Prep Phenyl OBD, 150 * 19 mm, 5 µm; eluent: 0.05% NH₃·H₂O+10 mmol/L NH₄HCO₃/CH₃CN+MeOH = 1:1; gradient: 12% to 32%; flow rate: 20 mL/min; run time: 11 min; retention time: 7.9 min) to obtain compound **8b** (18.0 mg, Y: 12%). ¹H NMR (400 MHz, DMSO-*d₆*) δ 8.11-8.01 (m, 1H), 7.57 (s, 1H), 7.48-7.42 (m, 2H), 7.38-7.23 (m, 3H), 6.93 (d, *J* = 10.0 Hz, 1H), 4.99 (t, *J* = 6.4 Hz, 1H), 4.87 (br, 2H), 4.35-4.25 (m, 2H), 3.82-3.73 (m, 2H), 3.60-3.49 (m, 1H), 3.08-3.02 (m, 1H), 2.78-2.63 (m, 3H), 2.36 (d, *J* = 4.4 Hz, 3H), 1.52-1.32 (m, 4H). LC-MS m/z (ESI): 527 [M+H]⁺.

### Example 9

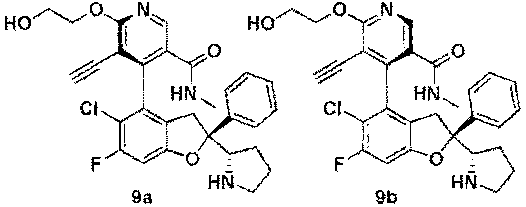

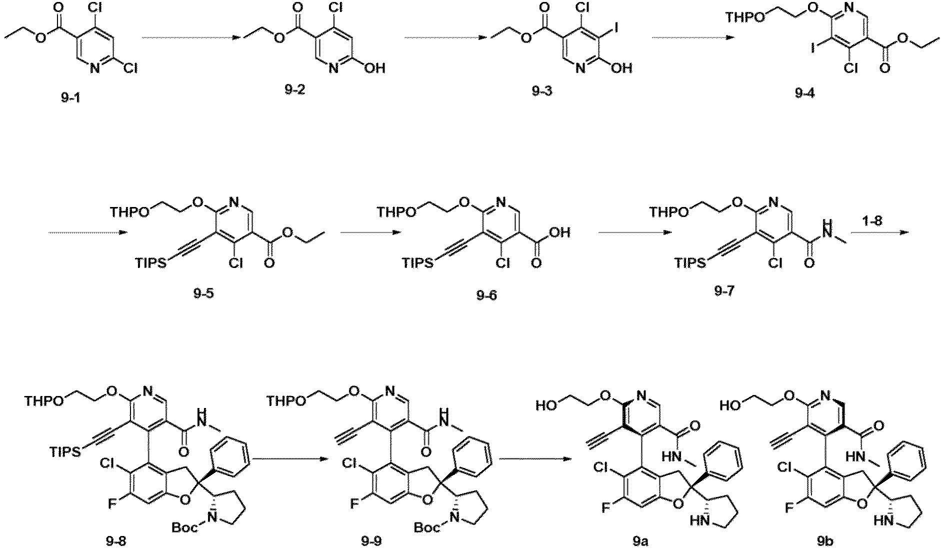

Step 1: Compound **9-1** (50 g, 227.2 mmol) was dissolved in acetic acid (500 mL), then sodium acetate (95 g, 1.135 mol) was added thereto under nitrogen atmosphere, and the reaction mixture was gradually heated to 110°C and reacted for 13 hours. TLC (PE: EA = 3:1) showed that the starting material was completely reacted. The reaction mixture was cooled to 35°C and poured into ice water (1 L). A solid was precipitated, filtered, and rinsed with water to adjust the pH to 6. The filter cake was collected, slurried with petroleum ether (200 mL) for 2 hours, and filtered. The filter cake was collected and dried to obtain compound **9-2** (27.1 g, 59%). LC-MS m/z (ESI): 202 [M+H]⁺.

Step 2: Compound **9-2** (26 g, 129.3 mmol) was dissolved in acetonitrile (300 mL), then N-iodosuccinimide (43.4 g, 194.0 mmol) was added thereto in batches under nitrogen atmosphere, and the reaction mixture was heated to 80°C and reacted for 16 hours. LCMS showed that the starting material was completely reacted. The reaction mixture was cooled to 25°C, filtered, washed with water (300 mL), and then washed with ethyl acetate (30 mL). The filter cake was collected and dried to obtain compound **9-3** (27.2 g, Y: 64%). ¹H NMR (400 MHz, DMSO-*d₆*) δ 8.09 (s, 1H), 4.26-4.17 (m, 2H), 1.27 (t, *J* =7.2 Hz, 3H). LC-MS m/z (ESI): 328 [M+H]⁺.

Step 3: Compound **9-3** (27 g, 82.5 mmol) and 2-(tetrahydro-2H-pyran-2-yloxy)ethanol (12 g, 82.5 mmol) were dissolved in dichloromethane (270 mL). The reaction mixture was added with triphenylphosphine (25.9 g, 99.1 mmol) under nitrogen atmosphere, then added dropwise with diethyl azodicarboxylate (20 g, 99.1 mmol), and reacted at 35°C for 1.5 hours. LCMS showed that the starting material was completely reacted. The reaction mixture was washed with water (100 mL × 2), and the organic phase was dried over anhydrous sodium sulfate and filtered. The filtrate was concentrated under reduced pressure and then purified by silica gel column chromatography (PE: EA = 10:1) to obtain compound **9-4** (6.4 g, Y: 17%). ¹H NMR (400 MHz, DMSO-*d₆*) δ 8.54 (s, 1H), 4.72 (s, 1H), 4.54-4.48 (m, 2H), 4.34-4.28 (m, 2H), 3.97-3.91 (m, 1H), 3.84-3.73 (m, 2H), 3.46-3.40 (m, 1H), 1.71-1.61(m, 2H), 1.52-1.43 (m, 4H), 1.34-1.27 (m, 3H). LC-MS m/z (ESI): 478 [M+Na]⁺.

Step 4: Compound **9-4** (1 g, 2.2 mol) was dissolved in tetrahydrofuran (10 mL). The reaction mixture was sequentially added with (triisopropylsilyl)acetylene (1.2 g, 6.6 mmol), triethylamine (665 mg, 6.6 mmol), cuprous iodide (41.6 mg, 220 µmol), and bis(triphenylphosphine)palladium(II) chloride (259 mg, 440 µmol) under nitrogen atmosphere, gradually heated to 70°C, and reacted for 2 hours. TLC (PE: EA = 8:1) showed that the starting material was completely reacted. The reaction mixture was cooled to 35°C, added with water (10 mL), and extracted with ethyl acetate (10 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 10:1) to obtain compound **9-5** (680 mg, Y: 61%). ¹H NMR (400 MHz, DMSO-*d₆*) δ 8.60 (s, 1H), 4.62 (s, 1H), 4.56-4.54 (m, 2H), 4.35-4.28 (m, 2H), 3.96-3.91 (m, 1H), 3.73-3.67 (m, 2H), 3.43-3.38 (m, 1H), 1.68-1.55 (m, 2H), 1.49-1.41 (m, 4H), 1.34-1.28 (m, 3H), 1.18-1.08 (m, 21H).

Step 5: Compound **9-5** (500 mg, 980 µmol) was dissolved in tetrahydrofuran (5 mL) and water (5 mL), then sodium hydroxide (235 mg, 5.88 mmol) was added thereto under nitrogen atmosphere, and the reaction mixture was gradually heated to 50°C and reacted for 3 hours. TLC (PE: EA = 5:1) showed that the starting material was completely reacted. The reaction mixture was poured into ice-cold 2 M hydrochloric acid (10 mL) to quench, and extracted with ethyl acetate (10 mL × 3). The organic phases were combined, washed with saturated brine (20 mL), dried over anhydrous sodium sulfate, filtered, and the filtrate was concentrated under reduced pressure to obtain compound **9-6** (500 mg, crude).

Step 6: Compound 9-6 (500 mg, 1.03 mmol) was dissolved in N,N-dimethylformamide (16 mL), then 2-(7-azabenzotriazol-1-yl)-N,N,N',N'-tetramethyluronium hexafluorophosphate (770 mg, 2.07 mmol), diisopropylethylamine (400 mg, 3.1 mmol), and methylamine hydrochloride (139 mg, 2.07 mmol) were sequentially added thereto under nitrogen atmosphere, and the reaction mixture was reacted at 50°C for 2 hours. LCMS showed that the starting material was completely reacted. The reaction mixture was cooled to 0°C, poured into 1 M hydrochloric acid (30 mL) to quench, and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 5:1) to obtain compound **9-7** (380 mg, Y: 91%).

Step 7: Compound **9-7** (250 mg, 504 µmol) and compound **1-8** (329 mg, 605 µmol) were dissolved in toluene (2 mL) and water (0.4 mL). The reaction mixture was sequentially added with tris(dibenzylideneacetone)dipalladium (46.2 mg, 50.4 µmol), 4,5-bis(diphenylphosphino)-9,9-dimethylxanthene (58.3 mg, 100 µmol), and potassium phosphate (209 mg, 1.51 mmol) under nitrogen atmosphere, gradually heated to 110°C, and reacted for 12 hours. TLC (PE: EA = 1:1) showed that the starting material was completely reacted. The reaction mixture was cooled to 25°C, poured into saturated ammonium chloride (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compound **9-8** (100 mg, Y: 22%). LC-MS m/z (ESI): 876.1 [M+H]⁺.

Step 8: Compound **9-8** (100 mg, 114 µmol) was dissolved in tetrahydrofuran (1 mL), then a solution of tetrabutylammonium fluoride in tetrahydrofuran (208 mg, 798 µmοl/2 mL) was added dropwise thereto under nitrogen atmosphere, and the reaction mixture was reacted at 25°C for 1 hour. TLC (PE: EA = 1:1) showed that the starting material was completely reacted. The reaction mixture was poured into saturated ammonium chloride (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compound **9-9** (80 mg, Y: 97%). LC-MS m/z (ESI): 692.10 [M+H]⁺.

Step 9: Compound **9-9** (80 mg, 111 µmol) was dissolved in a solution of 20% trifluoroacetic acid in dichloromethane (2 mL), and the reaction mixture was reacted at 25°C for 1 hour. TLC (DCM: MeOH = 10:1) showed that the starting material was completely reacted. The reaction mixture was cooled to 0°C, poured into saturated sodium carbonate (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was purified by preparative high-pressure liquid chromatography (column model: YMC-Actus Triart C18, 150 * 20 mm, 5 µm; eluent: 0.05% NH₃·H₂O+10 mmol/L NH₄HCO₃/CH₃CN+MeOH = 1:1; gradient: 53% to 73%; flow rate: 20 mL/min; run time: 11 min; retention time: 9.1 min for **9a** and 7.9 min for **9b**), and lyophilized to obtain compound **9a** (3.3 mg, Y: 5%) and compound **9b** (3.6 mg, Y: 6%). **9a:** ¹H NMR (400 MHz, DMSO-*d*₆) δ 8.43-8.20 (m, 1H), 7.48-7.24 (m, 6H), 7.09-6.96 (m, 1H), 4.91-4.85 (m, 1H), 4.48-4.35 (m, 2H), 3.97-3.85 (m, 1H), 3.78-3.72 (m, 2H), 3.52-3.46 (m, 1H), 2.95-2.90 (m, 1H), 2.75-2.56 (m, 4H), 2.06 (s, 1H), 1.55-1.20 (m, 4H). LC-MS m/z (ESI): 536 [M+H]⁺. **9b:** ¹H NMR (400 MHz, DMSO-*d*₆) δ 8.38-8.25 (m, 1H), 7.46-7.40 (m, 2H), 7.33-7.24 (m, 4H), 7.03-6.96 (m, 1H), 4.94-4.88 (m, 1H), 4.55-4.31 (m, 3H), 4.37-4.32 (m, 1H), 3.80-3.75 (m, 2H), 3.52-3.48 (m, 1H), 3.05-2.99 (m, 1H), 2.35-2.29 (m, 3H), 2.05-1.99 (m, 1H), 1.58-1.23 (m, 4H). LC-MS m/z (ESI): 536 [M+H]⁺.

### Example 10

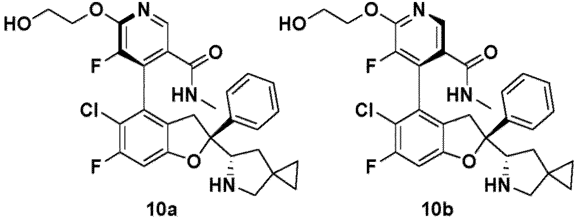

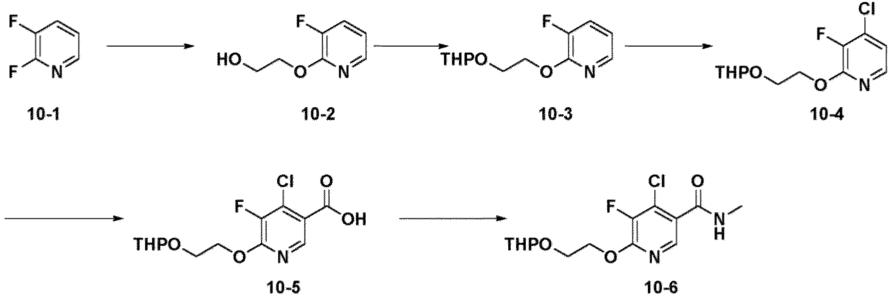

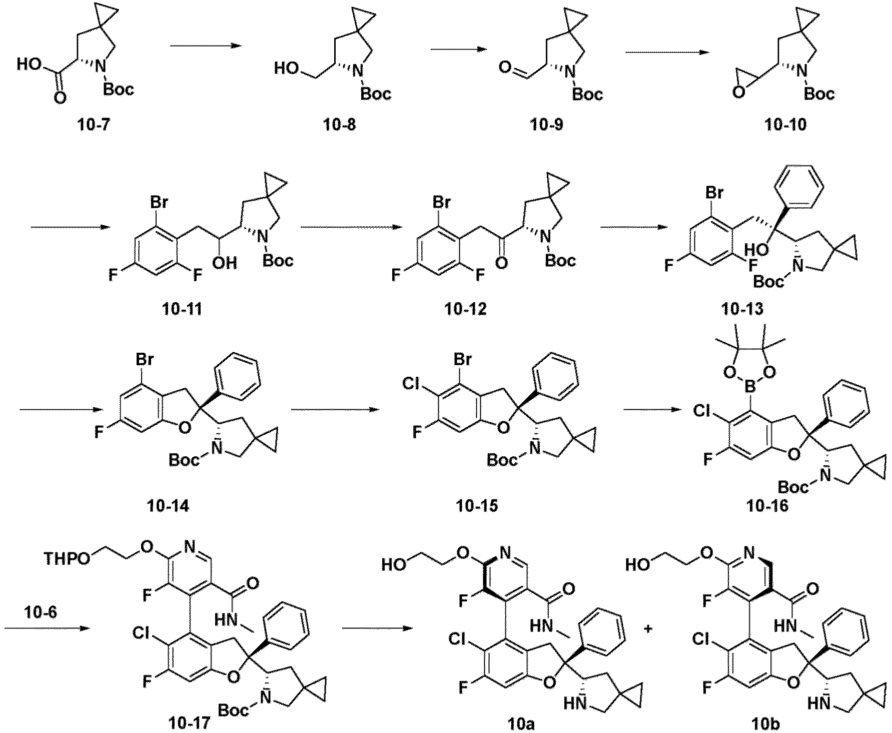

Step 1: Compound **10-1** (50.0 g, 434 mmol) and ethylene glycol (135 g, 2.17 mol) were dissolved in tetrahydrofuran (500 mL), then potassium tert-butoxide (53.63 g, 478 mmol) was added thereto in batches, and the resulting mixture was stirred at room temperature for 2 hours. The reaction mixture was diluted with water (300 mL), and extracted with ethyl acetate (300 mL × 3). The organic phases were combined, washed with saturated brine (500 mL), dried over anhydrous sodium sulfate, and then concentrated under reduced pressure to obtain compound **10-2** (48.4 g, crude). LC-MS m/z (ESI): 158 [M+H]⁺.

Step 2: Compound **10-2** (5.00 g, 31.8 mmol) and p-toluenesulfonic acid (6.03 g, 35.0 mmol) were dissolved in tetrahydrofuran (100 mL), and 3,4-dihydro-2H-pyran (5.35 g, 63.6 mmol) was added dropwise thereto. After the dropwise addition was completed, the reaction mixture was stirred at room temperature for 4 hours. The reaction mixture was diluted with water (80 mL), and extracted with ethyl acetate (100 mL × 3). The organic phases were combined, washed with saturated brine (100 mL), dried over anhydrous sodium sulfate, and then concentrated under reduced pressure to obtain compound **10-3** (9.30 g, crude). LC-MS m/z (ESI): 242 [M+H]⁺.

Step 3: Compound **10-3** (7.56 g, 31.3 mmol) was dissolved in anhydrous tetrahydrofuran (35 mL), then n-butyllithium (2.61 g, 40.7 mmol, 2.5 M) was added dropwise thereto at -78°C, and the reaction mixture was reacted at 78°C for 2 hours. A solution of hexachloroethane (9.64 g, 40.7 mmol) in anhydrous tetrahydrofuran (5 mL) was added dropwise thereto at -78°C. After the dropwise addition was completed, the reaction mixture was stirred at room temperature for 2 hours. The reaction mixture was cooled to 0°C, added dropwise with water (40 mL) to quench, and extracted with ethyl acetate (30 mL × 3). The organic phases were combined, washed with saturated brine (50 mL), dried over anhydrous sodium sulfate, concentrated under reduced pressure, and purified by silica gel column chromatography (PE: EA = 20: 1) to obtain compound **10-4** (8.05 g, Y: 93%).

Step 4: Diisopropylamine (2.39 g, 23.6 mmol) was dissolved in anhydrous tetrahydrofuran (45 mL), and n-butyllithium (9.4 mL, 23.6 mmol, 2.5 M) was added dropwise thereto at -75°C. After the dropwise addition was completed, the reaction mixture was reacted at -78°C for minutes, then added dropwise with a solution of compound **10-4** (5.00 g, 18.1 mmol) in anhydrous tetrahydrofuran (5 mL) at - 78°C, and reacted at -78°C for 30 minutes. Carbon dioxide was then introduced thereto, and the reaction mixture was reacted at -78°C for 1 hour. After the reaction was completed, the reaction mixture was added with 1 N hydrochloric acid aqueous solution at 0°C to quench and to adjust the pH to 2 to 3, and extracted with ethyl acetate (50 mL × 3). The organic phases were combined, washed with saturated brine (50 mL), dried over anhydrous sodium sulfate, concentrated under reduced pressure, and subjected to silica gel column chromatography to obtain compound **10-5** (4.30 g, Y: 74%).

Step 5: Compound **10-5** (7.00 g, 21.9 mmol) and diisopropylethylamine (14.2 g, 109 mmol) were dissolved in N,N-dimethylformamide (70 mL), then methylamine hydrochloride (2.96 g, 43.8 mmol) and 2-(7-azabenzotriazol-1-yl)-N,N,N',N'-tetramethyluronium hexafluorophosphate (16.6 g, 43.8 mmol) were added thereto, and the reaction mixture was reacted at room temperature for 20 minutes. After the reaction was completed, the reaction mixture was diluted with water (200 mL), added with 1 N sodium hydroxide aqueous solution to adjust the pH to 10, and extracted with ethyl acetate (100 mL × 3). The organic phases were combined, washed with 1 N hydrochloric acid (100 mL × 3) and saturated brine (100 mL × 3), respectively, dried over anhydrous sodium sulfate, concentrated under reduced pressure, and purified by silica gel column chromatography (DCM: MeOH = 10:1) to obtain compound **10-6** (5.56 g, Y: 76%).

Step 6: Compound **10-7** (15.0 g, 62.2 mmol) was dissolved in tetrahydrofuran (150 mL) under nitrogen atmosphere. The reaction mixture was cooled to 0°C, added dropwise with a solution of borane-dimethyl sulfide in tetrahydrofuran (10 M, 12.5 mL, 124.4 mmol), and reacted at 25°C for 16 hours. LCMS showed that the starting material was completely reacted. The reaction mixture was cooled to 0°C, added dropwise with methanol (30 mL) to quench, and concentrated under reduced pressure to obtain a yellow oil, which was diluted with ethyl acetate (150 mL), and washed with water (50 mL) and saturated brine (50 mL). The organic phase was dried over anhydrous sodium sulfate, filtered, and the filtrate was concentrated under reduced pressure to obtain compound **10-8** (12.4 g, Y: 87%). LC-MS m/z (ESI): 228.3 [M+H]⁺.

Step 7: Compound **10-8** (12.4 g, 54.5 mmol) was dissolved in dichloromethane (120 mL) under nitrogen atmosphere. The reaction mixture was sequentially added with dimethyl sulfoxide (42.5 g, 545 mmol) and N,N-diisopropylethylamine (42.2 g, 327 mmol), then added with pyridine sulfur trioxide complex (34.6 g, 218 mmol) in batches, and reacted at 25°C for 4 hours. LCMS showed that the starting material was completely reacted. The reaction mixture was diluted with dichloromethane (240 mL), and washed with saturated sodium bicarbonate (120 mL), water (120 mL), and saturated brine (120 mL). The organic phase was dried over anhydrous sodium sulfate, filtered, and the filtrate was concentrated under reduced pressure and then purified by silica gel column chromatography (EA: PE = 1:10) to obtain compound **10-9** (10.1 g, Y: 82%). ¹H NMR (400 MHz, DMSO-*d₆*): δ 9.52-9.48 (m, 1H), 4.23-4.17 (m, 1H), 3.33-3.27 (m, 1H), 3.24-3.18 (m, 1H), 2.09-2.06 (m, 1H), 1.86-1.80 (m, 1H), 1.45-1.30 (s, 9H), 0.62-0.42 (m, 4H). LC-MS m/z (ESI): 170.2 [M-tBu+H]⁺.

Step 8: Trimethylsulfoxonium iodide (11.8 g, 53.7 mmol) was dissolved in dimethyl sulfoxide (50 mL) and tetrahydrofuran (50 mL). The reaction mixture was added with sodium hydride (2.1 g, 53.7 mmol, 60%) in batches at room temperature, reacted at 25°C for 2 hours, cooled to 0°C, added dropwise with a solution of compound **10-9** (10.1 g, 44.8 mmol) in tetrahydrofuran (50 mL), gradually warmed to 25°C, and reacted for 16 hours. LCMS showed that the starting material was completely reacted. The reaction mixture was added with ice water (100 mL) to quench, and extracted with ethyl acetate (100 mL × 3). The organic phases were combined, washed with saturated brine (100 mL), dried over anhydrous sodium sulfate, and filtered. The filtrate was concentrated under reduced pressure and then purified by silica gel column chromatography (EA: PE = 1:10) to obtain compound **10-10** (4.2 g, Y: 39%). LC-MS m/z (ESI): 184.2 [M-tBu+H]⁺.

Step 9: 2,4-Difluoro-6-bromoiodobenzene (6.7 g, 21.0 mmol) was dissolved in tetrahydrofuran (42 mL). The reaction mixture was cooled to -40°C under nitrogen atmosphere, added dropwise with isopropylmagnesium chloride (2 M, 10.5 mL, 21.0 mmol), reacted at -40°C for 1 hour, added with cuprous iodide (0.7 g, 3.5 mmol), warmed to -10°C, added dropwise with a solution of compound **10-10** (4.2 g, 17.5 mmol) in tetrahydrofuran (10 mL), warmed to 25°C, and reacted for 16 hours. LCMS showed that the starting material was completely reacted. The reaction mixture was added with saturated ammonium chloride (100 mL) to quench, and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:9) to obtain compound **10-11** (3.5 g, Y: 45%). LC-MS m/z (ESI): 454.1 [M+Na]⁺.

Step 10: Compound **10-11** (3.5 g, 8.0 mmol) was dissolved in dichloromethane (35 mL), then Dess-Martin periodinane (6.8 g, 16.0 mmol) was added thereto in batches, and the reaction mixture was reacted at 25°C for 2 hours. TLC (EA: PE = 1:8) showed that the starting material was completely reacted. The reaction mixture was diluted with dichloromethane (70 mL), and washed with saturated sodium bicarbonate (50 mL), water (50 mL), and saturated brine (50 mL). The organic phase was dried over anhydrous sodium sulfate, filtered, and the filtrate was concentrated under reduced pressure and then purified by silica gel column chromatography (EA: PE = 1:10) to obtain compound **10-12** (2.8 g, Y: 80%).

Step 11: Compound **10-12** (3.1 g, 7.1 mmol) was dissolved in dichloromethane (28 mL) and n-heptane (28 mL). The reaction mixture was cooled to -40°C under nitrogen atmosphere, and added dropwise with phenylmagnesium bromide (1 M, 14,2 mL, 14.2 mmol). After the dropwise addition was completed, the reaction mixture was gradually warmed to 25°C, and reacted for 2 hours. LCMS showed that the starting material was completely reacted. The reaction mixture was added with saturated ammonium chloride (50 mL) to quench, extracted with ethyl acetate (50 mL × 3), and washed with saturated brine (50 mL). The organic phase was dried over anhydrous sodium sulfate, filtered, and the filtrate was concentrated under reduced pressure and then purified by silica gel column chromatography (EA: PE = 1:10) to obtain compound **10-13** (1.4 g, Y: 38%). LC-MS m/z (ESI): 508.2 [M+H]⁺.

Step 12: Compound **10-13** (1.4 g, 2.7 mmol) was dissolved in tetrahydrofuran (15 mL). The reaction mixture was cooled to 0°C under nitrogen atmosphere, added with potassium tert-butoxide (0.6 g, 5.4 mmol), and reacted at 0°C for 1 hour. TLC (EA: PE = 1:8) showed that the starting material was completely reacted. The reaction mixture was added with water (50 mL) to quench, and extracted with ethyl acetate (30 mL × 3). The organic phases were combined, washed with saturated brine (30 mL), dried over anhydrous sodium sulfate, and filtered. The filtrate was concentrated under reduced pressure and then purified by silica gel column chromatography (EA: PE = 1:10) to obtain compound **10-14** (1.1 g, Y: 81%).

Step 13: Compound **10-14** (1.1 g, 2.2 mmol) was dissolved in tetrahydrofuran (5 mL) and acetonitrile (10 mL), then p-toluenesulfonic acid (416 mg, 2.4 mmol) and N-chlorosuccinimide (320 mg, 2.4 mmol) were added thereto, and the reaction mixture was reacted at 25°C for 1 hour. LCMS showed that the starting material was completely reacted. The reaction mixture was added with water (25 mL) to quench, and extracted with ethyl acetate (20 mL × 3). The organic phases were combined, washed with saturated brine (30 mL), dried over anhydrous sodium sulfate, and filtered. The filtrate was concentrated under reduced pressure and then purified by silica gel column chromatography (EA: PE = 1:8) to obtain compound **10-15** (0.7 g, Y: 59%). ¹H NMR (400 MHz, CDCl₃): δ 7.50-7.44 (m, 2H), 7.36-7.26 (m, 3H), 6.68-6.62 (m, 1H), 4.66-4.61 (m, 1H), 4.10-4.05 (m, 1H), 3.45-3.35 (m, 2H), 2.89-2.83 (m, 1H), 2.19-2.13 (m, 1H), 1.74-1.69 (m, 1H), 1.40 (s, 9H), 0.48-0.35 (m, 2H), 0.16-0.06 (m, 2H). LC-MS m/z (ESI): 468.1 [M-tBu+H]⁺.

Step 14: Compound **10-15** (350 mg, 0.66 mmol) was dissolved in toluene (5 mL), then bis(pinacolato)diboron (184 mg, 0.72 mmol), potassium acetate (129 mg, 1.32 mmol), and [1,1'-bis(diphenylphosphino)ferrocene]palladium(II) dichloride (51 mg, 0.07 mmol) were sequentially added thereto, and the reaction mixture was heated to 100°C and reacted for 16 hours under nitrogen atmosphere. LCMS showed that the starting material was completely reacted. The reaction mixture was concentrated under reduced pressure and then purified by silica gel column chromatography (EA: PE = 1:7) to obtain compound **10-16** (290 mg, Y: 75%). LC-MS m/z (ESI): 592.4 [M+Na]⁺.

Step 15: Compound **10-16** (260 mg, 0.45 mmol) and compound **10-6** (164 mg, 0.49 mmol) were dissolved in toluene (4 mL), then water (0.8 mL), potassium phosphate (191 mg, 0.90 mmol), 4,5-bis(diphenylphosphino)-9,9-dimethylxanthene (52 mg, 0.09 mmol), and tris(dibenzylideneacetone)dipalladium (33 mg, 0.045 mmol) were added thereto, and the reaction mixture was reacted at 100°C for 16 hours under nitrogen atmosphere. LCMS showed that the starting material was completely reacted. The reaction mixture was cooled to 25°C, concentrated under reduced pressure, and then purified by silica gel column chromatography (EA: PE = 1:2) to obtain compound **10-17** (91 mg, Y: 26%). LC-MS m/z (ESI): 740.4 [M+H]⁺.

Step 16: Compound **10-17** (100 mg, 0.13 mmol) was dissolved in dichloromethane (5 mL), then trifluoroacetic acid (0.5 mL) was added thereto, and the reaction mixture was reacted at 25°C for 1 hour. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was purified by preparative high-pressure liquid chromatography (column model: YMC Triart Prep C18-S, 250 * 50 mm, 10 µm; eluent: 0.05% NH₃·H₂O+10 mmol/L NH₄HCO₃/CH₃CN+MeOH = 1:1; gradient: 41% to 61%; flow rate: 120 mL/min; run time: 12 min; retention time: 6.8 min for **10a** and 5.7 min for **10b**), and lyophilized to obtain compound **10a** (7.5 mg, Y: 9%) and compound **10b** (14.4 mg, Y: 19%). **10a:** ¹H NMR (400 MHz, DMSO-*d₆*): δ 8.42-8.38 (m, 1H), 8.28 (s, 1H), 7.48-7.42 (m, 2H), 7.37-7.31 (m, 2H), 7.29-7.25 (m, 1H), 7.07 (d, *J* = 10.0 Hz, 1H), 4.96 (t, *J* = 5.6 Hz, 1H), 4.50-4.38 (m, 2H), 3.79-3.68 (m, 3H), 3.51 (d, *J* = 15.2 Hz, 1H), 2.98 (d, *J* = 16.0 Hz, 1H), 2.70-2.60 (m, 6H), 1.59-1.53 (m, 1H), 1.36-1.23 (m, 1H), 0.40-0.25 (m, 4H). LC-MS m/z(ESI): 556.30 [M+H]⁺. **10b:** ¹H NMR (400 MHz, DMSO-*d₆*): δ 8.44 -8.40 (m, 1H), 8.23 (s, 1H), 7.48-7.41 (m, 2H), 7.35-7.25 (m, 3H), 7.04 (d, *J* = 10.0 Hz, 1H), 4.98 (t, *J* = 5.6 Hz, 1H), 4.52-4.40 (m, 2H), 3.83-3.65 (m, 3H), 3.40 (d, *J* = 15.2 Hz, 1H), 3.10 (d, *J* = 16.0 Hz, 1H), 2.70-2.59 (m, 3H), 2.35 (d, *J* = 4.0 Hz, 3H), 1.54-1.47 (m, 1H), 1.32-1.22 (m, 1H), 0.38-0.24 (m, 4H). LC-MS m/z (ESI): 556.30 [M+H]⁺.

### Example 11

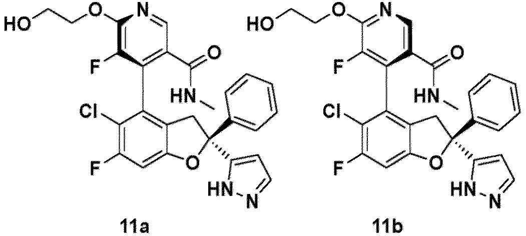

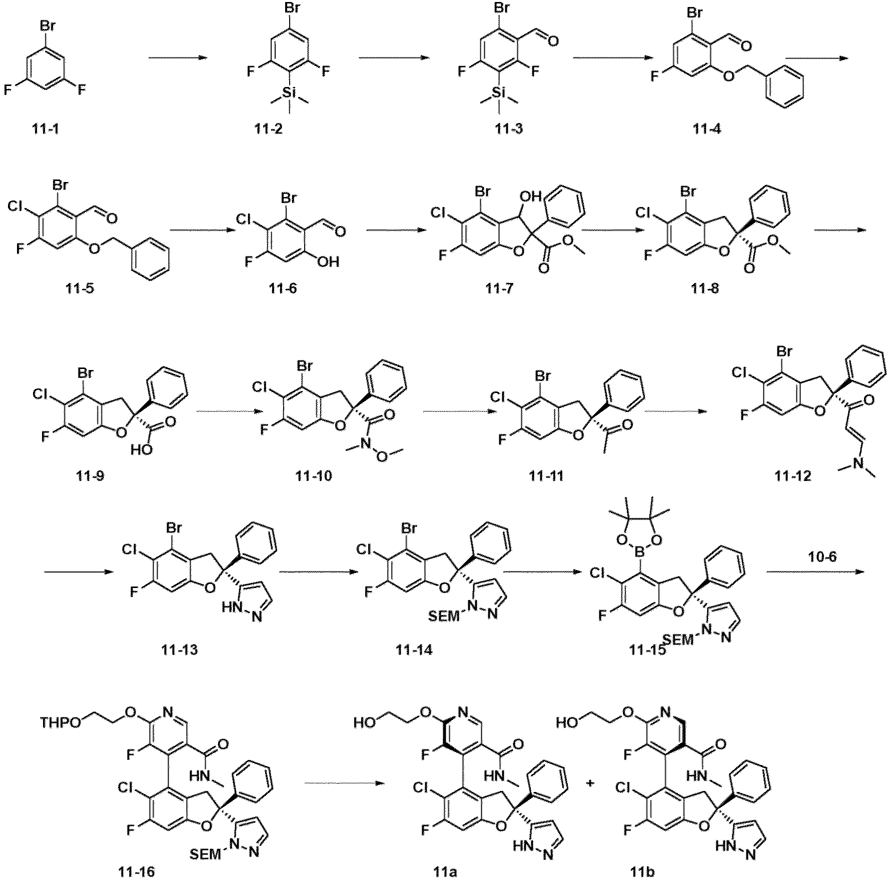

Step 1: Compound **11-1** (300 g, 1.55 mol) was dissolved in tetrahydrofuran (5000 mL). The reaction mixture was cooled to -78°C under nitrogen atmosphere, and added dropwise with lithium diisopropylamide (855 mL, 2 M). After the addition was completed, the reaction mixture was reacted at - 78°C for 2 hours, then added dropwise with chlorotrimethylsilane (203.3 g, 1.86 mol) at -78°C, kept at - 78°C and reacted for 1 hour. After the reaction was completed, the reaction mixture was directly concentrated under reduced pressure, added with n-hexane (500 mL), and then filtered. The filtrate was collected and concentrated under reduced pressure to obtain compound **11-2** (370.0 g, Y: 90%). LC-MS m/z (ESI): 282 [M+H]⁺.

Step 2: Compound **11-2** (370 g, 1.40 mol) was dissolved in tetrahydrofuran (5000 mL). The reaction mixture was cooled to -78°C under nitrogen atmosphere, and added dropwise with lithium diisopropylamide (840 mL, 2 M). After the addition was completed, the reaction mixture was reacted at - 78°C for 1 hour. The system was then added dropwise with N,N-dimethylformamide (204.4 g, 2.80 mol) dissolved in tetrahydrofuran (500 mL), and the reaction mixture was reacted at -78°C for 1 hour. TLC (PE: EA = 12:1) showed that the starting material was completely reacted. The reaction mixture was returned to room temperature, added dropwise with a solution of acetic acid (300 mL)/water (1400 mL), then stirred at room temperature for 1 hour, and extracted with organic solution (EA/PE = 1/10, 1000 mL × 2). The organic phases were combined, washed with saturated brine (1000 mL), dried over anhydrous sodium sulfate, and filtered. The filtrate was concentrated under reduced pressure and then purified by silica gel column chromatography (EA: PE = 1:20) to obtain compound **11-3** (287.0 g, Y: 70%). ¹H NMR (400 MHz, DMSO-*d₆*) δ 10.14 (s, 1H), 7.65-7.55 (m, 1H), 0.46-0.26 (m, 1H).

Step 3: Compound **11-3** (287.0 g, 0.98 mol) was dissolved in benzyl alcohol (480 mL), and sodium benzyloxide solution (1.07 mol, dissolved in 1000 mL of benzyl alcohol) was added dropwise thereto. After the dropwise addition was completed, the reaction mixture was heated to 40°C and stirred for 15 minutes. After the reaction was completed, the reaction mixture was added dropwise with saturated ammonium chloride solution (1000 mL) to quench, and extracted with ethyl acetate (1000 mL × 2). The organic phases were combined, washed with saturated brine (1000 mL), dried over anhydrous sodium sulfate, and filtered. The filtrate was concentrated under reduced pressure and then purified by reversed-phase chromatography to obtain compound **11-4** (65.5 g, Y: 21%). ¹H NMR (400 MHz, DMSO-*d₆*) δ 10.26 (s, 1H), 7.54-7.48 (m, 2H), 7.45-7.39 (m, 2H), 7.39-7.30 (m, 3H), 5.29 (s, 1H).

Step 4: Compound **11-4** (65.5 g, 212.7 mmol) was dissolved in acetonitrile (500 mL), then N-chlorosuccinimide (33.9 g, 255.2 mmol) and p-toluenesulfonic acid (54.9 g, 319.1 mmol) were added thereto under nitrogen atmosphere, and the reaction mixture was reacted at 25°C for 16 hours. After the reaction was completed, the reaction mixture was added with saturated sodium bicarbonate solution to quench, and extracted with ethyl acetate (500 mL × 2). The organic phases were combined, washed with saturated brine, dried over anhydrous sodium sulfate, and filtered. The filtrate was concentrated under reduced pressure and then purified by silica gel column chromatography (EA: PE = 1:20) to obtain compound **11-5** (51.5 g, Y: 71%). ¹H NMR (400 MHz, DMSO-*d₆*) δ 10.38 (s, 1H), 7.47-7.36 (m, 2H), 6.89-6.75 (m, 1H), 5.16(s, 2H).

Step 5: Compound **11-5** (51.0 g, 149.1 mmol) was dissolved in dichloromethane (800 mL). The reaction mixture was cooled to -78°C under nitrogen atmosphere, added dropwise with a solution of boron tribromide (41.2 g, 164.1 mmol) in dichloromethane (200 mL), and reacted at -78°C for 2 hours. After the reaction was completed, the reaction mixture was added dropwise with methanol (150 mL) to quench, slowly warmed to room temperature and stirred overnight, concentrated under reduced pressure, and purified by silica gel column chromatography (EA: PE = 1:10) to obtain compound **11-6** (22.3 g, Y: 59%).

Step 6: Compound **11-6** (20.0 g, 78.9 mmol) was dissolved in acetonitrile (400 mL), then diisopropylethylamine (20.4 g, 157.8 mmol) and methyl bromophenylacetate (21.7 g, 94.7 mmol) were sequentially added thereto under nitrogen atmosphere, and the reaction mixture was gradually heated to 85°C and reacted for 16 hours. TLC (PE: EA = 20:1) showed that the starting material was completely reacted. The reaction mixture was directly concentrated under reduced pressure and then purified by silica gel column chromatography (EA: PE = 1:16) to obtain compound **11-7** (27.0 g, Y: 85%). ¹H NMR (400 MHz, DMSO-*d₆*) δ 7.68-7.62 (m, 1H), 7.55-7.50 (m, 1H), 7.46-7.37 (m, 4H), 6.92 (d, J= 7.2 Hz, 0.5H), 6.11 (d, J= 8.8 Hz, 0.5H), 5.58 (d, J= 8.8 Hz, 0.5H), 5.41 (d, J= 7.6 Hz, 0.5H), 3.75-3.64 (m, 3H).

Step 7: Compound **11-7** (27.0 g, 67.5 mmol) was dissolved in dichloromethane (300 mL). The reaction mixture was cooled to 0°C under nitrogen atmosphere, then the system was added dropwise with triethylsilane (39.2 g, 337.5 mmol) and boron trifluoride diethyl etherate (32.1 g, 135.0 mmol), and the reaction mixture was warmed to 25°C and reacted for 16 hours. After the reaction was completed, the reaction mixture was poured into saturated sodium bicarbonate solution, and extracted with dichloromethane (200 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:20) to obtain a yellow solid (20.8 g). The solid was subjected to chiral resolution (chiral column model: Chiralpak AD-3100 × 3.0 mm 3.0 µm, solvent: isopropanol, flow rate: 2.0 mL/min, t1 = 1.01 min, t2 = 0.87 min) to obtain compound **11-8** (10.1 g, t1 = 1.01 min, Y: 38%). ¹H NMR (400 MHz, DMSO-*d₆*) δ 7.57-7.51 (m, 2H), 7.43-7.35 (m, 3H), 6.83 (d, *J=* 8.8 Hz, 1H), 4.22-4.15 (m, 1H), 3.77 (s, 3H), 3.58-3.51 (m, 1H).

Step 8: Compound **11-8** (1.9 g, 4.9 mmol) was dissolved in tetrahydrofuran (30 mL), then the system was added dropwise with lithium hydroxide (0.24 g, 9.8 mmol) dissolved in water (15 mL), and the reaction mixture was reacted at 25°C for 16 hours under nitrogen atmosphere. LCMS showed that the starting material was completely reacted. The reaction mixture was added dropwise with hydrochloric acid aqueous solution (30 mL, 1 M) in an ice bath to adjust the pH to 5 to 6, and extracted with ethyl acetate (50 mL × 2). The organic phases were combined, washed with saturated brine (50 mL), dried over anhydrous sodium sulfate, filtered, and the filtrate was concentrated under reduced pressure to obtain compound **11-9** (1.5 g, Y: 83%). LC-MS m/z (ESI): 369 [M-H]⁺.

Step 9: Compound **11-9** (1.5 g, 4.1 mmol), diisopropylethylamine (2.6 g, 20.1 mmol), and methoxymethylamine hydrochloride (0.79 g, 8.2 mmol) were dissolved in N,N-dimethylformamide (30 mL), then 2-(7-azabenzotriazol-1-yl)-N,N,N',N'-tetramethyluronium hexafluorophosphate (2.1 g, 5.3 mmol) was added thereto, and the reaction mixture was reacted at room temperature for 3 hours. LCMS showed that the starting material was completely reacted. The reaction mixture was extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (EA: PE = 1:8) to obtain compound **11-10** (1.5 g, Y: 93%). LC-MS m/z (ESI): 414 [M+H]⁺.

Step 10: Compound **11-10** (1.3 g, 3.14 mmol) was dissolved in tetrahydrofuran (40 mL), then methylmagnesium bromide (6.3 mL, 1 M) was added dropwise thereto at 0°C, and the reaction mixture was stirred and reacted at 0°C for 2 hours. TLC (PE: EA = 8:1) showed that the starting material was completely reacted. The reaction mixture was poured into saturated ammonium chloride solution (100 mL) to quench, and extracted with ethyl acetate (20 mL × 3). The organic phases were combined, washed with saturated brine (100 mL), dried over anhydrous sodium sulfate, and filtered. The filtrate was concentrated under reduced pressure and then purified by silica gel column chromatography (EA: PE = 1:12) to obtain compound **11-11** (0.9 g, Y: 77%). LC-MS m/z (ESI): 371 [M+H]⁺.

Step 11: Compound **11-11** (800 mg, 2.16 mmol) was dissolved in N,N-dimethylformamide dimethyl acetal (30 mL), and the reaction mixture was stirred and reacted at 80°C for 16 hours under nitrogen atmosphere. After the starting material was completely reacted, the reaction mixture was cooled to 20°C, poured into water (50 mL), and extracted with ethyl acetate (50 mL × 3). The organic phases were combined, washed with saturated brine (100 mL), dried over anhydrous sodium sulfate, filtered, and the filtrate was concentrated under reduced pressure to obtain compound **11-12** (820 mg, crude). LC-MS m/z (ESI): 424 [M+H]⁺.

Step 12: Compound **11-12** (820 mg, 1.93 mmol) was dissolved in hydrazine hydrate (40 mL), and the reaction mixture was stirred and reacted at 60°C for 4 hours. LCMS showed that the starting material was completely reacted. The reaction mixture was poured into H₂O (100 mL) to quench, and extracted with ethyl acetate (50 mL × 3). The organic phases were combined, washed with saturated brine (100 mL), dried over anhydrous sodium sulfate, and filtered. The filtrate was concentrated under reduced pressure and then purified by silica gel column chromatography (EA: PE = 1:8) to obtain compound **11-13** (420 mg, Y: 52%). LC-MS m/z (ESI): 394 [M+H]⁺.

Step 13: Compound **11-13** (520 mg, 1.32 mmol) was dissolved in tetrahydrofuran (30 mL). The reaction mixture was cooled to 0°C, added with sodium hydride (110 mg, 60%, 2.65 mmol), and stirred for 30 minutes. The system was then added dropwise with 2-(trimethylsilyl)ethoxymethyl chloride (300 mg, 1.76 mmol) dissolved in tetrahydrofuran (3 mL), and the reaction mixture was reacted for 2 hours. After the starting material was completely reacted, the reaction mixture was poured into water (50 mL) to quench, and extracted with ethyl acetate (50 mL × 3). The organic phases were combined, washed with saturated brine (100 mL), dried over anhydrous sodium sulfate, and filtered. The filtrate was concentrated under reduced pressure and then purified by silica gel column chromatography (EA: PE = 1:15) to obtain compound **11-14** (410 mg, Y: 59%). LC-MS m/z (ESI): 523 [M+H]⁺.

Step 14: Compound **11-14** (390 mg, 744 µmol) was dissolved in toluene (10 mL). The reaction mixture was sequentially added with [1,1'-bis(diphenylphosphino)ferrocene]palladium(II) dichloride (62.6 mg, 74.5 µmol), bis(pinacolato)diboron (567 mg, 2.3 mmol), and potassium acetate (146 mg, 1.5 mmol) under nitrogen atmosphere, heated to 100°C, and reacted for 16 hours. LCMS showed that the starting material was completely reacted. The reaction mixture was cooled to 25°C, directly concentrated under reduced pressure, and then purified by silica gel column chromatography (EA: PE = 1:20) to obtain compound **11-15** (350 mg, Y: 82%). LC-MS m/z (ESI): 572 [M+H]⁺.

Step 15: Compound **11-15** (300 mg, 525 µmol) and compound **10-6** (175 mg, 525 µmol) were dissolved in toluene (2 mL) and water (0.4 mL). The reaction mixture was sequentially added with tris(dibenzylideneacetone)dipalladium (96.2 mg, 105 µmol), 4,5-bis(diphenylphosphino)-9,9-dimethylxanthene (116 mg, 210 µmol), and potassium carbonate (218 mg, 1.58 mmol) under nitrogen atmosphere, heated to 120°C, and reacted for 16 hours. LCMS showed that the starting material was completely reacted. The reaction mixture was cooled to 25°C, poured into saturated ammonium chloride (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (EA: PE = 1:8) to obtain compound **11-16** (105 mg, Y: 26%). LC-MS m/z (ESI): 763.2 [M+Na]⁺.

Step 16: Compound **11-16** (105 mg, 240 µmol) was dissolved in a solution of 20% trifluoroacetic acid in dichloromethane (2 mL), and the reaction mixture was reacted at 25°C for 0.5 hours. TLC (DCM: MeOH = 10:1) showed that the starting material was completely reacted. The reaction mixture was cooled to 0°C, poured into saturated sodium carbonate (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O/CH₃CN; gradient: 37% to 57%; flow rate: 20 mL/min; run time: 12 min; retention time: 8.2 min for **11a** and 6.7 min for **11b**) to obtain compound **11a** (8.2 mg, Y: 11%) and compound **11b** (6.0 mg, Y: 8%). **11a:** ¹H NMR (400 MHz, DMSO-*d*₆) δ 8.52 (d, J= 4.4 Hz, 1H), 8.27 (s, 1H), 7.70 (s, 1H), 7.33-7.15 (m, 6H), 6.11 (s, 1H), 4.93 (t, *J* = 5.2 Hz, 1H), 4.46-4.39 (m, 2H), 4.15 (d, *J* = 16.0 Hz, 1H), 3.81-3.73 (m, 2H), 3.20-3.14 (m, 1H), 2.66-2.62 (m, 3H). LC-MS m/z (ESI): 527.1 [M+H]⁺. **11b:** ¹H NMR (400 MHz, DMSO-*d*₆) δ 8.42 (d, *J* = 4.8 Hz, 1H), 8.22 (s, 1H), 7.69 (s, 1H), 7.35-7.14 (m, 6H), 6.08 (s, 1H), 4.99 (t, *J* = 5.2 Hz, 1H), 4.49-4.43 (m, 2H), 4.08 (d, *J* = 16.0 Hz, 1H), 3.83-3.76 (m, 2H), 3.31-3.24 (m, 1H), 2.40-2.31 (m, 3H). LC-MS m/z (ESI): 527.1 [M+H]⁺.

### Example 12

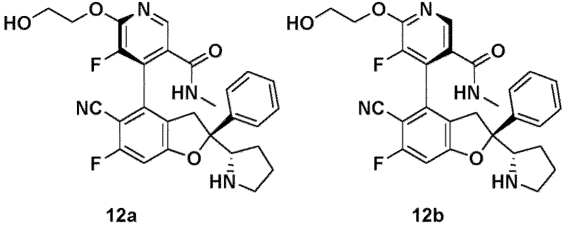

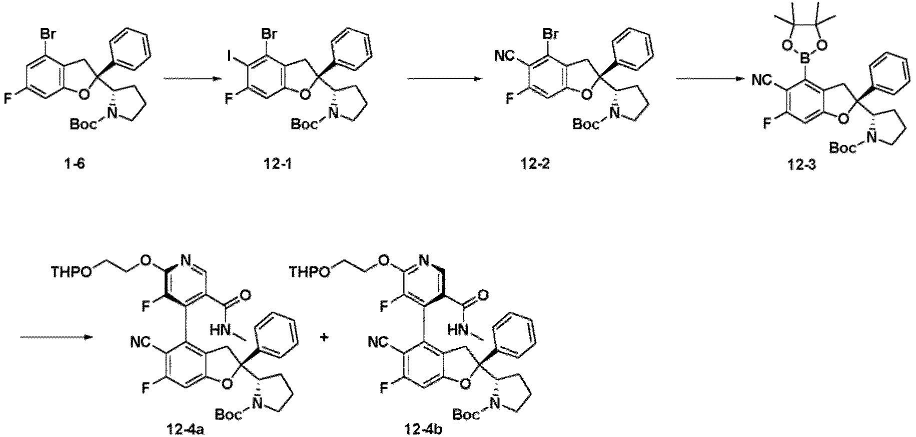

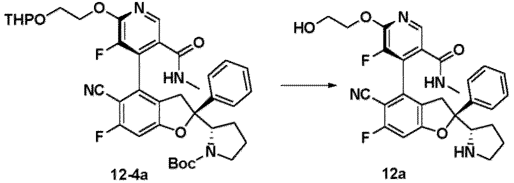

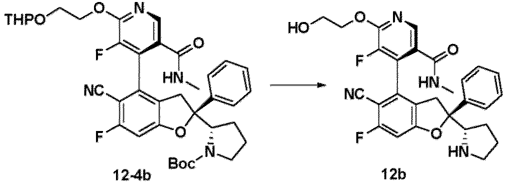

Step 1: Compound **1-6** (12.0 g, 26 mmol) was dissolved in acetonitrile/tetrahydrofuran (100 mL/50 mL). The reaction mixture was added with p-toluenesulfonic acid (4.5 g, 26 mmol), then added with N-iodosuccinimide (7.6 g, 33.8 mmol) in batches, and reacted at room temperature for 12 hours. TLC (PE: EA = 15:1) showed that the reaction was completed. The reaction mixture was added with saturated sodium bicarbonate solution to adjust the pH to 6 to 7. The filtrate was concentrated under reduced pressure and then subjected to silica gel column chromatography (PE: EA = 15:1) to obtain compound **12-1** (9.7 g, crude).

Step 2: Compound **12-1** (9.7 g, 16.5 mmol) was dissolved in dimethyl sulfoxide (100 mL), then cuprous cyanide (2.9 g, 33 mmol) was added thereto, and the reaction mixture was heated to 90°C and reacted for 12 hours. TLC (PE: EA = 5:1) showed that the reaction was completed. The reaction mixture was cooled to room temperature, poured into ice water (200 mL), and a solid was precipitated. The solid was collected and purified by silica gel column chromatography (EA: PE = 1:5) to obtain compound **12-2** (7.7 g, crude). LC-MS m/z (ESI): 486 [M+H]⁺.

Step 3: Compound **12-2** (500 mg, 1.0 mmol) was dissolved in tetrahydrofuran (15 mL). The system was cooled to -78°C under nitrogen atmosphere, added dropwise with n-butyllithium (2.5 mol/L, 0.62 mL, 1.5 mmol), reacted at -78°C for 30 minutes, then added with 2-isopropoxy-4,4,5,5-tetramethyl-1,3,2-dioxaborolane (287 mg, 1.5 mmol), gradually warmed to room temperature, and reacted for 2 hours. The reaction mixture was added dropwise with saturated ammonium chloride aqueous solution (50 mL) to quench, and the phases were separated. The organic phase was collected, concentrated under reduced pressure, and purified by reversed-phase chromatography to obtain compound **12-3** (240 mg, Y: 48%).

Step 4: Compound **12-3** (240 mg, 0.45 mmol) was dissolved in toluene (3 mL) and water (0.6 mL), then compound **10-6** (164 mg, 0.49 mmol), cesium fluoride (203.6 mg, 1.35 mmol), 4,5-bis(diphenylphosphino)-9,9-dimethylxanthene (52 mg, 0.09 mmol), and tris(dibenzylideneacetone)dipalladium (41 mg, 0.045 mmol) were sequentially added thereto, and the reaction mixture was reacted at 100°C for 16 hours under nitrogen atmosphere. The reaction mixture was cooled to room temperature, concentrated under reduced pressure, and then purified by a preparative silica gel plate (EA: PE = 1:1) to obtain compound **12-4a** (20 mg, Y: 8%, Rf = 0.3) and compound **12-4b** (30 mg, Y: 12%, Rf = 0.35). LC-MS m/z (ESI): 705.3 [M+H]⁺.

Step 5: Compound **12-4a** (20 mg, 0.028 mmol) was dissolved in dichloromethane (5 mL), then trifluoroacetic acid (1 mL) was added thereto at 5°C, and the reaction mixture was reacted at 5°C for 1 hour. TLC (PE: EA = 1:1) showed that the starting material was completely reacted. The reaction mixture was concentrated under reduced pressure and then purified by preparative high-pressure liquid chromatography (column model: YMC-Actus Triart C18, 150 * 20 mm, 5 µm; eluent: 10 mmol/L NH₄HCO₃/CH₃CN; gradient: 62% to 82%; flow rate: 20 mL/min; run time: 10 min; retention time: 7.5 min) to obtain compound 12a (6.7 mg, Y: 45%). ¹H NMR (400 MHz, DMSO-*d*₆) δ 8.76 (d, *J* = 4.0 Hz, 1H), 8.35 (s, 1H), 7.76 (d, *J* = 6.4 Hz, 1H), 7.33-7.26 (m, 5H), 4.96 (t, *J* = 5.2 Hz, 1H), 4.51-4.45 (m, 2H), 3.80-3.70 (m, 3H), 3.42-3.33 (m, 2H), 2.77-2.66 (m, 5H), 1.52-1.23 (m, 4H). LC-MS m/z (ESI): 521.3 [M+H]⁺.

Step 6: Compound **12-4b** (30 mg, 0.043 mmol) was dissolved in dichloromethane (5 mL), then trifluoroacetic acid (1 mL) was added thereto at 5°C, and the reaction mixture was reacted at 5°C for 1 hour. TLC (PE: EA = 1:1) showed that the starting material was completely reacted. The reaction mixture was concentrated under reduced pressure and then purified by preparative high-pressure liquid chromatography (column model: YMC-Actus Triart C18, 150 * 20 mm, 5 µm; eluent: 10 mmol/L NH₄HCO₃/CH₃CN; gradient: 62% to 82%; flow rate: 20 mL/min; run time: 11 min; retention time: 7.9 min) to obtain compound 12b (10.5 mg, Y: 47%). ¹H NMR (400 MHz, DMSO-*d*₆) δ 8.62-8.56 (m, 1H), 8.34 (s, 1H), 7.73 (d, *J* = 6.4 Hz, 1H), 7.37-7.26 (m, 5H), 4.95 (t, *J* = 5.2 Hz, 1H), 4.51-4.46 (m, 2H), 3.83-3.76 (m, 2H), 3.71 (d, *J* = 16.0 Hz, 1H), 3.46-3.38 (m, 2H), 2.73-2.58 (m, 2H), 2.46 (d, *J* = 4.4 Hz), 1.42-1.33 (m, 4H). LC-MS m/z (ESI): 521.3 [M+H]⁺.

### Example 13

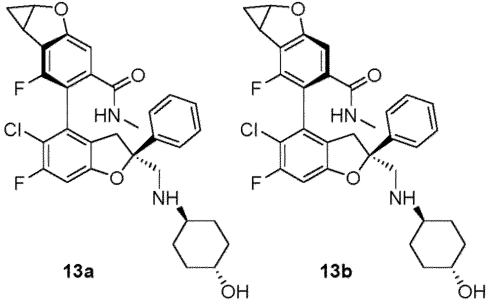

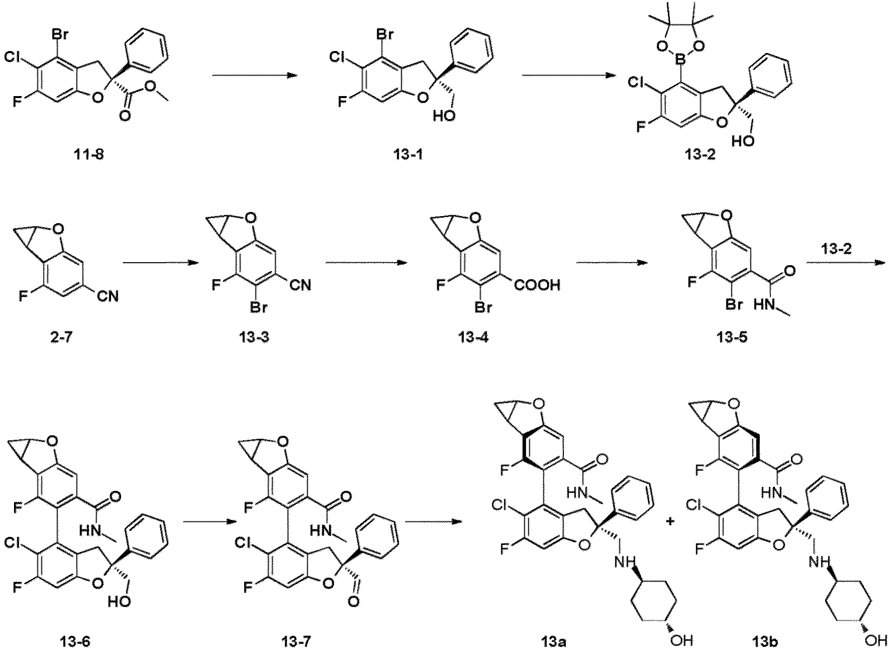

Step 1: Compound **11-8** (1 g, 2.81 mmol) was dissolved in methanol (10 mL). The reaction mixture was cooled to 0°C under nitrogen atmosphere, added with sodium borohydride (531 mg, 14.0 mmol) in batches, and reacted at 0°C for 2 hours. TLC (PE: EA = 10:1) showed that the starting material was completely reacted. The reaction mixture was poured into saturated ammonium chloride (20 mL) at 0°C to quench, and extracted with ethyl acetate (30 mL × 3). The organic phases were combined, washed with saturated brine (30 mL), dried over anhydrous sodium sulfate, filtered, and the filtrate was concentrated under reduced pressure to obtain compound **13-1** (800 mg, crude).

Step 2: Compound **13-1** (800 mg, 2.2 mmol) was dissolved in toluene (10 mL). The reaction mixture was sequentially added with [1,1'-bis(diphenylphosphino)ferrocene]palladium(II) dichloride (160 mg, 223 µmol), bis(pinacolato)diboron (1.14 g, 4.5 mmol), and potassium acetate (660 mg, 6.7 mmol) under nitrogen atmosphere, heated to 90°C, and reacted for 16 hours. LCMS showed that the starting material was completely reacted. The reaction mixture was cooled to 25°C, directly concentrated under reduced pressure, and then purified by silica gel column chromatography (EA: PE = 1:20) to obtain compound **13-2** (400 mg, Y: 44%). LC-MS m/z (ESI): 405 [M+H]⁺.

Step 3: Compound **2-7** (1.3 g, 7.42 mmol) was dissolved in tetrahydrofuran (13 mL). The reaction mixture was cooled to -70°C under nitrogen atmosphere, added dropwise with lithium diisopropylamide (2 M, 4.5 mL), stirred at -70°C for 30 minutes, then added with 1,2-dibromotetrachloroethane (3.2 g, 8.9 mmol), returned to 25°C with dry ice bath removed, and reacted for 1 hour. TLC (PE: EA = 20:1) showed that the starting material was completely reacted. The reaction mixture was poured into saturated ammonium chloride (20 mL) at 0°C to quench, and extracted with ethyl acetate (30 mL × 3). The organic phases were combined, washed with saturated brine (30 mL), dried over anhydrous sodium sulfate, and filtered. The filtrate was concentrated under reduced pressure and then purified by silica gel column chromatography (EA: PE = 1:10) to obtain compound **13-3** (1.5 g, Y: 79%). ¹H NMR (400 MHz, CDCl₃) δ 6.94 (s, 1H), 5.03-4.96 (m, 1H), 2.87-2.80 (m, 1H), 1.32-1.25 (m, 1H), 0.51-0.45 (m, 1H).

Step 4: Compound **13-3** (1.5 g, 5.9 mmol) was dissolved in a mixed solvent of ethanol (10 mL) and water (10 mL), then sodium hydroxide (1.4 g, 35.4 mmol) was added thereto under nitrogen atmosphere, and the reaction mixture was gradually heated to 110°C and reacted for 40 hours. LCMS showed that the starting material was completely reacted. The reaction mixture was cooled to 0°C, poured into 1 M hydrochloric acid (30 mL) to quench, and extracted with ethyl acetate (50 mL × 3). The organic phases were combined, washed with saturated brine (50 mL), dried over anhydrous sodium sulfate, filtered, and the filtrate was concentrated under reduced pressure to obtain compound **13-4** (1.6 g, crude). LC-MS m/z (ESI): 271 [M-H]⁺.

Step 5: Compound **13-4** (1.6 g, 5.86 mmol) was dissolved in N,N-dimethylformamide (16 mL), then 2-(7-azabenzotriazol-1-yl)-N,N,N',N'-tetramethyluronium hexafluorophosphate (4.4 g, 11.7 mmol), diisopropylethylamine (2.2 g, 17.5 mmol), and methylamine hydrochloride (791 mg, 11.7 mmol) were sequentially added thereto under nitrogen atmosphere, and the reaction mixture was reacted at room temperature for 2 hours. LCMS showed that the starting material was completely reacted. The reaction mixture was cooled to 0°C, poured into 1 M hydrochloric acid (30 mL) to quench, and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:5) to obtain compound **13-5** (1.3 g, Y: 77%). LC-MS m/z (ESI): 285.9 [M+H]⁺.

Step 6: Compound **13-5** (200 mg, 699 µmol) and compound **13-2** (339 mg, 838 µmol) were dissolved in toluene (2 mL) and water (0.4 mL). The reaction mixture was sequentially added with tris(dibenzylideneacetone)dipalladium (63.9 mg, 69.9 µmol), 4,5-bis(diphenylphosphino)-9,9-dimethylxanthene (80.8 mg, 139 µmol), and potassium phosphate (444 mg, 2.1 mmol) under nitrogen atmosphere, gradually heated to 110°C, and reacted for 12 hours. TLC (PE: EA = 1:1) showed that the starting material was completely reacted. The reaction mixture was cooled to 25°C, poured into saturated ammonium chloride (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (EA: PE = 2:1) to obtain compound **13-6** (200 mg, Y: 59%). LC-MS m/z (ESI): 484 [M+H]⁺.

Step 7: Oxalyl chloride (43.5 mg, 321 µmol) was dissolved in dichloromethane (1 mL). The reaction mixture was cooled to -70°C under nitrogen atmosphere and stirred for 30 minutes, added with dimethyl sulfoxide (53 mg, 650 µmol) and stirred at -70°C for 30 minutes, then sequentially added with compound **13-6** (110 mg, 228 µmol) and triethylamine (135 mg, 1.20 mmol) and stirred for 30 minutes, gradually warmed to 25°C, poured into saturated ammonium chloride (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **13-7** (100 mg, crude).

Step 8: Compound **13-7** (100 mg, 206 µmol) was dissolved in dichloroethane (1 mL). The reaction mixture was sequentially added with trans-4-aminocyclohexanol (47.8 mg, 414 µmol) and acetic acid (1.2 mg, 20.6 µmol) under nitrogen atmosphere and stirred at 85°C for 30 minutes, then added with sodium triacetoxyborohydride (7.84 mg, 207 µmol) and stirred for 30 minutes, gradually cooled to 25°C, poured into saturated sodium bicarbonate (5 mL) to quench, and extracted with ethyl acetate (5 mL × 3). The organic phases were combined, washed with saturated brine (10 mL), dried over anhydrous sodium sulfate, and filtered. The filtrate was concentrated under reduced pressure to obtain a yellow oil, which was then purified by preparative high-pressure liquid chromatography (column model: Xselect CSH Prep Fluoro-Phenyl, 150 * 19 mm, 5 µm; eluent: 0.05% NH₃·H₂O+10 mmol/L NH₄HCO₃/CH₃CN+MeOH = 1:1; gradient: 17% to 37%; flow rate: 20 mL/min; run time: 11 min; retention time: 7.5 min for **13b** and 6.1 min for **13a**) to obtain compound **13a** (10 mg, Y: 8%) and compound **13b** (4 mg, Y: 3%). **13a:** ¹H NMR (400 MHz, DMSO-*d*₆) δ 8.28-8.18 (m, 1H), 7.45-7.20 (m, 5H), 7.10-6.90 (m, 2H), 5.16-5.10 (m, 1H), 4.42 (s, 1H), 3.50-3.46 (m, 2H), 2.96-2.72 (m, 4H), 2.60 (d, *J* = 4.5 Hz, 3H), 2.25-2.17 (m, 1H), 1.78-1.62 (m, 4H), 1.30-0.85 (m, 5H), 0.40-0.30 (m, 1H). LC-MS m/z (ESI): 581.2 [M+H]⁺. **13b:** ¹H NMR (400 MHz, DMSO-*d*₆) δ 8.15-8.03 (m, 1H), 7.50-7.28 (m, 5H), 7.12-6.85 (m, 2H), 5.20-5.14 (m, 1H), 3.50-3.36 (m, 2H), 3.05-2.82 (m, 4H), 2.12-1.98 (m, 4H), 1.82-1.68(m, 3H), 1.40-0.80 (m, 5H), 0.42-0.25 (m, 1H). LC-MS m/z (ESI): 581.2 [M+H]⁺.

### Example 14

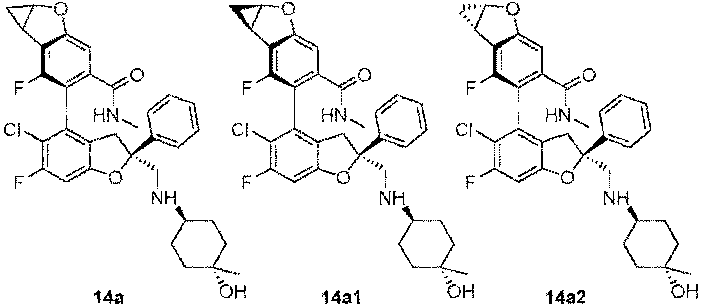

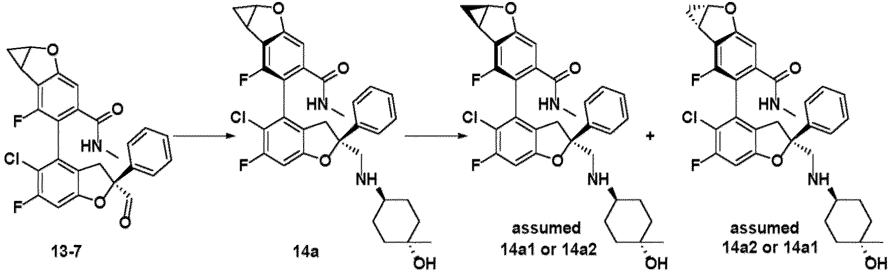

Step 1: Compound **13-7** (70 mg, 145 µmol) was dissolved in dichloroethane (1 mL). The reaction mixture was sequentially added with trans-4-amino-1-methylcyclohexanol (129 mg, 290 µmol) and acetic acid (0.8 mg, 14.5 µmol) under nitrogen atmosphere and stirred at 85°C for 30 minutes, then added with sodium triacetoxyborohydride (10.9 mg, 290 µmol) and stirred for 30 minutes, gradually cooled to 25°C, poured into saturated sodium bicarbonate (5 mL) to quench, and extracted with ethyl acetate (5 mL × 3). The organic phases were combined, washed with saturated brine (10 mL), dried over anhydrous sodium sulfate, and filtered. The filtrate was concentrated under reduced pressure to obtain a yellow oil, which was then purified by preparative high-pressure liquid chromatography (column model: Xselect CSH Prep Fluoro-Phenyl, 150 * 19 mm, 5 µm; eluent: 0.05% FA/CH₃CN; gradient: 39% to 59%; flow rate: 20 mL/min; run time: 10 min; retention time: 8.0 min) to obtain compound **14a** (7 mg, Y: 8%). ¹H NMR (400 MHz, DMSO-*d*₆) δ 8.23 (s, 1H), 8.13 (s, 1H), 7.48-7.26 (m, 6H), 7.10-7.00 (m, 1H), 6.95 (s, 1H), 5.17-5.11 (m, 1H), 4.10 (s, 1H), 3.53-3.45 (m, 2H), 3.03-2.93 (m, 2H), 2.88-2.80 (m, 3H), 2.58 (d, *J* = 4.6 Hz, 3H), 1.70-1.60 (m, 2H), 1.45-1.35 (m, 2H), 1.30-1.10 (m, 5H), 1.08-0.90 (m, 3H), 0.38-0.32 (m, 1H). LC-MS m/z (ESI): 595.2 [M+H]⁺.

Step 2: Compound **14a** (24 mg, 40.3 µmol) was subjected to chiral resolution (chiral column model: Cellulose-SB; eluent: carbon dioxide/(methanol+20 mM NH₃); gradient: 10%; flow rate: 3.0 mL/min; run time: 4 min) to obtain compound **14a1** (7.0 mg, Y: 29%, RT = 1.89 min) and compound **14a2** (5.0 mg, Y: 20%, RT = 2.12 min). **14a1:** LC-MS m/z (ESI): 595.3 [M+H]⁺. **14a2:** LC-MS m/z (ESI): 595.3 [M+H]⁺.

### Example 15

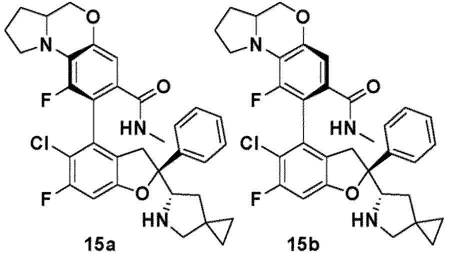

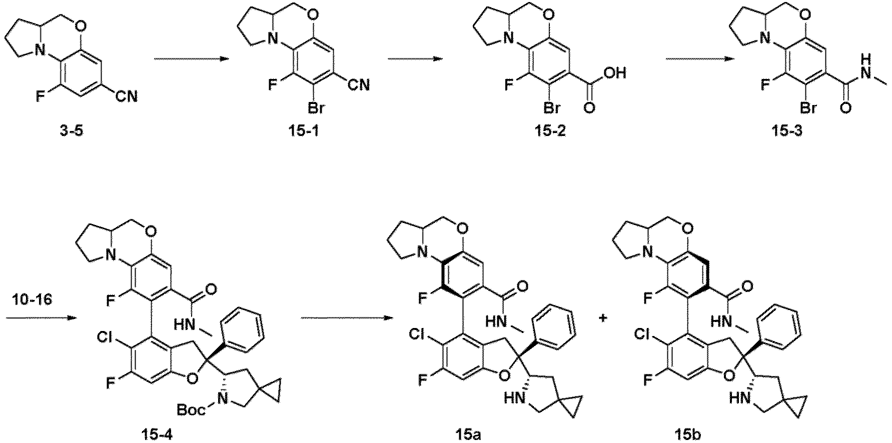

Step 1: Compound **3-5** (500 mg, 2.29 mmol) was dissolved in anhydrous tetrahydrofuran (10 mL). The reaction mixture was cooled to -70°C under nitrogen atmosphere, and added dropwise with lithium diisopropylamide (1 M, 2.75 mL, 2.75 mmol). After the dropwise addition was completed, the reaction mixture was reacted at -70°C for 0.5 hours, added dropwise with a solution of 1,2-dibromotetrachloroethane (903 mg, 3.44 mmol) in tetrahydrofuran (2 mL), reacted at -70°C for 0.5 hours, then gradually warmed to 25°C, and reacted for 2 hours. LCMS monitored that the starting material was completely reacted. The reaction mixture was cooled to 0°C, added with saturated ammonium chloride solution (10 mL) to quench, and extracted with ethyl acetate (10 mL × 3). The organic phases were combined, washed with saturated brine (10 mL), dried over anhydrous sodium sulfate, and filtered. The filtrate was concentrated under reduced pressure and then purified by silica gel column chromatography (PE: EA = 4:1) to obtain compound **15-1** (550 mg, Y: 73%). LC-MS m/z (ESI): 297 [M+H]⁺.

Step 2: Compound **15-1** (500 mg, 1.68 mmol) was dissolved in ethanol (5 mL) and water (5 mL), then sodium hydroxide (673 mg, 16.8 mmol) was added thereto, and the reaction mixture was reacted at 100°C for 16 hours. LCMS monitored that the starting material was completely reacted. The reaction mixture was cooled to 25°C, added with water (10 mL), then added with 1 M hydrochloric acid to adjust the pH to 5, and extracted with ethyl acetate (10 mL × 3). The organic phases were combined, washed with saturated brine (10 mL), dried over anhydrous sodium sulfate, filtered, and the filtrate was concentrated under reduced pressure to obtain compound **15-2** (450 mg, crude). LC-MS m/z (ESI): 316 [M+H]⁺.

Step 3: Compound **15-2** (430 mg, 1.3 mmol) was dissolved in dichloromethane (10 mL), then methylamine hydrochloride (185 mg, 2.7 mmol), diisopropylethylamine (1.2 g, 9.5 mmol), and 2-(7-azabenzotriazol-1-yl)-N,N,N',N'-tetramethyluronium hexafluorophosphate (734 mg, 1.9 mmol) were sequentially added thereto, and the reaction mixture was reacted at 25°C for 1 hour. LCMS monitored that the starting material was completely reacted. The reaction mixture was added with water (10 mL), and extracted with ethyl acetate (10 mL × 3). The organic phases were combined, washed with saturated brine (10 mL), dried over anhydrous sodium sulfate, and filtered. The filtrate was concentrated under reduced pressure and then purified by silica gel column chromatography (PE: EA = 2:1) to obtain compound **15-3** (400 mg, Y: 89%). ¹H NMR (400 MHz, DMSO-*d₆*) δ 8.17-8.12 (m, 1H), 6.78-6.73 (m, 1H), 4.42-4.36 (m, 1H), 3.85-3.77 (m, 1H), 3.20-3.12 (m, 1H), 2.75-2.65 (m, 5H), 2.18-2.04 (m, 1H), 1.95-1.79 (m, 2H), 1.60-1.45 (m, 1H).

Step 4: Compound **15-3** (192 mg, 0.58 mmol) and compound **10-16** (350 mg, 0.61 mmol) were dissolved in toluene (5 mL) and water (1 mL), then tris(dibenzylideneacetone)dipalladium (107 mg, 0.12 mmol), 4,5-bis(diphenylphosphino)-9,9-dimethylxanthene (135 mg, 0.23 mmol), and potassium phosphate (372 mg, 1.75 mmol) were added thereto under nitrogen atmosphere, and the reaction mixture was reacted at 100°C for 24 hours under nitrogen atmosphere. LCMS monitored that the starting material was completely reacted. The reaction mixture was cooled to 25°C, added with water (5 mL), and extracted with ethyl acetate (10 mL × 3). The organic phases were combined, washed with saturated brine (10 mL), dried over anhydrous sodium sulfate, and filtered. The filtrate was concentrated under reduced pressure and then purified by silica gel column chromatography (PE: EA = 3:1) to obtain compound **15-4** (120 mg, 29%). LC-MS m/z (ESI): 692 [M+H]⁺.

Step 5: Compound **15-4** (138 mg, 0.20 mmol) was dissolved in dichloromethane (5 mL), then trifluoroacetic acid (1 mL) was added thereto, and the reaction mixture was reacted at 25°C for 1 hour. LCMS monitored that the starting material was completely reacted. The reaction mixture was added with water (5 mL), then added with saturated sodium bicarbonate to adjust the pH to 8, and extracted with ethyl acetate (5 mL × 3). The organic phases were combined, washed with saturated brine (10 mL), dried over anhydrous sodium sulfate, and filtered. The filtrate was concentrated under reduced pressure, then purified by preparative high-pressure liquid chromatography (column model: Xbridge Prep Phenyl OBD, 150 * 19 mm, 5 µm; eluent: 0.05% NH₃·H₂O+10 mmol/L NH₄HCO₃/CH₃CN+MeOH = 1:1; gradient: 12% to 32%; flow rate: 20 mL/min; run time: 11 min; retention time: 7.9 min for **15a** and 6.7 min for **15b**), and lyophilized to obtain compound **15a** (8.1 mg, Y: 6%) and compound **15b** (12.7 mg, Y: 10%). **15a:** ¹H NMR (400 MHz, DMSO-*d₆*) δ 7.87 (s, 1H), 7.49-7.22 (m, 5H), 7.04-6.91 (m, 2H), 4.50-4.38 (m, 1H), 3.76-3.68 (m, 2H), 3.54-3.36 (m, 3H), 3.22-3.06 (m, 1H), 3.00-2.81 (m, 1H), 2.70-2.62 (m, 2H), 2.62-2.57 (m, 3H), 2.20-1.75 (m, 3H), 1.68-1.25 (m, 4H), 0.45-0.20 (m, 4H). LC-MS m/z (ESI): 592 [M+H]⁺. **15b:** ¹H NMR (400 MHz, DMSO-*d₆*) δ 8.02-7.90 (m, 1H), 7.50-7.40 (m, 2H), 7.38-7.20 (m, 3H), 7.05-6.85 (m, 2H), 4.50-4.32 (m, 1H), 3.90-3.65 (m, 2H), 3.51-3.36 (m, 3H), 3.27-3.00 (m, 2H), 2.77-2.57 (m, 2H), 2.35-2.20 (m, 3H), 2.20-2.06 (m, 1H), 2.09-1.82 (m, 2H), 1.65-1.20 (m, 4H), 0.47-0.20 (m, 4H). LC-MS m/z (ESI): 592 [M+H]⁺.

### Example 16

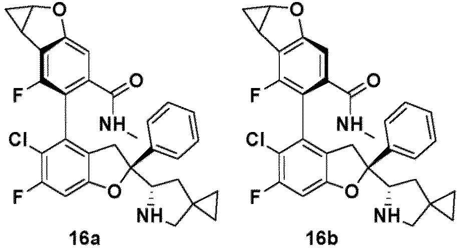

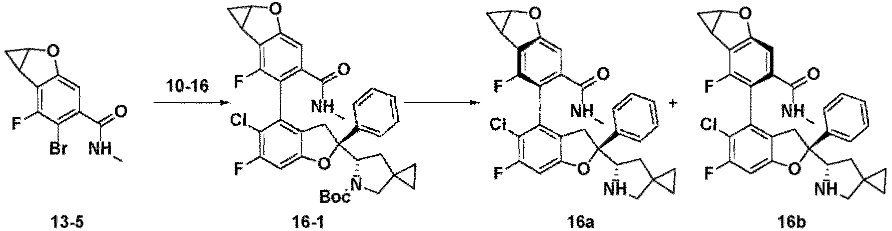

Step 1: Compound **13-5** (100 mg, 0.35 mmol), compound **10-16** (338 mg, 0.59 mmol), tris(dibenzylideneacetone)dipalladium (64 mg, 0.07 mmol), 4,5-bis(diphenylphosphino)-9,9-dimethylxanthene (81 mg, 0.14 mmol), and potassium carbonate (145 mg, 1.05 mmol) were dissolved in toluene (5 mL) and water (1 mL), and the reaction mixture was reacted at 120°C for 12 hours under nitrogen atmosphere. LCMS monitored that the reaction was completed. The reaction mixture was added with water (5 mL), and extracted with ethyl acetate (5 mL × 3). The organic phases were combined, washed with saturated brine (10 mL), dried over anhydrous sodium sulfate, and filtered. The filtrate was concentrated under reduced pressure and then purified by silica gel column chromatography (PE: EA = 7:3) to obtain compound **16-1** (50 mg, Y: 22%). LC-MS m/z (ESI): 649 [M+H]⁺.

Step 2: Compound **16-1** (95 mg, 0.15 mmol), dichloromethane (5 mL), and trifluoroacetic acid (1 mL) were reacted at room temperature for 1 hour. LCMS monitored that the reaction was completed. The reaction mixture was added with water (5 mL), then added with saturated sodium bicarbonate to adjust the pH to 8, and extracted with ethyl acetate (5 mL × 3). The organic phases were combined, washed with saturated brine (10 mL), dried over anhydrous sodium sulfate, and filtered. The filtrate was concentrated under reduced pressure, then purified by preparative high-pressure liquid chromatography (column model: Xselect CSH Prep Fluoro-Phenyl, 150 * 19 mm, 5 µm; eluent: 0.05% FA/CH₃CN; gradient: 48% to 68%; flow rate: 20 mL/min; run time: 12 min; retention time: 7.9 min for **16a** and 6.9 min for **16b**), and lyophilized to obtain compound **16a** (9.8 mg, Y: 12%) and compound **16b** (18.2 mg, Y: 22%). **16a:** ¹H NMR (400 MHz, DMSO-*d₆*) δ 8.16-8.08 (m, 1H), 7.48-7.40 (m, 2H), 7.38-7.22 (m, 3H), 7.06-6.96 (m, 2H), 5.20-5.13 (m, 1H), 3.79-3.72 (m, 1H), 3.53-3.45 (m, 1H), 2.95-2.85 (m, 2H), 2.70-2.60 (m, 2H), 2.60-2.54 (m, 3H), 1.61-1.52 (m, 1H), 1.43-1.20 (m, 2H), 0.40-0.28 (m, 5H). LC-MS m/z (ESI): 549 [M+H]⁺. **16b:** ¹H NMR (400 MHz, DMSO-*d₆*) δ 8.16-8.08 (m, 1H), 7.50-7.28 (m, 5H), 7.10-6.90 (m, 2H), 5.18-5.16 (m, 1H), 3.50-3.32 (m, 3H), 3.17-3.06 (m, 1H), 2.95-2.61 (m, 4H), 2.29-2.15 (m, 3H), 1.80-1.07 (m, 3H), 0.58-0.30 (m, 5H). LC-MS m/z (ESI): 549 [M+H]⁺.

### Example 17

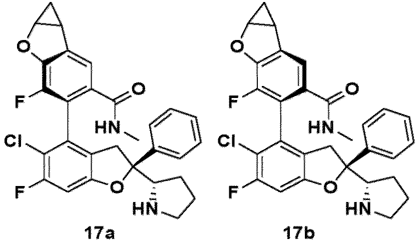

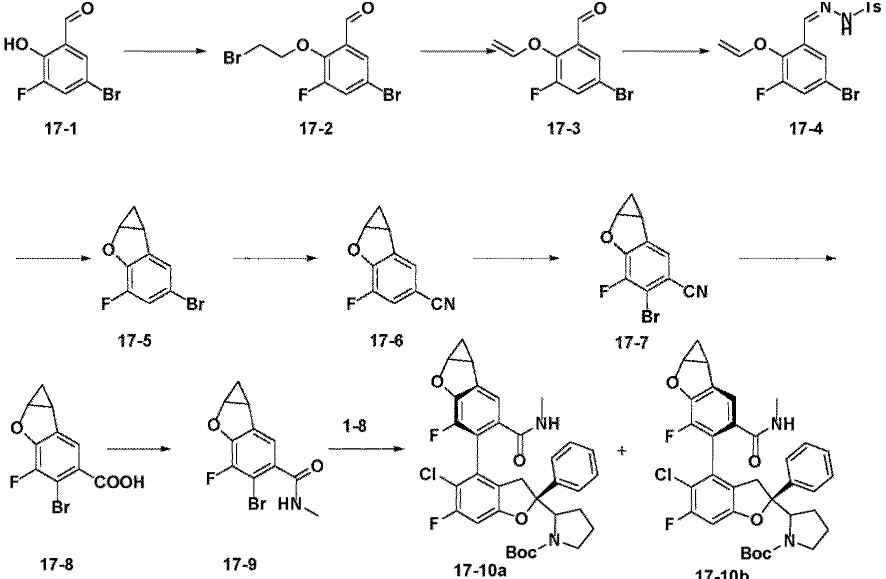

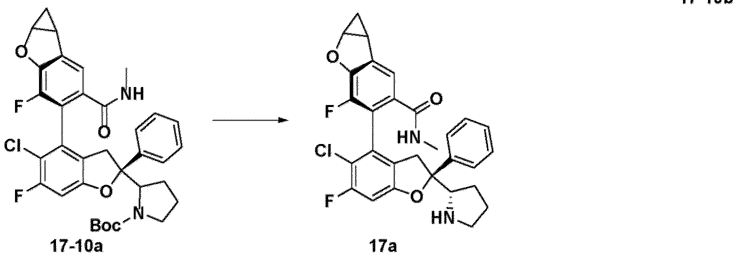

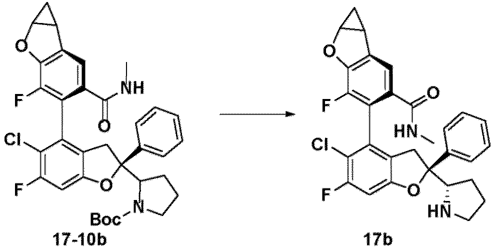

Step 1: Compound **17-1** (200 g, 913.21 mmol) was dissolved in acetonitrile (3 L), and potassium carbonate (252.40 g, 1.83 mol) was added thereto in batches at room temperature. After the addition was completed, the reaction mixture was slowly added dropwise with 1,2-dibromoethane (856 g, 4.56 mol) and reacted at 80°C for 12 hours. TLC (PE: EA = 10:1) showed that the starting material was completely reacted. The reaction mixture was cooled to room temperature, filtered under reduced pressure, and the filter cake was washed with acetonitrile (300 mL × 3). The filtrate was collected, concentrated under reduced pressure, and purified by silica gel column chromatography (EA: PE = 1:10) to obtain compound **17-2** (201.4 g, Y: 67%).

LC-MS m/z (ESI): 325.0, 327.0 [M+1]⁺.

Step 2: Compound **17-2** (110 g, 337.47 mmol) was dissolved in tetrahydrofuran (1.5 L). The reaction mixture was cooled to -30°C, slowly added dropwise with a solution of potassium tert-butoxide (676.90 mL, 674.94 mmol) in tetrahydrofuran, and reacted at -30°C for 2 hours. TLC (PE: EA = 10:1) showed that the starting material was completely reacted. The reaction mixture was added dropwise with saturated ammonium chloride aqueous solution (400 mL) at -30°C to quench, and extracted with ethyl acetate (500 mL × 3). The organic phases were combined, washed with saturated brine (100 mL × 3), dried over anhydrous sodium sulfate, filtered, and the filtrate was concentrated under reduced pressure to obtain compound **17-3** (95.2 g, crude). LC-MS m/z (ESI): 246.1 [M+H]⁺.

Step 3: Compound **17-3** (95.2 g, 388.5 mmol) was dissolved in tetrahydrofuran (1 L), then 4-methylbenzenesulfonhydrazide (72.3 g, 388.50 mmol) was added thereto in batches, and the reaction mixture was reacted at 70°C for 12 hours. TLC (PE: EA = 1:1) showed that the starting material was completely reacted. The reaction mixture was cooled to room temperature, added dropwise with saturated ammonium chloride (400 mL) to quench, and extracted with ethyl acetate (400 mL × 3). The organic phases were combined, washed with saturated brine (100 mL × 3), dried over anhydrous sodium sulfate, and filtered. The filtrate was concentrated under reduced pressure and then purified by silica gel column chromatography (EA: PE = 1:1) to obtain compound **17-4** (59.0 g, Y: 36%). LC-MS m/z (ESI): 415.1 [M+H]⁺.

Step 4: Compound **17-4** (47.5 g, 114.9 mmol) was dissolved in toluene (500 mL). The reaction mixture was added with sodium hydride (5.07 g, 211.25 mmol) in batches, stirred for 5 minutes, then added with rhodium(II) acetate dimer (1.53 g, 3.45 mmol), and reacted at 100°C for 12 hours. TLC (PE: EA = 20:1) showed that the starting material was completely reacted. The reaction mixture was cooled to room temperature, added dropwise with water (200 mL) to quench, and extracted with ethyl acetate (300 mL × 3). The organic phases were combined, washed with saturated brine (100 mL × 3), dried over anhydrous sodium sulfate, and filtered. The filtrate was concentrated under reduced pressure and then purified by silica gel column chromatography (EA: PE = 1:20) to obtain compound **17-5** (18.50 g, Y: 70%).

Step 5: Compound **17-5** (18.50 g, 80.77 mmol) and zinc cyanide (5.69 g, 48.46 mmol) were dissolved in N,N-dimethylacetamide (200 mL), then tris(dibenzylideneacetone)dipalladium (1.67 g, 1.62 mmol), 1,1'-bis(diphenylphosphino)ferrocene (1.80 g, 3.23 mmol), and zinc powder (0.53 g, 8.08 mmol) were sequentially added thereto under nitrogen atmosphere, and the reaction mixture was gradually heated to 120°C and reacted for 6 hours. TLC (PE: EA = 10:1) showed that the starting material was completely reacted. The reaction mixture was cooled to room temperature, added with water (200 mL) to quench, and extracted with ethyl acetate (300 mL × 3). The organic phases were combined, washed with saturated brine (100 mL × 3), dried over anhydrous sodium sulfate, and filtered. The filtrate was concentrated under reduced pressure and then purified by silica gel column chromatography (EA: PE = 1:10) to obtain compound **17-6** (11.00 g, Y: 77%). ¹H NMR (400 MHz, CDCl₃) δ 7.40 (d, *J* = 1.6 Hz, 1H), 7.22-7.19 (m, 1H), 5.05-5.02 (m, 1H), 2.72-2.67 (m, 1H), 1.23-1.18 (m, 1H), 0.46-0.42 (m, 1H).

Step 6: Compound **17-6** (11.00 g, 62.80 mmol) was dissolved in tetrahydrofuran (110 mL). The reaction mixture was cooled to -70°C, slowly added dropwise with lithium diisopropylamide (34.57 mL, 69.08 mmol), stirred at -70°C for 0.5 hours, and then added with 1,2-dibromotetrachloroethane (19.80 g, 75.36 mmol) in batches. After the addition was completed, the reaction mixture was warmed from -70°C to room temperature and reacted for 2 hours. TLC (PE: EA = 5:1) showed that the starting material was completely reacted. The reaction mixture was added with saturated ammonium chloride (100 mL) to quench, and extracted with ethyl acetate (200 mL × 3). The organic phases were combined, washed with saturated brine (100 mL × 3), dried over anhydrous sodium sulfate, and filtered. The filtrate was concentrated under reduced pressure and then purified by silica gel column chromatography (EA: PE = 1:5) to obtain compound **17-7** (9.30 g, Y: 48%). ¹H NMR (400 MHz, CDCl₃) δ 7.42 (s, 1H), 5.06-5.00 (m, 1H), 2.73-2.66 (m, 1H), 1.24-1.17 (m, 1H), 0.47-0.41 (m, 1H).

Step 7: Compound **17-7** (6.15 g, 24.21 mmol) was dissolved in ethanol (70 mL), then sodium hydroxide (4.84 g, 121.04 mmol) was added thereto in batches, and the reaction mixture was reacted at 100°C for 6 hours. TLC (PE: EA = 5:1) showed that the starting material was completely reacted. The reaction mixture was cooled to room temperature, added with 2 M hydrochloric acid to adjust the pH to 3, and extracted with ethyl acetate (200 mL × 3). The organic phases were combined, washed with saturated brine (100 mL × 3), dried over anhydrous sodium sulfate, and filtered. The filtrate was concentrated under reduced pressure and then purified by silica gel column chromatography (EA: PE = 5:1, with 2% glacial acetic acid) to obtain compound **17-8** (3.90 g, Y: 58%).

Step 8: Compound **17-8** (3.86 g, 14.14 mmol) was dissolved in N,N-dimethylformamide (40 mL). The reaction mixture was cooled to 0°C, sequentially added with diisopropylethylamine (6.38 g, 49.48 mmol) and 2-(7-azabenzotriazol-1-yl)-N,N,N',N'-tetramethyluronium hexafluorophosphate (5.91 g, 15.55 mmol), reacted at 0°C for 0.5 hours, and then added with methylamine hydrochloride (1.91 g, 28.27 mmol). After the addition was completed, the reaction mixture was reacted at 0°C for 2 hours. TLC (PE: EA = 1:1) showed that the starting material was completely reacted. The reaction mixture was added with water (100 mL) to quench, and extracted with ethyl acetate (200 mL). The organic phases were combined, washed with saturated brine (100 mL × 3), dried over anhydrous sodium sulfate, and filtered. The filtrate was concentrated under reduced pressure and then purified by silica gel column chromatography (EA: PE = 1:1) to obtain compound **17-9** (1.99 g, Y: 49%). LC-MS m/z (ESI): 286.1 [M+H]⁺.

Step 9: Compound **1-8** (385 mg, 708 µmol) and compound **17-9** (203 mg, 708 µmol) were dissolved in toluene (3 mL) and water (0.3 mL). The reaction mixture was sequentially added with tris(dibenzylideneacetone)dipalladium (58 mg, 70.88 µmol), 4,5-bis(diphenylphosphino)-9,9-dimethylxanthene (65 mg, 141.76 µmol), and potassium carbonate (232 mg, 141.76 µmol) under nitrogen atmosphere, gradually heated to 100°C, and reacted for 12 hours. LCMS showed that the starting material was completely reacted. The reaction mixture was cooled to room temperature, concentrated under reduced pressure, and then purified by a preparative silica gel plate (EA: PE = 1:1) to obtain compound **17-10a** (70.4 mg, Y: 19%, Rf = 0.3) and compound **17-10b** (105.6 mg, Y: 29%, Rf = 0.35). LC-MS m/z (ESI): 623.2 [M+H]⁺.

Step 10: Compound **17-10a** (70.4 mg, 0.11 mmol) was dissolved in a solution of 20% trifluoroacetic acid/dichloromethane (5 mL), and the reaction mixture was reacted at 5°C for 1 hour. TLC (DCM: MeOH = 10:1) showed that the starting material was completely reacted. The reaction mixture was cooled to 0°C, poured into saturated sodium carbonate (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O+10 mmol/L NH₄HCO₃/CH₃CN; gradient: 61% to 81%; flow rate: 20 mL/min; run time: 14 min; retention time: 8.0 min) to obtain compound **17a** (33.0 mg, Y: 55%). ¹H NMR (400 MHz, DMSO-*d₆*) δ 8.11-8.07 (m, 1H), 7.51 (s, 1H), 7.48-7.39 (m, 2 H), 7.38-7.22 (m, 3H), 6.98 (d, *J* = 9.6 Hz, 1H), 5.24-5.17 (m, 1H), 3.52-3.32 (m, 2H), 3.30-3.28 (m, 1H), 2.91-2.86 (m, 2H), 2.75-2.69 (m, 1H), 2.67-2.55 (m, 4H), 1.58-1.22 (m, 6H), 0.50-0.40 (m, 1H). LC-MS m/z (ESI): 523.3/525.3 [M+H]⁺.

Step 11: Compound **17-10b** (105.6 mg, 0.17 mmol) was dissolved in a solution of 20% trifluoroacetic acid/dichloromethane (5 mL), and the reaction mixture was reacted at 5°C for 1 hour. TLC (DCM: MeOH = 10:1) showed that the starting material was completely reacted. The reaction mixture was cooled to 0°C, poured into saturated sodium carbonate (10 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O+10 mmol/L NH₄HCO₃/CH₃CN; gradient: 59% to 79%; flow rate: 20 mL/min; run time: 13 min; retention time: 8.3 min) to obtain compound **17b** (35.0 mg, Y: 39%). ¹H NMR (400 MHz, DMSO-*d₆*) δ 8.15-8.10 (m, 1H), 7.47-7.41 (m, 3H), 7.35-7.27 (m, 3H), 6.95 (d, *J* = 9.6 Hz, 1H), 5.28-5.20 (m, 1H), 3.50-3.38 (m, 1H), 3.29-3.21 (m, 1H), 3.11-3.03 (m, 1H), 2.90-2.82 (m, 1H), 2.73-2.55 (m, 2H), 2.31-2.21 (m, 3H), 1.48-1.23 (m, 6H), 0.50-0.42 (m, 1H). LC-MS m/z (ESI): 523.3/525.3 [M+H]⁺.

### Example 18

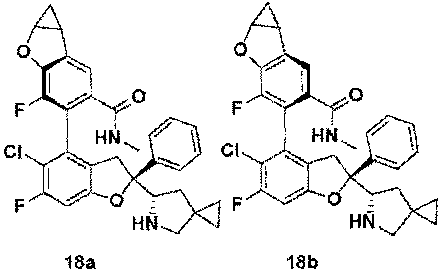

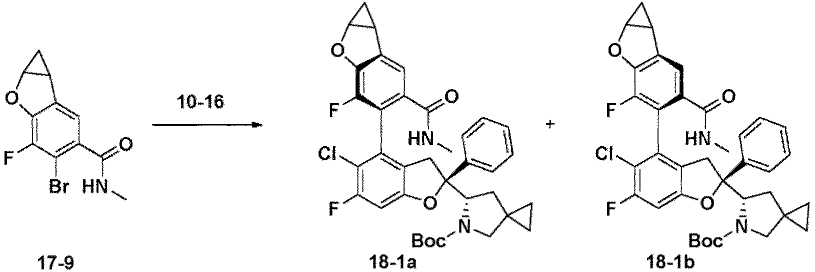

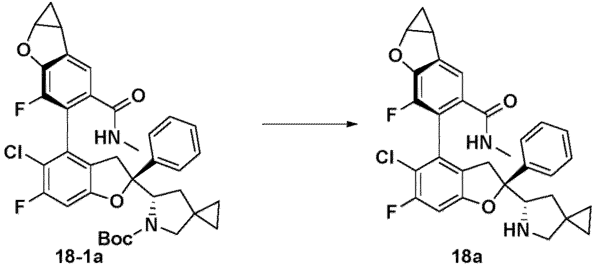

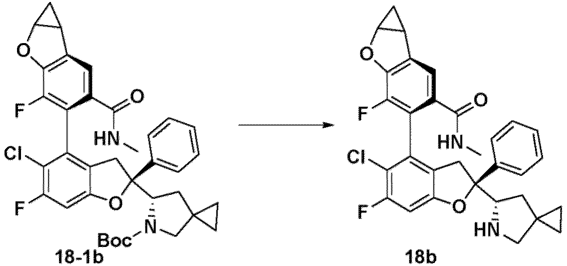

Step 1: Compound **17-9** (350 mg, 0.61 mmol) and compound **10-16** (192.5 mg, 0.68 mmol) were dissolved in toluene (3 mL) and water (0.6 mL), and tris(dibenzylideneacetone)dipalladium (56.2 mg, 0.06 mmol), 4,5-bis(diphenylphosphino)-9,9-dimethylxanthene (70.9 mg, 0.12 mmol), and potassium carbonate (212 mg, 1.53 mmol) were added thereto. After the addition was completed, the reaction mixture was heated to 100°C and reacted for 16 hours under nitrogen atmosphere. After the reaction was completed, the reaction mixture was concentrated under reduced pressure and purified by a preparative silica gel plate (EA: PE = 1:1) to obtain compound **18-1a** (50 mg, Y: 22%, Rf = 0.3) and compound **18-1b** (70 mg, Y: 31%, Rf = 0.35). LC-MS m/z (ESI): 649.2 [M+H]⁺.

Step 2: Compound **18-1a** (50 mg, 0.077 mmol) was dissolved in dichloromethane (5 mL), then trifluoroacetic acid (1 mL) was added thereto at 5°C, and the reaction mixture was reacted at 5°C for another 2 hours. TLC (PE: EA = 1:1) showed that the starting material was completely reacted. The reaction mixture was concentrated under reduced pressure and then purified by preparative high-pressure liquid chromatography (column model: YMC-Actus Triart C18, 150 * 20 mm, 5 µm; eluent: 0.05% NH₃·H₂O+10 mmol/L NH₄HCO₃/CH₃CN; gradient: 62% to 82%; flow rate: 20 mL/min; run time: 12 min; retention time: 7.9 min) to obtain compound **18a** (15 mg, Y: 35%). ¹H NMR (400 MHz, DMSO-*d₆*) δ 8.06 (d, *J* = 4.0 Hz, 1H), 7.58-7.50 (m, 1H), 7.48-7.40 (m, 2H), 7.38-7.24 (m, 3H), 7.02 (d, *J* = 9.6 Hz, 1H), 5.23-5.17 (m, 1H), 3.82-3.74 (m, 1H), 3.52-3.42 (m, 1H), 2.90-2.82 (m, 2H), 2.68 (s, 2H), 2.65-2.55 (m, 3H), 1.64-1.54 (m, 1H), 1.46-1.38 (m, 1H), 1.29-1.21 (m, 3H), 0.49-0.26 (m, 5H). LC-MS m/z (ESI): 549.3/551.3 [M+H]⁺.

Step 3: Compound **18-1b** (70 mg, 0.11 mmol) was dissolved in dichloromethane (5 mL), then trifluoroacetic acid (1 mL) was added thereto at 5°C, and the reaction mixture was reacted at 5°C for another 2 hours. TLC (PE: EA = 1:1) showed that the starting material was completely reacted. The reaction mixture was concentrated under reduced pressure and then purified by preparative high-pressure liquid chromatography (column model: YMC-Actus Triart C18, 150 * 20 mm, 5 µm; eluent: 0.05% NH₃·H₂O+10 mmol/L NH₄HCO₃/CH₃CN; gradient: 57% to 77%; flow rate: 20 mL/min; run time: 13 min; retention time: 7.7 min) to obtain compound 18b (13 mg, Y: 22%). ¹H NMR (400 MHz, DMSO-*d₆*) δ 8.16-8.11 (m, 1H), 7.48-7.41 (m, 3H), 7.35-7.27 (m, 3H), 6.95 (d*, J* = 9.6 Hz, 1H), 5.25-5.18 (m, 1H), 3.73-3.66 (m, 1H), 3.38-3.31 (m, 1H), 3.10-3.00 (m, 1H), 2.90-2.80 (m, 1H), 2.68-2.57 (m, 2H), 2.33-2.25 (m, 3H), 1.50-1.43 (m, 1H), 1.33-1.23 (m, 3H), 0.48-0.23 (m, 5H). LC-MS m/z (ESI): 549.3/551.3 [M+H]⁺.

### Example 19

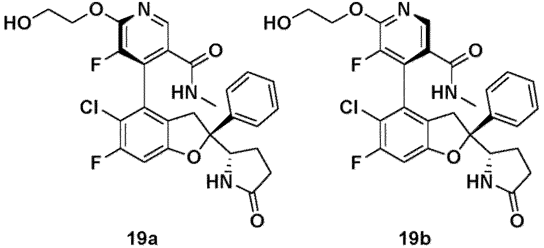

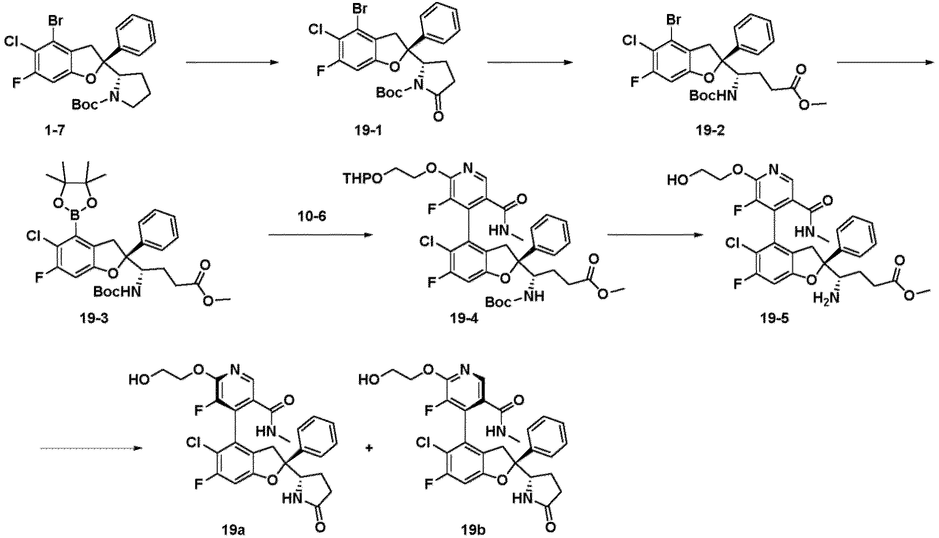

Step 1: Compound **1-7** (5.0 g, 10.0 mmol) was dissolved in ethyl acetate (50 mL), and the system was cooled to 0°C. The above reaction mixture was then slowly added dropwise with sodium periodate (10.8 g, 50.5 mmol) dissolved in water (50 mL) at 0°C, then added with ruthenium(III) chloride (620 mg, 3.0 mmol), and reacted at 25°C for 3 hours. LCMS showed that the starting material was completely reacted. The reaction mixture was added dropwise with ice-cold sodium sulfite aqueous solution (100 mL) to quench, and extracted with ethyl acetate (30 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:4) to obtain compound **19-1** (2.0 g, Y: 38%). LC-MS m/z (ESI): 532.0 [M+Na]⁺.

Step 2: Compound **19-1** (1.5 g, 2.9 mmol) was dissolved in anhydrous methanol (20 mL), and the system was cooled to 0°C. The above reaction mixture was then slowly added dropwise with sodium methoxide (0.7 mL, 3.5 mmol, 5 mol/L) at 0°C, and reacted at 0°C for 15 minutes. LCMS showed that the starting material was completely reacted. The reaction mixture was added with water (10 mL), then added with 1 M hydrochloric acid to adjust the pH to 7, and extracted with ethyl acetate (15 mL × 3). The organic phases were combined, washed with saturated brine (30 mL), dried over anhydrous sodium sulfate, and filtered. The filtrate was concentrated under reduced pressure and then purified by silica gel column chromatography (EA: PE = 1:4) to obtain compound **19-2** (1.2 g, Y: 76%). LC-MS m/z (ESI): 564.0 [M+Na]⁺.

Step 3: Compound **19-2** (1.4 g, 2.6 mmol) was dissolved in toluene (15 mL). The above reaction mixture was then added with bis(pinacolato)diboron (1.3 g, 5.16 mmol), anhydrous potassium acetate (0.5 g, 5.16 mmol), and [1,1'-bis(diphenylphosphino)ferrocene]palladium(II) dichloride (0.22 g, 0.26 mmol), and reacted at 100°C overnight under nitrogen atmosphere. LCMS showed that the starting material was completely reacted. The reaction mixture was cooled to 25°C, concentrated under reduced pressure, and purified by silica gel column chromatography (EA: PE = 1:9) to obtain 1.5 g of a crude product, which was then purified by reversed-phase low-pressure liquid chromatography (C18 column, water/CH₃CN, 85% CH₃CN to 90% CH₃CN) to obtain compound **19-3** (0.7 g, Y: 46%). LC-MS m/z (ESI): 612.0 [M+Na]⁺.

Step 4: Compound **19-3** (227 mg, 0.38 mmol) and compound **10-6** (100 mg, 0.3 mmol) were dissolved in toluene (3 mL) and water (0.5 mL), then tris(dibenzylideneacetone)dipalladium (55 mg, 0.06 mmol), 4,5-bis(diphenylphosphino)-9,9-dimethylxanthene (70 mg, 0.12 mmol), and anhydrous potassium carbonate (124 mg, 0.9 mmol) were sequentially added thereto, and the reaction mixture was reacted at 120°C overnight under nitrogen atmosphere. LCMS showed that the starting material was completely reacted. The reaction mixture was cooled to 25°C, concentrated under reduced pressure, and purified by silica gel column chromatography (THF: PE = 1:3) to obtain compound **19-4** (74 mg, Y: 32%). LC-MS m/z (ESI): 760.0 [M+H]⁺.

Step 5: Compound **19-4** (200 mg, 0.26 mol) was dissolved in dichloromethane (5 mL), then trifluoroacetic acid (1 mL) was added thereto, and the reaction mixture was reacted at room temperature for 2 hours. LCMS showed that the starting material was completely reacted. The reaction mixture was cooled to 0°C, poured into saturated sodium carbonate aqueous solution (10 mL) to quench, and extracted with ethyl acetate (10 mL × 3). The organic phases were combined, washed with saturated brine (10 mL), dried over anhydrous sodium sulfate, filtered, and the filtrate was concentrated under reduced pressure to obtain crude compound **19-5** (120 mg, crude). LC-MS m/z (ESI): 576.0 [M+H]⁺.

Step 6: Compound **19-5** (140 mg, 0.24 mol) was dissolved in anhydrous methanol (5 mL), then triethylamine (1 mL) was added thereto, and the reaction mixture was reacted at room temperature overnight. LCMS showed that the starting material was completely reacted. The reaction mixture was added with water (10 mL), and extracted with ethyl acetate (10 mL × 3). The organic phases were combined, washed with water (10 mL × 3), dried over anhydrous sodium sulfate, and filtered. The filtrate was concentrated under reduced pressure to obtain a yellow solid, which was then separated by preparative high-performance liquid chromatography (column model: Xselect CSH Prep Fluoro-Phenyl, 150 * 19 mm, 5 µm; eluent: 0.1% TFA/CH₃CN; gradient: 21% to 41%; flow rate: 20 mL/min; run time: 12 min; retention time: KG0126A: 8.5 min; KG026: 9.1 min). The fractions were collected separately, added with saturated sodium carbonate solution to adjust the pH to 8, and extracted with ethyl acetate (10 mL × 3). The organic phases were combined, washed with saturated brine (10 mL), dried over anhydrous sodium sulfate, and filtered. The filtrate was concentrated under reduced pressure and then lyophilized to obtain compound **19a** (55.0 mg, Y: 42%) and compound **19b** (60.3 mg, Y: 46%). **19a:** ¹H NMR (400 MHz, DMSO-*d₆*): δ 8.48-8.42 (m, 1H), 8.31 (s, 1H), 7.76 (s, 1H), 7.45-7.28 (m, 5H), 7.17 (d, *J* = 9.6 Hz, 1H), 4.51-4.39 (m, 2H), 4.05-3.95 (m, 1H), 3.85-3.70 (m, 2H), 3.50-3.40 (m, 2H), 3.05-2.95 (m, 1H), 2.72-2.63 (m, 3H), 2.10-1.79 (m, 3H), 1.68-1.52 (m, 1H). LC-MS m/z (ESI): 544.2 [M+H]⁺. **19b:** ¹H NMR (400 MHz, DMSO-*d₆*): δ 8.45-8.8.37 (m, 1H), 8.25 (s, 1H), 7.94 (s, 1H), 7.48-7.31 (m, 5H), 7.12 (d, *J* = 9.6 Hz, 1H), 5.03-4.95 (m, 1H), 4.55-4.38 (m, 2H), 4.05-3.95 (m, 1H), 3.90-3.75 (m, 2H), 3.40-3.25 (m, 1H), 3.20-3.05 (m, 1H), 2.42-2.30 (m, 3H), 1.97-1.65 (m, 3H), 1.57-1.40 (m, 1H). LC-MS m/z (ESI): 544.2 [M+H]⁺.

### Example 20

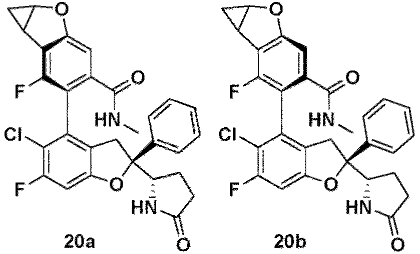

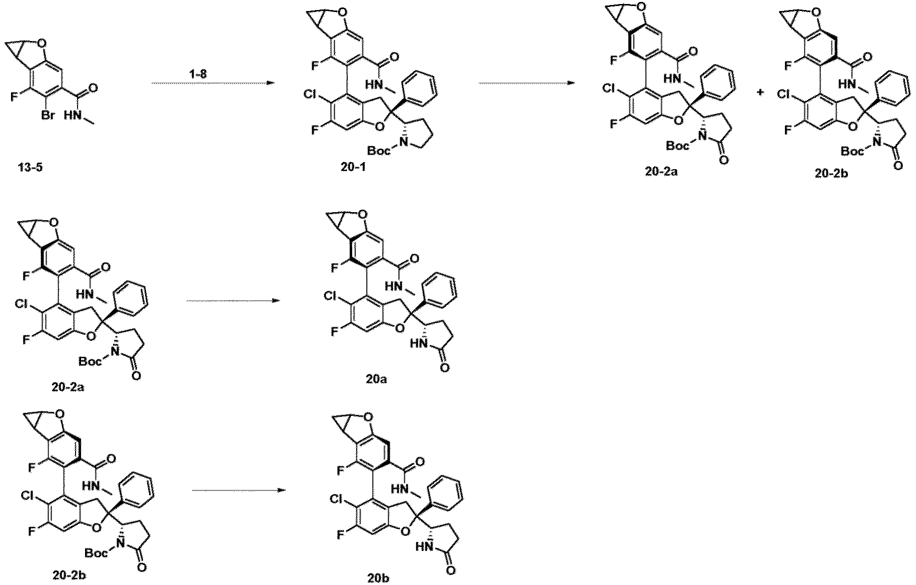

Step 1: Compound **13-5** (100 mg, 0.35 mmol) was dissolved in toluene (3 mL) and water (0.5 mL). The reaction mixture was sequentially added with compound **1-8** (240 mg, 0.44 mmol), tris(dibenzylideneacetone)dipalladium (64 mg, 0.07 mmol), 4,5-bis(diphenylphosphino)-9,9-dimethylxanthene (81 mg, 0.14 mmol), and anhydrous potassium carbonate (145 mg, 1.05 mmol), vacuumed and replaced with nitrogen three times, and reacted at 120°C overnight. LCMS showed that the starting material was completely reacted. The reaction mixture was cooled to 25°C, added with water (5 mL), and extracted with ethyl acetate (5 mL × 3). The organic phases were combined, washed with saturated brine (10 mL), dried over anhydrous sodium sulfate, and filtered. The filtrate was concentrated under reduced pressure and then purified by silica gel column chromatography (PE: EA = 7:3) to obtain compound **20-1** (74 mg, Y: 33%). LC-MS m/z (ESI): 623.3 [M+H]⁺.

Step 2: Compound **20-1** (210 mg, 0.34 mmol) was dissolved in ethyl acetate (5 mL). The reaction mixture was cooled to 0°C, added dropwise with a solution of sodium periodate (361 mg, 1.68 mmol) in water (5 mL) under nitrogen atmosphere, then added with ruthenium(III) chloride (14 mg, 0.07 mmol), warmed to 25°C, and reacted at 25°C for 3 hours. LCMS monitored that the reaction was completed. The reaction mixture was cooled to 0°C, added with saturated sodium sulfite solution (5 mL) to quench, and extracted with ethyl acetate (5 mL × 3). The organic phases were combined, washed with saturated brine (10 mL), dried over anhydrous sodium sulfate, and filtered. The filtrate was concentrated under reduced pressure and then purified by silica gel column chromatography (PE: EA = 1:1) to obtain compound **20-2a** (50.1 mg, Y: 23%, Rf = 0.4) and compound **20-2b** (48.5 mg, Y: 22%, Rf = 0.6).

Step 3: Compound **20-2a** (62 mg, 0.097 mmol) was dissolved in dichloromethane (2.5 mL), then trifluoroacetic acid (0.5 mL) was added dropwise thereto at 0°C, and the reaction mixture was reacted at room temperature for 2 hours. LCMS showed that the starting material was completely reacted. The reaction mixture was diluted with water (5 mL), then added with saturated sodium carbonate to adjust the pH to 8, and extracted with ethyl acetate (5 mL × 3). The organic phases were combined, washed with saturated brine (10 mL), dried over anhydrous sodium sulfate, and filtered. The filtrate was concentrated under reduced pressure and then purified by preparative high-pressure liquid chromatography (column model: Xbridge Prep Phenyl OBD, 150 * 19 mm, 5 µm; eluent: 0.05% NH₃·H₂O/CH₃CN; gradient: 25% to 45%; flow rate: 20 mL/min; run time: 12 min; retention time: 8.9 min) to obtain compound **20a** (10.5 mg, Y: 20%). ¹H NMR (400 MHz, DMSO-*d₆*) δ 8.21-8.10 (m, 1H), 7.78-7.67 (m, 1H), 7.45-7.30 (m, 5H), 7.11-7.08 (m, 1H), 6.99 (s, 1H), 5.20-5.11 (m, 1H), 4.02-3.91 (m, 1H), 3.48-3.40 (m, 1H), 3.00-2.81 (m, 2H), 2.62-2.58 (m, 3H), 2.06-1.80 (m, 3H), 1.65-1.50 (m, 1H), 1.30-1.20 (m, 1H), 0.41-0.30(m, 1H). LC-MS m/z (ESI): 537.2 [M+H]⁺.

Step 4: Compound **20-2b** (60 mg, 0.094 mmol) was dissolved in dichloromethane (2.5 mL), then trifluoroacetic acid (0.5 mL) was added dropwise thereto at 0°C, and the reaction mixture was reacted at room temperature for 2 hours. LCMS showed that the starting material was completely reacted. The reaction mixture was diluted with water (5 mL), then added with saturated sodium carbonate to adjust the pH to 8, and extracted with ethyl acetate (5 mL × 3). The organic phases were combined, washed with saturated brine (10 mL), dried over anhydrous sodium sulfate, and filtered. The filtrate was concentrated under reduced pressure and then purified by preparative high-pressure liquid chromatography (column model: Xbridge Prep Phenyl OBD, 150 * 19 mm, 5 µm; eluent: 0.05% NH₃·H₂O/CH₃CN; gradient: 54% to 74%; flow rate: 20 mL/min; run time: 12 min; retention time: 10.2 min) to obtain compound **20b** (15.4 mg, Y: 30%). ¹H NMR (400 MHz, DMSO-*d₆*) δ 8.19-8.07 (m, 1H), 7.98-7.90 (m, 1H), 7.49-7.30 (m, 5H), 7.10-7.03 (m, 1H), 6.95-6.90 (m, 1H), 5.22-5.12 (m, 1H), 4.05-3.95 (m, 1H), 3.16-3.12 (m, 1H), 2.98-2.87 (m, 1H), 2.70-2.61 (m, 1H), 2.31-2.23 (m, 3H), 2.05-1.65 (m, 3H), 1.58-1.40 (m, 1H), 1.34-1.20 (m, 1H), 0.48-0.33 (m, 1H). LC-MS m/z (ESI): 537.2 [M+H]⁺.

### Example 21

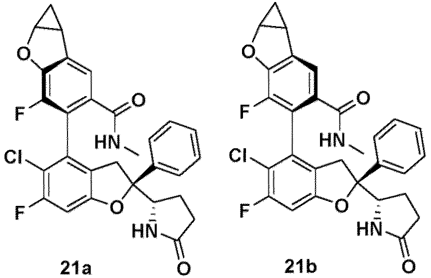

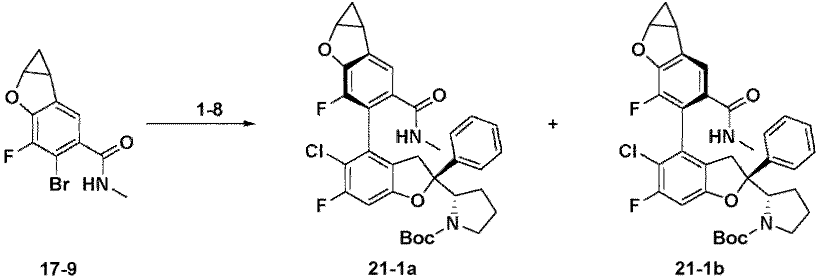

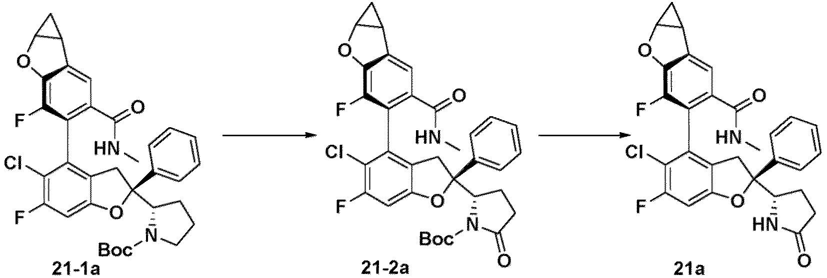

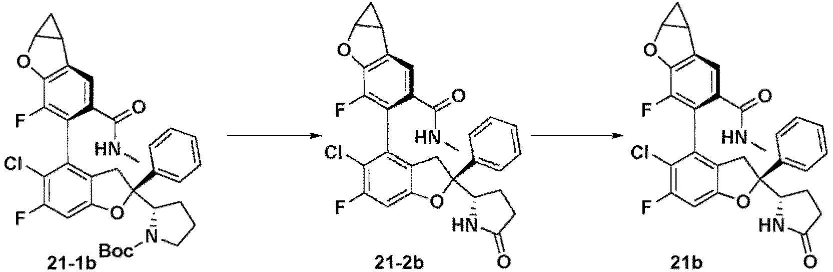

Step 1: Compound **17-9** (500 mg, 1.8 mmol) and compound **1-8** (1.2 g, 2.25 mmol) were dissolved in toluene (10 mL) and water (2 mL), then tris(dibenzylideneacetone)dipalladium (329 mg, 0.36 mmol), 4,5-bis(diphenylphosphino)-9,9-dimethylxanthene (415 mg, 0.74 mmol), and anhydrous potassium carbonate (729 mg, 5.4 mmol) were sequentially added thereto, and the reaction mixture was reacted at 120°C overnight under nitrogen atmosphere. LCMS showed that the starting material was completely reacted. The reaction mixture was cooled to 25°C, concentrated under reduced pressure, and then purified by silica gel column chromatography (EA: PE = 1:5) to obtain compound **21-1a** (140 mg, Y: 12%, Rf = 0.5) and compound **21-1b** (180 mg, Y: 16%, Rf = 0.4). LC-MS m/z (ESI): 645.2 [M+Na]⁺.

Step 2: Compound **21-1a** (140 mg, 0.22 mmol) was dissolved in ethyl acetate (4 mL), and the system was cooled to 0°C. The above reaction mixture was then slowly added dropwise with sodium periodate (305 mg, 1.1 mmol) dissolved in water (4 mL) at 0°C, then added with ruthenium(III) chloride (9 mg, 0.04 mmol), and reacted at 25°C for 3 hours. The reaction mixture was added dropwise with ice-cold sodium sulfite aqueous solution (200 mL) to quench, and extracted with ethyl acetate (100 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:1) to obtain compound **21-2a** (60 mg, Y: 42%) as a yellow solid. LC-MS m/z (ESI): 537.2 [M+H-Boc]⁺.

Step 3: Compound **21-2a** (60 mg, 0.09 mmol) was dissolved in dichloromethane (0.5 mL), then trifluoroacetic acid (0.1 mL) was added thereto, and the reaction mixture was reacted at room temperature for 2 hours. LCMS showed that the starting material was completely reacted. The reaction mixture was cooled to 0°C, poured into saturated sodium carbonate aqueous solution (10 mL) to quench, and extracted with ethyl acetate (10 mL × 3). The organic phases were combined, washed with saturated brine (10 mL), dried over anhydrous sodium sulfate, and filtered. The filtrate was concentrated under reduced pressure and then purified by preparative high-performance liquid chromatography (column model: Xbridge Prep Phenyl OBD, 150 * 19 mm, 5 µm; eluent: 0.05% NH₃·H₂O/CH₃CN; gradient: 60% to 70%; flow rate: 20 mL/min; run time: 15 min; retention time: 11.2 min) to obtain compound **21a** (13.7 mg, Y: 28.5%). ¹H NMR (400 MHz, DMSO-*d₆*): δ 8.18-8.14(m, 1H), 7.72-7.68 (m, 1H), 7.52 (s, 1H), 7.38-7.28 (m, 5H), 7.10-7.07 (m, 1H), 5.22-5.19 (m, 1H), 3.97-3.93 (m, 1H), 3.38-3.35 (m, 1H), 2.96-2.81 (m, 2H), 2.63-2.58 (m, 3H), 2.02-1.78 (m, 3H), 1.57-1.51 (m, 1H), 1.28-1.23 (m, 1H), 0.44-0.41 (m, 1H). LC-MS m/z (ESI): 537.2 [M+H]⁺.

Step 4: Compound **21-1b** (180 mg, 0.29 mmol) was dissolved in ethyl acetate (4 mL), and the system was cooled to 0°C. The above reaction mixture was then slowly added dropwise with sodium periodate (313 mg, 1.45 mmol) dissolved in water (4 mL) at 0°C, then added with ruthenium(III) chloride (12 mg, 0.06 mmol), and reacted at 25°C for 3 hours. The reaction mixture was added dropwise with ice-cold sodium sulfite aqueous solution (200 mL) to quench, and extracted with ethyl acetate (100 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:1) to obtain compound **21-2b** (65 mg, Y: 35%). LC-MS m/z (ESI): 537.2 [M+H-Boc]⁺.

Step 5: Compound **21-2b** (65 mg, 0.1 mmol) was dissolved in dichloromethane (0.5 mL), then trifluoroacetic acid (0.1 mL) was added thereto, and the reaction mixture was reacted at room temperature for 2 hours. LCMS showed that the starting material was completely reacted. The reaction mixture was cooled to 0°C, poured into saturated sodium carbonate aqueous solution (10 mL) to quench, and extracted with ethyl acetate (10 mL × 3). The organic phases were combined, washed with saturated brine (10 mL), dried over anhydrous sodium sulfate, and filtered. The filtrate was concentrated under reduced pressure to obtain a yellow solid, which was then purified by preparative high-performance liquid chromatography (column model: Xbridge Prep Phenyl OBD, 150 * 19 mm, 5 µm; eluent: 0.05% NH₃·H₂O/CH₃CN; gradient: 40% to 65%; flow rate: 20 mL/min; run time: 12 min; retention time: 10.5 min) to obtain compound **21b** (18.7 mg, Y: 34%). ¹H NMR (400 MHz, DMSO-*d₆*): δ 8.15-8.10(m, 1H), 7.92 (s, 1H), 7.48-7.32 (m, 6H), 7.05 (d, *J* = 9.6 Hz, 1H), 5.26-5.23 (m, 1H), 3.99-3.96 (m, 1H), 3.28-3.23 (m, 1H), 3.11-3.05 (m, 1H), 2.86-2.82 (m, 1H), 2.31-2.27 (m, 3H), 1.95-1.65 (m, 3H), 1.47-1.41 (m, 1H), 1.32-1.28 (m, 1H), 0.45-0.41 (m, 1H). LC-MS m/z (ESI): 537.2 [M+H]⁺.

### Example 22

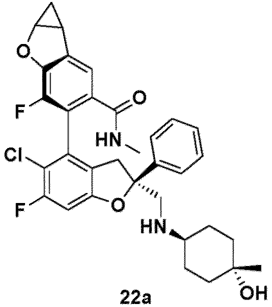

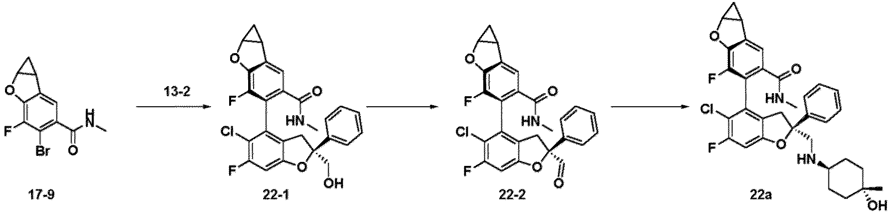

Step 1: Compound **17-9** (50 mg, 0.17 mmol) was dissolved in toluene (1 mL) and water (0.2 mL), then compound **13-2** (106 mg, 0.26 mmol), tris(dibenzylideneacetone)dipalladium (47.9 mg, 0.052 mmol), 4,5-bis(diphenylphosphino)-9,9-dimethylxanthene (60.6 mg, 0.10 mmol), and anhydrous potassium phosphate (111 mg, 0.52 mmol) were sequentially added thereto under nitrogen atmosphere, and the reaction mixture was reacted at 110°C for 16 hours. TLC (PE: EA = 1:1) showed that the starting material was completely reacted. 8 batches were combined for processing according to the same operation as above. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1, Rf = 0.3 for product, Rf = 0.36 for isomer) to obtain compound **22-1** (60.0 mg, Y: 9%). ¹H NMR (400 MHz, DMSO-*d₆*) δ 8.14-8.06 (m, 1H), 7.53-7.24 (m, 6H), 7.02 (d, *J* = 5.0 Hz, 1H), 5.24-5.18 (m, 2H), 3.64-3.59 (m, 2H), 2.89-2.81 (m, 1H), 2.78-2.70 (m, 1H), 2.61 (d, *J* = 2.4 Hz, 3H), 0.88-0.80 (m, 1H), 0.48-0.39 (m, 1H). LC-MS m/z (ESI): 484.2 [M+H]⁺.

Step 2: Oxalyl chloride (47.2 mg, 0.371 mmol) was dissolved in dichloromethane (3 mL). The reaction mixture was cooled to -70°C under nitrogen atmosphere and stirred for 30 minutes, added with dimethyl sulfoxide (58.1 mg, 0.744 mmol) and stirred at -70°C for 30 minutes, then added with starting material **22-1** (60 mg, 0.124 mmol) dissolved in dichloromethane (3 mL) at -70°C and stirred for 30 minutes, added with triethylamine (113 mg, 1.12 mmol) and stirred for 30 minutes, and warmed to 25°C and stirred for 1 hour. The reaction mixture was poured into saturated ammonium chloride (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **22-2** (60 mg, crude).

Step 3: Compound **22-2** (60 mg, 0.125 mmol) was dissolved in 1,2-dichloroethane (4 mL). The reaction mixture was sequentially added with trans-4-atnino-1-methylcyclohexanol (32.2 mg, 0.25 mmol) and acetic acid (0.74 mg, 0.012 mmol) under nitrogen atmosphere, heated to 85°C and stirred for 30 minutes, then added with sodium triacetoxyborohydride (52.8 mg, 0.25 mmol), and stirred at 85°C for 2 hours. LCMS showed that the starting material was completely reacted. The reaction mixture was cooled to 25°C, poured into saturated sodium bicarbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05%NH₃·H₂O/CH₃CN; gradient: 55.6% to 75.6%; flow rate: 20 mL/min; run time: 12 min; retention time: 8.9 min) to obtain compound **22a** (18.4 mg, Y: 24%). ¹H NMR (400 MHz, DMSO-*d₆*) δ 8.25-8.15 (m, 1H), 7.52-7.18 (m, 6H), 7.03 (d, *J* = 4.8 Hz, 1H), 5.25-5.17 (m, 1H), 4.15-4.05 (m, 1H), 3.48-3.38 (m, 2H), 3.02-2.71 (m, 4H), 2.63 (d, *J* = 2.2 Hz, 3H), 1.72-1.58 (m, 2H), 1.48-1.38 (m, 2H), 1.35-1.15 (m, 4H), 1.12-1.04 (m, 2H), 1.01 (s, 3H), 0.45-0.38 (m, 1H). LC-MS m/z (ESI): 595.2 [M+H]⁺.

### Example 23

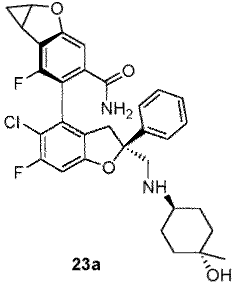

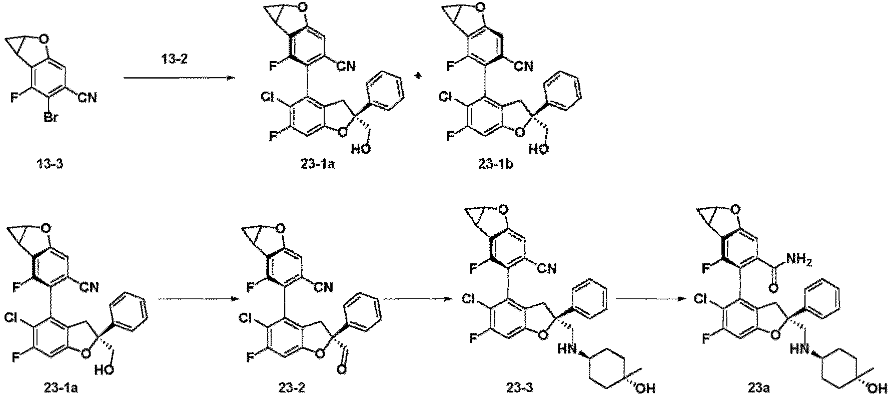

Step 1: Compound **13-3** (60 mg, 0.23 mmol) was dissolved in toluene (1 mL) and water (0.2 mL), then compound **13-2** (114 mg, 0.28 mmol), tris(dibenzylideneacetone)dipalladium (43.3 mg, 0.047 mmol), 4,5-bis(diphenylphosphino)-9,9-dimethylxanthene (54.6 mg, 0.094 mmol), and anhydrous potassium phosphate (150 mg, 0.71 mmol) were sequentially added thereto under nitrogen atmosphere, and the reaction mixture was reacted at 100°C for 16 hours. TLC (PE: EA = 1:1) showed that the starting material was completely reacted. The same operation as above was repeated 6 times, and 7 batches were combined and processed. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compounds **23-1a** (340 mg, Y: 45%, R_{f} = 0.35) and **23-1b** (100 mg, Y: 15%, R_{f} = 0.30). **23-1a:** LC-MS (1.5 mL-5 min-5-100-FA) Rt = 2.93, m/z (ESI): 474.1 [M+Na]⁺. **23-1b:** LC-MS (1.5 mL-5 min-5-100-FA) Rt = 2.90, m/z (ESI): 474.1 [M+Na]⁺.

Step 2: Oxalyl chloride (286 mg, 2.26 mmol) was dissolved in dichloromethane (3 mL). The reaction mixture was cooled to -78°C under nitrogen atmosphere and stirred for 30 minutes, added with dimethyl sulfoxide (352 mg, 4.51 mmol) and stirred at -78°C for 30 minutes, then added with starting material **23-1a** (340 mg, 0.752 mmol) dissolved in dichloromethane (3 mL) at -78°C and stirred for 30 minutes, added with triethylamine (685 mg, 6.77 mmol) and stirred for 30 minutes, and warmed to 25°C and stirred for 1 hour. The reaction mixture was poured into saturated ammonium chloride (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **23-2** (340 mg, crude).

Step 3: Compound **23-2** (340 mg, 0.75 mmol) was dissolved in 1,2-dichloroethane (4 mL). The reaction mixture was sequentially added with trans-4-amino-1-methylcyclohexanol (117 mg, 0.91 mmol) and acetic acid (4.54 mg, 0.075 mmol) under nitrogen atmosphere, heated to 85°C and stirred for 30 minutes, then added with sodium triacetoxyborohydride (57.1 mg, 1.51 mmol), and stirred at 85°C for 2 hours. LCMS showed that the starting material was completely reacted. The reaction mixture was cooled to 25°C, poured into saturated sodium bicarbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compound **23-3** (280 mg, Y: 65%). LC-MS m/z (ESI): 563.3 [M+H]⁺.

Step 4: Compound **23-3** (280 mg, 0.48 mmol) was dissolved in dimethyl sulfoxide (2 mL). The reaction mixture was sequentially added with anhydrous potassium carbonate (103 mg, 0.95 mmol) and 30% hydrogen peroxide (5.49 mg, 0.048 mmol) under nitrogen atmosphere, and stirred at 25°C for 3 hours. LCMS showed that the starting material was completely reacted. The reaction mixture was poured into saturated ammonium chloride (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O-10 mmol/L NH₄HCO₃-CAN; gradient: 55.9% to 75.9%; flow rate: 20 mL/min; run time: 10 min; retention time: 7.2 min) to obtain compound **23a** (48.4 mg, Y: 16%). ¹H NMR (400 MHz, DMSO-*d₆*) δ 7.67-7.58 (m, 1H), 7.44-7.23 (m, 6H), 7.05-6.92 (m,2H), 5.16-5.11 (m, 1H), 4.04 (s, 1H), 3.50-3.43 (m, 1H), 3.02-2.78 (m, 4H), 1.69-1.54 (m, 2H), 1.42-1.30 (m, 2H), 1.24-1.13 (m, 4H), 1.09-0.97 (m, 5H), 0.41-0.32 (m, 1H). LC-MS m/z (ESI): 581.3 [M+H]⁺.

### Example 24

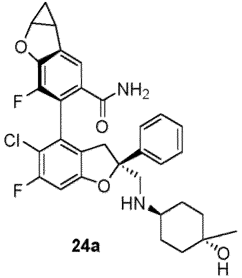

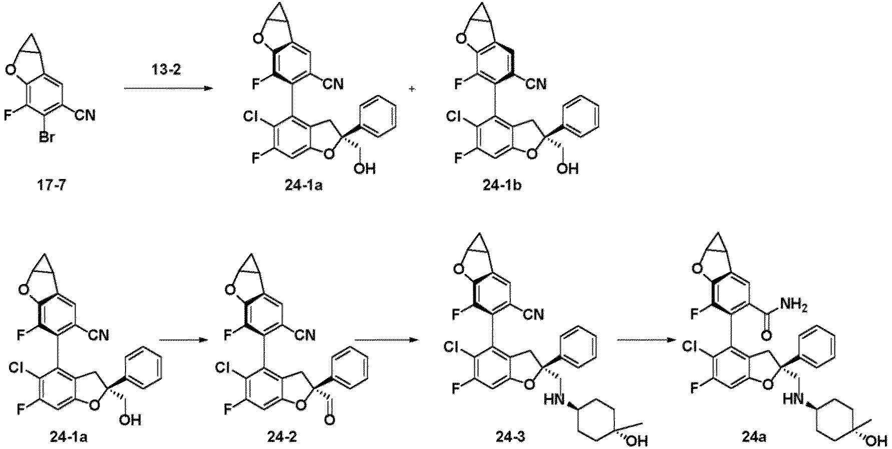

Step 1: Compound **17-7** (60 mg, 0.23 mmol) was dissolved in toluene (1 mL) and water (0.2 mL), then compound **13-2** (114 mg, 0.28 mmol), tris(dibenzylideneacetone)dipalladium (43.3 mg, 0.047 mmol), 4,5-bis(diphenylphosphino)-9,9-dimethylxanthene (54.6 mg, 0.094 mmol), and anhydrous potassium phosphate (150 mg, 0.71 mmol) were sequentially added thereto under nitrogen atmosphere, and the reaction mixture was reacted at 120°C for 16 hours. TLC (PE: EA = 3:1) showed that the starting material was completely reacted. The same operation was repeated nine times for combined processing. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 3:1) to obtain compounds **24-1b** (240 mg, Y: 24%, Rf = 0.4) and **24-1a** (150 mg, Y: 15%, Rf = 0.3). **24-1a:** LC-MS (1.5 mL-2.0 min-5 to 90% B): Rt = 1.49, m/z (ESI): 515.1 [M+CH₃CN+Na]⁺. **24-1b:** LC-MS (1.5 mL-2.0 min-5 to 90% B): Rt = 1.45, m/z (ESI): 515.1 [M+CH₃CN+Na]⁺.

Step 2: Oxalyl chloride (83 mg, 1.32 mmol) was dissolved in dichloromethane (2 mL). The reaction mixture was cooled to -78°C under nitrogen atmosphere and stirred for 30 minutes, added with dimethyl sulfoxide (52 mg, 4.51 mmol) and stirred at -78°C for 30 minutes, then added with starting material **24-1a** (150 mg, 0.33 mmol) dissolved in dichloromethane (2 mL) at -78°C and stirred for 30 minutes, added with triethylamine (168 mg, 1.65 mmol) and stirred for 30 minutes, and warmed to 25°C and stirred for 1 hour. The reaction mixture was poured into saturated ammonium chloride (10 mL) to quench, and extracted with dichloromethane (20 mL × 3). The organic phases were combined, washed with saturated brine (20 mL), dried over anhydrous sodium sulfate, filtered, and the filtrate was concentrated under reduced pressure to obtain compound **24-2** (140 mg, crude).

Step 3: Compound **24-2** (140 mg, 0.31 mmol) was dissolved in 1,2-dichloroethane (3 mL). The reaction mixture was sequentially added with trans-4-amino-1-methylcyclohexanol (80 mg, 0.62 mmol) and acetic acid (1.8 mg, 0.031 mmol) under nitrogen atmosphere, heated to 85°C and stirred for 30 minutes, then added with sodium triacetoxyborohydride (130 mg, 0.62 mmol), and stirred at 85°C for 2 hours. LCMS showed that the starting material was completely reacted. The reaction mixture was cooled to 25°C, poured into saturated sodium bicarbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compound **24-3** (130 mg, Y: 74.2%). LC-MS m/z (ESI): 563.2 [M+H]⁺.

Step 4: Compound **24-3** (130 mg, 0.23 mmol) was dissolved in anhydrous ethanol (4 mL) and water (2 mL), then sodium hydroxide (92 mg, 2.3 mmol) was added thereto, and the reaction mixture was stirred at 100°C for 16 hours. LCMS showed that the starting material was completely reacted. The reaction mixture was poured into saturated ammonium chloride (20 mL) to quench, and extracted with dichloromethane (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: YMC-Actus Triart C18, 150 * 20 mm, 5 µm; eluent: 0.05% NH₃·H₂O-10 mmol/L NH₄HCO₃-ACN; gradient: 56% to 76%; flow rate: 20 mL/min; run time: 10 min; retention time: 7.2 min) to obtain compound **24a** (12.6 mg, Y: 10%). ¹H NMR (400 MHz, DMSO-*d₆*) δ 7.65-7.55 (m, 2H), 7.46-7.12 (m, 6H), 7.03 (d, *J* = 10.0 Hz, 1H), 5.22-5.18 (m, 1H), 4.04 (br, 1H), 3.47-3.40 (m, 1H), 2.99-2.75 (m, 4H), 1.69-1.54 (m, 2H), 1.46-0.95 (m, 10H), 0.48-0.39 (m, 1H). LC-MS m/z (ESI): 581.3 [M+H]⁺.

### Example 25

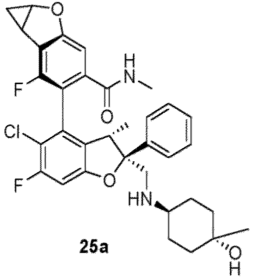

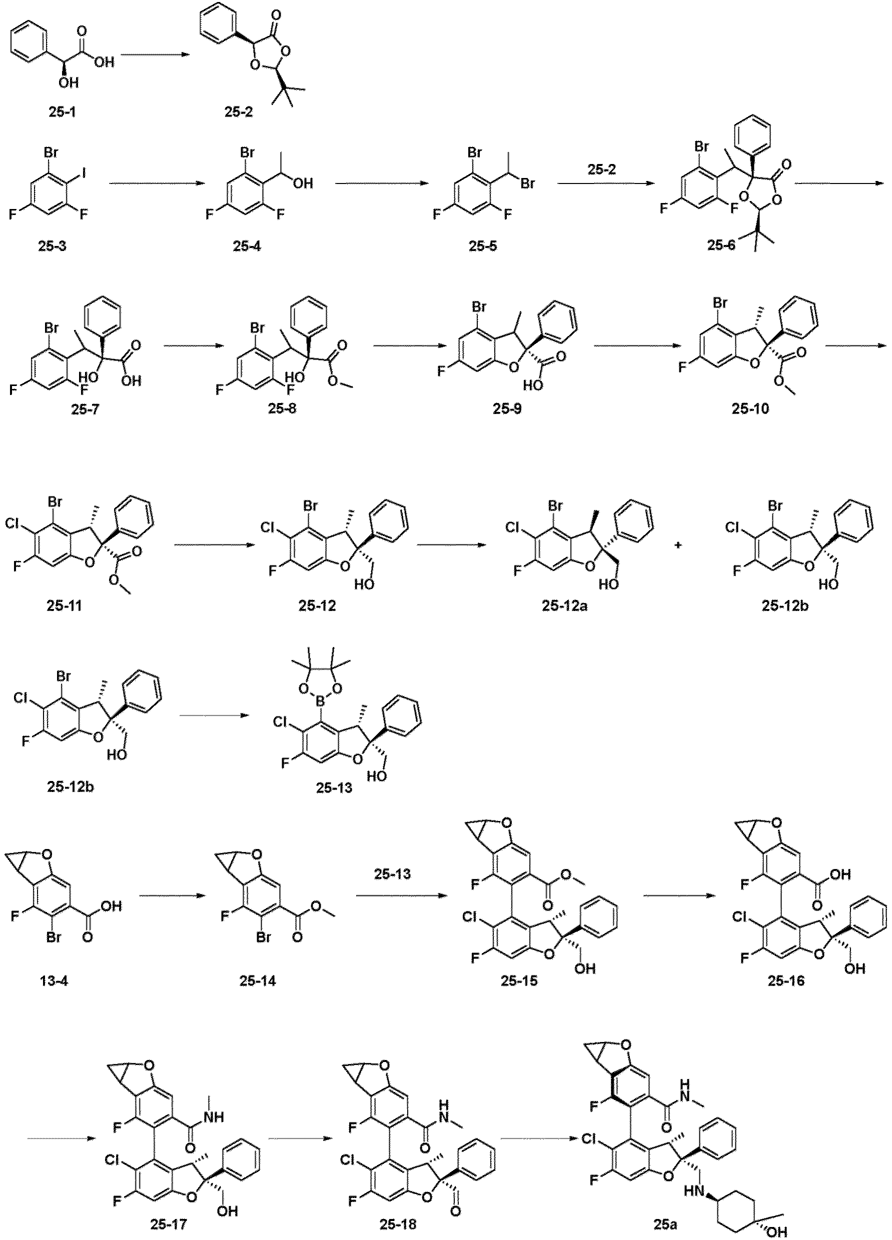

Step 1: Compound **25-1** (120.0 g, 789.5 mmol) and pivaldehyde (81.0 g, 1.0 mol) were dissolved in n-pentane (1.2 L). The reaction mixture was cooled to 0°C, and slowly added with trifluoromethanesulfonic anhydride (66.8 g, 236.8 mmol). After the addition was completed, the reaction mixture was reacted at 36°C for 8 hours. TLC showed that the starting material was completely reacted. The reaction mixture was cooled to 25°C and filtered. The filter cake was collected, added with saturated sodium carbonate aqueous solution (500 mL), stirred for 0.5 hours, then added with ethyl acetate (200 mL), stirred for 0.5 hours, and filtered. The filter cake was collected, dissolved in ethyl acetate (2.6 L), and washed with saturated brine (1.0 L). The organic phase was dried over anhydrous sodium sulfate, filtered, and the filtrate was concentrated under reduced pressure to obtain compound **25-2** (110.0 g, Y: 63%) as a white solid. ¹H NMR (400 MHz, DMSO-*d₆*): δ 7.52-7.37 (m, 5H), 5.57 (s, 1H), 5.48 (s, 1H), 1.01 (s, 9H).

Step 2: Compound **25-3** (150.0 g, 470.2 mmol) was dissolved in anhydrous tetrahydrofuran (1.2 L). The reaction mixture was cooled to -78°C, slowly added dropwise with isopropylmagnesium chloride lithium chloride (1.3 M, 434.1 mL, 564.3 mmol), and reacted at -78°C for 0.5 hours. The reaction mixture was added dropwise with a solution of acetaldehyde (62.0 g, 1.4 mol)/tetrahydrofuran (100 mL) at -78°C, reacted at -78°C for 0.5 hours, then slowly warmed to room temperature, and reacted for 4 hours. LCMS showed that the starting material was completely reacted. The reaction mixture was cooled to 0°C, added dropwise with saturated ammonium chloride (200 mL) to quench, and extracted with methyl tert-butyl ether (500 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:9) to obtain compound **25-4** (75.0 g, Y: 67%). LC-MS m/z (ESI): 219.0/221.0 [M-H₂O+H]⁺.

Step 3: Compound **25-4** (110.0 g, 466 mmol) was dissolved in dichloromethane (1.1 L). The reaction mixture was cooled to 0°C, and added dropwise with phosphorus tribromide (125.0 g, 466 mmol). After the dropwise addition was completed, the reaction mixture was reacted at 0°C for 2 hours. TLC showed that the starting material was completely reacted. The reaction mixture was added dropwise with saturated ammonium chloride (200 mL) at 0°C to quench, and extracted with dichloromethane (500 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:50) to obtain compound **25-5** (107.0 g, Y: 76%).

Step 4: Compound **25-2** (157.0 g, 713 mmol) was dissolved in N,N-dimethylformamide (1.3 L). The system was cooled to 0°C, added with sodium hydride (60% wt, 71.0 g, 1.7 mol) in batches, and kept at 0°C and reacted for 0.5 hours. The system was added dropwise with a solution of compound **25-5** (107.0 g, 356.7 mol)/tetrahydrofuran (100 mL) at 0°C, reacted at 0°C for 0.5 hours, and showed a reddish-brown color. TLC showed that the starting material was completely reacted. The reaction mixture was poured into saturated ammonium chloride aqueous solution (500 mL) at 0°C to quench, and extracted with methyl tert-butyl ether (500 mL × 3). The organic phases were combined, washed with saturated brine (300 mL × 2), dried over anhydrous sodium sulfate, and filtered. The filtrate was concentrated under reduced pressure and then purified by silica gel column chromatography (PE: EA = 1:10) to obtain compound **25-6** (96.0 g, Y: 61%). ¹H NMR (400 MHz, DMSO-*d₆*): δ 7.69-7.65 (m, 1H), 7.53-7.34 (m, 3H), 7.32-7.20 (m, 3H), 5.41 (d, *J* = 44 Hz, 1H), 4.16-4.05 (m, 1H), 1.46-1.24 (m, 3H), 0.83 (s, 9H).

Step 5: Compound **25-6** (85.0 g, 193.2 mmol) was dissolved in methanol (1300 mL), and sodium methoxide (5 M, 154.5 mmol) was added to the above reaction mixture in batches at 25°C. After the addition was completed, the reaction mixture was reacted at 60°C for 16 hours. TLC showed that the reaction was completed. The reaction mixture was cooled to 10°C, slowly added dropwise with saturated ammonium chloride aqueous solution (1.1 L) to quench, and extracted with ethyl acetate (500 mL × 3). The organic phases were combined, washed with saturated brine (200 mL), dried over anhydrous sodium sulfate, filtered, and the filtrate was concentrated under reduced pressure to obtain compound **25-7** (64.0 g, Y: 90%).

Step 6: Compound **25-7** (50.0 g, 134.7 mmol) was dissolved in acetone (500 mL), then anhydrous potassium carbonate (27.8 g, 202.1 mmol) and iodomethane (23.0 g, 161.7 mmol) were sequentially added thereto, and the reaction mixture was reacted at 25°C for 16 hours. TLC showed that the reaction was completed. The reaction mixture was added with water (300 mL), and extracted with ethyl acetate (300 mL × 3). The organic phases were combined, washed with saturated brine (200 mL), dried over anhydrous sodium sulfate, and filtered. The filtrate was concentrated under reduced pressure and then purified by silica gel column chromatography (PE: EA = 20:1) to obtain compound **25-8** (49.0 g, Y: 96%).

Step 7: Compound **25-8** (50.0 g, 130.2 mmol) was dissolved in N,N-dimethylformamide (1.3 L). The reaction system was cooled to 0°C, added with sodium hydride (60% wt, 5.7 g, 143.2 mmol) in batches, and reacted at room temperature for 16 hours. LCMS showed that the starting material was completely reacted. The reaction mixture was cooled to 0°C, added dropwise with saturated ammonium chloride aqueous solution (500 mL) to quench, and extracted with methyl tert-butyl ether (300 mL × 3). The organic phases were combined, washed with saturated brine (100 mL), dried over anhydrous sodium sulfate, filtered, and the filtrate was concentrated under reduced pressure to obtain compound **25-9** (42.0 g, crude).

Step 8: Compound **25-9** (42.0 g, 119.6 mmol) was dissolved in acetone (420 mL), then anhydrous potassium carbonate (24.7 g, 179.5 mmol) and iodomethane (20.4 g, 143.6 mmol) were sequentially added thereto, and the reaction mixture was reacted at 25°C for 16 hours. TLC showed that the reaction was completed. The reaction mixture was added with water (300 mL), and extracted with ethyl acetate (300 mL × 3). The organic phases were combined, washed with saturated brine (200 mL), dried over anhydrous sodium sulfate, and filtered. The filtrate was concentrated under reduced pressure and then purified by silica gel column chromatography (PE: EA = 20:1) to obtain compound **25-10** (18.8 g, Y: 38%). ¹H NMR (400 MHz, DMSO-*d₆*): δ 7.63-7.58 (m, 2H), 7.42-7.30 (m, 3H), 7.10-7.03 (m, 2H), 3.95-3.88 (m, 1H), 3.74 (s, 3H), 1.31 (t, *J* = 6.8 Hz, 3H).

Step 9: Compound **25-10** (10.0 g, 27.4 mmol) was dissolved in acetonitrile (200 mL), then N-chlorosuccinimide (4.1 g, 30.1 mmol) and p-toluenesulfonic acid (9.5 g, 54.8 mmol) were sequentially added thereto at 25°C, and the reaction mixture was reacted at 60°C for 12 hours. TLC showed that the starting material was completely reacted. The reaction system was cooled to 0°C, added dropwise with saturated sodium carbonate (50 mL) to quench, and extracted with dichloromethane (100 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 20:1) to obtain compound **25-11** (7.8 g, Y: 71%).

Step 10: Compound **25-11** (10.0 g, 25 mmol) was dissolved in tetrahydrofuran (100 mL) and methanol (10 mL). The reaction mixture was cooled to 10°C, added with sodium borohydride (5.6 g, 150 mmol) in batches, and reacted at 25°C for 16 hours. TLC showed that the reaction was completed. The reaction mixture was cooled to 10°C, slowly added dropwise with saturated ammonium chloride (50 mL) to quench, and extracted with ethyl acetate (150 mL × 3). The organic phases were combined, washed with saturated brine (50 mL), dried over anhydrous sodium sulfate, filtered, and the filtrate was concentrated under reduced pressure to obtain compound **25-12** (6.7 g, Y: 72%). ¹H NMR (400 MHz, DMSO-*d₆*): δ 7.46-7.41 (m, 2H), 7.35-7.30 (m, 2H), 7.27-7.17 (m, 2H), 5.07 (t, *J* = 5.6 Hz, 1H), 4.02-3.89 (m, 2H), 3.58-3.50 (m, 1H), 1.44 (d, *J* = 6.8 Hz, 3H).

Step 11: Compound **25-12** (20 g, 25 mmol) was subjected to chiral resolution to obtain compound **25-12a** (8.8 g, Y: 44.0%) and compound **25-12b** (10.9 g, Y: 54%). **25-12a:** Column model: Lux-4 100 × 4.6 mm 3.0 µm; elution system: CO₂/IPA (50% Hex) 20 mM NH₃; elution gradient: 10% IPA (50% Hex) to 50% IPA (50% Hex); flow rate: 3 mL/min; run time: 4 min, retention time: 1.718. **25-12b:** Column model: Lux-4 100 × 4.6 mm 3.0 µm; elution system: CO₂/IPA (50% Hex) 20 mM NH₃; elution gradient: 10% IPA (50% Hex) to 50% IPA (50% Hex); flow rate: 3 mL/min; run time: 4 min, retention time: 1.906.

Step 12: **25-12b** (2.0 g, 5.4 mmol), bis(pinacolato)diboron (2.7 g, 10.8 mmol), and anhydrous potassium acetate (880 mg, 8.98 mmol) were dissolved in anhydrous toluene (50 mL), then [1,1'-bis(diphenylphosphino)ferrocene]palladium(II) dichloride (380 mg, 0.54 mmol) was added thereto under nitrogen atmosphere, and the reaction mixture was replaced with nitrogen three times and reacted at 90°C for 16 hours. TLC showed that the starting material was completely reacted. The reaction mixture was directly concentrated under reduced pressure and then purified by silica gel column chromatography (EA: PE = 1:25) to obtain compound **25-13** (1.1 g, Y: 48%).

Step 13: Compound **13-4** (860 mg, 3.2 mmol) was dissolved in N,N-dimethylformamide (8 mL). The reaction mixture was cooled to 0°C, and sequentially added with anhydrous potassium carbonate (870 mg, 6.3 mmol) and iodomethane (680 mg, 4.8 mmol). The reaction mixture was reacted at room temperature for 2 hours under nitrogen atmosphere. TLC showed that the starting material was completely reacted. The reaction mixture was added with water (80 mL) and brine (10 mL), and extracted with ethyl acetate (50 mL × 2). The organic phases were combined, washed with saturated brine (20 mL), dried over anhydrous sodium sulfate, and filtered. The filtrate was concentrated under reduced pressure and then purified by silica gel column chromatography (EA: PE = 1:20) to obtain compound **25-14** (650 mg, Y: 71%) as a colorless transparent oil.

Step 14: Compound **25-14** (320 mg, 1.1 mmol) and compound **25-13** (466 mg, 1.1 mmol) were dissolved in toluene (8 mL) and water (1.6 mL), and tris(dibenzylideneacetone)dipalladium (100 mg, 0.1 mmol), 4,5-bis(diphenylphosphino)-9,9-dimethylxanthene (120 mg, 0.2 mmol), and anhydrous potassium phosphate (650 mg, 3.1 mmol) were sequentially added thereto. The reaction mixture was reacted at 100°C overnight under nitrogen atmosphere. TLC showed that the starting material was completely reacted. The reaction mixture was cooled to 25°C, concentrated under reduced pressure, and purified by silica gel column chromatography (EA: PE = 1:10) to obtain compound **25-15** (250 mg, Y: 44.9%). LC-MS m/z (ESI): 521.2/523.1 [M+Na]⁺.

Step 15: Compound **25-15** (250 mg, 0.5 mmol) was dissolved in ethanol (5 mL) and water (5 mL), and sodium hydroxide (200 mg, 5.0 mmol) was added thereto. The reaction mixture was reacted at 85°C for 12 hours. TLC showed that the starting material was completely reacted. The reaction mixture was cooled to 0°C, added with water (10 mL), then slowly added with dilute hydrochloric acid (0.5 M) until the pH of the reaction system was 5 to 6, and extracted with ethyl acetate (30 mL × 3). The organic phases were combined, washed with saturated brine (30 mL), dried over anhydrous sodium sulfate, filtered, and the filtrate was concentrated under reduced pressure to obtain compound **25-16** (200 mg, crude).

Step 16: Compound **25-16** (200 mg, 0.41 mmol) was dissolved in anhydrous N,N-dimethylformamide (2 mL), and 2-(7-azabenzotriazol-1-yl)-N,N,N',N'-tetramethyluronium hexafluorophosphate (185 mg, 0.49 mmol), N,N-diisopropylethylamine (250 mg, 1.94 mmol), and methylamine hydrochloride (55 mg, 0.82 mmol) were sequentially added thereto. The reaction mixture was reacted at room temperature for 16 hours under nitrogen atmosphere. LCMS showed that the starting material was completely reacted. The reaction mixture was added with water (20 mL), and extracted with ethyl acetate (20 mL × 2). The organic phases were combined, washed with saturated brine (15 mL), dried over anhydrous sodium sulfate, and filtered. The filtrate was concentrated under reduced pressure and then purified by silica gel column chromatography (EA: PE = 2:1) to obtain compound **25-17** (185 mg, Y: 90%). LC-MS m/z (ESI): 498.3/500.3 [M+H]⁺.

Step 17: Dimethyl sulfoxide (190 mg, 2.44 mmol) was dissolved in anhydrous dichloromethane (1 mL), and the reaction system was cooled to -70°C. The above reaction mixture was then slowly added dropwise with oxalyl chloride (150 mg, 1.18 mmol) dissolved in anhydrous dichloromethane (0.3 mL) at - 70°C and reacted at -70°C for 0.5 hours, slowly added dropwise with compound **25-17** (185 mg, 0.37 mmol) dissolved in dichloromethane (0.3 mL) at -70°C and reacted at -70°C for 0.5 hours, slowly added dropwise with triethylamine (370 mg, 3.66 mmol) dissolved in dichloromethane (0.3 mL) at -70°C and reacted at - 70°C for 0.5 hours, and naturally warmed to 25°C and stirred for another 2 hours. TLC showed that the starting material was completely reacted. The reaction mixture was added with water (20 mL), and extracted with dichloromethane (10 mL). The organic phase was washed with saturated brine (20 mL), dried over anhydrous sodium sulfate, filtered, and the filtrate was concentrated under reduced pressure to obtain compound **25-18** (160 mg, crude).

Step 18: Compound **25-18** (160 mg, 0.32 mmol) was dissolved in dichloroethane (5 mL), then trans-4-amino-1-methylcyclohexanol (50 mg, 0.39 mmol) and acetic acid (19 mg, 0.32 mmol) were added thereto, and the reaction mixture was reacted at 85°C for 2 hours. The reaction mixture was cooled to 25°C, added with sodium triacetoxyborohydride (140 mg, 0.66 mmol), and reacted at 60°C for 0.5 hours. LCMS showed that the starting material was completely reacted. The reaction mixture was cooled to 25°C, added with water (20 mL), and extracted with dichloromethane (30 mL × 2). The organic phase was washed with saturated brine (20 mL), dried over anhydrous sodium sulfate, and filtered. The filtrate was concentrated under reduced pressure, and then purified by preparative high-performance liquid chromatography (column model: YMC-Actus Triart C18, 150 * 20 mm, 5 µm; eluent: 0.05% NH₃·H₂O-10 mmol/L NH₄HCO₃-ACN; gradient: 50% to 70%; flow rate: 20 mL/min; run time: 12 min; retention time: 6.87 min/7.9 min, where Rt = 7.9 min was for target product) to obtain compound **25a** (14 mg, Y: 7%). ¹H NMR (400 MHz, DMSO-*d₆*): δ 8.23 (br, 1H), 7.50-7.23 (m, 5H), 7.08-7.03 (m, 1H), 7.00 (s, 1H), 5.16-5.11 (m, 1H), 4.10-4.00 (m, 1H), 3.28-3.21 (m, 1H), 3.15-3.00 (m, 1H), 2.85-2.78 (m, 1H), 2.60 (d, *J* = 4.4 Hz, 1H), 2.35-2.28 (m, 1H), 1.70-1.52 (m, 2H), 1.42-0.90 (m, 15H), 0.41-0.31 (m, 1H). LC-MS m/z (ESI): 609.3/611.3 [M+H]⁺.

### Example 26

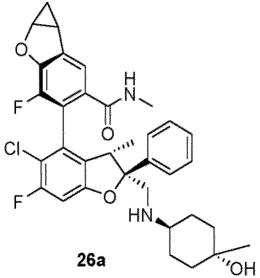

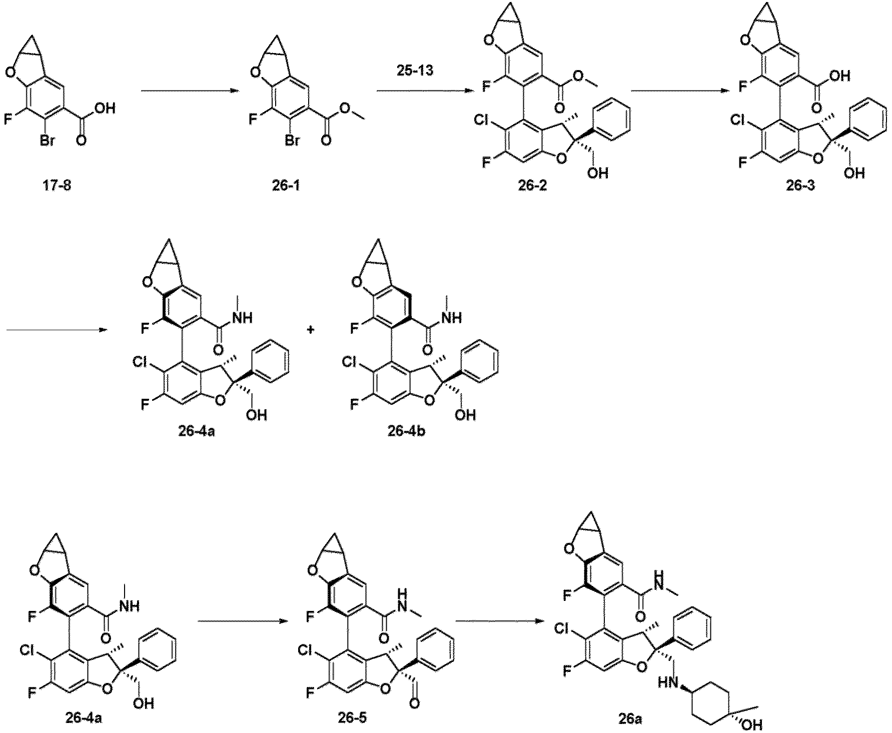

Step 1: Compound **17-8** (1.4 g, 5.1 mmol) was dissolved in N,N-dimethylacetamide (15 mL), then potassium carbonate (1.42 g, 10.2 mmol) and iodomethane (1.09 g, 7.69 mmol) were sequentially added thereto, and the reaction mixture was reacted at room temperature for 2 hours. TLC (PE: EA = 1:9) showed that the starting material was completely reacted. The reaction mixture was poured into ice water (100 mL) to quench, and extracted with ethyl acetate (100 mL × 3). The organic phases were combined, washed with saturated brine (100 mL), dried over anhydrous sodium sulfate, and filtered. The filtrate was concentrated under reduced pressure and then purified by silica gel column chromatography (PE: EA = 3:1) to obtain compound **26-1** (1.35 g, Y: 91%).

Step 2: Compound **26-1** (100 mg, 0.35 mmol) was dissolved in toluene (2 mL) and water (0.4 mL), then compound **25-13** (182 mg, 0.43 mmol), tris(dibenzylideneacetone)dipalladium (63 mg, 0.07 mmol), 4,5-bis(diphenylphosphino)-9,9-dimethylxanthene (80 mg, 0.14 mmol), and anhydrous potassium carbonate (144 mg, 1.04 mmol) were sequentially added thereto under nitrogen atmosphere, and the reaction mixture was reacted at 120°C overnight. TLC (PE: EA = 4:1) showed that the starting material was completely reacted. The above operation was repeated three times for combined processing. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 3:1) to obtain compound **26-2** (580 mg, Y: 83%). LC-MS m/z (ESI): 499.3 [M+H]⁺.

Step 3: Compound **26-2** (580 mg, 1.6 mmol) and sodium hydroxide (4.6 g, 11.6 mmol) were dissolved in ethanol (6 mL) and water (6 mL), and the reaction mixture was reacted at room temperature for 12 hours. TLC (PE: EA = 4:1) showed that the starting material was completely reacted. The reaction mixture was added dropwise with hydrochloric acid solution (200 mL, 1 M) to quench, and extracted with ethyl acetate (100 mL × 3). The organic phases were combined, washed with saturated brine (200 mL), dried over anhydrous sodium sulfate, and filtered. The filtrate was concentrated under reduced pressure, and then the reaction mixture was concentrated under reduced pressure to obtain compound **26-3** (500 mg, Y: 88%).

Step 4: Compound **26-3** (500 mg, 1.03 mmol) was dissolved in N,N-dimethylformamide (10 mL), then methylamine hydrochloride (139 mg, 2.06 mmol), 2-(7-azabenzotriazol-1-yl)-N,N,N',N'-tetramethyluronium hexafluorophosphate (1.18 g, 3.09 mmol), and N,N-diisopropylethylamine (399 mg, 3.09 mmol) were sequentially added thereto, and the reaction mixture was reacted at room temperature for 16 hours. LCMS showed that the starting material was completely reacted. The reaction mixture was added with water (100 mL), and extracted with ethyl acetate (50 mL × 3). The organic phases were combined, washed with saturated brine (100 mL), dried over anhydrous sodium sulfate, and filtered. The filtrate was concentrated under reduced pressure and then purified by silica gel column chromatography (PE: EA = 1:1) to obtain compound **26-4a** (160 mg, Y: 31%, R_{f} = 0.6) and compound **26-4b** (240 mg, Y: 46%, R_{f} = 0.50) as white solids. LC-MS m/z (ESI): 498.2 [M+H]⁺.

Step 5: Oxalyl chloride (81 mg, 0.64 mmol) was dissolved in dichloromethane (1 mL). The reaction mixture was cooled to -78°C under nitrogen atmosphere, added with dimethyl sulfoxide (50 mg, 0.64 mmol) and stirred at -78°C for 30 minutes, then added with starting material **26-4a** (160 mg, 0.32 mmol) dissolved in dichloromethane (2 mL) at -78°C and stirred for 30 minutes, added with triethylamine (685 mg, 6.77 mmol) and stirred at -78°C for 30 minutes, and warmed to 25°C and stirred for 30 minutes. The reaction mixture was poured into saturated ammonium chloride (50 mL) to quench, and extracted with ethyl acetate (30 mL × 3). The organic phases were combined, washed with saturated brine (30 mL), dried over anhydrous sodium sulfate, filtered, and the filtrate was concentrated under reduced pressure to obtain compound **26-5** (150 mg, Y: 94%).

Step 6: Compound **26-5** (150 mg, 0.31 mmol) was dissolved in 1,2-dichloroethane (3 mL). The reaction mixture was sequentially added with trans-4-amino-1-methylcyclohexanol (80 mg, 0.62 mmol) and acetic acid (2 mg, 0.03 mmol) under nitrogen atmosphere, heated to 85°C and stirred for 1 hour, then added with sodium triacetoxyborohydride (131 mg, 0.62 mmol), and stirred at 85°C for 2 hours. LCMS showed that the starting material was completely reacted. The reaction mixture was cooled to 25°C, poured into saturated sodium bicarbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and purified by silica gel column chromatography (PE: EA = 1:2) to obtain a white solid compound, which was then purified by preparative high-pressure liquid chromatography (column model: Xselect CSH Prep Fluoro-Phenyl, 150 * 19 mm, 5 µm; eluent: 0.05% NH₃·H₂O+10 mmol/L NH₄HCO₃/CH₃CN+MeOH = 1:1; gradient: 35% to 57%; flow rate: 20 mL/min; run time: 11 min; retention time: 6.5 min) to obtain compound **26a** (32.8 mg, Y: 17%). ¹H NMR (400 MHz, DMSO-*d₆*) δ 8.29-8.14 (m, 1H), 7.56 (s, 1H), 7.44-7.23 (m, 5H), 7.09-7.01 (m, 1H), 5.22-5.17 (m, 1H), 4.06 (br, 1H), 3.19-3.00 (m, 1H), 2.90-2.80 (m, 1H), 2.61 (d, *J* = 4.0 Hz, 3H), 2.43-2.20 (m, 1H), 1.70-1.52 (m, 2H), 1.39-1.10 (m, 7H), 1.03-0.85 (m, 6H), 0.43-0.39 (m, 1H). LC-MS m/z (ESI): 609.3 [M+H]⁺.

### Example 27

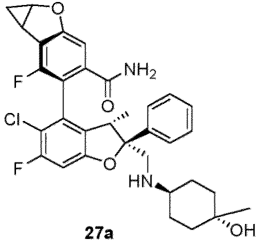

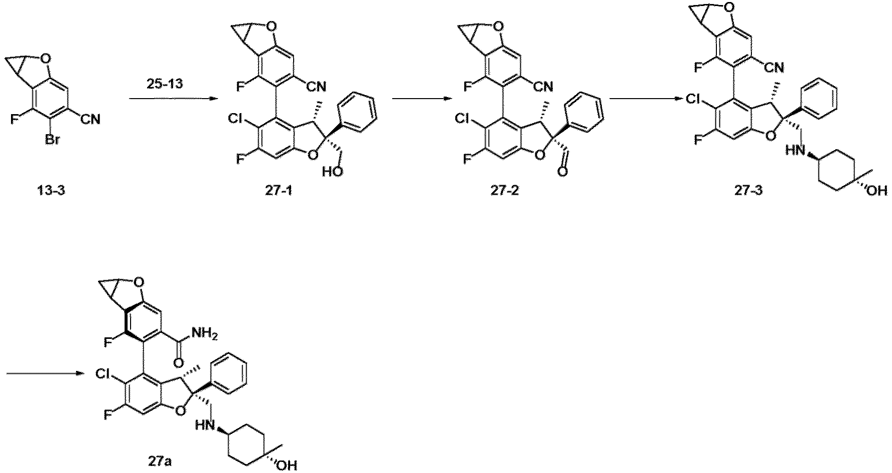

Step 1: Compound **13-3** (50 mg, 0.019 mmol) was dissolved in toluene (1 mL) and water (0.2 mL), then compound **25-13** (99 mg, 0.024 mmol), tris(dibenzylideneacetone)dipalladium (36.1 mg, 0.039 mmol), 4,5-bis(diphenylphosphino)-9,9-dimethylxanthene (45.5 mg, 0.079 mmol), and anhydrous potassium phosphate (125 mg, 0.059 mmol) were sequentially added thereto under nitrogen atmosphere, and the reaction mixture was reacted at 110°C for 16 hours. TLC (PE: EA = 1:1) showed that the starting material was completely reacted. The same operation was repeated four times for combined processing. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compound **27-1** (100 mg, Y: 27%). LC-MS m/z (ESI): 488.1 [M+Na]⁺.

Step 2: Oxalyl chloride (81.7 mg, 0.64 mmol) was dissolved in dichloromethane (3 mL). The reaction mixture was cooled to -70°C under nitrogen atmosphere, added with dimethyl sulfoxide (100 mg, 1.29 mmol) and stirred at -78°C for 30 minutes, then added with starting material **27-1** (100 mg, 0.21 mmol) dissolved in dichloromethane (3 mL) at -78°C and stirred for 30 minutes, added with triethylamine (195 mg, 1.93 mmol) and stirred for 30 minutes, and warmed to 25°C and stirred for 1 hour. The reaction mixture was poured into saturated ammonium chloride (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **27-2** (99 mg, crude).

Step 3: Compound **27-2** (99 mg, 0.21 mmol) was dissolved in 1,2-dichloroethane (4 mL). The reaction mixture was sequentially added with trans-4-atnino-1-methylcyclohexanol (55.1 mg, 0.43 mmol) and acetic acid (1.24 mg, 0.21 mmol) under nitrogen atmosphere, heated to 85°C and stirred for 30 minutes, then added with sodium triacetoxyborohydride (16.1 mg, 0.42 mmol), and stirred at 85°C for 2 hours. LCMS showed that the starting material was completely reacted. The reaction mixture was cooled to 25°C, poured into saturated sodium bicarbonate (20 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **27-3** (100 mg, crude). LC-MS m/z (ESI): 488.1 [M+H]⁺.

Step 4: Compound **27-3** (100 mg, 0.17 mmol) was dissolved in dimethyl sulfoxide (2 mL). The reaction mixture was sequentially added with anhydrous potassium carbonate (36.7 mg, 0.34 mmol) and 30% hydrogen peroxide (1.96 mg, 0.017 mmol) under nitrogen atmosphere, and stirred at 25°C for 3 hours. LCMS showed that the starting material was completely reacted. The reaction mixture was poured into saturated ammonium chloride (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O/CH₃CN; gradient: 55.6% to 75.6%; flow rate: 20 mL/min; run time: 10 min; retention time: 8.1 min) to obtain compound **27a** (4.2 mg, Y: 4%). ¹H NMR (400 MHz, DMSO-*d₆*) δ 7.64-7.12 (m, 7H), 7.07-6.98 (m, 1H), 5.15-5.10 (m, 1H), 4.10-4.02 (m, 1H), 3.43-3.35 (m, 1H), 3.21-3.03 (m, 2H), 2.86-2.75 (m, 1H), 2.03-1.92 (m, 1H), 1.71-1.48 (m, 1H), 1.38-0.82 (m, 14H), 0.40-0.28 (m, 1H). LC-MS m/z (ESI): 595.2 [M+H]⁺.

### Example 28

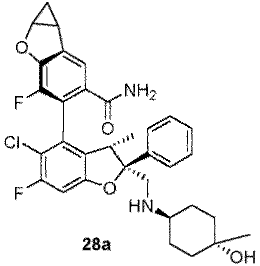

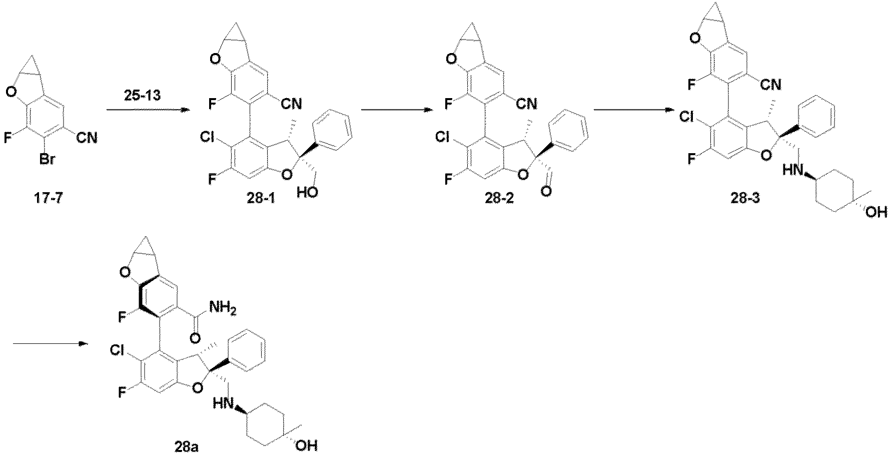

Step 1: Compound **17-7** (50 mg, 0.019 mmol) was dissolved in toluene (1 mL) and water (0.2 mL), then compound **25-13** (99 mg, 0.024 mmol), tris(dibenzylideneacetone)dipalladium (36.1 mg, 0.039 mmol), 4,5-bis(diphenylphosphino)-9,9-dimethylxanthene (45.5 mg, 0.079 mmol), and anhydrous potassium phosphate (125 mg, 0.059 mmol) were sequentially added thereto under nitrogen atmosphere, and the reaction mixture was reacted at 100°C for 16 hours. TLC (PE: EA = 1:1) showed that the starting material was completely reacted. The same operation was repeated four times for combined processing. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compound **28-1** (100 mg, Y: 27%).

Step 2: Oxalyl chloride (81.7 mg, 0.64 mmol) was dissolved in dichloromethane (3 mL). The reaction mixture was cooled to -78°C under nitrogen atmosphere, added with dimethyl sulfoxide (100 mg, 1.29 mmol) and stirred at -78°C for 30 minutes, then added with starting material **28-1** (100 mg, 0.21 mmol) dissolved in dichloromethane (3 mL) at -78°C and stirred for 30 minutes, added with triethylamine (195 mg, 1.93 mmol) and stirred for 30 minutes, and warmed to 25°C and stirred for 1 hour. The reaction mixture was poured into saturated ammonium chloride (10 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **28-2** (99 mg, crude) as a yellow solid.

Step 3: Compound **28-2** (99 mg, 0.21 mmol) was dissolved in 1,2-dichloroethane (4 mL). The reaction mixture was sequentially added with trans-4-atnino-1-methylcyclohexanol (55.1 mg, 0.43 mmol) and acetic acid (1.24 mg, 0.21 mmol) under nitrogen atmosphere, heated to 85°C and stirred for 30 minutes, then added with sodium triacetoxyborohydride (16.1 mg, 0.42 mmol), and stirred at 85°C for 2 hours. LCMS showed that the starting material was completely reacted. The reaction mixture was cooled to 25°C, poured into saturated sodium bicarbonate (20 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **28-3** (100 mg, crude). LC-MS m/z (ESI): 577.3 [M+H]⁺.

Step 4: Compound **28-3** (100 mg, 0.17 mmol) was dissolved in dimethyl sulfoxide (2 mL). The reaction mixture was sequentially added with anhydrous potassium carbonate (36.7 mg, 0.34 mmol) and 30% hydrogen peroxide (1.96 mg, 0.017 mmol) under nitrogen atmosphere, and stirred at 25°C for 3 hours. LCMS showed that the starting material was completely reacted. The reaction mixture was poured into saturated ammonium chloride (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O/CH₃CN; gradient: 54.1% to 74.1%; flow rate: 20 mL/min; run time: 11 min; retention time: 7.9 min) to obtain compound **28a** (2.6 mg, Y: 2.5%). ¹H NMR (400 MHz, DMSO-*d*₆) δ 7.84-7.32 (m, 7H), 7.21-7.02 (m,2H), 5.25-5.13 (m, 1H), 4.02 (s, 1H), 3.58-3.41 (m, 1H), 3.02-2.75 (m, 3H), 1.95-1.85 (m, 1H), 1.71-1.48 (m, 1H), 1.59-1.38 (m, 4H), 1.36-1.12 (m, 4H), 1.05 (s, 3H), 0.95-0.89 (m, 3H), 0.88-0.79 (m, 1H), 0.42-0.35 (m, 1H). LC-MS m/z (ESI): 595.2 [M+H]⁺.

### Example 30

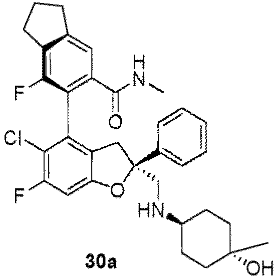

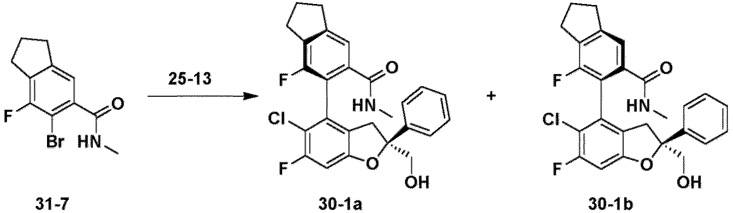

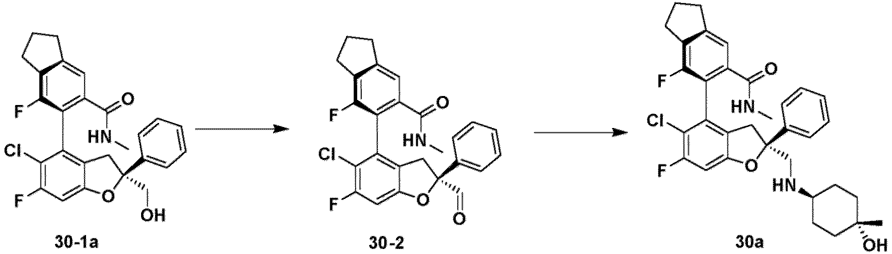

Step 1: Compound **31-7** (200 mg, 0.73 mmol) and compound **25-13** (372 mg, 0.91 mmol) were dissolved in toluene (5 mL) and water (1 mL), then tris(dibenzylideneacetone)dipalladium (134 mg, 0.13 mmol), 4,5-bis(diphenylphosphino)-9,9-dimethylxanthene (170 mg, 0.28 mmol), and anhydrous potassium carbonate (3.4 mg, 2.2 mmol) were sequentially added thereto, and the reaction mixture was reacted at 100°C for 16 hours. LCMS showed that the starting material was completely reacted. The reaction mixture was cooled to 25°C, concentrated under reduced pressure, and then purified by silicagel column chromatography (EA: PE = 1:5) to obtain compound **30-1a** (70 mg, Y: 20%, R_{f} = 0.6) and compound **30-1b** (110 mg, Y: 31%, Rf = 0.4). **30-1a:** LC-MS (1.5 mL-2.0 min-5 to 95% B): Rt = 1.38, m/z (ESI): 470.2 [M+H]⁺. **30-1b:** LC-MS (1.5 mL-2.0 min-5 to 95% B): Rt = 1.31, m/z (ESI): 470.2 [M+H]⁺.

Step 2: Compound dimethyl sulfoxide (23 mg, 0.3 mol) was dissolved in dichloromethane (1 mL). The system was cooled to -78°C, added dropwise with oxalyl chloride (38 mg, 0.3 mol) and reacted at -78°C for 0.5 hours, and added dropwise with **30-1a** (70 mg, 0.15 mol) dissolved in dichloromethane (1 mL) and reacted at -78°C for 0.75 hours. The reaction mixture was added with triethylamine (75 mg, 0.75 mol) and reacted at -78°C for 0.5 hours. LCMS showed that the starting material was completely reacted. The reaction mixture was added with water (20 mL), and extracted with dichloromethane (10 mL × 3). The organic phases were combined, washed with saturated brine (20 mL), dried over anhydrous sodium sulfate, filtered, and the filtrate was concentrated under reduced pressure to obtain compound **30-2** (60.0 mg, Y: 87%).

Step 3: Compound **30-2** (50 mg, 0.11 mol) was dissolved in 1,2-dichloroethane (1 mL). The reaction system was sequentially added with acetic acid (0.6 mg, 0.01 mol) and (1r,4r)-4-amino-1-methylcyclohexanol (28 mg, 0.22 mol), reacted at 85°C for 1.5 hours, and added with sodium triacetoxyborohydride (45 mg, 0.22 mol). LCMS showed that the starting material was completely reacted. The reaction mixture was cooled to 25°C, added with water (20 mL), and extracted with ethyl acetate (10 mL × 3). The organic phases were combined, washed with saturated brine (10 mL × 2), dried over anhydrous sodium sulfate, and filtered. The filtrate was concentrated under reduced pressure and then purified by preparative high-performance liquid chromatography (column model: Xbridge Prep Phenyl OBD, 150 * 19 mm, 5 µm; eluent: 0.05% NH₃·H₂O/CH₃CN; gradient: 60% to 80%; flow rate: 20 mL/min; run time: 15 min; retention time: 12.5 min) to obtain compound 30a (12.8 mg, Y: 20%). ¹H NMR (400 MHz, DMSO-*d₆*): δ 8.22-8.18 (m, 1H), 7.40-7.29 (m, 6H), 7.02 (d, *J* = 9.6 Hz, 1H), 4.01 (s, 1H), 3.48-3.42 (m, 1H), 2.96-2.79 (m, 6H), 2.75-2.71 (m, 1H), 2.64-2.60 (m, 3H), 2.11-2.01 (m, 2H), 1.68-1.52 (m, 2H), 1.42-1.31 (m, 2H), 1.24-1.12 (m, 4H), 1.10-1.03 (m, 2H), 1.01 (s, 3H). LC-MS m/z (ESI): 581.3 [M+H]⁺.

### Example 31

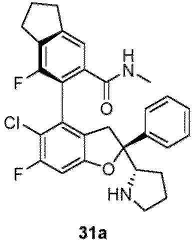

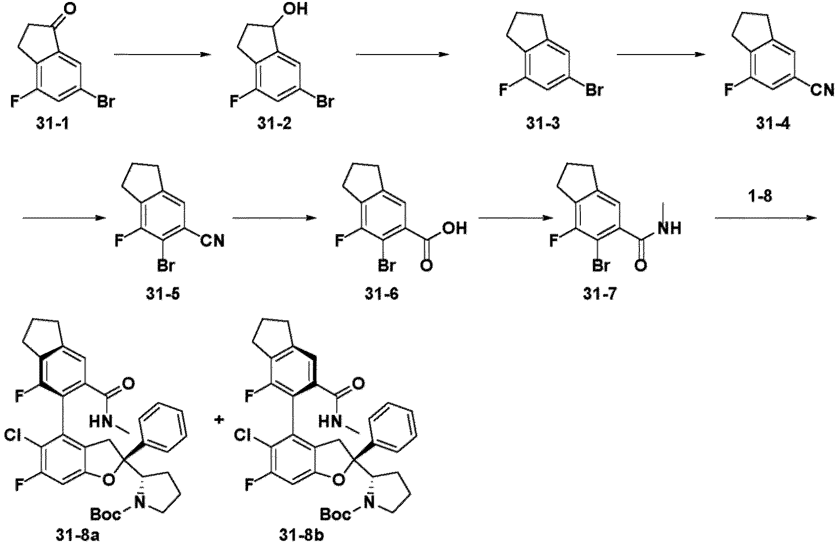

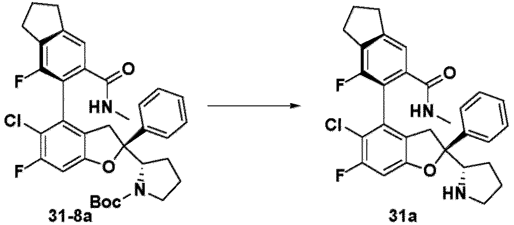

Step 1: Compound **31-1** (5.0 g, 21.8 mmol) was dissolved in methanol (50 mL), then sodium borohydride (1.65 g, 43.66 mmol) was added thereto, and the reaction mixture was reacted at room temperature for 2 hours. TLC showed that the starting material was completely reacted. The reaction mixture was added dropwise with saturated ammonium chloride aqueous solution (200 mL) to quench, and extracted with ethyl acetate (100 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 20:1) to obtain compound **31-2** (3.8 g, Y: 75%). ¹H NMR (400 MHz, CDCl₃): δ 7.31 (s, 1H), 7.12-7.05 (m, 1H), 5.20 (t, *J* = 6.4 Hz, 1H), 3.04-2.96 (m, 1H), 2.77-2.70 (m, 1H), 2.54-2.46 (m, 1H), 1.97-1.90 (m, 1H).

Step 2: Compound **31-2** (3.5 g, 15.2 mmol) was dissolved in dichloromethane (70 mL). The reaction system was cooled to 0°C, then the reaction mixture was sequentially and slowly added dropwise with triethylsilane (8.8 g, 75.7 mmol) and trifluoroacetic acid (4.3 g, 30.3 mmol), and reacted at 25°C for 3 hours. TLC showed that the starting material was completely reacted. The reaction mixture was added dropwise with sodium bicarbonate aqueous solution (400 mL) to quench, and extracted with ethyl acetate (100 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 30:1) to obtain compound **31-3** (3.10 g, Y: 95%).

Step 3: Compound **31-3** (3.0 g, 13.9 mmol) was dissolved in N,N-dimethylformamide (60 mL), then zinc cyanide (1.9 g, 16.7 mmol), tris(dibenzylideneacetone)dipalladium (0.7 g, 0.7 mmol), and 1,1'-bis(diphenylphosphino)ferrocene (0.75 g, 1.4 mmol) were sequentially added thereto, and the reaction mixture was reacted at 100°C for 16 hours. TLC showed that the starting material was completely reacted. The reaction mixture was cooled to 25°C, added with water (200 mL), and extracted with ethyl acetate (100 mL × 3). The organic phases were combined, washed with saturated brine (100 mL), dried over anhydrous sodium sulfate, and filtered. The filtrate was concentrated under reduced pressure and then purified by silica gel column chromatography (PE: EA = 25:1) to obtain compound **31-4** (1.5 g, Y: 66%).

Step 4: Compound **31-4** (1.4 g, 8.7 mmol) was dissolved in tetrahydrofuran (28 mL). The reaction system was cooled to -78°C, added dropwise with lithium diisopropylamide (4.8 mL, 9.6 mmol, 2 M), and reacted at -78°C for 1 hour. The reaction mixture was added with 1,2-dibromotetrachloroethane (2.8 g, 8.7 mmol), slowly warmed to room temperature and reacted for 2 hours. TLC showed that the starting material was completely reacted. The reaction mixture was added dropwise with ice-cold ammonium chloride aqueous solution (200 mL) to quench, and extracted with ethyl acetate (100 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 20:1) to obtain compound **31-5** (1.4 g, Y: 67%) as a white solid. ¹H NMR (400 MHz, CDCl₃): δ 7.32 (s, 1H), 3.04 (t, *J* = 7.6 Hz, 2H), 2.96 (t, *J* = 7.6 Hz, 2H), 2.23-2.15 (m, 2H).

Step 5: Compound **31-5** (700 mg, 2.9 mmol) and sodium hydroxide (1.17 g, 29.2 mmol) were dissolved in ethanol (7 mL) and water (7 mL), and the reaction mixture was heated to 110°C and reacted for 16 hours. TLC showed that the starting material was completely reacted. The reaction mixture was added dropwise with hydrochloric acid solution (100 mL, 2 M) to quench, and extracted with ethyl acetate (100 mL × 3). The organic phases were combined, washed with saturated brine (200 mL), dried over anhydrous sodium sulfate, filtered, and the filtrate was concentrated under reduced pressure to obtain compound **31-6** (600 mg, crude).

Step 6: Compound **31-6** (600 mg, 2.3 mmol) was dissolved in N,N-dimethylformamide (6 mL), then methylamine hydrochloride (312 mg, 4.6 mmol), 2-(7-azabenzotriazol-1-yl)-N,N,N',N'-tetramethyluronium hexafluorophosphate (1.7 g, 4.6 mmol), and N,N-diisopropylethylamine (588 mg, 4.6 mmol) were sequentially added thereto, and the reaction mixture was reacted at 25°C for 2 hours. TLC showed that the starting material was completely reacted. The reaction mixture was added with water (100 mL), and extracted with ethyl acetate (50 mL × 3). The organic phases were combined, washed with saturated brine (100 mL), dried over anhydrous sodium sulfate, and filtered. The filtrate was concentrated under reduced pressure and then purified by silica gel column chromatography (PE: EA = 1:1) to obtain compound **31-7** (500 mg, Y: 79%). LC-MS m/z (ESI): 272.1/274.4 [M+H]⁺.

Step 7: Compound **31-7** (300 mg, 1.1 mmol) and compound **1-8** (749 mg, 1.3 mmol) were dissolved in toluene (5 mL) and water (1 mL), then tris(dibenzylideneacetone)dipalladium (201 mg, 0.22 mmol), 4,5-bis(diphenylphosphino)-9,9-dimethylxanthene (254 mg, 0.44 mmol), and anhydrous potassium carbonate (455 mg, 3.3 mmol) were sequentially added thereto, and the reaction mixture was reacted at 110°C for 16 hours. LCMS showed that the starting material was completely reacted. The reaction mixture was cooled to 25°C, concentrated under reduced pressure, and then purified by silica gel column chromatography (EA: PE = 1:4) to obtain compound **31-8a** (80 mg, Y: 11%, R_{f} = 0.6) and compound **31-8b** (180 mg, Y: 26%, R_{f} = 0.4). LC-MS m/z (ESI): 609.5 [M+H]⁺.

Step 2: Compound **31-8a** (80 mg, 0.13 mmol) was dissolved in dichloromethane (2 mL), then trifluoroacetic acid (0.4 mL) was added thereto, and the reaction mixture was reacted at 25°C for 0.5 hours. LCMS showed that the starting material was completely reacted. The reaction mixture was cooled to 0°C, poured into saturated sodium carbonate (20 mL) to quench, and extracted with ethyl acetate (10 mL × 3). The organic phases were combined, washed with saturated brine (20 mL), dried over anhydrous sodium sulfate, and filtered. The filtrate was concentrated under reduced pressure and then purified by preparative high-performance liquid chromatography (column model: Xbridge Prep Phenyl OBD, 150 * 19 mm, 5 µm; eluent: 0.05% NH₃·H₂O/CH₃CN; gradient: 60% to 70%; flow rate: 20 mL/min; run time: 15 min; retention time: 11.2 min) to obtain compound **31a** (22.3 mg, Y: 33%). ¹H NMR (400 MHz, DMSO-*d₆*): δ 8.12-8.07 (m, 1H), 7.46-7.40 (m, 2H), 7.35-7.22 (m, 4H), 6.99 (d, *J* = 9.6 Hz, 1H), 3.59-3.46 (m, 1H), 3.42-3.38 (m, 1H), 3.01-2.82 (m, 5H), 2.78-2.2.67 (m, 1H), 2.68-2.61 (m, 3H), 2.59-2.51 (m, 1H), 2.20-2.05 (m, 2H), 1.57-1.40 (m, 4H). LC-MS m/z (ESI): 509.2 [M+H]⁺.

### Example 36

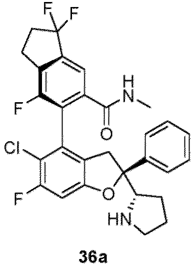

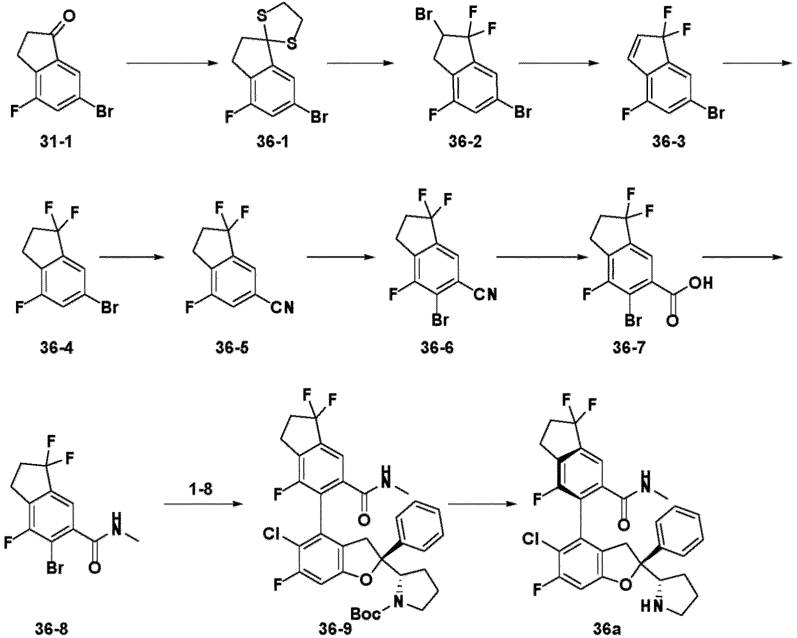

Step 1: Compound **31-1** (10.0 g, 43.7 mmol) was dissolved in toluene (200 mL), then 1,2-ethanedithiol (2.7 g, 24.0 mmol) and p-toluenesulfonic acid (1.7 g, 8.7 mmol) were sequentially added thereto, and the reaction mixture was refluxed to remove water at 120°C for 24 hours. TLC showed that the starting material was completely reacted. The reaction mixture was cooled to 25°C, concentrated under reduced pressure, and purified by silica gel column chromatography (EA: PE = 1:30) to obtain compound **36-1** (7.0 g, Y: 52%). ¹H NMR (400 MHz, CDCl₃-*d*): δ 7.45 (d, *J* = 1.6 Hz, 1H), 7.09- (d, *J* = 8.0 Hz, 1H), 3.56-3.50 (m, 2H), 3.48-3.41 (m, 2H), 2.93 (t, *J* = 6.8 Hz, 1H), 2.70 (t, *J* = 6.8 Hz, 1H).

Step 2: 1,3-Dibromo-5,5-dimethylhydantoin (18.7 g, 65.6 mmol) and pyridine hydrofluoride (22.5 g, 147.5 mmol) were dissolved in dichloromethane (50 mL). The reaction system was cooled to -70°C, added dropwise with a solution of compound **36-1** (5.0 g, 16.4 mmol) in dichloromethane (25 mL), reacted at -70°C for 3 hours, and reacted at room temperature for 13 hours. TLC showed that the starting material was completely reacted. The reaction mixture was added dropwise with sodium hydroxide solution (60 mL, 1 N) and saturated sodium sulfite (30 mL) to quench, stirred for 10 minutes, and extracted with dichloromethane (100 mL × 3). The organic phases were combined, washed with saturated brine (150 mL), dried over anhydrous sodium sulfate, and filtered. The filtrate was concentrated under reduced pressure and then purified by silica gel column chromatography (EA: PE = 1:100) to obtain compound **36-2** (3.1 g, Y: 55%). ¹H NMR (400 MHz, CDCl₃-*d*): δ 7.55 (s, 1H), 7.36 (d, *J* = 8.0 Hz, 1H), 4.61-4.52 (m, 1H), 3.64-3.57 (m, 1H), 3.21-3.15 (m, 1H).

Step 3: Compound **36-2** (3.0, 9.1 mmol) was dissolved in dichloromethane (45 mL). The reaction mixture was cooled to 0°C, added with 1,8-diazabicyclo[5.4.0]undec-7-ene (2.8 g, 18.2 mmol), and reacted at room temperature for 6 hours. TLC showed that the starting material was completely reacted. The reaction mixture was added with dilute hydrochloric acid (18 mL, 1 N) and water (30 mL), and extracted with dichloromethane (50 mL × 2). The organic phases were combined, washed with saturated brine (30 mL), dried over anhydrous sodium sulfate, and filtered. The filtrate was concentrated under reduced pressure and then purified by silica gel column chromatography (EA: PE = 1:100) to obtain compound **36-3** (2.0 g, Y: 88%). ¹H NMR (400 MHz, CDCl₃-*d*): δ 7.41 (d, *J* = 0.8 Hz, 1H), 7.26 (d*, J* = 8.0 Hz, 1H), 6.89 (d, *J* = 6.4 Hz, 1H), 6.17 (d, *J* = 6.4 Hz, 1H).

Step 4: Compound **36-3** (2.0 g, 8.0 mmol) and 2-nitrobenzenesulfonyl chloride (3.6 g, 16.1 mmol) were dissolved in acetonitrile (40 mL). The reaction mixture was cooled to 0°C, added dropwise with hydrazine hydrate (1.29 g, 32.13 mmol, 80%), and reacted at 25°C for 24 hours. TLC showed that the starting material was completely reacted. The reaction mixture was added with water (100 mL), and extracted with ethyl acetate (60 mL × 2). The organic phases were combined, washed with saturated brine (30 mL), dried over anhydrous sodium sulfate, and filtered. The filtrate was concentrated under reduced pressure and then purified by silica gel column chromatography (EA: PE = 1:100) to obtain compound **36-4** (1.8 g, Y: 71%).

Step 5: Compound **36-4** (1.0 g, 3.98 mmol) was dissolved in N,N-dimethylformamide (15 mL), and zinc cyanide (0.7 g, 6.0 mmol), 1,1'-bis(diphenylphosphino)ferrocene (0.44 g, 0.8 mmol), and tris(dibenzylideneacetone)dipalladium (0.35 g, 0.4 mmol) were sequentially added thereto. The reaction mixture was reacted at 100°C for 16 hours under nitrogen atmosphere. TLC showed that the starting material was completely reacted. The reaction mixture was cooled to 25°C, poured into ice water (100 mL), and extracted with ethyl acetate (60 mL × 3). The organic phases were combined, washed with saturated brine (80 mL), dried over anhydrous sodium sulfate, and filtered. The filtrate was concentrated under reduced pressure and then purified by silica gel column chromatography (EA: PE = 1:100) to obtain compound **36-5** (0.45 g, Y: 57%). ¹H NMR (400 MHz, CDCl₃-*d*): δ 7.49 (d, *J* = 1.2 Hz, 1H), 7.30 (d*, J* = 8.0 Hz, 1H), 3.03-2.95 (m, 2H), 2.68-2.5 (m, 2H).

Step 6: Compound **36-5** (0.45 g, 2.3 mmol) was dissolved in tetrahydrofuran (10 mL). The reaction mixture was cooled to -70°C, added dropwise with a solution of lithium diisopropylamide in tetrahydrofuran (1.3 mL, 2.5 mmol, 2 M) under nitrogen atmosphere, reacted at -70°C for 1 hour, then added dropwise with 1,2-dibromotetrachloroethane (0.8 g, 2.3 mmol) in tetrahydrofuran (4 mL), and reacted at - 70°C for 1 hour. TLC showed that the starting material was completely reacted. The reaction mixture was slowly added dropwise with saturated ammonium chloride solution (50 mL), and extracted with ethyl acetate (30 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:100) to obtain compound **36-6** (0.27 g, Y: 43%) as a yellow solid. ¹H NMR (400 MHz, CDCl₃-*d*): δ 7.67 (s, 1H), 3.19-3.12 (m, 2H), 2.74-2.63 (m, 2H).

Step 7: Compound **36-6** (0.27 g, 0.98 mmol) was dissolved in ethanol (3 mL) and water (3 mL), then sodium hydroxide (0.39 g, 9.78 mmol) was added thereto, and the reaction mixture was reacted at 80°C for 6 hours. TLC showed that the starting material was completely reacted. The reaction mixture was cooled to 0°C, added with water (10 mL), and then slowly added with dilute hydrochloric acid (0.5 M) until the pH of the reaction system was 3 to 4. The reaction mixture was extracted with ethyl acetate (30 mL × 3). The organic phases were combined, washed with saturated brine (20 mL), dried over anhydrous sodium sulfate, filtered, and the filtrate was concentrated under reduced pressure to obtain compound **36-7** (0.25 g, crude).

Step 8: Compound **36-7** (0.25 g, 0.85 mmol) was dissolved in N,N-dimethylformamide (2.5 mL), then diisopropylethylamine (0.44 g, 3.41 mmol), 2-(7-azabenzotriazol-1-yl)-N,N,N',N'-tetramethyluronium hexafluorophosphate (0.39 g, 1.00 mmol), and methylamine hydrochloride (0.11 g, 1.69 mmol) were sequentially added thereto, and the reaction mixture was reacted at 25°C for 2 hours. LCMS showed that the starting material was completely reacted. The reaction mixture was added with water (20 mL), and extracted with ethyl acetate (20 mL × 2). The organic phases were combined, washed with saturated brine (15 mL), dried over anhydrous sodium sulfate, and filtered. The filtrate was concentrated under reduced pressure and then purified by silica gel column chromatography (EA: PE = 1:10) to obtain compound **36-8** (60 mg, Y: 22%). LC-MS m/z (ESI): 308.1 [M+H]⁺.

Step 9: Compound **36-8** (60 mg, 0.19 mmol) was dissolved in toluene (1 mL) and (0.2 mL), then compound **1-8** (106 mg, 0.19 mmol), tris(dibenzylideneacetone)dipalladium (20 mg, 0.02 mmol), 4,5-bis(diphenylphosphino)-9,9-dimethylxanthene (22 mg, 0.02 mmol), and potassium carbonate (87 mg, 0.63 mmol) were sequentially added thereto, and the reaction mixture was reacted at 110°C overnight under nitrogen atmosphere. LCMS showed that the starting material was completely reacted. The reaction mixture was cooled to room temperature, concentrated under reduced pressure, and purified by silica gel column chromatography (EA: PE= 1:100) to obtain compound **36-9** (30 mg, Y: 23.8%). LC-MS m/z (ESI): 645.3 [M+H]⁺.

Step 10: Compound **36-9** (30 mg, 46.5 µmol) was dissolved in dichloromethane (0.5 mL), then a solution of hydrochloric acid in ethyl acetate (0.2 mL, 4.0 M) was added thereto, and the reaction mixture was reacted at room temperature for 2 hours. LCMS showed that the starting material was completely reacted. The reaction mixture was concentrated under reduced pressure, and then purified by preparative high-performance liquid chromatography (column model: YMC-Actus Triart C18, 150 * 20 mm, 5 µm; eluent: 0.05%NH₃·H₂O-10 mmol/L NH₄HCO₃-ACN; gradient: 41% to 65%; flow rate: 20 mL/min; run time: 13 min; retention time: 8.8 min/9.5 min, where Rt = 9.5 min was for target product) to obtain compound **36a** (9.0 mg, Y: 35%). ¹H NMR (400 MHz, DMSO-*d₆*): δ 8.40 (br, 1H), 7.66 (s, 1H), 7.43 (d, *J* = 7.2 Hz, 2H), 7.35-7.23 (m, 3H), 7.04 (d, *J* = 10.0 Hz, 1H), 3.60-3.50 (m, 1H), 3.45 (d, *J* = 16.8 Hz, 1H), 3.18-3.08 (m, 2H), 3.90 (d, *J* = 8.4 Hz, 1H), 2.80-2.55 (m, 8H), 1.60-1.35 (m, 4H). LC-MS m/z (ESI): 545.2/547.2 [M+H]⁺.

### Example 37

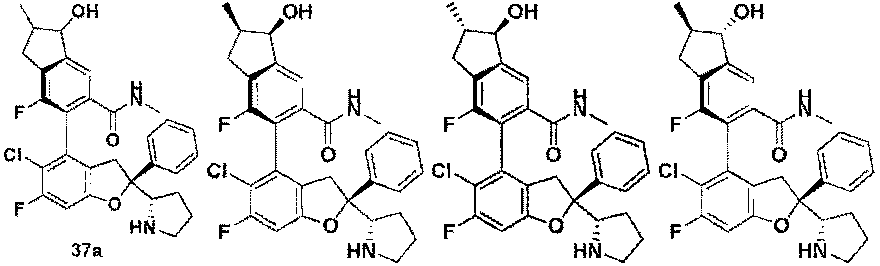

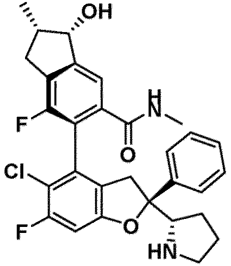

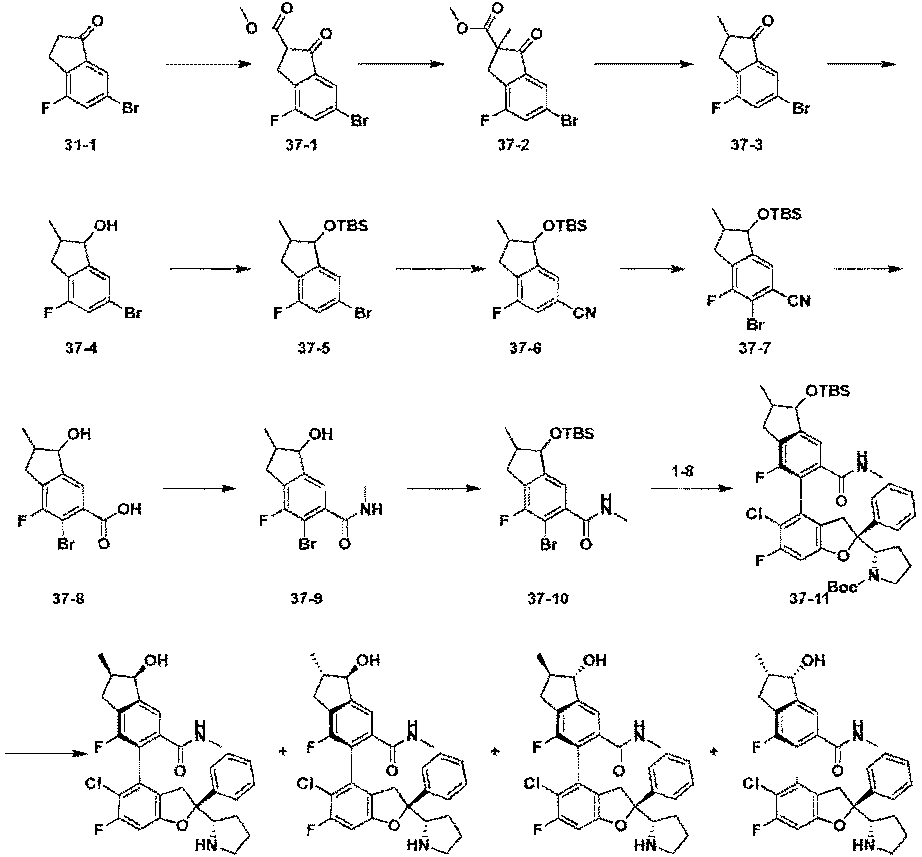

Step 1: Compound **31-1** (60.0 g, 263.2 mmol) was dissolved in anhydrous N,N-dimethylformamide (700 mL). The reaction system was cooled to 0°C, and slowly added with sodium hydride (15.7 g, 392.5 mmol, 60%) and dimethyl carbonate (75.0 g, 833.3 mmol) in batches. After the addition was completed, the reaction system was heated to 60°C and reacted for 1 hour. TLC showed that the starting material was completely reacted. The reaction system was cooled to 0°C, slowly added with ice water (1.0 L) under stirring, and extracted with ethyl acetate (800 mL × 3). The organic phases were combined, washed with saturated brine (600 mL), dried over anhydrous sodium sulfate, filtered, and the filtrate was concentrated under reduced pressure to obtain compound **37-1** (64.0 g, crude).

Step 2: Compound **37-1** (64.0 g, 223.8 mmol) was dissolved in anhydrous N,N-dimethylformamide (700 mL). The reaction mixture was cooled to 0°C, sequentially added with anhydrous potassium carbonate (78.0 g, 565.2 mmol) and iodomethane (38.5 g, 271.1 mmol), and reacted at 50°C for 12 hours under nitrogen atmosphere. TLC showed that the starting material was completely reacted. The reaction mixture was added with water (1.0 L), and extracted with ethyl acetate (700 mL × 3). The organic phases were combined, washed with saturated brine (600 mL), dried over anhydrous sodium sulfate, and filtered. The filtrate was concentrated under reduced pressure and then purified by silica gel column chromatography (EA: PE = 1:60) to obtain compound **37-2** (40.5 g, Y: 60%). ¹H NMR (400 MHz, CDCl₃): δ 7.72 (d, *J* = 1.2 Hz, 1H), 7.48-7.44 (m, 1H), 3.69 (s, 3H), 3.64 (d, *J* = 17.6 Hz, 1H), 2.94 (d, *J* = 17.6 Hz, 1H), 1.53 (s, 3H).

Step 3: Compound **37-2** (40.5 g, 135.0 mmol) was dissolved in methanol (300 mL). The reaction mixture was cooled to 0°C, slowly added with a solution of sodium hydroxide (40.0 g, 1.0 mol) in water (300 mL), and reacted at room temperature for 0.5 hours. TLC showed that the starting material was completely reacted. The reaction mixture was added with water (500 mL), and extracted with ethyl acetate (500 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:100) to obtain compound **37-3** (18.2 g, Y: 55%).

Step 4: Compound **37-3** (18.0 g, 74.4 mmol) was dissolved in methanol (200 mL). The reaction mixture was cooled to 0°C, and added with sodium borohydride (4.3 g, 113.2 mmol) in batches. The reaction mixture was reacted at 0°C for 2 hours. TLC showed that the starting material was completely reacted. The reaction mixture was slowly added with ice water (600 mL), and extracted with ethyl acetate (600 mL × 3). The organic phases were combined, washed with saturated brine (200 mL × 2), dried over anhydrous sodium sulfate, filtered, and the filtrate was concentrated under reduced pressure to obtain compound **37-4** (14.5 g, Y: 80%). ¹H NMR (400 MHz, CDCl₃): δ 7.32 (d, *J* = 14.0 Hz, 1H), 7.13-7.09 (t, *J* = 7.2 Hz, 1H), 5.03-4.70 (m, 1H), 3.18-2.92 (m, 1H), 2.68-2.54 (m, 1H), 2.40-2.24 (m, 1H), 1.28-1.11 (m, 3H).

Step 5: Compound **37-4** (12.0 g, 49.2 mmol) was dissolved in dichloromethane (180 mL), and imidazole (10.0 g, 147.1 mmol) and tert-butyldimethylsilyl chloride (11.0, 73.0 mmol) were sequentially added thereto. The reaction mixture was reacted at room temperature for 12 hours under nitrogen atmosphere. TLC showed that the starting material was completely reacted. The reaction mixture was concentrated under reduced pressure and then purified by silica gel column chromatography (EA: PE = 1:100) to obtain compound **37-5** (13.2 g, Y: 75%). ¹H NMR (400 MHz, CDCl₃): δ 7.16 (d, *J* = 7.6 Hz, 1H), 7.09-7.04 (m, 1H), 5.07-4.69 (m, 1H), 3.15-2.80 (m, 1H), 2.64-2.51 (m, 1H), 2.38-2.25 (m, 1H), 0.98-0.88 (m, 12H), 0.21-0.14 (m, 6H).

Step 6: Compound **37-5** (8.0 g, 22.3 mmol) was dissolved in anhydrous N,N-dimethylformamide (90 mL), and zinc cyanide (1.8 g, 15.4 mmol), 1,1'-bis(diphenylphosphino)ferrocene (2.5 g, 4.5 mmol), and tris(dibenzylideneacetone)dipalladium (2.0 g, 2.2 mmol) were sequentially added thereto. The reaction mixture was reacted at 100°C for 16 hours under nitrogen atmosphere. TLC showed that the starting material was completely reacted. The reaction mixture was cooled to 25°C, poured into ice water (400 mL), and extracted with ethyl acetate (500 mL × 2). The organic phases were combined, washed with saturated brine (100 mL × 2), dried over anhydrous sodium sulfate, and filtered. The filtrate was concentrated under reduced pressure and then purified by silica gel column chromatography (EA: PE = 1:100) to obtain compound **37-6** (5.1 g, Y: 75%). ¹H NMR (400 MHz, CDCl₃): δ 7.31 (d, *J* = 12.0 Hz, 1H), 7.22-7.18 (m, 1H), 5.10-4.71 (m, 1H), 3.21-2.92 (m, 1H), 2.74-2.38 (m, 2H), 1.23-0.96 (m, 3H), 0.95-0.83 (m, 9H), 0.20-0.15 (m, 6H).

Step 7: Compound **37-6** (5.1 g, 16.7 mmol) was dissolved in tetrahydrofuran (50 mL). The reaction mixture was cooled to -70°C, added dropwise with a solution of lithium diisopropylamide in tetrahydrofuran (9.2 mL, 18.4 mmol, 2 M) under nitrogen atmosphere, reacted at -70°C for 1 hour, then added dropwise with 1,2-dibromotetrafluoroethane (5.7 g, 21.9 mmol) in tetrahydrofuran (2 mL), and reacted at -70°C for 1 hour. TLC showed that the starting material was completely reacted. The reaction mixture was slowly added dropwise with saturated ammonium chloride solution (200 mL), and extracted with ethyl acetate (300 mL × 3). The organic phases were combined, washed with saturated brine (150 mL), dried over anhydrous sodium sulfate, and filtered. The filtrate was concentrated under reduced pressure and then purified by silica gel column chromatography (EA: PE = 1:90) to obtain compound **37-7** (4.9 g, Y: 76%). ¹H NMR (400 MHz, CDCl₃): δ 7.31 (d, *J* = 12.4 Hz, 1H), 5.06-4.67 (m, 1H), 3.24-2.92 (m, 1H), 2.80-2.31 (m, 2H), 1.21-0.96 (m, 3H), 0.95-0.92 (m, 9H), 0.20-0.15 (m, 6H).

Step 8: Compound **37-7** (4.9 g, 12.8 mmol) was dissolved in ethanol (6 mL) and water (6 mL), then sodium hydroxide (3.5 g, 87.5 mmol) was added thereto, and the reaction mixture was reacted at 100°C for 16 hours. TLC showed that the starting material was completely reacted. The reaction mixture was cooled to 0°C, added with water (20 mL), and then slowly added with dilute hydrochloric acid (0.5 M) until the pH of the reaction system was 3 to 4. The reaction mixture was extracted with ethyl acetate (100 mL × 3). The organic phases were combined, washed with saturated brine (60 mL), dried over anhydrous sodium sulfate, filtered, and the filtrate was concentrated under reduced pressure to obtain compound **37-8** (2.8 g, crude).

Step 9: Compound **37-8** (2.8 g, 9.7 mmol) was dissolved in N,N-dimethylformamide (20 mL), then diisopropylethylamine (4.0 g, 31.0 mmol), 2-(7-azabenzotriazol-1-yl)-N,N,N',N'-tetramethyluronium hexafluorophosphate (4.1 g, 10.8 mmol), and methylamine hydrochloride (1.0 g, 14.9 mmol) were sequentially added thereto, and the reaction mixture was reacted at room temperature for 2 hours under nitrogen atmosphere. LCMS showed that the starting material was completely reacted. The reaction mixture was added with water (70 mL), and extracted with ethyl acetate (60 mL × 2). The organic phases were combined, washed with saturated brine (40 mL), dried over anhydrous sodium sulfate, and filtered. The filtrate was concentrated under reduced pressure and then purified by silica gel column chromatography (EA: PE = 1:5) to obtain compound **37-9** (2.2 g, Y: 75%). LC-MS m/z (ESI): 302.1/304.1 [M+H]⁺.

Step 10: Compound **37-9** (600 mg, 2.0 mmol) was dissolved in dichloromethane (8 mL), and imidazole (450 mg, 6.6 mmol) and tert-butyldimethylsilyl chloride (590 mg, 3.9 mmol) were sequentially added thereto. The reaction mixture was reacted at room temperature for 12 hours under nitrogen atmosphere. TLC showed that the starting material was completely reacted. The reaction mixture was concentrated under reduced pressure and then purified by silica gel column chromatography (EA: PE = 1:10) to obtain compound **37-10** (680 mg, Y: 82%). ¹H NMR (400 MHz, CDCl₃): δ 7.18 (s, 1H), 5.92 (br, 1H), 5.06-4.67 (m, 1H), 3.24-2.92 (m, 4H), 2.75-2.32 (m, 2H), 1.22-0.98 (m, 3H), 0.97-0.88 (m, 9H), 0.20-0.14 (m, 6H).

Step 11: Compound **37-10** (370 mg, 0.89 mmol) and compound **1-8** (480 mg, 0.88 mmol) were dissolved in toluene (6 mL) and water (1.2 mL), and tris(dibenzylideneacetone)dipalladium (82 mg, 0.09 mmol), 4,5-bis(diphenylphosphino)-9,9-dimethylxanthene (100 mg, 0.17 mmol), and anhydrous potassium carbonate (370 mg, 2.68 mmol) were sequentially added thereto. The reaction mixture was reacted at 110°C overnight under nitrogen atmosphere. LCMS showed that the starting material was completely reacted. The reaction mixture was cooled to 25°C, concentrated under reduced pressure, and purified by silica gel column chromatography (EA: PE = 1:5 to 1:1, R_{f} = 0.6 and R_{f} = 0.5, where Rf = 0.5 was for target product) to obtain compound **37-11** (100 mg, Y: 15%). LC-MS (1.5 mL-3.2 min-5 to 95% B): RT = 2.03 min, m/z (ESI): 753.5 [M+H]⁺.

Step 12: Compound **37-11** (100 mg, 0.13 mmol) was dissolved in hydrochloric acid/dioxane solution (1 mL, 4.0 M), and the reaction mixture was reacted at 25°C for 0.5 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.05% NH₃H₂O-10 mmol/L NH₄HCO₃-CH₃CN; gradient: 19 to 39% CH₃CN; flow rate: 20 mL/min; run time: 12 min; retention time: Rt = 7.5 min/7.7 min/7.9 min/8.3 min) to obtain a mixture of three compounds. Compound **37a1** (9.3 mg, Y: 12.9%, Rt = 7.5 min/7.7 min), compound **37a2** (23.9 mg, Y: 33.3%, Rt = 7.7 min/7.9 min), and compound **37a3** (12.9 mg, Y: 18%, Rt = 8.3 min).

**37a1:** ¹H NMR (400 MHz, DMSO-*d₆*): δ 8.23-8.15 (m, 2H), 7.44-7.22 (m, 7H), 6.99 (d, *J* = 9.6 Hz, 1H), 4.97-4.61 (m, 1H), 3.58-3.40 (m, 3H), 3.10-2.55 (m, 7H), 2.54-2.52 (m, 1H), 2.48-2.20 (m, 3H), 1.59-1.38 (m, 4H), 1.23-1.00 (m, 6H). LC-MS m/z (ESI): 529.2/531.2 [M+H]⁺. Chiral analysis (column model: Amylose-SA, 100 * 4.6 mm, 5 µm; eluent: hexane (0.1% DEA)-IPA; gradient: isocratic 9/1, flow rate: 1 mL/min; run time: 8.2 min; retention time: 4.21 min/4.71 min, dr = 54 (4.21 min):46 (4.71 min)).

**37a2:** ¹H NMR (400 MHz, DMSO-*d₆*): δ 8.23-8.14 (m, 3H), 7.43-7.24 (m, 12H), 6.99 (d*, J* = 13.2 Hz, 2H), 4.97 (d, *J* = 6.0 Hz, 1H), 4.62 (d, *J* = 7.6 Hz, 1H), 3.57-3.48 (m, 6H), 3.10-2.70 (m, 6H), 2.62 (d, *J* = 4.4 Hz, 6H), 2.60-2.54 (m, 1H), 2.48-2.20 (m, 6H), 1.60-1.35 (m, 8H), 1.22 (d, *J* = 6.8 Hz, 3H), 1.02 (d, *J* = 7.2 Hz, 3H). LC-MS m/z (ESI): 539.2/541.3 [M+H]⁺. Chiral analysis (column model: Amylose-SA, 100 * 4.6 mm, 5 µm; eluent: hexane (0.1% DEA)-IPA; gradient: isocratic 9/1, flow rate: 1 mL/min; run time: 9.8 min; retention time: 4.60 min/6.10 min, dr = 48 (4.60 min):52 (6.10 min)).

**37a3:** ¹H NMR (400 MHz, DMSO-*d₆*): δ 8.18-8.12 (m, br, 2H), 7.40-7.21 (m, 6H), 6.97 (d, *J* = 9.6 Hz, 1H), 4.62 (d, *J* = 7.6 Hz, 1H), 3.54-3.38 (m, 3H), 3.06-3.00 (m, 1H), 2.84-2.68 (m, 2H), 2.58 (d, *J* = 4.4 Hz, 3H), 2.40-2.33 (m, 3H), 1.58-1.33 (m, 4H), 1.18 (d, *J* = 6.8 Hz, 3H). LC-MS m/z (ESI): 539.3/541.3 [M+H]⁺. Chiral analysis (column model: Amylose-SA, 100 * 4.6 mm, 5 µm; eluent: hexane (0.1% DEA)-IPA; gradient: isocratic 9/1, flow rate: 1 mL/min; run time: 12.5 min; retention time: 6.20 min/6.81 min, dr = 8 (6.20 min):92 (6.81 min)).

### Example 38

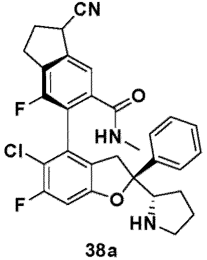

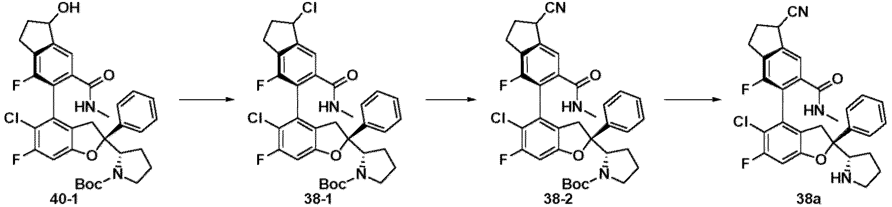

Step 1: Compound **40-1** (100 mg, 0.16 mmol) was dissolved in anhydrous dichloromethane (2.0 mL), then compound triethylamine (48.5 mg, 0.48 mmol) and methanesulfonyl chloride (36.6 mg, 0.32 mmol) were sequentially added thereto under nitrogen atmosphere, and the reaction mixture was reacted at 25°C for 3 hours. TLC (PE: EA = 1:1) showed that the starting material was completely reacted. The reaction mixture was poured into saturated ammonium chloride (10 mL) to quench, and extracted with ethyl acetate (10 mL × 3). The organic phases were combined, washed with saturated brine (10 mL), dried over anhydrous sodium sulfate, and filtered. The filtrate was concentrated under reduced pressure and then purified by silica gel column chromatography (PE: EA = 5:1) to obtain compound **38-1** (70 mg, Y: 68%). LC-MS m/z (ESI): 643.3/645.3 [M+H]⁺.

Step 2: Compound **38-1** (70 mg, 0.11 mmol) was dissolved in anhydrous tetrahydrofuran (3 mL), then compound trimethylsilyl cyanide (21.5 mg, 0.22 mmol) and tetrabutylammonium fluoride (tetrahydrofuran solution) (1 M, 0.2 mL) were sequentially added thereto under nitrogen atmosphere, and the reaction mixture was reacted at 60°C for 3 hours. TLC (PE: EA = 2:1) showed that the starting material was completely reacted. The reaction mixture was cooled to 25°C, poured into saturated brine (10 mL) to quench, and extracted with ethyl acetate (10 mL × 3). The organic phases were combined, dried over anhydrous sodium sulfate, and filtered. The filtrate was concentrated under reduced pressure and then purified by silica gel column chromatography (PE: EA = 1:1) to obtain compound **38-2** (35 mg, Y: 50%). LC-MS m/z (ESI): 634.6 [M+H]⁺.

Step 3: Compound **38-2** (35 mg, 0.052 mmol) was dissolved in dichloromethane (4 mL), then compound trifluoroacetic acid (1 mL) was sequentially added thereto under nitrogen atmosphere, and the reaction mixture was reacted at room temperature for 2 hours. TLC (PE: EA = 1:2) showed that the starting material was completely reacted. The reaction mixture was poured into saturated sodium carbonate (10 mL), and extracted with ethyl acetate (20 mL × 3). The organic phases were combined, washed with saturated brine (10 mL), dried over anhydrous sodium sulfate, and filtered. The filtrate was concentrated under reduced pressure to obtain a yellow oil, which was then purified by preparative high-pressure liquid chromatography (column model: Xselect CSH Prep Fluoro-Phenyl, 150 * 19 mm, 5 µm; eluent: 0.1% HCl-CAN; gradient: 44% to 64%; flow rate: 20 mL/min; run time: 12 min; retention time: 8.5 min) to obtain compound **38a** (2.40 mg, Y: 8.14%). ¹H NMR (400 MHz, DMSO-*d₆*) δ 8.28-8.23 (m, 1H), 8.15-8.11 (m, 1H), 7.53-7.24 (m, 6H), 7.05-6.98 (m, 1H), 5.54-5.48 (m, 1H), 4.68-4.61 (m, 1H), 3.55-3.40 (m, 3H), 3.12-2.71 (m, 6H), 2.65-2.59 (m, 3H), 1.62-1.34(m, 4H). LC-MS m/z (ESI): 534.1 [M+H]⁺. Chiral analysis (column model: Celluiose-SC, 100 * 4.6 mm, 5 µm; eluent: hexane (0.1% DEA)/IPA; gradient: isocratic 9/1, flow rate: 1 mL/min; run time: 50 min; retention time: 27.08 min/39.51 min, dr = 32:68).

### Example 40

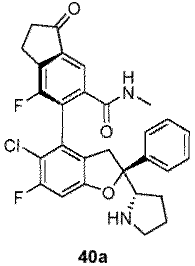

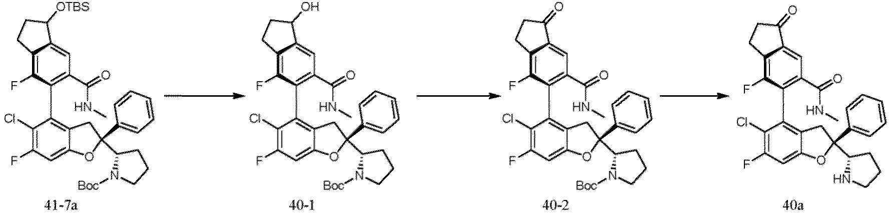

Step 1: Compound **41-7a** (200.0 mg, 270 µmol) was dissolved in tetrahydrofuran (20 mL). The reaction mixture was cooled to 0°C under nitrogen atmosphere, added with tetrabutylammonium fluoride (1 M, 0.54 mL), gradually warmed to 25°C, and reacted for 2 hours. LCMS showed that the starting material was completely reacted. The reaction mixture was added with water (50 mL) to quench, and extracted with ethyl acetate (50 mL × 3). The organic phases were combined, washed with saturated brine (50 mL × 3), dried over anhydrous sodium sulfate, and filtered. The filtrate was concentrated under reduced pressure and then purified by silica gel column chromatography (PE: EA = 10:1) to obtain compound **40-1** (130.0 mg, Y: 76%). LC-MS m/z (ESI): 625.3 [M+H]⁺.

Step 2: Compound **40-1** (130 mg, 208 µmol) was dissolved in dichloromethane (20 mL). The reaction mixture was cooled to 0°C under nitrogen atmosphere, added with Dess-Martin periodinane (176 mg, 416 µmol) in batches, warmed from 0°C to room temperature, and reacted for 3 hours. LCMS showed that the starting material was completely reacted. The reaction mixture was added with water (50 mL) to quench, and extracted with ethyl acetate (30 mL × 3). The organic phases were combined, washed with saturated brine (30 mL × 3), dried over anhydrous sodium sulfate, and filtered. The filtrate was concentrated under reduced pressure and then purified by silica gel column chromatography (PE: EA = 10:1) to obtain compound **40-2** (70.0 mg, Y: 54%) as a white solid. LC-MS m/z (ESI): 623.4 [M+H]⁺.

Step 3: Compound **40-2** (60 mg, 0.96 mol) was dissolved in dichloromethane (1 mL), then a solution of hydrochloric acid in dioxane (4 M, 1 mL) was added thereto, and the reaction mixture was reacted at 25°C for 2 hours. LCMS showed that the starting material was completely reacted. The filtrate was concentrated under reduced pressure to obtain a yellow solid, poured into ice-cold saturated sodium bicarbonate aqueous solution (10 mL) to quench, and extracted with ethyl acetate (10 mL × 3). The organic phases were combined, dried over anhydrous sodium sulfate, and filtered. The filtrate was concentrated under reduced pressure and then purified by preparative high-performance liquid chromatography (column model: Xbridge Prep Phenyl OBD, 150 * 19 mm, 5 µm; eluent: 0.05% FA/CH₃CN; gradient: 50% to 65%; flow rate: 20 mL/min; run time: 15 min; retention time: 11.5 min) to obtain compound **40a** (9.3 mg, Y: 18%). ¹H NMR (400 MHz, DMSO-*d₆*): δ 8.49 (s, 1H), 8.19 (s, 1H), 7.73 (s, 1H), 7.47-7.26 (m, 5H), 7.06 (d, *J* = 9.6 Hz, 1H), 3.62-3.55 (m, 2H), 3.48-3.39 (m, 1H), 3.19-3.08 (m, 2H), 2.92-2.88 (m, 1H), 2.82-2.74 (m, 2H), 2.71-2.61 (m, 4H), 1.68-1.35 (m, 4H). LC-MS m/z (ESI): 523.2 [M+H]⁺.

### Example 41

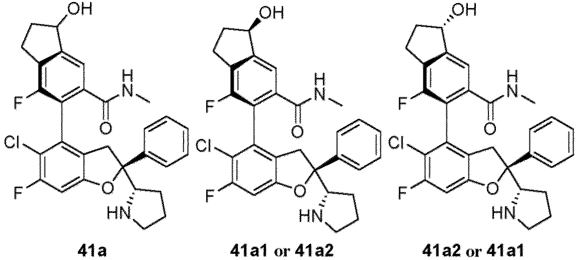

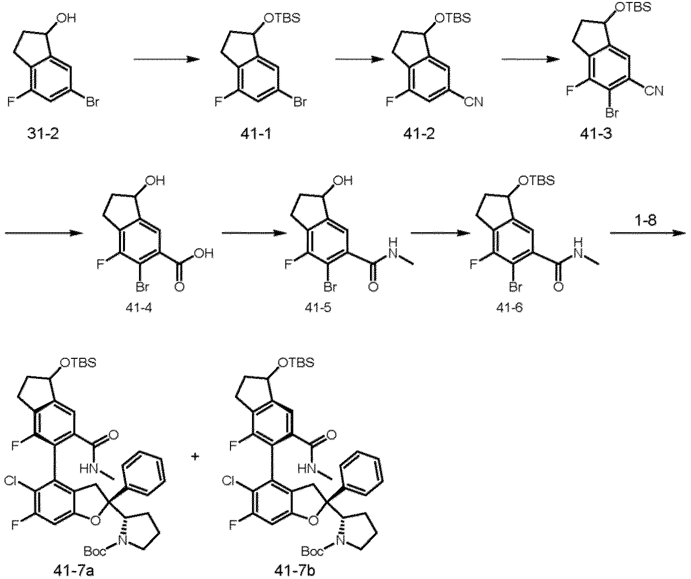

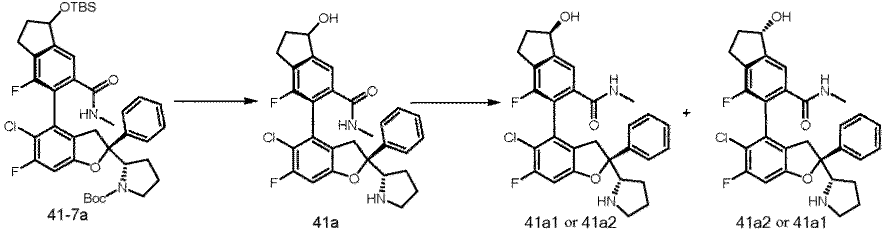

Step 1: Compound **31-2** (20.0 g, 86.56 mmol) was dissolved in dichloromethane (200 mL), then imidazole (11.79 g, 173.11 mmol) and tert-butyldimethylsilyl chloride (19.57 g, 129.84 mmol) were sequentially added to the above reaction mixture, and the reaction mixture was reacted at 25°C for 12 hours. TLC (EA: PE = 1:10) showed that the starting material was completely reacted. The reaction mixture was diluted with water, and extracted with dichloromethane (200 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:20) to obtain compound **41-1** (29.0 g, Y: 94%).

Step 2: Compound **41-1** (10.0 g, 28.96 mmol) was dissolved in N,N-dimethylformamide (100 mL), then zinc cyanide (5.44 g, 46.33 mmol), tris(dibenzylideneacetone)dipalladium (2.65 g, 2.90 mmol), and 1,1'-bis(diphenylphosphino)ferrocene (3.21 g, 5.79 mmol) were sequentially added to the above reaction mixture, and the reaction mixture was reacted at 100°C for 16 hours under nitrogen atmosphere. TLC (EA: PE = 1:15) showed that the starting material was completely reacted. The reaction mixture was cooled to 25°C, added with water (200 mL), and extracted with ethyl acetate (100 mL × 3). The organic phases were combined, washed with saturated brine (100 mL), dried over anhydrous sodium sulfate, and filtered. The filtrate was concentrated under reduced pressure and then purified by silica gel column chromatography (PE: EA = 1:25) to obtain compound **41-2** (7.0 g, Y: 82%).

Step 3: Compound **41-2** (5.0 g, 17.16 mmol) was dissolved in anhydrous tetrahydrofuran (100 mL). The reaction system was cooled to -70°C, added dropwise with lithium diisopropylamide (9.4 mL, 18.8 mmol, 2 M), and reacted at -70°C for 1 hour. The reaction mixture was added dropwise with 1,2-dibromotetrachloroethane (5.59 g, 17.16 mmol) at -70°C, reacted at -70°C for 1 hour, slowly warmed to room temperature, and reacted for 1 hour. TLC showed that the starting material was completely reacted. The reaction mixture was added dropwise with ice-cold ammonium chloride aqueous solution (200 mL) to quench, and extracted with ethyl acetate (100 mL × 3). The organic phases were combined, washed with saturated brine (200 mL), dried over anhydrous sodium sulfate, and filtered. The filtrate was concentrated under reduced pressure and then purified by silica gel column chromatography (EA: PE = 1:20) to obtain compound **41-3** (5.5 g, Y: 86%). ¹H NMR (400 MHz, CDCl₃): δ 7.36 (s, 1H), 5.21 (t, *J* = 7.2 Hz, 1H), 3.19-3.09 (m, 1H), 2.89-2.79 (m, 1H), 2.56-2.46 (m, 1H), 2.04-1.94 (m, 1H), 0.93 (s, 9H), 0.16 (d, *J* = 9.6 Hz, 6H).

Step 4: Compound **41-3** (5.0 g, 13.5 mmol) was dissolved in ethanol (50 mL), then sodium hydroxide (5.4 g, 135.0 mmol) dissolved in water (50 mL) was added thereto, and the reaction mixture was reacted at 100°C for 16 hours. LCMS showed that the starting material was completely reacted. The reaction mixture was cooled to room temperature, added with hydrochloric acid (1 M) to adjust the pH to 5 to 6, and extracted with ethyl acetate (50 mL × 3). The organic phases were combined, washed with saturated brine (100 mL), dried over anhydrous sodium sulfate, filtered, and the filtrate was concentrated under reduced pressure to obtain compound **41-4** (3.1 g, Y: 84%). LC-MS m/z (ESI): 273.0 [M-H]⁺.

Step 5: Compound **41-4** (3.1 g, 11.3 mmol) was dissolved in N,N-dimethylformamide (50 mL), then N,N-diisopropylethylamine (4.3 g, 33.8 mmol), methylamine hydrochloride (1.5 g, 22.5 mmol), and 2-(7-azabenzotriazol-1-yl)-N,N,N',N'-tetramethyluronium hexafluorophosphate were sequentially added thereto, and the reaction mixture was reacted at room temperature for 2 hours. LCMS showed that the starting material was completely reacted. The reaction mixture was added with water (100 mL) to quench, and extracted with ethyl acetate (100 mL × 3). The organic phases were combined, washed with saturated brine (100 mL), dried over anhydrous sodium sulfate, and filtered. The filtrate was concentrated under reduced pressure and then purified by silica gel column chromatography (THF: PE = 1:1) to obtain compound **41-5** (2.1 g, Y: 63%). LC-MS m/z (ESI): 288.0 [M+H]⁺.

Step 6: Compound **41-5** (1.6 g, 5.57 mmol) and imidazole (1.1 g, 16.7 mmol) were dissolved in dichloromethane (20 mL). The reaction mixture was replaced with nitrogen three times, stirred at 30°C for 0.5 hours, then added with tert-butyldimethylsilyl chloride (1.7 g, 11.1 mmol), and reacted for 16 hours. LCMS showed that the starting material was completely reacted. The reaction mixture was poured into water (30 mL) to quench, and extracted with dichloromethane (20 mL × 3). The organic phases were combined, washed with saturated brine (20 mL), dried over anhydrous sodium sulfate, and filtered. The filtrate was concentrated under reduced pressure and then purified by silica gel column chromatography (THF: PE = 1:4) to obtain compound **41-6** (1.4 g, Y: 69%).

Step 7: Compound **41-6** (200 mg, 0.5 mmol) and compound **1-8** (326 mg, 0.6 mmol) were dissolved in toluene (3 mL) and water (0.5 mL), then tris(dibenzylideneacetone)dipalladium (92 mg, 0.1 mmol), 4,5-bis(diphenylphosphino)-9,9-dimethylxanthene (120 mg, 0.2 mmol), and anhydrous potassium carbonate (210 mg, 1.5 mmol) were sequentially added thereto, and the reaction mixture was reacted at 110°C overnight under nitrogen atmosphere. LCMS showed that the starting material was completely reacted. The reaction mixture was cooled to 25°C, concentrated under reduced pressure, and then subjected to silica gel column chromatography (EA: PE = 1:4) to obtain compound **41-7a** (80 mg, Y: 21%) and compound **41-7b** (150 mg, Y: 40%). **41-7a:** LC-MS (1.5 mL-5 min-5 to 90% B): Rt = 3.72 min, m/z (ESI): 739.0 [M+H]⁺. **41-7b:** LC-MS (1.5 mL-5 min-5 to 90% B): Rt = 3.65 min, m/z (ESI): 739.0 [M+H]⁺.

Step 8: Compound **41-7a** (80 mg, 0.1 mmol) was dissolved in dichloromethane (5 mL), then trifluoroacetic acid (1 mL) was added thereto, and the reaction mixture was reacted at room temperature for 2 hours. LCMS showed that the starting material was completely reacted. The reaction mixture was cooled to 0°C, poured into saturated sodium bicarbonate aqueous solution (10 mL) to quench, and extracted with dichloromethane (10 mL × 3). The organic phases were combined, washed with saturated brine (10 mL), dried over anhydrous sodium sulfate, and filtered. The filtrate was concentrated under reduced pressure to obtain a brown solid, which was then purified by preparative high-performance liquid chromatography (column model: Xselect CSH Prep Fluoro-Phenyl, 150 * 19 mm, 5 µm; eluent: 0.05% NH₃H₂O-10 mmol/L NH₄HCO₃-ACN; gradient: 57% to 77%; flow rate: 20 mL/min; run time: 12 min; retention time: 8.5 min) to obtain compound **41a** (12.2 mg, Y: 21%). ¹H NMR (400 MHz, DMSO-*d₆*): δ 8.25-8.15 (m, 1H), 7.49-7.40 (m, 3H), 7.38-7.27 (m, 3H), 7.06-6.98 (m, 1H), 5.65-5.58 (m, 1H), 5.20-5.11 (m, 1H), 3.68-3.52 (s, 1H), 3.50-3.39 (m, 1H), 3.03-2.91 (m, 1H), 2.90-2.84 (m, 1H), 2.81-2.68 (m, 4H), 2.65-2.60 (m, 3H), 1.95-1.80 (m, 1H), 1.71-1.35 (m, 4H), 1.30-1.20 (m, 1H). LC-MS m/z (ESI): 525.2 [M+H]⁺.

Step 9: Compound **41a** (78 mg) was subjected to chiral resolution (chiral column model: Cellulose-SB 100 × 4.6 mm 3.0 µm, CO₂/(methanol+20 mM NH₃), 10%, 35°C, flow rate: 3.0 mL/min) to obtain compound **41a1** (34.3 mg, Y: 24%) and compound **41a2** (26.4 mg, Y: 18%). **41a1:** ¹H NMR (400 MHz, DMSO-*d₆*) δ 8.38-8.32 (m, 1H), 7.53-7.35 (m, 6H), 7.12 (d, *J* = 9.2 Hz, 1 H), 5.16-5.10 (m, 1H), 4.27-4.21 (m, 1H), 3.55-3.45 (m, 2H), 3.24-2.70 (m, 6H), 2.64 (d, *J* = 3.6 Hz, 3H), 2.20-1.65 (m, 4H). LC-MS (1.5 mL-3.2 min-5-95-FA): Rt = 1.48, m/z (ESI): 525.2 [M+H]⁺. Chiral analysis (column model: Celluiose-SB, 100 * 4.6 mm, 5 µm; eluent: hexane/IPA; gradient: isocratic 7/3, flow rate: 1 mL/min; run time: 7 min; retention time: 2.43 min). **41a2:** ¹H NMR (400 MHz, DMSO-d6) δ 8.19-8.11 (m, 1H), 7.47-7.23 (m, 6H), 7.00 (d, *J* = 9.2 Hz, 1 H), 5.59 (d, *J* = 9.6 Hz, 1H), 5.21-5.14 (m, 1H), 3.59-3.40 (m, 3H), 3.03-2.70 (m, 6H), 2.62 (d, *J* = 4.4 Hz, 3H), 1.94-1.82 (m, 1H), 1.64-1.34 (m, 3H). LC-MS (1.5 mL-3.2 min-5-95-FA): Rt = 1.51, m/z(ESI): 525.2 [M+H]⁺. Chiral analysis (column model: Celluiose-SB, 100 * 4.6 mm, 5 µm; eluent: hexane/IPA; gradient: isocratic 7/3, flow rate: 1 mL/min; run time: 7 min; retention time: 3.37 min).

### Example 42

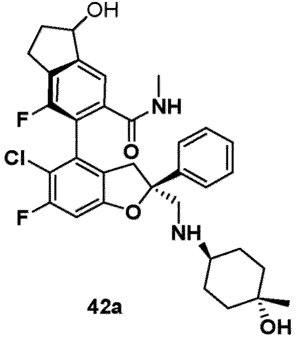

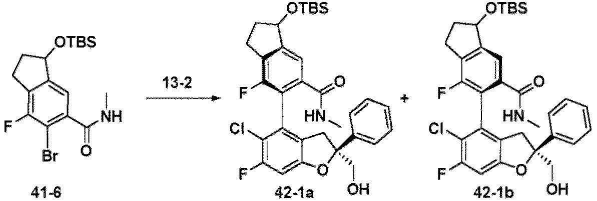

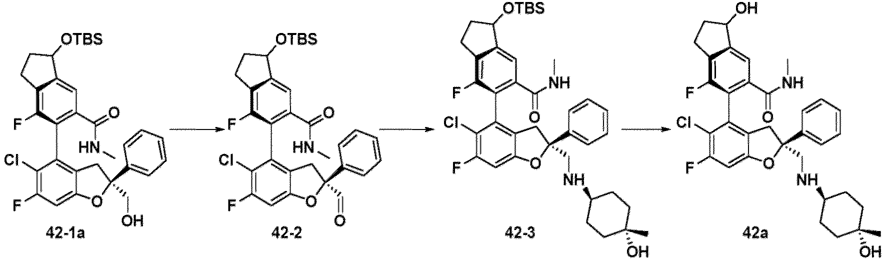

Step 1: Compound **41-6** (200 mg, 0.50 mmol) and compound **13-2** (325 mg, 0.81 mmol) were dissolved in toluene (5 mL) and water (1 mL), then tris(dibenzylideneacetone)dipalladium (91 mg, 0.11 mmol), 4,5-bis(diphenylphosphino)-9,9-dimethylxanthene (116 mg, 0.21 mmol), and anhydrous potassium carbonate (319 mg, 1.5 mmol) were sequentially added thereto, and the reaction mixture was reacted at 100°C for 16 hours under nitrogen atmosphere. Two batches were added in parallel. LCMS showed that the starting material was completely reacted. The reaction mixture was cooled to 25°C, concentrated under reduced pressure, and then purified by silica gel column chromatography (EA: PE = 3:1) to obtain compound **42-1b** (164 mg, Y: 27%, R_{f} = 0.3) and compound **42-1a** (160 mg, Y: 26%, R_{f} = 0.2). LC-MS (1.5 mL-2.0 min-5 to 95% B): Rt = 1.633, m/z (ESI): 600.0 [M+H]⁺.

Step 2: Compound **42-1a** (100 mg, 0.17 mmol) was dissolved in dichloromethane (2 mL), then Dess-Martin periodinane (140 mg, 0.33 mmol) was added thereto under nitrogen atmosphere, and the reaction mixture was reacted at room temperature for 2 hours. TLC (PE: EA = 1:1) showed that the starting material was completely reacted. The reaction mixture was poured into saturated sodium sulfite (5 mL) to quench, and extracted with ethyl acetate (10 mL × 3). The organic phases were combined, washed with saturated sodium carbonate (10 mL) and saturated brine (10 mL), dried over anhydrous sodium sulfate, filtered, and the filtrate was concentrated under reduced pressure to obtain compound **42-2** (90 mg, Y: 90%).

Step 3: Compound **42-2** (90 mg, 0.17 mmol) was dissolved in 1,2-dichloroethane (2 mL). The reaction mixture was sequentially added with trans-4-amino-1-methylcyclohexanol (32.2 mg, 0.34 mmol), chlorotitanium triisopropoxide (43.7 mg, 0.17 mmol), and sodium triacetoxyborohydride (31.5 mg, 0.51 mmol) under nitrogen atmosphere, heated to 85°C, and stirred for 16 hours. LCMS showed that the starting material was completely reacted. The reaction mixture was cooled to 25°C, poured into saturated sodium bicarbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silicagel column chromatography (PE: EA= 1:1) to obtain compound **42-3** (30.00 mg, Y: 25%) as a yellow solid. LC-MS m/z (ESI): 711.3 [M+H]⁺.

Step 4: Compound **42-3** (30 mg, 0.042 mmol) was dissolved in dichloromethane (1 mL). The reaction mixture was cooled to 0°C under nitrogen atmosphere, added with trifluoroacetic acid (0.2 mL), and stirred at room temperature for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: YMC-Actus Triart C18, 150 * 20 mm, 5 µm; eluent: 0.05% NH₃·H₂O/CH₃CN; gradient: 40% to 50%; flow rate: 20 mL/min; run time: 10 min; retention time: 8.5 min) to obtain compound **42a** (10.7 mg, Y: 42). ¹H NMR (400 MHz, DMSO-*d₆*) δ 8.27-8.16 (m, 1H), 7.48-7.20 (m, 6H), 7.02 (d, *J* = 9.6 Hz, 1H), 6.11-6.00 (m, 1H), 5.59-5.52 (m, 1H), 5.20-5.10 (m, 1H), 4.04 (s, 1H), 3.49-3.41 (m, 2H), 3.01-2.69 (m, 4H), 2.66-2.62 (m, 3H), 1.72-1.16 (m, 10H), 1.01 (s, 3H). LC-MS m/z (ESI): 597.2 [M+H]⁺. Chiral analysis (column model: Amylose-C, 100 * 4.6 mm, 3 µm; eluent: hexane (0.1% DEA)/IPA; gradient: isocratic 8/2, flow rate: 1 mL/min; run time: 7 min; retention time: 2.86 min and 3.75 min; dr = 52 (2.86 min):48 (3.75 min)).

### Example 43

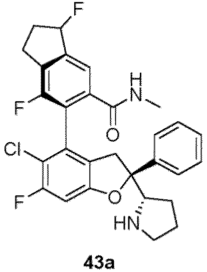

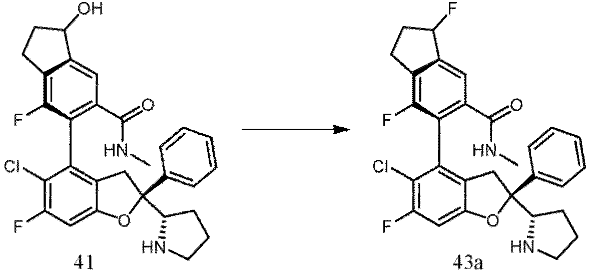

Step 1: Compound **41** (34 mg, 0.06 mmol) was dissolved in dichloromethane (5 mL), then diethylaminosulfur trifluoride (20 mg, 0.12 mmol) was added thereto, and the reaction mixture was reacted at room temperature for 2 hours. LCMS showed that the starting material was completely reacted. The reaction mixture was cooled to 0°C, poured into ice-cold saturated sodium bicarbonate aqueous solution (10 mL) to quench, and extracted with ethyl acetate (10 mL × 3). The organic phases were combined, washed with water (10 mL × 3), dried over anhydrous sodium sulfate, and filtered. The filtrate was concentrated under reduced pressure to obtain a yellow solid, which was then subjected to preparative high-performance liquid chromatography (column model: Xbridge Prep Phenyl OBD, 150 * 19 mm, 5 µm; eluent: 0.05% NH₃·H₂O/CH₃CN; gradient: 44% to 64%; flow rate: 20 mL/min; run time: 11 min; retention time: 7.6 min) to obtain compound **43a** (2.0 mg, Y: 6%) as a white solid. ¹H NMR (400 MHz, DMSO-*d₆*): δ 8.31-8.23 (m, 1H), 7.61 (s, 1H), 7.50-7.15 (m, 6H), 7.02 (d, *J* = 10.0 Hz, 1H), 6.23-6.05 (m, 1H), 5.36-5.28 (m, 1H), 3.02-2.80 (m, 3H), 2.76-2.69 (m, 1H), 2.63 (d, *J* = 4.0 Hz, 3H), 2.09-1.92 (m, 2H), 1.62-1.31 (m, 5H), 0.87-0.83 (m, 1H). LC-MS m/z (ESI): 527.3 [M+H]⁺.

### Example 44

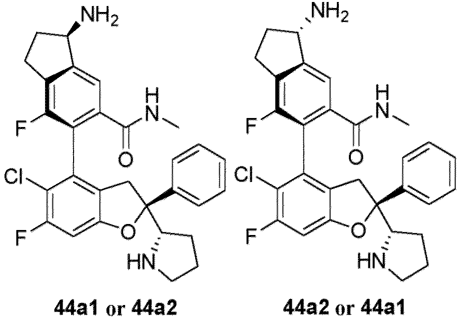

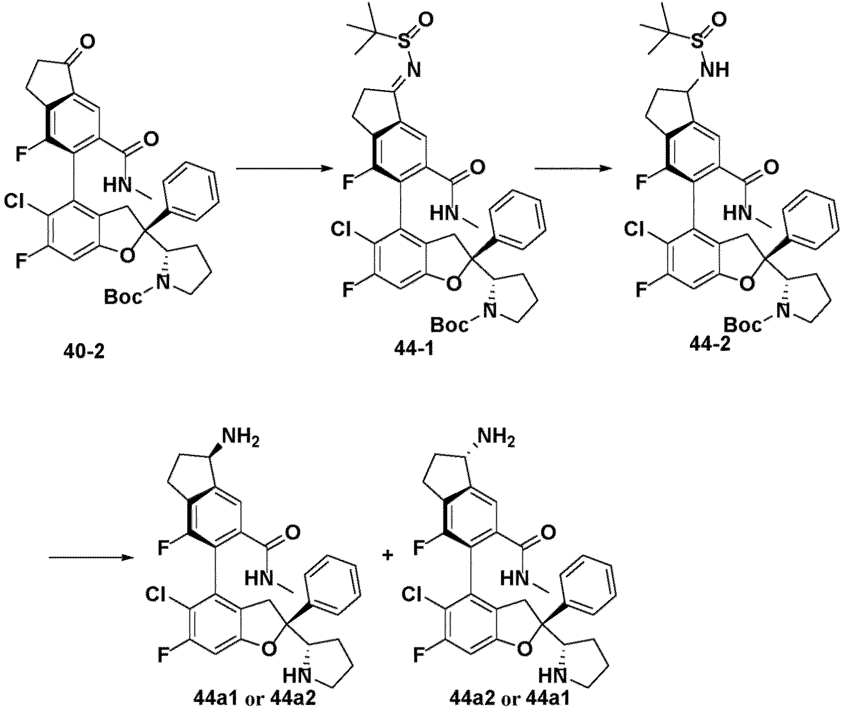

Step 1: Compound **40-2** (100 mg, 0.16 mmol) was dissolved in anhydrous tetrahydrofuran (2 mL), then compound tert-butanesulfinamide (38.9 mg, 0.32 mmol) and titanium ethoxide (109 mg, 0.48 mmol) were sequentially added thereto under nitrogen atmosphere, and the reaction mixture was reacted at 90°C for 6 hours. TLC (PE: EA = 2:1) showed that the starting material was completely reacted. The reaction mixture was cooled to 25°C, poured into saturated sodium bicarbonate (20 mL) to quench, and extracted with ethyl acetate (10 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 10:1) to obtain compound **44-1** (100 mg, Y: 85%). LC-MS m/z (ESI): 726.4 [M+H]⁺.

Step 2: Compound **44-1** (100 mg, 0.13 mmol) was dissolved in tetrahydrofuran (1 mL) and methanol (0.2 mL). The reaction mixture was cooled to 0°C under nitrogen atmosphere, added with sodium borohydride (10.4 mg, 0.25 mmol) in batches, stirred for 5 minutes, warmed to room temperature, and reacted for 2 hours. TLC (PE: EA = 1:1) showed that the starting material was completely reacted. The reaction mixture was cooled to 0°C, poured into saturated ammonium chloride (20 mL) to quench, and extracted with ethyl acetate (10 mL × 3). The organic phases were combined, washed with saturated brine (20 mL), dried over anhydrous sodium sulfate, filtered, and the filtrate was concentrated under reduced pressure to obtain compound **44-2** (70 mg, Y: 69%). LC-MS m/z (ESI): 728.4 [M+H]⁺.

Step 3: Compound **44-2** (70 mg, 0.096 mmol) was dissolved in 1,4-dioxane (1 mL), then a solution of hydrogen chloride in dioxane (4 M, 1 mL) was added thereto under nitrogen atmosphere, and the reaction mixture was reacted at room temperature for 1 hour. TLC (PE: EA = 1:2) showed that the starting material was completely reacted. The reaction mixture was poured into saturated sodium carbonate (10 mL), and extracted with ethyl acetate (20 mL × 3). The organic phases were combined, washed with saturated brine (10 mL), dried over anhydrous sodium sulfate, filtered, and the filtrate was concentrated under reduced pressure to obtain a yellow oil. The yellow oil was purified by preparative high-performance liquid chromatography (column model: Xselect CSH Prep Fluoro-Phenyl, 150 * 19 mm, 5 µm; eluent: 0.1% HCl-CAN; gradient: 40% to 60%; flow rate: 20 mL/min; run time: 12 min) to obtain compound **44a1** (9.1 mg, Y: 18%, retention time: 5.4 min) and compound **44a2** (2.5 mg, Y: 5%, retention time: 6.3 min). **44a1:** ¹H NMR (400 MHz, DMSO-*d₆*) δ 9.57-9.42 (m, 1H), 8.75-8.63 (m, 3H), 8.50-8.40 (m, 1H), 8.35-8.28 (m, 1H), 7.85 (s, 1H), 7.53-7.34 (m, 5H), 7.16 (d, *J* = 9.2 Hz, 1H), 4.86-4.75 (m, 1H), 4.32-4.21 (m, 1H), 3.63-3.55 (m, 1H)), 3.53-3.40 (m, 1H), 3.23-2.84 (m, 5H), 2.65 (d, *J* = 4.4 Hz, 3H), 2.20-1.61 (m, 5H). LC-MS m/z (ESI): 524.2 [M+H]⁺. Chiral analysis (column model: Celluiose-SC, 100 * 4.6 mm, 5 µm; eluent: hexane (0.1% DEA)/IPA; gradient: isocratic 7/3, flow rate: 1 mL/min; run time: 17.5 min; retention time: 8.87 min). **44a2:** ¹H NMR (400 MHz, DMSO-*d₆*) δ 9.57-9.42 (m, 1H), 8.81-8.67 (m, 3H), 8.46-8.33 (m, 2H), 7.82 (s, 1H), 7.56-7.34 (m, 5H), 7.17 (d, *J* = 9.6 Hz, 1H), 4.89-4.75 (m, 1H), 4.45-4.33 (m, 1H), 3.68-3.57 (m, 1H)), 3.53-3.40 (m, 1H), 3.23-2.83 (m, 5H), 2.64 (d, *J* = 4.8 Hz, 3H), 2.22-1.68 (m, 4H). LC-MS m/z (ESI): 524.2 [M+H]⁺. Chiral analysis (column model: Celluiose-SB, 100 * 4.6 mm, 5 µm; eluent: hexane/IPA; gradient: isocratic 7/3, flow rate: 1 mL/min; run time: 17.5 min; retention time: 9.60 min).

### Example 45

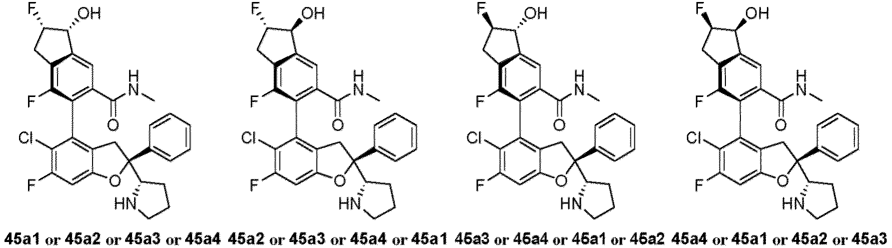

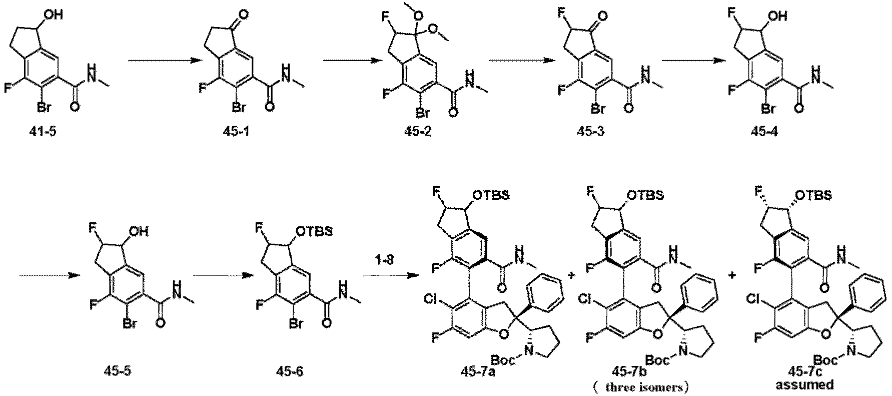

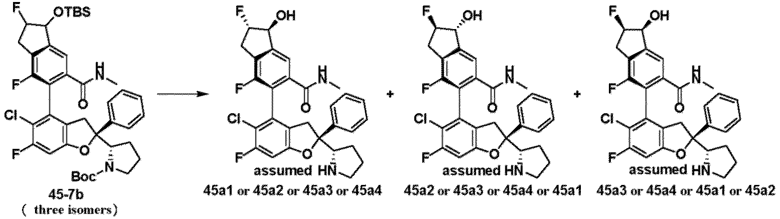

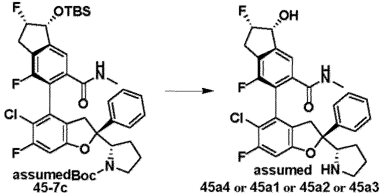

Step 1: Compound **41-5** (2.0 g, 6.94 mmol) was dissolved in anhydrous dichloromethane (40 mL), then Dess-Martin periodinane (4.4 g, 10.37 mmol) was added thereto, and the reaction mixture was reacted at room temperature for 1 hour under nitrogen atmosphere. TLC showed that the starting material was completely reacted. The reaction mixture was slowly added with saturated sodium thiosulfate aqueous solution (50 mL) and saturated sodium bicarbonate aqueous solution (50 mL), stirred for 0.5 hours, and extracted with dichloromethane (60 mL × 3). The organic phases were combined, washed with saturated brine (60 mL), dried over anhydrous sodium sulfate, and filtered. The filtrate was concentrated under reduced pressure and then purified by silica gel column chromatography (EA: PE = 1:5) to obtain compound **45-1** (1.0 g, Y: 50%).

Step 2: Compound **45-1** (1.0 g, 3.50 mmol) was dissolved in anhydrous methanol (15 mL), then 1-chloromethyl-4-fluoro-1,4-diazabicyclo[2.22]octane bis(tetrafluoroborate) (1.4 g, 3.95 mmol) and concentrated sulfuric acid (68 mg, 0.69 mmol) were sequentially added thereto, and the reaction mixture was reacted at 60°C for 12 hours under nitrogen atmosphere. TLC showed that the starting material was completely reacted. The reaction mixture was cooled to 25°C, the solid was filtered, and the filter cake was washed with methanol (30 mL). The filtrate was directly concentrated under reduced pressure and then purified by silica gel column chromatography (EA: PE = 1:8) to obtain compound **45-2** (1.0 g, Y: 82%).

Step 3: Compound **45-2** (1.0 g, 2.86 mmol) was dissolved in acetone (10 mL) and water (5 mL), then p-toluenesulfonic acid (540 mg, 3.14 mmol) was added thereto, and the reaction mixture was reacted at 65°C for 2 hours under nitrogen atmosphere. LCMS showed that the starting material was completely reacted. The reaction mixture was cooled to 25°C, added with saturated sodium bicarbonate aqueous solution (30 mL), and extracted with ethyl acetate (40 mL × 3). The organic phases were combined, washed with saturated brine (30 mL), dried over anhydrous sodium sulfate, filtered, and the filtrate was concentrated under reduced pressure to obtain compound **45-3** (650 mg, crude). LC-MS m/z (ESI): 304.1/306.1 [M+H]⁺.

Step 4: **45-3** (650 mg, 2.14 mmol) was dissolved in tetrahydrofuran (8 mL). The reaction mixture was cooled to 0°C, slowly added with solid sodium borohydride (160 mg, 4.27 mmol), and reacted at 0°C for 0.5 hours under nitrogen atmosphere. LCMS showed that the starting material was completely reacted. The reaction mixture was slowly added with water (40 mL), and extracted with ethyl acetate (50 mL × 2). The organic phases were combined, washed with saturated brine (30 mL), dried over anhydrous sodium sulfate, filtered, and the filtrate was concentrated under reduced pressure to obtain compound **45-4** (500 mg, crude). LC-MS m/z (ESI): 306.1/308.2 [M+H]⁺.

Step 5: Compound **45-4** (500 mg, 1.63 mmol) was dissolved in anhydrous dichloromethane (8 mL). The reaction mixture was cooled to 0°C, sequentially added with imidazole (390 mg, 5.72 mmol) and tert-butyldimethylsilyl chloride (610 mg, 4.06 mmol), and reacted at room temperature for 3 hours under nitrogen atmosphere. TLC showed that the starting material was completely reacted. The reaction mixture was added with water (40 mL), and extracted with dichloromethane (40 mL × 2). The organic phases were combined, washed with saturated brine (10 mL), dried over anhydrous sodium sulfate, and filtered. The filtrate was concentrated under reduced pressure and then purified by silica gel column chromatography (EA: PE = 1:15) to obtain compound **45-5** (600 mg, Y: 87%). LC-MS m/z (ESI): 420.2/422.1 [M+H]⁺.

Step 6: Compound **45-5** (380 mg, 0.90 mmol) and compound **1-8** (544 mg, 0.90 mmol) were dissolved in toluene (10 mL) and water (2 mL), and tris(dibenzylideneacetone)dipalladium (80 mg, 0.09 mmol), 4,5-bis(diphenylphosphino)-9,9-dimethylxanthene (100 mg, 0.17 mmol), and anhydrous potassium phosphate (430 mg, 3.11 mmol) were sequentially added thereto. The reaction mixture was reacted at 110°C overnight under nitrogen atmosphere. TLC showed that the starting material was completely reacted. The reaction mixture was cooled to 25°C, concentrated under reduced pressure, and purified by silica gel column chromatography (EA: PE = 1:5 to 1:1, PE: EA = 5:1, Rf = 0.7, Rf = 0.6, and Rf = 0.5, where Rf = 0.6/0.5 was for target product) to obtain compounds **45-7a** (40 mg, Y: 5.8%, Rf = 0.7, Rt = 1.68 min/1.72 min), **45-7b** (310 mg, crude, Rf = 0.6, Rt = 1.81 min), and **45-7c** (70 mg, Y: 10%, Rf = 0.5, Rt = 1.85 min). LC-MS (1.5 mL-3.2 min-5 to 95% B) m/z (ESI): 757.4 [M+H]⁺.

Step 7: Compound **45-7b** (310 mg, 0.41 mmol) was dissolved in hydrochloric acid/dioxane solution (4 mL, 4.0 M), and the reaction mixture was reacted at 25°C for 0.5 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% HCl-CH₃CN; gradient: 41% to 61%; flow rate: 20 mL/min; run time: 12 min; retention time: 7.9 min/8.2 min/8.4 min) to obtain compounds **45a1** (3.10 mg, HCl salt, Y: 1.3%, Rt = 8.4 min), **45a2** (3.9 mg, HCl salt, Y: 1.6%, Rt = 8.2 min), and **45a3** (36.4 mg, HCl salt, Y: 15%, Rt = 7.9 min).

**45a1:** ¹H NMR (400 MHz, DMSO-*d₆*): δ 9.45-9.30 (br, 1H), 8.50-8.28 (m, 2H), 7.60-7.35 (m, 6H), 7.14 (d, *J* = 9.6 Hz, 1H), 6.30-6.18 (br, 1H), 5.30-5.10 (m, 2H), 4.35-4.22 (m, 1H), 3.60-3.50 (m, 2H), 3.20-2.90 (m, 4H), 2.63 (d, *J* = 4.0 Hz, 3H), 2.20-1.70 (m, 4H). LC-MSm/z (ESI): 543.2/545.3 [M+H]⁺. Chiral analysis (column model: Celluiose-C, 100 * 4.6 mm, 5 µm; eluent: hexane (0.1% DEA)-IPA; gradient: isocratic 4/1, flow rate: 1 mL/min; run time: 10 min; retention time: 4.16 min).

**45a2:** ¹H NMR (400 MHz, DMSO-*d₆*): δ 9.50-9.30 (br, 1H), 8.46-8.28 (m, 2H), 7.58-7.34 (m, 6H), 7.14 (d, *J* = 9.2 Hz, 1H), 6.10-5.90 (br, 1H), 5.40-5.10 (m, 2H), 4.32-4.22 (m, 1H), 3.60-3.50 (m, 2H), 3.20-2.86 (m, 4H), 2.63 (d, *J* = 4.4 Hz, 3H), 2.18-1.70 (m, 4H). LC-MSm/z(ESI): 543.3/545.2 [M+H]⁺. Chiral analysis (column model: Celluiose-C, 100 * 4.6 mm, 5 µm; eluent: hexane (0.1% DEA)-IPA; gradient: isocratic 4/1, flow rate: 1 mL/min; run time: 10 min; retention time: 2.68 min).

**45a3:** ¹H NMR (400 MHz, DMSO-*d₆*): δ 9.50-9.40 (br, 1H), 8.48-8.30 (m, 2H), 8.59-7.35 (m, 6H), 7.14 (d, *J* = 9.6 Hz, 1H), 6.28-6.20 (br, 1H), 5.30-5.08 (m, 2H), 4.32-4.22 (m, 1H), 3.57 (d, *J* = 16.4 Hz, 1H), 3.25-3.02 (m, 4H), 2.90 (d, *J* = 16.4 Hz, 1H), 2.64 (d, *J* = 4.4 Hz, 3H), 2.18-1.70 (m, 4H). LC-MS m/z (ESI): 543.3/545.3 [M+H]⁺. Chiral analysis (column model: Celluiose-C, 100 * 4.6 mm, 5 µm; eluent: hexane (0.1% DEA)-IPA; gradient: isocratic 4/1, flow rate: 1 mL/min; run time: 10 min; retention time: 4.92 min).

Step 8: Compound **45-7c** (70 mg, 0.09 mmol) was dissolved in hydrochloric acid/dioxane solution (2 mL, 4.0 M), and the reaction mixture was reacted at 25°C for 0.5 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% HCl-CH₃CN; gradient: 42% to 62%; flow rate: 20 mL/min; run time: 12 min; retention time: 8.9 min) to obtain compound **45a4** (16.8 mg, HCl salt, Y: 31%).

**45a4:** ¹H NMR (400 MHz, DMSO-*d₆*): δ 9.33 (br, 1H), 8.44-8.40 (m, 1H), 8.33 (br, 1H), 7.52-7.35 (m, 6H), 7.13 (d, *J* = 9.2 Hz, 1H), 6.00 (br, 1H), 5.37-5.20 (m, 1H), 5.15-5.06 (m, 1H), 4.32-4.22 (m, 1H), 3.54 (d, *J* = 16.4 Hz, 1H), 3.20-3.02 (m, 4H), 2.89 (d, *J* = 16.4 Hz, 1H), 2.64 (d, *J* = 4.4 Hz, 3H), 2.16-2.07 (m, 1H), 2.00-1.70 (m, 3H). LC-MSm/z (ESI): 543.2/545.3 [M+H]⁺. Chiral analysis (column model: Celluiose-C, 100 * 4.6 mm, 5 µm; eluent: hexane (0.1% DEA)-IPA; gradient: isocratic 4/1, flow rate: 1 mL/min; run time: 10 min; retention time: 4.87 min).

### Example 46

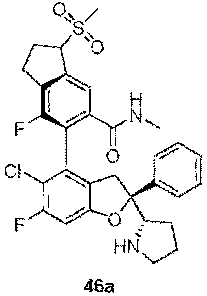

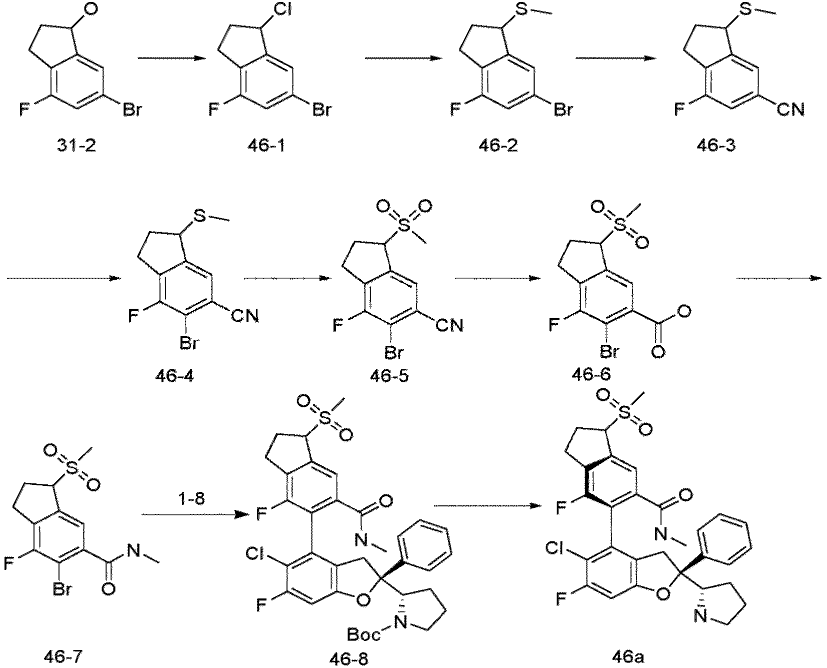

Step 1: Compound **31-2** (20.0 g, 86.9 mmol) was dissolved in anhydrous dichloromethane (200 mL). The reaction mixture was cooled to 0°C, sequentially added with triethylamine (27.0 g, 267.3 mmol) and methanesulfonyl chloride (14.8 g, 129.8 mmol), and reacted at room temperature for 4 hours under nitrogen atmosphere. TLC showed that the starting material was completely reacted. The reaction mixture was added with ice water (100 mL), and extracted with ethyl acetate (100 mL × 3). The organic phases were combined, washed with saturated brine (100 mL), dried over anhydrous sodium sulfate, and filtered. The filtrate was concentrated under reduced pressure and then purified by silica gel column chromatography (EA: PE = 0:1) to obtain compound **46-1** (15.9 g, Y: 73%). ¹H NMR (400 MHz, CDCl₃): δ 7.36 (s, 1H), 7.16-7.11 (m, 1H), 5.36-5.31 (m, 1H), 3.16-3.08 (m, 1H), 2.98-2.88 (m, 1H), 2.70-2.60 (m, 1H), 2.46-2.36 (m, 1H).

Step 2: Compound **46-1** (6.0 g, 24.2 mmol) was dissolved in N,N-dimethylformamide (60 mL). The reaction mixture was cooled to 0°C, slowly added with solid sodium thiomethoxide (1.8 g, 25.7 mmol), and reacted at room temperature for 2 hours under nitrogen atmosphere. TLC showed that the starting material was completely reacted. The reaction mixture was added with ice water (80 mL), and extracted with ethyl acetate (80 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:10) to obtain compound **46-2** (5.0 g, Y: 79%). ¹H NMR (400 MHz, CDCl₃): δ 7.44 (s, 1H), 7.21 (d, J= 8.0 Hz, 1H), 4.31-4.24 (m, 1H), 3.28-3.08 (m, 1H), 3.02-2.92 (m, 1H), 2.66-2.56 (m, 1H), 2.29-2.19 (m, 1H), 2.04 (s, 3H).

Step 3: Compound **46-2** (5.0 g, 19.2 mmol) was dissolved in N,N-dimethylformamide (60 mL), and zinc cyanide (1.8 g, 15.4 mmol), tris(dibenzylideneacetone)dipalladium (1.7 g, 1.9 mmol), and 1,1'-bis(diphenylphosphino)ferrocene (2.1 g, 3.8 mmol) were sequentially added thereto. The reaction mixture was reacted at 110°C for 16 hours under nitrogen atmosphere. TLC showed that the starting material was completely reacted. The reaction mixture was cooled to 25°C, added with ice water (80 mL), and extracted with ethyl acetate (60 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:10) to obtain compound **46-3** (3.3 g, Y: 82%). ¹H NMR (400 MHz, CDCl₃): δ 7.28 (s, 1H), 7.08 (d, *J* = 8.4 Hz, 1H), 4.28-4.20 (m, 1H), 3.05-2.95 (m, 1H), 3.80-3.70 (m, 1H), 2.62-2.50 (m, 1H), 2.24-2.15 (m, 1H), 2.03 (s, 3H).

Step 4: Compound **46-3** (3.3 g, 15.9 mmol) was dissolved in anhydrous tetrahydrofuran (35 mL). The reaction system was cooled to -70°C, added dropwise with lithium diisopropylamide (2 M, 8.7 mL), and reacted at -70°C for 1 hour. The reaction mixture was slowly added dropwise with 1,2-dibromotetrachloroethane (6.2 g, 19.1 mmol) dissolved in anhydrous tetrahydrofuran (6 mL) at -70°C, and reacted at -70°C for 1 hour. TLC showed that the starting material was completely reacted. The reaction mixture was slowly added with saturated ammonium chloride aqueous solution (30 mL) and water (50 mL), and extracted with ethyl acetate (60 mL × 3). The organic phases were combined, washed with saturated brine (20 mL), dried over anhydrous sodium sulfate, and filtered. The filtrate was concentrated under reduced pressure and then purified by silica gel column chromatography (EA: PE = 1:10) to obtain compound **46-4** (1.4 g, Y: 30%). ¹H NMR (400 MHz, CDCl₃): δ 7.39 (s, 1H), 4.26-4.20 (m, 1H), 3.18-2.92 (m, 2H), 2.65-2.55 (m, 1H), 2.25-2.14 (m, 1H), 2.0 (s, 1H).

Step 5: Compound **46-4** (1.2 g, 4.2 mmol) was dissolved in dichloromethane (12 mL), and hydrogen peroxide (1.4 g, 30% w/w, 12.6 mmol) and trifluoroacetic acid (480 mg, 4.2 mmol) were sequentially added thereto. The reaction mixture was reacted at 40°C for 2 hours under nitrogen atmosphere. TLC showed that the starting material was completely reacted. The reaction mixture was concentrated under reduced pressure to obtain compound **46-5** (1.0 g, crude).

Step 6: Compound **46-5** (1.0 g, 3.2 mmol) was dissolved in ethanol (3 mL), and water (9 mL) and sodium hydroxide (1.2 g, 30.0 mmol) were added thereto. The reaction mixture was reacted at 100°C for 12 hours under nitrogen atmosphere. TLC showed that the starting material was completely reacted. The reaction mixture was cooled to 0°C, added with water (10 mL), and then slowly added with dilute hydrochloric acid (0.5 M) until the pH of the reaction system was 5 to 6. The reaction mixture was filtered, and the filter cake was washed with water (10 mL) and dried to obtain compound **46-6** (800 mg, Y: 75%).

Step 7: Compound **46-6** (300 mg, 0.89 mmol) was dissolved in N,N-dimethylformamide (3 mL), and 2-(7-azabenzotriazol-1-yl)-N,N,N',N'-tetramethyluronium hexafluorophosphate (450 mg, 1.2 mmol), N,N-diisopropylethylamine (500 mg, 3.9 mmol), and methylamine hydrochloride (100 mg, 1.5 mmol) were sequentially added thereto. The reaction mixture was reacted at room temperature for 2 hours under nitrogen atmosphere. LCMS showed that the starting material was completely reacted. The reaction mixture was purified by reversed-phase silica gel column chromatography (C18, 12 g, eluent: water/acetonitrile, gradient: 0 to 50%; flow rate: 30 mL/min; run time: 10 min; retention time: 8.0 min) to obtain compound **46-7** (210 mg, Y: 67%). LC-MS m/z (ESI): 350.1/352.1 [M+H]⁺.

Step 8: Compound **46-7** (300 mg, 0.86 mmol) and compound **1-8** (466 mg, 0.86 mmol) were dissolved in toluene (8 mL) and water (1.6 mL), and tris(dibenzylideneacetone)dipalladium (80 mg, 0.09 mmol), 4,5-bis(diphenylphosphino)-9,9-dimethylxanthene (120 mg, 0.2 mmol), and anhydrous potassium phosphate (700 mg, 3.3 mmol) were sequentially added thereto. The reaction mixture was reacted at 100°C overnight under nitrogen atmosphere. TLC showed that the starting material was completely reacted. The reaction mixture was cooled to 25°C, concentrated under reduced pressure, and purified by silica gel column chromatography (EA) to obtain compound **46-8** (140 mg, Y: 23%). LC-MS m/z (ESI): 687.4/689.4 [M+H]⁺.

Step 9: Compound **46-8** (140 mg, 0.2 mmol) was dissolved in dichloromethane (2 mL), then hydrochloric acid/dioxane solution (0.4 mL, 4.0 M) was added thereto, and the reaction mixture was reacted at 25°C for 0.5 hours. LCMS showed that the starting material was completely reacted. The reaction mixture was concentrated under reduced pressure, and then purified by preparative high-performance liquid chromatography (column model: YMC-Actus Triart C18, 150 * 20 mm, 5 µm; eluent: 0.05% NH₃·H₂O-10 mmol/L NH₄HCO₃-ACN; gradient: 44% to 64%; flow rate: 20 mL/min; run time: 12 min; retention time: 7.5/8.6 min, where Rt = 8.6 min was for target product) to obtain compound **46a** (10 mg, Y: 8%). ¹H NMR (400 MHz, DMSO-*d₆*): δ 8.25 (br, 1H), 7.55 (s, 1H), 7.48-7.22 (m, 54H), 7.04 (d, *J* = 9.6 Hz, 1H), 5.02-4.94 (m, 1H), 3.60-3.48 (m, 2H), 3.15-2.75 (m, 7H), 2.70-2.60 (m, 4H), 2.46-2.40 (m, 1H), 1.68-1.34 (m, 4H). LC-MS m/z (ESI): 587.2/589.2 [M+H]⁺.

### Example 49

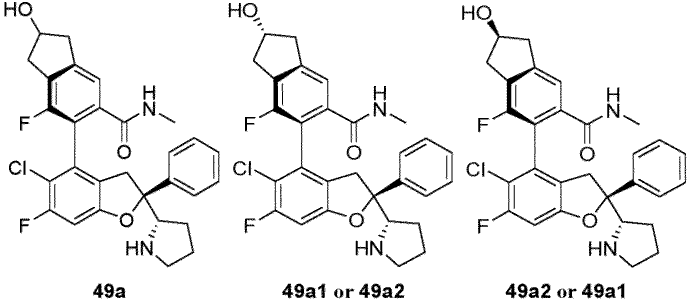

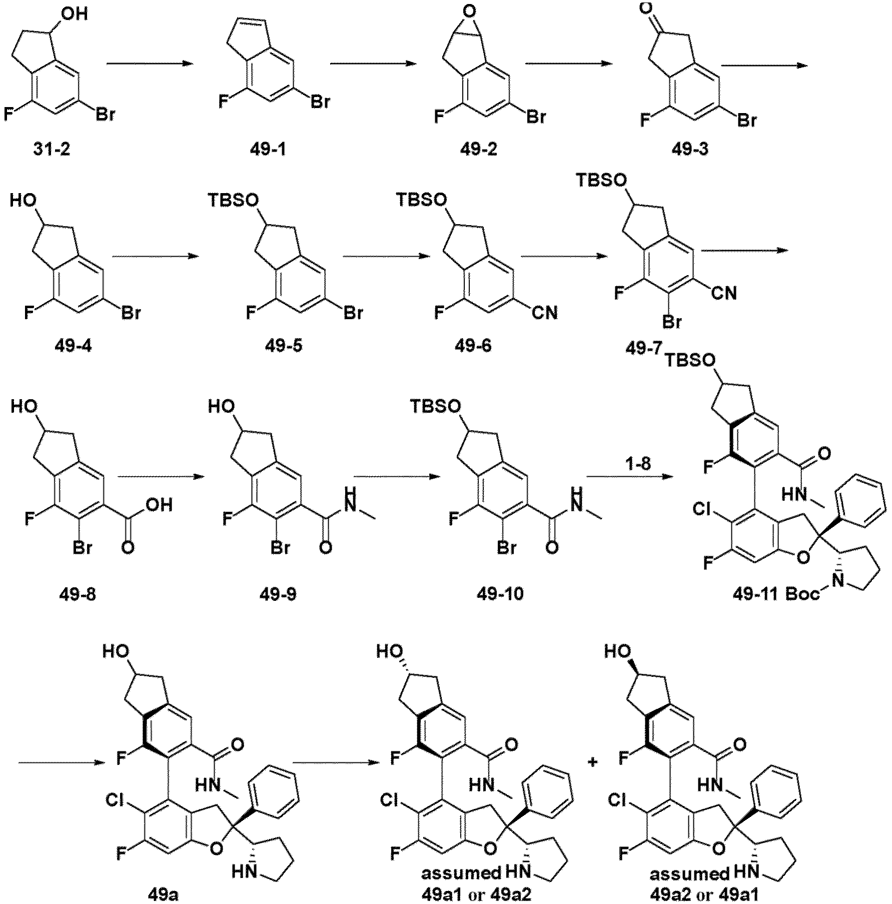

Step 1: Compound **31-2** (60.0 g, 260 mmol) was dissolved in toluene (1000 mL), then p-toluenesulfonic acid (4.48 g, 26 mmol) was added thereto under nitrogen atmosphere, and the reaction mixture was reacted at 90°C for 16 hours. TLC (EA: PE = 1:10) showed that the starting material was completely reacted. The reaction mixture was cooled to 25°C, concentrated under reduced pressure, and purified by silica gel column chromatography (EA: PE = 1:100) to obtain compound **49-1** (44.6 g, Y: 80%).

Step 2: Compound **49-1** (50.0 g, 235.8 mmol) was dissolved in dichloromethane (500 mL), then 3-chloroperoxybenzoic acid (80.0 g, 471.6 mmol, 2.0 eq) was added thereto under nitrogen atmosphere, and the reaction mixture was reacted at room temperature for 16 hours. TLC (EA: PE = 1:20) showed that the starting material was completely reacted. The reaction mixture was poured into saturated sodium sulfite (500 mL) to quench, and extracted with dichloromethane (500 mL × 2). The organic phases were combined, washed with saturated brine (400 mL), dried over anhydrous sodium sulfate, and filtered. The filtrate was concentrated under reduced pressure and then purified by silica gel column chromatography (EA: PE = 1:30) to obtain compound **49-2** (38.0 g, Y: 73%). Key-INT A22-2 ¹H NMR (400 MHz, CDCl₃) δ 7.44 (s, 1H), 7.18-7.12 (m, 1H), 4.27-4.13 (m, 2H), 3.27-3.18 (m, 1H), 2.95-2.85 (m, 1H).

Step 3: Starting material **49-2** (34.0 g, 148.4 mmol) was dissolved in toluene (480 mL), then zinc iodide (23.7 g, 74.2 mmol) was added thereto, and the reaction mixture was reacted at 25°C for 16 hours. TLC (EA: PE = 1:50) showed that the starting material was completely reacted. The reaction mixture was added with saturated ammonium chloride (500 mL) to quench, and extracted with ethyl acetate (500 mL × 2). The organic phases were combined, washed with saturated brine (500 mL), dried over anhydrous sodium sulfate, and filtered. The filtrate was concentrated under reduced pressure and then purified by silica gel column chromatography (THF: PE = 2:98) to obtain compound **49-3** (23.2 g, Y: 68%). ¹H NMR (400 MHz, CDCl₃) δ 7.28 (s, 1H), 7.18 (d, *J* = 8.4 Hz, 1H), 3.57 (s, 2H), 3.51 (s, 2H).

Step 4: Compound **49-3** (14.0 g, 61.1 mmol) was dissolved in anhydrous tetrahydrofuran (160 mL). The reaction mixture was cooled to 0°C, and added with sodium borohydride (4.6 g, 122.1 mmol) in batches. The reaction mixture was reacted at room temperature for 4 hours. TLC showed that the starting material was completely reacted. The reaction mixture was slowly added with ice water (300 mL), and extracted with ethyl acetate (300 mL × 3). The organic phases were combined, washed with saturated brine (100 mL), dried over anhydrous sodium sulfate, and filtered. The filtrate was concentrated under reduced pressure and then purified by silica gel column chromatography (EA: PE = 1:20) to obtain compound **49-4** (6.5 g, Y: 46%). ¹H NMR (400 MHz, DMSO-*d₆*): δ 7.29 (s, 1H), 7.26 (d, *J* = 8.8, 1H), 5.00 (d, *J* = 4.0, 1H), 4.56-4.52 (m, 1H), 3.13-2.98 (m, 2H), 2.81-2.69 (m, 2H).

Step 5: Compound **49-4** (6.5 g, 28.1 mmol) was dissolved in anhydrous dichloromethane (75 mL). The reaction mixture was cooled to 0°C, sequentially added with imidazole (3.9 g, 57.4 mmol) and tert-butyldimethylsilyl chloride (6.4 g, 42.5 mmol), and reacted at room temperature for 5 hours under nitrogen atmosphere. TLC showed that the starting material was completely reacted. The reaction mixture was added with water (100 mL), and extracted with ethyl acetate (100 mL × 3). The organic phases were combined, washed with saturated brine (80 mL), dried over anhydrous sodium sulfate, and filtered. The filtrate was concentrated under reduced pressure and then purified by silica gel column chromatography (PE) to obtain compound **49-5** (8.4 g, Y: 86%). ¹H NMR (400 MHz, DMSO-*d₆*): δ 7.30 (s, 1H), 7.28 (d, *J* = 8.4, 1H), 4.73-4.68 (m, 1H), 3.21-3.08 (m, 2H), 2.82-2.67 (m, 2H), 0.85 (s, 9H), 0.07 (s, 6H).

Step 6: Compound **49-5** (8.4 g, 24.3 mmol) was dissolved in N,N-dimethylformamide (90 mL), and zinc cyanide (2.3 g, 19.6 mmol), tris(dibenzylideneacetone)dipalladium (2.3 g, 2.5 mmol), and 1,1'-bis(diphenylphosphino)ferrocene (2.7 g, 4.9 mmol) were sequentially added thereto. The reaction mixture was reacted at 100°C for 16 hours under nitrogen atmosphere. TLC showed that the starting material was completely reacted. The reaction mixture was cooled to 25°C, added with ice water (300 mL), and extracted with ethyl acetate (200 mL × 3). The organic phases were combined, washed with saturated brine (100 mL), dried over anhydrous sodium sulfate, and filtered. The filtrate was concentrated under reduced pressure and then purified by silica gel column chromatography (EA: PE = 1:10) to obtain compound **49-6** (5.5 g, Y: 77%). ¹H NMR (400 MHz, DMSO-*d₆*): δ 7.62 (d, *J* = 8.8, 1H), 7.59 (s, 1H), 4.78-4.73 (m, 1H), 3.26-3.19 (m, 2H), 2.86-2.79 (m, 2H), 0.84 (s, 9H), 0.07 (s, 6H).

Step 7: Compound **49-6** (2.5 g, 8.6 mmol) was dissolved in anhydrous tetrahydrofuran (35 mL). The reaction system was cooled to -78°C, added dropwise with lithium diisopropylamide (2 M, 5.1 mL), and reacted at -70°C for 1 hour. The reaction mixture was slowly added dropwise with 1,2-dibromotetrachloroethane (3.4 g, 10.4 mmol) dissolved in anhydrous tetrahydrofuran (6 mL) at -70°C, and reacted at -70°C for 1 hour. TLC showed that the starting material was completely reacted. The reaction mixture was slowly added with saturated ammonium chloride aqueous solution (40 mL) and water (50 mL), and extracted with ethyl acetate (60 mL × 3). The organic phases were combined, washed with saturated brine (20 mL), dried over anhydrous sodium sulfate, and filtered. The filtrate was concentrated under reduced pressure and then purified by silica gel column chromatography (EA: PE = 1:10) to obtain compound **49-7** (2.2 g, Y: 69%). ¹H NMR (400 MHz, CDCl₃): δ 7.31 (s, 1H), 4.76-4.70 (m, 1H), 3.26-3.11 (m, 2H), 3.00-2.87 (m, 1H), 0.87 (s, 9H), 0.93 (s, 6H).

Step 8: Compound **49-7** (2.2 g, 5.9 mmol) was dissolved in ethanol (15 mL), and water (15 mL) and sodium hydroxide (2.1 g, 52.5 mmol) were added thereto. The reaction mixture was reacted at 100°C for 16 hours under nitrogen atmosphere. TLC showed that the starting material was completely reacted. The reaction mixture was cooled to 0°C, added with water (30 mL), and then slowly added with dilute hydrochloric acid (0.5 M) until the pH of the reaction system was 4 to 5. The reaction mixture was extracted with ethyl acetate (100 mL × 3). The organic phases were combined, washed with saturated brine (50 mL), dried over anhydrous sodium sulfate, filtered, and the filtrate was concentrated under reduced pressure to obtain compound **49-8** (1.1 g, crude).

Step 9: Compound **49-8** (1.1 g, 4.0 mmol) was dissolved in N,N-dimethylformamide (10 mL), and 2-(7-azabenzotriazol-1-yl)-N,N,N',N'-tetramethyluronium hexafluorophosphate (1.7 g, 4.5 mmol), N,N-diisopropylethylamine (2.0 g, 15.5 mmol), and methylamine hydrochloride (380 mg, 5.7 mmol) were sequentially added thereto. The reaction mixture was reacted at room temperature for 2 hours under nitrogen atmosphere. LCMS showed that the starting material was completely reacted. The reaction mixture was added with ice water (50 mL), and extracted with ethyl acetate (40 mL × 3). The organic phases were combined, washed with saturated brine (30 mL), dried over anhydrous sodium sulfate, and filtered. The filtrate was concentrated under reduced pressure and then purified by silica gel column chromatography (EA: PE = 1:1) to obtain compound **49-9** (750 mg, Y: 65%). LC-MS m/z (ESI): 288.2/283.2 [M+H]⁺.

Step 10: Compound **49-9** (750 mg, 2.6 mmol) was dissolved in dichloromethane (10 mL), and imidazole (530 mg, 7.8 mmol) and tert-butyldimethylsilyl chloride (780 mg, 5.2 mmol) were sequentially added thereto. The reaction mixture was reacted at room temperature for 3 hours under nitrogen atmosphere. TLC showed that the starting material was completely reacted. The reaction mixture was concentrated under reduced pressure and then purified by silica gel column chromatography (EA: PE = 1:10) to obtain compound **49-10** (820 mg, Y: 78%).

Step 11: Compound **49-10** (150 mg, 0.37 mmol) and compound **1-8** (202 mg, 0.37 mmol) were dissolved in toluene (1.5 mL) and water (0.3 mL), and tris(dibenzylideneacetone)dipalladium (34 mg, 0.04 mmol), 4,5-bis(diphenylphosphino)-9,9-dimethylxanthene (43 mg, 0.07 mmol), and anhydrous potassium phosphate (180 mg, 1.3 mmol) were sequentially added thereto. The reaction mixture was reacted at 110°C overnight under nitrogen atmosphere. TLC showed that the starting material was completely reacted. The reaction mixture was cooled to 25°C, concentrated under reduced pressure, and purified by silica gel column chromatography (EA: PE = 1:5 to 1:1, Rf = 0.6 and Rf = 0.5, where Rf = 0.5 was for target product) to obtain compound **49-11** (70 mg, Y: 25%).

Step 12: Compound **49-11** (70 mg, 0.09 mmol) was dissolved in hydrochloric acid/dioxane solution (1 mL, 4.0 M), and the reaction mixture was reacted at 25°C for 0.5 hours. LCMS showed that the starting material was completely reacted. The reaction mixture was concentrated under reduced pressure, and then purified by preparative high-performance liquid chromatography (column model: YMC-Actus Triart C18, 150 * 20 mm, 5 µm; eluent: 0.05% NH₃H₂O-10 mmol/L NH₄HCO₃-ACN-ME; gradient: 57% to 77%; flow rate: 20 mL/min; run time: 12 min; retention time: 7.7 min) to obtain compound **49a** (8.0 mg, Y: 16%). ¹H NMR (400 MHz, DMSO-*d₆*): δ 8.26 (br, 1H), 7.52-7.28 (m, 7H), 7.04 (d, *J* = 3.6 Hz, 1H), 5.08-5.02 (m, 1H), 4.68-4.55 (m, 1H), 4.08-3.90 (m, 1H), 3.58-3.42 (m, 1H), 3.22-2.70 (m, 7H), 2.63 (d, *J* = 4.4 Hz, 3H), 1.98-1.50 (m, 4H). LC-MS m/z (ESI): 525.2/527.2 [M+H]⁺.

Step 13: Compound **49a** (80 mg, 0.15 mmol) was purified by preparative chiral resolution (CHIRALPAK IA-3, 50 * 4.6 mm, 3 µm IA30CB-BX003; A: n-hexane (0.1% DEA) B: ethanol; gradient: 10 to 30%; flow rate: 15 mL/min, P1: Rt = 1.14 min, P2: Rt = 1.46 min), and concentrated under reduced pressure. P1 was then purified by preparative high-performance liquid chromatography (column model: Xselect CSH Prep Fluoro-Phenyl, 150 * 19 mm, 5 µm; eluent: 0.1% FA-CAN; gradient: 35 to 55% MeCN in H₂O; flow rate: 20 mL/min: run time: 9 min; retention time: 7.6 min) to obtain compound **49a1** (18.1 mg, Y: 22%). P2 was purified by preparative high-performance liquid chromatography (column model: Xselect CSH Prep Fluoro-Phenyl, 150 * 19 mm, 5 µm; eluent: 0.1% FA-CAN; gradient: 35% to 55% MeCN in H₂O; flow rate: 20 mL/min; run time: 9 min; retention time: 8.1 min) to obtain compound **49a2** (16.4 mg, Y: 20%).

**49a1:** ¹H NMR (400 MHz, DMSO-*d₆*): δ 8.22-8.10 (br, 2H), 7.44-7.41 (m, 2H), 7.35-7.25 (m, 4H), 7.00 (d, *J* = 9.6 Hz, 1H), 5.06 (br, 1H), 4.65-4.58 (m, 1H), 3.59-3.56 (m, 1H), 3.45-3.41 (m, 1H), 3.20-3.08 (m, 2H), 2.90-2.65 (m, 5H), 2.62 (d, *J* = 4.4 Hz, 3H), 1.62-1.420 (m, 4H). LC-MS m/z (ESI): 525.2/527.3 [M+H]⁺. Chiral analysis (column model: Amylose-C, 100 * 4.6 mm, 5 µm; eluent: hexane-IPA; gradient: isocratic 7/3, flow rate: 1 mL/min; run time: 5.0 min; retention time: 2.197 min).

**49a2:** ¹H NMR (400 MHz, DMSO-*d₆*): δ 8.17-8.13 (br, 2H), 7.46-7.44 (m, 2H), 7.38-7.27 (m, 4H), 7.02 (d, *J* = 9.6 Hz, 1H), 4.65-4.56 (m, 1H), 3.72-3.68 (m, 1H), 3.50-3.46 (m, 1H), 3.22-3.08 (m, 2H), 2.88-2.76 (m, 5H), 2.62 (d, *J* = 4.8 Hz, 3H), 1.71-1.46 (m, 4H). LC-MS m/z (ESI): 525.2/527.3 [M+H]⁺. Chiral analysis (column model: Amylose-C, 100 * 4.6 mm, 5 µm; eluent: hexane-IPA; gradient: isocratic 7/3, flow rate: 1 mL/min; run time: 5.0 min; retention time: 2.646 min).

### Example 51

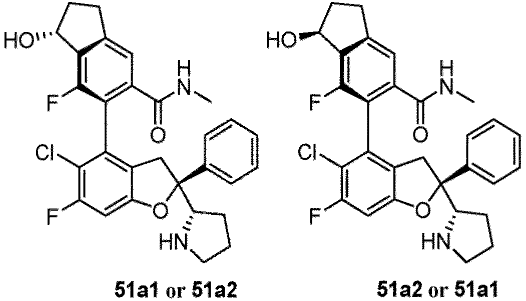

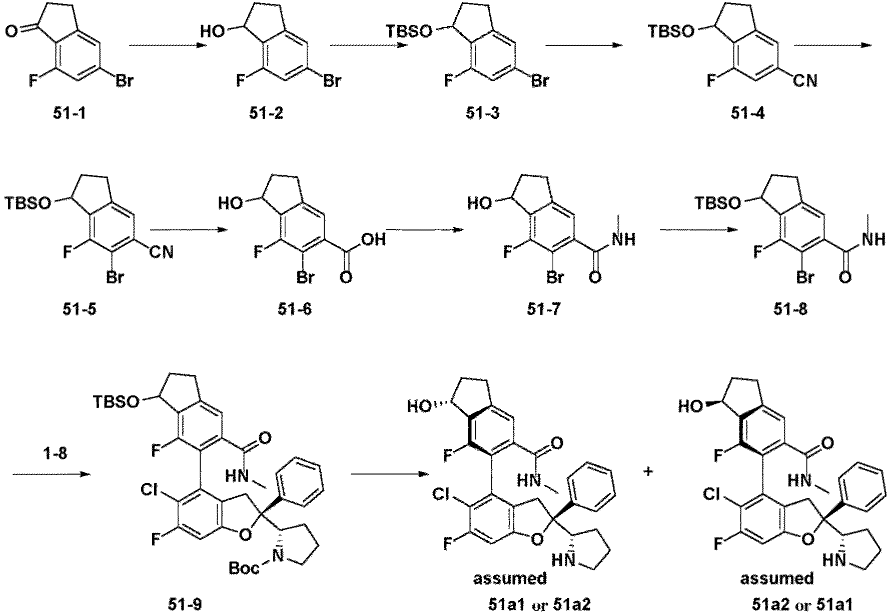

Step 1: Compound **51-1** (3.3 g, 14.41 mmol) was dissolved in methanol (30 mL). The reaction system was cooled to 0°C, added with sodium borohydride (2.2 g, 57.63 mmol) at 0°C in batches, warmed to room temperature, and reacted for 1 hour. TLC (PE: EA = 10:1) showed that the starting material was completely reacted. The reaction mixture was added with ammonium chloride aqueous solution (150 mL) to quench, and extracted with ethyl acetate (50 mL × 3). The organic phases were combined, washed with saturated brine (100 mL), dried over anhydrous sodium sulfate, filtered, and the filtrate was concentrated under reduced pressure to obtain compound **51-2** (3.3 g, Y: 99%).

Step 2: Compound **51-2** (3.3 g, 14.28 mmol) was dissolved in dichloromethane (30 mL), then imidazole (1.9 g, 28.56 mmol) and tert-butyldimethylsilyl chloride (3.2 g, 21.42 mmol) were added thereto, and the reaction mixture was reacted at 25°C for 12 hours. TLC (PE: EA = 10:1) showed that the starting material was completely reacted. The reaction mixture was concentrated under reduced pressure, then diluted with dichloromethane (100 mL), and washed with saturated brine (100 mL). The organic phase was dried over anhydrous sodium sulfate, filtered, and the filtrate was concentrated under reduced pressure and then purified by silica gel column chromatography (EA: PE = 1:20) to obtain compound **51-3** (2.5 g, Y: 50%).

Step 3: Compound **51-3** (2.5 g, 21.42 mmol) was dissolved in N,N-dimethylformamide (25 mL), then zinc cyanide (1.36 g, 11.58 mmol), tris(dibenzylideneacetone)dipalladium (663 mg, 0.72 mmol), and 1,1'-bis(diphenylphosphino)ferrocene (806 mg, 1.45 mmol) were added thereto, and the reaction mixture was reacted at 100°C for 16 hours under nitrogen atmosphere. TLC (PE: EA = 10:1) showed that the starting material was completely reacted. The reaction mixture was cooled to 25°C, poured into ice water (250 mL), and extracted with ethyl acetate (100 mL × 3). The organic phases were combined, washed with saturated brine (100 mL × 3), dried over anhydrous sodium sulfate, and filtered. The filtrate was concentrated under reduced pressure and then purified by silica gel column chromatography (EA: PE = 1:20) to obtain compound **51-4** (1.8 g Y: 84%). ¹H NMR (400 MHz, CDCl₃): δ 7.31 (s, 1H), 7.16 (d, *J* = 8.8 Hz, 1H), 5.52-5.44 (m, 1H), 3.25-3.11 (m, 1H), 2.92-2.78 (m, 1H), 2.48-2.32 (m, 1H), 2.13-2.01 (m, 1H), 0.90 (s, 9H), 0.16-0.13 (m, 6H).

Step 4: Compound **51-4** (1.8 g, 6.18 mmol) was dissolved in anhydrous tetrahydrofuran (20 mL), and the system was cooled to -70°C under nitrogen atmosphere. The above reaction mixture was then slowly added dropwise with lithium diisopropylamide (3.4 mL, 6.79 mmol), and reacted at -70°C for 1 hour. The reaction mixture was then slowly added with dibromotetrachloroethane (2.0 g, 6.18 mmol) dissolved in anhydrous tetrahydrofuran (5 mL) at -70°C, warmed to room temperature and reacted for 1 hour after the addition was completed. LCMS showed that the starting material was completely reacted. The reaction mixture was added dropwise with ice-cold saturated ammonium chloride aqueous solution (60 mL) to quench, and extracted with ethyl acetate (30 mL × 3). The organic phases were combined, washed with saturated brine (50 mL), dried over anhydrous sodium sulfate, and filtered. The filtrate was concentrated under reduced pressure and then purified by silica gel column chromatography (EA: PE = 1:15) to obtain compound **51-5** (1.5 g, Y: 65%).

Step 5: Compound **51-5** (1.5 g, 4.05 mmol) and sodium hydroxide (1.6 g, 40.50 mmol) were dissolved in anhydrous ethanol (30 mL) and water (30 mL), and the reaction mixture was reacted at 100°C for 16 hours. TLC (PE: EA = 10:1) showed that the starting material was completely reacted. The reaction mixture was added dropwise with hydrochloric acid solution (50 mL, 1 M/L) to quench, and extracted with ethyl acetate (30 mL × 3). The organic phases were combined, washed with saturated brine (50 mL), dried over anhydrous sodium sulfate, filtered, and the filtrate was concentrated under reduced pressure to obtain compound **51-6** (670 mg, Y: 60%).

Step 6: Compound **51-6** (610 mg, 2.22 mmol) was dissolved in N,N-dimethylformamide (10 mL). The reaction mixture was sequentially added with N,N-diisopropylethylamine (860 mg, 6.65 mmol), methylamine hydrochloride (299 mg, 4.44 mmol), and 2-(7-azabenzotriazol-1-yl)-N,N,N',N'-tetramethyluronium hexafluorophosphate (1.0 g, 2.66 mmol), and reacted at room temperature for 2 hours. TLC showed that the starting material was completely reacted. The reaction mixture was added with water (30 mL), and extracted with ethyl acetate (25 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 1:1) to obtain compound **51-7** (360 mg, Y: 56%). LC-MS m/z (ESI): 288.0/290.0 [M+H]⁺.

Step 7: Compound **51-7** (320 mg, 1.11 mmol) was dissolved in dichloromethane (6 mL), then imidazole (151 mg, 2.22 mmol) and tert-butyldimethylsilyl chloride (251 mg, 1.67 mmol) were added thereto, and the reaction mixture was reacted at 25°C for 12 hours. LCMS showed that the starting material was completely reacted. The reaction mixture was concentrated under reduced pressure, then diluted with ethyl acetate (20 mL), and washed with saturated brine (30 mL). The organic phase was dried over anhydrous sodium sulfate, filtered, and the filtrate was concentrated under reduced pressure and then purified by silica gel column chromatography (PE: EA = 1:10) to obtain compound **51-8** (307 mg, Y: 68%).

Step 8: Compound **51-8** (160 mg, 0.40 mmol) and compound **1-8** (260 mg, 0.48 mmol) were dissolved in toluene (6 mL) and water (1.2 mL), then tris(dibenzylideneacetone)dipalladium (73 mg, 0.08 mmol), 4,5-bis(diphenylphosphino)-9,9-dimethylxanthene (92 mg, 0.16 mmol), and anhydrous potassium carbonate (220 mg, 1.59 mmol) were added thereto, and the reaction mixture was reacted at 110°C overnight under nitrogen atmosphere. LCMS showed that the starting material was completely reacted. 2 batches were combined for processing as the same operation as above was repeated once. The reaction mixture was cooled to 25°C, concentrated under reduced pressure, and purified by silica gel column chromatography (EA: PE = 1:10) to obtain compound **51-9** (150 mg, Y: 25%). LC-MS m/z (ESI): 739.3 [M+H]⁺.

Step 9: Compound **51-9** (150 mg, 0.20 mmol) was dissolved in dichloromethane (5.0 mL), then trifluoroacetic acid (1.0 mL) was added thereto, and the reaction mixture was reacted at room temperature for 2 hours. LCMS showed that the starting material was completely reacted. The reaction mixture was cooled to 0°C, poured into saturated sodium carbonate aqueous solution (10 mL) to quench, and extracted with dichloromethane (10 mL × 3). The organic phases were combined, washed with saturated brine (10 mL), dried over anhydrous sodium sulfate, and filtered. The filtrate was concentrated under reduced pressure and then purified by preparative medium-pressure liquid chromatography (column model: C18 spherical 20 to 35 µm 100 A 120 g; eluent: CH₃CN/H₂O; gradient: 25% to 35%; flow rate: 70 mL/min; run time: 20 min; retention time: 13 min and 15 min) to obtain pre-peak (24 mg, Rt = 13 min) and post-peak (23 mg, Rt = 15 min). The pre-peak (24 mg) was purified by preparative high-performance liquid chromatography (column model: Xbridge Prep Phenyl OBD, 150 * 19 mm, 5 µm; eluent: 0.05% HCI/CH₃CN; gradient: 30% to 40%; flow rate: 20 mL/min; run time: 15 min; retention time: 7.2 min and 8.7 min, where 8.7 min was for **51a2**) to obtain compound **51a2** (6.8 mg, Y: 6%, retention time: 8.7 min). The post-peak (23 mg) was purified by preparative high-performance liquid chromatography (column model: Xbridge Prep Phenyl OBD, 150 * 19 mm, 5 µm; eluent: 0.05% HCl/CH₃CN; gradient: 30% to 40%; flow rate: 20 mL/min; run time: 15 min; retention time: 10.1 min and 11.2 min, where 11.2 min was for **51a1**) to obtain compound **51a1** (7.0 mg, Y: 6%, retention time: 11.2 min). Both compounds were in hypothetical structures. **51a2:** Chiral analysis (column model: Celluiose-SB, 100 * 4.6 mm, 5 µm; eluent: hexane (0.1% DEA)/IPA; gradient: isocratic 9/1, flow rate: 1 mL/min; run time: 10 min; retention time: 6.50 min). ¹H NMR (400 MHz, DMSO-*d₆*): δ 9.10 (br, 1H), 8.40-8.20 (m, 2H), 7.57-7.49 (m, 2H), 7.48-7.42 (m, 2H), 7.41-7.32 (m, 2H), 7.17-7.08 (m, 1H), 5.43-5.32 (m, 1H), 5.28-5.18 (m, 1H), 5.47-5.14 (m, 2H), 4.28-4.19 (m, 1H), 3.62-3.41 (m, 2H), 3.23-3.02 (m, 2H), 2.96-2.76 (m, 2H), 2.63 (d, *J* = 3.6 Hz, 3H), 2.39-2.25 (m, 1H), 2.20-2.05 (m, 1H), 2.01-1.67 (m, 4H). LC-MS m/z (ESI): 537.2 [M+H]⁺. **51a1:** Chiral analysis (column model: Celluiose-SB, 100 * 4.6 mm, 5 µm; eluent: hexane (0.1% DEA)/IPA; gradient: isocratic 9/1, flow rate: 1 mL/min; run time: 10 min; retention time: 7.62 min). ¹H NMR (400 MHz, DMSO-*d₆*): δ 9.06 (br, 1H), 8.39-8.20 (m, 2H), 7.58-7.49 (m, 2H), 7.48-7.35 (m, 4H), 7.17-7.09 (m, 1H), 5.39-5.28 (m, 1H), 5.27-5.20 (m, 1H), 4.39-4.21 (m, 1H), 3.61-3.45 (m, 2H), 3.23-3.02 (m, 3H), 2.96-2.80 (m, 2H), 2.63 (d, *J* = 4.8 Hz, 3H), 2.20-2.08 (m, 1H), 2.01-1.65 (m, 5H). LC-MS m/z (ESI): 537.2 [M+H]⁺.

### Example 52

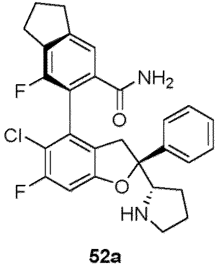

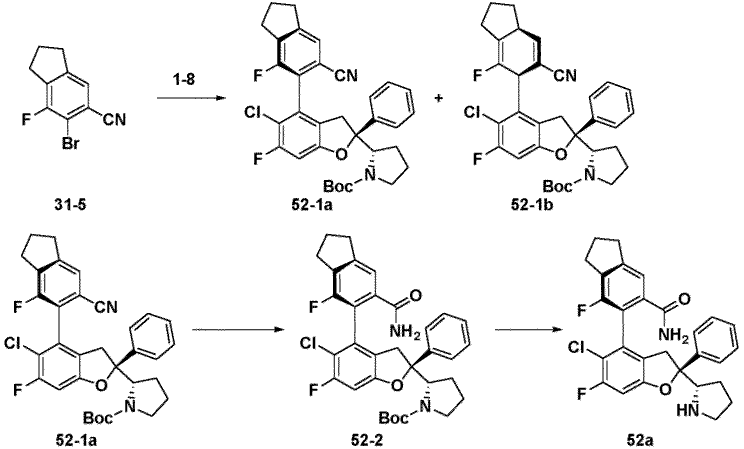

Step 1: Compound **31-5** (50 mg, 0.21 mmol) was dissolved in toluene (1 mL) and water (0.2 mL), then compound **1-8** (141 mg, 0.26 mmol), tris(dibenzylideneacetone)dipalladium (38.3 mg, 0.02 mmol), 4,5-bis(diphenylphosphino)-9,9-dimethylxanthene (48.6 mg, 0.083 mmol), and anhydrous potassium carbonate (86 mg, 0.62 mmol) were sequentially added thereto under nitrogen atmosphere, and the reaction mixture was reacted at 120°C overnight. TLC (PE: EA = 4:1) showed that the starting material was completely reacted. The reaction mixture was cooled to 25°C, poured into water (50 mL), and extracted with ethyl acetate (20 mL × 3). The organic phases were combined, washed with saturated brine (20 mL), dried over anhydrous sodium sulfate, filtered, and the filtrate was concentrated under reduced pressure. 3 batches were combined for processing according to the same operation as above, and the residue was purified by silica gel column chromatography (PE: EA = 2:1) to obtain compound **52-1a** (180 mg, Y: 50%, R_{f} = 0.40) and **52-1b** (150 mg, Y: 42%, R_{f} = 0.35). LC-MS m/z (ESI): 599.4 [M+Na]⁺.

Step 2: Compound **52-1a** (160 mg, 0.23 mmol) was dissolved in dimethyl sulfoxide (4 mL) and hydrogen peroxide (1 mL). The reaction mixture was cooled to 0°C, added with sodium hydroxide (110 mg, 2.77 mmol), gradually warmed to 25°C, and stirred for 2 hours. TLC (PE: EA = 2:1) showed that the starting material was completely reacted. The reaction mixture was added dropwise with hydrochloric acid solution (50 mL, 1 M) to quench, and extracted with ethyl acetate (30 mL × 3). The organic phases were combined, washed with saturated brine (30 mL), dried over anhydrous sodium sulfate, filtered, and the filtrate was concentrated under reduced pressure to obtain compound **52-2** (140 mg, Y: 84%). LC-MS m/z (ESI): 595.6 [M+H]⁺.

Step 3: Compound **52-2** (140 mg, 0.24 mol) was dissolved in dichloromethane (2 mL). The reaction mixture was cooled to 0°C, added with trifluoroacetic acid (1 mL), and reacted at room temperature for 2 hours. LCMS showed that the starting material was completely reacted. The reaction mixture was poured into ice-cold saturated sodium bicarbonate (30 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by preparative high-performance liquid chromatography (column model: Xbridge Prep Phenyl OBD, 150 * 19 mm, 5 µm; eluent: 0.05% NH₃·H₂O/CH₃CN; gradient: 50% to 65%; flow rate: 20 mL/min; run time: 15 min; retention time: 10.5 min) to obtain compound **52a** (31.9 mg, Y: 27%). ¹H NMR (400 MHz, DMSO-*d₆*): δ 7.59 (s, 1H), 7.44-7.22 (m, 7H), 6.99 (d, *J* = 9.6 Hz, 1H), 3.55-3.45 (m, 2H), 3.05-2.80 (m, 5H), 2.74-2.53 (m, 2H), 2.16-2.08 (m, 2H), 1.65-1.52 (m, 1H), 1.51-1.36 (m, 2H), 1.35-1.26 (m, 1H). LC-MS m/z (ESI): 495.2 [M+H]⁺.

### Example 53

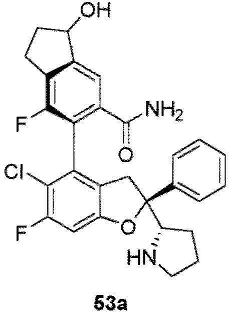

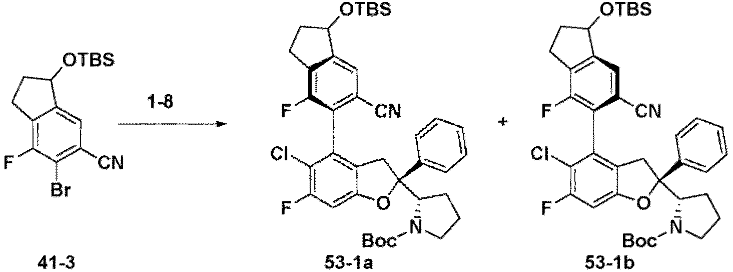

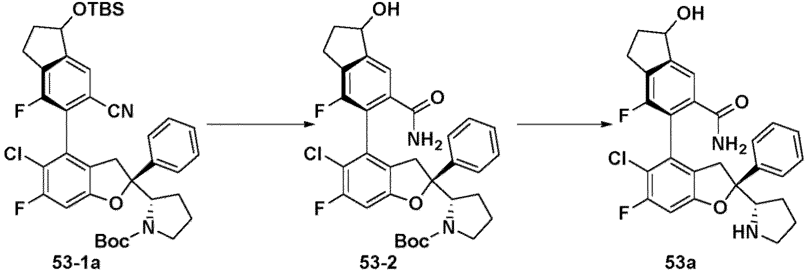

Step 1: Compound **41-3** (100 mg, 0.270 mmol) and compound **1-8** (190 mg, 0.351 mol) were dissolved in toluene (3 mL) and water (0.5 mL), then tris(dibenzylideneacetone)dipalladium (25 mg, 0.027 mmol), 4,5-bis(diphenylphosphino)-9,9-dimethylxanthene (31.3 mg, 0.054 mmol), and anhydrous potassium carbonate (111 mg, 0.81 mmol) were sequentially added thereto, and the reaction mixture was replaced with nitrogen three times and reacted at 120°C overnight. The same operation as above was repeated four times. LCMS showed that the starting material was completely reacted. 4 batches were combined for processing. The reaction mixture was cooled to 25°C, concentrated under reduced pressure, and purified by silica gel column chromatography (EA: PE = 1:10) to obtain a crude product as a yellow solid, which was then purified by preparative thin-layer chromatography (EA: PE = 1:7) to obtain compound **53-1a** (160 mg, Y: 20%, Rf = 0.5) and compound **53-1b** (140 mg, Y: 18%, Rf = 0.4). LC-MS: m/z (ESI): 651.4 [M-tBu+H]⁺.

Step 2: Compound **53-1a** (160 mg, 0.23 mmol) was dissolved in dimethyl sulfoxide (2 mL), then sodium hydroxide (18.4 mg, 0.46 mmol) and 30% hydrogen peroxide (30 wt%, 0.2 mL) were sequentially added thereto under nitrogen atmosphere, and the reaction mixture was reacted at 25°C for 2 hours. LCMS showed that the starting material was completely reacted. The reaction mixture was poured into saturated ammonium chloride (20 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **53-2** (120 mg, Y: 69%). LC-MS m/z (ESI): 611.2 [M+H]⁺.

Step 3: Compound **53-2** (120 mg, 0.19 mmol) was dissolved in dichloromethane (2 mL). The reaction mixture was cooled to 0°C under nitrogen atmosphere, added with a solution of hydrochloric acid in dioxane (2 mL, 4 M), and stirred at room temperature for 2 hours. LCMS showed that the starting material was completely reacted. The reaction mixture was cooled to 0°C, poured into saturated sodium bicarbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O/CH₃CN; gradient: 40% to 60%; flow rate: 20 mL/min; run time: 11 min; retention time: 7.9 min) to obtain compound **53a** (21.4 mg, Y: 21%). ¹H NMR (400 MHz, DMSO-*d₆*) δ 7.64 (s, 1H), 7.49-7.23 (m, 7H), 7.01 (d, *J* = 9.6 Hz, 1H), 5.58 (d, *J* = 5.6 Hz, 1H), 5.20-5.14 (m, 1H), 3.54-3.46 (m, 2H), 3.02-2.65 (m, 5H), 1.95-1.83 (m, 1H), 1.63-1.54 (m, 1H), 1.51-1.37 (m, 3H), 1.33-1.25 (m, 1H). LC-MS m/z (ESI): 511.2 [M+H]⁺.

### Example 54

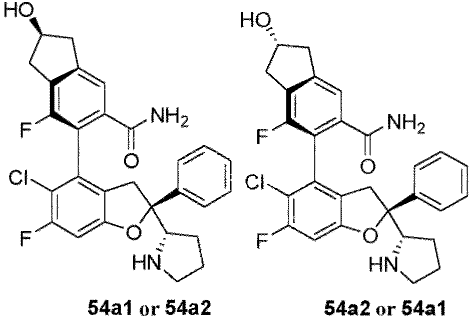

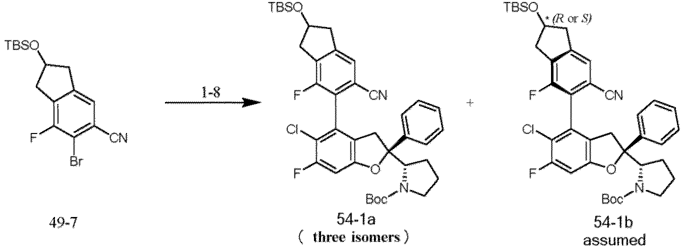

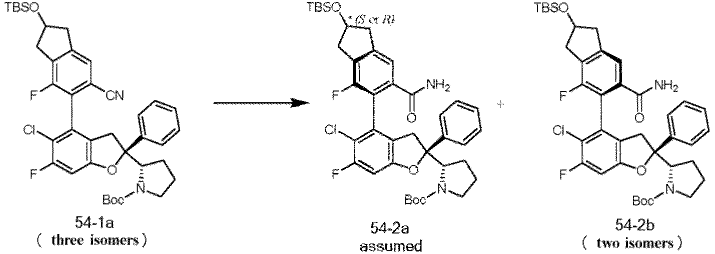

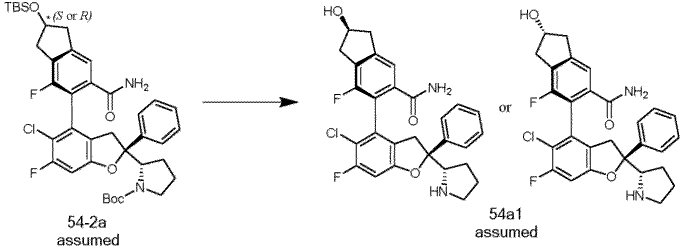

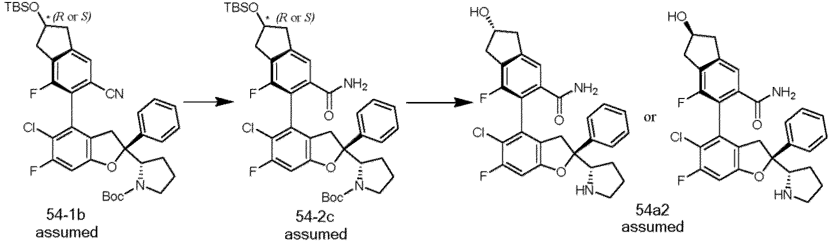

Step 1: Compound **49-7** (240 mg, 0.65 mmol) and compound **1-8** (380 mg, 0.70 mmol) were dissolved in toluene (6 mL) and water (1.2 mL), and tris(dibenzylideneacetone)dipalladium (65 mg, 0.07 mmol), 4,5-bis(diphenylphosphino)-9,9-dimethylxanthene (75 mg, 0.13 mmol), and anhydrous potassium phosphate (310 mg, 2.24 mmol) were sequentially added thereto. The reaction mixture was reacted at 110°C overnight under nitrogen atmosphere. TLC showed that the starting material was completely reacted. The reaction mixture was cooled to 25°C, concentrated under reduced pressure, and then purified by silica gel column chromatography (EA: PE = 1:30 to 1:2) to obtain compound **54-1a** (150 mg, Y: 32%, TLC (PE/EA= 5/1): Rf = 0.6, containing three isomers) and compound **54-1b** (90 mg, Y: 19%, TLC (PE/EA = 5/1): Rf = 0.5, containing one isomer, wherein * indicates R-configuration or S-configuration). LC-MS m/z (ESI): 651.3/653.4 [M-tBu+H]⁺.

Step 2: Compound **54-1a** (150 mg, 0.21 mmol) and anhydrous potassium carbonate (120 mg, 0.87 mmol) were dissolved in dimethyl sulfoxide (5 mL). The reaction mixture was cooled to 10°C, slowly added dropwise with hydrogen peroxide (599 mg, 5.28 mmol, 30%), and reacted at room temperature for 3 hours. LCMS showed that the reaction was completed. The reaction mixture was poured into water (30 mL), and extracted with ethyl acetate (20 mL × 2). The organic phases were combined, washed with saturated brine (30 mL), dried over anhydrous sodium sulfate, and filtered. The filtrate was concentrated under reduced pressure and then purified by a preparative silica gel plate (EA: PE = 1:2) to obtain compound **54-2a** (60 mg, Y: 39%, Rf = 0.6, containing one isomer, wherein * indicates S-configuration or R-configuration) and compound **54-2b** (30 mg, Y: 19%, Rf = 0.5, containing two isomers) as white solids.

Step 3: Compound **54-2a** (60 mg, 0.08 mmol) was dissolved in hydrochloric acid/dioxane solution (1 mL, 4.0 M), and the reaction mixture was reacted at 25°C for 0.5 hours. LCMS showed that the starting material was completely reacted. The reaction mixture was concentrated under reduced pressure and then purified by preparative high-performance liquid chromatography (column model: YMC-Actus Triart C18, 150 * 20 mm, 5 µm; eluent: 0.05% NH₃·H₂O-10 mmol/L NH₄HCO₃-ACN; gradient: 53% to 73%; flow rate: 20 mL/min; run time: 12 min; retention time: 7.8 min) to obtain compound **54a1** (17.7 mg, Y: 42%). Chiral analysis (column model: Celluiose-SB, 100 * 4.6 mm, 5 µm; eluent: hexane (0.1% DEA)/IPA; gradient: isocratic 4/1, flow rate: 1 mL/min; run time: 9 min; retention time: 4.68 min). ¹H NMR (400 MHz, DMSO-*d₆*): δ 7.63 (br, 1H), 7.48-7.26 (m, 7H), 7.01 (d, *J* = 9.6 Hz, 1H), 5.05 (d, *J* = 3.6 Hz, 1H), 4.65-4.58 (m, 1H), 3.58-3.50 (m, 2H), 3.25-3.08 (m, 2H), 2.90-2.60 (m, 5H), 1.69-1.22 (m, 4H). LC-MS m/z (ESI): 511.3/513.2 [M+H]⁺.

Step 4: Compound **54-1b** (90 mg, 0.13 mmol) and anhydrous potassium carbonate (100 mg, 0.72 mmol) were dissolved in dimethyl sulfoxide (5 mL). The reaction mixture was cooled to 10°C, slowly added dropwise with hydrogen peroxide (400 mg, 3.53 mmol, 30%), and reacted at room temperature for 0.5 hours. LCMS showed that the starting material was completely reacted. The reaction mixture was poured into water (20 mL), and extracted with ethyl acetate (20 mL × 2). The organic phases were combined, washed with saturated brine (10 mL), dried over anhydrous sodium sulfate, filtered, and the filtrate was concentrated under reduced pressure to obtain compound **54-2c** (60 mg, crude). LC-MS m/z (ESI): 725.3/727.4 [M+H]⁺.

Step 5: Compound **54-2c** (60 mg, 0.08 mmol) was dissolved in hydrochloric acid/dioxane solution (1 mL, 4.0 M), and the reaction mixture was reacted at 25°C for 0.5 hours. LCMS showed that the starting material was completely reacted. The reaction mixture was concentrated under reduced pressure, and then purified by preparative high-performance liquid chromatography (column model: YMC-Actus Triart C18, 150 * 20 mm, 5 µm; eluent: 0.1% HCl-ACN; gradient: 19% to 39%; flow rate: 20 mL/min; run time: 12 min; retention time: 8.0 min) to obtain compound **54a2** (23.9 mg, Y: 52%). Chiral analysis (column model: Celluiose-SB, 100 * 4.6 mm, 5 µm; eluent: hexane (0.1% DEA)/IPA; gradient: isocratic 4/1, flow rate: 1 mL/min; run time: 9 min; retention time: 4.20 min). ¹H NMR (400 MHz, DMSO-*d₆*): δ 9.58-9.42 (br, 1H), 8.32-8.22 (br, 1H), 7.85 (br, 1H), 7.55-7.50 (m, 2H), 7.48-7.32 (m, 5H), 7.11 (d, *J* = 9.2 Hz, 1H), 4.64-4.56 (m, 1H), 4.31-4.20 (m, 1H), 3.60 (d, *J* = 16.4 Hz, 1H), 3.20-3.02 (m, 4H), 2.92-2.72 (m, 3H), 2.22-2.12 (m, 1H), 1.96-1.70 (m, 3H). LC-MS m/z (ESI): 511.3/513.2 [M+H]⁺.

### Example 55

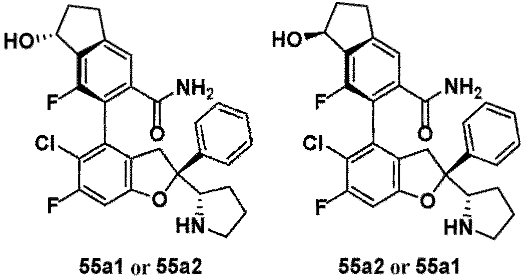

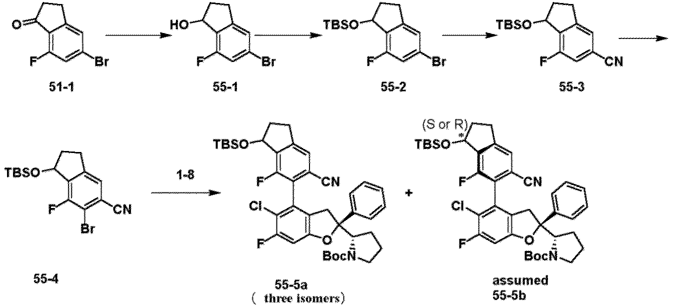

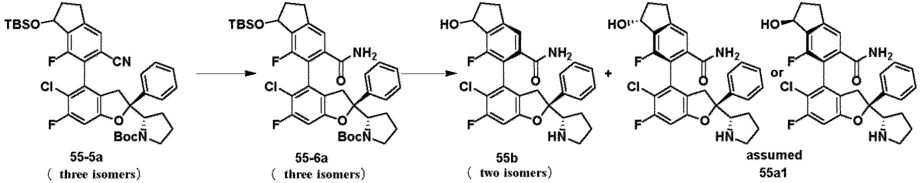

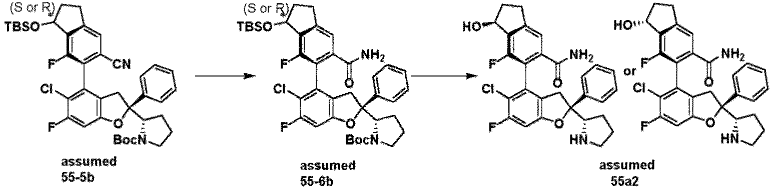

Step 1: Compound **51-1** (15.0 g, 65.8 mmol) was dissolved in methanol (160 mL). The reaction system was cooled to 0°C, and slowly added with sodium borohydride (3.7 g, 97.4 mmol) in batches. The reaction mixture was reacted at room temperature for 1 hour. TLC showed that the starting material was completely reacted. TLC showed that the starting material was completely reacted. The reaction mixture was slowly added with ice water (200 mL), and extracted with ethyl acetate (300 mL × 3). The organic phases were combined, washed with saturated brine (100 mL × 2), dried over anhydrous sodium sulfate, filtered, and the filtrate was concentrated under reduced pressure to obtain compound **55-1** (15.0 g, crude).

Step 2: Compound **55-1** (15.0 g, 65.2 mmol) was dissolved in dichloromethane (180 mL), and imidazole (10.0 g, 147.1 mmol) and tert-butyldimethylsilyl chloride (11.0, 73.0 mmol) were sequentially added thereto. The reaction mixture was reacted at room temperature for 12 hours under nitrogen atmosphere. TLC showed that the starting material was completely reacted. The reaction mixture was concentrated under reduced pressure and then purified by silica gel column chromatography (EA: PE = 1:100) to obtain compound **55-2** (16.0 g Y: 71%). ¹H NMR (400 MHz, CDCl₃): δ 7.15 (s, 1H), 7.02 (d, *J* = 8.8 Hz, 1H), 5.42-5.35 (m, 1H), 3.18-3.07 (m, 1H), 2.82-2.71 (m, 1H), 2.40-2.30 (m, 1H), 2.07-1.98 (m, 1H), 0.92-0.86 (m, 9H), 0.16-0.10 (m, 6H).

Step 3: Compound **55-2** (5.0 g, 14.5 mmol) was dissolved in anhydrous N,N-dimethylformamide (40 mL), and zinc cyanide (1.8 g, 15.4 mmol), 1,1'-bis(diphenylphosphino)ferrocene (1.6 g, 2.9 mmol), and tris(dibenzylideneacetone)dipalladium (1.3 g, 1.4 mmol) were sequentially added thereto. The reaction mixture was reacted at 100°C for 16 hours under nitrogen atmosphere. TLC showed that the starting material was completely reacted. The reaction mixture was cooled to 25°C, poured into ice water (200 mL), and extracted with ethyl acetate (200 mL × 2). The organic phases were combined, washed with saturated brine (80 mL × 2), dried over anhydrous sodium sulfate, and filtered. The filtrate was concentrated under reduced pressure and then purified by silica gel column chromatography (EA: PE = 1:100) to obtain compound **55-3** (3.0 g, Y: 71%). ¹H NMR (400 MHz, CDCl₃): δ 7.31 (s, 1H), 7.16 (d, *J* = 8.4 Hz, 1H), 5.46-5.40 (m, 1H), 3.20-3.10 (m, 1H), 2.90-2.79 (m, 1H), 2.45-2.33 (m, 1H), 2.11-2.00 (m, 1H), 0.89 (s, 9H), 0.19-0.13 (m, 6H).

Step 4: Compound **55-3** (3.0 g, 10.3 mmol) was dissolved in tetrahydrofuran (25 mL). The reaction mixture was cooled to -70°C, added dropwise with a solution of lithium diisopropylamide in tetrahydrofuran (5.7 mL, 11.4 mmol, 2 M) under nitrogen atmosphere, reacted at -70°C for 1 hour, then added dropwise with 1,2-dibromotetrafluoroethane (3.3 g, 12.7 mmol) in tetrahydrofuran (2 mL), and reacted at -70°C for 1 hour. TLC showed that the starting material was completely reacted. The reaction mixture was slowly added dropwise with saturated ammonium chloride solution (100 mL), and extracted with ethyl acetate (100 mL × 3). The organic phases were combined, washed with saturated brine (100 mL), dried over anhydrous sodium sulfate, and filtered. The filtrate was concentrated under reduced pressure and then purified by silica gel column chromatography (EA: PE = 1:90) to obtain compound **55-4** (2.8 g, Y: 73%) as a white solid. ¹H NMR (400 MHz, CDCl₃): δ 7.34 (s, 1H), 5.48-5.42 (m, 1H), 3.17-3.08 (m, 1H), 2.84-2.75 (m, 1H), 2.48-2.37 (m, 1H), 2.11-2.02 (m, 1H), 0.90 (s, 9H), 0.19-0.11 (m, 6H).

Step 5: Compound **55-4** (400 mg, 1.08 mmol) and compound **1-8** (500 mg, 0.92 mmol) were dissolved in toluene (6 mL) and water (1.2 mL), and tris(dibenzylideneacetone)dipalladium (95 mg, 0.10 mmol), 4,5-bis(diphenylphosphino)-9,9-dimethylxanthene (110 mg, 0.19 mmol), and anhydrous potassium carbonate (450 mg, 3.26 mmol) were sequentially added thereto. The reaction mixture was reacted at 110°C overnight under nitrogen atmosphere. TLC showed that the starting material was completely reacted. The reaction mixture was cooled to 25°C, concentrated under reduced pressure, and then purified by silica gel column chromatography (EA: PE = 1:10 to 1:1) to obtain compound **55-5a** (240 mg, Y: 31%, Rf = 0.6) and compound **55-5b** (150 mg, Y: 19%, Rf = 0.5). **55-5a:** LC-MS (1.5 mL-5 min-5 to 95% B): Rt = 3.930, m/z (ESI): 651.3 [M-56+H]⁺. **55-5b:** LC-MS (1.5 mL-5 min-5 to 95% B): Rt = 3.923, m/z (ESI): 651.3 [M-56+H]⁺.

Step 6: Compound **55-5a** (240 mg, 0.34 mmol) was dissolved in dimethyl sulfoxide (8 mL). The reaction mixture was added with potassium carbonate (140 mg, 1.01 mmol), cooled to 5 to 10°C, slowly added dropwise with hydrogen peroxide (120 mg, 1.06 mmol, 30%), and reacted at 25°C for 1 hour. TLC showed that the starting material was completely reacted. The reaction mixture was poured into water (20 mL), and extracted with ethyl acetate (30 mL × 2). The organic phases were combined, washed with saturated brine (20 mL), dried over anhydrous sodium sulfate, filtered, and the filtrate was concentrated under reduced pressure to obtain compound **55-6a** (120 mg, crude).

Step 7: Compound **55-6a** (120 mg, 0.12 mmol) was dissolved in anhydrous dichloromethane (3 mL), then trifluoroacetic acid (1.5 mL) was added thereto, and the reaction mixture was reacted at 25°C for 0.5 hours. LCMS showed that the starting material was completely reacted. The reaction mixture was slowly added with saturated sodium carbonate aqueous solution (40 mL), and extracted with ethyl acetate (40 mL × 2). The organic phases were combined, washed with saturated brine (15 mL), dried over anhydrous sodium sulfate, and filtered. The filtrate was concentrated under reduced pressure, and then purified by preparative high-performance liquid chromatography (column model: YMC-Actus Triart C18, 150 * 20 mm, 5 µm; eluent: 0.1% HCl-CH₃CN; gradient: 30% to 70% CH₃CN; flow rate: 20 mL/min; run time: 14 min; retention time: RT1 = 7.2 min (P1), RT2 = 8.9 min (P2), RT3 = 9.4 min (P3)) and lyophilized to obtain a mixture of isomers P1 and P2 as compound **55b** without purification. P3 was purified by preparative high-performance liquid chromatography (column model: Xselect CSH Prep Fluoro-Phenyl, 150 * 19 mm, 5 µm; eluent: 0.1% FA-CH₃CN; gradient: 35% to 55% CH₃CN; flow rate: 20 mL/min; run time: 9 min; retention time: 8.6 min) to obtain compound **55a1** (30.9 mg, Y: 33%, FA salt). ¹H NMR (400 MHz, DMSO-*d₆*): δ 8.16 (br, 1H), 7.69 (s, 1H), 7.48-7.27 (m, 7H), 7.03 (d, *J* = 10.0 Hz, 1H), 5.38-5.22 (m, 2H), 3.73-3.62 (m, 1H), 3.54 (d, *J* = 15.6 Hz, 1H), 3.17-3.08 (m, 1H), 2.92-2.62 (m, 5H), 2.36-2.29 (m, 1H), 1.99-1.36 (m, 4H). LC-MS m/z (ESI): 511.2/513.2 [M+H]⁺. Chiral analysis (column model: Amylose-SA, 100 * 4.6 mm, 5 µm; eluent: hexane (0.1% DEA)-IPA; gradient: isocratic 4/1, flow rate: 1 mL/min; run time: 6.4 min; retention time: 3.14 min).

Step 8: Compound **55-5b** (150 mg, 0.21 mmol) was dissolved in dimethyl sulfoxide (4 mL). The reaction mixture was added with potassium carbonate (88 mg, 0.64 mmol), cooled to 5 to 10°C, slowly added dropwise with hydrogen peroxide (80 mg, 0.71 mmol, 30%), and reacted at 25°C for 1 hour. TLC showed that the starting material was completely reacted. The reaction mixture was poured into water (10 mL), and extracted with ethyl acetate (10 mL × 2). The organic phases were combined, washed with saturated brine (10 mL), dried over anhydrous sodium sulfate, filtered, and the filtrate was concentrated under reduced pressure to obtain compound **55-6b** (90 mg, crude).

Step 9: Compound **55-6b** (90 mg, 0.12 mmol) was dissolved in anhydrous dichloromethane (1 mL), then trifluoroacetic acid (0.5 mL) was added thereto, and the reaction mixture was reacted at 25°C for 0.5 hours. LCMS showed that the starting material was completely reacted. The reaction mixture was slowly added with saturated sodium carbonate aqueous solution (20 mL), and extracted with ethyl acetate (30 mL × 2). The organic phases were combined, washed with saturated brine (10 mL), dried over anhydrous sodium sulfate, and filtered. The filtrate was concentrated under reduced pressure and then purified by preparative high-performance liquid chromatography (column model: Xselect CSH Prep Fluoro-Phenyl, 150 * 19 mm, 5 µm; eluent: 0.1% FA-CH₃CN; gradient: 31 to 50% CH₃CN in H₂O; flow rate: 20 mL/min; run time: 9 min; retention time: 7.2 min) to obtain compound **55a2** (29.1 mg, Y: 42%, FA salt) as a white solid. ¹H NMR (400 MHz, DMSO-*d₆*): δ 8.17 (br, 1H), 7.65 (s, 1H), 7.48-7.23 (m, 7H), 7.01 (d, *J* = 9.6 Hz, 1H), 5.28-5.23 (m, 1H), 3.63-3.58 (m, 1H), 3.47 (d, *J* = 16.0 Hz, 1H), 3.18-3.08 (m, 1H), 2.90-2.64 (m, 4H), 2.54-2.51 (m, 1H), 2.40-2.30 (m, 1H), 2.01-1.89 (m, 1H), 1.70-1.30 (m, 3H). LC-MS m/z (ESI): 511.3/513.2 [M+H]⁺. Chiral analysis (column model: Amylose-SA, 100 * 4.6 mm, 5 µm; eluent: hexane (0.1% DEA)-IPA; gradient: isocratic 4/1, flow rate: 1 mL/min; run time: 6.4 min; retention time: 3.04 min).

### Example 56

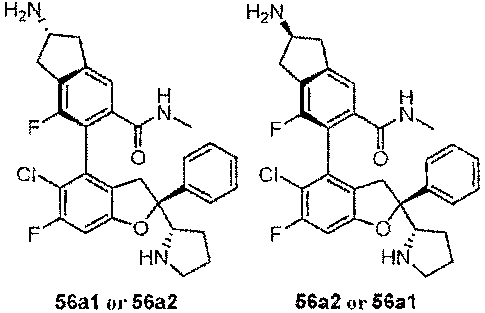

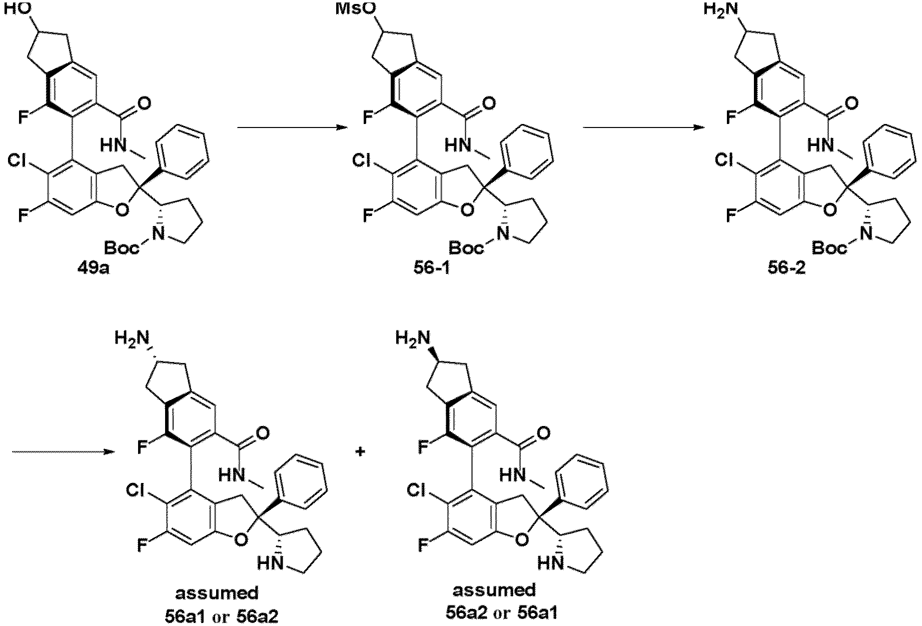

Step 1: Compound **49a** (80 mg, 0.13 mmol) was dissolved in anhydrous dichloromethane (2 mL), then triethylamine (40 mg, 0.39 mmol) and methanesulfonyl chloride (30 mg, 0.26 mmol) were sequentially added thereto, and the reaction mixture was reacted at room temperature for 1 hour under nitrogen atmosphere. LCMS showed that the starting material was completely reacted. The reaction mixture was added with water (20 mL), and extracted with ethyl acetate (15 mL × 2). The organic phases were combined, washed with saturated brine (10 mL), dried over anhydrous sodium sulfate, and filtered. The filtrate was concentrated under reduced pressure and then purified by silica gel column chromatography (EA: PE = 1:2) to obtain compound **56-1** (70 mg, Y: 77%). LC-MS m/z (ESI): 703.3/705.3 [M+H]⁺.

Step 2: Compound **56-1** (70 mg, 0.10 mmol) was dissolved in dioxane (1 mL), then ammonia water (0.3 mL, 25%) was added thereto, and the reaction mixture was reacted at 60°C for 12 hours under airtight conditions. LCMS showed that the starting material was completely reacted. The reaction mixture was cooled to 25°C, concentrated under reduced pressure, and purified by silica gel column chromatography (EA: PE = 2:1) to obtain compound **56-2** (40 mg, Y: 64%). LC-MS m/z (ESI): 624.3/626.3 [M+H]⁺.

Step 3: Compound **56-2** (40 mg, 0.06 mmol) was dissolved in hydrochloric acid/dioxane solution (1 mL, 4.0 M), and the reaction mixture was reacted at 25°C for 0.5 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% HCl-ACN; gradient: 19% to 39%; flow rate: 20 mL/min; run time: 13 min; retention time: 7.9 min/8.4 min) to obtain compound **56a1** (8.2 mg, HCl salt, Y: 22%, Rt = 8.4 min) and compound **56a2** (7.2 mg, HCl salt, Y: 20%, Rt = 7.9 min).

**56a1:** ¹H NMR (400 MHz, DMSO-*d₆*): δ 9.40-9.30 (br, 1H), 8.45-8.30 (br, 4H), 7.52-7.36 (m, 6H), 7.14 (d, *J* = 9.2 Hz, 1H), 4.36-4.29 (m, 1H), 4.18-4.07 (m, 1H), 3.57-3.30 (m, 1H), 3.20-2.85 (m, 5H), 2.63 (d, *J* = 4.4 Hz, 3H), 2.18-2.05 (m, 1H), 1.98-1.65 (m, 3H). LC-MS m/z (ESI): 524.2/526.3 [M+H]⁺. Chiral analysis (column model: Celluiose-SB 100 * 4.6 mm, 5 µm; eluent: hexane (0.1% DEA)-IPA; gradient: isocratic 6/4, flow rate: 1 mL/min; run time: 8.0 min; retention time: 3.89 min).

**56a2:** ¹H NMR (400 MHz, DMSO-*d₆*): ¹H NMR (400 MHz, DMSO-*d₆*): δ 9.38-9.26 (br, 1H), 8.46-8.30 (br, 4H), 7.55-7.35 (m, 6H), 7.14 (d, *J* = 9.6 Hz, 1H), 4.34-4.24 (m, 1H), 4.16-4.05 (m, 1H), 3.53-3.38 (m, 2H), 3.21-2.85 (m, 5H), 2.64 (d, *J* = 4.4 Hz, 3H), 2.15-2.06 (m, 1H), 1.97-1.68 (m, 3H). LC-MS m/z (ESI): 524.3/526.3 [M+H]⁺. Chiral analysis (column model: Celluiose-SB 100 * 4.6 mm, 5 µm; eluent: hexane (0.1% DEA)-IPA; gradient: isocratic 6/4, flow rate: 1 mL/min; run time: 8.0 min; retention time: 3.53 min).

### Example 57

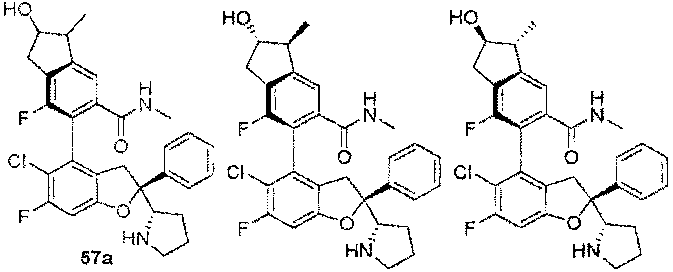

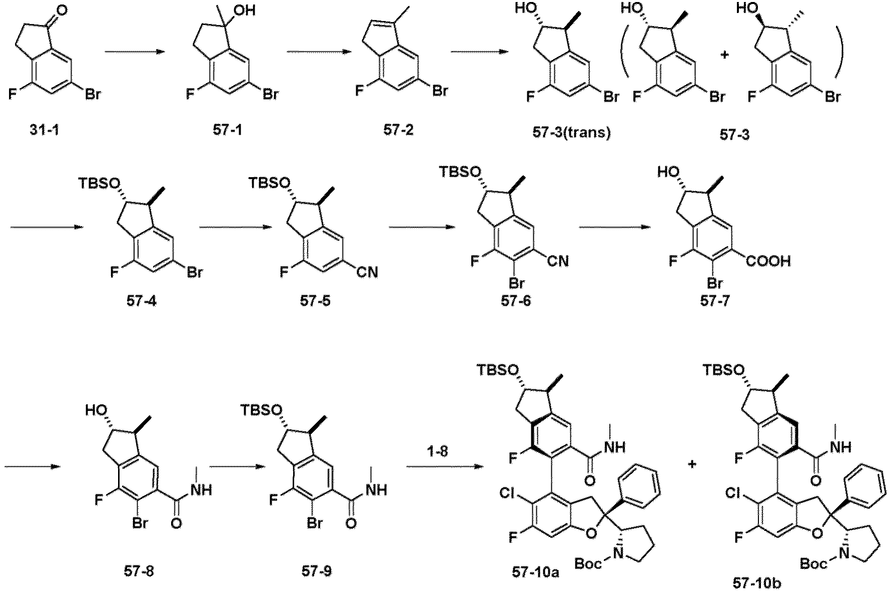

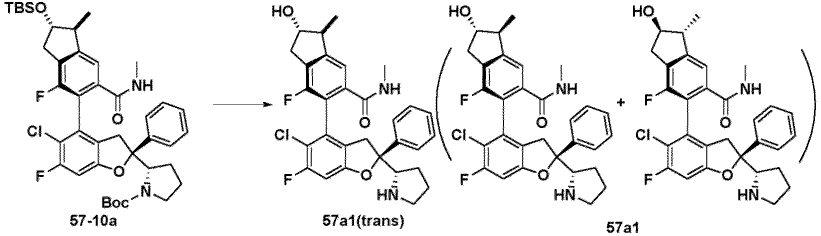

Step 1: **31-1** (10.0 g, 43.6 mmol) was dissolved in toluene (200 mL). The reaction mixture was cooled to -50°C under nitrogen atmosphere, added dropwise with methylmagnesium bromide (3.0 M, 21.8 mL), and reacted at -50°C for 1 hour. TLC (PE: EA = 5:1) showed that the starting material was completely reacted. The reaction mixture was warmed to 0°C, poured into saturated ammonium chloride (200 mL) to quench, and extracted with ethyl acetate (100 mL × 3). The organic phases were combined, washed with saturated brine (300 mL), dried over anhydrous sodium sulfate, filtered, and the filtrate was concentrated under reduced pressure to obtain compound **57-1** (10.0 g, Y: 93%).

Step 2: Compound **57-1** (10.0 g, 40.6 mmol) was dissolved in toluene (60 mL), then anhydrous p-toluenesulfonic acid (931 mg, 4.06 mmol) was added thereto under nitrogen atmosphere, and the reaction mixture was reacted at 100°C for 16 hours. TLC (PE: EA = 5:1) showed that the starting material was completely reacted. The reaction mixture was cooled to 25°C, poured into saturated sodium bicarbonate (100 mL) to quench, and extracted with ethyl acetate (50 mL × 3). The organic phases were combined, washed with saturated brine (100 mL), dried over anhydrous sodium sulfate, and filtered. The filtrate was concentrated under reduced pressure and then purified by silica gel column chromatography (PE: EA = 50:1) to obtain compound **57-2** (6.1 g, Y: 66%).

Step 3: Compound **57-2** (6.1 g, 26.8 mmol) was dissolved in tetrahydrofuran (60 mL). The reaction mixture was cooled to 0°C under nitrogen atmosphere, added dropwise with a solution of borane in tetrahydrofuran (1 M, 35.2 mL) and stirred for 10 minutes, warmed to room temperature and stirred for 12 hours, cooled to 0°C, sequentially added dropwise with sodium hydroxide solution (3 M, 4.7 mL) and 30% hydrogen peroxide (3.4 mL) and stirred for 10 minutes, and warmed to room temperature and stirred for 4 hours. TLC (PE: EA = 10:1) showed that the starting material was completely reacted. The reaction mixture was poured into saturated sodium thiosulfate (100 mL) to quench, and extracted with ethyl acetate (50 mL × 3). The organic phases were combined, washed with saturated brine (100 mL), dried over anhydrous sodium sulfate, and filtered. The filtrate was concentrated under reduced pressure and then purified by silica gel column chromatography (PE: EA = 10:1) to obtain compound **57-3** (2.2 g, Y: 19%). ¹H NMR (400 MHz, DMSO-*d₆*) δ 7.32-7.26 (m, 1H), 7.25 (s, 1H), 5.24 (d, *J* = 5.2 Hz, 1H), 4.03-3.96 (m, 1H), 3.13-3.05 (m, 1H), 2.99-2.90 (m, 1H), 2.63-2.56 (m, 1H), 1.20 (d, *J* = 7.2 Hz, 3H). NOESY identified compound **57-3** as being in the trans configuration.

Step 4: Compound **57-3** (2.2 g, 9.02 mmol) was dissolved in dichloromethane (20 mL), then compound tert-butyldimethylsilyl chloride (1.01 g, 12.2 mmol) and imidazole (1.39 g, 20.4 mmol) were sequentially added thereto under nitrogen atmosphere, and the reaction mixture was reacted at 25°C for 12 hours. TLC (PE: EA = 10:1) showed that the starting material was completely reacted. The reaction mixture was poured into saturated ammonium chloride (50 mL), and extracted with ethyl acetate (30 mL × 3). The organic phases were combined, washed with saturated brine (50 mL), dried over anhydrous sodium sulfate, filtered, and the filtrate was concentrated under reduced pressure to obtain compound **57-4** (2.5 g, Y: 68%).

Step 5: Compound **57-4** (2.5 g, 6.96 mmol) was dissolved in N,N-dimethylacetamide (25 mL), then compound zinc cyanide (816 mg, 6.96 mmol), tris(dibenzylideneacetone)dipalladium (636 mg, 0.695 mmol), and 1,1'-bis(diphenylphosphino)ferrocene (770 mg, 1.39 mmol) were sequentially added thereto under nitrogen atmosphere, and the reaction mixture was reacted at 100°C for 16 hours. TLC (PE: EA = 20:1) showed that the starting material was completely reacted. The reaction mixture was cooled to 25°C, poured into water (100 mL), and extracted with ethyl acetate (50 mL × 3). The organic phases were combined, washed with saturated brine (100 mL), dried over anhydrous sodium sulfate, and filtered. The filtrate was concentrated under reduced pressure and then purified by silica gel column chromatography (PE: EA = 5:1) to obtain compound **57-5** (2.0 g, Y: 94%). ¹H NMR (400 MHz, DMSO-*d*₆) δ 7.66-7.56 (m, 2H), 4.23-4.16 (m, 1H), 3.33-3.24 (m, 1H), 3.07-2.98 (m, 1H), 2.76-2.68 (m, 1H), 1.24(d, *J* = 7.2 Hz, 3H), 0.88 (s, 9H), 0.10 (s, 6H).

Step 6: Compound **57-5** (2.0 g, 2.84 mmol) was dissolved in tetrahydrofuran (20 mL). The reaction mixture was cooled to -70°C under nitrogen atmosphere, added with compound lithium diisopropylamide (2 M, 4.26 mL) and stirred at -70°C for 1 hour, then added with compound 1,2-dibromotetrachloroethane (2.21 g, 8.51 mmol) dissolved in tetrahydrofuran (20 mL) at -70°C and stirred for 30 minutes, and warmed to room temperature and stirred for 1 hour. TLC (PE: EA = 4:1) showed that the starting material was completely reacted. The reaction mixture was poured into saturated ammonium chloride (100 mL) to quench, and extracted with ethyl acetate (50 mL × 3). The organic phases were combined, washed with saturated brine (100 mL), dried over anhydrous sodium sulfate, and filtered. The filtrate was concentrated under reduced pressure and then purified by silica gel column chromatography (PE: EA = 5:1) to obtain compound **57-6** (1.3 g, Y: 67%). ¹H NMR (400 MHz, DMSO-*d*₆) δ 7.71 (s, 1H), 4.25-4.15 (m, 1H), 3.39-3.31 (m, 1H), 3.04-2.74 (m, 2H), 2.76-2.68 (m, 1H), 1.23 (d, *J* = 7.2 Hz, 3H), 0.84 (s, 9H), 0.09 (s, 6H).

Step 7: Compound **57-6** (800 mg, 2.08 mmol) was dissolved in anhydrous ethanol (10 mL) and water (5 mL), then compound sodium hydroxide (832 mg, 20.8 mmol) was added thereto, and the reaction mixture was reacted at 100°C for 16 hours. TLC (PE: EA = 10:1) showed that the starting material was completely reacted. The reaction mixture was cooled to 25°C, poured into saturated ammonium chloride (50 mL), and extracted with ethyl acetate (30 mL × 3). The organic phases were combined, washed with saturated brine (50 mL), dried over anhydrous sodium sulfate, filtered, and the filtrate was concentrated under reduced pressure to obtain compound **57-7** (500 mg, Y: 67%). LC-MS m/z (ESI): 286.9 [M-H]⁻.

Step 8: Compound **57-7** (500 mg, 1.73 mmol) was dissolved in N,N-dimethylformamide (8 mL), then compound methylamine hydrochloride (280 mg, 4.15 mmol), N,N-diisopropylethylamine (0.54 mL, 3.11 mmol), and 2-(7-azabenzotriazol-1-yl)-N,N,N',N'-tetramethyluronium hexafluorophosphate (1.59 g, 4.15 mmol) were sequentially added thereto, and the reaction mixture was reacted at room temperature for 2 hours. LCMS showed that the starting material was completely reacted. The reaction mixture was poured into saturated ammonium chloride (50 mL), and extracted with ethyl acetate (30 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 5:1) to obtain compound **57-8** (200 mg, Y: 31%) as a yellow oil. LC-MS m/z (ESI): 302.1 [M+H]⁺.

Step 9: Compound **57-8** (200 mg, 0.66 mmol) was dissolved in dichloromethane (2 mL), then compound tert-butyldimethylsilyl chloride (199 mg, 1.32 mmol) and imidazole (1.35 mg, 1.99 mmol) were sequentially added thereto under nitrogen atmosphere, and the reaction mixture was reacted at 25°C for 12 hours. TLC (PE: EA = 1:1) showed that the starting material was completely reacted. The reaction mixture was poured into saturated ammonium chloride (20 mL), and extracted with ethyl acetate (10 mL × 3). The organic phases were combined, washed with saturated brine (20 mL), dried over anhydrous sodium sulfate, and filtered. The filtrate was concentrated under reduced pressure and then purified by silica gel column chromatography (PE: EA= 10:1) to obtain compound **57-9** (150 mg, Y: 54%). ¹H NMR (400 MHz, DMSO-*d*₆) δ 8.38-8.29 (m, 1H), 7.08 (s, 1H), 4.20-4.13 (m, 1H), 3.30-3.22 (m, 1H), 3.00-2.89 (m, 1H), 2.79-2.65 (m, 4H), 1.22 (d, *J* = 7.2 Hz, 3H), 0.88 (s, 9H), 0.09 (s, 6H).

Step 10: Compound **57-9** (140 mg, 0.33 mmol) was dissolved in toluene (1 mL) and water (0.2 mL), then compound **1-8** (274 mg, 0.51 mmol), tris(dibenzylideneacetone)dipalladium (61.5 mg, 0.067 mmol), 4,5-bis(diphenylphosphino)-9,9-dimethylxanthene (69.6 mg, 0.13 mmol), and anhydrous potassium carbonate (139 mg, 1.01 mmol) were sequentially added thereto under nitrogen atmosphere, and the reaction mixture was reacted at 110°C overnight. TLC (PE: EA = 1:1) showed that the starting material was completely reacted. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compounds **57-10a** (50.00 mg, Y: 19%, Rf = 0.30) and **57-10b** (100 mg, Y: 39%, Rf = 0.35). **57-10a:** LC-MS (1.5 mL-5 min-5 to 90% B): Rt = 3.897 m/z, m/z (ESI): 753.3 [M+H]⁺. **57-10b:** LC-MS (1.5 mL-5 min-5 to 90% B): Rt = 3.830, m/z (ESI): 753.3 [M+H]⁺.

Step 11: Compound **57-10a** (50 mg, 0.066 mmol) was dissolved in dichloromethane (1 mL). The reaction mixture was cooled to 0°C under nitrogen atmosphere, added with trifluoroacetic acid (1 mL), warmed to room temperature, and stirred for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-pressure liquid chromatography (column model: YMC-Actus Triart C18, 150 * 20 mm, 5 µm; eluent: 0.05% NH₃·H₂O/CH₃CN; gradient: 39.9% to 59.9%; flow rate: 20 mL/min; run time: 13 min; retention time: 10.5 min) to obtain compound **57a1** (15.7 mg, Y: 43%). ¹H NMR (400 MHz, DMSO-*d*₆) δ 8.16-8.09 (m, 1H), 7.47-7.22 (m, 6H), 7.02-6.96 (m, 1H), 5.30-5.24 (m, 1H), 4.11-4.02 (m, 1H), 3.53-3.39 (m, 2H), 3.21-2.79 (m, 3H), 2.75-2.56 (m, 5H), 1.60-1.21 (m, 7H). LC-MS m/z (ESI): 539.2 [M+H]⁺. Chiral analysis (column model: Amylose-C, 100 * 4.6 mm, 5 µm; eluent: hexane (0.1% DEA)/IPA; gradient: isocratic 8/2, flow rate: 1 mL/min; run time: 6 min; retention time: 1.95 min and 2.88 min, dr = 65 (1.95 min):35 (2.88 min)).

### Example 64

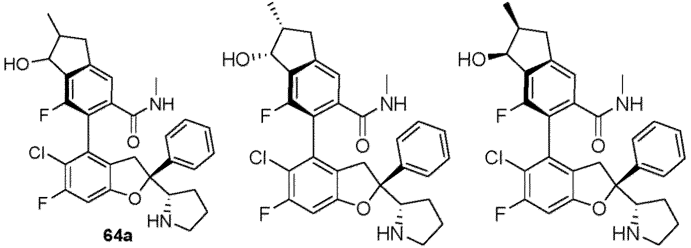

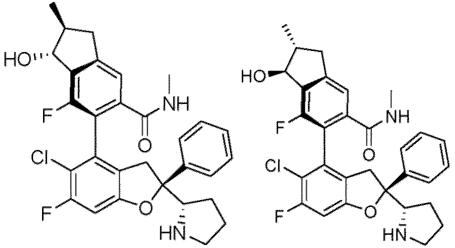

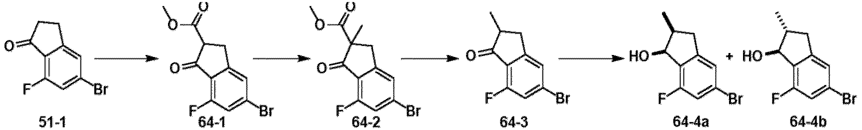

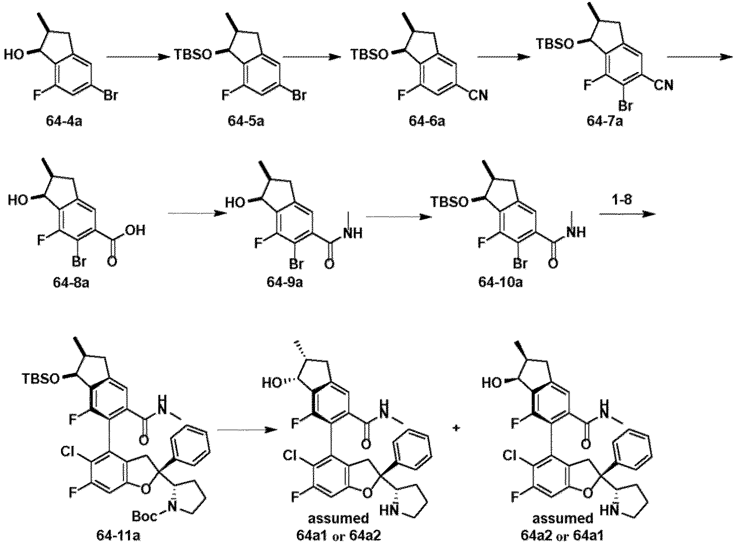

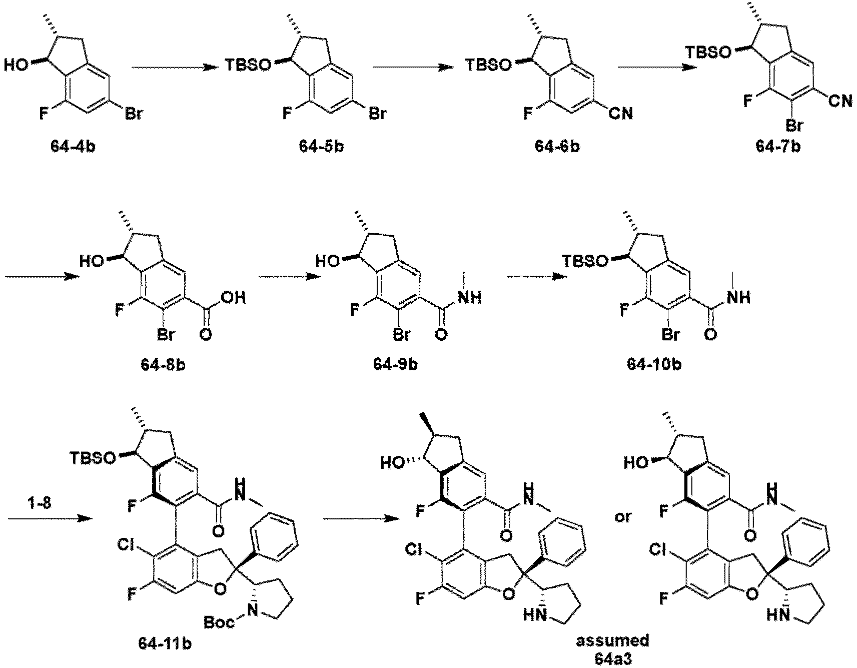

Step 1: Compound **51-1** (16.0 g, 69.9 mmol) was dissolved in dimethyl carbonate (70 mL). The reaction system was cooled to 0°C, and slowly added with sodium hydride (8.3 g, 209.6 mmol, 60%) in batches. The reaction mixture was reacted at room temperature for 1 hour under nitrogen atmosphere. LCMS showed that the starting material was completely reacted. The reaction mixture was poured into ice water (300 mL), and extracted with ethyl acetate (400 mL × 3). The organic phases were combined, washed with saturated brine (100 mL × 2), dried over anhydrous sodium sulfate, and filtered. The filtrate was concentrated under reduced pressure and then purified by silica gel column chromatography (EA: PE = 1:5) to obtain compound **64-1** (11.3 g, Y: 56%) as a white solid. LC-MS m/z (ESI): 287.2/289.1 [M+H]⁺.

Step 2: Compound **64-1** (11.3 g, 39.4 mmol) was dissolved in N,N-dimethylformamide (100 mL), and iodomethane (11.2 g, 78.8 mmol) and anhydrous potassium carbonate (11.0, 79.7 mmol) were sequentially added thereto. The reaction mixture was reacted at 50°C for 12 hours under nitrogen atmosphere. LCMS showed that the starting material was completely reacted. The reaction mixture was poured into ice water (300 mL), and extracted with ethyl acetate (200 mL × 2). The organic phases were combined, washed with saturated brine (80 mL × 2), dried over anhydrous sodium sulfate, and filtered. The filtrate was concentrated under reduced pressure and then purified by silica gel column chromatography (EA: PE = 1:8) to obtain compound **64-2** (10.1 g, Y: 85%). LC-MS m/z (ESI): 301.1/303.1 [M+H]⁺.

Step 3: Compound **64-2** (5.8 g, 19.3 mmol) was dissolved in methanol (44 mL), then sodium hydroxide aqueous solution (2.3 g, 57.5 mmol) dissolved in water (11 mL) was added thereto, and the reaction mixture was reacted at room temperature for 2 hours. LCMS showed that the starting material was completely reacted. The reaction mixture was added with water (600 mL), and extracted with ethyl acetate (150 mL × 2). The organic phases were combined, washed with saturated brine (30 mL × 2), dried over anhydrous sodium sulfate, filtered, and the filtrate was concentrated under reduced pressure to obtain compound **64-3** (3.9 g, Y: 83%). LC-MS m/z (ESI): 243.1/245.0 [M+H]⁺.

Step 4: Compound **64-3** (3.9 g, 16.0 mmol) was dissolved in tetrahydrofuran (35 mL). The reaction mixture was cooled to 0°C, added with solid sodium borohydride (1.2 g, 31.6 mmol) in batches, and reacted at room temperature for 3 hours. TLC showed that the starting material was completely reacted. The reaction mixture was slowly added with ice water (200 mL), and extracted with ethyl acetate (150 mL × 2). The organic phases were combined, washed with saturated brine (40 mL), dried over anhydrous sodium sulfate, and filtered. The filtrate was concentrated under reduced pressure and then purified by silica gel column chromatography (EA: PE = 1:10 to EA: PE = 1:1, Rf = 0.5 and Rf = 0.4) to obtain compounds **64-4a** (1.1 g, Y: 28%, Rf = 0.5) and **64-4b** (1.3 g, Y: 33%, Rf = 0.4). NOESY identified compound **64-4a** as being in the cis configuration and **64-4b** as being in the trans configuration. **64-4a:** ¹H NMR (400 MHz, CDCl₃): δ 7.17 (s, 1H), 7.06 (d, *J* = 8.4 Hz, 1H), 5.15 (d, *J* = 6.0 Hz, 1H), 2.98-2.90 (m, 1H), 2.78-2.70 (m, 1H), 2.54-2.42 (m, 1H), 1.20 (d, *J* = 6.8 Hz, 3H). **64-4b:** ¹H NMR (400 MHz, CDCl₃): δ 7.15 (s, 1H), 7.06 (d, *J* = 8.8 Hz, 1H), 4.95 (d, *J* = 4.8 Hz, 1H), 3.28-3.17 (m, 1H), 2.49-2.36 (m, 2H), 1.21-1.16 (m, 3H).

Step 5: Compound **64-4a** (1.1 g, 4.5 mmol) was dissolved in anhydrous dichloromethane (15 mL). The reaction mixture was cooled to 0°C, sequentially added with imidazole (0.9 g, 13.2 mmol) and tert-butyldimethylsilyl chloride (1.0 g, 6.67 mmol), and reacted at room temperature for 5 hours under nitrogen atmosphere. TLC showed that the starting material was completely reacted. The reaction mixture was added with water (60 mL), and extracted with ethyl acetate (80 mL × 2). The organic phases were combined, washed with saturated brine (30 mL), dried over anhydrous sodium sulfate, and filtered. The filtrate was concentrated under reduced pressure and then purified by silica gel column chromatography (EA: PE = 1:40) to obtain compound **64-5a** (1.3 g, Y: 80%) as a colorless oil.

Step 6: Compound **64-5a** (1.3 g, 3.6 mmol) was dissolved in N,N-dimethylformamide (10 mL), and zinc cyanide (260 mg, 2.2 mmol), tris(dibenzylideneacetone)dipalladium (330 mg, 0.36 mmol), and 1,1'-bis(diphenylphosphino)ferrocene (400 mg, 0.72 mmol) were sequentially added thereto. The reaction mixture was reacted at 100°C for 16 hours under nitrogen atmosphere. TLC showed that the starting material was completely reacted. The reaction mixture was cooled to 25°C, added with ice water (100 mL), and extracted with ethyl acetate (150 mL × 3). The organic phases were combined, washed with saturated brine (80 mL), dried over anhydrous sodium sulfate, and filtered. The filtrate was concentrated under reduced pressure and then purified by silica gel column chromatography (EA: PE = 1:30) to obtain compound **64-6a** (700 mg, Y: 63%). ¹H NMR (400 MHz, CDCl₃): δ 7.29 (s, 1H), 7.15 (d, *J* = 8.8, 1H), 5.17 (d, *J* = 5.2, 1H), 2.91-2.72 (m, 2H), 2.49-2.38 (m, 1H), 1.13 (d, *J* = 6.8, 3H), 0.86 (s, 9H), 0.15 (s, 3H), 0.06 (s, 3H).

Step 7: Compound **64-6a** (700 mg, 2.3 mmol) was dissolved in tetrahydrofuran (5 mL). The reaction mixture was cooled to -70°C, added dropwise with a solution of lithium diisopropylamide in tetrahydrofuran (1.3 mL, 2.6 mmol, 2 M) under nitrogen atmosphere, reacted at -70°C for 1 hour, then added dropwise with 1,2-dibromotetrafluoroethane (670 mg, 2.6 mmol) in tetrahydrofuran (1 mL), and reacted at -70°C for 1 hour. TLC showed that the starting material was completely reacted. The reaction mixture was slowly added dropwise with saturated ammonium chloride solution (50 mL), and extracted with ethyl acetate (50 mL × 3). The organic phases were combined, washed with saturated brine (15 mL), dried over anhydrous sodium sulfate, and filtered. The filtrate was concentrated under reduced pressure and then purified by silica gel column chromatography (EA: PE = 1:30) to obtain compound **64-7a** (640 mg, Y: 72%) as a light yellow solid. ¹H NMR (400 MHz, CDCl₃): δ 7.32 (s, 1H), 5.19 (d, *J* = 5.2, 1H), 2.90-2.69 (m, 2H), 2.51-2.41 (m, 1H), 1.12 (d, *J* = 6.8, 3H), 0.87 (s, 9H), 0.16 (s, 3H), 0.07 (s, 3H).

Step 8: Compound **64-7a** (640 mg, 1.7 mmol) was dissolved in ethanol (4 mL) and water (4 mL), then sodium hydroxide (660 mg, 16.5 mmol) was added thereto, and the reaction mixture was reacted at 100°C for 16 hours. TLC showed that the starting material was completely reacted. The reaction mixture was cooled to 0°C, added with water (20 mL), and then slowly added with dilute hydrochloric acid (0.5 M) until the pH of the reaction system was 3 to 4. The reaction mixture was extracted with ethyl acetate (70 mL × 3). The organic phases were combined, washed with saturated brine (30 mL), dried over anhydrous sodium sulfate, filtered, and the filtrate was concentrated under reduced pressure to obtain compound **64-8a** (420 mg, crude).

Step 9: Compound **64-8a** (420 mg, 1.5 mmol) was dissolved in N,N-dimethylformamide (5 mL), then diisopropylethylamine (760 mg, 5.9 mmol), 2-(7-azabenzotriazol-1-yl)-N,N,N',N'-tetratnethyluronium hexafluorophosphate (600 mg, 1.6 mmol), and methylamine hydrochloride (180 mg, 2.7 mmol) were sequentially added thereto, and the reaction mixture was reacted at room temperature for 2 hours under nitrogen atmosphere. LCMS showed that the starting material was completely reacted. The reaction mixture was added with water (30 mL), and extracted with ethyl acetate (30 mL × 2). The organic phases were combined, washed with saturated brine (20 mL), dried over anhydrous sodium sulfate, and filtered. The filtrate was concentrated under reduced pressure and then purified by silica gel column chromatography (EA: PE = 1:5) to obtain compound **64-9a** (380 mg, Y: 86%). LC-MS m/z (ESI): 302.0/304.0 [M+H]⁺.

Step 10: Compound **64-9a** (380 mg, 1.7 mmol) was dissolved in dichloromethane (6 mL), and imidazole (260 mg, 3.8 mmol) and tert-butyldimethylsilyl chloride (450 mg, 3.0 mmol) were sequentially added thereto. The reaction mixture was reacted at room temperature for 12 hours under nitrogen atmosphere. TLC showed that the starting material was completely reacted. The reaction mixture was concentrated under reduced pressure and then purified by silica gel column chromatography (EA: PE = 1:10) to obtain compound **64-10a** (400 mg, Y: 76%). ¹H NMR (400 MHz, CDCl₃): δ 7.15 (s, 1H), 5.93 (br, 1H), 5.16 (d, *J* = 5.2, 1H), 3.01 (d, *J* = 5.2, 3H), 2.84-2.78 (m, 1H), 2.76-2.66 (m, 1H), 2.45-2.38 (m, 1H), 1.11(d, *J=* 6.8, 3H), 0.86 (s, 9H), 0.15 (s, 3H), 0.07 (s, 3H).

Step 11: Compound **64-10a** (270 mg, 0.65 mmol) and compound **1-8** (353 mg, 0.65 mmol) were dissolved in toluene (6 mL) and water (1.2 mL), and tris(dibenzylideneacetone)dipalladium (60 mg, 0.07 mmol), 4,5-bis(diphenylphosphino)-9,9-dimethylxanthene (75 mg, 0.13 mmol), and anhydrous potassium carbonate (270 mg, 1.96 mmol) were sequentially added thereto. The reaction mixture was reacted at 110°C overnight under nitrogen atmosphere. LCMS showed that the starting material was completely reacted. The reaction mixture was cooled to 25°C, concentrated under reduced pressure, and purified by silica gel column chromatography (EA: PE = 1:5 to 1:1, Rf = 0.6 and Rf = 0.5, where Rf = 0.5 was for target product) to obtain compound **64-11a** (100 mg, Y: 20%). LC-MS (1.5 mL-3.2 min-5 to 95% B): Rt = 2.450, m/z (ESI): 753.4/755.5 [M+H]⁺.

Step 12: Compound **64-11a** (100 mg, 0.13 mmol) was dissolved in hydrochloric acid/dioxane solution (2 mL, 4.0 M), and the reaction mixture was reacted at 25°C for 0.5 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% FA-CAN; gradient: 20 to 65 MeCN in H₂O; flow rate: 20 mL/min; run time: 12 min; retention time: Rt = 8.0 min/9.0 min) to obtain compounds **64a1** (41.7 mg, Y: 58%, Rt = 9.0 min) and **64a2** (9.9 mg, Y: 14%, Rt = 8.0 min).

**64a1:** ¹H NMR (400 MHz, DMSO-*d₆*): δ 8.22-8.17 (br, 2H), 7.44-7.25 (m, 6H), 7.02 (d, *J* = 9.6 Hz, 1H), 4.96 (d, *J* = 6.0 Hz, 1H), 3.66-3.59 (m, 1H), 3.49-3.44 (m, 2H), 2.99-2.67 (m, 4H), 2.62 (d, *J* = 4.8 Hz, 3H), 2.44-2.37 (m, 1H), 1.68-1.41 (m, 4H), 1.10 (d, *J* = 6.8 Hz, 3H). LC-MS m/z (ESI): 539.2/541.2 [M+H]⁺. Chiral analysis (column model: Celluiose-SB, 100 * 4.6 mm, 5 µm; eluent: hexane (0.1% DEA)-IPA; gradient: isocratic 9/1, flow rate: 1 mL/min; run time: 10.0 min; retention time: 5.75 min).

**64a2:** ¹H NMR (400 MHz, DMSO-*d₆*): δ 8.24 (br, 1H), 8.14-8.11 (br, 1H), 7.43-7.24 (m, 6H), 7.00 (d, *J* = 9.6 Hz, 1H), 5.15 (br, 1H), 4.96 (d, *J* = 5.6 Hz, 1H), 3.53-3.49 (m, 1H), 3.43-3.38 (m, 2H), 2.98-2.64 (m, 4H), 2.62 (d, *J* = 4.4 Hz, 3H), 2.43-2.37 (m, 1H), 1.59-1.35 (m, 4H), 1.10 (d, *J* = 6.8 Hz, 3H). LC-MS m/z (ESI): 539.3/541.3 [M+H]⁺. Chiral analysis (column model: Celluiose-SB, 100 * 4.6 mm, 5 µm; eluent: hexane (0.1% DEA)-IPA; gradient: isocratic 9/1, flow rate: 1 mL/min; run time: 8.0 min; retention time: 4.76 min).

Step 13: Compound **64-4b** (1.3 g, 5.3 mmol) was dissolved in anhydrous dichloromethane (15 mL). The reaction mixture was cooled to 0°C, sequentially added with imidazole (1.1 g, 16.2 mmol) and tert-butyldimethylsilyl chloride (1.6 g, 10.7 mmol), and reacted at room temperature for 5 hours under nitrogen atmosphere. TLC showed that the starting material was completely reacted. The reaction mixture was added with water (8 mL), and extracted with ethyl acetate (80 mL × 2). The organic phases were combined, washed with saturated brine (30 mL), dried over anhydrous sodium sulfate, and filtered. The filtrate was concentrated under reduced pressure and then purified by silica gel column chromatography (EA: PE = 1:40) to obtain compound **64-5b** (1.6 g, Y: 84%). ¹H NMR (400 MHz, CDCl₃): δ 7.14 (s, 1H), 7.02 (d, *J* = 9.6 Hz, 1H), 5.09 (d, *J* = 5.2 Hz, 1H), 2.85-2.70 (m, 2H), 2.42-2.34 (m, 1H), 1.12 (d, *J* = 7.2 Hz, 3H), 0.86 (s, 9H), 0.14 (s, 3H), 0.04 (s, 3H).

Step 14: Compound **64-5b** (1.6 g, 4.5 mmol) was dissolved in N,N-dimethylformamide (10 mL), and zinc cyanide (320 mg, 2.7 mmol), tris(dibenzylideneacetone)dipalladium (400 mg, 0.44 mmol), and 1,1'-bis(diphenylphosphino)ferrocene (500 mg, 0.90 mmol) were sequentially added thereto. The reaction mixture was reacted at 100°C for 16 hours under nitrogen atmosphere. TLC showed that the starting material was completely reacted. The reaction mixture was cooled to 25°C, added with ice water (100 mL), and extracted with ethyl acetate (150 mL × 3). The organic phases were combined, washed with saturated brine (80 mL), dried over anhydrous sodium sulfate, and filtered. The filtrate was concentrated under reduced pressure and then purified by silica gel column chromatography (EA: PE = 1:20) to obtain compound **64-6b** (900 mg, Y: 66%). ¹H NMR (400 MHz, CDCl₃): δ 7.29 (s, 1H), 7.16 (d, *J* = 8.4, 1H), 4.95 (d, *J* = 3.2, 1H), 3.34-3.28 (m, 1H), 2.48-2.38 (m, 2H), 1.08 (d, *J* = 6.8, 3H), 0.89 (s, 9H), 0.16 (s, 3H), 0.13 (s, 3H).

Step 15: Compound **64-6b** (900 mg, 2.9 mmol) was dissolved in tetrahydrofuran (10 mL). The reaction mixture was cooled to -70°C, added dropwise with a solution of lithium diisopropylamide in tetrahydrofuran (1.7 mL, 3.4 mmol, 2 M) under nitrogen atmosphere, reacted at -70°C for 1 hour, then added dropwise with 1,2-dibromotetrafluoroethane (850 mg, 3.3 mmol) in tetrahydrofuran (1 mL), and reacted at -70°C for 1 hour. TLC showed that the starting material was completely reacted. The reaction mixture was slowly added dropwise with saturated ammonium chloride solution (50 mL), and extracted with ethyl acetate (50 mL × 3). The organic phases were combined, washed with saturated brine (15 mL), dried over anhydrous sodium sulfate, and filtered. The filtrate was concentrated under reduced pressure and then purified by silica gel column chromatography (EA: PE = 1:20) to obtain compound **64-7b** (750 mg, Y: 66%). ¹H NMR (400 MHz, CDCl₃): δ 7.32 (s, 1H), 4.95 (d, *J* = 3.2, 1H), 3.31-3.23 (m, 1H), 2.50-2.39 (m, 2H), 1.08 (d, *J* = 7.2, 3H), 0.89 (s, 9H), 0.16 (s, 3H), 0.14 (s, 3H).

Step 16: Compound **64-7b** (750 mg, 1.9 mmol) was dissolved in ethanol (5 mL) and water (5 mL), then sodium hydroxide (780 mg, 19.5 mmol) was added thereto, and the reaction mixture was reacted at 100°C for 16 hours. TLC showed that the starting material was completely reacted. The reaction mixture was cooled to 0°C, added with water (30 mL), and then slowly added with dilute hydrochloric acid (0.5 M) until the pH of the reaction system was 3 to 4. The reaction mixture was extracted with ethyl acetate (80 mL × 3). The organic phases were combined, washed with saturated brine (30 mL), dried over anhydrous sodium sulfate, filtered, and the filtrate was concentrated under reduced pressure to obtain compound **64-8b** (480 mg, crude).

Step 17: Compound **64-8b** (480 mg, 1.7 mmol) was dissolved in N,N-dimethylformamide (5 mL), then diisopropylethylamine (790 mg, 6.1 mmol), 2-(7-azabenzotriazol-1-yl)-N,N,N',N'-tetramethyluronium hexafluorophosphate (650 mg, 1.7 mmol), and methylamine hydrochloride (190 mg, 2.8 mmol) were sequentially added thereto, and the reaction mixture was reacted at room temperature for 2 hours under nitrogen atmosphere. TLC showed that the starting material was completely reacted. The reaction mixture was added with water (30 mL), and extracted with ethyl acetate (30 mL × 2). The organic phases were combined, washed with saturated brine (20 mL), dried over anhydrous sodium sulfate, and filtered. The filtrate was concentrated under reduced pressure and then purified by silica gel column chromatography (EA: PE = 1:4) to obtain compound **64-9b** (450 mg, Y: 89%).

Step 18: Compound **64-9b** (450 mg, 1.7 mmol) was dissolved in dichloromethane (6 mL), and imidazole (260 mg, 3.8 mmol) and tert-butyldimethylsilyl chloride (450 mg, 3.0 mmol) were sequentially added thereto. The reaction mixture was reacted at room temperature for 12 hours under nitrogen atmosphere. TLC showed that the starting material was completely reacted. The reaction mixture was concentrated under reduced pressure and then purified by silica gel column chromatography (EA: PE = 1:10) to obtain compound **64-10b** (400 mg, Y: 64%). LC-MS m/z (ESI): 416.3/418.2 [M+H]⁺.

Step 19: Compound **64-10b** (270 mg, 0.65 mmol) and compound **1-8** (353 mg, 0.65 mmol) were dissolved in toluene (6 mL) and water (1.2 mL), and tris(dibenzylideneacetone)dipalladium (60 mg, 0.07 mmol), 4,5-bis(diphenylphosphino)-9,9-dimethylxanthene (75 mg, 0.13 mmol), and anhydrous potassium carbonate (270 mg, 1.96 mmol) were sequentially added thereto. The reaction mixture was reacted at 110°C overnight under nitrogen atmosphere. LCMS showed that the starting material was completely reacted. The reaction mixture was cooled to 25°C, concentrated under reduced pressure, and purified by silica gel column chromatography (EA: PE = 1:5 to 1:1, Rf = 0.5 and Rf = 0.4, where Rf = 0.4 was for target product) to obtain compound **64-11b** (90 mg, Y: 18%). LC-MS (1.5 mL-3.2 min-5 to 95% B): Rt = 2.080, m/z (ESI): 753.5/755.5 [M+H]⁺.

Step 20: Compound **64-11b** (90 mg, 0.12 mmol) was dissolved in hydrochloric acid/dioxane solution (2 mL, 4.0 M), and the reaction mixture was reacted at 25°C for 0.5 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% FA-CAN; gradient: 30 to 50 MeCN in H₂O; flow rate: 20 mL/min; run time: 12 min; retention time: 9.8 min) to obtain compound **64a3** (26.6 mg, Y: 38%). ¹H NMR (400 MHz, DMSO-*d₆*): δ 8.17 (br, 2H), 7.44-7.25 (m, 6H), 7.01 (d, *J* = 9.6 Hz, 1H), 5.48 (br, 1H), 4.78 (d, *J* = 5.6 Hz, 1H), 3.65-3.61 (m, 1H), 3.48-3.44 (m, 1H), 3.22-3.16 (m, 1H), 2.87-2.67 (m, 3H), 2.61 (d, *J* = 4.4 Hz, 3H), 2.46-2.44 (m, 1H), 2.33-2.26 (m, 1H), 1.68-1.42 (m, 4H), 1.10 (d, *J* = 6.8 Hz, 3H). LC-MS m/z (ESI): 539.3/541.2 [M+H]⁺. Chiral analysis (column model: Celluiose-SB, 100 * 4.6 mm, 5 µm; eluent: hexane (0.1% DEA)-IPA; gradient: isocratic 4/1, flow rate: 1 mL/min; run time: 7.0 min; retention time: 3.06 min).

### Example 65

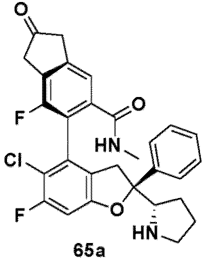

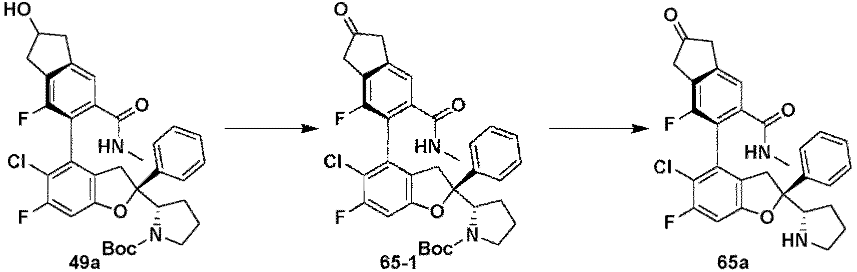

Step 1: Compound **49a** (60 mg, 0.10 mmol) was dissolved in anhydrous dichloromethane (2 mL), and Dess-Martin periodinane (120 mg, 0.28 mmol) was added thereto. The reaction mixture was reacted at room temperature for 12 hours under nitrogen atmosphere. TLC showed that the starting material was completely reacted. The reaction mixture was slowly added with saturated sodium sulfite solution (30 mL), stirred at room temperature for 5 minutes, then added with saturated sodium bisulfate solution (30 mL), and extracted with dichloromethane (20 mL × 2). The organic phases were combined, washed with saturated brine (20 mL), dried over anhydrous sodium sulfate, and filtered. The filtrate was concentrated under reduced pressure and then purified by silica gel column chromatography (EA: PE = 1:1) to obtain compound **65-1** (30 mg, Y: 50%).

Step 2: Compound **65-1** (30 mg, 0.05 mmol) was dissolved in hydrochloric acid/dioxane solution (0.5 mL, 4.0 M), and the reaction mixture was reacted at room temperature for 0.5 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.05% FA-MeCN; gradient: 21 to 41% MeCN; flow rate: 20 mL/min; run time: 12 min; retention time: 8.5 min) to obtain compound **65a** (5.2 mg, Y: 19%, FA salt). ¹H NMR (400 MHz, DMSO-*d₆*): δ 8.22-8.16 (br, m, 2H), 7.48-7.25 (m, 7H), 7.04 (d, *J* = 10.4 Hz, 1H), 3.74-3.55 (m, 5H), 3.20-3.15 (m, 1H), 2.89-2.73 (m, 3H), 2.63 (d, *J* = 4.4 Hz, 3H), 1.63-1.38 (m, 4H). LC-MS m/z (ESI): 523.2/525.2 [M+H]⁺. Chiral analysis (column model: Amylose-SA, 100 * 4.6 mm, 5 µm; eluent: hexane (0.1% DEA)-IPA; gradient: isocratic 9/1, flow rate: 1 mL/min; run time: 12 min; retention time: 6.56 min).

### Example 67

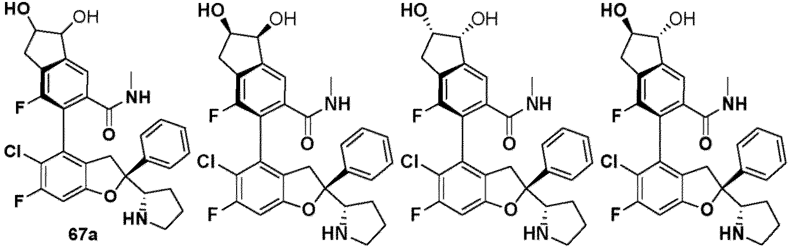

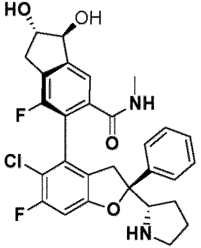

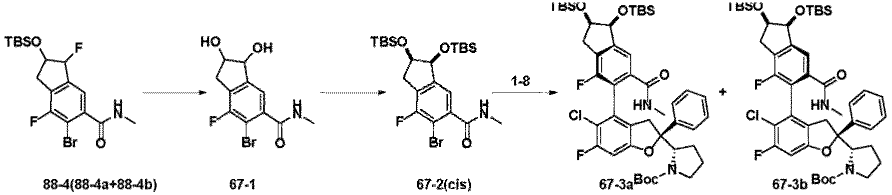

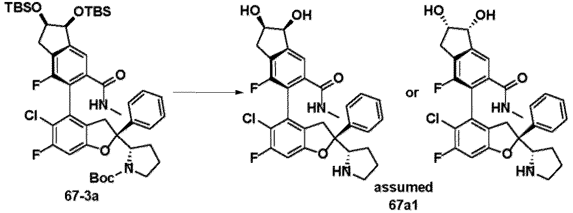

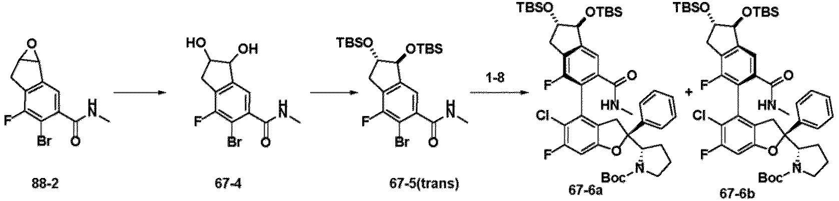

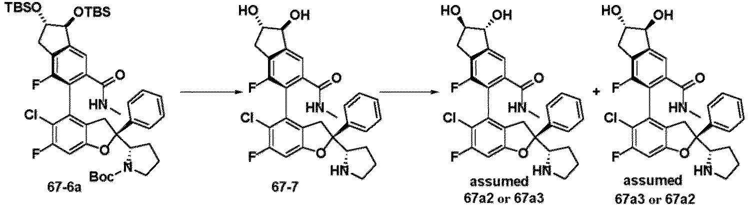

Step 1: Compound **88-4** (400 mg, 0.95 mmol) was dissolved in trifluoroacetic acid (2 mL), and the reaction mixture was reacted at 50°C for 16 hours under nitrogen atmosphere. TLC (PE: EA = 1:1) showed that the starting material was completely reacted. The reaction mixture was cooled to 25°C, poured into saturated sodium bicarbonate (20 mL), and extracted with ethyl acetate (30 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 1:1) to obtain compound **67-1** (100 mg, Y: 34%).

Step 2: Compound **67-1** (100 mg, 0.32 mmol) was dissolved in dichloromethane (2 mL), then compound tert-butyldimethylsilyl chloride (297 mg, 1.97 mmol) and imidazole (179 mg, 2.63 mmol) were sequentially added thereto under nitrogen atmosphere, and the reaction mixture was reacted at room temperature for 2 hours. TLC (DCM: MeOH = 10:1) showed that the starting material was completely reacted. The reaction mixture was poured into saturated ammonium chloride (50 mL), and extracted with ethyl acetate (30 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 5:1) to obtain compound **67-2** (80 mg, Y: 45%). ¹H NMR (400 MHz, DMSO-*d₆*) δ 8.46-8.32 (m, 1H), 7.02 (s, 1H), 4.98 (d, *J* = 4.4 Hz, 1H), 4.46-4.41 (m, 1H), 3.10-3.01 (m, 1H), 2.85-2.78 (m, 1H), 2.75 (d, *J* = 4.4 Hz, 3H), 0.93-0.80 (m, 18H), 0.16-0.06 (m, 12H). NOESY identified the compound as being in the cis configuration.

Step 3: Compound **67-2** (80 mg, 0.15 mmol) was dissolved in toluene (2 mL) and water (0.4 mL), then compound **1-8** (122 mg, 0.23 mmol), tris(dibenzylideneacetone)dipalladium (27.4 mg, 0.03 mmol), 4,5-bis(diphenylphosphino)-9,9-dimethylxanthene (31.1 mg, 0.06 mmol), and anhydrous potassium carbonate (95 mg, 0.45 mmol) were sequentially added thereto under nitrogen atmosphere, and the reaction mixture was reacted at 110°C overnight. TLC (PE: EA = 1:1) showed that the starting material was completely reacted. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 5:1 to 1:1) to obtain compounds **67-3a** (34 mg, Y: 26%, R_{f} = 0.30) and **67-3b** (40 mg, Y: 30%, R_{f} = 0.40). **67-3a:** LC-MS (D-1.5 mL-3 min-10 to 100% B): Rt = 1.850, m/z (ESI): 869.4 [M+H]⁺. **67-3b:** LC-MS (D-1.5 mL-3 min-10 to 100% B): Rt = 1.697, m/z (ESI): 869.4 [M+H]⁺.

Step 4: Compound **67-3a** (34 mg, 0.039 mmol) was dissolved in dichloromethane (1 mL). The reaction mixture was cooled to 0°C under nitrogen atmosphere, added with a solution of HCl in dioxane (4 M, 1 mL), warmed to room temperature, and stirred for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: Xselect CSH Prep Fluoro-Phenyl, 150 * 19 mm, 5 µm; eluent: 0.1% FA-ACN; gradient: 5% to 20%; flow rate: 20 mL/min; run time: 20 min; retention time: 9.0 min) to obtain compound **67a1** (4.6 mg, Y: 21%). ¹H NMR (400 MHz, DMSO-*d₆*) δ 8.22-8.13 (m, 2H), 7.46-7.24 (m, 6H), 7.01 (d, *J* = 10.0 Hz, 1H), 5.44-5.34 (m, 1H), 4.96-4.79 (m, 2H), 4.40-4.35 (m, 1H), 3.57-3.43 (m, 2H), 3.03-2.95 (m, 1H), 2.87-2.70 (m, 2H), 2.63 (d, *J* = 4.4 Hz, 3H), 1.63-1.34 (m, 4H). LC-MS m/z (ESI): 541.3 [M+H]⁺. Chiral analysis (column model: Celluiose-SB 100 * 4.6 mm, 3 µm; eluent: hexane (0.1% DEA)/EtOH; gradient: isocratic 8/2, flow rate: 1 mL/min; run time: 10 min; retention time: 3.32 min).

Step 5: Compound **88-2** (460 mg, 1.61 mmol) was dissolved in water (5.0 mL) and tetrahydrofuran (5.0 mL), and the reaction mixture was reacted at 50°C for 12 hours under nitrogen atmosphere. TLC (PE: EA = 5:1) showed that the starting material was completely reacted. The reaction mixture was cooled to 25°C, diluted with water (20 mL), and extracted with ethyl acetate (20 mL × 3). The organic phases were combined, washed with saturated brine (10 mL), dried over anhydrous sodium sulfate, filtered, and the filtrate was concentrated under reduced pressure to obtain compound **67-4** (480 mg, Y: 98%). LC-MS m/z (ESI): 304.0 [M+H]⁺.

Step 6: Compound **67-4** (480 mg, 1.58 mmol) was dissolved in dichloromethane (20 mL), then compound tert-butyldimethylsilyl chloride (1.43 g, 9.47 mmol) and imidazole (967 mg, 14.2 mmol) were sequentially added thereto under nitrogen atmosphere, and the reaction mixture was reacted at room temperature for 2 hours. TLC (DCM: MeOH = 10:1) showed that the starting material was completely reacted. The reaction mixture was poured into saturated ammonium chloride (50 mL), and extracted with ethyl acetate (30 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 5:1) to obtain compound **67-5** (460 mg, Y: 54%). ¹H NMR (400 MHz, DMSO-*d₆*) δ 8.41-8.34 (m, 1H), 6.96 (s, 1H), 4.93 (d, *J* = 5.6 Hz, 1H), 4.32-4.25 (m, 1H), 3.30-3.22 (m, 1H), 2.75 (d, *J* = 4.4 Hz, 3H), 2.69-2.62 (m, 1H), 0.93-0.88 (m, 18H), 0.18-0.09 (m, 12H). NOESY identified the compound as being in the trans configuration.

Step 7: Compound **67-5** (200 mg, 0.38 mmol) was dissolved in toluene (2 mL) and water (0.4 mL), then compound **1-8** (306 mg, 0.56 mmol), tris(dibenzylideneacetone)dipalladium (68.7 mg, 0.075 mmol), 4,5-bis(diphenylphosphino)-9,9-dimethylxanthene (77.8 mg, 0.15 mmol), and anhydrous potassium carbonate (238 mg, 1.13 mmol) were sequentially added thereto under nitrogen atmosphere, and the reaction mixture was reacted at 110°C overnight. TLC (PE: EA = 1:1) showed that the starting material was completely reacted. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 5:1 to 1:1) to obtain compounds **67-6a** (194 mg, Y: 29%, R_{f} = 0.30) and **67-6b** (400 mg, Y: 61%, R_{f} = 0.40). **67-6a:** LC-MS (D-1.5 mL-3 min-10 to 100% B): Rt = 1.900, m/z (ESI): 869.4 [M+H]⁺. **67-6b:** LC-MS (D-1.5 mL-3 min-10 to 100% B): Rt = 1.797, m/z (ESI): 869.4 [M+H]⁺.

Step 8: Compound **67-6a** (194 mg, 0.066 mmol) was dissolved in dichloromethane (1 mL). The reaction mixture was cooled to 0°C under nitrogen atmosphere, added with a solution of HCl in dioxane (4 M, 1 mL), warmed to 25°C, and stirred for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: YMC-Actus Triart C18, 150 * 20 mm, 5 µm; eluent: 0.05% NH₃·H₂O/CH₃CN; gradient: 34% to 54%; flow rate: 20 mL/min; run time: 20 min; retention time: 10.0 min) to obtain compound **67-7** (37.7 mg, Y: 31%). ¹H NMR (400 MHz, DMSO-*d₆*) δ 8.16-8.09 (m, 1H), 7.47-7.22 (m, 6H), 7.02-6.96 (m, 1H), 5.78-5.72 (m, 1H), 5.39-5.33 (m, 1H), 4.82-4.74 (m, 1H), 4.23-4.15 (m, 1H), 3.48-3.32 (m, 2H), 3.20-3.11 (m, 1H), 2.87-2.63 (m, 2H), 2.65-2.60 (m, 3H), 1.59-1.28 (m, 4H). LC-MS m/z (ESI): 540.8 [M+H]⁺. Chiral analysis (column model: Celluiose-SB 100 * 4.6 mm, 3 µm; eluent: hexane (0.1% DEA)/EtOH; gradient: isocratic 8/2, flow rate: 1 mL/min; run time: 6 min; retention time: 2.67 min and 3.14 min).

Step 9: Compound **67-7** was subjected to chiral resolution (chiral column model: Cellulose-SB 50 × 250 mm 10 µm, CO₂/(methanol+10 mM NH₃), 10%, 35°C, flow rate: 20 mL/min) to obtain compound **67a2** (14.0 mg, Y: 37%) and compound **67a3** (16.6 mg, Y: 44%). **67a2:** ¹H NMR (400 MHz, DMSO-*d*₆) δ 8.17-8.13 (m, 1H), 7.45-7.23 (m, 6H), 7.00 (d, *J* = 10.0 Hz, 1H), 5.77 (d, *J* = 5.6 Hz, 1H), 5.37 (d, *J* = 5.2 Hz, 1H), 4.84-4.78 (m, 1H), 4.23-4.16 (m, 1H), 3.54-3.39 (m, 2H), 3.21-3.13 (m, 1H), 2.88-2.69 (m, 2H), 2.62 (d, *J* = 4.4 Hz, 3H), 1.59-1.28 (m, 4H). LC-MS m/z (ESI): 541.2 [M+H]⁺. Chiral analysis (column model: Celluiose-SB 100 * 4.6 mm, 3 µm; eluent: hexane (0.1% DEA)/EtOH; gradient: isocratic 8/2, flow rate: 1 mL/min; run time: 6 min; retention time: 3.12 min). **67a3:** ¹H NMR (400 MHz, DMSO-*d*₆) δ 8.17-8.12 (m, 1H), 7.45-7.23 (m, 6H), 6.99 (d, *J* = 10.0 Hz, 1H), 5.73 (d, *J* = 6.0 Hz, 1H), 5.39 (d, *J* = 4.8 Hz, 1H), 4.81-4.76 (m, 1H), 4.23-4.17 (m, 1H), 3.54-3.39 (m, 2H), 3.21-3.13 (m, 1H), 2.88-2.69 (m, 2H), 2.62 (d, *J* = 4.8 Hz, 3H), 1.59-1.31 (m, 4H). LC-MS m/z (ESI): 541.2 [M+H]⁺. Chiral analysis (column model: Celluiose-SB 100 * 4.6 mm, 3 µm; eluent: hexane (0.1% DEA)/EtOH; gradient: isocratic 8/2, flow rate: 1 mL/min; run time: 6 min; retention time: 2.67 min).

### Example 68

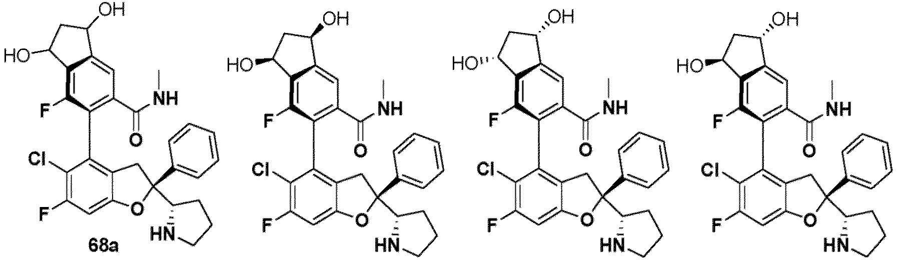

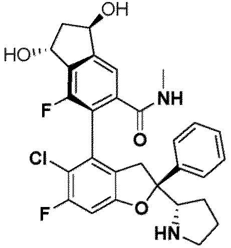

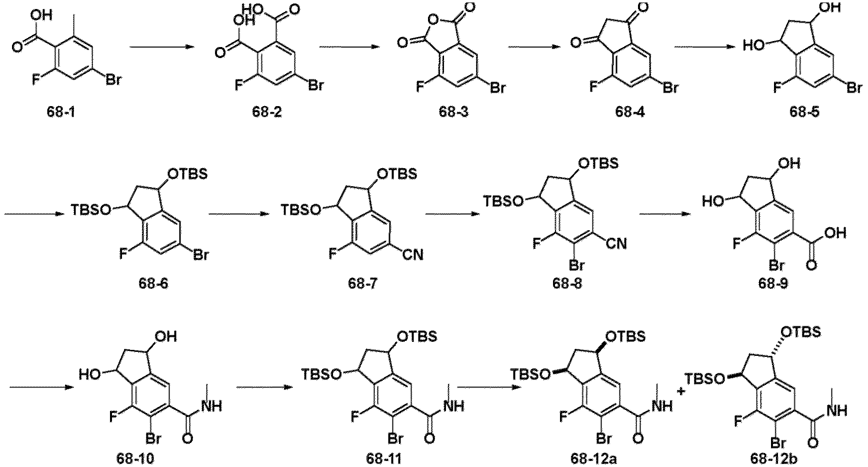

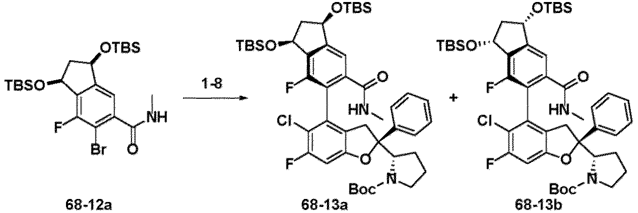

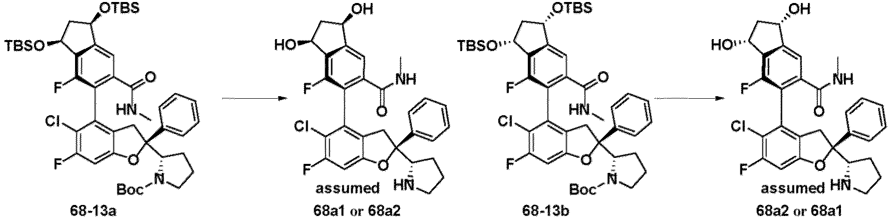

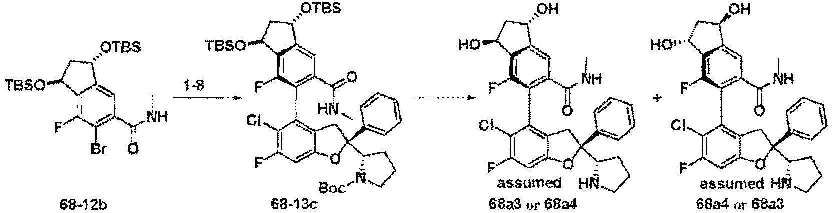

Step 1: Sodium hydroxide (51.5 g, 1.3 mol) was dissolved in water (800 mL). The reaction mixture was added with compound **68-1** (60.0 g, 0.26 mol), then added with potassium permanganate (122.0 g, 0.78 mol) in batches, and reacted at 100°C for 16 hours. TLC (DCM: MeOH = 10:1) showed that the starting material was completely reacted. The reaction mixture was cooled to 25°C, poured into saturated sodium bisulfite (2000 mL) to quench, added with hydrochloric acid (2 M, 1000 mL) to adjust the pH to 2 to 3, and extracted with ethyl acetate (500 mL × 3). The organic phases were combined, washed with saturated brine (500 mL), dried over anhydrous sodium sulfate, filtered, and the filtrate was concentrated under reduced pressure to obtain compound **68-2** (45.0 g, Y: 66%).

Step 2: Compound **68-2** (24.0 g, 91.2 mmol) was dissolved in thionyl chloride (200 mL), and the reaction mixture was reacted at 90°C for 16 hours. TLC (DCM: MeOH = 10:1) showed that the starting material was completely reacted. The reaction mixture was cooled to room temperature and concentrated under reduced pressure to obtain compound **68-3** (20.0 g, Y: 89%). ¹H NMR (400 MHz, CDCl₃) δ 7.99 (s, 1H), 7.78-7.72 (m, 1H).

Step 3: Compound **68-3** (20.0 g, 80.2 mmol) was dissolved in acetic anhydride (160 mL), then triethylamine (33.0 g, 320.7 mmol) and tert-butyl acetoacetate (25.8 g, 160.4 mmol) were added thereto, and the reaction mixture was reacted at 60°C for 16 hours. TLC (EA: PE = 3:1) showed that the starting material was completely reacted. The reaction mixture was slowly poured into dilute hydrochloric acid (100 mL, 2 M) to quench, and extracted with dichloromethane (200 mL × 3). The organic phases were combined, washed with saturated brine (50 mL), dried over anhydrous sodium sulfate, and filtered. The filtrate was concentrated to dryness under reduced pressure, added with concentrated hydrochloric acid (50 mL), stirred at 100°C for 15 minutes, and the reaction mixture was concentrated to dryness under reduced pressure to obtain compound **68-4** (13.2 g, Y: 66%). ¹H NMR (400 MHz, CDCl₃) δ 7.93 (s, 1H), 7.67-7.62 (m, 1H), 3.28 (s, 2H).

Step 4: Compound **68-4** (13.1 g, 53.9 mmol) was dissolved in tetrahydrofuran (130 mL). The reaction system was cooled to 0°C, added with sodium borohydride (12.3 g, 323.4 mmol) in batches, and reacted at 0°C for 4 hours. TLC (EA: PE = 1:1) showed that the starting material was completely reacted. The reaction mixture was slowly poured into ice-cold saturated ammonium chloride (200 mL) to quench, added with dilute hydrochloric acid (2 M) to adjust the pH to 5 to 6, and extracted with ethyl acetate (100 mL × 3). The organic phases were combined, washed with saturated brine (100 mL), dried over anhydrous sodium sulfate, filtered, and the filtrate was concentrated to dryness under reduced pressure to obtain compound **68-5** (10.4 g, Y: 78%).

Step 5: Compound **68-5** (10.0 g, 40.48 mmol) was dissolved in dichloromethane (100 mL), then imidazole (16.6 g, 242.91 mmol) and tert-butyldimethylsilyl chloride (24.3 g, 161.94 mmol) were sequentially added thereto, and the reaction mixture was reacted at room temperature for 2 hours under nitrogen atmosphere. TLC (EA: PE = 1:50) showed that the starting material was completely reacted. The reaction mixture was diluted with dichloromethane (100 mL), washed with water (50 mL × 2), washed with saturated brine (100 mL), dried over anhydrous sodium sulfate, and filtered. The filtrate was concentrated to dryness under reduced pressure and then purified by silica gel column chromatography (EA: PE = 1:50) to obtain compound **68-6** (13.2 g, Y: 68%).

Step 6: Compound **68-6** (13.2 g, 27.7 mmol) was dissolved in N,N-dimethylformamide (140 mL), then zinc cyanide (5.18 g, 44.3 mmol), tris(dibenzylideneacetone)dipalladium (2.5 g, 2.7 mmol), and 1,1'-bis(diphenylphosphino)ferrocene (3.0 g, 5.4 mmol) were sequentially added thereto, and the reaction mixture was reacted at 100°C for 16 hours under nitrogen atmosphere. TLC (PE: EA = 20:1) showed that the starting material was completely reacted. The reaction mixture was diluted with ethyl acetate (200 mL), washed with water (50 mL × 2), and washed with saturated brine (100 mL). The organic phase was dried over anhydrous sodium sulfate, filtered, and the filtrate was concentrated to dryness under reduced pressure and then purified by silica gel column chromatography (PE: THF = 20:1) to obtain compound **68-7** (7.0 g, Y: 60%). ¹H NMR (400 MHz, DMSO-*d₆*): δ 7.37 (s, 1H), 7.24-7.19 (m, 1H), 5.51- 4.91 (m, 2H), 2.92-1.95 (m, 2H), 0.98-0.89 (m, 18H), 0.21-0.11 (m, 12H).

Step 7: Compound **68-7** (7.0 g, 16.6 mmol) was dissolved in tetrahydrofuran (70 mL). The reaction mixture was cooled to -70°C under nitrogen atmosphere, added dropwise with lithium diisopropylamide (10 mL, 19.9 mmol), kept at -70°C and stirred for 1 hour, then added dropwise with dibromotetrachloroethane (4.3 g, 16.6 mmol), reacted at -70°C for 0.5 hours, naturally warmed to room temperature, and reacted for 1 hour. TLC (PE: EA = 20:1) showed that the starting material was completely reacted. The reaction mixture was cooled to 0°C, added dropwise with saturated ammonium chloride (100 mL) to quench, and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 50:1) to obtain compound **68-8** (5.0 g, Y: 60%). ¹H NMR (400 MHz, DMSO-*d₆*): δ 7.38 (s, 1H), 5.50-4.88 (m, 2H), 2.90-2.30 (m, 1H), 2.20-1.91 (m, 1H), 0.99-0.89 (m, 18H), 0.21-0.10 (m, 12H).

Step 8: Compound **68-8** (3.0 g, 5.98 mmol) was dissolved in ethanol (60 mL) and water (60 mL), then sodium hydroxide (2.4 g, 59.8 mmol) was added thereto, and the reaction mixture was reacted at 100°C for 16 hours. LCMS showed that the starting material was completely reacted. The reaction mixture was added with dilute hydrochloric acid (2 M) to adjust the pH to 2 to 3, and extracted with ethyl acetate (100 mL × 3). The organic phases were combined, washed with saturated brine (100 mL), dried over anhydrous sodium sulfate, filtered, and the filtrate was concentrated to dryness under reduced pressure to obtain compound **68-9** (1.5 g, crude). LC-MS: m/z (ESI): 288.9 [M-H]⁻.

Step 9: Compound **68-9** (1.5 g, 5.1 mmol) was dissolved in N,N-dimethylformamide (20 mL), then N,N-diisopropylethylamine (2.0 g, 15.5 mmol), methylamine hydrochloride (70.3 mg, 10.3 mmol), and 2-(7-azabenzotriazol-1-yl)-N,N,N',N'-tetramethyluronium hexafluorophosphate (2.4 g, 6.2 mmol) were sequentially added thereto, and the reaction mixture was reacted at room temperature for 2 hours. LCMS showed that the starting material was completely reacted. The reaction mixture was extracted with ethyl acetate (50 mL × 3). The organic phases were combined, washed with saturated brine (20 mL), dried over anhydrous sodium sulfate, filtered, and the filtrate was concentrated to dryness under reduced pressure to obtain compound **68-10** (1.5 g, crude).

Step 10: Compound **68-10** (1.5 g, 4.9 mmol) was dissolved in N,N-dimethylformamide (20 mL), then imidazole (2.1 g, 30.6 mmol) and tert-butyldimethylsilyl chloride (3.1 g, 20.7 mmol) were sequentially added thereto, and the reaction mixture was reacted at room temperature for 2 hours under nitrogen atmosphere. LCMS showed that the starting material was completely reacted. The reaction mixture was added with ethyl acetate (50 mL), and washed with water (20 mL) and saturated brine (20 mL). The organic phase was dried over anhydrous sodium sulfate, filtered, and the filtrate was concentrated under reduced pressure and then purified by silica gel column chromatography (PE: EA = 10:1) to obtain compound **68-11** (1.3 g, Y: 50%).

Step 11: Compound **68-11** (1.3 g, 2.4 mmol) was purified by preparative high-performance liquid chromatography (column model: YMC-Pack ODS-AQ, 150 * 20 mm, 5 µm; eluent: 0.05% NH₃H₂O-10 mmol/L NH₄HCO₃-ACN; gradient: 66% to 86%; flow rate: 100 mL/min: run time: 18 min, retention time: 14 min and 16 min) to obtain compound **68-12a** (501 mg, Y: 38%, retention time: 14 min) and compound **68-12b** (505 mg, Y: 38%, retention time: 16 min). **NOESY** identified compound **68-12a** as being in the cis configuration and compound **68-12b** as being in the trans configuration.

Step 12: Compound **68-12a** (500 mg, 0.94 mmol) and compound **1-8** (613 mg, 1.13 mmol) were dissolved in toluene (5 mL) and water (1 mL), then tris(dibenzylideneacetone)dipalladium (172 mg, 0.19 mmol), 4,5-bis(diphenylphosphino)-9,9-dimethylxanthene (217 mg, 0.38 mmol), and potassium phosphate (597 mg, 2.82 mmol) were sequentially added thereto, and the reaction mixture was reacted at 110°C overnight under nitrogen atmosphere. LCMS showed that the starting material was completely reacted. The reaction mixture was cooled to 25°C, concentrated under reduced pressure, and then purified by silica gel column chromatography (EA: PE = 1:3, Rf = 0.30, Rf = 0.35, Rf = 0.40, where Rf = 0.30 and Rf = 0.35 were for the target products) to obtain compound **68-13a** (120 mg, Y: 14%, Rf = 0.35) and compound **68-13b** (120 mg, Y: 14%, Rf = 0.30). **68-13a:** LC-MS (D-1.5 mL-3 min-10 to 100% B): Rt = 1.757, m/z (ESI): 869.4 [M+H]⁺. **68-13b:** LC-MS m/z (D-1.5 mL-3 min-10 to 100% B): Rt = 1.910, (ESI): 869.4 [M+H]⁺.

Step 13: Compound **68-13a** (100 mg, 0.12 mmol) was dissolved in a solution of hydrochloric acid in dioxane (3 mL, 4 mol/L), and the reaction mixture was reacted at room temperature for 2 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% HCl-ACN; gradient: 21 to 41; flow rate: 20 mL/min; run time: 12.0 min; retention time: 9.5 min) to obtain compound **68a1** (4.0 mg, Y: 6.4%). ¹H NMR (400 MHz, DMSO-*d₆*): δ 9.15 (br, 1H), 8.43-8.36 (m, 2H), 7.55-7.35 (m, 6H), 7.13 (d, *J* = 9.2 Hz, 1H), 5.79-5.49 (m, 2H), 5.09-5.01 (m, 1H), 4.88-4.80 (m, 1H), 4.31-4.20 (m, 1H), 3.55-3.46 (m, 1H), 3.21-3.01 (m, 2H), 2.93-2.78 (m, 2H), 2.63 (d, *J* = 4.8 Hz, 3H), 2.17-2.06 (m, 1H), 2.00-1.68 (m, 4H). LC-MS m/z (ESI): 541.3 [M+H]⁺. Chiral analysis (column model: Amylose-C, 100 * 4.6 mm, 3 µm; eluent: hexane/IPA; gradient: isocratic 85/15; flow rate: 1 mL/min; run time: 11 min; retention time: 4.10 min).

Step 14: Compound **68-13b** (100 mg, 0.12 mmol) was dissolved in a solution of hydrochloric acid in dioxane (4 mL, 4 mol/L), and the reaction mixture was reacted at room temperature for 2 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% FA-ACN; gradient: 5 to 16; flow rate: 20 mL/min; run time: 9.0 min; retention time: 8.0 min) to obtain compound **68a2** (14.6 mg,, Y: 23%). ¹H NMR (400 MHz, DMSO-*d₆*): δ 8.27-8.18 (m, 2H), 7.47-7.40 (m, 3H), 7.38-7.23 (m, 3H), 7.02 (d, *J* = 9.6 Hz, 1H), 5.13-5.07 (m, 1H), 4.93-4.87 (m, 1H), 3.67-3.42 (m, 2H), 2.89-2.72 (m, 2H), 2.70-2.60 (m, 5H), 1.79-1.70 (m, 1H), 1.68-1.35 (m, 4H). LC-MS m/z (ESI): 541.3 [M+H]⁺. Chiral analysis (column model: Amylose-C, 100 * 4.6 mm, 3 µm; eluent: hexane/IPA; gradient: isocratic 85/15; flow rate: 1 mL/min; run time: 11 min; retention time: 4.47 min).

Step 15: Compound **68-12b** (500 mg, 0.94 mmol) and compound **1-8** (613 mg, 1.13 mmol) were dissolved in toluene (5 mL) and water (1 mL), then tris(dibenzylideneacetone)dipalladium (172 mg, 0.19 mmol), 4,5-bis(diphenylphosphino)-9,9-dimethylxanthene (217 mg, 0.38 mmol), and potassium phosphate (597 mg, 2.82 mmol) were sequentially added thereto, and the reaction mixture was reacted at 110°C overnight under nitrogen atmosphere. LCMS showed that the starting material was completely reacted. The reaction mixture was cooled to 25°C, concentrated under reduced pressure, and purified by silica gel column chromatography (EA: PE = 1:3, Rf = 0.3 and 0.4, where Rf = 0.3 was for target product) to obtain compound **68-13c** (120 mg, Y: 14%). LC-MS (1.2 mL-5 min-5 to 90% B): Rt = 4.487, m/z (ESI): 869.3 [M+H]⁺.

Step 16: Compound **68-13c** (100 mg, 0.12 mmol) was dissolved in a solution of hydrochloric acid in dioxane (5 mL, 4 mol/L), and the reaction mixture was reacted at room temperature for 1 hour. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% FA-ACN; gradient: 30 to 50; flow rate: 20 mL/min; run time: 9.0 min; retention time: 7.1 min and 8.0 min) to obtain compound **68a3** (8.4 mg, Y: 13%, retention time: 7.1 min) and compound **68a4** (15.5 mg, Y: 24%, retention time: 8.0 min). **68a3:** ¹H NMR (400 MHz, DMSO-*d₆*): δ 8.29-8.15 (m, 2H), 7.57-7.21 (m, 6H), 7.01 (d, *J* = 9.6 Hz, 1H), 5.51-5.23 (m, 2H), 3.62-3.38 (m, 2H), 2.93-2.71 (m, 2H), 2.69-2.64 (m, 1H), 2.62 (d, *J* = 4.4 Hz, 3H), 2.31-2.23 (m, 1H), 2.11-2.02 (m, 1H), 1.78-1.30 (m, 4H)). LC-MS m/z (ESI): 541.3 [M+H]⁺. Chiral analysis (column model: Amylose-C, 100 * 4.6 mm, 3 µm; eluent: hexane/IPA; gradient: isocratic 85/15; flow rate: 1 mL/min; run time: 11 min; retention time: 5.59 min). **68a4:** ¹H NMR (400 MHz, DMSO-*d₆*): δ 8.30-8.10 (m, 2H), 7.52-7.21 (m, 6H), 7.02 (d, *J* = 9.2 Hz, 1H), 5.40-5.25 (m, 2H), 3.68-3.40 (m, 2H), 2.90-2.70 (m, 3H), 2.63 (d, *J* = 4.4 Hz, 3H), 2.32-2.27 (m, 1H), 2.11-2.03 (m, 1H), 1.70-1.38 (m, 4H). LC-MS m/z (ESI): 541.2 [M+H]⁺. Chiral analysis (column model: Amylose-C, 100 * 4.6 mm, 3 µm; eluent: hexane/IPA; gradient: isocratic 85/15; flow rate: 1 mL/min; run time: 11 min; retention time: 4.29 min).

### Example 69

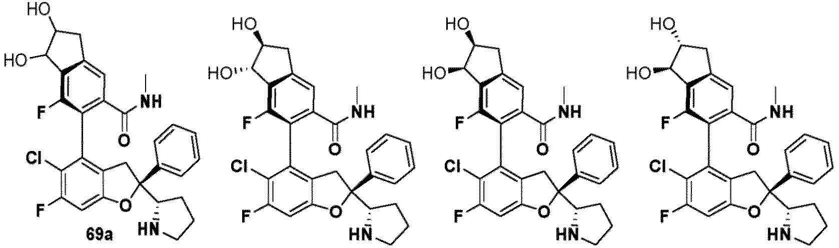

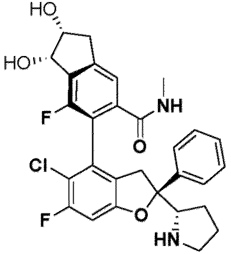

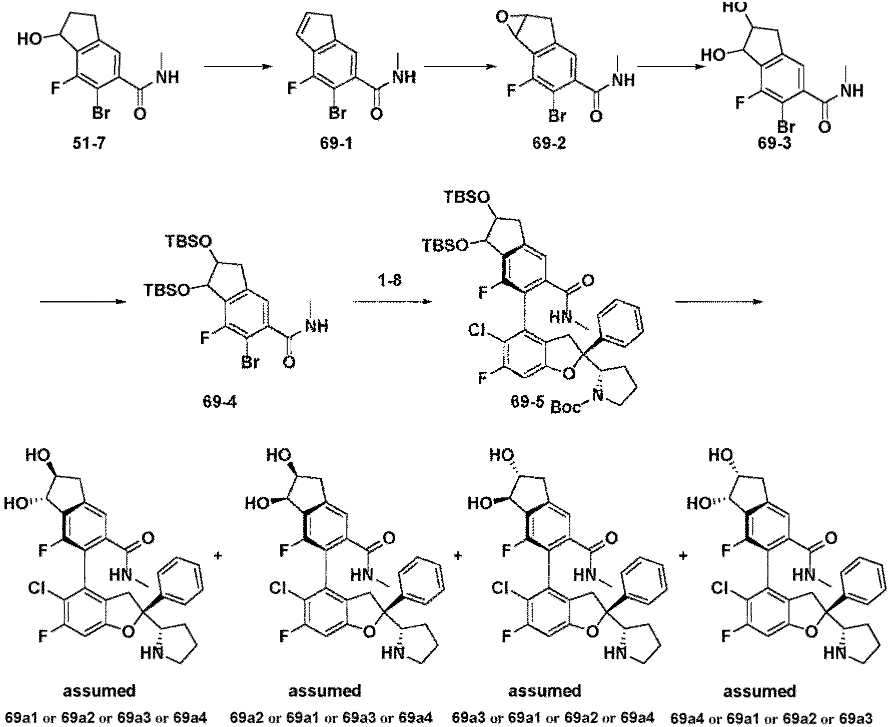

Step 1: Compound **51-7** (2.5 g, 8.68 mmol) was dissolved in toluene (30 mL), then p-toluenesulfonic acid (0.3 g, 1.74 mmol) was added thereto, and the reaction mixture was reacted at 90°C for 16 hours under nitrogen atmosphere. TLC (PE: EA = 10:1) showed that the starting material was completely reacted. The reaction mixture was cooled to 25°C, concentrated under reduced pressure, and purified by silica gel column chromatography (EA: PE = 1:15 to 1:8) to obtain compound **69-1** (1.5 g, Y: 64%). LC-MS m/z (ESI): 270.1/272.0 [M+H]⁺.

Step 2: Compound **69-1** (1.5 g, 5.56 mmol) was dissolved in anhydrous dichloromethane (20 mL), then 3-chloroperoxybenzoic acid (1.8 g, 80%) was added thereto, and the reaction mixture was reacted at 25°C for 16 hours under nitrogen atmosphere. TLC (PE: EA = 10:1) showed that the starting material was completely reacted. The reaction mixture was added with saturated sodium bicarbonate aqueous solution (90 mL), and extracted with ethyl acetate (80 mL × 2). The organic phases were combined, washed with saturated brine (50 mL × 2), dried over anhydrous sodium sulfate, filtered, and the filtrate was concentrated under reduced pressure to obtain compound **69-2** (0.5 g, crude) as a crude product. LC-MS m/z (ESI): 286.0/288.0 [M+H]⁺.

Step 3: Compound **69-2** (0.5 g, 1.74 mmol) was dissolved in tetrahydrofuran (9 mL) and water (3 mL), and the reaction mixture was reacted at 50°C for 12 hours under nitrogen atmosphere. TLC (PE: EA = 1:1) showed that the starting material was completely reacted. The reaction mixture was concentrated under reduced pressure to obtain crude compound **69-3** (300 mg, crude).

Step 4: Compound **69-3** (300 mg, 0.99 mmol) was dissolved in dichloromethane (8 mL). The reaction mixture was cooled to 0°C, sequentially added with imidazole (400 mg, 5.92 mmol) and tert-butyldimethylsilyl chloride (590 mg, 3.95 mmol), and reacted at room temperature for 2 hours under nitrogen atmosphere. TLC (PE: EA= 1:8 Rf = 0.5) showed that the starting material was completely reacted. The reaction mixture was concentrated under reduced pressure and then purified by silica gel column chromatography (EA: PE = 1:10) to obtain compound **69-4** (280 mg, Y: 53%). LC-MS m/z (ESI): 532.2/534.2 [M+H]⁺.

Step 5: Compound **69-3** (280 mg, 0.53 mmol) and compound **1-8** (340 mg, 0.63 mmol) were dissolved in toluene (6 mL) and water (1.2 mL), and tris(dibenzylideneacetone)dipalladium (64 mg, 0.07 mmol), 4,5-bis(diphenylphosphino)-9,9-dimethylxanthene (80 mg, 0.14 mmol), and anhydrous potassium carbonate (220 mg, 1.59 mmol) were sequentially added thereto. The reaction mixture was reacted at 110°C overnight under nitrogen atmosphere. LCMS showed that the starting material was completely reacted. The reaction mixture was cooled to 25°C, concentrated under reduced pressure, and purified by silica gel column chromatography (EA: PE = 1:8 to 1:1, Rf = 0.6, Rf = 0.5, where Rf = 0.5 was for target product) to obtain compound **69-4** (100 mg, Y: 22%). LC-MS (D-1.5 mL-3 min-10 to 100% B): Rt = 1.833, m/z (ESI): 869.4/871.5 [M+H]⁺.

Step 6: Compound **69-4** (100 mg, 0.11 mmol) was dissolved in dichloromethane (1 mL), then trifluoroacetic acid (4 mL) was added thereto, and the reaction mixture was reacted at 25°C for 2 hours. LCMS showed that the starting material was completely reacted. The reaction mixture was concentrated under reduced pressure, added with saturated sodium carbonate aqueous solution (6 mL), and extracted with ethyl acetate (15 mL × 2). The organic phase was dried over anhydrous sodium sulfate, filtered, and the filtrate was concentrated under reduced pressure and then purified by preparative high-performance liquid chromatography (column model: Xselect CSH Prep Fluoro-Phenyl, 150 * 19 mm, 5 µm; eluent: 0.1% FA-CAN; gradient: 10 to 60% MeCN in H₂O; flow rate: 20 mL/min; run time: 16 min; retention time: 7.2 min/8.1 min/8.6 min/10.0 min) to obtain compounds **69a1** (4.5 mg, Y: 7.1%, FA salt), **69a2** (5.2 mg, Y: 8.1%, FA salt), **69a3** (12.8 mg, Y: 19%, FA salt), and **69a4** (2.6 mg, Y: 4.2%, FA salt).

**69a1:** ¹H NMR (400 MHz, DMSO-*d₆*): δ 8.25-8.12 (m, 2H), 7.46-7.26 (m, 6H), 7.01 (d, *J* = 9.6 Hz, 1H), 5.70-5.40 (br, 1H), 4.87 (d, *J* = 2.4 Hz, 1H), 4.26-4.19 (m, 1H), 3.63-3.56 (m, 1H), 3.49-3.41 (m, 1H), 3.35-3.27 (m, 2H), 2.90-2.66 (m, 3H), 2.62 (d, *J* = 4.4 Hz, 3H), 1.66-1.35 (m, 4H). LC-MS m/z (ESI): 541.2/543.3 [M+H]⁺. Chiral analysis (column model: Celluiose-SC, 100 * 4.6 mm, 3 µm; eluent: hexane (0.1% DEA)-IPA; gradient: isocratic 4/1, flow rate: 1 mL/min; run time: 15.0 min; retention time: 6.65 min).

**69a2:** ¹H NMR (400 MHz, DMSO-*d₆*): δ 8.25-8.13 (br, 2H), 7.48-7.26 (m, 6H), 7.02 (d, *J* = 9.6 Hz, 1H), 4.93 (d, *J* = 4.8 Hz, 1H), 4.30-4.22 (m, 1H), 3.69-3.60 (m, 1H), 3.46-3.39 (m, 1H), 3.08-2.70 (m, 5H), 2.61 (d, *J* = 4.8 Hz, 3H), 1.70-1.40 (m, 4H). LC-MS m/z (ESI): 541.3/543.3 [M+H]⁺. Chiral analysis (column model: Celluiose-SC, 100 * 4.6 mm, 3 µm; eluent: hexane (0.1% DEA)-IPA; gradient: isocratic 4/1, flow rate: 1 mL/min; run time: 15.0 min; retention time: 11.46 min).

**69a3:** ¹H NMR (400 MHz, DMSO-*d₆*): δ 8.24-8.15 (br, 2H), 7.48-7.26 (m, 6H), 7.03 (d, *J* = 9.6 Hz, 1H), 5.60-5.40 (br, 1H), 4.86 (d, *J* = 2.0 Hz, 1H), 4.25-4.20 (m, 1H), 3.75-3.65 (m, 1H), 3.50-3.43 (m, 1H), 3.35-3.26 (m, 2H), 2.90-2.68 (m, 3H), 2.62 (d, *J* = 4.8 Hz, 3H), 1.75-1.42 (m, 4H). LC-MS m/z (ESI): 541.3/543.3 [M+H]⁺. Chiral analysis (column model: Celluiose-SC, 100 * 4.6 mm, 3 µm; eluent: hexane (0.1% DEA)-IPA; gradient: isocratic 4/1, flow rate: 1 mL/min; run time: 15.0 min; retention time: 5.68 min).

**69a4:** ¹H NMR (400 MHz, DMSO-*d₆*): δ 8.23 (br, 1H), 8.19-8.12 (br, 1H), 7.46-7.25 (m, 6H), 7.00 (d, *J* = 9.6 Hz, 1H), 4.94 (d, *J* = 5.2 Hz, 1H), 4.30-4.24 (m, 1H), 3.60-3.44 (m, 2H), 3.09-2.64 (m, 5H), 2.62 (d, *J* = 4.4 Hz, 3H), 1.65-1.38 (m, 4H). LC-MS m/z (ESI): 541.2/543.2 [M+H]⁺. Chiral analysis (column model: Celluiose-SC, 100 * 4.6 mm, 3 µm; eluent: hexane (0.1% DEA)-IPA; gradient: isocratic 4/1, flow rate: 1 mL/min; run time: 15.0 min; retention time: 6.71 min).

### Example 72

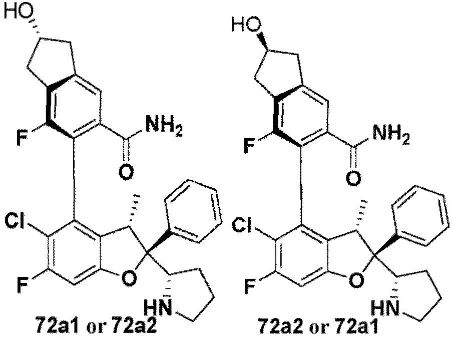

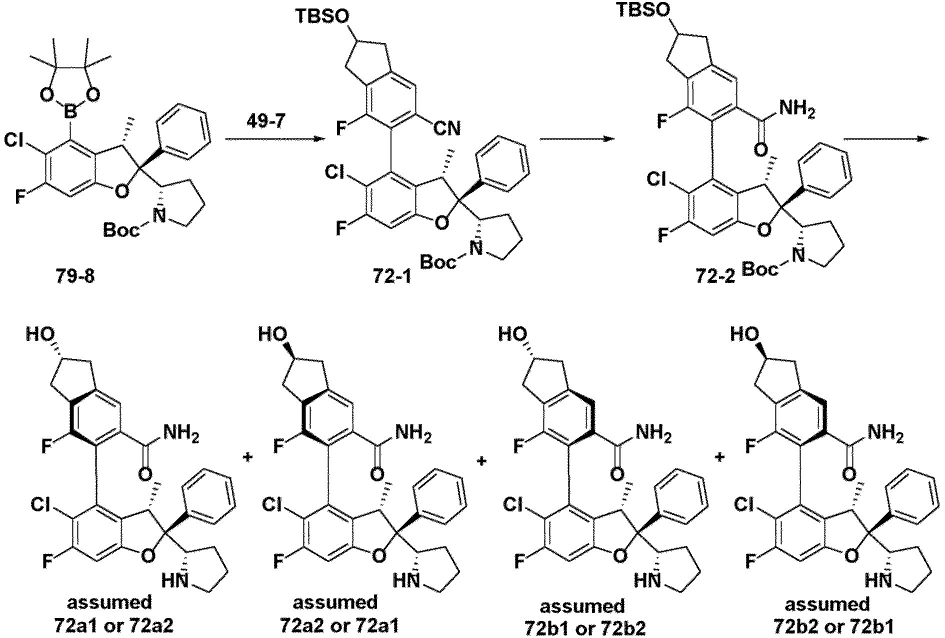

Step 1: Compound **49-7** (250 mg, 0.67 mmol) was dissolved in toluene (1 mL) and water (0.2 mL), then compound **79-8** (500 mg, 0.81 mmol), tris(dibenzylideneacetone)dipalladium (92.6 mg, 0.1 mmol), 4,5-bis(diphenylphosphino)-9,9-dimethylxanthene (104 mg, 0.2 mmol), and anhydrous potassium carbonate (429 mg, 2.03 mmol) were sequentially added thereto under nitrogen atmosphere, and the reaction mixture was reacted at 110°C overnight. TLC (PE: EA = 1:1) showed that the starting material was completely reacted. The reaction mixture was cooled to room temperature, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 5:1) to obtain compound **72-1** (240 mg, Y: 49%). LC-MS (D-1.5 mL-3 min-10 to 100% B): Rt = 1.603/1.637, m/z (ESI): 743.3 [M+Na]⁺.

Step 2: Compound **72-1** (240 mg, 0.33 mmol) was dissolved in dimethyl sulfoxide (4 mL), then anhydrous potassium carbonate (130 mg, 1.00 mmol) and 30% hydrogen peroxide (0.2 mL) were sequentially added thereto under nitrogen atmosphere, and the reaction mixture was stirred at room temperature for 2 hours. LCMS showed that the starting material was completely reacted. The reaction mixture was poured into saturated ammonium chloride (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compound **72-2** (192 mg, Y: 78%). LC-MS (D-1.5 mL-3 min-10 to 100% B): Rt = 1.443/1.483, m/z (ESI): 761.3 [M+Na]⁺.

Step 3: Compound **72-2** (192 mg, 0.26 mmol) was dissolved in dichloromethane (1 mL). The reaction mixture was cooled to 0°C under nitrogen atmosphere, added with a solution of HCl in dioxane (4 M, 1 mL), warmed to room temperature, and stirred for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: Xselect CSH Prep Fluoro-Phenyl, 150 * 19 mm, 5 µm; eluent: (0.1% FA) H₂O-ACN; gradient: 66% to 86%; flow rate: 20 mL/min; run time: 12 min; retention time: 6.5 min, 8.1 min, 9.1 min, and 10.3 min) to obtain compound **72a1** (22.0 mg, Y: 16%, retention time: 10.3 min) and compound **72a2** (15.1 mg, Y: 14%, retention time: 9.1 min). **72b1** (retention time: 8.1 min) and **72b2** (retention time: 6.5 min) were used as isomers without purification. **72a1:** ¹H NMR (400 MHz, DMSO-*d₆*) δ 8.94 (br, 1H), 8.23 (br, 1H), 7.86 (s, 1H), 7.61-7.19 (m, 7H), 7.09 (d, *J* = 9.2 Hz, 1H), 4.99 (br, 1H), 4.87-4.62 (m, 1H), 4.49-4.37 (m, 1H), 3.45-3.36 (m, 1H), 3.21-2.68 (m, 6H), 2.20-1.73 (m, 4H), 0.95 (d, *J* = 7.6 Hz, 3H). LC-MS m/z (ESI): 525.2 [M+H]⁺. Chiral analysis (column model: Celluiose-SB 100 * 4.6 mm 3 µm; eluent: hexane/IPA; gradient: isocratic 8/2, flow rate: 1 mL/min; run time: 10 min; retention time: 6.21 min). **72a2:** ¹H NMR (400 MHz, DMSO-*d*₆) δ 8.15 (s, 1H), 7.76 (s, 1H), 7.54-7.12 (m, 7H), 7.05 (d, *J* = 9.2 Hz, 1H), 5.09-4.95 (m, 1H), 4.62-4.52 (m, 1H), 4.00-3.84 (m, 1H), 3.25-3.04 (m, 4H), 2.87-2.66 (m, 3H), 1.94-1.05 (m, 4H), 0.99 (d, *J* = 7.2 Hz, 3H). LC-MS m/z (ESI): 525.2 [M+H]⁺. Chiral analysis (column model: Celluiose-SB 100 * 4.6 mm 3 µm; eluent: hexane/IPA; gradient: isocratic 8/2, flow rate: 1 mL/min; run time: 10 min; retention time: 4.71 min).

### Example 73

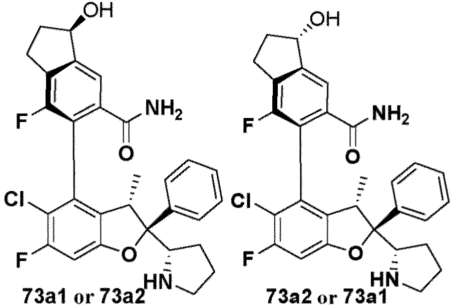

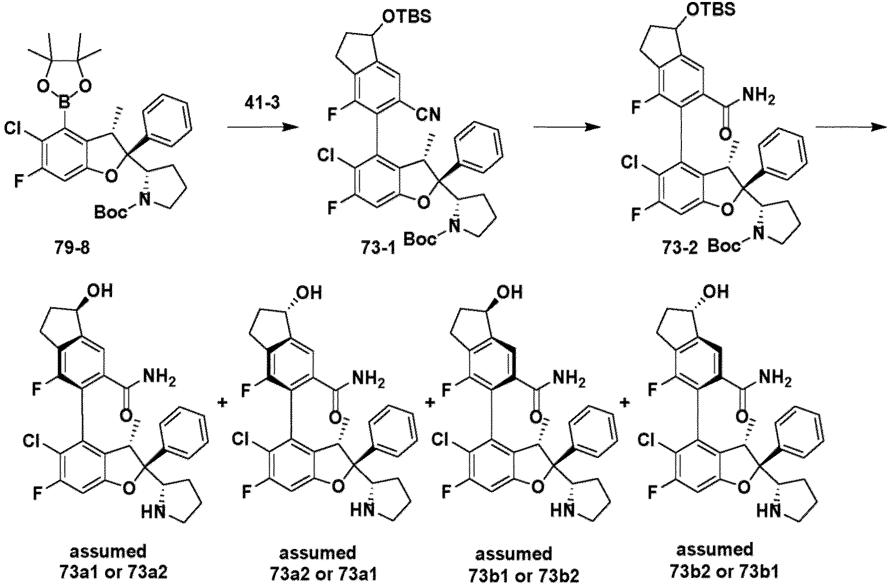

Step 1: Compound **41-3** (250 mg, 0.67 mmol) was dissolved in toluene (1 mL) and water (0.2 mL), then compound **79-8** (500 mg, 0.81 mmol), tris(dibenzylideneacetone)dipalladium (92.6 mg, 0.1 mmol), 4,5-bis(diphenylphosphino)-9,9-dimethylxanthene (104 mg, 0.2 mmol), and anhydrous potassium carbonate (429 mg, 2.03 mmol) were sequentially added thereto under nitrogen atmosphere, and the reaction mixture was reacted at 110°C overnight. TLC (PE: EA = 1:1) showed that the starting material was completely reacted. The reaction mixture was cooled to room temperature, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 5:1) to obtain compound **73-1** (110 mg, Y: 22%). LC-MS m/z (ESI): 743.3 [M+Na]⁺.

Step 3: Compound **73-1** (100 mg, 0.069 mmol) was dissolved in dimethyl sulfoxide (2 mL), then anhydrous potassium carbonate (10.8 mg, 0.13 mmol) and 30% hydrogen peroxide (0.1 mL) were sequentially added thereto under nitrogen atmosphere, and the reaction mixture was stirred at room temperature for 2 hours. LCMS showed that the starting material was completely reacted. The reaction mixture was poured into saturated ammonium chloride (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compound **73-2** (90 mg, Y: 87%). LC-MS m/z (D-1.5 mL-3 min-10 to 100% B): Rt = 1.373/1.410, (ESI): 761.3 [M+Na]⁺.

Step 4: Compound **73-2** (90 mg, 0.11 mmol) was dissolved in dichloromethane (1 mL). The reaction mixture was cooled to 0°C under nitrogen atmosphere, added with a solution of HCl in dioxane (4 M, 1 mL), warmed to room temperature, and stirred for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: Xselect CSH Prep Fluoro-Phenyl, 150 * 19 mm, 5 µm; eluent: (0.1% FA) H₂O-CN; gradient: 35% to 55%; flow rate: 20 mL/min; run time: 11 min; retention time: 9.2 min/8.4 min/7.6 min/6.8 min) to obtain compound **73a1** (15.3 mg, Y: 23%, retention time: 9.2 min), compound **73a2** (12.8 mg, Y: 19%, retention time: 8.4 min), compound **73b1** (4.7 mg, Y: 7.3%, retention time: 7.6 min), and compound **73b2** (5.9 mg, Y: 9.1%, retention time: 6.8 min). **73a1**: ¹HNMR (400 MHz, DMSO-*d*₆) δ 7.91 (br, 1H), 7.60-7.35 (m, 7H), 7.23 (br, 1H), 7.09 (d, *J* = 9.6 Hz, 1H), 5.58 (d, *J* = 6.0 Hz, 1H), 5.18-5.11 (m, 1H), 4.39 (br, 1H), 3.47-3.37 (m, 1H), 2.98-2.61 (m, 4H), 2.17-1.63 (m, 6H), 0.96 (d, *J* = 7.6 Hz, 3H). LC-MS m/z (ESI): 525.0 [M+H]⁺. Chiral analysis (column model: Amylose-SA 100 * 4.6 mm, 3 µm; eluent: hexane (0.1% DEA)/EtOH; gradient: isocratic 8/2, flow rate: 1 mL/min; run time: 8 min; retention time: 3.90 min). **73a2:** ¹H NMR (400 MHz, DMSO-*d*₆) δ 8.14 (s, 1H), 7.79 (br, 1H), 7.58-7.12 (m, 7H), 7.06 (d, *J* = 9.6 Hz, 1H), 5.57 (d, *J* = 6.0 Hz, 1H), 5.19-5.12 (m, 1H), 4.05-3.91 (m, 1H), 3.47-3.37 (m, 1H), 2.95-2.86 (m, 1H), 2.78-2.69 (m, 2H), 1.93-1.82 (m, 2H), 1.59-1.42 (m, 2H), 1.21-1.11 (m, 2H), 0.99 (d, *J* = 7.2 Hz, 3H). LC-MS m/z (ESI): 524.8 [M+H]⁺. Chiral analysis (column model: Amylose-SA 100 * 4.6 mm, 3 µm; eluent: hexane (0.1% DEA)/EtOH; gradient: isocratic 8/2, flow rate: 1 mL/min; run time: 8 min; retention time: 3.02 min). **73b1:** ¹H NMR (400 MHz, DMSO-*d*₆) δ 8.15 (s, 1H), 7.56 (br, 1H), 7.52-7.46, (m, 2H), 7.36 (s, 1H), 7.32-7.21 (m, 3H), 7.05 (d, *J* = 9.6 Hz, 1H), 6.50 (br, 1H), 5.63 (d, *J* = 5.6 Hz, 1H), 5.20-5.15 (m, 1H), 3.91-3.80 (m, 1H), 3.61-3.53 (m, 1H), 3.04-2.95 (m, 1H), 2.86-2.71 (m, 2H), 2.66-2.63 (m, 1H), 1.96-1.75 (m, 2H), 1.54-1.36 (m, 2H), 1.18-1.03 (m, 2H), 0.95 (d, *J* = 7.2 Hz, 3H). LC-MS m/z (ESI): 525.1 [M+H]⁺. Chiral analysis (column model: Amylose-SA 100 * 4.6 mm, 3 µm; eluent: hexane (0.1% DEA)/EtOH; gradient: isocratic 8/2, flow rate: 1 mL/min; run time: 8 min; retention time: 3.35 min). **73b2:** ¹H NMR (400 MHz, DMSO-*d*₆) δ 8.14 (s, 1H), 7.62-7.21 (m, 7H), 7.06 (d, *J* = 9.2 Hz, 1H), 6.58 (br, 1H), 5.55 (d, *J* = 6.0 Hz, 1H), 5.19-5.13 (m, 1H), 4.01-3.88 (m, 1H), 3.59-3.48 (m, 1H), 3.07-2.95 (m, 1H), 2.84-2.70 (m, 2H), 2.66-2.63 (m, 1H), 1.94-1.18 (m, 6H), 0.90 (d, *J* = 6.8 Hz, 3H). LC-MS m/z (ESI): 525.1 [M+H]⁺. Chiral analysis (column model: Amylose-SA 100 * 4.6 mm, 3 µm; eluent: hexane (0.1% DEA)/EtOH; gradient: isocratic 8/2, flow rate: 1 mL/min; run time: 8 min; retention time: 4.57 min).

### Example 74

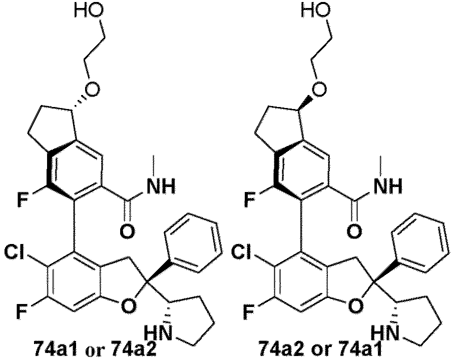

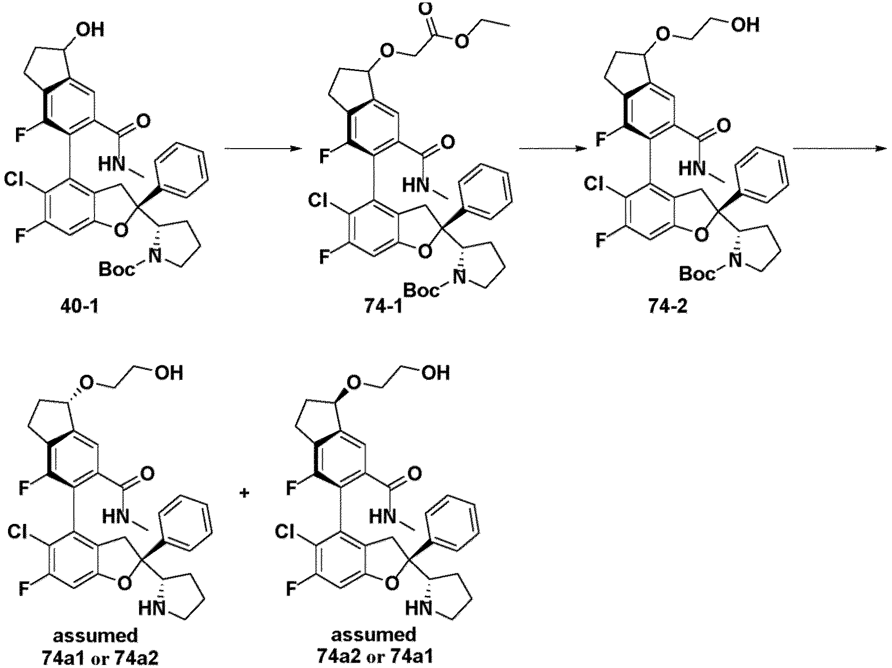

Step 1: Compound **40-1** (230 mg, 0.38 mmol) was dissolved in acetonitrile (5 mL), then ethyl bromoacetate (246 mg, 1.47 mmol) and cesium carbonate (480 mg, 1.47 mmol) were sequentially added thereto, and the reaction mixture was reacted at 80°C for 2 hours. TLC (PE: EA = 3:1) showed that the starting material was completely reacted. The reaction mixture was poured into water (50 mL) to quench, and extracted with ethyl acetate (20 mL × 3). The organic phases were combined, washed with saturated brine (30 mL), dried over anhydrous sodium sulfate, and filtered. The filtrate was concentrated under reduced pressure and then purified by silica gel column chromatography (PE: EA = 4:1) to obtain compound **74-1** (150 mg, Y: 57%) as a colorless oil. LC-MS m/z (ESI): 711.5 [M+H]⁺.

Step 2: Compound **74-1** (150 mg, 0.21 mmol) was dissolved in methanol (3 mL), then sodium borohydride (32 mg, 0.84 mmol) was slowly added thereto at 0°C, and the reaction mixture was warmed to room temperature and reacted for 2 hours. TLC (PE: EA = 1:1) showed that the starting material was completely reacted. The reaction mixture was poured into saturated ammonium chloride solution (30 mL), and extracted with ethyl acetate (30 mL × 3). The organic phases were combined, washed with saturated brine (30 mL), dried over anhydrous sodium sulfate, and filtered. The filtrate was concentrated under reduced pressure and then purified by silica gel column chromatography (PE: EA = 2:1) to obtain compound **74-2** (60 mg, Y: 42%). LC-MS m/z (ESI): 669.0 [M+H]⁺.

Step 3: Compound **74-2** (60 mg, 0.09 mmol) was dissolved in dichloromethane (1 mL), then trifluoroacetic acid (1 mL) was added thereto, and the reaction mixture was warmed to room temperature and stirred for 2 hours. LCMS showed that the starting material was completely reacted. The reaction mixture was added dropwise with sodium bicarbonate (50 mL) to quench, and extracted with ethyl acetate (20 mL × 3). The organic phases were combined, washed with saturated brine (50 mL), dried over anhydrous sodium sulfate, and filtered. The filtrate was concentrated under reduced pressure and then purified by preparative high-performance liquid chromatography (column model: Xselect CSH Prep Fluoro-Phenyl, 150 * 19 mm, 5 µm; eluent: 0.05% FA/CH₃CN = 1:1; gradient: 40% to 60%; flow rate: 20 mL/min; run time: 15 min) to obtain compound **74a1** (5.2 mg, Y: 10%, retention time: 8.0 min) and compound **74a2** (4.0 mg, Y: 7.8%, retention time: 8.7 min).

**74a1**: ¹H NMR (400 MHz, DMSO-*d*₆) δ 8.22-8.13 (m, 2H), 7.49 (s, 1H), 7.46-7.40 (m, 2H), 7.39-7.23 (m, 3H), 7.01 (d, *J* = 10.0 Hz, 1H), 5.05-5.00 (m, 1H), 4.63 (br, 1H), 3.66-3.41 (m, 6H), 3.06-2.92 (m, 1H), 2.90-2.70 (m, 5H), 2.67-2.62 (m, 3H), 2.09-2.00 (m, 1H), 1.66-1.37 (m, 4H). LC-MS m/z (ESI): 569.3 [M+H]⁺. Chiral analysis (column model: Amylose-SA 100 * 4.6 mm, 3 µm; eluent: hexane (0.1% DEA)/EtOH; gradient: isocratic 9/1, flow rate: 1 mL/min; run time: 10 min; retention time: 4.97 min).

**74a2:** ¹H NMR (400 MHz, DMSO-*d*₆) δ 8.22-8.13 (m, 2H), 7.48 (s, 1H), 7.46-7.41 (m, 2H), 7.38-7.22 (m, 3H), 7.01 (d, *J* = 10.0 Hz, 1H), 5.05-5.00 (m, 1H), 4.65 (br, 1H), 3.66-3.43 (m, 6H), 3.05-2.93 (m, 1H), 2.91-2.71 (m, 5H), 2.66-2.62 (m, 3H), 2.09-2.00 (m, 1H), 1.61-1.37 (m, 4H). LC-MS m/z (ESI): 569.3 [M+H]⁺. Chiral analysis (column model: Amylose-SA 100 * 4.6 mm, 3 µm; eluent: hexane (0.1% DEA)/EtOH; gradient: isocratic 9/1, flow rate: 1 mL/min; run time: 10 min; retention time: 5.22 min).

### Example 75

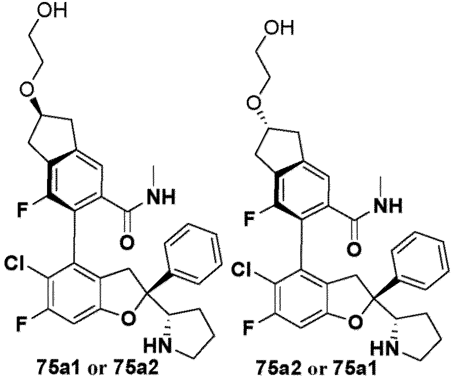

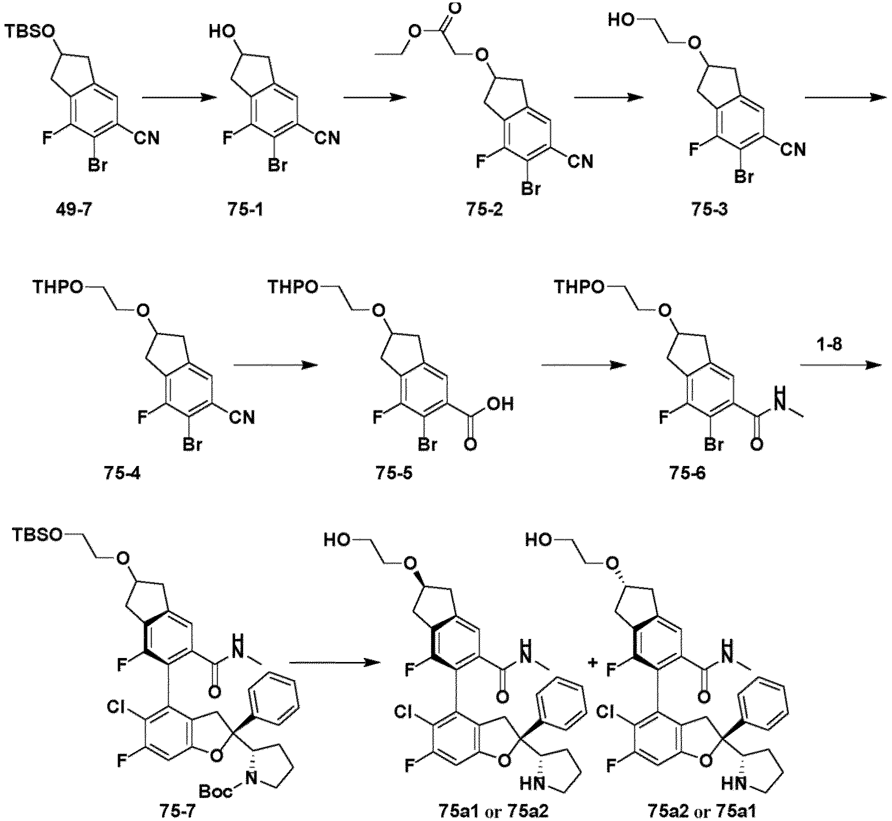

Step 1: Compound **49-7** (4.0 g, 10.8 mmol) was dissolved in anhydrous tetrahydrofuran (40 mL), then tetrabutylammonium fluoride/tetrahydrofuran solution (11 mL, 1.0 M) was slowly added thereto, and the reaction mixture was reacted at room temperature for 1 hour under nitrogen atmosphere. TLC (EA: PE = 1:5) showed that the starting material was completely reacted. The reaction mixture was added with water (70 mL), and extracted with ethyl acetate (50 mL × 3). The organic phases were combined, washed with saturated brine (30 mL × 2), dried over anhydrous sodium sulfate, and filtered. The filtrate was concentrated under reduced pressure and then purified by silica gel column chromatography (EA: PE = 1:5) to obtain compound **75-1** (2.0 g, Y: 72%) as a white solid.

Step 2: **75-1** (2.0 g, 7.8 mmol) was dissolved in anhydrous dichloromethane (30 mL). The reaction system was cooled to 0°C, sequentially added with sodium hydride (630 mg, 15.7 mmol, 60% wt) and ethyl bromoacetate (2.6 g, 15.6 mmol), and reacted at 25°C for 12 hours under nitrogen atmosphere. TLC (EA: PE = 1:7) showed that the starting material was completely reacted. The reaction system was cooled to 0°C, slowly added dropwise with water (80 mL) to quench, and extracted with ethyl acetate (100 mL × 2). The organic phases were combined, washed with saturated brine (50 mL × 2), dried over anhydrous sodium sulfate, and filtered. The filtrate was concentrated under reduced pressure and then purified by silica gel column chromatography (EA: PE = 1:7) to obtain compound **75-2** (1.2 g, Y: 45%). ¹H NMR (400 MHz, CDCl₃) δ 7.34 (s, 1H), 4.57-4.50 (m, 1H), 4.27-4.19 (m, 2H), 4.11 (s, 2H), 3.27-3.09 (m, 4H), 1.33-1.24 (m, 3H).

Step 3: Compound **75-2** (1.2 g, 3.5 mmol) was dissolved in methanol (6 mL) and tetrahydrofuran (6 mL). The reaction system was cooled to 0°C, and slowly added with sodium borohydride (0.6 g, 15.8 mmol). The reaction mixture was reacted at room temperature for 12 hours. TLC (EA: PE = 1:4) showed that the starting material was completely reacted. The reaction mixture was slowly added with ice water (100 mL), and extracted with ethyl acetate (100 mL × 2). The organic phases were combined, washed with saturated brine (60 mL), dried over anhydrous sodium sulfate, and filtered. The filtrate was concentrated under reduced pressure and then purified by silica gel column chromatography (EA: PE = 1:3) to obtain compound **75-3** (0.9 g, Y: 85%). ¹HNMR (400 MHz, CDCl₃) δ 7.34 (s, 1H), 4.49-4.41 (m, 1H), 3.78-3.68 (m, 3H), 3.64-3.58 (m, 2H), 3.29-3.02 (m, 4H).

Step 4: **75-3** (0.9 g, 3.0 mmol) was dissolved in dichloromethane (10 mL), then 3,4-dihydro-2H-pyran (0.75 g, 8.9 mmol) and p-toluenesulfonic acid (100 mg, 0.58 mmol) were sequentially added thereto, and the reaction mixture was reacted at room temperature for 12 hours under nitrogen atmosphere. TLC (EA: PE = 1:3) showed that the starting material was completely reacted. The reaction mixture was concentrated under reduced pressure and then purified by silica gel column chromatography (EA: PE = 1:7) to obtain compound **75-4** (810 mg, Y: 70%). ¹H NMR (400 MHz, CDCl₃) δ 7.33 (s, 1H), 4.62-4.53 (m, 1H), 4.50-4.42 (m, 1H), 3.90-3.80 (m, 2H), 3.78-3.63 (m, 3H), 3.61-3.52 (m, 1H), 3.23-3.02 (m, 4H), 1.62-1.45 (m, 6H).

Step 5: Compound **75-4** (1.0 g, 2.60 mmol) was dissolved in ethanol (3 mL) and water (8 mL), then sodium hydroxide (1.0 g, 25.0 mmol) was added thereto, and the reaction mixture was reacted at 90°C for 12 hours. TLC (EA: PE = 1:6) showed that the starting material was completely reacted. The reaction mixture was cooled to 0°C, diluted with water (50 mL), and then slowly added with dilute hydrochloric acid (0.5 M) to adjust the pH to 6. The reaction mixture was extracted with ethyl acetate (80 mL × 3). The organic phases were combined, washed with saturated brine (30 mL × 2), dried over anhydrous sodium sulfate, filtered, and the filtrate was concentrated under reduced pressure to obtain compound **75-5** (0.65 g, crude). LC-MS m/z (ESI): 401.1/403.1 [M-H]⁻.

Step 6: Compound **75-5** (0.65 g, 1.61 mmol) was dissolved in N,N-dimethylformamide (7 mL), then diisopropylethylamine (0.8 g, 6.20 mmol), 2-(7-azabenzotriazol-1-yl)-N,N,N',N'-tetramethyluronium hexafluorophosphate (0.9 g, 2.37 mmol), and methylamine hydrochloride (160 mg, 2.39 mmol) were sequentially added thereto, and the reaction mixture was reacted at room temperature for 2 hours under nitrogen atmosphere. TLC (DCM: MeOH = 10:1) showed that the starting material was completely reacted. The reaction mixture was added with water (50 mL), and extracted with ethyl acetate (40 mL × 3). The organic phases were combined, washed with saturated brine (20 mL × 2), dried over anhydrous sodium sulfate, and filtered. The filtrate was concentrated under reduced pressure and then purified by silica gel column chromatography (EA: PE = 1:2) to obtain compound **75-6** (300 mg, Y: 44%). ¹H NMR (400 MHz, CDCl₃) δ 7.17 (s, 1H), 5.95 (br, 1H), 4.63-4.58 (m, 1H), 4.48-4.41 (m, 1H), 3.90-3.80 (m, 2H), 3.68-3.63 (m, 2H), 3.61-3.53 (m, 1H), 3.51-3.45 (m, 1H), 3.24-2.96 (m, 7H), 1.75-1.45 (m, 6H).

Step 7: Compound **75-6** (300 mg, 0.72 mmol) and compound **1-8** (391 mg, 0.72 mmol) were dissolved in toluene (6 mL) and water (1.2 mL), and tris(dibenzylideneacetone)dipalladium (132 mg, 0.14 mmol), 4,5-bis(diphenylphosphino)-9,9-dimethylxanthene (166 mg, 0.29 mmol), and anhydrous potassium carbonate (300 mg, 2.34 mmol) were sequentially added thereto. The reaction mixture was reacted at 110°C overnight under nitrogen atmosphere. LCMS showed that the starting material was completely reacted. The reaction mixture was cooled to 25°C, concentrated under reduced pressure, and purified by silica gel column chromatography (EA: PE = 1:10 to 1:1, Rf = 0.6 and Rf = 0.5, where Rf = 0.5 was for target product) to obtain compound **75-7** (85 mg, Y: 15%). LC-MS (1.5 mL-3.2 min-5 to 95% B): Rt = 1.700, m/z (ESI): 753.4/755.5 [M+H]⁺.

Step 8: Compound **75-7** (80 mg, 0.11 mmol) was dissolved in hydrochloric acid/dioxane solution (2 mL, 4.0 M), and the reaction mixture was reacted at room temperature for 1 hour. LCMS showed that the starting material was completely reacted. The reaction mixture was concentrated under reduced pressure and then purified by preparative high-performance liquid chromatography (column model: Xselect CSH Prep Fluoro-Phenyl, 150 * 19 mm, 5 µm; eluent: 0.1% FA-ACN; gradient: 10 to 50% MeCN in H₂O; flow rate: 20 mL/min; run time: 12 min; retention time: 8.1 min/8.9 min) to obtain compound **75a1** (22.6 mg, Y: 32%, FA salt) and compound **75a2** (18.8 mg, Y: 27%, FA salt).

**75a1:** ¹H NMR (400 MHz, DMSO-*d₆*): δ 8.22 (br, 1H), 8.19-8.12 (m, 1H), 7.46-7.23 (m, 6H), 7.01 (d, *J* = 9.6 Hz, 1H), 4.46-4.37 (m, 1H), 3.61-3.40 (m, 7H), 3.28-2.64 (m, 7H), 2.61 (d, *J* = 4.4 Hz, 3H), 1.65-1.36 (m, 4H). LC-MS m/z (ESI): 569.3 [M+H]⁺. Chiral analysis (column model: Celluiose-SB, 100 * 4.6 mm, 5 µm; eluent: hexane (0.1% DEA)-IPA; gradient: isocratic 85/15, flow rate: 1 mL/min; run time: 12.0 min; retention time: 7.21 min).

**75a2:** ¹H NMR (400 MHz, DMSO-*d₆*): δ 8.21 (br, 1H), 8.19-8.14 (m, 1H), 7.48-7.24 (m, 6H), 7.01 (d, *J* = 9.6 Hz, 1H), 4.48-4.39 (m, 1H), 3.62-3.39 (m, 8H), 3.29-2.66 (m, 6H), 2.62 (d, *J* = 4.8 Hz, 3H), 1.67-1.39 (m, 4H). LC-MS m/z (ESI): 569.3 [M+H]⁺. Chiral analysis (column model: Celluiose-SB, 100 * 4.6 mm, 5 µm; eluent: hexane (0.1% DEA)-IPA; gradient: isocratic 85/15, flow rate: 1 mL/min; run time: 12.0 min; retention time: 8.17 min).

### Example 76

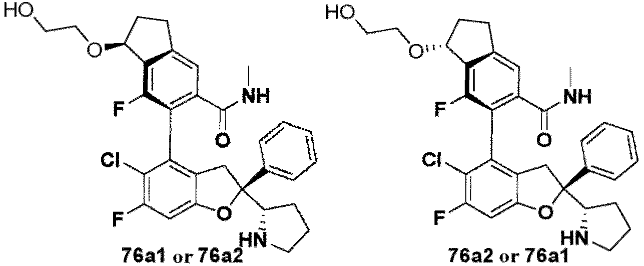

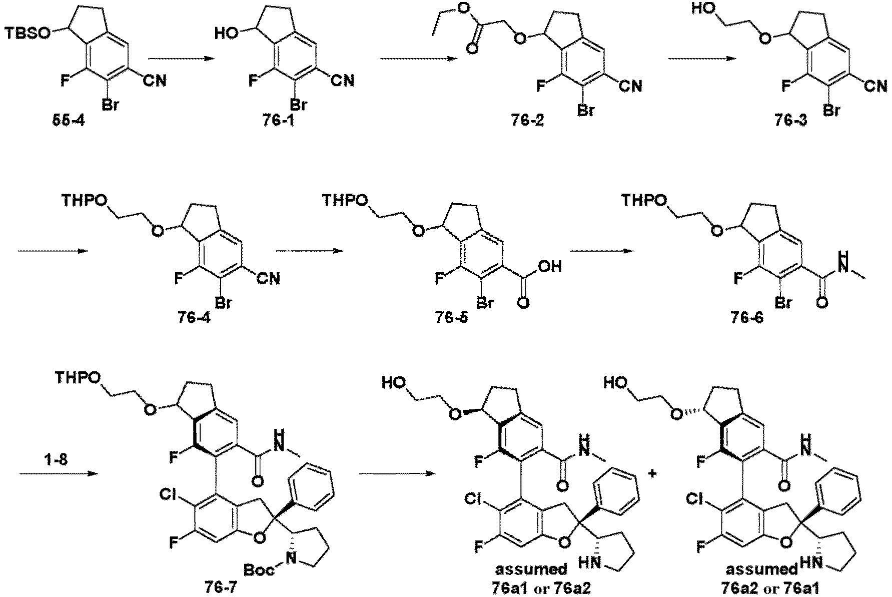

Step 1: Compound **55-4** (6.0 g, 16.2 mmol) was dissolved in anhydrous tetrahydrofuran (60 mL), then tetrabutylammonium fluoride/tetrahydrofuran solution (17.0 mL, 1.0 M) was slowly added thereto, and the reaction mixture was reacted at room temperature for 1 hour under nitrogen atmosphere. TLC (EA: PE = 1:4) showed that the starting material was completely reacted. The reaction mixture was added with water (100 mL), and extracted with ethyl acetate (50 mL × 3). The organic phases were combined, washed with saturated brine (60 mL × 2), dried over anhydrous sodium sulfate, and filtered. The filtrate was concentrated under reduced pressure and then purified by silica gel column chromatography (EA: PE = 1:4) to obtain compound **76-1** (3.1 g, Y: 74%).

Step 2: Compound **76-1** (3.1 g, 12.1 mmol) was dissolved in acetonitrile (40 mL), then cesium carbonate powder (11.8 g, 36.2 mmol) and ethyl bromoacetate (6.0 g, 35.9 mmol) were sequentially added thereto, and the reaction mixture was reacted at 80°C for 12 hours under nitrogen atmosphere. TLC (EA: PE = 1:5) showed that the starting material was completely reacted. The reaction system was cooled to 25°C, added with water (200 mL), and extracted with ethyl acetate (200 mL × 3). The organic phases were combined, washed with saturated brine (80 mL × 2), dried over anhydrous sodium sulfate, and filtered. The filtrate was concentrated under reduced pressure and then purified by silica gel column chromatography (EA: PE = 1:5) to obtain compound **76-2** (1.9 g, Y: 46%). ¹H NMR (400 MHz, CDCl₃) δ 7.39 (s, 1H), 5.23-5.20 (m, 1H), 4.24-4.18 (m, 4H), 3.26-3.15 (m, 1H), 2.90-2.81 (m, 1H), 2.41-2.34 (m, 2H), 1.32-1.24 (m, 3H).

Step 3: Compound **76-2** (2.0 g, 5.8 mmol) was dissolved in methanol (10 mL) and tetrahydrofuran (10 mL). The reaction system was cooled to 0°C, and slowly added with sodium borohydride (0.9 g, 23.7 mmol) in batches. The reaction mixture was reacted at room temperature for 12 hours. TLC (EA: PE = 1:5) showed that the starting material was completely reacted. The reaction mixture was slowly added with ice water (100 mL), and extracted with ethyl acetate (100 mL × 2). The organic phases were combined, washed with saturated brine (60 mL), dried over anhydrous sodium sulfate, filtered, and the filtrate was concentrated under reduced pressure to obtain compound **76-3** (1.5 g, crude).

Step 4: Compound **76-3** (1.5 g, 5.0 mmol) was dissolved in dichloromethane (18 mL), then 3,4-dihydro-2H-pyran (1.2 g, 14.3 mmol) and p-toluenesulfonic acid (170 mg, 0.1 mmol) were sequentially added thereto, and the reaction mixture was reacted at room temperature for 12 hours under nitrogen atmosphere. TLC (EA: PE = 1:3) showed that the starting material was completely reacted. The reaction mixture was concentrated under reduced pressure and then purified by silica gel column chromatography (EA: PE = 1:5) to obtain compound **76-4** (1.6 g, Y: 83%).

Step 5: Compound **76-4** (1.6 g, 4.17 mmol) was dissolved in ethanol (5 mL) and water (15 mL), then sodium hydroxide (1.6 g, 40.0 mmol) was added thereto, and the reaction mixture was reacted at 90°C for 12 hours. TLC (EA: PE = 1:5) showed that the starting material was completely reacted. The reaction mixture was cooled to 0°C, added with water (50 mL), and then slowly added with dilute hydrochloric acid (0.5 M) until the pH of the reaction system was 6. The reaction mixture was extracted with ethyl acetate (80 mL × 3). The organic phases were combined, washed with saturated brine (30 mL × 2), dried over anhydrous sodium sulfate, filtered, and the filtrate was concentrated under reduced pressure to obtain compound **76-5** (1.4 g, crude). LC-MS m/z (ESI): 401.1/403.1 [M-H]⁻.

Step 6: Compound **76-5** (1.4 g, 3.47 mmol) was dissolved in N,N-dimethylformamide (20 mL), then diisopropylethylamine (2.2 g, 17.05 mmol), 2-(7-azabenzotriazol-1-yl)-N,N,N',N'-tetramethyluronium hexafluorophosphate (2.0 g, 5.26 mmol), and methylamine hydrochloride (460 mg, 6.86 mmol) were sequentially added thereto, and the reaction mixture was reacted at room temperature for 2 hours under nitrogen atmosphere. LCMS showed that the starting material was completely reacted. The reaction mixture was added with water (60 mL), and extracted with ethyl acetate (40 mL × 3). The organic phases were combined, washed with saturated brine (20 mL × 2), dried over anhydrous sodium sulfate, and filtered. The filtrate was concentrated under reduced pressure and then purified by silica gel column chromatography (EA: PE = 1:4) to obtain compound **76-6** (900 mg, Y: 62%). LC-MS m/z (ESI): 438.2/440.0 [M+Na]⁺.

Step 7: Compound **76-6** (410 mg, 0.99 mmol) and compound **1-8** (535 mg, 0.99 mmol) were dissolved in toluene (8 mL) and water (1.6 mL), and tris(dibenzylideneacetone)dipalladium (160 mg, 0.17 mmol), 4,5-bis(diphenylphosphino)-9,9-dimethylxanthene (220 mg, 0.38 mmol), and anhydrous potassium carbonate (410 mg, 2.97 mmol) were sequentially added thereto. The reaction mixture was reacted at 110°C overnight under nitrogen atmosphere. LCMS showed that the starting material was completely reacted. The reaction mixture was cooled to 25°C, concentrated under reduced pressure, and purified by silica gel column chromatography (EA: PE = 1:10 to 1:1, Rf = 0.6 and Rf = 0.55, where Rf = 0.55 was for target product) to obtain compound **76-7** (80 mg, Y: 10%). LC-MS (1.5 mL-3.2 min-5 to 95% B): Rt = 1.777, m/z (ESI): 753.4/755.5 [M+H]⁺.

Step 8: Compound **76-7** (80 mg, 0.11 mmol) was dissolved in hydrochloric acid/dioxane solution (2 mL, 4.0 M), and the reaction mixture was reacted at room temperature for 1 hour. LCMS showed that the starting material was completely reacted. The reaction mixture was concentrated under reduced pressure and then purified by preparative high-performance liquid chromatography (column model: Xselect CSH Prep Fluoro-Phenyl, 150 * 19 mm, 5 µm; eluent: 0.1% FA-ACN; gradient: 10 to 50% MeCN in H₂O; flow rate: 20 mL/min; run time: 12 min; retention time: 8.7 min/9.2 min) to obtain compounds **76a1** (9.6 mg, Y: 13.1%, FA salt, retention time: 8.7 min) and **76a2** (12.3 mg, Y: 18.7%, FA salt, retention time: 9.2 min).

**76a1:** ¹H NMR (400 MHz, DMSO-*d₆*): δ 8.32 (br, 1H), 8.22-8.16 (m, 1H), 7.45-7.23 (m, 6H), 7.01 (d, *J* = 9.6 Hz, 1H), 5.12-5.07 (m, 1H), 3.57-3.30 (m, 9H), 3.19-2.64 (m, 4H), 2.62 (d, *J* = 4.8 Hz, 3H), 2.33-2.11 (m, 2H), 1.60-1.21 (m, 4H). LC-MSm/z(ESI): 569.3/571.3 [M+H]⁺. Chiral analysis (column model: Celluiose-SC, 100 * 4.6 mm, 3 µm; eluent: hexane (0.1% DEA)-IPA; gradient: isocratic 4/1, flow rate: 1 mL/min; run time: 15.0 min; retention time: 9.45 min).

**76a2:** ¹H NMR (400 MHz, DMSO-*d₆*): δ 8.26-8.18 (m, 2H), 7.47-7.23 (m, 6H), 7.00 (d, *J* = 10.0 Hz, 1H), 5.12-5.07 (m, 1H), 3.58-3.28 (m, 8H), 3.19-2.82 (m, 4H), 2.80-2.70 (m, 1H), 2.63 (d, *J* = 4.4 Hz, 3H), 2.36-2.10 (m, 2H), 1.60-1.32 (m, 4H). LC-MS m/z (ESI): 569.2/571.3 [M+H]⁺. Chiral analysis (column model: Celluiose-SC, 100 * 4.6 mm, 3 µm; eluent: hexane (0.1% DEA)-IPA; gradient: isocratic 4/1, flow rate: 1 mL/min; run time: 9.0 min; retention time: 6.63 min).

### Example 77

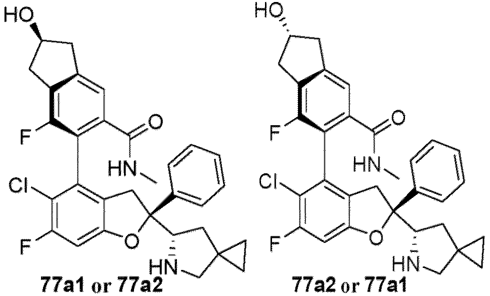

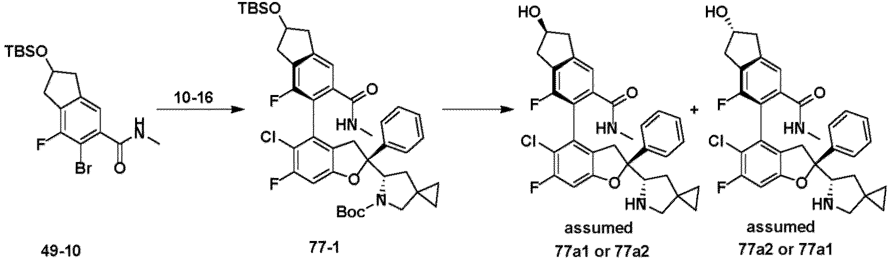

Step 1: Compound **49-10** (600 mg, 1.49 mmol) and compound **10-16** (570 mg, 1.49 mmol) were dissolved in toluene (12 mL) and water (2.4 mL), and tris(dibenzylideneacetone)dipalladium (205 mg, 0.22 mmol), 4,5-bis(diphenylphosphino)-9,9-dimethylxanthene (260 mg, 0.45 mmol), and anhydrous potassium carbonate (620 mg, 4.48 mmol) were sequentially added thereto. The reaction mixture was reacted at 110°C overnight under nitrogen atmosphere. LCMS showed that the starting material was completely reacted. The reaction mixture was cooled to 25°C, concentrated under reduced pressure, and purified by silica gel column chromatography (EA: PE = 1:10 to 1:1, PE: EA = 5:1, Rf = 0.6, Rf = 0.5, where Rf = 0.5 was for target product) to obtain compound **77-1** (250 mg, Y: 22%) as a solid. LC-MS (1.5 mL-3.2 min-5 to 95% B): Rt = 2.183/2.223, m/z (ESI): 765.5/767.4 [M+H]⁺.

Step 2: Compound **77-1** (250 mg, 0.33 mmol) was dissolved in dichloromethane (3 mL), then trifluoroacetic acid (3 mL) was added thereto, and the reaction mixture was reacted at room temperature for 2 hours. LCMS showed that the starting material was completely reacted. The reaction mixture was concentrated under reduced pressure at 25°C, slowly added with saturated sodium carbonate aqueous solution (3 mL), and extracted with ethyl acetate (10 mL × 2). The organic phases were combined, washed with saturated brine (5 mL), dried over anhydrous sodium sulfate, and filtered. The filtrate was concentrated under reduced pressure and then purified by preparative high-performance liquid chromatography (column model: Xbridge Prep Phenyl OBD, 150 * 19 mm, 5 µm; eluent: 0.1% FA-ACN; gradient: 55 to 75%; flow rate: 20 mL/min; run time: 12 min; retention time: 8.6 min/9.6 min) to obtain compounds **77a1** (32.3 mg, Y: 17%, Rt = 8.6 min) and **77a2** (50.9 mg, Y: 27.6%, Rt = 9.6 min) as white solids.

**77a1:** ¹H NMR (400 MHz, DMSO-*d₆*): δ 8.22-8.14 (m, 1H), 7.54-7.47 (m, 2H), 7.43-7.29 (m, 4H), 7.07 (d, *J* = 9.2 Hz, 1H), 5.04 (d, *J* = 3.6 Hz, 1H), 4.64-4.58 (m, 1H), 4.18-4.05 (m, 1H), 3.52-3.45 (m, 1H), 3.21-3.06 (m, 2H), 2.92-2.71 (m, 5H), 2.60 (d, *J* = 4.8 Hz, 3H), 1.80-1.61 (m, 2H), 0.59-0.40 (m, 4H). LC-MS m/z (ESI): 551.1/553.2 [M+H]⁺. Chiral analysis (column model: Amylose-SA, 100 * 4.6 mm, 3 µm; eluent: hexane (0.1% DEA)-IPA; gradient: isocratic 85/15, flow rate: 1 mL/min; run time: 12.0 min; retention time: 3.21 min).

**77a2:** ¹H NMR (400 MHz, DMSO-*d₆*): ¹H NMR (400 MHz, DMSO-*d₆*): δ 8.24-8.12 (m, 1H), 7.58-7.45 (m, 2H), 7.43-7.29 (m, 4H), 7.07 (d, *J* = 9.6 Hz, 1H), 5.10-5.00 (m, 1H), 4.68-4.58 (m, 1H), 4.22-4.10 (m, 1H), 3.55-3.48 (m, 1H), 3.22-3.07 (m, 2H), 2.96-2.72 (m, 5H), 2.59 (d, *J* = 4.4 Hz, 3H), 1.82-1.63 (m, 2H), 0.61-0.40 (m, 4H). LC-MS m/z (ESI): 551.1/553.2 [M+H]⁺. Chiral analysis (column model: Amylose-SA, 100 * 4.6 mm, 3 µm; eluent: hexane (0.1% DEA)-IPA; gradient: isocratic 85/15, flow rate: 1 mL/min; run time: 12.0 min; retention time: 7.29 min).

### Example 78

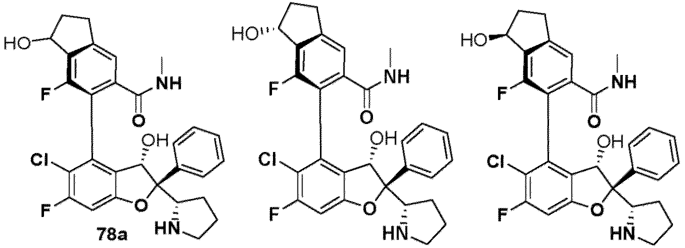

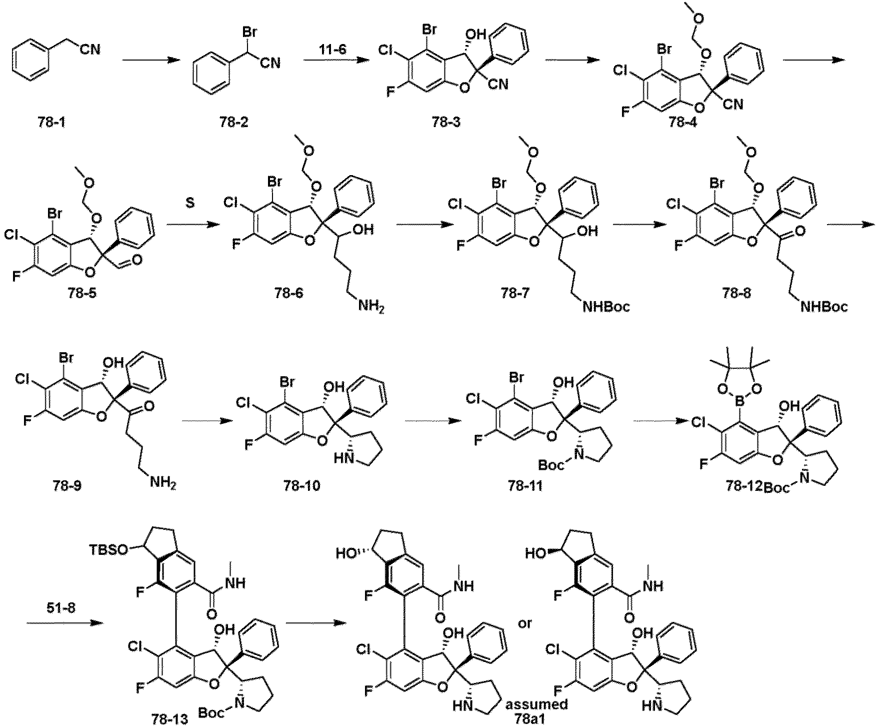

Step 1: Compound **78-1** (50.0 g, 426.8 mmol) was dissolved in carbon tetrachloride (500 mL), then N-bromosuccinimide (98.76 g, 454.7 mmol) and benzoyl peroxide (5.16 g, 21.3 mmol) were sequentially added thereto under nitrogen atmosphere, and the reaction mixture was gradually heated to 80°C and reacted for 16 hours. TLC (PE: EA = 10:1) showed that the starting material was completely reacted. The reaction mixture was poured into ice water (500 mL) to quench, and extracted with ethyl acetate (200 mL × 3). The organic phases were combined, washed with saturated brine (200 mL × 3), dried over anhydrous sodium sulfate, and filtered. The filtrate was concentrated under reduced pressure and then purified by silica gel column chromatography (PE: EA = 10:1) to obtain compound **78-2** (65.6 g, Y: 78%).

Step 2: Compound **78-2** (46.8 g, 238.70 mmol) was dissolved in dichloromethane (500 mL), then compound **11-5** (55.0 g, 217.00 mmol) and N,N-diisopropylethylamine (39.26 g, 303.8 mmol) were added thereto under nitrogen atmosphere, and the reaction mixture was reacted at 25°C for 16 hours. TLC (PE: EA = 10:1) showed that the starting material was completely reacted. The reaction mixture was poured into water (500 mL) to quench, and extracted with ethyl acetate (500 mL × 3). The organic phases were combined, washed with saturated brine (300 mL × 3), dried over anhydrous sodium sulfate, and filtered. The filtrate was concentrated under reduced pressure and then purified by silica gel column chromatography (PE: EA = 25:1) to obtain a white solid mixture (47.6 g, Y: 59%). The mixture was subjected to chiral purification (Amylose-C Neo, 5 cm × 25 cm, 20 µm, MeOH (20 mM NH₃), 100 mL/min) to obtain compound **78-3** (17.1 g, ee > 99%, Y: 35%). ¹H NMR (400 MHz, DMSO-*d₆*): δ 7.58-7.46 (m, 7H), 7.31-7.25 (m, 1H). Chiral analysis (Celluiose-SC 100 * 4.6 mm 3 µm; 0.8 mL-95% hexane-5% IPA; run time: 9 min; retention time: 5.806).

Step 3: Compound **78-3** (15.0 g, 39.3 mmol) was dissolved in tetrahydrofuran (300 mL). The reaction mixture was cooled to 0°C under nitrogen atmosphere, added with sodium hydride (1.9 g, 60 wt%, 46.4 mmol) in batches, stirred for 30 minutes, and then added dropwise with compound bromomethyl methyl ether (6.6 g, 50.3 mmol). The reaction mixture was stirred for 30 minutes, then warmed to 25°C, and reacted for 1 hour. TLC (EA: PE = 1:7) showed that the starting material was completely reacted. The reaction mixture was poured into saturated ammonium chloride aqueous solution (300 mL) to quench, and extracted with ethyl acetate (300 mL × 2). The organic phases were combined, washed with saturated brine (200 mL), dried over anhydrous sodium sulfate, and filtered. The filtrate was concentrated under reduced pressure and then purified by silica gel column chromatography (PE: EA = 10:1) to obtain compound **78-4** (14.3 g, Y: 85%).

Step 4: Compound **78-4** (14.3 g, 34.7 mmol) was dissolved in anhydrous dichloromethane (300 mL). The reaction mixture was cooled to -78°C, added dropwise with diisobutylaluminium hydride (1 M, 45.1 mL), warmed to 25°C, and reacted for 1 hour. TLC (EA: PE = 1:7) showed that the starting material was completely reacted. The reaction mixture was cooled to 0°C, added dropwise with 15% sodium hydroxide aqueous solution (200 mL) to quench, and extracted with dichloromethane (200 mL × 3). The organic phases were combined, washed with saturated brine (200 mL), dried over anhydrous sodium sulfate, and filtered. The filtrate was concentrated under reduced pressure and then purified by silica gel column chromatography (PE: EA = 10:1) to obtain compound **78-5** (8.2 g, Y: 56%). ¹HNMR (400 MHz, CDCl₃) δ 9.84 (s, 1H), 7.62-7.56 (m, 2H), 7.46-7.35 (m, 3H), 6.97 (d, *J* = 8.8 Hz, 1H), 5.42 (s, 1H), 5.18 (d, *J* = 6.8 Hz, 1H), 4.84 (d, *J* = 7.28 Hz, 1H), 3.49 (s, 3H).

Step 5: Compound **78-5** (10.1 g, 24.3 mmol) was dissolved in anhydrous tetrahydrofuran (50 mL). The reaction mixture was cooled to 0°C, added dropwise with Grignard reagent **S** (0.36 M, 150 mL), and reacted at 0°C for 1 hour. LCMS showed that the starting material was completely reacted. The reaction mixture was added dropwise with ice-cold ammonium chloride aqueous solution (200 mL) to quench, and extracted with ethyl acetate (100 mL × 3). The organic phases were combined, washed with saturated brine (200 mL), dried over anhydrous sodium sulfate, and filtered. The filtrate was concentrated under reduced pressure and then purified by silica gel column chromatography (1% NH₃ in DCM: MeOH = 1:5) to obtain compound **78-6** (4.1 g, Y: 35%). LC-MS (ESI): 474.1/476.1 [M+H]⁺.

Step 6: Compound **78-6** (4.0 g, 8.4 mmol) was dissolved in dichloromethane (50 mL). The reaction mixture was cooled to 0°C, added with triethylamine (2.5 g, 25.2 mmol), then added dropwise with di-tert-butyl dicarbonate (2.7 g, 12.6 mmol) dissolved in dichloromethane (20 mL), warmed to room temperature, and reacted for 2 hours. The reaction mixture was added dropwise with water (100 mL) to quench, and extracted with dichloromethane (100 mL × 2). The organic phases were combined, washed with saturated brine (100 mL), dried over anhydrous sodium sulfate, and filtered. The filtrate was concentrated under reduced pressure and then purified by silica gel column chromatography (PE: EA = 7:1) to obtain compound **78-7** (3.9 g, Y: 80%). LC-MS (ESI): 596.4/598.1 [M+Na]⁺.

Step 7: Compound **78-7** (4.0 g, 6.96 mmol) was dissolved in dichloromethane (40 mL), then compound Dess-Martin periodinane (5.90 g, 13.9 mmol) was added thereto under nitrogen atmosphere, and the reaction mixture was reacted at room temperature for 2 hours. TLC (PE: EA = 5:1) showed that the starting material was completely reacted. The reaction mixture was poured into saturated sodium sulfite (100 mL) to quench, extracted with ethyl acetate (50 mL × 3), and the organic phase was washed with saturated sodium carbonate (100 mL). The organic phases were combined, washed with saturated brine (100 mL), dried over anhydrous sodium sulfate, and filtered. The filtrate was concentrated under reduced pressure and then purified by silica gel column chromatography (PE: EA = 5:1) to obtain compound **78-8** (2.3 g, Y: 52%). LC-MS m/z (ESI): 594.3/596.1 [M+Na]⁺.

Step 8: Compound **78-8** (2.2 g, 3.84 mmol) was dissolved in dichloromethane (20 mL). The reaction mixture was cooled to 0°C under nitrogen atmosphere, added with a solution of HCl in dioxane (4 M, 10 mL), warmed to room temperature, and stirred for 2 hours. TLC (PE: EA = 5:1) showed that the starting material was completely reacted. The reaction mixture was cooled to 0°C, poured into saturated sodium bicarbonate (50 mL) to quench, and extracted with ethyl acetate (50 mL × 3). The organic phases were combined, washed with saturated brine (50 mL), dried over anhydrous sodium sulfate, filtered, and the filtrate was concentrated under reduced pressure to obtain compound **78-9** (1.7 g, Y: 95%, HCl salt).

Step 9: Compound **78-9** (1.6 g, 3.44 mmol) was dissolved in anhydrous methanol (20 mL), then chlorotitanium triisopropoxide (447 mg, 1.72 mmol) and sodium triacetoxyborohydride (648 mg, 10.3 mmol) were sequentially added thereto under nitrogen atmosphere, and the reaction mixture was stirred at room temperature for 16 hours. LCMS showed that the starting material was completely reacted. The reaction mixture was poured into saturated sodium bicarbonate (50 mL) to quench, and extracted with ethyl acetate (50 mL × 3). The organic phases were combined, washed with saturated brine (50 mL), dried over anhydrous sodium sulfate, filtered, and the filtrate was concentrated under reduced pressure to obtain compound **78-10** (1.2 g, Y: 84%). LC-MS m/z (ESI): 412.1/414.1 [M+H]⁺.

Step 10: Compound **78-10** (1.2 g, 2.80 mmol) was dissolved in dichloromethane (10 mL), then triethylamine (685 mg, 6.78 mmol) and di-tert-butyl dicarbonate (888 mg, 4.07 mmol) were sequentially added thereto under nitrogen atmosphere, and the reaction mixture was stirred at room temperature for 2 hours. LCMS showed that the starting material was completely reacted. The reaction mixture was poured into saturated ammonium chloride (100 mL) to quench, and extracted with ethyl acetate (150 mL × 3). The organic phases were combined, washed with saturated brine (150 mL), dried over anhydrous sodium sulfate, and filtered. The filtrate was concentrated under reduced pressure and then purified by silica gel column chromatography (PE: EA = 1:1) to obtain compound **78-11** (1.0 g, Y: 67%). LC-MS m/z (ESI): 577.1 [M+Na+ACN]⁺.

Step 11: Compound **78-11** (200 mg, 0.4 mmol) was dissolved in toluene (3 mL), then compounds bis(pinacolato)diboron (373 mg, 1.6 mmol), [1,1'-bis(diphenylphosphino)ferrocene]palladiutn(II) dichloride (70.9 mg, 0.08 mmol), and potassium acetate (240 mg, 1.2 mmol) were sequentially added thereto under nitrogen atmosphere, and the reaction mixture was reacted at 80°C for 16 hours. LCMS showed that the starting material was completely reacted. The reaction mixture was cooled to 25°C, concentrated under reduced pressure, and then purified by reversed-phase chromatography (C18, CAN: H₂O = 95:5) to obtain compound **78-12** (100 mg, Y: 46%). LC-MS m/z (ESI): 582.2 [M+Na]⁺.

Step 12: Compound **51-8** (200 mg, 0.50 mmol) was dissolved in toluene (3 mL) and water (0.5 mL), then compound **78-12** (392 mg, 0.70 mmol), tris(dibenzylideneacetone)dipalladium (92.6 mg, 0.1 mmol), 4,5-bis(diphenylphosphino)-9,9-dimethylxanthene (104 mg, 0.2 mmol), and anhydrous potassium carbonate (207 mg, 1.5 mmol) were sequentially added thereto under nitrogen atmosphere, and the reaction mixture was reacted at 110°C overnight. LCMS showed that the starting material was completely reacted. The reaction mixture was cooled to room temperature, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 3:1) to obtain compound **78-13** (20 mg, Y: 5.3%). LC-MS (1.2 mL-3.2 min-5 to 90% B): Rt = 2.767, m/z (ESI): 752.9 [M-H]⁻.

Step 13: Compound **78-13** (20 mg, 0.027 mmol) was dissolved in dichloromethane (0.5 mL). The reaction mixture was cooled to 0°C under nitrogen atmosphere, added with a solution of HCl in dioxane (4 M, 3 mL), warmed to room temperature, and stirred for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: Xselect CSH Prep Fluoro-Phenyl, 150 * 19 mm, 5 µm; eluent: (0.1% FA) H₂O-CAN; gradient: 30% to 50%; flow rate: 20 mL/min; run time: 11 min; retention time: 8.9 min) to obtain compound **78a1** (2.5 mg, Y: 17%). ¹HNMR (400 MHz, DMSO-*d*₆) δ 8.76-8.68 (m, 1H), 8.49-8.11 (m, 2H), 7.59-7.53 (m, 1H), 7.49-7.32 (m, 5H), 7.06-7.00 (m, 1H), 5.43-5.38 (m, 1H), 5.28-5.17 (m, 2H), 4.21-4.00 (m, 2H), 3.19-2.80 (m, 4H), 2.63 (d, *J* = 4.8 Hz, 3H), 2.11-1.80 (m, 6H). LC-MS m/z (ESI): 541.3 [M+H]⁺. Chiral analysis (column model: Celluiose-C 100 * 4.6 mm 3 µm; eluent: hexane (0.1% DEA)/IPA; gradient: isocratic 9/1, flow rate: 1 mL/min; run time: 10 min; retention time: 5.46 min).

### Example 79

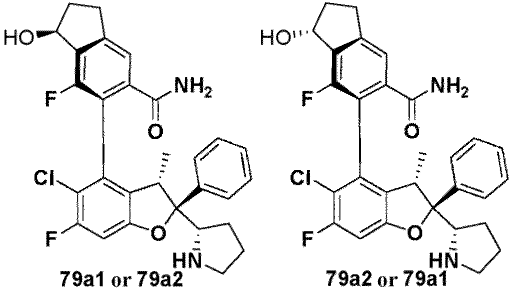

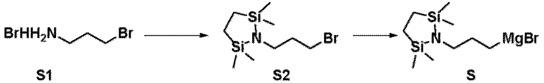

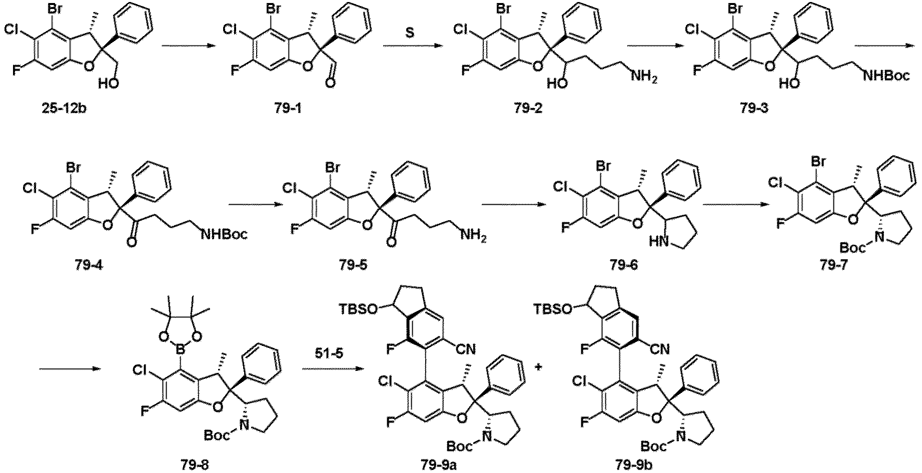

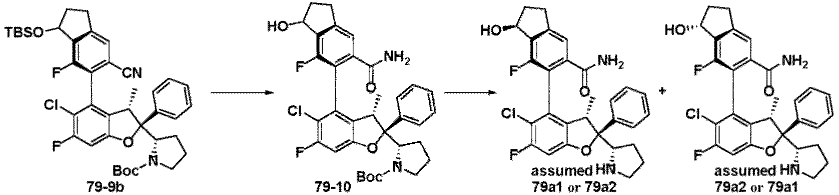

Step 1: Oxalyl chloride (3.8 g, 30.1 mmol) was dissolved in dichloromethane (50 mL). The reaction system was cooled to -70°C under nitrogen atmosphere, added dropwise with dimethyl sulfoxide (4.4 g, 56.5 mmol) dissolved in dichloromethane (10 mL), stirred for 30 minutes, and then added dropwise with compound **25-12b** (7.0 g, 18.8 mmol) dissolved in dichloromethane (10 mL). The reaction mixture was stirred for 30 minutes, then added with triethylamine (5.7 g, 56.5 mmol), warmed to room temperature, and stirred for 1 hour. TLC (EA: PE = 1:7) showed that the starting material was completely reacted. The reaction mixture was poured into water (200 mL) to quench, and extracted with dichloromethane (200 mL × 2). The organic phases were combined, washed with saturated brine (200 mL), dried over anhydrous sodium sulfate, filtered, and the filtrate was concentrated under reduced pressure to obtain compound **79-2** (6.1 g, Y: 88%).

Step 2: Compound S1 (19.7 g, 91.4 mmol) was dissolved in dichloromethane (200 mL). The reaction mixture was cooled to 0°C, added dropwise with triethylamine (27.7 g, 274.1 mmol), stirred at 0°C for 10 minutes, and added dropwise with a solution of 1,2-bis(chlorodimethylsilyl)ethane (20.0 g, 91.4 mmol) in dichloromethane (100 mL). After the dropwise addition was completed, the reaction mixture was warmed to 25°C and reacted for 2 hours. The reaction mixture was concentrated under reduced pressure, slurried with n-hexane (200 mL), filtered, and the filtrate was concentrated to obtain compound **S2** (14.50 g, Y: 59%). ¹H NMR (400 MHz, DMSO-*d₆*) δ 3.51 (t, *J* = 6.8 Hz, 2H), 2.90 (t, *J* = 7.2 Hz, 2H), 1.92-1.82 (m, 2H), 0.67 (s, 4H), 0.04 (s, 12H). Compound **S2** (24.0 g, 85.6 mmol) was dissolved in anhydrous tetrahydrofuran (240 mL) for later use. Magnesium chips (4.2 g, 171.2 mmol) and iodine (0.22 g, 0.86 mmol) were dissolved in anhydrous tetrahydrofuran (20 mL), then about one-tenth of a solution of compound **S2** in tetrahydrofuran was added dropwise thereto under nitrogen atmosphere, and the reaction mixture was heated to 50°C and reacted. After the reaction was initiated, the reaction system was added dropwise with the remaining compound **S2** and reacted at 50°C for 1.5 hours. The reaction mixture was cooled to room temperature to obtain gray liquid **S** (0.36 M, 240 mL). Compound **79-2** (5.1 g, 13.7 mmol) was dissolved in anhydrous tetrahydrofuran (50 mL). The reaction mixture was cooled to 0°C, added dropwise with **S** (0.36 M, 133 mL, 48.0 mmol), and reacted at 0°C for 1 hour. Three batches were added in parallel. LCMS showed that the starting material was completely reacted. Three batches of reaction mixture were combined, added dropwise with ice-cold ammonium chloride aqueous solution (200 mL) to quench, and extracted with ethyl acetate (100 mL × 3). The organic phases were combined, washed with saturated brine (200 mL), dried over anhydrous sodium sulfate, and filtered. The filtrate was concentrated under reduced pressure and then purified by silica gel column chromatography (DCM: MeOH: NH₃H₂O = 10:1:0.1) to obtain compound **79-3** (5.7 g, Y: 32%). LC-MS m/z (ESI): 428.1/430.3 [M+H]⁺.

Step 3: Compound **79-2** (5.5 g, 12.8 mmol) was dissolved in dichloromethane (50 mL). The reaction mixture was cooled to 0°C, added with triethylamine (3.9 g, 38.5 mmol), then added dropwise with di-tert-butyl dicarbonate (4.2 g, 19.2 mmol) dissolved in dichloromethane (20 mL), warmed to room temperature, and reacted for 1 hour. The reaction mixture was added dropwise with water (100 mL) to quench, and extracted with ethyl acetate (100 mL × 2). The organic phases were combined, washed with saturated brine (100 mL), dried over anhydrous sodium sulfate, and filtered. The filtrate was concentrated under reduced pressure and then purified by silica gel column chromatography (PE: EA = 7:1) to obtain compound **79-3** (5.7 g, Y: 84%).

Step 4: Compound **79-3** (2.3 g, 4.35 mmol) was dissolved in dichloromethane (30 mL), then compound Dess-Martin periodinane (3.69 g, 8.70 mmol) was added thereto under nitrogen atmosphere, and the reaction mixture was reacted at room temperature for 2 hours. TLC (PE: EA = 1:1) showed that the starting material was completely reacted. The reaction mixture was poured into saturated sodium sulfite (100 mL) to quench, extracted with ethyl acetate (50 mL × 3), and the organic phase was washed with saturated sodium carbonate (100 mL). The organic phases were combined, washed with saturated brine (100 mL), dried over anhydrous sodium sulfate, and filtered. The filtrate was concentrated under reduced pressure and then purified by silica gel column chromatography (PE: EA = 1:1) to obtain compound **79-4** (4.00 g, Y: 87%). LC-MS m/z (ESI): 548.1 [M+Na]⁺.

Step 5: Compound **79-4** (3.5 g, 6.64 mmol) was dissolved in dichloromethane (20 mL). The reaction mixture was cooled to 0°C under nitrogen atmosphere, added with a solution of HCl in dioxane (4 M, 5 mL), warmed to room temperature, and reacted for 1 hour. LCMS showed that the starting material was completely reacted. The reaction mixture was concentrated under reduced pressure to obtain compound **79-5** (3.00 g, Y: 97%, HCl salt). LC-MS m/z (ESI): 426.1 [M+H]⁺.

Step 6: Compound **79-5** (2 g, 4.69 mmol) was dissolved in anhydrous methanol (20 mL), then chlorotitanium triisopropoxide (609.32 mg, 2.34 mmol) and sodium triacetoxyborohydride (883.63 mg, 14.06 mmol) were sequentially added thereto under nitrogen atmosphere, and the reaction mixture was reacted at 25°C for 4 hours. LCMS showed that the starting material was completely reacted. The reaction mixture was poured into saturated sodium bicarbonate (50 mL) to quench, and extracted with ethyl acetate (50 mL × 3). The organic phases were combined, washed with saturated brine (50 mL), dried over anhydrous sodium sulfate, filtered, and the filtrate was concentrated under reduced pressure to obtain compound **79-6** (1.90 g, Y: 98%) as a yellow oil. LC-MS m/z (ESI): 410.1 [M+H]⁺.

Step 7: Compound **79-6** (3 g, 7.29 mmol) was dissolved in dichloromethane (30 mL), then triethylamine (1.45 g, 14.6 mmol) and di-tert-butyl dicarbonate (2.39 g, 10.9 mmol) were sequentially added thereto under nitrogen atmosphere, and the reaction mixture was reacted at room temperature for 1 hour. LCMS showed that the starting material was completely reacted. The reaction mixture was poured into saturated ammonium chloride (100 mL) to quench, and extracted with dichloromethane (50 mL × 3). The organic phases were combined, washed with saturated brine (150 mL), dried over anhydrous sodium sulfate, and filtered. The filtrate was concentrated under reduced pressure and then purified by silica gel column chromatography (PE: EA = 1:1) to obtain compound **79-7** (3.00 g, Y: 80%) as a yellow solid. ¹H NMR (400 MHz, DMSO-*d₆*) δ 7.42-7.17 (m, 6H), 4.69-4.60 (m, 1H), 3.88-3.70 (m, 1H), 3.50-3.35 (m, 2H), 2.18-1.98 (m, 2H), 1.72-1.51 (m, 5H), 1.30 (s, 9H).

Step 8: Compound **79-7** (500 mg, 0.98 mmol) was dissolved in toluene (5 mL), then compounds bis(pinacolato)diboron (373 mg, 1.47 mmol), [1,1'-bis(diphenylphosphino)ferrocene]palladium(II) dichloride (70.9 mg, 0.098 mmol), and potassium acetate (240 mg, 2.45 mmol) were sequentially added thereto under nitrogen atmosphere, and the reaction mixture was reacted at 100°C for 15 minutes. TLC (PE: EA = 10:1) showed that the starting material was completely reacted. The reaction mixture was cooled to 25°C, poured into saturated ammonium chloride (20 mL) to quench, and extracted with ethyl acetate (20 mL × 3). The organic phases were combined, washed with saturated brine (20 mL), dried over anhydrous sodium sulfate, filtered, and the filtrate was concentrated under reduced pressure to obtain compound **79-8** (500 mg, Y: 91%).

Step 9: Compound **51-5** (250 mg, 0.67 mmol) was dissolved in toluene (1 mL) and water (0.2 mL), then compound **79-8** (500 mg, 0.81 mmol), tris(dibenzylideneacetone)dipalladium (92.6 mg, 0.1 mmol), 4,5-bis(diphenylphosphino)-9,9-dimethylxanthene (104 mg, 0.2 mmol), and anhydrous potassium carbonate (429 mg, 2.03 mmol) were sequentially added thereto under nitrogen atmosphere, and the reaction mixture was reacted at 110°C overnight. TLC (PE: EA = 1:1) showed that the starting material was completely reacted. The reaction mixture was cooled to room temperature, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 5:1) to obtain compounds **79-9a** (90 mg, Y: 18%, R_{f} = 0.30, R_{T} = 1.64/1.68) and **79-9b** (120 mg, Y: 24%, R_{f} = 0.35, R_{T} = 1.58/1.63). **79-9a:** LC-MS (D-1.5 mL-3 min-10 to 100% B): Rt = 1.643/1.683, m/z (ESI): 721.3 [M+H]⁺. **79-9b:** LC-MS (D-1.5 mL-3 min-10 to 100% B): Rt = 1.587/1.633, m/z (ESI): 721.3 [M+H]⁺.

Step 10: Compound **79-9a** (90 mg, 0.12 mmol) was dissolved in dimethyl sulfoxide (2 mL), then anhydrous potassium carbonate (49.9 mg, 0.57 mmol) and 30% hydrogen peroxide (0.1 mL) were sequentially added thereto under nitrogen atmosphere, and the reaction mixture was stirred at room temperature for 2 hours. LCMS showed that the starting material was completely reacted. The reaction mixture was poured into saturated ammonium chloride (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compound **79-10** (60 mg, Y: 77%). LC-MS m/z (ESI): 625.2 [M+H]⁺.

Step 11: Compound **79-10** (60 mg, 0.098 mmol) was dissolved in dichloromethane (1 mL). The reaction mixture was cooled to 0°C under nitrogen atmosphere, added with a solution of HCl in dioxane (4 M, 1 mL), warmed to room temperature, and stirred for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: Xselect CSH Prep Fluoro-Phenyl, 150 * 19 mm, 5 µm; eluent: (0.1% FA) H₂O-ACN; gradient: 5% to 17%; flow rate: 20 mL/min; run time: 9 min) to obtain compound **79a2** (8.2 mg, Y: 15%, Rt = 6.5 min) and **79a1** (4.6 mg, Y: 8.8%, Rt = 5.9 min). **79a2:** ¹H NMR (400 MHz, DMSO-*d*₆) δ 8.15 (s, 1H), 7.74 (s, 1H), 7.57-7.13 (m, 7H), 7.04 (d, *J* = 9.6 Hz, 1H), 5.41-5.13 (m, 2H), 3.99-3.84 (m, 1H), 3.50-3.39 (m, 1H), 3.17-3.06 (m, 1H), 2.89-2.75 (m, 1H), 2.73-2.68 (m, 1H), 2.46-2.40 (m, 1H), 2.02-1.78 (m, 2H), 1.58-1.03 (m, 4H), 1.02 (d, *J* = 7.2 Hz, 3H). LC-MS m/z (ESI): 525.0 [M+H]⁺. Chiral analysis (column model: Celluiose-SB 100 * 4.6 mm 3 µm; eluent: hexane (0.1% DEA)/IPA; gradient: isocratic 85/15, flow rate: 1 mL/min; run time: 12 min; retention time: 6.42 min). **79a1**: ¹H NMR (400 MHz, DMSO-*d*₆) δ 8.15 (s, 1H), 7.57-7.42 (m, 3H), 7.36-7.18 (m, 4H), 7.04 (d, *J* = 9.2 Hz, 1H), 6.53 (s, 1H), 5.43-5.22 (m, 2H), 3.93-3.80 (m, 1H), 3.58-3.49 (m, 1H), 3.19-3.07 (m, 1H), 2.91-2.78 (m, 1H), 2.73-2.68 (m, 1H), 2.46-2.40 (m, 1H), 2.02-1.72 (m, 2H), 1.58-1.03 (m, 4H), 0.97 (d, *J* = 7.2 Hz, 3H). LC-MS m/z (ESI): 525.0 [M+H]⁺. Chiral analysis (column model: Celluiose-SB 100 * 4.6 mm 3 µm; eluent: hexane (0.1% DEA)/IPA; gradient: isocratic 85/15, flow rate: 1 mL/min; run time: 12 min; retention time: 4.47 min).

### Example 80

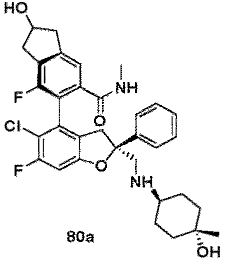

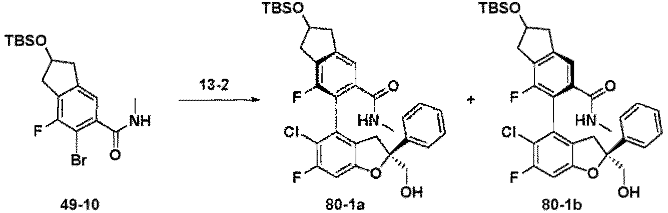

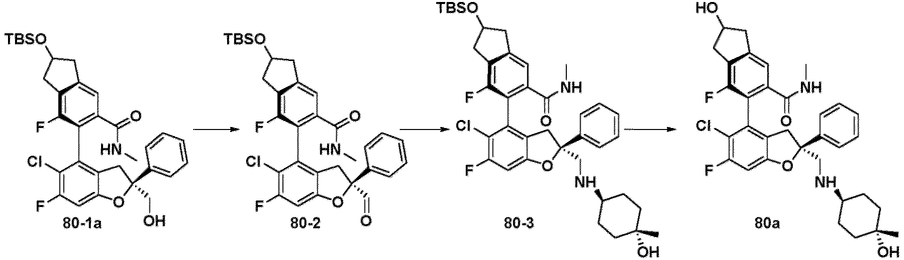

Step 1: Compound **49-10** (200 mg, 0.50 mmol) was dissolved in toluene (5 mL) and water (1 mL), then compound **13-2** (302 mg, 0.74 mmol), tris(dibenzylideneacetone)dipalladium (91 mg, 0.099 mmol), 4,5-bis(diphenylphosphino)-9,9-dimethylxanthene (115 mg, 0.20 mmol), and anhydrous potassium carbonate (206 mg, 1.49 mmol) were sequentially added thereto under nitrogen atmosphere, and the reaction mixture was reacted at 110°C for 16 hours. TLC (PE: EA = 3:1) showed that the starting material was completely reacted. Three batches were added in parallel for combined processing. The reaction mixture was cooled to 25°C, concentrated under reduced pressure, and then purified by silica gel column chromatography (PE: EA = 3:1) to obtain compound **80-1b** (240 mg, Y: 40%, R_{f} = 0.40) and compound **80-1a** (220 mg, Y: 37%, R_{f} = 0.30). **80-1b:** LC-MS (1.5 mL-2.0 min-5 to 95% B): Rt = 1.577, m/z (ESI): 600.2 [M+H]⁺. **80-1a:** LC-MS (1.5 mL-2.0 min-5 to 95% B): Rt = 1.693, m/z (ESI): 600.2 [M+H]⁺.

Step 2: Compound **80-1a** (50 mg, 0.08 mmol) was dissolved in dichloromethane (2 mL), then Dess-Martin periodinane (53 mg, 0.13 mmol) and sodium bicarbonate (21 mg, 0.25 mmol) were sequentially added thereto, and the reaction mixture was reacted at room temperature for 1 hour. LCMS showed that the starting material was completely reacted. Four batches were added in parallel for combined processing. The reaction mixture was poured into saturated sodium sulfite solution (50 mL) to quench, and extracted with dichloromethane (30 mL × 3). The organic phases were combined, washed with saturated brine (50 mL), dried over anhydrous sodium sulfate, filtered, and the filtrate was concentrated under reduced pressure to obtain compound **80-2** (140 mg, crude) as a yellow solid. LC-MS m/z (ESI): 598.3 [M+H]⁺.

Step 3: Compound **80-2** (50 mg, 0.08 mmol) was dissolved in methanol (1 mL). The reaction mixture was sequentially added with trans-4-amino-1-methylcyclohexanol (22 mg, 0.17 mmol) and acetic acid (1 mg, 0.02 mmol), heated to 55°C and stirred for 2 hours, then added with sodium borohydride (6 mg, 0.17 mmol), and reacted at 55°C for 1 hour. LCMS showed that the starting material was completely reacted. Three batches were added in parallel for combined processing. The reaction mixture was cooled to 25°C, poured into saturated sodium bicarbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compound **80-3** (90 mg, Y: 75%). LC-MS m/z (ESI): 711.0 [M+H]⁺.

Step 4: Compound **80-3** (90 mg, 0.13 mmol) was dissolved in dichloromethane (2 mL), then trifluoroacetic acid (1 mL) was added thereto, and the reaction mixture was reacted at room temperature for 1 hour. LCMS showed that the starting material was completely reacted. The reaction mixture was poured into saturated sodium bicarbonate (50 mL) to quench, and extracted with dichloromethane (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: YMC-Actus Triart C18, 150 * 20 mm, 5 µm; eluent: 0.05% NH₃H₂O-10 mmol/L NH₄HCO₃-ACN; gradient: 38% to 58%; flow rate: 20 mL/min; run time: 9.0 min; retention time: 7.1 min) to obtain compound **80a** (15.2 mg, Y: 20%). ¹H NMR (400 MHz, DMSO-*d*₆) δ 8.23-8.16 (m, 1H), 7.41-7.22 (m, 6H), 7.01 (d, *J* = 9.6 Hz, 1H), 5.11-5.01 (m, 1H), 4.63-4.56 (m, 1H), 4.11-4.04 (m, 1H), 3.68-3.50 (m, 3H), 3.21-3.05 (m, 2H), 2.96-2.76 (m, 4H), 2.62 (d, *J* = 4.4 Hz, 3H), 1.70-1.53 (m, 2H), 1.46-1.33 (m, 2H), 1.30-1.03 (m, 4H), 1.01 (s, 3H). LC-MS m/z (ESI): 597.3 [M+H]⁺. Chiral analysis (column model: Celluiose-SC 100 * 4.6 mm 3 µm; eluent: hexane/IPA; gradient: isocratic 8/2, flow rate: 1 mL/min; run time: 9.0 min; retention time: 4.97 min and 6.09 min, dr = 49 (4.97 min):51 (6.09 min)).

### Example 81

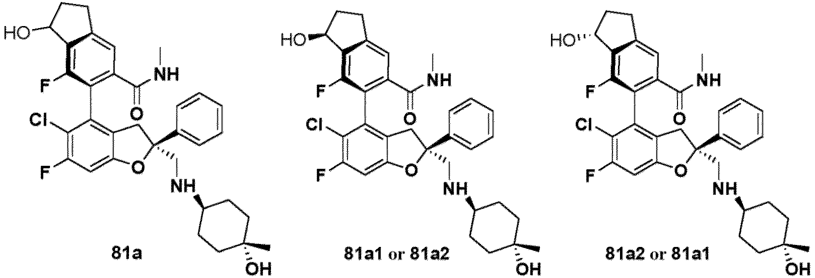

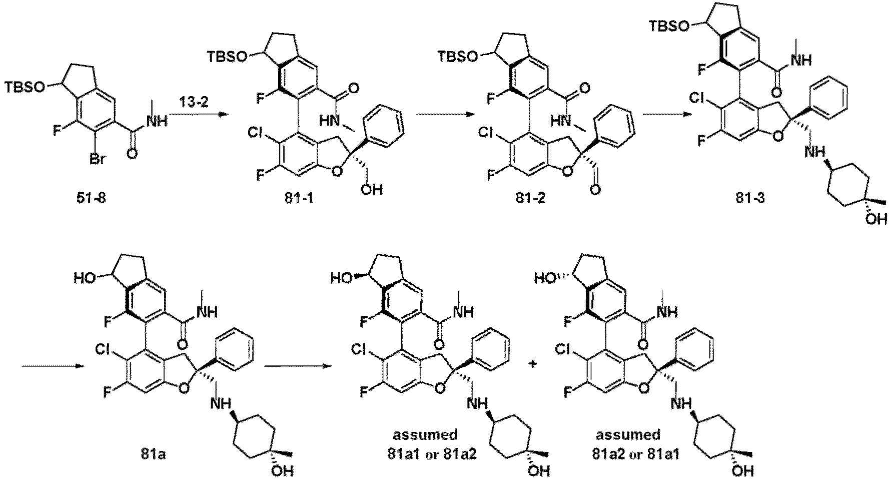

Step 1: Compound **51-8** (200 mg, 0.50 mmol) was dissolved in toluene (5 mL) and water (1 mL), then compound **13-2** (302 mg, 0.74 mmol), tris(dibenzylideneacetone)dipalladium (91 mg, 0.099 mmol), 4,5-bis(diphenylphosphino)-9,9-dimethylxanthene (115 mg, 0.20 mmol), and anhydrous potassium carbonate (206 mg, 1.49 mmol) were sequentially added thereto under nitrogen atmosphere, and the reaction mixture was reacted at 100°C for 16 hours. TLC (PE: EA = 3:1) showed that the starting material was completely reacted. Three batches were added in parallel for combined processing. The reaction mixture was cooled to 25°C, concentrated under reduced pressure, and purified by silica gel column chromatography (PE: EA = 2:3, Rf = 0.20 and Rf = 0.30, where Rf = 0.20 was for target product) to obtain compound **81-1** (180 mg, Y: 30%). LC-MS (1.5 mL-2.0 min-5 to 95% B): Rt = 1.667, m/z (ESI): 600.2 [M+H]⁺.

Step 2: Compound **81-1** (180 mg, 0.30 mmol) was dissolved in dichloromethane (3 mL), then compound Dess-Martin periodinane (253 mg, 0.60 mmol) was added thereto under nitrogen atmosphere, and the reaction mixture was reacted at room temperature for 2 hours. TLC (PE: EA = 1:1) showed that the starting material was completely reacted. The reaction mixture was poured into saturated sodium sulfite (5 mL) to quench, extracted with dichloromethane (10 mL × 3), and the organic phase was washed with saturated sodium carbonate (10 mL). The organic phases were combined, washed with saturated brine (10 mL), dried over anhydrous sodium sulfate, filtered, and the filtrate was concentrated under reduced pressure to obtain compound **81-2** (150 mg, Y: 83%).

Step 3: Compound **81-2** (100 mg, 0.17 mmol) was dissolved in anhydrous methanol (2 mL). The reaction mixture was sequentially added with trans-4-amino-1-methylcyclohexanol (64.8 mg, 0.50 mmol), chlorotitanium triisopropoxide (43.7 mg, 0.17 mmol), and sodium cyanoborohydride (31.5 mg, 0.50 mmol) under nitrogen atmosphere, heated to 45°C, and reacted for 16 hours. LCMS showed that the starting material was completely reacted. The reaction mixture was cooled to 25°C, poured into saturated sodium bicarbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA= 1:1) to obtain compound **81-3** (80.0 mg, Y: 67%). LC-MS m/z (ESI): 711.3 [M+H]⁺.

Step 4: Compound **81-3** (80 mg, 0.12 mmol) was dissolved in dichloromethane (1 mL). The reaction mixture was cooled to 0°C under nitrogen atmosphere, added with trifluoroacetic acid (0.2 mL), and stirred at room temperature for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: YMC-Actus Triart C18, 150 * 20 mm, 5 µm; eluent: 0.05% NH₃H₂O-10 mmol/L NH₄HCO₃-ACN; gradient: 36% to 56%; flow rate: 20 mL/min; run time: 9 min; retention time: 7 min) to obtain compound **81a** (20.0 mg, Y: 30%). LC-MS m/z (ESI): 597.0 [M+H]⁺.

Step 5: Compound **81a** (20 mg, 0.034 mmol) was purified by chiral preparation (Lux-4, 50 * 250 mm, 10 µm; eluent: 20 mM NH₃H₂O-IPA-50% hexane; 35°C, flow rate: 20 mL/min) to obtain compound **81a2** (3.1 mg, Y: 15%) and compound **81a1** (3.0 mg, Y: 15.0%). **81a2:** ¹H NMR (400 MHz, DMSO-*d*₆) δ 8.30-8.20 (m, 1H), 7.43-7.22 (m, 6H), 7.03 (d, *J* = 9.6 Hz, 1H), 5.37-5.22 (m, 2H), 4.11-4.01 (m, 1H), 3.53-3.42 (m, 2H), 3.17-2.70 (m, 5H), 2.63 (d, *J* = 4.8 Hz, 3H), 2.39-2.28 (m, 1H), 2.00-1.89 (m, 1H), 1.71-1.54 (m, 2H), 1.47-1.03 (m, 6H), 1.01 (s, 3H). LC-MS m/z (ESI): 597.3 [M+H]⁺. Chiral analysis (column model: Celluiose-C 100 * 4.6 mm 3 µm; eluent: hexane (0.1% DEA)/IPA; gradient: isocratic 4/1, flow rate: 1 mL/min; run time: 8 min; retention time: 3.52 min). **81a1:** ¹H NMR (400 MHz, DMSO-*d₆*) δ 8.29-8.20 (m, 1H), 7.43-7.21 (m, 6H), 7.02 (d, *J* = 9.6 Hz, 1H), 6.22-6.09 (m, 1H), 5.50-5.28 (m, 1H), 5.50-5.28 (m, 1H), 5.26-5.20 (m, 1H), 4.12-3.98 (m, 1H), 3.16-3.00 (m, 2H), 2.94-2.72 (m, 4H), 2.62 (d, *J* = 4.0 Hz, 3H), 2.36-2.27 (m, 1H), 2.00-1.88 (m, 1H), 1.70-1.53 (m, 2H), 1.47-1.03 (m, 6H), 1.00 (s, 3H). LC-MS m/z (ESI): 597.3 [M+H]⁺. Chiral analysis (column model: Celluiose-C 100 * 4.6 mm 3 µm; eluent: hexane (0.1% DEA)/IPA; gradient: isocratic 4/1, flow rate: 1 mL/min; run time: 8 min; retention time: 3.88 min).

### Example 82

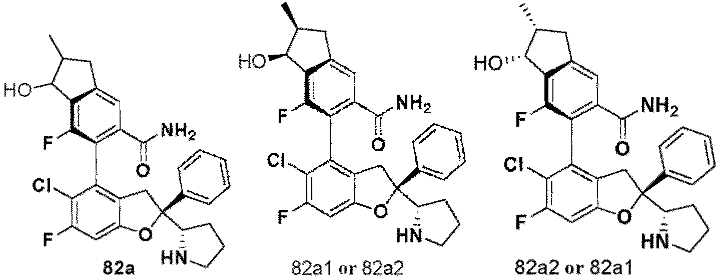

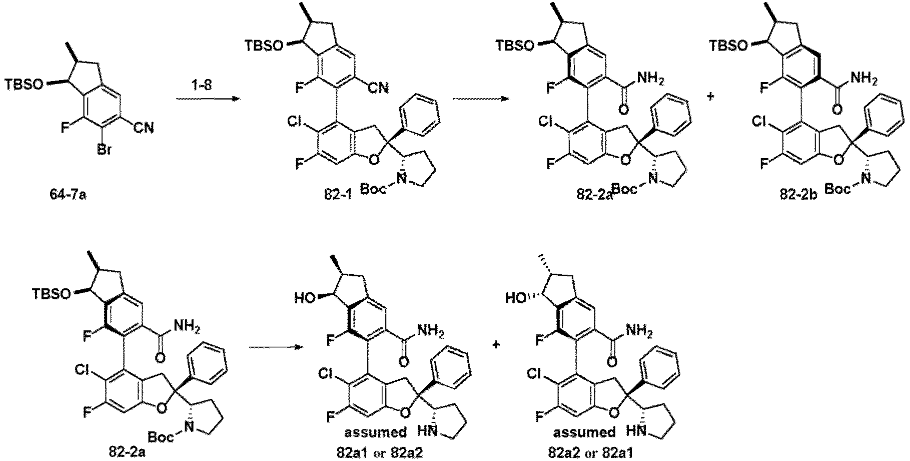

Step 1: Compound **64-7a** (300 mg, 0.78 mmol) and compound **1-8** (382 mg, 0.70 mmol) were dissolved in toluene (6 mL) and water (1.2 mL), and tris(dibenzylideneacetone)dipalladium (107 mg, 0.11 mmol), 4,5-bis(diphenylphosphino)-9,9-dimethylxanthene (135 mg, 0.23 mmol), and anhydrous potassium carbonate (323 mg, 2.34 mmol) were sequentially added thereto. The reaction mixture was reacted at 110°C overnight under nitrogen atmosphere. LCMS showed that the starting material was completely reacted. The reaction mixture was cooled to 25°C, concentrated under reduced pressure, and purified by silica gel column chromatography (EA: PE = 1:8) to obtain compound **82-1** (390 mg, Y: 69%). LC-MS: m/z (ESI): 665.3 [M-tBu+H]⁺.

Step 2: Compound **82-1** (390 mg, 0.54 mmol) and anhydrous potassium carbonate (250 mg, 1.81 mmol) were dissolved in dimethyl sulfoxide (7 mL). The reaction mixture was cooled to 10°C, slowly added dropwise with hydrogen peroxide (190 mg, 30%), and reacted at room temperature for 1 hour. LCMS showed that the starting material was completely reacted. The reaction mixture was poured into water (50 mL), and extracted with ethyl acetate (50 mL × 2). The organic phases were combined, washed with saturated brine (10 mL), dried over anhydrous sodium sulfate, and filtered. The filtrate was concentrated under reduced pressure and then purified by silica gel column chromatography (EA: PE = 1:20 to 1:5, Rf = 0.6, Rf = 0.5, where Rf = 0.5 was for target product) to obtain compound **82-2a** (150 mg, Y: 37%). LC-MS m/z (1.5 mL-5 min-5 to 95% B): Rt = 3.910/3.957, (ESI): 739.3 [M+H]⁺.

Step 3: Compound **82-2a** (150 mg, 0.20 mmol) was dissolved in hydrochloric acid/dioxane solution (4 mL, 4.0 M), and the reaction mixture was reacted at 25°C for 0.5 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% FA-ACN; gradient: 20 to 55% ACN in H₂O; flow rate: 20 mL/min; run time: 12 min; retention time: Rt = 7.8 min/8.9 min) to obtain compounds **82a1** (53.0 mg, FA salt, Y: 45%, Rt = 7.8 min) and **82a2** (46.9 mg, FA salt, Y: 40%, Rt = 8.9 min).

**82a1:** ¹H NMR (400 MHz, DMSO-*d₆*): δ 8.26-8.19 (br, 1H), 7.66 (br, 1H), 7.49-7.40 (m, 2H), 7.38-7.24 (m, 5H), 7.02 (d, *J* = 9.6 Hz, 1H), 4.96 (d, *J* = 6.4 Hz, 1H), 3.69-3.56 (m, 1H), 3.52-3.43 (m, 1H), 3.00-2.61 (m, 5H), 2.43-2.36 (m, 1H), 1.71-1.31 (m, 4H), 1.11 (d, *J* = 7.2 Hz, 3H). LC-MS m/z (ESI): 525.2/527.2 [M+H]⁺. Chiral analysis (column model: Amylose-SA, 100 * 4.6 mm, 3 µm; eluent: hexane (0.1% DEA)-IPA; gradient: isocratic 9/1, flow rate: 1 mL/min; run time: 8.5 min; retention time: 4.91 min).

**82a2:** ¹H NMR (400 MHz, DMSO-*d₆*): δ 8.22 (br, 1H), 7.67 (br, 1H), 7.46-7.22 (m, 7H), 7.02 (d, *J* = 9.6 Hz, 1H), 4.96 (d, *J* = 6.0 Hz, 1H), 3.67-3.59 (m, 1H), 3.54 (d, *J* = 16.0 Hz, 1H), 3.00-2.61 (m, 5H), 2.45-2.37 (m, 1H), 1.76-1.31 (m, 4H), 1.11 (d, *J* = 6.8 Hz, 3H). LC-MS m/z (ESI): 525.2/527.3 [M+H]⁺. Chiral analysis (column model: Amylose-SA, 100 * 4.6 mm, 3 µm; eluent: hexane (0.1% DEA)-IPA; gradient: isocratic 9/1, flow rate: 1 mL/min; run time: 8.5 min; retention time: 5.05 min).

### Example 83

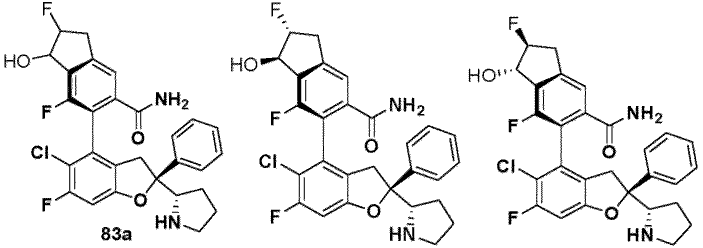

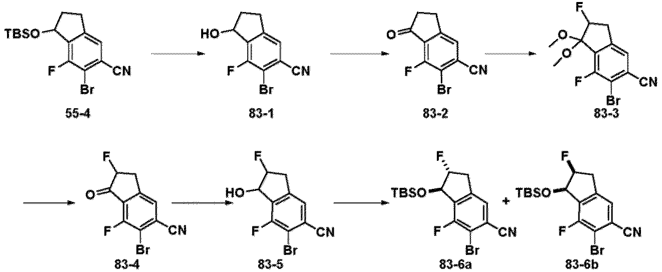

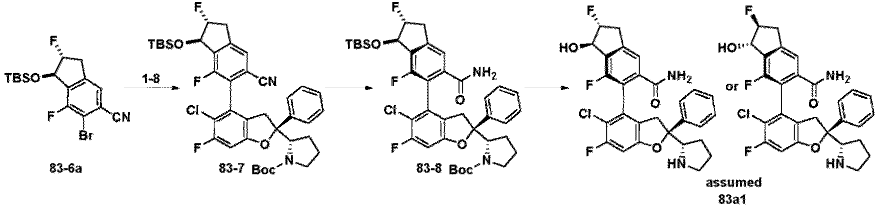

Step 1: **55-4** (3.5 g, 9.5 mmol) was dissolved in anhydrous tetrahydrofuran (30 mL). The reaction mixture was cooled to 0°C, and slowly added with tetrabutylammonium fluoride (9.5 mL, 9.5 mmol, 1.0 M in THF). The reaction mixture was reacted at room temperature for 0.5 hours. TLC showed that the starting material was completely reacted. The reaction mixture was added with water (200 mL), and extracted with ethyl acetate (150 mL × 3). The organic phases were combined, washed with saturated brine (50 mL × 2), dried over anhydrous sodium sulfate, and filtered. The filtrate was concentrated under reduced pressure and then purified by silica gel column chromatography (EA: PE = 1:6) to obtain compound **83-1** (2.0 g, Y: 82%).

Step 2: Compound **83-1** (2.0 g, 7.8 mmol) was dissolved in anhydrous dichloromethane (20 mL), then Dess-Martin periodinane (5.0 g, 11.8 mmol) was added thereto, and the reaction mixture was reacted at room temperature for 1 hour under nitrogen atmosphere. TLC showed that the starting material was completely reacted. The reaction mixture was slowly added with saturated sodium thiosulfate aqueous solution (50 mL) and saturated sodium bicarbonate aqueous solution (50 mL), stirred for 0.5 hours, and extracted with dichloromethane (70 mL × 3). The organic phases were combined, washed with saturated brine (60 mL), dried over anhydrous sodium sulfate, and filtered. The filtrate was concentrated under reduced pressure and then purified by silica gel column chromatography (EA: PE = 1:7) to obtain compound **83-2** (1.8 g, Y: 90%).

Step 3: Compound **83-2** (1.8 g, 7.1 mmol) was dissolved in anhydrous methanol (18 mL), then 1-chloromethyl-4-fluoro-1,4-diazabicyclo[2.2.2]octane bis(tetrafluoroborate) (3.0 g, 8.5 mmol) and concentrated sulfuric acid (120 mg, 1.22 mmol) were sequentially added thereto, and the reaction mixture was reacted at 60°C for 12 hours under nitrogen atmosphere. TLC showed that the starting material was completely reacted. The reaction mixture was cooled to 25°C, then the solid was filtered, and the filter cake was washed with methanol (30 mL). The filtrate was directly concentrated under reduced pressure and then purified by silica gel column chromatography (EA: PE = 1:10) to obtain compound **83-3** (1.1 g, Y: 49%). ¹H NMR (400 MHz, CDCl₃): δ 7.39 (s, 1H), 5.21-5.07 (m, 1H), 3.52 (s, 3H), 3.33-3.20 (m, 4H), 3.11-3.00 (m, 1H).

Step 4: Compound **83-3** (1.1 g, 3.46 mmol) was dissolved in acetone (8 mL) and water (4 mL), then p-toluenesulfonic acid (650 mg, 3.78 mmol) was added thereto, and the reaction mixture was reacted at 65°C for 2 hours under nitrogen atmosphere. TLC showed that the starting material was completely reacted. The reaction mixture was cooled to 25°C, added with saturated sodium bicarbonate aqueous solution (30 mL), and extracted with ethyl acetate (40 mL × 3). The organic phases were combined, washed with saturated brine (30 mL), dried over anhydrous sodium sulfate, filtered, and the filtrate was concentrated under reduced pressure to obtain compound **83-4** (700 mg, crude).

Step 5: Compound **83-4** (700 mg, 2.57 mmol) was dissolved in tetrahydrofuran (8 mL). The reaction mixture was cooled to 0°C, slowly added with sodium borohydride (130 mg, 3.42 mmol), and reacted at room temperature for 0.5 hours under nitrogen atmosphere. TLC showed that the starting material was completely reacted. The reaction mixture was slowly added with water (30 mL), and extracted with ethyl acetate (40 mL × 2). The organic phases were combined, washed with saturated brine (20 mL), dried over anhydrous sodium sulfate, filtered, and the filtrate was concentrated under reduced pressure to obtain compound **83-5** (500 mg, crude).

Step 6: Compound **83-5** (500 mg, 1.82 mmol) was dissolved in anhydrous dichloromethane (6 mL). The reaction mixture was cooled to 0°C, sequentially added with imidazole (390 mg, 5.72 mmol) and tert-butyldimethylsilyl chloride (610 mg, 4.06 mmol), and reacted at room temperature for 1 hour under nitrogen atmosphere. TLC showed that the starting material was completely reacted. The reaction mixture was added with water (40 mL), and extracted with dichloromethane (40 mL × 2). The organic phases were combined, washed with saturated brine (10 mL), dried over anhydrous sodium sulfate, and filtered. The filtrate was concentrated under reduced pressure and then purified by silica gel column chromatography (EA: PE = 1:25 to 1:15, Rf = 0.7 and Rf = 0.65) to obtain compounds **83-6a** (500 mg, Y: 70%, Rf = 0.65) and **83-6b** (30 mg, Y: 4.2%, Rf = 0.7). NOESY identified compound **83-6a** as being in the trans configuration and compound **83-6b** as being in the cis configuration. **83-6a:** ¹H NMR (400 MHz, CDCl₃): δ 7.36 (s, 1H), 5.38-5.32 (m, 1H), 5.21-5.04 (m, 1H), 3.31-3.03 (m, 2H), 0.94 (s, 9H), 0.20 (s, 6H). **83-6b:** ¹H NMR (400 MHz, CDCl₃): δ 7.38 (s, 1H), 5.41-5.37 (m, 1H), 5.22-5.06 (m, 1H), 3.50-3.37 (m, 1H), 3.10-2.99 (m, 1H), 0.89 (s, 9H), 0.22 (s, 3H), 0.17 (s, 3H).

Step 7: Compound **83-6a** (300 mg, 0.77 mmol) and compound **1-8** (420 mg, 0.77 mmol) were dissolved in toluene (6 mL) and water (1.2 mL), and tris(dibenzylideneacetone)dipalladium (80 mg, 0.09 mmol), 4,5-bis(diphenylphosphino)-9,9-dimethylxanthene (100 mg, 0.17 mmol), and anhydrous potassium phosphate (320 mg, 2.32 mmol) were sequentially added thereto. The reaction mixture was reacted at 110°C overnight under nitrogen atmosphere. TLC showed that the starting material was completely reacted. The reaction mixture was cooled to 25°C, concentrated under reduced pressure, and purified by silica gel column chromatography (EA: PE = 1:8) to obtain compound **83-7** (200 mg, Y: 35%). LC-MS m/z (ESI): 669.3/671.3 [M-tBu+H]⁺.

Step 8: Compound **83-7** (200 mg, 0.28 mmol) and anhydrous potassium carbonate (120 mg, 0.87 mmol) were dissolved in dimethyl sulfoxide (2 mL) and ethanol (4 mL). The reaction mixture was cooled to 10°C, slowly added dropwise with hydrogen peroxide (400 mg, 3.53 mmol, 30%), and reacted at room temperature for 1 hour. LCMS showed that the starting material was completely reacted. The reaction mixture was poured into water (20 mL), and extracted with ethyl acetate (20 mL × 2). The organic phases were combined, washed with saturated brine (10 mL), dried over anhydrous sodium sulfate, and filtered. The filtrate was concentrated under reduced pressure and then purified by reversed-phase silica gel column chromatography (C18, 40 g, 0.1% FA-MeCN, 90% to 100% MeCN in H₂O, flow rate: 40 mL/min: run time: 12 min; retention time: 8.0 min/9.2 min, where Rt = 9.2 min was for target product) to obtain compound **83-8** (70 mg, Y: 34%). LC-MS m/z (ESI): 743.3 [M+H]⁺.

Step 9: Compound **83-8** (70 mg, 0.09 mmol) was dissolved in hydrochloric acid/dioxane solution (2 mL, 4.0 M), and the reaction mixture was reacted at 25°C for 0.5 hours. LCMS showed that the starting material was completely reacted. The reaction mixture was concentrated under reduced pressure and purified by preparative high-performance liquid chromatography (column model: YMC-Actus Triart C18, 150 * 20 mm, 5 µm; eluent: 0.1% FA-ACN; gradient: 31 to 51%; flow rate: 20 mL/min; run time: 10 min; retention time: 7.2 min) to obtain compound **83a1** (29.1 mg, Y: 58%). ¹H NMR (400 MHz, DMSO-*d₆*): δ 8.22-8.14 (br, 1H), 7.71 (s, 1H), 7.46-7.44 (m, 2H), 7.38-7.25 (m, 5H), 7.03 (d, *J* = 9.6 Hz, 1H), 5.74 (br, 1H), 5.32-5.15 (m, 2H), 3.62-3.55 (m, 1H), 3.53-3.50 (m, 1H), 3.25-3.14 (m, 2H), 2.91-2.87 (m, 1H), 2.78-2.65 (m, 2H), 1.75-1.38 (m, 4H). LC-MS m/z (ESI): 529.2/531.3 [M+H]⁺. Chiral analysis (column model: Celluiose-SC, 100 * 4.6 mm, 5 µm; eluent: hexane (0.1% DEA)-IPA; gradient: isocratic 4/1, flow rate: 1 mL/min; run time: 12.5 min; retention time: 7.00 min).

### Example 84

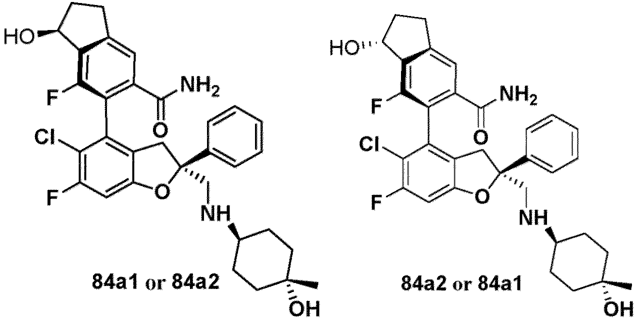

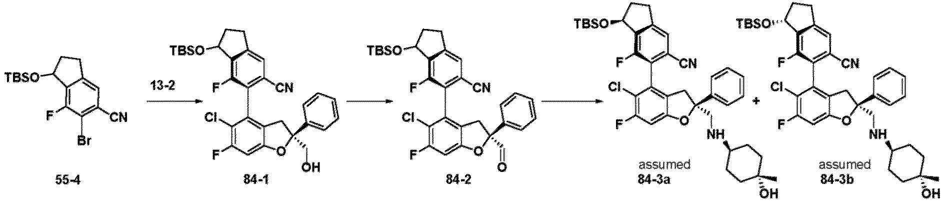

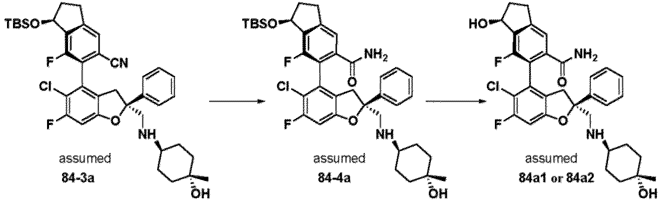

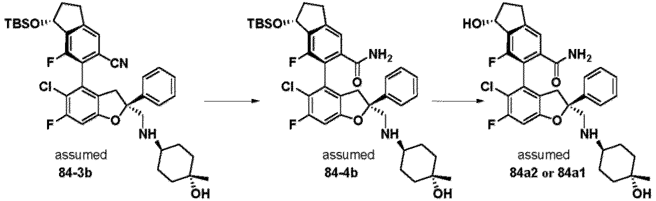

Step 1: Compound **55-4** (300 mg, 0.81 mmol) and compound **13-2** (327 mg, 0.81 mmol) were dissolved in toluene (6 mL) and water (1.2 mL), and tris(dibenzylideneacetone)dipalladium (75 mg, 0.08 mmol), 4,5-bis(diphenylphosphino)-9,9-dimethylxanthene (95 mg, 0.16 mmol), and anhydrous potassium phosphate (340 mg, 2.43 mmol) were sequentially added thereto. The reaction mixture was reacted at 110°C for 16 hours under nitrogen atmosphere. TLC showed that the starting material was completely reacted. The reaction mixture was cooled to 25°C, concentrated under reduced pressure, and purified by silica gel column chromatography (EA: PE = 1:15 to 1:5, Rf = 0.6, Rf = 0.5, where Rf = 0.5 was for target product) to obtain compound **84-1** (200 mg, Y: 43%). ¹H NMR (400 MHz, CDCl₃): δ 7.47 (s, 1H), 7.41-7.28 (m, 5H), 6.86 (d, *J* = 10.8 Hz, 1H), 5.52-5.43 (m, 1H), 3.92-3.80 (m, 2H), 3.56-3.48 (m, 1H), 3.29-3.17 (m, 1H), 3.13-3.02 (m, 1H), 2.96-2.86 (m, 1H), 2.50-2.37 (m, 1H), 2.16-2.06 (m, 1H), 2.01-1.92 (m, 1H), 0.86-0.84 (m, 9H), 0.11-0.07 (m, 6H). LC-MS (1.5 mL-3.2 min-5 to 95% B): Rt = 1.900, m/z (ESI): 590.2 [M+Na]⁺.

Step 2: Dimethyl sulfoxide (160 mg, 2.05 mmol) was dissolved in anhydrous dichloromethane (3 mL), and the reaction system was cooled to -78°C. The above reaction mixture was then slowly added dropwise with oxalyl chloride (135 mg, 1.06 mmol) dissolved in anhydrous dichloromethane (0.5 mL) at - 78°C and reacted at -78°C for 0.5 hours, slowly added dropwise with compound **84-1** (200 mg, 0.35 mmol) dissolved in anhydrous dichloromethane (0.5 mL) at -78°C and reacted at -78°C for 0.5 hours, slowly added dropwise with triethylamine (290 mg, 2.87 mmol) dissolved in dichloromethane (0.3 mL) at -78°C and reacted at -78°C for 0.5 hours, and naturally warmed to 25°C and stirred for another 2 hours. TLC showed that the starting material was completely reacted. The reaction mixture was added with water (20 mL), and extracted with dichloromethane (10 mL). The organic phase was washed with saturated brine (20 mL), dried over anhydrous sodium sulfate, filtered, and the filtrate was concentrated under reduced pressure to obtain compound **84-2** (170 mg, crude).

Step 3: Compound **84-2** (170 mg, 0.30 mmol) was dissolved in dichloroethane (5 mL), then trans-4-amino-1-methylcyclohexanol (40 mg, 0.31 mmol) and acetic acid (27 mg, 0.45 mmol) were added thereto, and the reaction mixture was reacted at 85°C for 2 hours. The reaction mixture was cooled to 25°C, added with sodium triacetoxyborohydride (127 mg, 0.60 mmol), and reacted at 60°C for 0.5 hours. LCMS showed that the starting material was completely reacted. The reaction mixture was cooled to 25°C, added with water (20 mL), and extracted with dichloromethane (30 mL × 2). The organic phase was washed with saturated brine (20 mL), dried over anhydrous sodium sulfate, and filtered. The filtrate was concentrated under reduced pressure and then purified by a silica gel plate (EA: PE = 1:2, Rf = 0.6, Rf = 0.5) to obtain compounds **84-3a** (30 mg, Y: 14%, Rf = 0.6) and **84-3b** (30 mg, Y: 14%, Rf = 0.5). **84-3a:** LC-MS (1.5 mL-2.0 min-5 to 90% B): Rt = 1.587, m/z (ESI): 679.2 [M+H]⁺. **84-3b:** LC-MS (1.5 mL-2.0 min-5 to 90% B): Rt = 1.650, m/z (ESI): 679.2 [M+H]⁺.

Step 4: Compound **84-3a** (48 mg, 0.07 mmol) and anhydrous potassium carbonate (30 mg, 0.22 mmol) were dissolved in dimethyl sulfoxide (1 mL). The reaction mixture was cooled to 10°C, slowly added dropwise with hydrogen peroxide (50 mg, 30%), and reacted at room temperature for 1 hour. LCMS showed that the starting material was completely reacted. The reaction mixture was poured into water (10 mL), and extracted with ethyl acetate (15 mL × 2). The organic phases were combined, washed with saturated brine (10 mL), dried over anhydrous sodium sulfate, filtered, and the filtrate was concentrated under reduced pressure to obtain compound **84-4a** (30 mg, crude). LC-MS m/z (ESI): 697.4 [M+H]⁺.

Step 5: Compound **84-4a** (30 mg, 0.04 mmol) was dissolved in anhydrous dichloromethane (1 mL), then trifluoroacetic acid (1 mL) was added thereto, and the reaction mixture was reacted at room temperature for 1 hour. LCMS showed that the starting material was completely reacted. The reaction mixture was poured into saturated sodium carbonate aqueous solution (50 mL), stirred for 10 minutes, and extracted with ethyl acetate (15 mL × 2). The organic phases were combined, washed with saturated brine (10 mL), dried over anhydrous sodium sulfate, and filtered. The filtrate was concentrated under reduced pressure and then purified by preparative high-performance liquid chromatography (column model: Xselect CSH Prep Fluoro-Phenyl, 150 * 20 mm, 5 µm; eluent: 0.1% FA-ACN; gradient: 48 to 68%; flow rate: 20 mL/min; run time: 12 min; retention time: 8.5 min) to obtain compound **84a1** (12.0 mg, Y: 47%). ¹H NMR (400 MHz, DMSO-*d₆*): δ 8.17 (s, 1H), 7.63 (s, 1H), 7.40-7.25 (m, 7H), 7.03 (d, *J* = 9.6 Hz, 1H), 5.38 (br, 1H), 5.28-5.20 (m, 1H), 3.20-3.08 (m, 1H), 2.92-2.75 (m, 4H), 2.40-2.22 (m, 2H), 1.98-1.91 (m, 1H), 1.65-1.56 (m, 2H), 1.42-1.35 (m, 2H), 1.22-1.16 (m, 2H),1.10-0.96 (m, 5H). LC-MS m/z (ESI): 583.3/585.3 [M+H]⁺. Chiral analysis (column model: Amylose-SA, 100 * 4.6 mm, 5 µm; eluent: hexane (0.1% DEA)-IPA; gradient: isocratic 4/1, flow rate: 1 mL/min; run time: 8.0 min; retention time: 3.56 min).

Step 6: Compound **84-3b** (48 mg, 0.07 mmol) and anhydrous potassium carbonate (30 mg, 0.22 mmol) were dissolved in dimethyl sulfoxide (1 mL). The reaction mixture was cooled to 10°C, slowly added dropwise with hydrogen peroxide (50 mg, 30%), and reacted at room temperature for 1 hour. TLC showed that the starting material was completely reacted. The reaction mixture was poured into water (10 mL), and extracted with ethyl acetate (15 mL × 2). The organic phases were combined, washed with saturated brine (10 mL), dried over anhydrous sodium sulfate, filtered, and the filtrate was concentrated under reduced pressure to obtain compound **84-4b** (30 mg, crude). LC-MS m/z (ESI): 697.4 [M+H]⁺.

Step 7: Compound **84-4b** (30 mg, 0.04 mmol) was dissolved in anhydrous dichloromethane (1 mL), then trifluoroacetic acid (1 mL) was added thereto, and the reaction mixture was reacted at room temperature for 1 hour. LCMS showed that the starting material was completely reacted. The reaction mixture was poured into saturated sodium carbonate aqueous solution (50 mL), stirred for 10 minutes, and extracted with ethyl acetate (15 mL × 2). The organic phases were combined, washed with saturated brine (10 mL), dried over anhydrous sodium sulfate, and filtered. The filtrate was concentrated under reduced pressure and then purified by preparative high-performance liquid chromatography (column model: Xselect CSH Prep Fluoro-Phenyl, 150 * 20 mm, 5 µm; eluent: 0.1% FA-CAN; gradient: 48 to 68%; flow rate: 20 mL/min; run time: 12 min; retention time: 9.1 min) to obtain compound **84a2** (11.9 mg, Y: 47%). ¹H NMR (400 MHz, DMSO-*d₆*): δ 8.26 (s, 1H), 7.63 (s, 1H), 7.40-7.25 (m, 7H), 7.03 (d, *J* = 9.6 Hz, 1H), 5.33 (br, 1H), 5.28-5.20 (m, 1H), 3.48-3.44 (m, 1H), 3.15-3.07 (m, 1H), 2.96-2.80 (m, 4H), 2.38-2.32 (m, 2H), 1.98-1.90 (m, 1H), 1.68-1.54 (m, 2H), 1.40-1.30 (m, 2H), 1.23-1.12 (m, 2H), 1.10-0.96 (m, 5H). LC-MS m/z (ESI): 583.2/585.4 [M+H]⁺. Chiral analysis (column model: Amylose-SA, 100 * 4.6 mm, 5 µm; eluent: hexane (0.1% DEA)-IPA; gradient: isocratic 4/1, flow rate: 1 mL/min; run time: 8.0 min; retention time: 3.40 min).

### Example 85

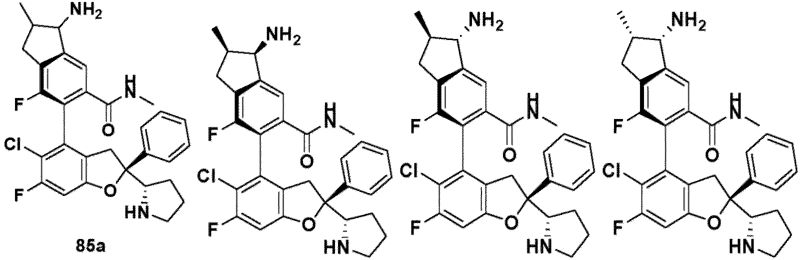

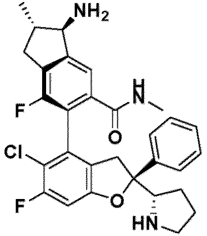

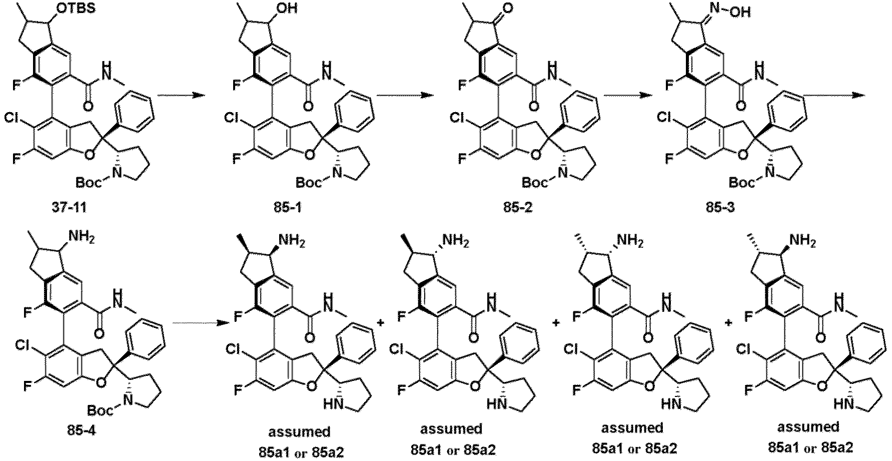

Step 1: Compound **37-11** (120 mg, 0.16 mmol) was dissolved in anhydrous tetrahydrofuran (3 mL), then tetrabutylammonium fluoride/tetrahydrofuran solution (0.4 mL, 1.0 M) was slowly added thereto, and the reaction mixture was reacted at room temperature for 0.5 hours under nitrogen atmosphere. TLC showed that the starting material was completely reacted. The reaction mixture was added with water (40 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (EA: PE = 1:1) to obtain compound **85-1** (100 mg, Y: 98%).

Step 2: Compound **85-1** (100 mg, 0.16 mmol) was dissolved in anhydrous dichloromethane (2 mL), and Dess-Martin periodinane (120 mg, 0.28 mmol) was added thereto. The reaction mixture was reacted at room temperature for 2 hours under nitrogen atmosphere. TLC showed that the starting material was completely reacted. The reaction mixture was slowly added with saturated sodium sulfite solution (20 mL), stirred at room temperature for 5 minutes, then added with saturated sodium bisulfate solution (20 mL), and extracted with dichloromethane (20 mL × 2). The organic phases were combined, washed with saturated brine (20 mL), dried over anhydrous sodium sulfate, and filtered. The filtrate was concentrated under reduced pressure and then purified by silica gel column chromatography (EA: PE = 1:1) to obtain compound **85-2** (90 mg, Y: 90%).

Step 3: Compound **85-2** (90 mg, 0.14 mmol) was dissolved in anhydrous ethanol (3 mL), then sodium acetate trihydrate (60 mg, 0.44 mmol) and hydroxylamine hydrochloride (20 mg, 0.29 mmol) were sequentially added thereto, and the reaction mixture was reacted at 50°C for 2 hours under nitrogen atmosphere. TLC showed that the starting material was completely reacted. The reaction mixture was added with water (20 mL), and extracted with ethyl acetate (20 mL × 3). The organic phases were combined, washed with saturated brine (10 mL), dried over anhydrous sodium sulfate, and filtered. The filtrate was concentrated under reduced pressure and then purified by silica gel column chromatography (EA: PE = 2:1) to obtain compound **85-3** (90 mg, Y: 97%). LC-MS m/z (ESI): 652.3 [M+H]⁺.

Step 4: Compound **85-3** (90 mg, 0.14 mmol) was dissolved in acetic acid (4 mL), then zinc powder solution (100 mg, 1.56 mmol) was added thereto, and the reaction mixture was reacted at 60°C for 2 hours under nitrogen atmosphere. LCMS detection was performed. The reaction mixture was cooled to 25°C, filtered through diatomite, and washed with ethyl acetate (20 mL). The filtrate was concentrated under reduced pressure and then purified by reversed-phase column chromatography (C18, 0.5% FA in H₂O, 70% to 90% MeCN in H₂O) to obtain compound **85-4** (55 mg, Y: 62%).

Step 5: Compound **85-4** (55 mg, 0.13 mmol) was dissolved in hydrochloric acid/dioxane solution (1 mL, 4.0 M), and the reaction mixture was reacted at 25°C for 0.5 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% HCl-CAN; gradient: 12 to 32% MeCN in H₂O; flow rate: 20 mL/min; run time: 9 min; retention time: 7.8 min/8.3 min) to obtain compound **85a1** (12.6 mg, Y: 12.7%, Rt = 7.8 min) and compound **85a2** (2.0 mg, Y: 2.0%, Rt = 8.3 min).

**85a1**: ¹H NMR (400 MHz, DMSO-*d₆*): δ 9.36 (br, 1H), 8.58 (br, 3H), 8.35 (br, 2H), 7.78 (s, 1H), 7.52-7.36 (m, 5H), 7.16 (d, *J* = 9.6 Hz, 1H), 4.67 (br, 1H), 4.44-4.32 (m, 1H), 3.64 (d, *J* = 16.8 Hz, 1H), 3.20-3.12 (m, 3H), 2.90-2.72 (m, 3H), 2.64 (d, *J* = 4.4 Hz, 3H), 2.22-2.12 (m, 1H), 2.00-1.50 (m, 3H), 1.15 (d, *J* = 6.8 Hz, 3H). LC-MS m/z (ESI): 538.3/540.3 [M+H]⁺. Chiral analysis (column model: Amylose-SA, 100 * 4.6 mm, 5 µm; eluent: hexane (0.1% DEA)-IPA; gradient: isocratic 4/1, flow rate: 1 mL/min; run time: 10.0 min; retention time: 3.45 min).

**85a2:** ¹H NMR (400 MHz, DMSO-*d₆*): δ 9.49 (br, 1H), 8.84 (br, 2H), 8.40-8.31 (br, 2H), 7.82 (s, 1H), 7.53-7.36 (m, 5H), 7.16 (d, *J* = 9.2 Hz, 1H), 4.42-4.32 (m, 2H), 3.63 (d, *J* = 16.4 Hz, 1H), 3.30-3.28 (m, 1H), 3.20-3.04 (m, 2H), 2.88 (d, *J* = 16.0 Hz, 1H), 2.63 (d, *J* = 4.4 Hz, 3H), 2.60-2.52 (m, 2H), 2.20-1.68 (m, 4H), 1.25 (d, *J* = 6.4 Hz, 3H). LC-MS m/z (ESI): 538.3/540.3 [M+H]⁺. Chiral analysis (column model: Amylose-SA, 100 * 4.6 mm, 5 µm; eluent: hexane (0.1% DEA)-IPA; gradient: isocratic 4/1, flow rate: 1 mL/min; run time: 10.0 min; retention time: 3.53 min).

### Example 86

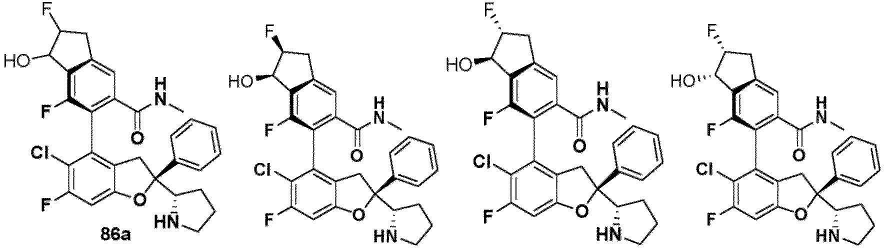

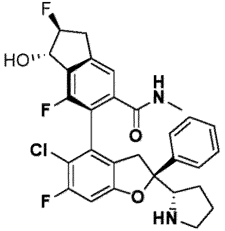

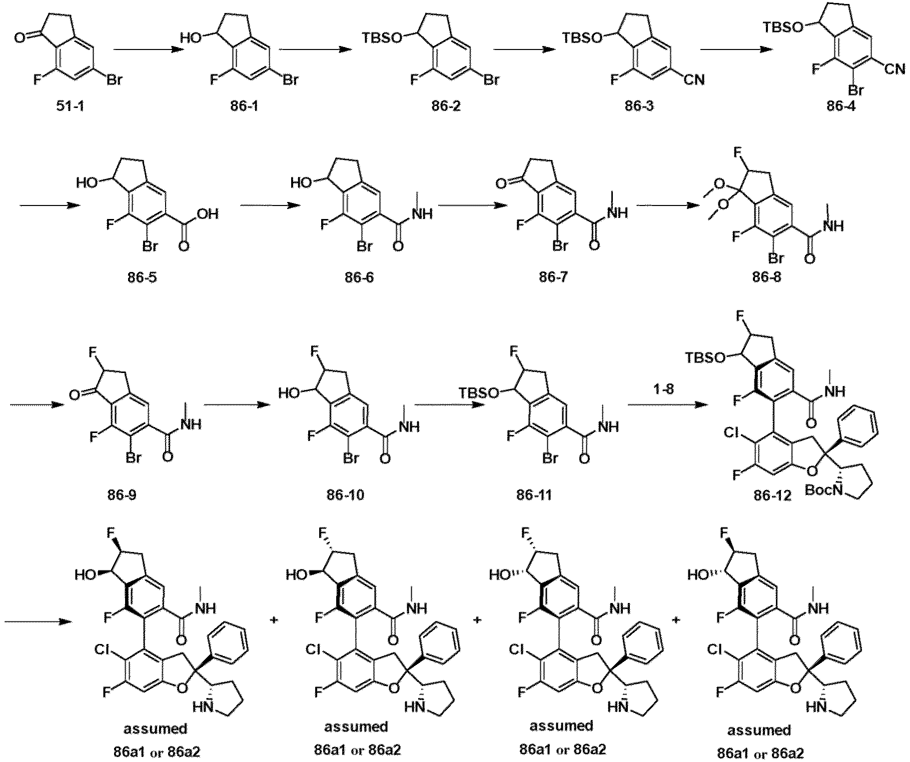

Step 1: **51-1** (15.0 g, 65.5 mmol) was dissolved in anhydrous tetrahydrofuran (160 mL). The reaction mixture was cooled to 0°C, and added with sodium borohydride (4.6 g, 122.1 mmol) in batches. The reaction mixture was reacted at room temperature for 1 hour. TLC showed that the starting material was completely reacted. The reaction mixture was slowly added with ice water (300 mL), and extracted with ethyl acetate (300 mL × 3). The organic phases were combined, washed with saturated brine (100 mL), dried over anhydrous sodium sulfate, filtered, and the filtrate was concentrated under reduced pressure to obtain compound **86-1** (14.8 g, Y: 98%). ¹H NMR (400 MHz, CDCl₃): δ 7.20 (s, 1H), 7.07 (d, *J* = 9.2, 1H), 5.47-5.44 (m, 1H), 3.18-3.10 (m, 1H), 2.87-2.79 (m, 1H), 2.49-2.40 (m, 1H), 2.11-2.05 (m, 1H).

Step 2: Compound **86-1** (14.8 g, 64.1 mmol) was dissolved in anhydrous dichloromethane (160 mL). The reaction mixture was cooled to 0°C, sequentially added with imidazole (13.1 g, 192.6 mmol) and tert-butyldimethylsilyl chloride (19.2 g, 128.0 mmol), and reacted at room temperature for 12 hours under nitrogen atmosphere. TLC showed that the starting material was completely reacted. The solid was filtered, and the filter cake was washed with dichloromethane (30 mL × 2). The filtrate was concentrated under reduced pressure and then purified by silica gel column chromatography (EA: PE = 1:50) to obtain compound **86-2** (16.0 g, Y: 72%). ¹H NMR (400 MHz, CDCl₃): δ 7.16 (s, 1H), 7.03 (d, *J* = 8.8, 1H), 5.40-5.37 (m, 1H), 3.15-3.07 (m, 1H), 2.81-2.73 (m, 1H), 2.38-2.29 (m, 1H), 2.05-1.97 (m, 1H), 0.89 (s, 9H), 0.14 (s, 3H), 0.12 (s, 3H).

Step 3: Compound **86-2** (13.0 g, 37.7 mmol) was dissolved in N,N-dimethylformamide (150 mL), and zinc cyanide (3.0 g, 25.6 mmol), tris(dibenzylideneacetone)dipalladium (2.8 g, 3.1 mmol), and 1,1'-bis(diphenylphosphino)ferrocene (3.4 g, 6.1 mmol) were sequentially added thereto. The reaction mixture was reacted at 100°C for 16 hours under nitrogen atmosphere. TLC showed that the starting material was completely reacted. The reaction mixture was cooled to 25°C, added with ice water (600 mL), and extracted with ethyl acetate (500 mL × 3). The organic phases were combined, washed with saturated brine (100 mL × 2), dried over anhydrous sodium sulfate, and filtered. The filtrate was concentrated under reduced pressure and then purified by silica gel column chromatography (EA: PE = 1:40) to obtain compound **86-3** (8.1 g, Y: 74%). ¹H NMR (400 MHz, CDCl₃): δ 7.31 (s, 1H), 7.15 (d, *J* = 8.4, 1H), 5.47-5.44 (m, 1H), 3.19-3.11 (m, 1H), 2.87-2.79 (m, 1H), 2.43-2.34 (m, 1H), 2.10-2.02 (m, 1H), 0.89 (s, 9H), 0.16 (s, 3H), 0.13 (s, 3H).

Step 4: Compound **86-3** (8.1 g, 27.8 mmol) was dissolved in anhydrous tetrahydrofuran (80 mL). The reaction system was cooled to -70°C, added dropwise with lithium diisopropylamide (15.3 mL, 2 M), and reacted at -70°C for 1 hour. The reaction mixture was slowly added dropwise with 1,2-dibromotetrafluoroethane (8.5 g, 32.8 mmol) dissolved in anhydrous tetrahydrofuran (7 mL) at -70°C, and reacted at -70°C for 1 hour. TLC showed that the starting material was completely reacted. The reaction mixture was slowly added with saturated ammonium chloride aqueous solution (200 mL) and water (100 mL), and extracted with ethyl acetate (300 mL × 3). The organic phases were combined, washed with saturated brine (80 mL × 2), dried over anhydrous sodium sulfate, and filtered. The filtrate was concentrated under reduced pressure and then purified by silica gel column chromatography (EA: PE = 1:30) to obtain compound **86-4** (7.7 g, Y: 74%). ¹H NMR (400 MHz, CDCl₃): δ 7.34 (s, 1H), 5.48-5.44 (m, 1H), 3.15-3.07 (m, 1H), 2.84-2.76 (m, 1H), 2.46-2.37 (m, 1H), 2.11-2.04 (m, 1H), 0.90 (s, 9H), 0.16 (s, 3H), 0.15 (s, 3H).

Step 5: Compound **86-4** (7.7 g, 20.8 mmol) was dissolved in ethanol (35 mL), and water (35 mL) and sodium hydroxide (8.3 g, 207.5 mmol) were added thereto. The reaction mixture was reacted at 100°C for 16 hours under nitrogen atmosphere. TLC showed that the starting material was completely reacted. The reaction mixture was cooled to 0°C, added with water (70 mL), and then slowly added with dilute hydrochloric acid (0.5 M) until the pH of the reaction system was 4 to 5. The reaction mixture was extracted with ethyl acetate (250 mL × 3). The organic phases were combined, washed with saturated brine (50 mL × 2), dried over anhydrous sodium sulfate, filtered, and the filtrate was concentrated under reduced pressure to obtain compound **86-5** (3.9 g, crude).

Step 6: Compound **86-5** (3.9 g, 14.2 mmol) was dissolved in N,N-dimethylformamide (40 mL), and 2-(7-azabenzotriazol-1-yl)-N,N,N',N'-tetramethyluronium hexafluorophosphate (6.4 g, 16.8 mmol), N,N-diisopropylethylamine (5.5 g, 42.6 mmol), and methylamine hydrochloride (1.5 g, 22.4 mmol) were sequentially added thereto. The reaction mixture was reacted at room temperature for 2 hours under nitrogen atmosphere. LCMS showed that the starting material was completely reacted. The reaction mixture was added with ice water (250 mL), and extracted with ethyl acetate (250 mL × 3). The organic phases were combined, washed with saturated brine (50 mL × 2), dried over anhydrous sodium sulfate, and filtered. The filtrate was concentrated under reduced pressure and then purified by silica gel column chromatography (EA: PE = 1:4) to obtain compound **86-6** (3.1 g, Y: 76%). LC-MS m/z (ESI): 288.1/290.1 [M+H]⁺.

Step 7: Compound **86-6** (1.6 g, 5.6 mmol) was dissolved in anhydrous dichloromethane (20 mL), then Dess-Martin periodinane (3.5 g, 8.3 mmol) was added thereto, and the reaction mixture was reacted at room temperature for 1 hour under nitrogen atmosphere. LCMS showed that the starting material was completely reacted. The reaction mixture was slowly added with saturated sodium thiosulfate aqueous solution (50 mL) and saturated sodium bicarbonate aqueous solution (50 mL), stirred for 0.5 hours, and extracted with dichloromethane (60 mL × 3). The organic phases were combined, washed with saturated brine (60 mL), dried over anhydrous sodium sulfate, and filtered. The filtrate was concentrated under reduced pressure and then purified by silica gel column chromatography (EA: PE = 1:5) to obtain compound **86-7** (1.3 g, Y: 82%). LC-MS m/z (ESI): 286.1/288.1 [M+H]⁺.

Step 8: **86-7** (1.3 g, 4.5 mmol) was dissolved in anhydrous methanol (15 mL), then 1-chloromethyl-4-fluoro- 1,4-diazabicyclo [2.2.2] octane bis(tetrafluoroborate) (1.9 g, 5.4 mmol) and concentrated sulfuric acid (68 mg, 0.69 mmol) were sequentially added thereto, and the reaction mixture was reacted at 60°C for 12 hours under nitrogen atmosphere. LCMS showed that the starting material was completely reacted. The reaction mixture was cooled to 25°C, then the solid was filtered, and the filter cake was washed with methanol (30 mL). The filtrate was directly concentrated under reduced pressure and then purified by silica gel column chromatography (EA: PE = 1:8) to obtain compound **86-8** (0.9 g, Y: 57%). LC-MS m/z (ESI): 350.2/352.2 [M+H]⁺.

Step 9: Compound **86-8** (0.9 g, 2.57 mmol) was dissolved in acetone (10 mL) and water (5 mL), then p-toluenesulfonic acid (540 mg, 3.14 mmol) was added thereto, and the reaction mixture was reacted at 65°C for 2 hours under nitrogen atmosphere. LCMS showed that the starting material was completely reacted. The reaction mixture was cooled to 25°C, added with saturated sodium bicarbonate aqueous solution (30 mL), and extracted with ethyl acetate (40 mL × 3). The organic phases were combined, washed with saturated brine (30 mL), dried over anhydrous sodium sulfate, filtered, and the filtrate was concentrated under reduced pressure to obtain crude compound **86-9** (550 mg, crude). LC-MS m/z (ESI): 304.1/306.1 [M+H]⁺.

Step 10: Compound **86-9** (550 mg, 1.64 mmol) was dissolved in tetrahydrofuran (6 mL). The reaction mixture was cooled to 0°C, slowly added with sodium borohydride (160 mg, 4.27 mmol), and reacted from 0°C to room temperature for 0.5 hours under nitrogen atmosphere. TLC showed that the starting material was completely reacted. The reaction mixture was slowly added with water (40 mL), and extracted with ethyl acetate (50 mL × 2). The organic phases were combined, washed with saturated brine (30 mL), dried over anhydrous sodium sulfate, filtered, and the filtrate was concentrated under reduced pressure to obtain crude compound **86-10** (480 mg, crude).

Step 11: Compound **86-10** (480 mg, 1.57 mmol) was dissolved in anhydrous dichloromethane (6 mL). The reaction mixture was cooled to 0°C, sequentially added with imidazole (390 mg, 5.72 mmol) and tert-butyldimethylsilyl chloride (610 mg, 4.06 mmol), and reacted at room temperature for 3 hours under nitrogen atmosphere. TLC showed that the starting material was completely reacted. The reaction mixture was added with water (40 mL), and extracted with dichloromethane (40 mL × 2). The organic phases were combined, washed with saturated brine (10 mL), dried over anhydrous sodium sulfate, and filtered. The filtrate was concentrated under reduced pressure and then purified by silica gel column chromatography (EA: PE = 1:15) to obtain compound **86-11** (520 mg, Y: 79%). ¹H NMR (400 MHz, CDCl₃): δ 7.18 (s, 1H), 5.89 (br, 1H), 5.32-5.29 (m, 1H), 5.17-4.99 (m, 1H), 3.27-3.04 (m, 2H), 3.01 (d, *J* = 4.8, 1H), 0.93 (s, 9H), 0.18 (s, 6H).

Step 12: Compound **86-11** (360 mg, 0.89 mmol) and compound **1-8** (389 mg, 0.72 mmol) were dissolved in toluene (8 mL) and water (1.6 mL), and tris(dibenzylideneacetone)dipalladium (80 mg, 0.09 mmol), 4,5-bis(diphenylphosphino)-9,9-dimethylxanthene (100 mg, 0.17 mmol), and anhydrous potassium phosphate (370 mg, 2.68 mmol) were sequentially added thereto. The reaction mixture was reacted at 110°C overnight under nitrogen atmosphere. TLC showed that the starting material was completely reacted. The reaction mixture was cooled to 25°C, concentrated under reduced pressure, and purified by silica gel column chromatography (EA: PE = 1:5 to 1:1, PE: EA = 5:1, Rf = 0.6, Rf = 0.5, where Rf = 0.6 was for target product) to obtain compound **86-12** (96 mg, Y: 14%). LC-MS (1.5 mL-5 min-5 to 95% B): Rt = 3.723, m/z (ESI): 757.3 [M+H]⁺.

Step 13: Compound **86-12** (96 mg, 0.13 mmol) was dissolved in hydrochloric acid/dioxane solution (2 mL, 4.0 M), and the reaction mixture was reacted at 25°C for 0.5 hours. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% FA-CAN; gradient: 435 to 56%; flow rate: 20 mL/min; run time: 12 min; retention time: 8.0 min/8.6 min) to obtain compounds **86a1** (22.5 mg, Y: 32.7%, Rt = 8.6 min) and **86a2** (4.1 mg, Y: 5.9%, Rt = 8.0 min).

**86a1:** ¹H NMR (400 MHz, DMSO-*d₆*): δ 8.23-8.16 (br, 2H), 7.58-7.25 (m, 6H), 7.01 (d, *J* = 9.6 Hz, 1H), 5.73 (br, 1H), 5.31-5.15 (m, 2H), 3.61-3.57 (m, 1H), 3.46 (d, *J* = 16.0 Hz, 1H), 3.25-3.14 (m, 2H), 2.90-2.67 (m, 3H), 2.62 (d, *J* = 4.8 Hz, 3H), 1.65-1.40 (m, 4H). LC-MS m/z (ESI): 543.2/545.3 [M+H]⁺. Chiral analysis (column model: Celluiose-SC, 100 * 4.6 mm, 5 µm; eluent: hexane (0.1% DEA)-IPA; gradient: isocratic 4/1, flow rate: 1 mL/min; run time: 12.5 min; retention time: 7.48 min).

**86a2:** ¹H NMR (400 MHz, DMSO-*d₆*): ¹H NMR (400 MHz, DMSO-*d₆*): δ 8.22-8.18 (br, 2H), 7.45-7.25 (m, 6H), 7.01 (d, *J* = 9.2 Hz, 1H), 5.31-5.16 (m, 2H), 3.60-3.52 (m, 1H), 3.45 (d, *J* = 15.2 Hz, 1H), 3.28-3.14 (m, 2H), 2.90-2.66 (m, 3H), 2.62 (d, *J* = 4.8 Hz, 3H), 1.60-1.39 (m, 4H). LC-MS m/z (ESI): 543.2/545.2 [M+H]⁺. Chiral analysis (column model: Celluiose-SC, 100 * 4.6 mm, 5 µm; eluent: hexane (0.1% DEA)-IPA; gradient: isocratic 4/1, flow rate: 1 mL/min; run time: 12.5 min; retention time: 8.86 min).

### Example 87

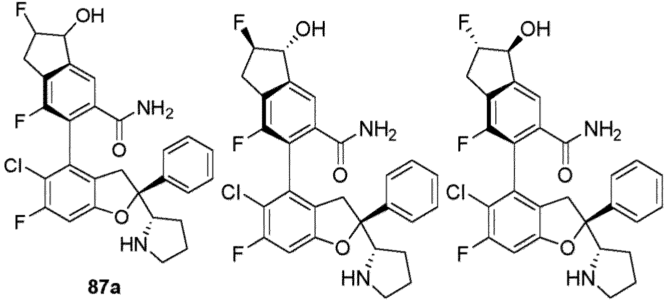

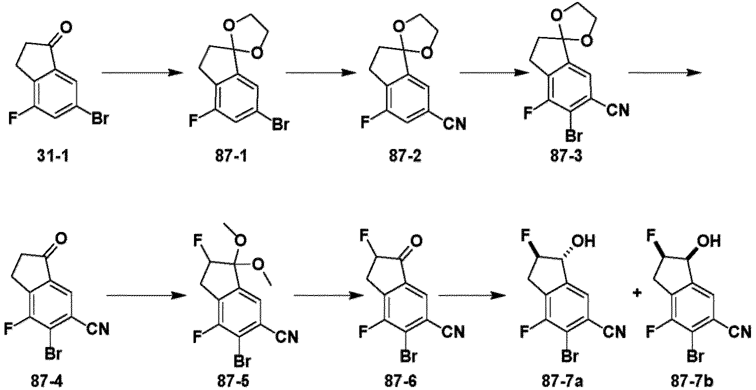

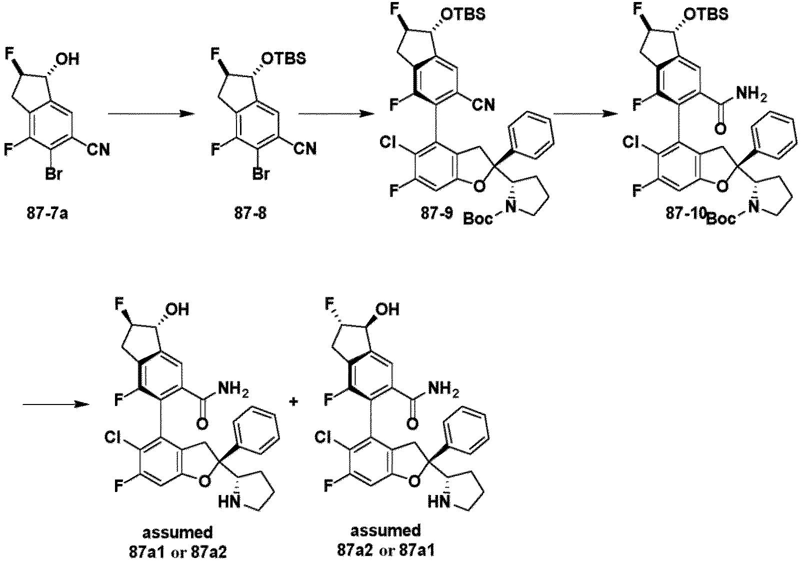

Step 1: Compound **31-1** (50.0 g, 219.3 mmol) was dissolved in toluene (600 mL), then ethylene glycol (40.7 g, 656.5 mmol) and p-toluenesulfonic acid (37.7 g, 219.2 mmol) were sequentially added thereto, and the reaction mixture was refluxed to remove water at 115°C for 72 hours. TLC showed that there was about 60% starting material and about 40% product. The reaction mixture was cooled to 25°C, concentrated under reduced pressure, and purified by silica gel column chromatography (EA: PE = 1:40) to obtain compound **87-1** (21.2 g, Y: 35%).

Step 2: Compound **87-1** (10.0 g, 36.8 mmol) was dissolved in N,N-dimethylformamide (120 mL), and zinc cyanide (2.6 g, 22.2 mmol), 1,1'-bis(diphenylphosphino)ferrocene (2.1 g, 3.8 mmol), and tris(dibenzylideneacetone)dipalladium (1.7 g, 1.9 mmol) were sequentially added thereto. The reaction mixture was reacted at 100°C for 16 hours under nitrogen atmosphere. TLC showed that the starting material was completely reacted. The reaction mixture was cooled to 25°C, poured into ice water (900 mL), and extracted with ethyl acetate (150 mL × 3). The organic phases were combined, washed with saturated brine (100 mL × 2), dried over anhydrous sodium sulfate, and filtered. The filtrate was concentrated under reduced pressure and then purified by silica gel column chromatography (EA: PE = 1:30) to obtain compound **87-2** (6.1 g, Y: 75%). ¹H NMR (400 MHz, DMSO-*d₆*): δ 7.82-7.76 (m, 2H), 4.19-4.15 (m, 2H), 4.04-3.99 (m, 2H), 2.97-2.93 (m, 2H), 2.30-2.26 (m, 2H).

Step 3: Compound **87-2** (6.0 g, 27.4 mmol) was dissolved in tetrahydrofuran (60 mL). The reaction mixture was cooled to -70°C, added dropwise with a solution of lithium diisopropylamide in tetrahydrofuran (16.4 mL, 32.8 mmol, 2 M) under nitrogen atmosphere, reacted at -70°C for 1 hour, then slowly added with 1,2-dibromotetrachloroethane (10.7 g, 32.9 mmol) in tetrahydrofuran (18 mL), and reacted at -70°C for 1 hour. TLC showed that the starting material was completely reacted. The reaction mixture was slowly added dropwise with saturated ammonium chloride solution (200 mL), and extracted with ethyl acetate (200 mL × 3). The organic phases were combined, washed with saturated brine (80 mL), dried over anhydrous sodium sulfate, and filtered. The filtrate was concentrated under reduced pressure and then purified by silica gel column chromatography (EA: PE = 1:30) to obtain compound **87-3** (2.1 g, Y: 26%). ¹H NMR (400 MHz, DMSO-*d₆*): δ 7.88 (s, 1H), 4.21-4.15 (m, 2H), 4.03-3.99 (m, 2H), 3.00-2.97 (m, 2H), 2.31-2.26 (m, 2H).

Step 4: Compound **87-3** (850 mg, 2.85 mmol) was dissolved in acetone (10 mL) and water (5 mL), then p-toluenesulfonic acid (540 mg, 3.14 mmol) was added thereto, and the reaction mixture was reacted at 60°C for 1 hour under nitrogen atmosphere. TLC showed that the starting material was completely reacted. The reaction mixture was directly concentrated under reduced pressure and then purified by silica gel column chromatography (EA: PE = 1:10) to obtain compound **87-4** (680 mg, Y: 93%).

Step 5: Compound **87-4** (680 mg, 2.68 mmol) was dissolved in anhydrous methanol (10 mL), then 1-chloromethyl-4-fluoro-1,4-diazabicyclo[2.22]octane bis(tetrafluoroborate) (1.0 g, 2.82 mmol) and concentrated sulfuric acid (52 mg, 0.53 mmol) were sequentially added thereto, and the reaction mixture was reacted at 60°C for 12 hours under nitrogen atmosphere. TLC showed that the starting material was completely reacted. The reaction mixture was cooled to 25°C, then the solid was filtered, and the filter cake was washed with methanol (20 mL). The filtrate was directly concentrated under reduced pressure and then purified by silica gel column chromatography (EA: PE = 1:15) to obtain compound **87-5** (710 mg, Y: 83%). ¹H NMR (400 MHz, CDCl₃): δ 7.53 (s, 1H), 5.24-5.10 (m, 1H), 3.49 (s, 3H), 3.39-3.24 (m, 1H), 3.19-3.14 (m, 4H).

Step 6: Compound **87-5** (710 mg, 2.23 mmol) was dissolved in acetone (6 mL) and water (3 mL), then p-toluenesulfonic acid (422 mg, 2.45 mmol) was added thereto, and the reaction mixture was reacted at 60°C for 1 hour under nitrogen atmosphere. TLC showed that the starting material was completely reacted. The reaction mixture was directly concentrated under reduced pressure and then purified by silica gel column chromatography (EA: PE = 1:10) to obtain compound **87-6** (510 mg, Y: 84%).

Step 4: Compound **87-6** (500 mg, 1.84 mmol) was dissolved in tetrahydrofuran (6 mL). The reaction mixture was cooled to 0°C, slowly added with solid sodium borohydride (140 mg, 3.68 mmol), and reacted at 0°C for 0.5 hours under nitrogen atmosphere. TLC showed that the starting material was completely reacted. The reaction mixture was slowly added with water (20 mL), and extracted with ethyl acetate (30 mL × 2). The organic phases were combined, washed with saturated brine (10 mL), dried over anhydrous sodium sulfate, and filtered. The filtrate was concentrated under reduced pressure and then purified by silica gel column chromatography (EA: PE = 1:20 to 1:5, Rf = 0.6, Rf = 0.4) to obtain compound **87-7a** (400 mg, Y: 79%, Rf = 0.4) and compound **87-7b** (60 mg, Y: 12%, Rf = 0.6). NOESY identified compound **87-7a** as being in the trans configuration and compound **87-7b** as being in the cis configuration. **87-7a:** ¹H NMR (400 MHz, CDCl₃): δ 7.59 (s, 1H), 5.45-5.29 (m, 1H), 5.20-5.08 (m, 1H), 3.46-3.35 (m, 1H), 3.25-3.08 (m, 1H), 2.65-2.53 (m, 1H). **87-7b:** ¹H NMR (400 MHz, CDCl₃): δ 7.55 (s, 1H), 5.35-5.12 (m, 2H), 3.60-3.48 (m, 1H), 3.25-3.08 (m, 1H), 2.62-2.54 (m, 1H).

Step 5: Compound **87-7a** (200 mg, 0.73 mmol) was dissolved in anhydrous dichloromethane (4 mL). The reaction mixture was cooled to 0°C, sequentially added with imidazole (150 mg, 2.19 mmol) and tert-butyldimethylsilyl chloride (220 mg, 1.46 mmol), and reacted at room temperature for 2 hours under nitrogen atmosphere. TLC showed that the starting material was completely reacted. The reaction mixture was added with water (10 mL), and extracted with ethyl acetate (20 mL × 3). The organic phases were combined, washed with saturated brine (10 mL), dried over anhydrous sodium sulfate, and filtered. The filtrate was concentrated under reduced pressure and then purified by silica gel column chromatography (EA: PE = 1:20) to obtain compound **87-8** (250 mg, Y: 88%). ¹H NMR (400 MHz, CDCl₃): δ 7.41 (s, 1H), 5.28-5.05 (m, 2H), 3.40-3.31 (m, 1H), 3.20-3.06 (m, 1H), 0.98-0.94 (m, 9H), 0.24-0.19 (m, 6H).

Step 6: Compound **87-8** (250 mg, 0.64 mmol) and compound **1-8** (350 mg, 0.64 mmol) were dissolved in toluene (6 mL) and water (1.2 mL), and tris(dibenzylideneacetone)dipalladium (80 mg, 0.09 mmol), 4,5-bis(diphenylphosphino)-9,9-dimethylxanthene (100 mg, 0.17 mmol), and anhydrous potassium phosphate (280 mg, 2.19 mmol) were sequentially added thereto. The reaction mixture was reacted at 110°C overnight under nitrogen atmosphere. TLC showed that the starting material was completely reacted. The reaction mixture was cooled to 25°C, concentrated under reduced pressure, and purified by silica gel column chromatography (EA: PE = 1:5 to 1:1, Rf = 0.6 and Rf = 0.5, where Rf = 0.5 was for target product) to obtain compound **87-9** (160 mg, Y: 34%, Rf = 0.5). LC-MS (1.5 mL-5 min-5 to 95% B): Rt = 3.74 min, m/z (ESI): 669.5 [M-56+H]⁺.

Step 7: Compound **87-9** (160 mg, 0.22 mmol) and potassium carbonate (90 mg, 0.65 mmol) were dissolved in dimethyl sulfoxide (6 mL) and ethanol (6 mL). The reaction mixture was cooled to 10°C, slowly added dropwise with hydrogen peroxide (599 mg, 5.28 mmol, 30%), and reacted at 25°C for 1 hour. LCMS showed that the starting material was completely reacted. The reaction mixture was poured into water (30 mL), and extracted with ethyl acetate (30 mL × 2). The organic phases were combined, washed with saturated brine (30 mL), dried over anhydrous sodium sulfate, filtered, and the filtrate was concentrated under reduced pressure to obtain compound **87-10** (150 mg, crude). LC-MS m/z (ESI): 743.4 [M+H]⁺.

Step 8: Compound **87-10** (140 mg, 0.19 mmol) was dissolved in hydrochloric acid/dioxane solution (3 mL, 4.0 M), and the reaction mixture was reacted at 25°C for 1 hour. LCMS showed that the starting material was completely reacted. The reaction mixture was concentrated under reduced pressure and purified by preparative high-performance liquid chromatography (column model: Xselect CSH Prep Fluoro-Phenyl, 150 * 19 mm, 5 µm; eluent: 0.1% HCl-CAN; gradient: 21 to 41%; flow rate: 20 mL/min; run time: 12 min; retention time: 7.2 min/8.5 min) to obtain compounds **87a1** (18.6 mg, Y: 15%, Rt = 7.2 min) and **87a2** (46.7 mg, Y: 38%, Rt = 8.5 min).

**87a1:** ¹H NMR (400 MHz, DMSO-*d₆*): δ 9.36 (br, 1H), 8.41 (br, 1H), 7.79 (s, 1H), 7.55-7.50 (m, 2H), 7.47-7.32 (m, 4H), 7.12-7.09 (m, 1H), 5.42-5.28 (m, 1H), 5.20-5.10 (m, 1H), 4.24-4.16 (m, 1H), 3.50-3.47 (m, 1H), 3.32-3.05 (m, 5H), 2.06-1.58 (m, 4H). LC-MS m/z (ESI): 529.2/531.2 [M+H]⁺. Chiral analysis (column model: Amylose-C, 100 * 4.6 mm, 5 µm; eluent: hexane (0.1% DEA)-IPA; gradient: isocratic 4/1, flow rate: 1 mL/min; run time: 12 min; retention time: 5.91 min).

**87a2:** ¹H NMR (400 MHz, DMSO-*d₆*): δ 9.43 (br, 1H), 8.29 (br, 1H), 7.93 (s, 1H), 7.55-7.34 (m, 7H), 7.13 (d, *J* = 9.2 Hz, 1H), 5.38-5.21 (m, 1H), 5.18-5.10 (m, 1H), 4.30-4.23 (m, 1H), 3.64-3.59 (m, 2H), 3.24-3.05 (m, 4H), 2.92-2.88 (m, 1H), 2.19-2.13 (m, 1H), 1.95-1.70 (m, 3H). LC-MS m/z (ESI): 529.3/531.3 [M+H]⁺. Chiral analysis (column model: Amylose-C, 100 * 4.6 mm, 5 µm; eluent: hexane (0.1% DEA)-IPA; gradient: isocratic 4/1, flow rate: 1 mL/min; run time: 12 min; retention time: 4.72 min).

### Example 88

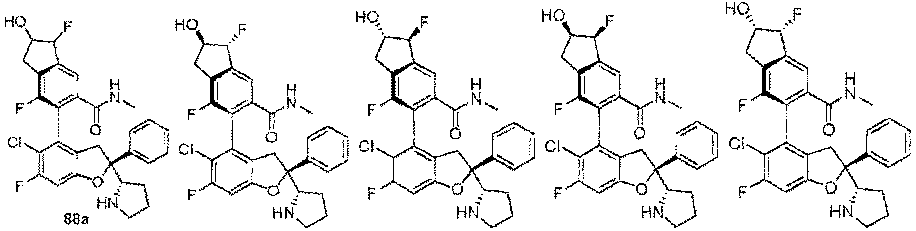

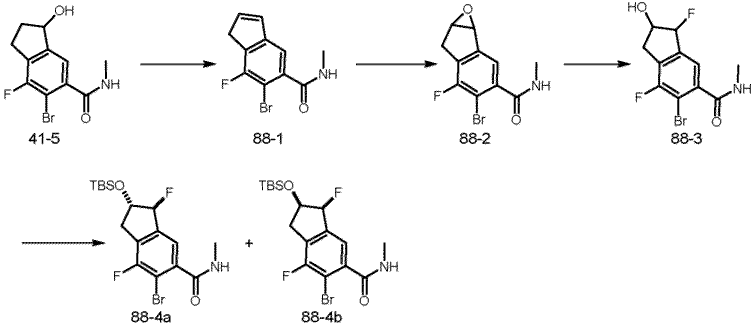

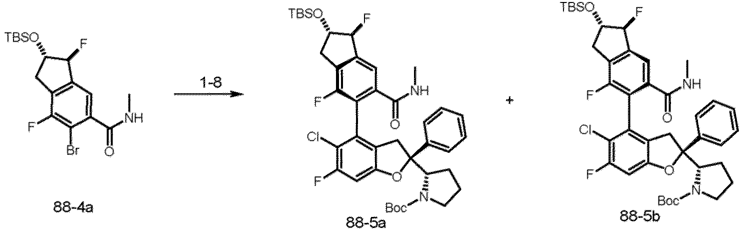

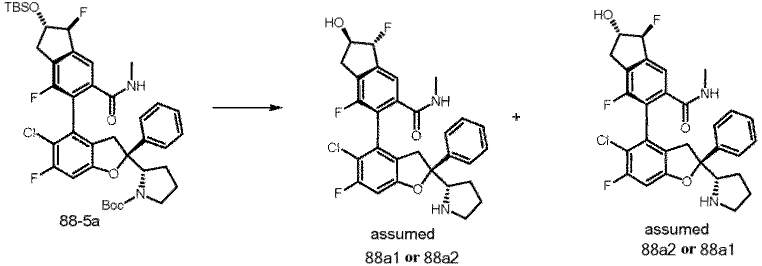

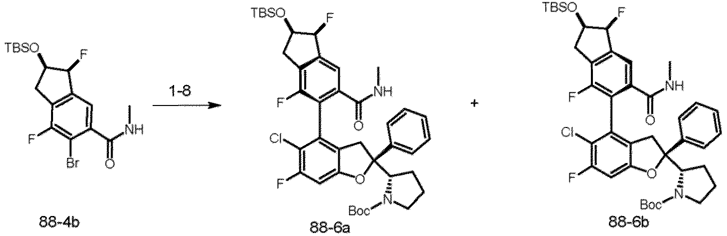

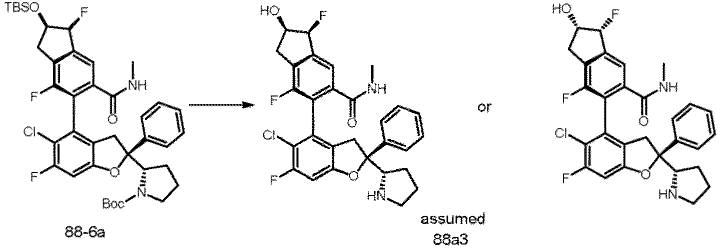

Step 1: Compound **41-5** (1.0 g, 3.47 mmol) was dissolved in toluene (10.0 mL), then anhydrous p-toluenesulfonic acid (36.2 mg, 0.67 mmol) was added thereto under nitrogen atmosphere, and the reaction mixture was reacted at 90°C for 16 hours. TLC (PE: EA = 1:1) showed that the starting material was completely reacted. The reaction mixture was cooled to 0°C, poured into saturated sodium bicarbonate (50 mL) to quench, and extracted with ethyl acetate (50 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 10:1) to obtain compound **88-1** (810 mg, Y: 43%). LC-MS m/z (ESI): 270.0 [M+H]⁺.

Step 2: Compound **88-1** (500 mg, 1.85 mmol) was dissolved in anhydrous dichloromethane (10 mL), then 3-chloroperoxybenzoic acid (638 mg, 3.70 mmol) was added thereto under nitrogen atmosphere, and the reaction mixture was reacted at room temperature for 16 hours. TLC (PE: EA = 5:1) showed that the starting material was completely reacted. The reaction mixture was poured into saturated sodium bicarbonate (20 mL) to quench, and extracted with ethyl acetate (10 mL × 3). The organic phases were combined, washed with saturated brine (20 mL), dried over anhydrous sodium sulfate, and filtered. The filtrate was concentrated under reduced pressure and then purified by silicagel column chromatography (PE: EA= 10:1) to obtain compound **88-2** (430 mg, Y: 81%). LC-MS m/z (ESI): 286.0 [M+H]⁺.

Step 3: Compound **88-2** (400 mg, 1.4 mmol) was dissolved in triethylamine trihydrofluoride (10 mL), and the reaction mixture was reacted at room temperature for 5 hours. TLC (PE: EA = 1:1) showed that the starting material was completely reacted. The reaction mixture was poured into saturated sodium bicarbonate (20 mL) to quench, and extracted with ethyl acetate (10 mL × 3). The organic phases were combined, washed with saturated brine (20 mL), dried over anhydrous sodium sulfate, filtered, and the filtrate was concentrated under reduced pressure to obtain compound **88-3** (400 mg, Y: 93%). LC-MS m/z (ESI): 306.0 [M+H]⁺.

Step 4: Compound **88-3** (400 mg, 1.31 mmol) was dissolved in anhydrous dichloromethane (10 mL), then compound tert-butyldimethylsilyl chloride (394 mg, 2.61 mmol) and imidazole (267 mg, 3.92 mmol) were sequentially added thereto under nitrogen atmosphere, and the reaction mixture was reacted at room temperature for 2 hours. TLC (PE: EA = 1:1) showed that the starting material was completely reacted. The reaction mixture was poured into saturated ammonium chloride (50 mL), and extracted with ethyl acetate (30 mL × 3). The organic phases were combined, washed with saturated brine (50 mL), dried over anhydrous sodium sulfate, and filtered. The filtrate was concentrated under reduced pressure and then purified by silica gel column chromatography (PE: EA = 10:1 to 1:1) to obtain compounds **86-4a** (200 mg, Y: 36%, Rf = 0.35) and **86-4b** (100 mg, Y: 18%, Rf = 0.30). **86-4a:** ¹H NMR (400 MHz, DMSO-*d*₆) δ 7.36 (s, 1H), 5.73 (d, *J* = 4.4 Hz, 0.5H), 5.59 (d, *J* = 4.4 Hz, 0.5H), 4.69-4.58 (m, 1H), 3.41-3.32 (m, 1H), 3.03 (d, *J* = 5.2 Hz, 3H), 2.86-2.77 (m, 1H), 0.91 (s, 9H), 0.14 (d, *J* = 5.2 Hz, 6H). LC-MS (1.5 mL-2 min-5 to 95% B): Rt = 1.483, m/z (ESI): 420.1/422.0 [M+H]⁺. **86-4b:** ¹H NMR (400 MHz, DMSO-*d*₆) δ 7.42 (s, 1H), 5.55 (d, *J* = 3.6 Hz, 0.5H), 5.41 (d, *J* = 5.6 Hz, 0.5H), 4.54-4.44 (m, 1H), 3.32-3.15 (m, 1H), 3.09-3.01 (m, 4H), 0.93 (s, 9H), 0.15 (d, *J* = 5.2 Hz, 6H). LC-MS (1.5 mL-2 min-5 to 95% B): Rt = 1.443, m/z (ESI): 420.1/422.0 [M+H]⁺. NOESY identified compound **86-4a** as being in the trans configuration and **86-4b** as being in the cis configuration.

Step 5: Compound **88-4a** (200 mg, 0.47 mmol) was dissolved in toluene (1 mL) and water (0.2 mL), then compound **1-8** (388 mg, 0.71 mmol), tris(dibenzylideneacetone)dipalladium (43.5 mg, 0.045 mmol), 4,5-bis(diphenylphosphino)-9,9-dimethylxanthene (49.2 mg, 0.09 mmol), and anhydrous potassium carbonate (302 mg, 1.43 mmol) were sequentially added thereto under nitrogen atmosphere, and the reaction mixture was reacted at 110°C for 16 hours. TLC (PE: EA = 1:1) showed that the starting material was completely reacted. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compounds **88-5a** (60.00 mg, Y: 16%, R_{f} = 0.30) and **88-5b** (100 mg, Y: 27%, R_{f} = 0.35). **88-5a:** LC-MS (1.5 mL-5 min-5 to 90% B): Rt = 3.600, m/z(ESI): 757.1 [M+H]⁺. **88-5b:** LC-MS (1.5 mL-5 min-5 to 90% B): Rt = 3.523, m/z (ESI): 757.1 [M+H]⁺.

Step 6: Compound **88-5a** (60 mg, 0.081 mmol) was dissolved in dichloromethane (1 mL). The reaction mixture was cooled to 0°C under nitrogen atmosphere, added with trifluoroacetic acid (1 mL), warmed to room temperature, and stirred for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: Xselect CSH Prep Fluoro-Phenyl, 150 * 19 mm, 5 µm; eluent: (0.1% FA) H₂O-ACN; gradient: 26.0% to 36.0%; flow rate: 20 mL/min; run time: 9 min) to obtain compound **88a1** (8.5 mg, Y: 19%, retention time: 7.3 min) and compound **88a2** (15.6 mg, Y: 35%, retention time: 8.2 min). **88a1:** ¹H NMR (400 MHz, DMSO-*d*₆) δ 8.32-8.26 (m, 1H), 8.16 (s, 1H), 7.56 (s, 1H), 7.47-7.25 (m, 5H), 7.03 (d, *J* = 9.6 Hz, 1H), 5.92-5.87 (m, 1H), 5.77-5.71 (m, 1H), 4.63-4.50 (m, 1H), 3.53-3.40 (m, 3H), 2.93-2.68 (m, 4H), 2.63 (d, *J* = 4.8 Hz, 3H), 1.68-1.38 (m, 4H). LC-MS m/z (ESI): 543.3 [M+H]⁺. Chiral analysis (column model: Amylose-C, 100 * 4.6 mm, 3 µm; eluent: hexane (0.1% DEA)/IPA; gradient: isocratic 8/2; flow rate: 1 mL/min; run time: 9 min; retention time: 2.09 min). **88a2:** ¹H NMR (400 MHz, DMSO-*d*₆) δ 8.32-8.26 (m, 1H), 8.17 (s, 1H), 7.56 (s, 1H), 7.47-7.25 (m, 5H), 7.03 (d, *J* = 10.0 Hz, 1H), 5.91-5.86 (m, 1H), 5.77-5.71 (m, 1H), 4.63-4.50 (m, 1H), 3.64-3.40 (m, 3H), 2.91-2.68 (m, 4H), 2.63 (d, *J* = 4.4 Hz, 3H), 1.68-1.35 (m, 4H). LC-MS m/z (ESI): 543.2 [M+H]⁺. Chiral analysis (column model: Amylose-C, 100 * 4.6 mm, 3 µm; eluent: hexane (0.1% DEA)/IPA; gradient: isocratic 8/2; flow rate: 1 mL/min; run time: 9 min; retention time: 4.03 min).

Step 7: Compound **88-4b** (100 mg, 0.24 mmol) was dissolved in toluene (1 mL) and water (0.2 mL), then compound **1-8** (194 mg, 0.36 mmol), tris(dibenzylideneacetone)dipalladium (21.7 mg, 0.023 mmol), 4,5-bis(diphenylphosphino)-9,9-dimethylxanthene (24.6 mg, 0.045 mmol), and anhydrous potassium carbonate (151 mg, 0.72 mmol) were sequentially added thereto under nitrogen atmosphere, and the reaction mixture was reacted at 110°C for 16 hours. LCMS showed that the starting material was completely reacted. The reaction mixture was cooled to 25°C, poured into saturated ammonium chloride (10 mL), and extracted with ethyl acetate (20 mL × 3). The organic phases were combined, washed with saturated brine (20 mL), dried over anhydrous sodium sulfate, and filtered. The filtrate was concentrated under reduced pressure and then purified by silica gel column chromatography (PE: EA = 1:1) to obtain compounds **88-6a** (20.0 mg, Y: 11%, R_{f} = 0.30) and **88-6b** (15 mg, Y: 8.3%, R_{f} = 0.35). **88-6a:** LC-MS (1.5 mL-5 min-5 to 90% B): Rt = 3.680, m/z (ESI): 757.1 [M+H]⁺. **88-6b:** LC-MS (1.5 mL-5 min-5 to 90% B): Rt = 3.647, m/z (ESI): 757.1 [M+H]⁺.

Step 8: Compound **88-6a** (20 mg, 0.026 mmol) was dissolved in dichloromethane (1 mL). The reaction mixture was cooled to 0°C under nitrogen atmosphere, added with trifluoroacetic acid (1 mL), warmed to room temperature, and reacted for 2 hours. LCMS showed that the starting material was completely reacted. The reaction mixture was cooled to 0°C, poured into saturated sodium carbonate (20 mL) to quench, and extracted with ethyl acetate (20 mL × 3). The organic phases were combined, washed with saturated brine (20 mL), dried over anhydrous sodium sulfate, and filtered. The filtrate was concentrated under reduced pressure to obtain a yellow oil, which was then purified by preparative high-performance liquid chromatography (column model: Xselect CSH Prep Fluoro-Phenyl, 150 * 19 mm, 5 µm; eluent: (0.1% FA) H₂O-ACN; gradient: 32.0% to 52.0%; flow rate: 20 mL/min; run time: 8 min, retention time: 7.2 min) to obtain compound **88a3** (9.7 mg, Y: 67%). ¹H NMR (400 MHz, DMSO-*d*₆) δ 8.29-8.24 (m, 1H), 8.17 (s, 1H), 7.60 (s, 1H), 7.44-7.24 (m, 5H), 7.03 (d, *J* = 9.6 Hz, 1H), 5.82-5.64 (m, 1H), 5.61-5.52 (m, 1H), 4.52-4.39 (m, 1H), 3.60-3.39 (m, 3H), 3.20-3.11 (m, 1H), 2.88-2.70 (m, 3H), 2.63 (d, *J* = 4.8 Hz, 3H), 1.66-1.32 (m, 4H). LC-MS m/z (ESI): 543.2 [M+H]⁺. Chiral analysis (column model: Amylose-C, 100 * 4.6 mm, 3 µm; eluent: hexane (0.1% DEA)/IPA; gradient: isocratic 8/2; flow rate: 1 mL/min; run time: 6 min; retention time: 2.92 min).

### Example 89

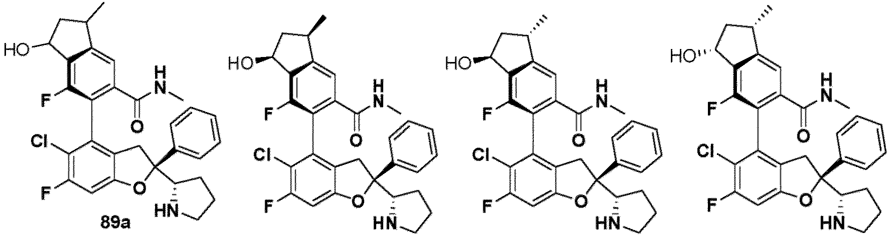

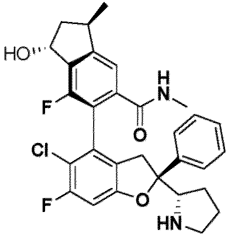

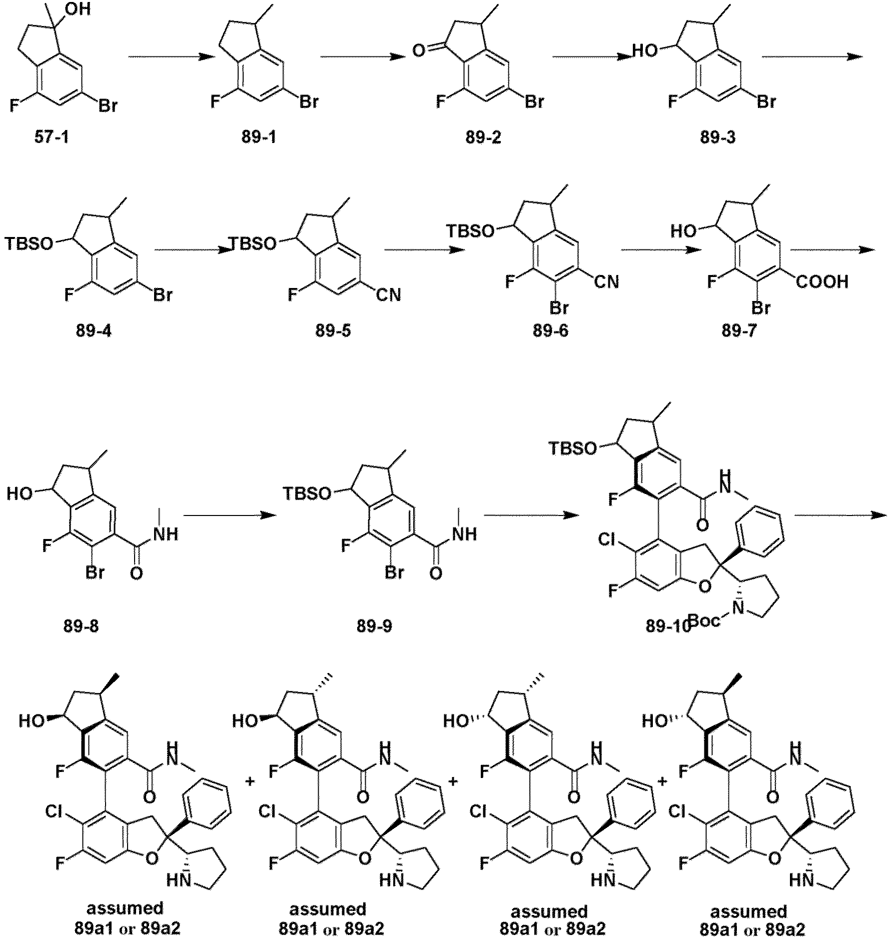

Step 1: Compound **57-1** (20.0 g, 81.6 mmol) was dissolved in dichloromethane (200 mL). The reaction mixture was cooled to 0°C under nitrogen atmosphere, sequentially added with triethylsilane (57.9 g, 408.0 mmol) and boron trifluoride diethyl etherate (48% wt, 37.8 g, 163.2 mmol), and the reaction mixture was reacted at room temperature for 12 hours under nitrogen atmosphere. TLC showed that the starting material was completely reacted. The reaction mixture was slowly poured into saturated sodium bicarbonate solution (400 mL) to quench, and extracted with dichloromethane (300 mL × 2). The organic phases were combined, washed with saturated brine (200 mL), dried over anhydrous sodium sulfate, and filtered. The filtrate was concentrated under reduced pressure and then purified by silica gel column chromatography (EA: PE = 1:50) to obtain compound **89-1** (16.0 g, Y: 85%).

Step 2: Compound **89-1** (16.0 g, 69.8 mmol) was dissolved in acetonitrile (200 mL) and pure water (100 mL), then copper(II) sulfate pentahydrate (16.7 g, 104.7 mmol) and potassium persulfate (56.6 g, 209.4 mmol) were sequentially added thereto, and the reaction mixture was reacted at 80°C for 16 hours under nitrogen atmosphere. TLC showed that the starting material was completely reacted. The reaction mixture was poured into water (400 mL), and extracted with ethyl acetate (200 mL × 2). The organic phases were combined, washed with saturated brine (300 mL), dried over anhydrous sodium sulfate, and filtered. The filtrate was concentrated under reduced pressure and then purified by silica gel column chromatography (EA: PE = 1:20) to obtain compound **89-2** (4.6 g, Y: 27%). ¹H NMR (400 MHz, CDCl₃) δ 7.48-7.42 (m, 1H), 7.22-7.14 (m, 1H), 3.47-3.39 (m, 1H), 2.98-2.87 (m, 1H), 2.34-2.21 (m, 1H), 1.45-1.34 (m, 3H).

Step 3: Compound **89-2** (4.6 g, 19.0 mmol) was dissolved in methanol (50 mL). The reaction mixture was cooled to -70°C, and added with sodium borohydride (1.0 g, 26.3 mmol) in batches. The reaction mixture was reacted at room temperature for 2 hours. TLC showed that the starting material was completely reacted. The reaction mixture was slowly added with ice water (200 mL), and extracted with ethyl acetate (150 mL × 3). The organic phases were combined, washed with saturated brine (80 mL), dried over anhydrous sodium sulfate, filtered, and the filtrate was concentrated under reduced pressure to obtain compound **89-3** (4.2 g, crude).

Step 4: Compound **89-3** (4.0 g, 16.4 mmol) was dissolved in dichloromethane (50 mL), and imidazole (3.4 g, 50.0 mmol) and tert-butyldimethylsilyl chloride (3.7 g, 24.7 mmol) were sequentially added thereto. The reaction mixture was reacted at room temperature for 12 hours under nitrogen atmosphere. TLC showed that the starting material was completely reacted. The reaction mixture was concentrated under reduced pressure and then purified by silica gel column chromatography (EA: PE = 1:40) to obtain compound **89-4** (4.1 g Y: 70%).

Step 5: Compound **89-4** (4.0 g, 11.2 mmol) was dissolved in anhydrous N,N-dimethylformamide (45 mL), and zinc cyanide (0.9 g, 7.7 mmol), 1,1'-bis(diphenylphosphino)ferrocene (1.2 g, 2.2 mmol), and tris(dibenzylideneacetone)dipalladium (1.0 g, 1.1 mmol) were sequentially added thereto. The reaction mixture was reacted at 100°C for 16 hours under nitrogen atmosphere. TLC showed that the starting material was completely reacted. The reaction mixture was cooled to 25°C, poured into ice water (200 mL), and extracted with ethyl acetate (200 mL × 2). The organic phases were combined, washed with saturated brine (50 mL × 2), dried over anhydrous sodium sulfate, and filtered. The filtrate was concentrated under reduced pressure and then purified by silica gel column chromatography (EA: PE = 1:30) to obtain compound **89-5** (2.2 g, Y: 64%). ¹H NMR (400 MHz, CDCl₃): δ 7.28 (s, 1H), 7.16 (d, *J* = 8.8 Hz, 1H), 5.42-5.32 (m, 1H), 3.10-3.02 (m, 1H), 2.68-2.61 (m, 1H), 1.72-1.62 (m, 1H), 1.36 (d, *J* = 6.8 Hz, 3H), 0.91 (s, 9H), 0.15 (m, 6H).

Step 6: Compound **89-5** (2.2 g, 7.2 mmol) was dissolved in tetrahydrofuran (15 mL). The reaction mixture was cooled to -70°C, added dropwise with a solution of lithium diisopropylamide in tetrahydrofuran (4.3 mL, 8.6 mmol, 2 M) under nitrogen atmosphere, reacted at -70°C for 1 hour, then added dropwise with 1,2-dibromotetrafluoroethane (2.3 g, 8.9 mmol) in tetrahydrofuran (2 mL), and reacted at - 70°C for 1 hour. TLC showed that the starting material was completely reacted. The reaction mixture was slowly added dropwise with saturated ammonium chloride solution (100 mL), and extracted with ethyl acetate (100 mL × 3). The organic phases were combined, washed with saturated brine (100 mL), dried over anhydrous sodium sulfate, and filtered. The filtrate was concentrated under reduced pressure and then purified by silica gel column chromatography (EA: PE = 1:30) to obtain compound **89-6** (2.1 g, Y: 76%). ¹H NMR (400 MHz, CDCl₃): δ 7.30 (s, 1H), 5.48-5.32 (m, 1H), 3.10-3.02 (m, 1H), 2.69-2.61 (m, 1H), 1.75-1.68 (m, 1H), 1.36 (d, *J* = 6.8 Hz, 3H), 0.91 (s, 9H), 0.16 (m, 6H).

Step 7: Compound **89-6** (1.1 g, 2.9 mmol) was dissolved in ethanol (4 mL) and water (4 mL), then sodium hydroxide (0.9 g, 22.5 mmol) was added thereto, and the reaction mixture was reacted at 100°C for 16 hours. TLC showed that the starting material was completely reacted. The reaction mixture was cooled to 0°C, added with water (15 mL), and then slowly added with dilute hydrochloric acid (0.5 M) until the pH of the reaction system was 3 to 4. The reaction mixture was extracted with ethyl acetate (60 mL × 3). The organic phases were combined, washed with saturated brine (30 mL), dried over anhydrous sodium sulfate, filtered, and the filtrate was concentrated under reduced pressure to obtain compound **89-7** (600 mg, crude).

Step 8: Compound **89-7** (0.6 g, 2.1 mmol) was dissolved in N,N-dimethylformamide (8 mL), then diisopropylethylamine (1.0 g, 7.8 mmol), 2-(7-azabenzotriazol-1-yl)-N,N,N',N'-tetramethyluronium hexafluorophosphate (1.0 g, 2.6 mmol), and methylamine hydrochloride (0.2 g, 2.9 mmol) were sequentially added thereto, and the reaction mixture was reacted at room temperature for 2 hours under nitrogen atmosphere. LCMS showed that the starting material was completely reacted. The reaction mixture was added with water (20 mL), and extracted with ethyl acetate (20 mL × 2). The organic phases were combined, washed with saturated brine (20 mL), dried over anhydrous sodium sulfate, and filtered. The filtrate was concentrated under reduced pressure and then purified by silica gel column chromatography (EA: PE = 1:1) to obtain compound **89-8** (450 mg, Y: 75%). LC-MS m/z (ESI): 302.2/304.2 [M+H]⁺.

Step 9: Compound **89-8** (450 mg, 1.5 mmol) was dissolved in dichloromethane (6 mL), and imidazole (310 mg, 4.6 mmol) and tert-butyldimethylsilyl chloride (330 mg, 2.2 mmol) were sequentially added thereto. The reaction mixture was reacted at room temperature for 12 hours under nitrogen atmosphere. TLC showed that the starting material was completely reacted. The reaction mixture was concentrated under reduced pressure and then purified by silica gel column chromatography (EA: PE = 1:10) to obtain compound **89-9** (500 mg Y: 80%). ¹H NMR (400 MHz, CDCl₃): δ 7.15 (s, 1H), 5.90 (br, 1H), 5.40-5.32 (m, 1H), 3.08-2.96 (m, 4H), 2.68-2.58 (m, 1H), 1.70-1.62 (m, 1H), 1.36 (d, *J* = 6.8 Hz, 3H), 0.91 (m, 9H), 0.15 (m, 6H).

Step 10: Compound **89-9** (320 mg, 0.77 mmol) and compound **1-8** (420 mg, 0.77 mmol) were dissolved in toluene (6 mL) and water (1.2 mL), and tris(dibenzylideneacetone)dipalladium (70 mg, 0.07 mmol), 4,5-bis(diphenylphosphino)-9,9-dimethylxanthene (90 mg, 0.16 mmol), and anhydrous potassium carbonate (320 mg, 2.32 mmol) were sequentially added thereto. The reaction mixture was reacted at 110°C overnight under nitrogen atmosphere. LCMS showed that the starting material was completely reacted. The reaction mixture was cooled to 25°C, concentrated under reduced pressure, and purified by silica gel column chromatography (EA: PE = 1:10 to 1:1, Rf = 0.6 and Rf = 0.5, where Rf = 0.5 was for target product) to obtain compound **89-10** (110 mg, Y: 19%). LC-MS (1.5 mL-5 min-5 to 90% B): Rt = 3.913, m/z (ESI): 753.4 [M+H]⁺.

Step 11: Compound **89-10** (110 mg, 0.15 mmol) was dissolved in hydrochloric acid/dioxane solution (1 mL, 4.0 M), and the reaction mixture was reacted at 25°C for 0.5 hours. LCMS showed that the starting material was completely reacted. The reaction mixture was slowly added with saturated sodium carbonate aqueous solution (40 mL), and extracted with ethyl acetate (40 mL × 2). The organic phases were combined, washed with saturated brine (15 mL), dried over anhydrous sodium sulfate, and filtered. The filtrate was concentrated under reduced pressure and then purified by preparative high-performance liquid chromatography (column model: Xselect CSH Prep Fluoro-Phenyl, 150 * 19 mm, 5 µm; eluent: 0.1% FA-CAN; gradient: 21 to 41% MeCN in H₂O; flow rate: 20 mL/min; run time: 12 min; retention time: 8.3 min and 9.2 min) to obtain compounds **89a1** (4.1 mg, Y: 4.8%, FA salt, retention time: 8.3 min) and **89a2** (30.8 mg, Y: 36%, FA salt, retention time: 9.2 min) as white solids.

**89a1:** ¹H NMR (400 MHz, DMSO-*d₆*): δ 9.23 (br, 1H), 8.37-8.24 (br, 2H), 7.53-7.35 (m, 6H), 7.12 (d, *J* = 9.6 Hz, 1H), 5.28-5.19 (m, 2H), 4.35-4.26 (m, 1H), 3.60-3.40 (m, 2H), 3.28-3.02 (m, 4H), 2.90 (d, *J* = 16.4 Hz, 1H), 2.64 (d, *J* = 4.8 Hz, 3H), 2.23-2.10 (m, 1H), 1.98-1.70 (m, 3H), 1.30 (d, *J* = 6.8 Hz, 3H). LC-MS m/z (ESI): 539.3/541.3 [M+H]⁺. Chiral analysis (column model: Amylose-SA, 100 * 4.6 mm, 3 µm; eluent: hexane (0.1% DEA)-IPA; gradient: isocratic 9/1, flow rate: 1 mL/min; run time: 10.0 min; retention time: 5.32 min).

**89a2:** ¹H NMR (400 MHz, DMSO-*d₆*): δ 8.22-8.13 (br, 1H), 7.48-7.24 (m, 6H), 7.02 (d, *J* = 9.6 Hz, 1H), 5.43 (br, 1H), 5.24-5.20 (m, 1H), 3.70-3.62 (m, 1H), 3.49 (d, *J* = 16.0 Hz, 1H), 3.15-3.05 (m, 1H), 2.90-2.70 (m, 4H), 2.63 (d, *J* = 4.4 Hz, 3H), 1.72-1.43 (m, 4H), 1.25 (d, *J* = 6.4 Hz, 3H). LC-MS m/z (ESI): 539.3 [M+H]⁺. Chiral analysis (column model: Amylose-SA, 100 * 4.6 mm, 5 µm; eluent: hexane (0.1% DEA)-IPA; gradient: isocratic 9/1, flow rate: 1 mL/min; run time: 10.0 min; retention time: 4.62 min).

### Example 90

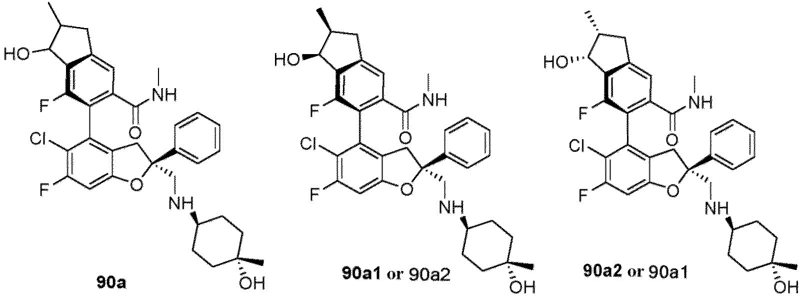

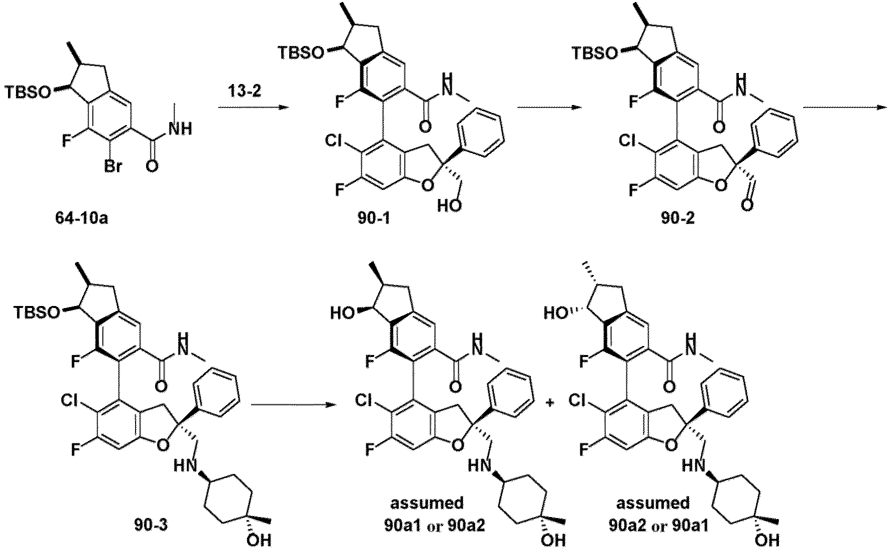

Step 1: Compound **64-10a** (300 mg, 0.72 mmol) and compound **13-2** (466 mg, 1.15 mmol) were dissolved in toluene (5 mL) and water (1 mL), and tris(dibenzylideneacetone)dipalladium (99 mg, 0.11 mmol), 4,5-bis(diphenylphosphino)-9,9-dimethylxanthene (125 mg, 0.22 mmol), and anhydrous potassium carbonate (298 mg, 2.16 mmol) were sequentially added thereto. The reaction mixture was reacted at 110°C overnight under nitrogen atmosphere. LCMS showed that the starting material was completely reacted. The reaction mixture was cooled to 25°C, concentrated under reduced pressure, and subjected to silica gel column chromatography (EA: PE = 1:10 to 1:1, Rf = 0.6 and Rf = 0.5, where Rf = 0.5 was for target product) to obtain a crude product. The crude product was purified by C18 silica gel column chromatography (C18, 40.0 g, gradient: 70 to 90% MeCN in H₂O; flow rate: 30 mL/min: run time: 10 min) to obtain compound **90-1** (160 mg, Y: 36%). LC-MS (1.5 mL-3.2 min-5 to 95% B): Rt = 1.847, m/z (ESI): 614.3/616.3 [M+H]⁺.

Step 2: Compound **90-1** (160 mg, 0.26 mmol) was dissolved in anhydrous dichloromethane (4 mL), then Dess-Martin periodinane (165 mg, 0.39 mmol) and solid sodium bicarbonate (60 mg, 0.71 mmol) were sequentially added thereto, and the reaction mixture was reacted at 25°C for 0.5 hours under nitrogen atmosphere. LCMS showed that the starting material was completely reacted. The reaction mixture was added with saturated sodium thiosulfate aqueous solution (20 mL), and extracted with ethyl acetate (15 mL × 2). The organic phases were combined, washed with saturated brine (10 mL × 2), dried over anhydrous sodium sulfate, filtered, and the filtrate was concentrated under reduced pressure to obtain compound **90-2** (120 mg, crude) as a crude product. LC-MS m/z (ESI): 612.2 [M+H]⁺.

Step 3: Compound **90-2** (120 mg, 0.20 mmol) was dissolved in dichloroethane (4 mL), then trans-4-amino-1-methylcyclohexanol (46 mg, 0.35 mmol) and acetic acid (15 mg, 0.25 mmol) were added thereto, and the reaction mixture was reacted at 80°C for 2 hours. The reaction mixture was cooled to 25°C, added with sodium triacetoxyborohydride (127 mg, 0.60 mmol), and reacted at 60°C for 0.5 hours. LCMS showed that the starting material was completely reacted. The reaction mixture was cooled to 25°C, added with water (20 mL), and extracted with dichloromethane (30 mL × 2). The organic phase was washed with saturated brine (20 mL), dried over anhydrous sodium sulfate, and filtered. The filtrate was concentrated under reduced pressure and then purified by a silica gel plate (EA: PE = 1:4) to obtain compound **90-3** (60 mg, Y: 42%). LC-MS m/z (ESI)): 725.4/727.4 [M+H]⁺.

Step 4: Compound **90-3** (60 mg, 0.08 mmol) was dissolved in hydrochloric acid/dioxane solution (2 mL, 4.0 M), and the reaction mixture was reacted at 25°C for 0.5 hours. LCMS showed that the starting material was completely reacted. The reaction mixture was concentrated under reduced pressure and then purified by preparative high-performance liquid chromatography (column model: YMC-Actus Triart C18, 150 * 20 mm, 5 µm; eluent: 0.1% FA-CAN; gradient: 20 to 50% MeCN in H₂O; flow rate: 20 mL/min; run time: 11 min; retention time: 8.5 min/9.3 min) to obtain compound **90a1** (4.5 mg, Y: 8.3%, FA salt, retention time: 8.5 min) and compound **90a2** (16.6 mg, Y: 30%, FA salt, retention time: 9.3 min).

**90a1:** ¹H NMR (400 MHz, DMSO-*d₆*): δ 8.25-8.19 (br, 1H), 7.40-7.24 (m, 6H), 7.02 (d, *J* = 10.0 Hz, 1H), 5.20-5.11 (br, 1H), 4.97-4.90 (m, 1H), 4.09-4.10 (br, 1H), 3.45-3.38 (m, 1H), 2.98-2.69 (m, 5H), 2.63 (d, *J* = 4.4 Hz, 3H), 2.43-2.34 (m, 1H), 1.70-1.14 (m, 6H), 1.13-1.02 (m, 5H), 1.00 (s, 3H). LC-MS m/z (ESI): 611.4/613.4 [M+H]⁺. Chiral analysis (column model: Celluiose-C, 100 * 4.6 mm, 3 µm; eluent: hexane (0.1% DEA)-IPA; gradient: isocratic 4/1, flow rate: 1 mL/min; run time: 8.0 min; retention time: 3.26 min).

**90a2:** ¹H NMR (400 MHz, DMSO-*d₆*): δ 8.27-8.11 (br, 1H), 8.17 (br, 1H), 7.40-7.25 (m, 6H), 7.03 (d, *J* = 9.6 Hz, 1H), 5.22-5.00 (br, 1H), 4.95 (d, *J* = 6.0 Hz, 1H), 4.15-3.90 (br, 1H), 3.50-3.43 (m, 1H), 3.00-2.65 (m, 5H), 2.63 (d, *J* = 4.4 Hz, 3H), 2.44-2.34 (m, 1H), 1.70-1.16 (m, 6H), 1.14-1.03 (m, 5H), 1.01 (s, 3H). LC-MS m/z (ESI): 611.4/613.4 [M+H]⁺. Chiral analysis (column model: Celluiose-C, 100 * 4.6 mm, 3 µm; eluent: hexane (0.1% DEA)-IPA; gradient: isocratic 4/1, flow rate: 1 mL/min; run time: 8.0 min; retention time: 2.90 min).

Compounds 91, 93, and 95 were prepared with reference to example 84; compounds 92 and 94 were prepared with reference to example 90; compounds 70, 71, 96, and 98 were prepared with reference to example 78; and compounds 97 and 99 were prepared with reference to example 79.

### Effect example 1: Tumor cell proliferation inhibition experiment

MSTO-211H and NCI-H2052 lung cancer cells were selected for the testing experiment on the effect of the compounds of the present disclosure, and the inhibitory activity IC₅₀ of the compounds in the examples to inhibit cell proliferation was tested. Cell Titer Glo luminescent cell viability assay kit was used to quantify the ATP content in cells, so as to quantify the number of viable cells. MSTO-211H and NCI-H2052 cells (ATCC) were inoculated in a 96-well plate at an inoculum size of 1000 cells (100 µL per well) and incubated overnight at 37°C in a 5% carbon dioxide incubator. On the second day, the compound was added to the 96-well plate after a three-fold serial dilution to eight concentrations, and incubated at 37°C for three days in the 5% carbon dioxide incubator. The culture medium was treated with the addition of Cell Titer Glo luminescent cell viability assay kit (Promega G7570) reagent, incubated for 10 minutes, and then placed in Envision (Pelkin Elmer) to read the luminescence signal. DMSO was used instead of the compound as a control to calculate the inhibition rate (inhibition rate% = (1 - compound signal/DMSO signal) * 100), and then standard four parameter curve fitting was used to calculate the inhibitory activity IC₅₀.

The compounds of the present disclosure have anti-proliferative activity on MSTO-211H and NCI-H2052 cells, and the IC₅₀ data for some of the compounds are shown in Table 1.

**Table 1**

| Test compound | MSTO-211H IC₅₀ (µM) | NCI-H2052 IC₅₀ (µM) |
|---|---|---|
| Compound 1a | 2.19 | 4.75 |
| Compound 2a | 0.414 | 0.928 |
| Compound 3a | 0.976 | 1.867 |
| Compound 4a | >10 | 8.37 |
| Compound 5a | 1.94 | 3.14 |
| Compound 6a | >10 | >10 |
| Compound 7a | 9.80 | 9.08 |
| Compound 8a | >10 | >10 |
| Compound 9a | >10 | 7.97 |
| Compound 10a | 0.236 | 0.328 |
| Compound 11a | >10 | >10 |
| Compound 12a | >10 | >10 |
| Compound 13a | 0.20 | 0.21 |
| Compound 14a | 0.32 | 0.36 |
| Compound 14a1 | 0.22 | 0.29 |
| Compound 14a2 | 0.39 | 0.59 |
| Compound 15a | 2.88 | 2.73 |
| Compound 16a | 1.73 | 1.85 |
| Compound 17a | 0.08 | 0.09 |
| Compound 18a | 0.69 | 0.73 |
| Compound 19a | >10 | >10 |
| Compound 20a | >10 | >10 |
| Compound 22a | 0.05 | 0.09 |
| Compound 23a | 0.11 | 0.20 |
| Compound 24a | 0.02 | 0.04 |
| Compound 25a | 0.15 | 0.26 |
| Compound 26a | 0.03 | 0.04 |
| Compound 27a | 0.10 | 0.21 |
| Compound 28a | 0.04 | 0.05 |
| Compound 30a | 0.30 | 0.55 |
| Compound 31a | 0.20 | 0.25 |
| Compound 36a | 0.45 | 0.65 |
| Compound 37a1 | 1.25 | 1.50 |
| Compound 37a2 | 0.28 | 0.33 |
| Compound 37a3 | 0.38 | 0.40 |
| Compound 38a | 0.99 | 0.75 |
| Compound 40a | 0.49 | 0.60 |
| Compound 41a | 0.19 | 0.42 |
| Compound 41a1 | 0.64 | 0.87 |
| Compound 41a2 | 0.13 | 0.20 |
| Compound 42a | 0.73 | 0.90 |
| Compound 43a | 0.44 | 0.51 |
| Compound 44a1 | 4.49 | 4.40 |
| Compound 44a2 | 2.21 | 1.69 |
| Compound 45a1 | 1.09 | 1.76 |
| Compound 45a2 | 0.32 | 0.45 |
| Compound 45a3 | 0.90 | 1.44 |
| Compound 45a4 | 0.37 | 0.40 |
| Compound 46a | 0.64 | 0.93 |
| Compound 49a | 0.15 | 0.24 |
| Compound 49a2 | 0.12 | 0.12 |
| Compound 51a1 | 0.26 | 0.32 |
| Compound 52a | 0.17 | 0.20 |
| Compound 53a | 0.15 | 0.16 |
| Compound 54a1 | 0.09 | 0.09 |
| Compound 55a1 | 0.13 | 0.18 |
| Compound 56a1 | 3.48 | 2.79 |
| Compound 57a1 | 0.39 | 0.45 |
| Compound 64a1 | 0.08 | 0.12 |
| Compound 64a3 | 0.26 | 0.36 |
| Compound 65a | >10 | >10 |
| Compound 67a1 | 0.69 | 0.59 |
| Compound 68a2 | 6.75 | 3.49 |
| Compound 69a4 | 1.53 | 1.37 |
| Compound 72a1 | 0.39 | 0.41 |
| Compound 73a1 | 0.53 | 0.53 |
| Compound 74a2 | 0.25 | 0.19 |
| Compound 75a2 | 0.28 | 0.34 |
| Compound 76a2 | 1.14 | 1.28 |
| Compound 77a2 | 1.19 | 1.37 |
| Compound 79a2 | 0.53 | 0.52 |
| Compound 80a | 0.49 | 0.58 |
| Compound 81a2 | 0.17 | 0.17 |
| Compound 82a2 | 0.17 | 0.19 |
| Compound 83a1 | 0.89 | 0.85 |
| Compound 84a2 | 0.28 | 0.28 |
| Compound 85a1 | 1.41 | 1.70 |
| Compound 85a2 | 1.48 | 1.86 |
| Compound 86a1 | 0.84 | 0.75 |
| Compound 87a2 | 0.33 | 0.38 |
| Compound 88a2 | 0.22 | 0.30 |
| Compound 89a1 | 0.31 | 0.31 |
| Compound 89a2 | 0.26 | 0.29 |
| Compound 90a2 | 0.08 | 0.08 |

The results show that the compounds of the present disclosure have good inhibitory activity.

### Effect example 2: Pharmacokinetic evaluation experiment in mice

120 healthy adult male ICR mice were selected and divided into 20 groups according to the principle of similar body weight, with 6 mice in each group of IV (10 groups) and 6 mice in each group of PO (10 groups). An appropriate amount of sample was weighed, sequentially added with an appropriate amount of DMA, Solutol, and Saline according to the volume ratio of 10:10:80, and stirred and sonicated to achieve a clarification of 1 mg/mL. After overnight fasting, mice in the IV group were intravenously administered with a volume of 5 mL/kg and a dose of 5 mg/kg, respectively; and mice in the PO group were intragastrically administered with a volume of 30 mL/kg and a dose of 30 mg/kg, respectively. Interleaved sampling was performed on every 6 mice after administration of the test compound. In the IV group, approximately 30 µL of blood was collected at 0.0833, 0.25, 0.5, 1, 2, 4, 6, 8, and 24 hours, and placed in a commercial anticoagulant tube pre-filled with EDTA-K2. In the PO group, approximately 30 µL of blood was collected at 0.25, 0.5, 1, 2, 4, 6, 8, and 24 hours, and placed in the commercial anticoagulant tube pre-filled with EDTA-K2. The tubes were centrifuged for 15 minutes to separate the plasma, and the plasma was stored at -80°C. Animals were allowed to eat 2 hours after administration. The content of the test compound in the plasma of mice after intravenous and intragastric administration was determined by LC/MS/MS method. The linear range of the method was 1.00 to 1000 ng/mL: plasma samples were analyzed after protein precipitation.

The pharmacokinetic data for the compounds of the present disclosure intravenously and intragastrically administered to mice are shown in Table 2 and Table 3.

**Table 2**

| Group | | Compound 2a | Compound 10a | Compound 13a | Compound 14a | Compound 16a | Compound 17a | Compound 18a |
|---|---|---|---|---|---|---|---|---|
| IV (5 mg/kg ) | CL (mL/min/kg) | 86.3 | 50.4 | 41.4 | 24.1 | 56.4 | 75.8 | 107 |
| | Vss (L/kg) | 28.0 | 2.42 | 8.50 | 4.34 | 11.4 | 11.7 | 12.2 |
| | AUC (hr*ng/mL) | 750 | 1653 | 2014 | 3462 | 1476 | 1100 | 804 |
| | T_{1/2} (hr) | 3.82 | 1.11 | 2.23 | 2.35 | 2.28 | 1.79 | 1.59 |
| PO (30 mg/kg ) | Cmax (ng/mL) | 474 | 603 | 2053 | 2697 | 434 | 424 | 199 |
| | Tmax (hr) | 4.00 | 0.500 | 2.00 | 2.00 | 2.00 | 1.33 | 0.80 |
| | AUC (hr*ng/mL) | 4297 | 1713 | 13108 | 21209 | 3572 | 3446 | 1679 |
| | F% | 95.4 | 17.3 | 108.5 | 102.1 | 40.3 | 52.2 | 27.9 |

**Table 3**

| Group | | Compound 31a | Compound 41a | Compound 53a |
|---|---|---|---|---|
| IV (5 mg/kg) | CL (mL/min/kg) | 17.9 | 36.5 | 44.2 |
| | Vss (L/kg) | 4.44 | 5.32 | 6.94 |
| | AUC (hr*ng/mL) | 2791 | 2284 | 1884 |
| | T_{1/2} (hr) | 3.52 | 2.19 | 4.18 |
| PO (30 mg/kg) | Cmax (ng/mL) | 928 | 1270 | 301 |
| | Tmax (hr) | 1.00 | 2.00 | 0.50 |
| | AUC (hr*ng/mL) | 7470 | 8464 | 1625 |
| | F% | 44.6 | 61.8 | 14.4 |

The results show that the compounds of the present disclosure have good pharmacokinetic properties.

## Claims

1. A compound of formula (I), a pharmaceutically acceptable salt thereof, or a solvate of the pharmaceutically acceptable salt thereof: 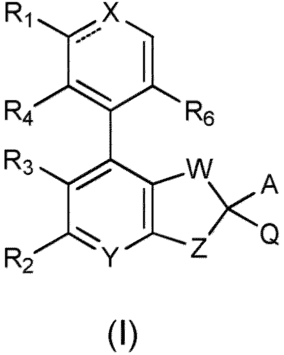 wherein
"-----" is a single bond or a double bond;
Yis CH or N;
W is O or CH-R_{W}; R_{W} is H, OH, C₁-C₆ alkoxy, -C₁-C₆ alkyl-OR_{W-1}, or C₁-C₆ alkyl; R_{W-1} is H or C₁-C₆ alkyl;
Z is CH₂, O, S, or NH;
R₂ is H or halogen;
R₆ is H, CN, -CONHR₆₋₁, -NHR₆₋₂, or C₁-C₆ alkoxy substituted by 1, 2, or 3 NH₂ or OH groups;
R₆₋₁ is H, C₁-C₆ alkyl, C₃-C₆ cycloalkyl, "5- to 6-membered monocyclic heteroaryl with 1, 2, or 3 heteroatoms selected from 1, 2, or 3 of N, O, and S", or "5- to 6-membered monocyclic heteroaryl with 1, 2, or 3 heteroatoms selected from 1, 2, or 3 of N, O, and S" substituted by 1, 2, or 3 C₁-C₆ alkyl groups;
R₆₋₂ is C₁-C₆ alkyl substituted by 1, 2, or 3 NH₂ or OH groups;
R₁, R₃, R₄, X, A, and Q are as defined in any one of the following schemes:
scheme 1:
when the "-----" connected to X is a double bond, X is N, CH, or C-R_{X1};
when X is C-R_{X1}, R_{X1}, R₁ together with the atom to which they are attached form a ring B, that is, 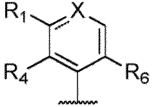 is 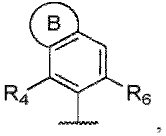 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when X is N or CH, R₁ is -(CH₂)ₘ₁-O-(CH₂)ₘ₂-O-R₁₋₆, m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when the "-----" connected to X is a single bond, X is N-R_{X2}, and R_{X2}, R₁ together with the atom to which they are attached form a ring C, that is, 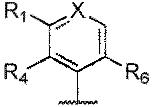 is 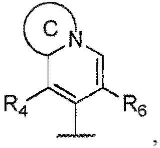 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, C₁-C₆ alkoxy substituted by 1, 2, or 3 R₁₋₁₋₂ groups, - (CH₂)ₙ₁-O-(CH₂)ₙ₂-O-C₃-C₆ cycloalkyl, n1 is 0, 1, or 2, n2 is 1 or 2; R₁₋₁₋₁ and R₁₋₁₋₂ are each independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
R₃ is halogen, CN, C₂-C₆ alkynyl, NH₂, OH, or C₁-C₆ alkoxy;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
scheme 2:
Q is "3- to 15-membered monocyclic or bicyclic heteroaryl with 1, 2, or 3 heteroatoms selected from 1, 2, or 3 of N, O, and S", "3- to 15-membered monocyclic or bicyclic heteroaryl with 1, 2, or 3 heteroatoms selected from 1, 2, or 3 of N, O, and S" substituted by 1, 2, or 3 R_{Q-2} groups, "3- to 6-membered heterocycloalkyl with 1, 2, or 3 heteroatoms selected from 1, 2, or 3 of N, O, and S" substituted by 1, 2, or 3 R_{Q-5} groups, or 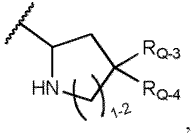
wherein R_{Q-3}, R_{Q-4} together with the atom to which they are attached form a 3- to 15-membered monocyclic or bicyclic saturated or unsaturated carbocyclic ring, a 3- to 15-membered monocyclic or bicyclic saturated or unsaturated carbocyclic ring substituted by 1, 2, or 3 R_{Q-1} groups, "a 3- to 15-membered monocyclic or bicyclic saturated or unsaturated heterocyclic ring with 1, 2, or 3 heteroatoms selected from 1, 2, or 3 of N, O, and S", or "a 3- to 15-membered monocyclic or bicyclic saturated or unsaturated heterocyclic ring with 1, 2, or 3 heteroatoms selected from 1, 2, or 3 of N, O, and S" substituted by 1, 2, or 3 R_{Q-2} groups;
R_{Q-1} and R_{Q-2} are independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
R_{Q-5} is independently oxo (=O), methylene (=CH₂), or C₂-C₆ alkenyl;
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)z-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 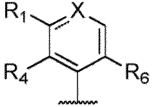 is 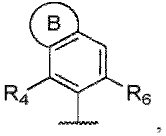 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 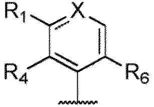 is 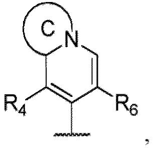 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, C₁-C₆ alkoxy substituted by 1, 2, or 3 R₁₋₁₋₂ groups, - (CH₂)ₙ₁-O-(CH₂)ₙ₂-O-C₃-C₆ cycloalkyl, n1 is 0, 1, or 2, n2 is 1 or 2; R₁₋₁₋₁ and R₁₋₁₋₂ are each independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
R₃ is halogen, CN, C₂-C₆ alkynyl, NH₂, OH, or C₁-C₆ alkoxy;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
scheme 3:
A is C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups, A is not 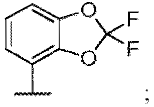
R_{A-1} is independently halogen, C₁-C₆ alkoxy, NH₂, CN, C₃-C₆ cycloalkyl, C₁-C₆ alkyl substituted by 1 hydroxyl group, or oxo (=O), and at least 1 R_{A-1} is C₁-C₆ alkoxy, NH₂, CN, C₃-C₆ cycloalkyl, C₁-C₆ alkyl substituted by 1 hydroxyl group, or oxo (=O);
R_{A-2} is independently halogen, C₁-C₆ alkoxy, NH₂, CN, C₃-C₆ cycloalkyl, C₁-C₆ alkyl substituted by 1 hydroxyl group, or oxo (=O);
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)z-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 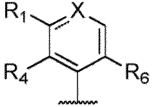 is 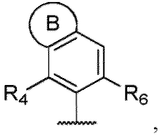 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 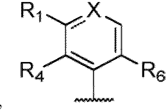 is 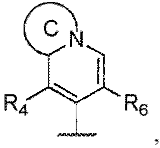 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, C₁-C₆ alkoxy substituted by 1, 2, or 3 R₁₋₁₋₂ groups, - (CH₂)ₙ₁-O-(CH₂)ₙ₂-O-C₃-C₆ cycloalkyl, n1 is 0, 1, or 2, n2 is 1 or 2; R₁₋₁₋₁ and R₁₋₁₋₂ are each independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
R₃ is halogen, CN, C₂-C₆ alkynyl, NH₂, OH, or C₁-C₆ alkoxy;
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
scheme 4:
R₄ is NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)z-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 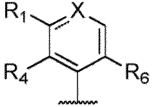 is 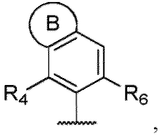 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 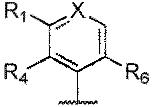 is 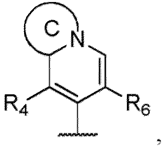 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, C₁-C₆ alkoxy substituted by 1, 2, or 3 R₁₋₁₋₂ groups, - (CH₂)ₙ₁-O-(CH₂)ₙ₂-O-C₃-C₆ cycloalkyl, n1 is 0, 1, or 2, n2 is 1 or 2; R₁₋₁₋₁ and R₁₋₁₋₂ are independently OH, NH₂, or halogen;
R₃ is halogen, CN, C₂-C₆ alkynyl, NH₂, OH, or C₁-C₆ alkoxy;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
scheme 5:
R₃ is C₂-C₆ alkynyl, NH₂, OH, or C₁-C₆ alkoxy;
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)z-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 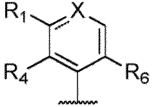 is 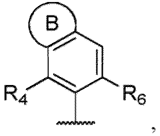 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 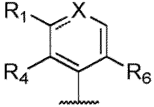 is 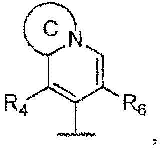 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, C₁-C₆ alkoxy substituted by 1, 2, or 3 R₁₋₁₋₂ groups, - (CH₂)ₙ₁-O-(CH₂)ₙ₂-O-C₃-C₆ cycloalkyl, n1 is 0, 1, or 2, n2 is 1 or 2; R₁₋₁₋₁ and R₁₋₁₋₂ are each independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂.

2. The compound of formula (I), the pharmaceutically acceptable salt thereof, or the solvate of the pharmaceutically acceptable salt thereof according to claim 1, wherein
"-----" is a single bond or a double bond;
Yis CH or N;
W is O or CH-R_{W}; R_{W} is H, OH, C₁-C₆ alkoxy, -C₁-C₆ alkyl-OR_{W-1}, or C₁-C₆ alkyl; R_{W-1} is H or C₁-C₆ alkyl;
Z is CH₂, O, S, or NH;
R₂ is H or halogen;
R₆ is H, CN, -CONHR₆₋₁, -NHR₆₋₂, or C₁-C₆ alkoxy substituted by 1, 2, or 3 NH₂ or OH groups;
R₆₋₁ is H, C₁-C₆ alkyl, C₃-C₆ cycloalkyl, "5- to 6-membered monocyclic heteroaryl with 1, 2, or 3 heteroatoms selected from 1, 2, or 3 of N, O, and S", or "5- to 6-membered monocyclic heteroaryl with 1, 2, or 3 heteroatoms selected from 1, 2, or 3 of N, O, and S" substituted by 1, 2, or 3 C₁-C₆ alkyl groups;
R₆₋₂ is C₁-C₆ alkyl substituted by 1, 2, or 3 NH₂ or OH groups;
R₁, R₃, R₄, X, A, and Q are as defined in any one of the following schemes:
scheme 1:
when the "-----" connected to X is a double bond, X is N, CH, or C-R_{X1};
when X is C-R_{X1}, R_{X1}, R₁ together with the atom to which they are attached form a ring B, that is, 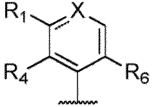 is 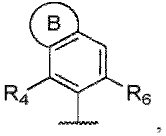 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when X is N or CH, R₁ is -(CH₂)ₘ₁-O-(CH₂)ₘ₂-O-R₁₋₆, m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when the "-----" connected to X is a single bond, X is N-R_{X2}, and R_{X2}, R₁ together with the atom to which they are attached form a ring C, that is, 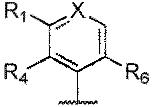 is 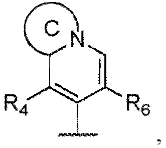 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
R₃ is halogen, CN, C₂-C₆ alkynyl, NH₂, OH, or C₁-C₆ alkoxy;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
scheme 2:
Q is "3- to 15-membered monocyclic or bicyclic heteroaryl with 1, 2, or 3 heteroatoms selected from 1, 2, or 3 of N, O, and S", "3- to 15-membered monocyclic or bicyclic heteroaryl with 1, 2, or 3 heteroatoms selected from 1, 2, or 3 of N, O, and S" substituted by 1, 2, or 3 R_{Q-2} groups, "3- to 6-membered heterocycloalkyl with 1, 2, or 3 heteroatoms selected from 1, 2, or 3 of N, O, and S" substituted by 1, 2, or 3 R_{Q-5} groups, or 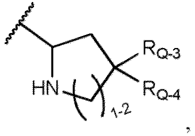
wherein R_{Q-3}, R_{Q-4} together with the atom to which they are attached form a 3- to 15-membered monocyclic or bicyclic saturated or unsaturated carbocyclic ring, a 3- to 15-membered monocyclic or bicyclic saturated or unsaturated carbocyclic ring substituted by 1, 2, or 3 R_{Q-1} groups, "a 3- to 15-membered monocyclic or bicyclic saturated or unsaturated heterocyclic ring with 1, 2, or 3 heteroatoms selected from 1, 2, or 3 of N, O, and S", or "a 3- to 15-membered monocyclic or bicyclic saturated or unsaturated heterocyclic ring with 1, 2, or 3 heteroatoms selected from 1, 2, or 3 of N, O, and S" substituted by 1, 2, or 3 R_{Q-2} groups;
R_{Q-1} and R_{Q-2} are independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
R_{Q-5} is independently oxo (=O), methylene (=CH₂), or C₂-C₆ alkenyl;
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)z-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 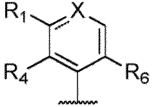 is 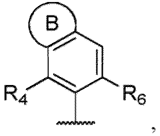 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 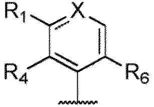 is 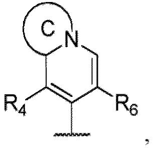 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
R₃ is halogen, CN, C₂-C₆ alkynyl, NH₂, OH, or C₁-C₆ alkoxy;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
scheme 3:
A is C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups, A is not 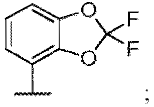
R_{A-1} is independently halogen, C₁-C₆ alkoxy, NH₂, CN, C₃-C₆ cycloalkyl, C₁-C₆ alkyl substituted by 1 hydroxyl group, or oxo (=O), and at least 1 R_{A-1} is C₁-C₆ alkoxy, NH₂, CN, C₃-C₆ cycloalkyl, C₁-C₆ alkyl substituted by 1 hydroxyl group, or oxo (=O);
R_{A-2} is independently halogen, C₁-C₆ alkoxy, NH₂, CN, C₃-C₆ cycloalkyl, C₁-C₆ alkyl substituted by 1 hydroxyl group, or oxo (=O);
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)₂-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 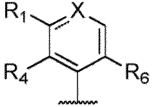 is 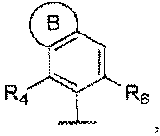 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 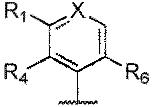 is 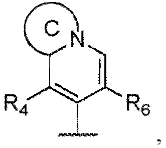 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
R₃ is halogen, CN, C₂-C₆ alkynyl, NH₂, OH, or C₁-C₆ alkoxy;
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
scheme 4:
R₄ is NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)z-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 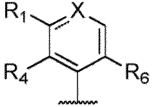 is 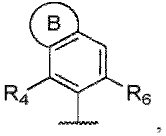 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 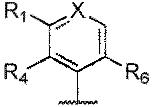 is 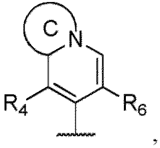 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
R₃ is halogen, CN, C₂-C₆ alkynyl, NH₂, OH, or C₁-C₆ alkoxy;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
scheme 5:
R₃ is C₂-C₆ alkynyl, NH₂, OH, or C₁-C₆ alkoxy;
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)z-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 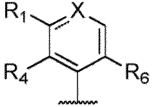 is 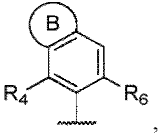 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 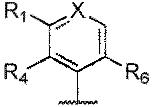 is 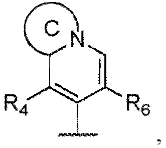 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups, C₃-C₆ cycloalkyl, or C₃-C₆ cycloalkyl substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂.

3. The compound of formula (I), the pharmaceutically acceptable salt thereof, or the solvate of the pharmaceutically acceptable salt thereof according to claim 1, wherein
"-----" is a single bond or a double bond;
Yis CH or N;
W is O or CH-R_{W}; R_{W} is H, OH, C₁-C₆ alkoxy, -C₁-C₆ alkyl-OR_{W-1}, or C₁-C₆ alkyl; R_{W-1} is H or C₁-C₆ alkyl;
Z is CH₂, O, S, or NH;
R₂ is H or halogen;
R₆ is H, CN, -CONHR₆₋₁, -NHR₆₋₂, or C₁-C₆ alkoxy substituted by 1, 2, or 3 NH₂ or OH groups;
R₆₋₁ is H, C₁-C₆ alkyl, C₃-C₆ cycloalkyl, "5- to 6-membered monocyclic heteroaryl with 1, 2, or 3 heteroatoms selected from 1, 2, or 3 of N, O, and S", or "5- to 6-membered monocyclic heteroaryl with 1, 2, or 3 heteroatoms selected from 1, 2, or 3 of N, O, and S" substituted by 1, 2, or 3 C₁-C₆ alkyl groups;
R₆₋₂ is C₁-C₆ alkyl substituted by 1, 2, or 3 NH₂ or OH groups;
R₁, R₃, R₄, X, A, and Q are as defined in any one of the following schemes:
scheme 1:
when the "-----" connected to X is a double bond, X is N, CH, or C-R_{X1};
when X is C-R_{X1}, R_{X1}, R₁ together with the atom to which they are attached form a ring B, that is, 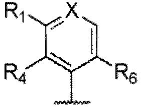 is 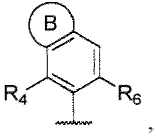 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when X is N or CH, R₁ is -(CH₂)ₘ₁-O-(CH₂)ₘ₂-O-R₁₋₆, m1 is 0, 1, or 2, m2 is 1 or 2; R₁₆ is C₃-C₆ cycloalkyl or "3- to 6-membered heterocycloalkyl with 1, 2, or 3 heteroatoms selected from 1, 2, or 3 of N, O, and S";
when the "-----" connected to X is a single bond, X is N-R_{X2}, and R_{X2}, R₁ together with the atom to which they are attached form a ring C, that is, 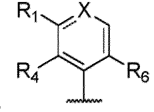 is 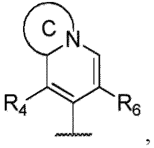 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
R₃ is halogen, CN, C₂-C₆ alkynyl, NH₂, OH, or C₁-C₆ alkoxy;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", or "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
scheme 2:
Q is "3- to 15-membered monocyclic or bicyclic heteroaryl with 1, 2, or 3 heteroatoms selected from 1, 2, or 3 of N, O, and S", "3- to 15-membered monocyclic or bicyclic heteroaryl with 1, 2, or 3 heteroatoms selected from 1, 2, or 3 of N, O, and S" substituted by 1, 2, or 3 R_{Q-2} groups, "3- to 6-membered heterocycloalkyl with 1, 2, or 3 heteroatoms selected from 1, 2, or 3 of N, O, and S" substituted by 1, 2, or 3 R_{Q-5} groups, or 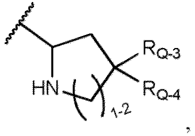
wherein R_{Q-3}, R_{Q-4} together with the atom to which they are attached form a 3- to 15-membered monocyclic or bicyclic saturated or unsaturated carbocyclic ring, a 3- to 15-membered monocyclic or bicyclic saturated or unsaturated carbocyclic ring substituted by 1, 2, or 3 R_{Q-1} groups, "a 3- to 15-membered monocyclic or bicyclic saturated or unsaturated heterocyclic ring with 1, 2, or 3 heteroatoms selected from 1, 2, or 3 of N, O, and S", or "a 3- to 15-membered monocyclic or bicyclic saturated or unsaturated heterocyclic ring with 1, 2, or 3 heteroatoms selected from 1, 2, or 3 of N, O, and S" substituted by 1, 2, or 3 R_{Q-2} groups;
R_{Q-1} and R_{Q-2} are independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
R_{Q-5} is independently oxo (=O), methylene (=CH₂), or C₂-C₆ alkenyl;
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)z-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 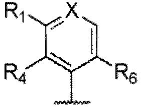 is 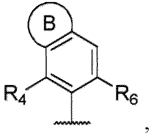 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 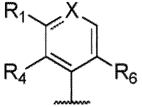 is 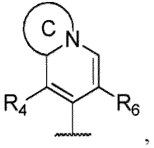 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
R₃ is halogen, CN, C₂-C₆ alkynyl, NH₂, OH, or C₁-C₆ alkoxy;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
scheme 3:
A is C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups, A is not 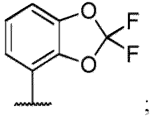
R_{A-1} is independently halogen, C₁-C₆ alkoxy, NH₂, CN, C₃-C₆ cycloalkyl, C₁-C₆ alkyl substituted by 1 hydroxyl group, or oxo (=O), and at least 1 R_{A-1} is C₁-C₆ alkoxy, NH₂, CN, C₃-C₆ cycloalkyl, C₁-C₆ alkyl substituted by 1 hydroxyl group, or oxo (=O);
R_{A-2} is independently halogen, C₁-C₆ alkoxy, NH₂, CN, C₃-C₆ cycloalkyl, C₁-C₆ alkyl substituted by 1 hydroxyl group, or oxo (=O);
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)₂-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 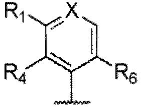 is 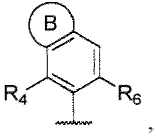 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 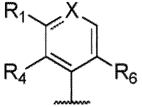 is 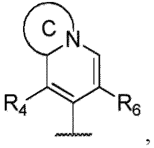 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
R₃ is halogen, CN, C₂-C₆ alkynyl, NH₂, OH, or C₁-C₆ alkoxy;
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", or "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
scheme 4:
R₄ is NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)z-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 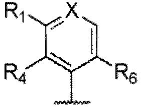 is 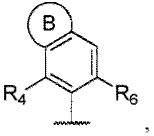 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 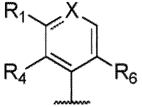 is 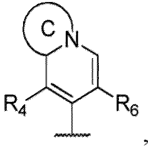 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
R₃ is halogen, CN, C₂-C₆ alkynyl, NH₂, OH, or C₁-C₆ alkoxy;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", or "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂;
scheme 5:
R₃ is C₂-C₆ alkynyl, NH₂, OH, or C₁-C₆ alkoxy;
when the "-----" connected to X is a double bond, X is N, CH, or C-R_{X1},
when X is N or CH, R₁ is H, OH, halogen, -L₁-R₁₋₄, "3- to 6-membered heteroaryl with 1, 2, or 3 heteroatoms selected from 1, 2, or 3 of N, O, and S", or -(CH₂)ₘ₁-O-(CH₂)ₘ₂-O-R₁₋₆;
L₁ is absent, -O-, -NH-, -S-, or -S(O)z-;
R₁₋₄ is C₁-C₆ alkyl or C₁-C₆ alkyl substituted by 1, 2, or 3 R₁₋₅ groups;
R₁₋₅ is independently OH, halogen, COOH, C₃-C₆ cycloalkyl, "3- to 6-membered heterocycloalkyl with 1, 2, or 3 heteroatoms selected from 1, 2, or 3 of N, O, and S", "3- to 6-membered heteroaryl with 1, 2, or 3 heteroatoms selected from 1, 2, or 3 of N, O, and S", C₁-C₆ alkoxy, or -S(O)₂-C₁-C₆ alkyl;
m1 is 0, 1, or 2, m2 is 1 or 2; R₁₋₆ is C₃-C₆ cycloalkyl or "3- to 6-membered heterocycloalkyl with 1, 2, or 3 heteroatoms selected from 1, 2, or 3 of N, O, and S";
when X is C-R_{X1}, R_{X1}, R₁ together with the atom to which they are attached form a ring B, that is, 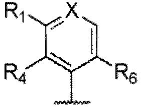 is 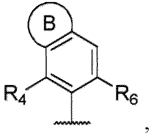 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
when the "-----" connected to X is a single bond, X is N-R_{X2}, and R_{X2}, R₁ together with the atom to which they are attached form a ring C, that is, 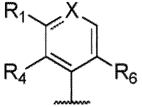 is 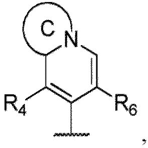 ring C is "a 5- to 15-membered monocyclic, bicyclic, or tricyclic saturated or unsaturated heterocyclic ring with 1, 2, or 3 heteroatoms selected from 1, 2, or 3 of N, O, and S" or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₃ groups;
R₁₋₁, R₁₋₂, and R₁₋₃ are independently oxo (=O), OH, NH₂, halogen, -S(O)₂-C₁-C₆ alkyl, CN, C₁-C₆ alkyl, C₁-C₆ alkyl substituted by 1, 2, or 3 R₁₋₁₋₁ groups, -(CH₂)ₙ₁-O-(CH₂)ₙ₂-O-C₃-C₆ cycloalkyl, n1 is 0, 1, or 2, n2 is 1 or 2; R₁₋₁₋₁ is independently OH, NH₂, or halogen;
R₄ is halogen, NH₂, CN, OH, C₁-C₆ alkoxy, C₁-C₆ alkyl substituted by 1 hydroxyl group, C₃-C₆ cycloalkyl, C₁-C₆ alkyl substituted by 1, 2, or 3 halogens, or C₂-C₆ alkynyl;
A is C₆-C₁₀ monocyclic or bicyclic aryl, C₆-C₁₀ monocyclic or bicyclic aryl substituted by 1, 2, or 3 R_{A-1} groups, "8- to 12-membered bicyclic heteroaryl with 1, 2, or 3 heteroatoms selected from 1, 2, or 3 of N, O, and S", or "8- to 12-membered bicyclic heteroaryl with 1, 2, or 3 heteroatoms selected from 1, 2, or 3 of N, O, and S" substituted by 1, 2, or 3 R_{A-2} groups;
R_{A-1} and R_{A-2} are independently halogen, C₁-C₆ alkoxy, NH₂, CN, C₃-C₆ cycloalkyl, C₁-C₆ alkyl substituted by 1 hydroxyl group, or oxo (=O);
Q is -CR₇R₈-NR₉-R₁₀ or R₁₁;
R₇, R₈, and R₉ are independently H or C₁-C₆ alkyl;
R₁₀ is H, C₁-C₆ alkyl, or -(CH₂)₀₋₂-R₁₀₋₁;
R₁₀₋₁ is C₁-C₆ alkyl substituted by 1, 2, or 3 OH groups, C₁-C₆ alkoxy, or R₁₀₋₂;
R₁₀₋₂ and R₁₁ are independently "a 3- to 15-membered monocyclic, bicyclic, or tricyclic saturated or unsaturated heterocyclic ring with 1, 2, or 3 heteroatoms selected from 1, 2, or 3 of N, O, and S", or "a 3- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R_{Q-1} groups;
R_{Q-1} is independently OH, halogen, NR_{Q-1-1}R_{Q-1-2}, C₁-C₆ alkyl, C₁-C₆ alkyl substituted by 1, 2, or 3 R_{Q-1-3} groups, C₁-C₆ alkoxy, C₁-C₆ alkoxy substituted by 1, 2, or 3 R_{Q-1-4} groups, oxo (=O), methylene (=CH₂), or C₂-C₆ alkenyl;
R_{Q-1-1} and R_{Q-1-2} are independently H or C₁-C₆ alkyl;
R_{Q-1-3} and R_{Q-1-4} are independently halogen, OH, or NH₂.

4. The compound of formula (I), the pharmaceutically acceptable salt thereof, or the solvate of the pharmaceutically acceptable salt thereof according to at least one of claims 1 to 3, wherein the compound of formula (I) satisfies one or more of the following conditions:
(1) each C₁-C₆ alkyl is independently methyl, ethyl, n-propyl, isopropyl, n-butyl, isobutyl, sec-butyl, or tert-butyl;
(2) each C₁-C₆ alkoxy is independently methoxy, ethoxy, n-propoxy, isopropoxy, n-butoxy, isobutoxy, sec-butoxy, or tert-butoxy;
(3) each halogen is independently F, Cl, Br, or I;
(4) each C₃-C₆ cycloalkyl is independently cyclopropyl, cyclobutyl, cyclopentyl, or cyclohexyl;
(5) each "5- to 6-membered monocyclic heteroaryl with 1, 2, or 3 heteroatoms selected from 1, 2, or 3 of N, O, and S" is independently "5- to 6-membered monocyclic heteroaryl with 1, 2, or 3 heteroatoms selected from 1 or 2 of N and O";
(6) each "3- to 6-membered heterocycloalkyl with 1, 2, or 3 heteroatoms selected from 1, 2, or 3 of N, O, and S" is independently "5- to 6-membered heterocycloalkyl with 1, 2, or 3 heteroatoms selected from 1 or 2 of N and O";
(7) each C₂-C₆ alkynyl is independently 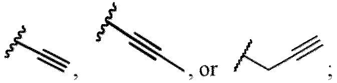
(8) each C₂-C₆ alkenyl is independently 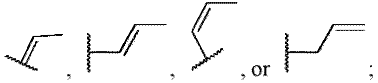
(9) each "C₆-C₁₀ monocyclic or bicyclic aryl" is independently phenyl or naphthyl;
(10) each "8- to 12-membered bicyclic heteroaryl with 1, 2, or 3 heteroatoms selected from 1, 2, or 3 of N, O, and S" is independently "9- or 10-membered bicyclic heteroaryl with 1, 2, or 3 heteroatoms selected from 1, 2, or 3 of N, O, and S";
(11) each "3- to 15-membered monocyclic or bicyclic heteroaryl with 1, 2, or 3 heteroatoms selected from 1, 2, or 3 of N, O, and S" is independently "5- to 10-membered monocyclic or bicyclic heteroaryl with 1, 2, or 3 heteroatoms selected from 1, 2, or 3 of N, O, and S";
(12) each 5- to 15-membered monocyclic, bicyclic, or tricyclic saturated or unsaturated carbocyclic ring is independently a 5- to 6-membered monocyclic, 6- to 10-membered bicyclic, or 8- to 15-membered tricyclic saturated or unsaturated carbocyclic ring;
(13) each "5- to 15-membered monocyclic, bicyclic, or tricyclic saturated or unsaturated heterocyclic ring with 1, 2, or 3 heteroatoms selected from 1, 2, or 3 of N, O, and S" is independently "5- to 6-membered monocyclic, 6- to 10-membered bicyclic, or 8- to 15-membered tricyclic saturated or unsaturated heterocyclic ring with 1, 2, or 3 heteroatoms selected from 1, 2, or 3 of N, O, and S";
(14) each 3- to 15-membered monocyclic or bicyclic saturated or unsaturated carbocyclic ring is independently a 3- to 6-membered monocyclic or 6- to 10-membered bicyclic saturated or unsaturated carbocyclic ring;
(15) each "3- to 15-membered monocyclic or bicyclic saturated or unsaturated heterocyclic ring with 1, 2, or 3 heteroatoms selected from 1, 2, or 3 of N, O, and S" is independently "3- to 6-membered monocyclic saturated heterocyclic ring or 6- to 10-membered bicyclic saturated or unsaturated heterocyclic ring with 1 or 2 heteroatoms selected from 1 or 2 of N and O".

5. The compound of formula (I), the pharmaceutically acceptable salt thereof, or the solvate of the pharmaceutically acceptable salt thereof according to at least one of claims 1 to 3, wherein the compound of formula (I) satisfies one or more of the following conditions:
(1) each C₁-C₆ alkyl is independently methyl or ethyl;
(2) each C₁-C₆ alkoxy is independently methoxy or ethoxy;
(3) each halogen is independently F or Cl;
(4) each C₃-C₆ cycloalkyl is independently cyclopropyl;
(5) each "5- to 6-membered monocyclic heteroaryl with 1, 2, or 3 heteroatoms selected from 1, 2, or 3 of N, O, and S" is independently 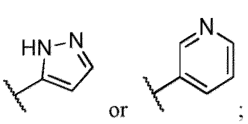
(6) each "3- to 6-membered heterocycloalkyl with 1, 2, or 3 heteroatoms selected from 1, 2, or 3 of N, O, and S" is independently 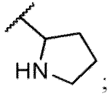
(7) each C₂-C₆ alkynyl is independently 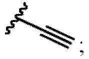
(8) each C₂-C₆ alkenyl is independently 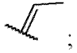
(9) each "C₆-C₁₀ monocyclic or bicyclic aryl" is independently phenyl;
(10) each "8- to 12-membered bicyclic heteroaryl with 1, 2, or 3 heteroatoms selected from 1, 2, or 3 of N, O, and S" is independently 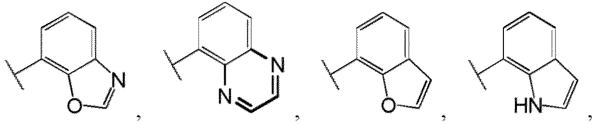 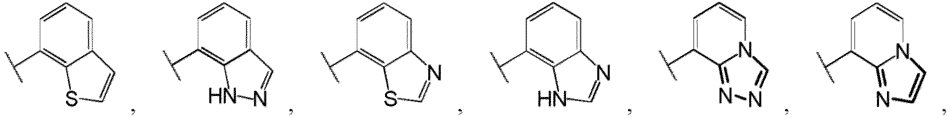 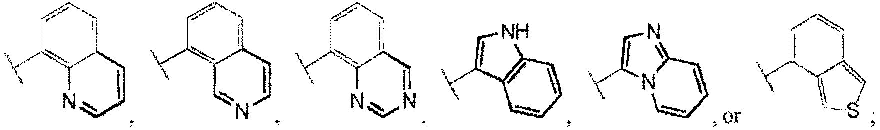
(11) each "3- to 15-membered monocyclic or bicyclic heteroaryl with 1, 2, or 3 heteroatoms selected from 1, 2, or 3 of N, O, and S" is independently 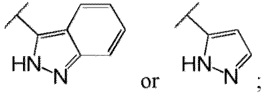
(12) each 5- to 15-membered monocyclic, bicyclic, or tricyclic saturated or unsaturated carbocyclic ring is independently a 5- to 6-membered monocyclic, 6- to 10-membered bicyclic, or 8- to 15-membered tricyclic saturated carbocyclic ring;
(13) each 3- to 15-membered monocyclic or bicyclic saturated or unsaturated carbocyclic ring is independently a 3- to 6-membered monocyclic saturated carbocyclic ring or 6- to 10-membered bicyclic saturated or unsaturated carbocyclic ring.

6. The compound of formula (I), the pharmaceutically acceptable salt thereof, or the solvate of the pharmaceutically acceptable salt thereof according to claim 1, wherein the compound of formula (I) satisfies one or more of the following conditions:
(1) W is CH-R_{W};
(2) R_{W} is H, OH, or C₁-C₆ alkyl;
(3) R₆ is -CONHR₆₋₁;
(4) R₆₋₁ is H or C₁-C₆ alkyl;
(5) R₁₋₁ is oxo (=O), OH, NH₂, CN, halogen, -S(O)₂-C₁-C₆ alkyl, C₁-C₆ alkyl, or C₁-C₆ alkoxy substituted by 1, 2, or 3 R₁₋₁₋₂ groups;
(6) R₁₋₁₋₂ is OH;
(7) R₁₋₂ is OH or halogen.

7. The compound of formula (I), the pharmaceutically acceptable salt thereof, or the solvate of the pharmaceutically acceptable salt thereof according to claim 1, wherein the compound of formula (I) satisfies one or more of the following conditions:
(1) W is CH-R_{W}, and R_{W} is H, OH, or C₁-C₆ alkyl, such as CH₂, CH-OH, or CH-CH₃;
(2) R₆ is 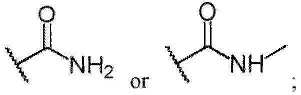
(3) R₁₋₁ is oxo (=O), OH, NH₂, CN, halogen, -S(O)₂-C₁-C₆ alkyl, C₁-C₆ alkyl, or C₁-C₆ alkoxy substituted by 1, 2, or 3 R₁₋₁₋₂ groups, and R₁₋₁₋₂ is OH.

8. The compound of formula (I), the pharmaceutically acceptable salt thereof, or the solvate of the pharmaceutically acceptable salt thereof according to at least one of claims 1 to 3, wherein the compound of formula (I) satisfies one or more of the following conditions:
(1) R₂ is halogen;
(2) R₁₋₆ is C₃-C₆ cycloalkyl.

9. The compound of formula (I), the pharmaceutically acceptable salt thereof, or the solvate of the pharmaceutically acceptable salt thereof according to at least one of claims 1 to 3, wherein the compound of formula (I) satisfies one or more of the following conditions:
(1) Y is CH;
(2) W is CH₂;
(3) Z is O;
(4) R₂ is F;
(5) R₆ is H, CN,

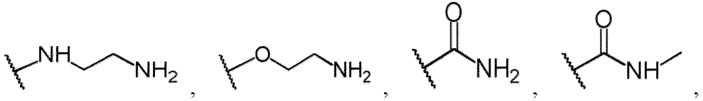

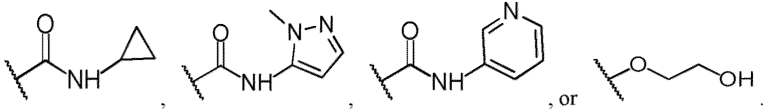

10. The compound of formula (I), the pharmaceutically acceptable salt thereof, or the solvate of the pharmaceutically acceptable salt thereof according to claim 1, wherein the compound of formula (I) satisfies one or more of the following conditions:
(1) in scheme 1, 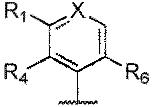 is 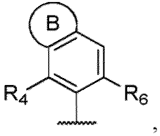 ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
R₁₋₁ is oxo (=O), OH, NH₂, CN, halogen, -S(O)₂-C₁-C₆ alkyl, C₁-C₆ alkyl, or C₁-C₆ alkoxy substituted by 1, 2, or 3 R₁₋₁₋₂ groups;
R₁₋₂ is OH or halogen;
R₁₋₁₋₂ is OH;
(2) in schemes 2 to 5, 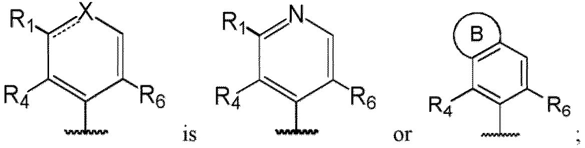
R₁ is -L₁-R₁₋₄;
L₁ is -O-;
R₁₋₄ is C₁-C₆ alkyl substituted by 1, 2, or 3 R₁₋₅ groups; R₁₋₅ is independently OH;
ring B is a 5- to 15-membered monocyclic, bicyclic, or tricyclic saturated or unsaturated carbocyclic ring, a 5- to 15-membered monocyclic, bicyclic, or tricyclic saturated or unsaturated carbocyclic ring substituted by 1, 2, or 3 R₁₋₁ groups, "a 5- to 15-membered monocyclic, bicyclic, or tricyclic saturated or unsaturated heterocyclic ring with 1, 2, or 3 heteroatoms selected from 1, 2, or 3 of N, O, and S", or "a 5- to 15-membered monocyclic, bicyclic, or tricyclic saturated or unsaturated heterocyclic ring with 1, 2, or 3 heteroatoms selected from 1, 2, or 3 of N, O, and S" substituted by 1, 2, or 3 R₁₋₂ groups;
R₁₋₁ is oxo (=O), OH, NH₂, CN, halogen, -S(O)₂-C₁-C₆ alkyl, C₁-C₆ alkyl, or C₁-C₆ alkoxy substituted by 1, 2, or 3 R₁₋₁₋₂ groups;
R₁₋₂ is OH or halogen;
R₁₋₁₋₂ is OH;
(3) in scheme 4, R₄ is OH, NH₂, C₁-C₆ alkoxy, or C₂-C₆ alkynyl, such as 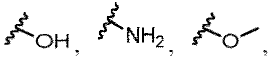 or 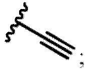
(4) in schemes 1 to 3 and 5, R₄ is independently halogen, OH, NH₂, C₁-C₆ alkoxy, or C₂-C₆ alkynyl, such as F,

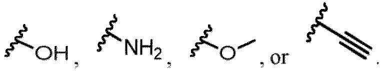

11. The compound of formula (I), the pharmaceutically acceptable salt thereof, or the solvate of the pharmaceutically acceptable salt thereof according to claim 1, wherein the compound of formula (I) satisfies one or more of the following conditions:
(1) in scheme 1, 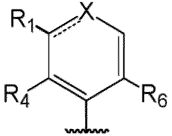 is 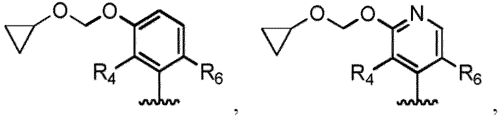 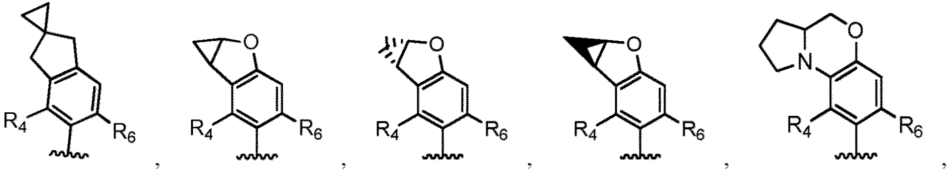 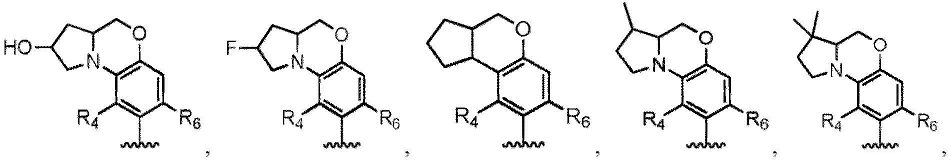 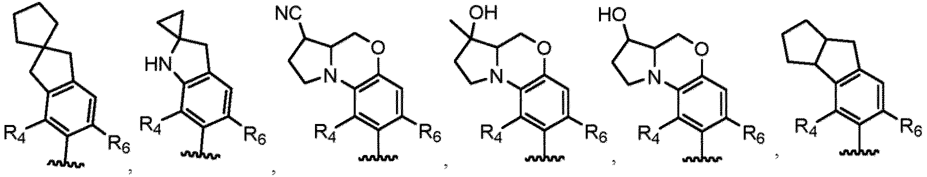 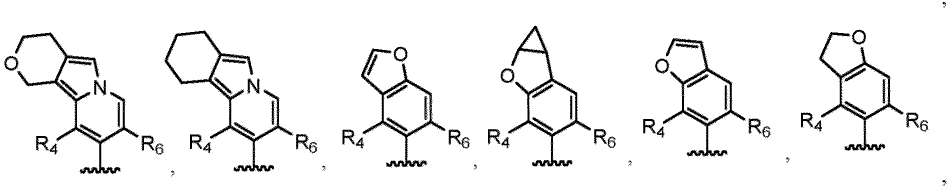 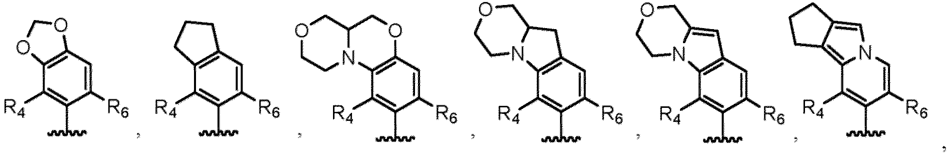 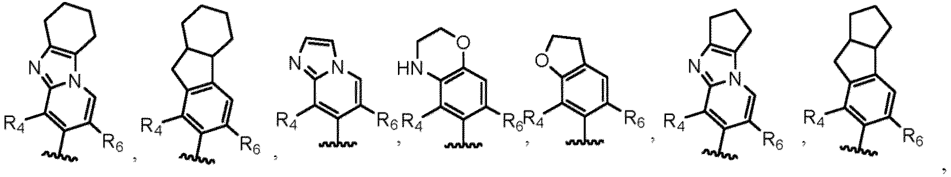 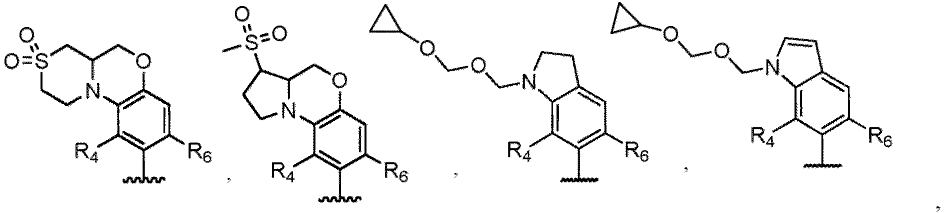 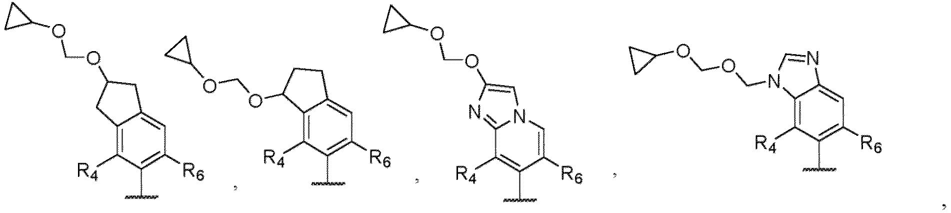 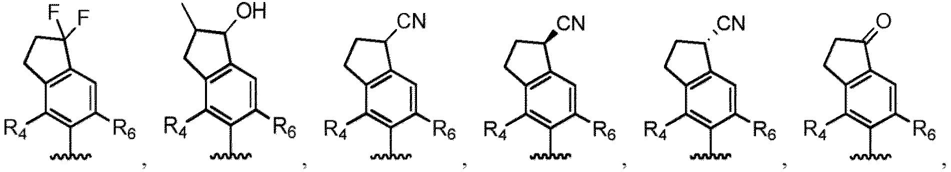 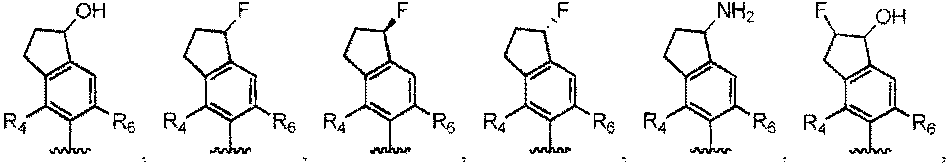 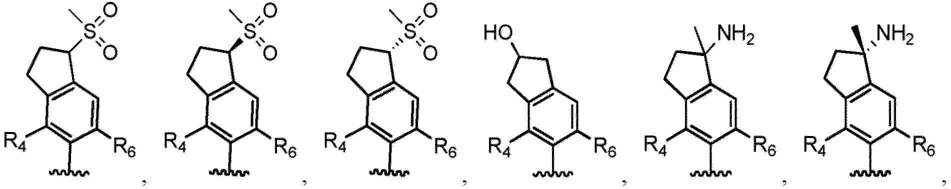 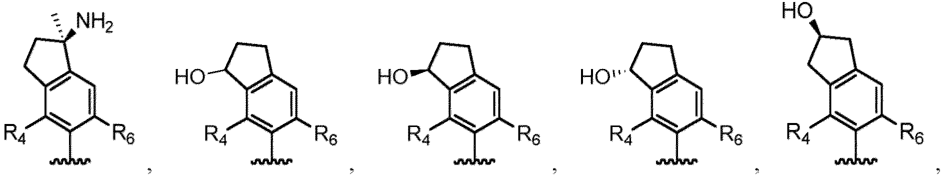 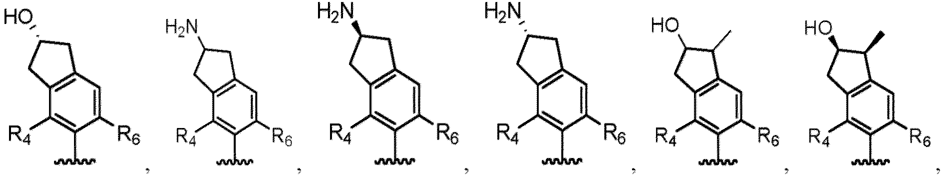 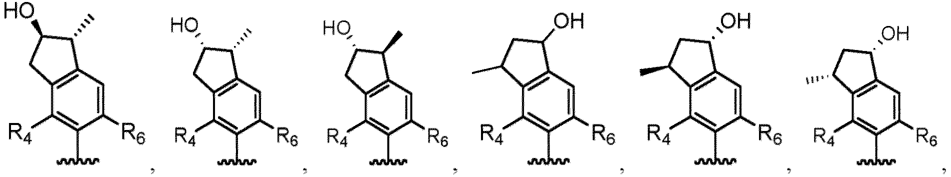 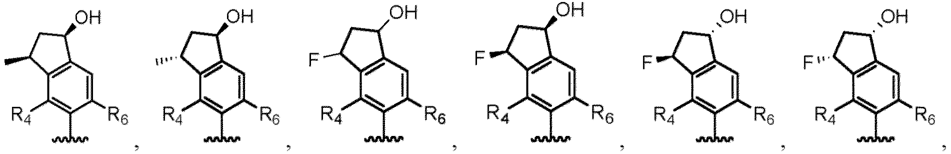 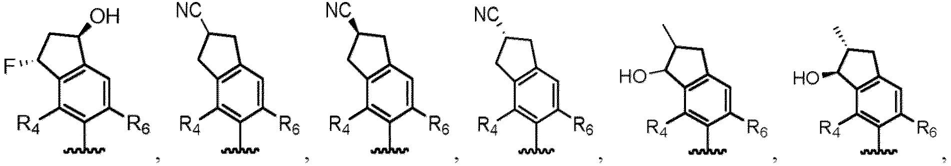 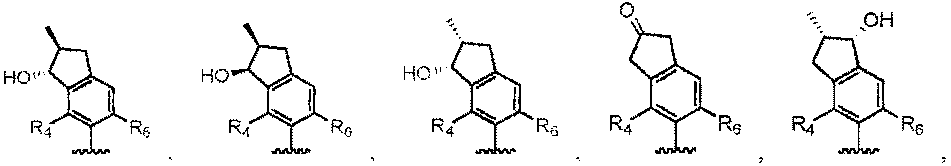 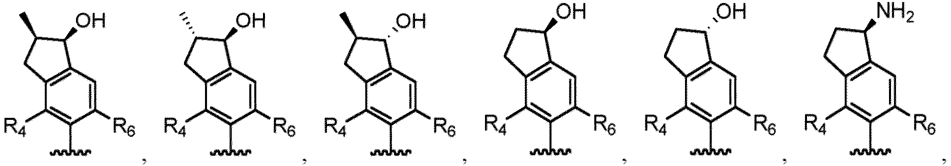 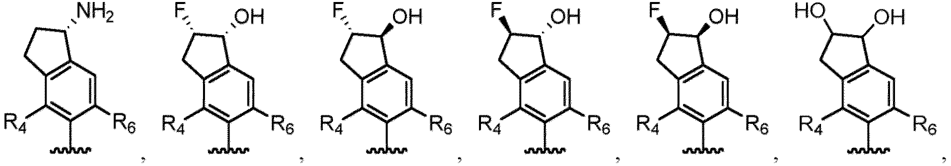 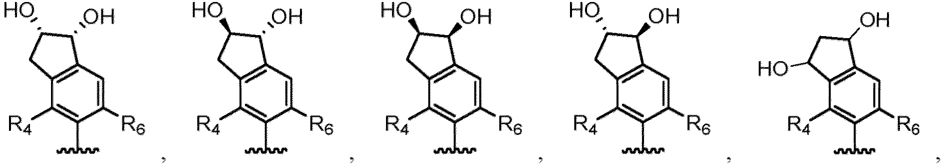 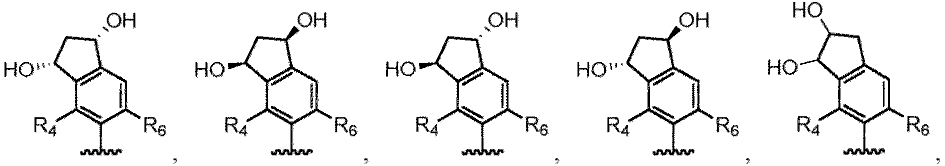 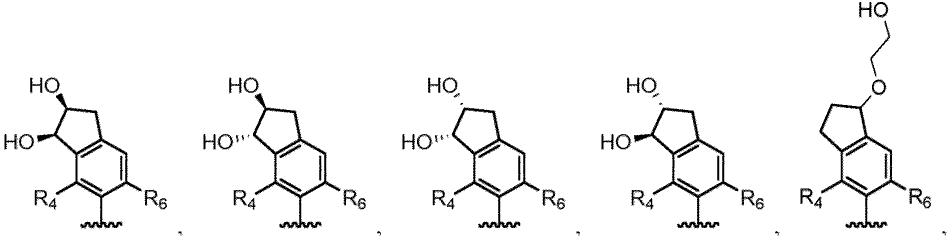 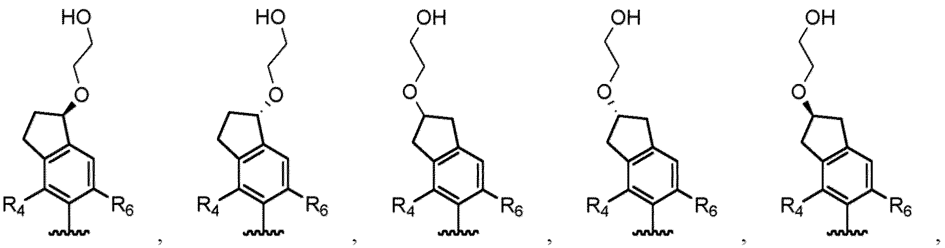 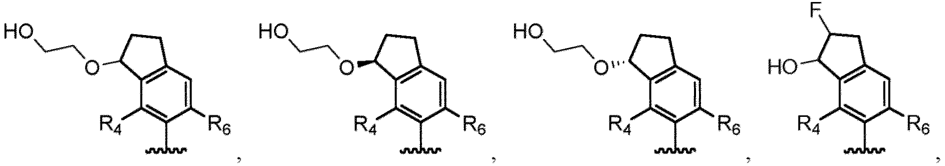 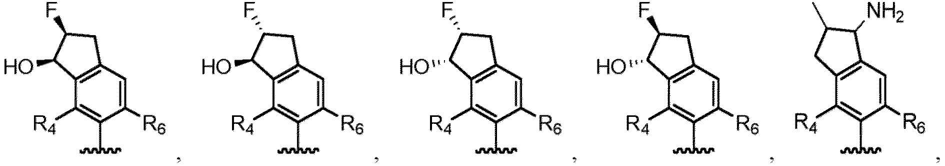 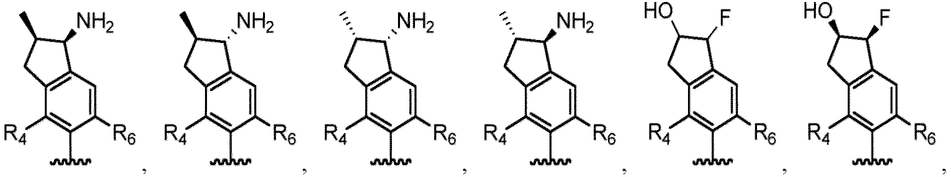 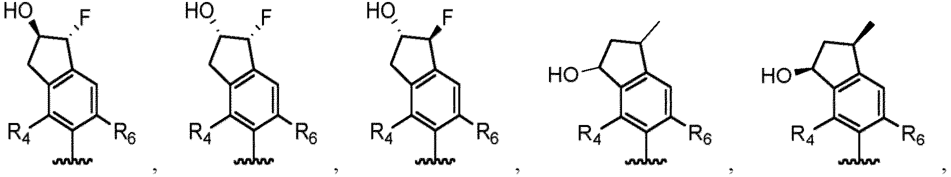 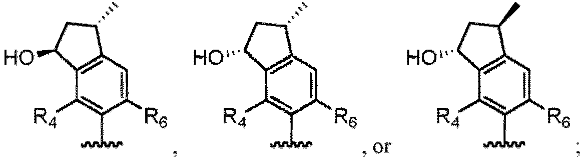
preferably 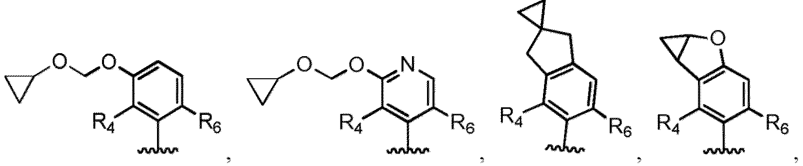 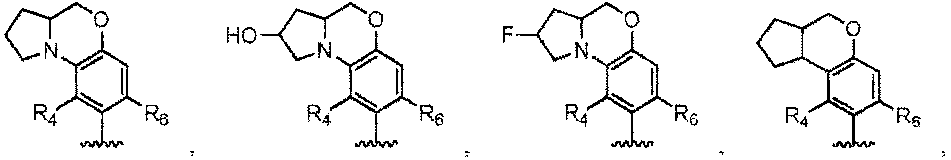 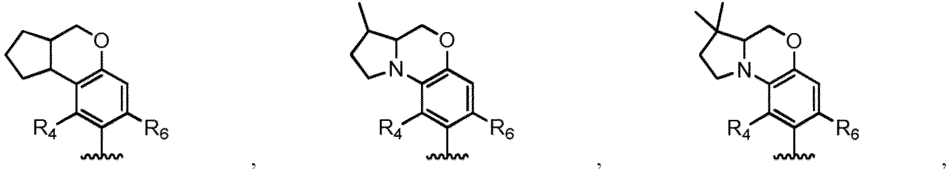 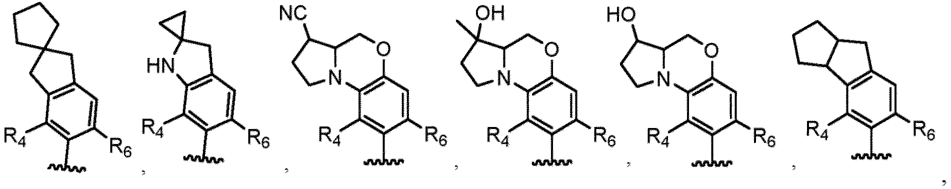 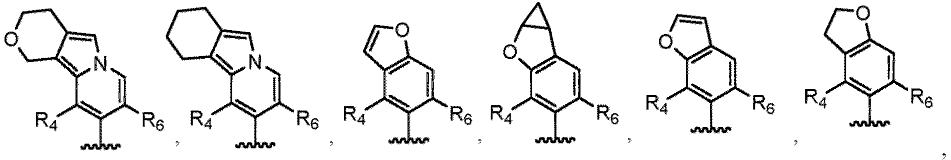 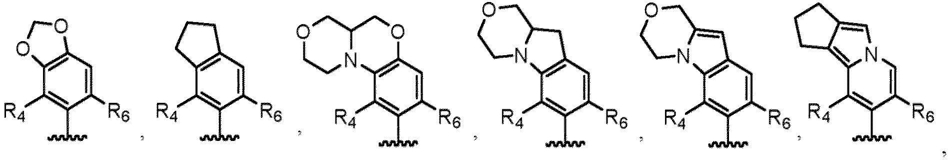 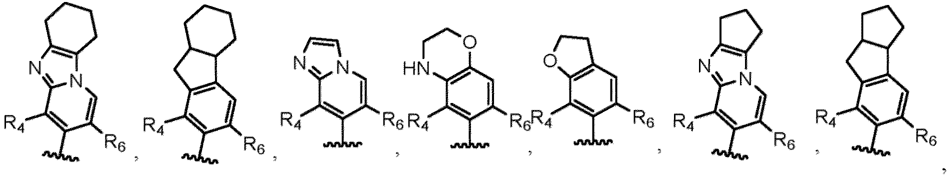 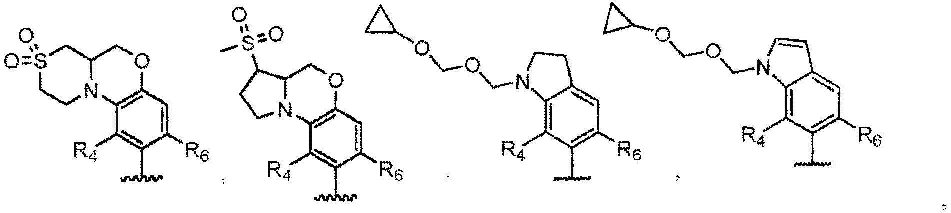 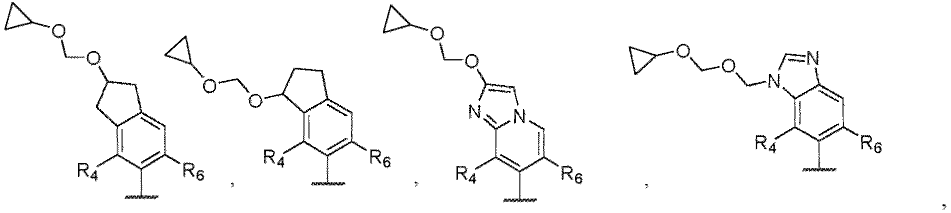 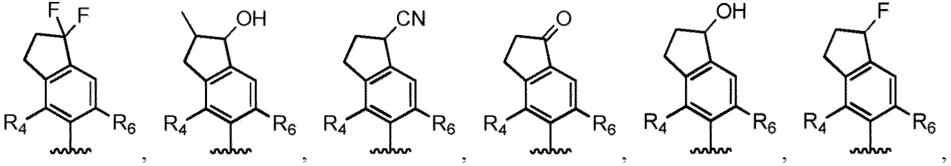 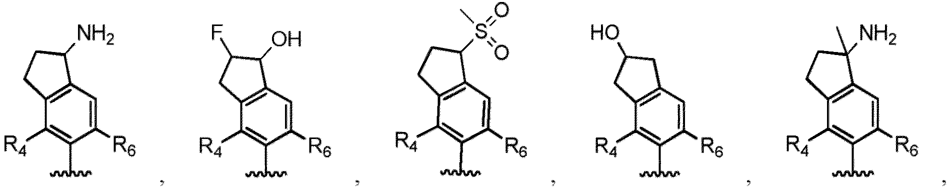 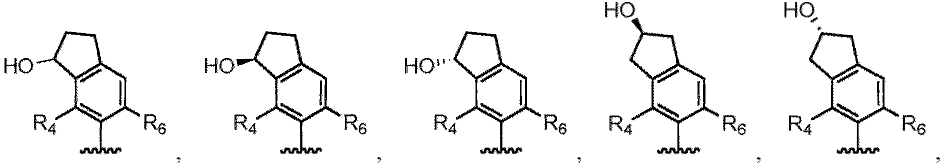 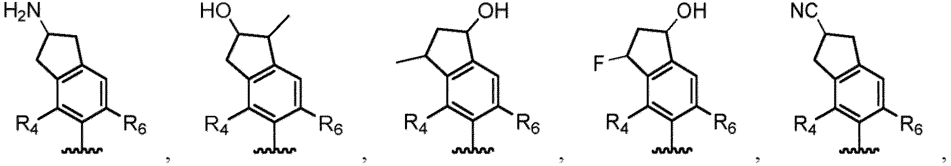 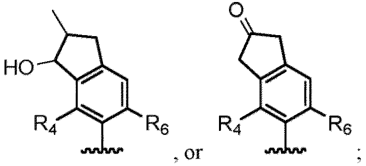
more preferably 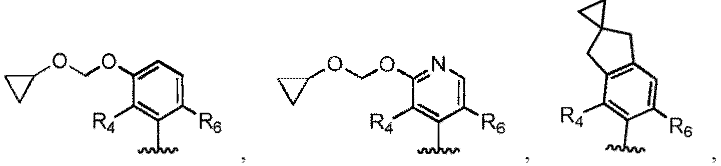 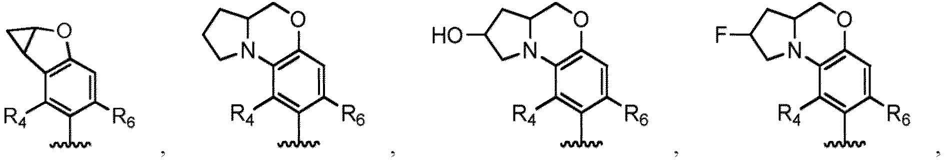 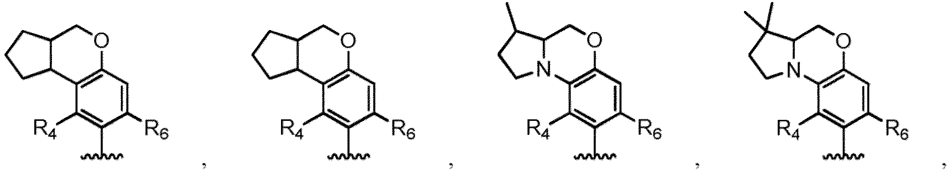 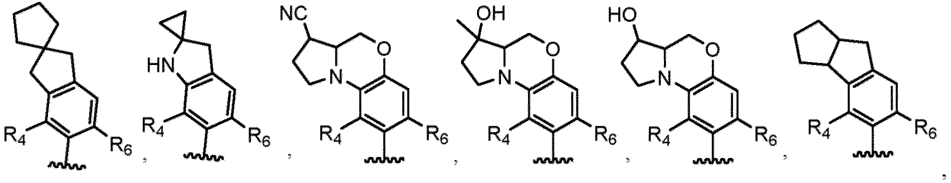 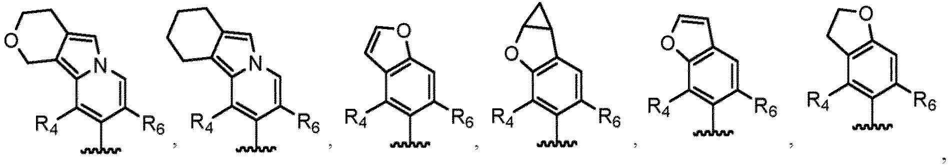 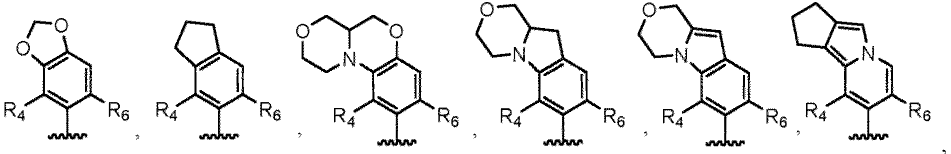 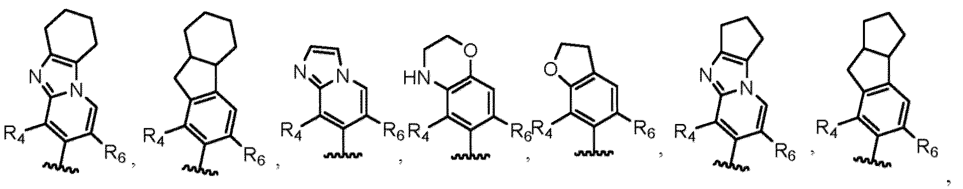 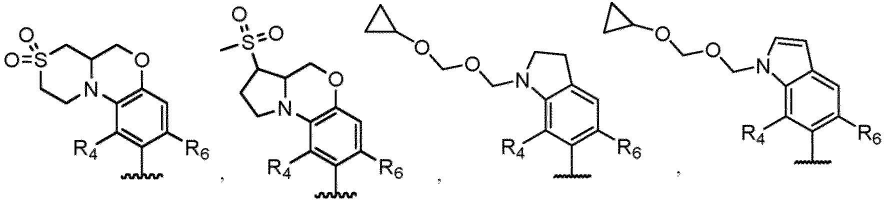 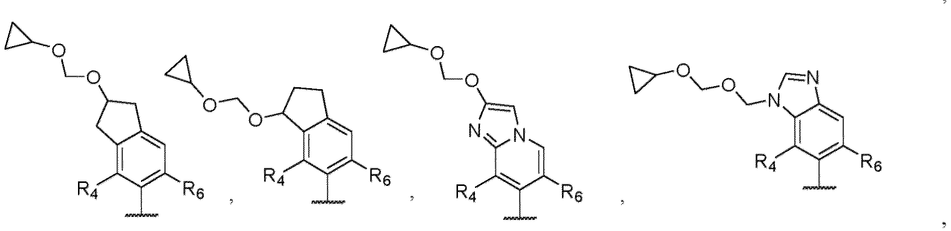 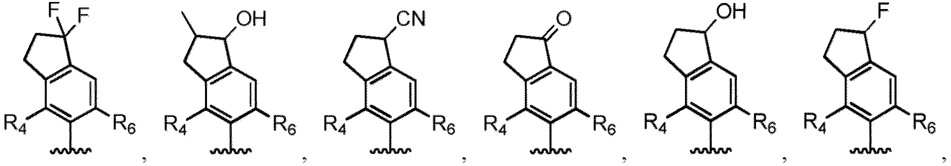 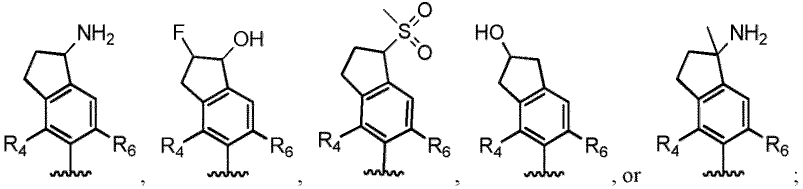
further more preferably 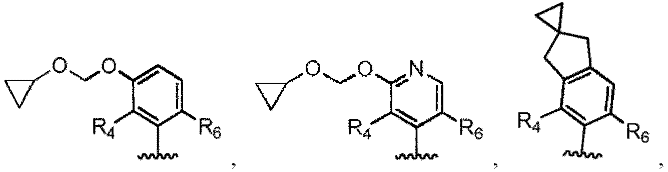 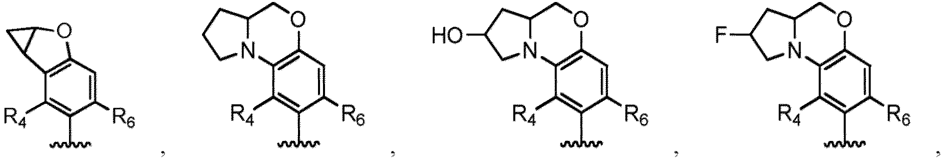 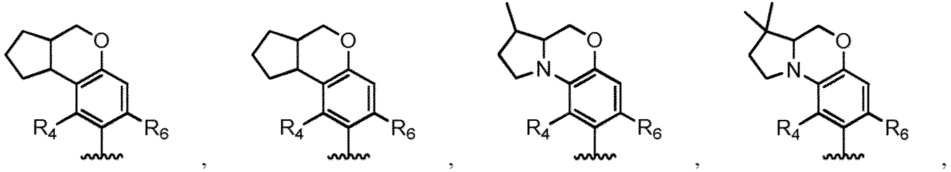 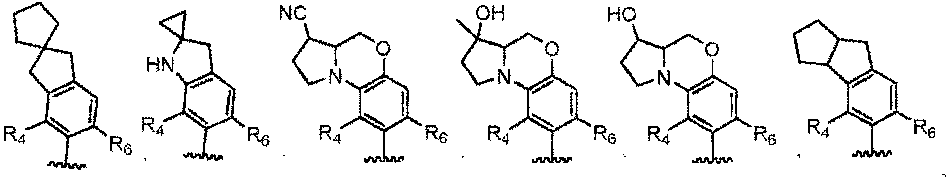 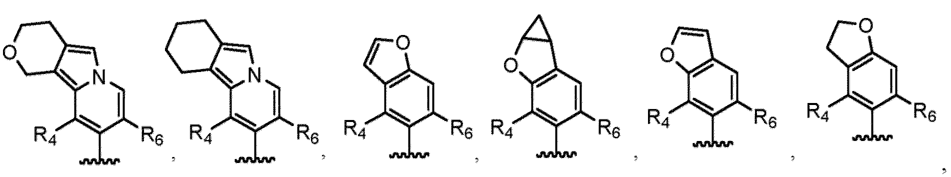 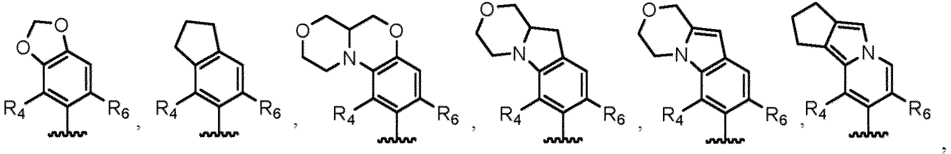 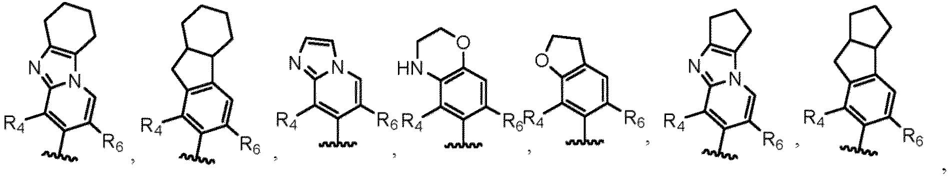 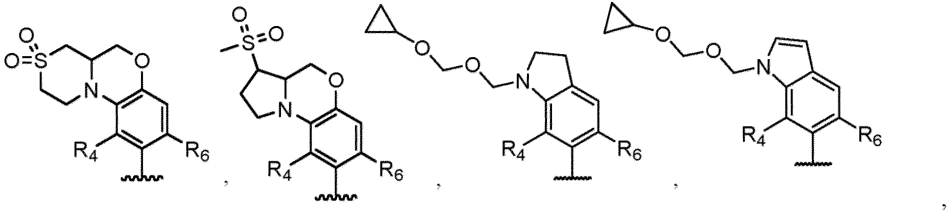 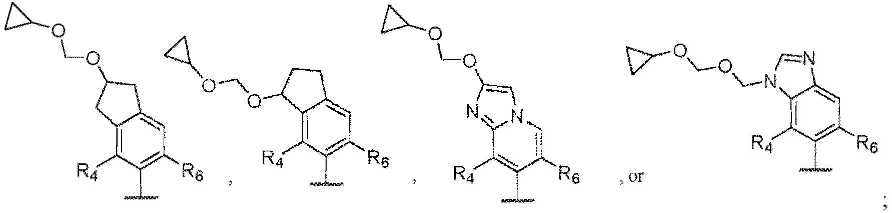
(2) In schemes 2 to 5, 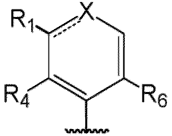 is independently 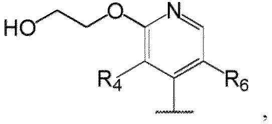 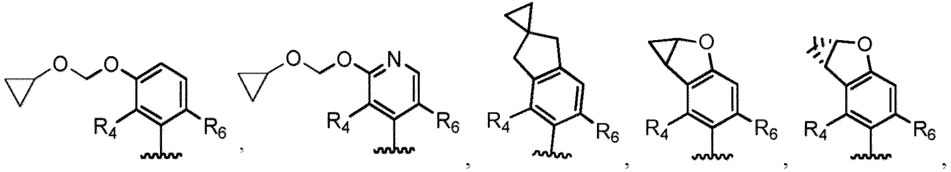 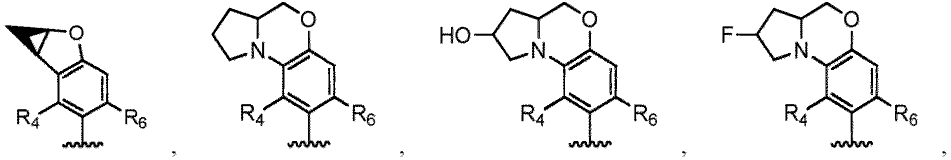 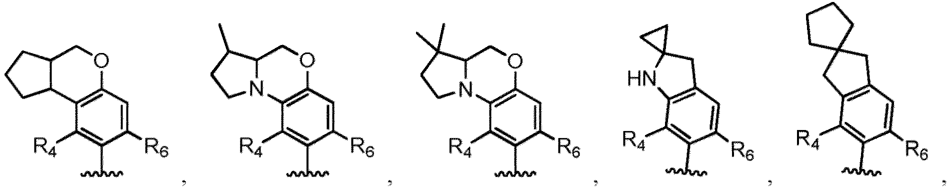 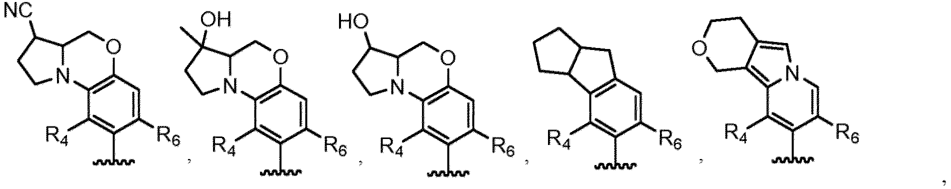 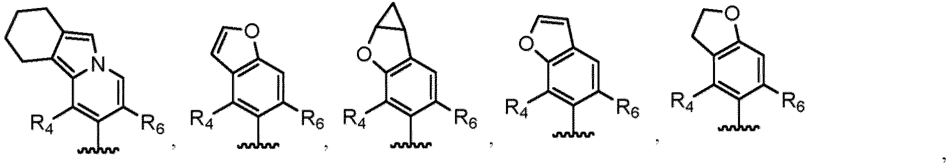 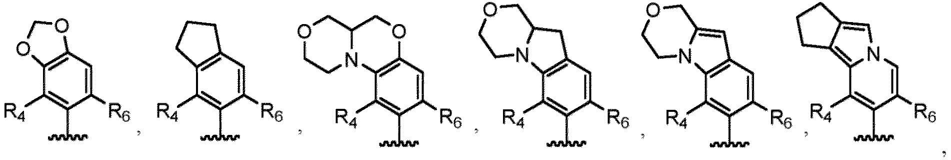 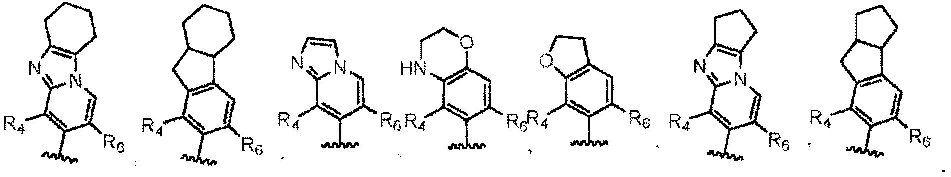 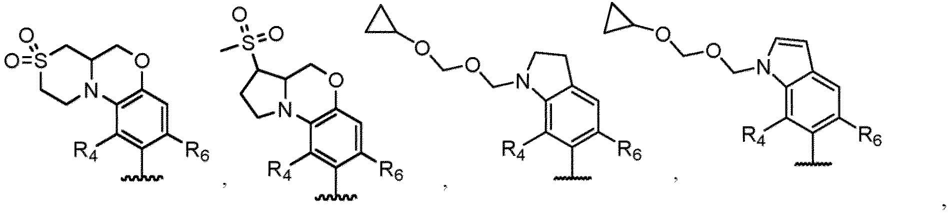 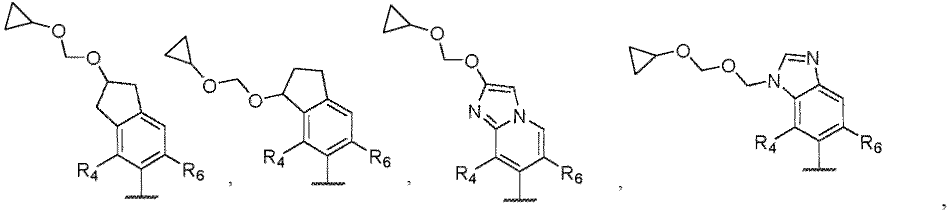 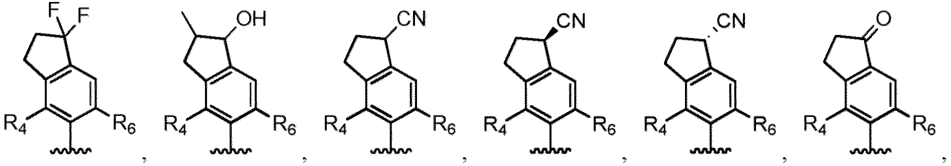 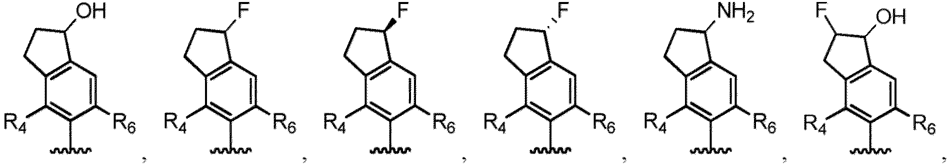 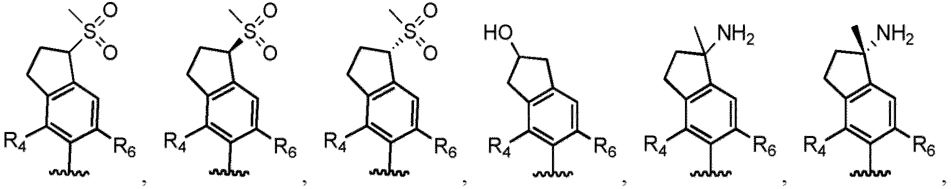 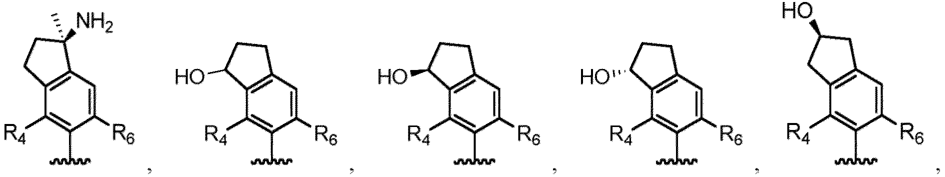 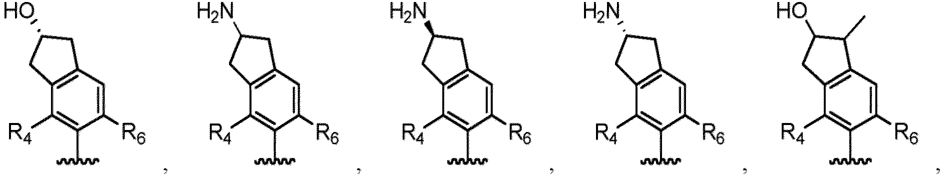 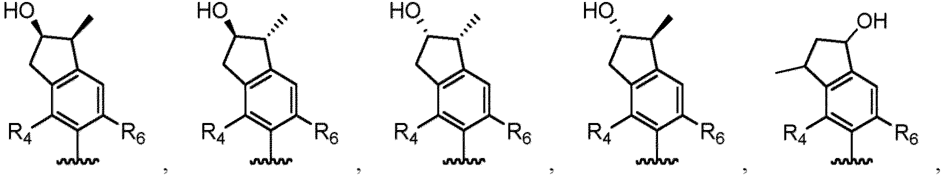 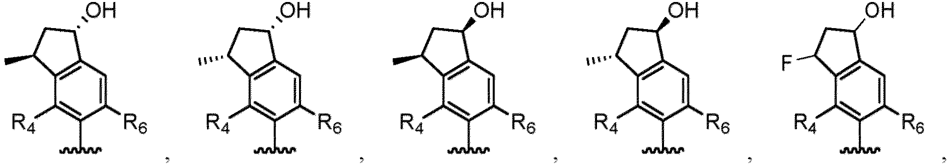 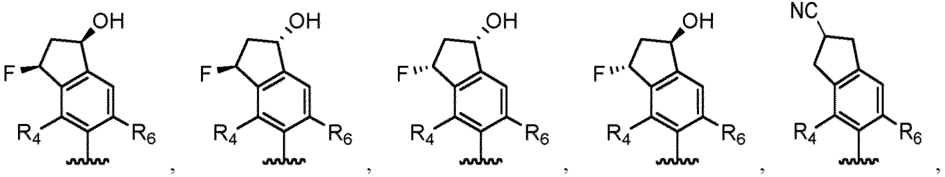 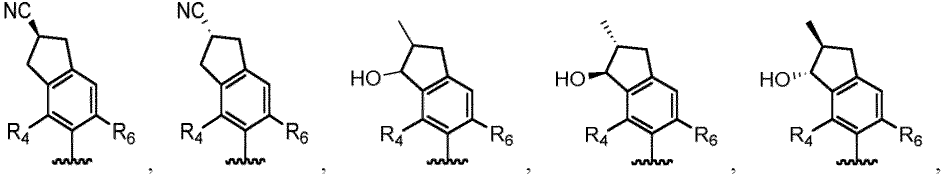 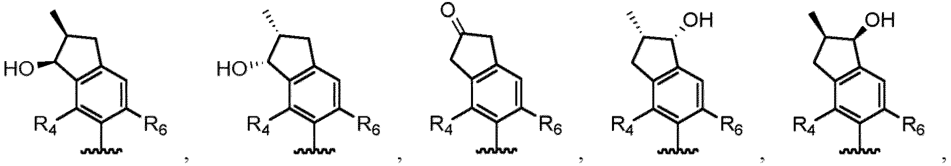 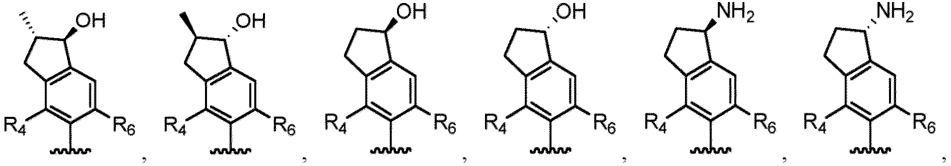 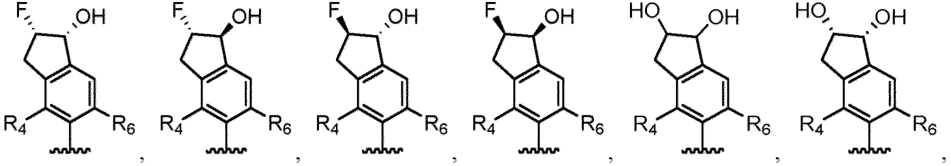 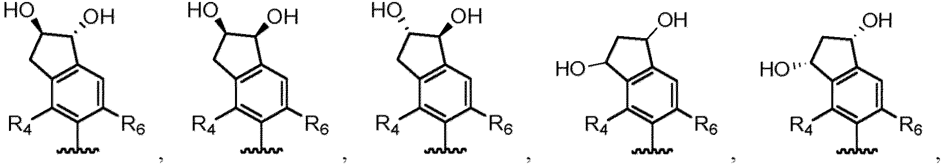 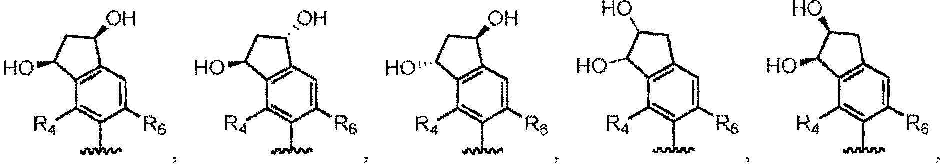 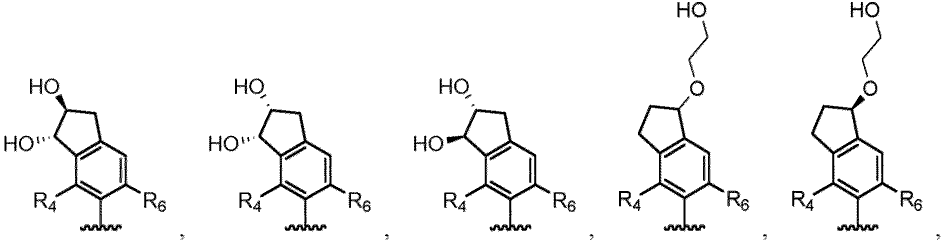 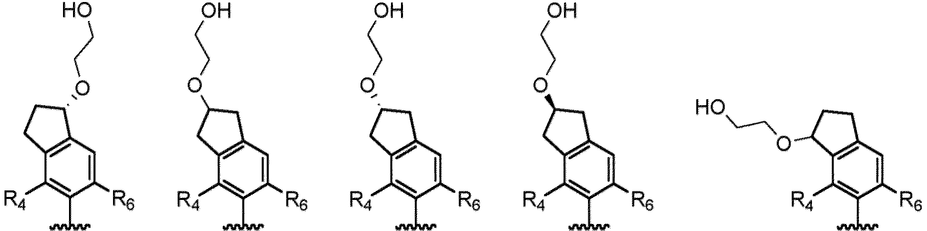 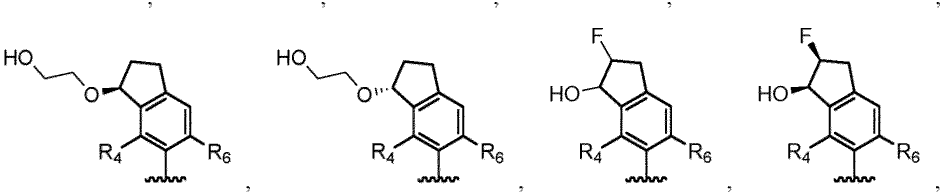 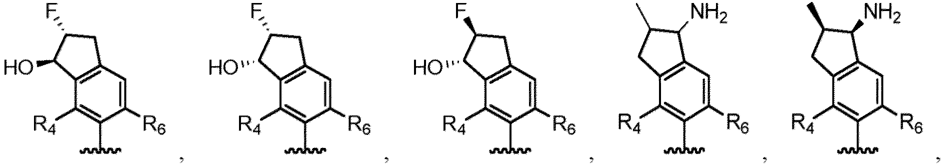 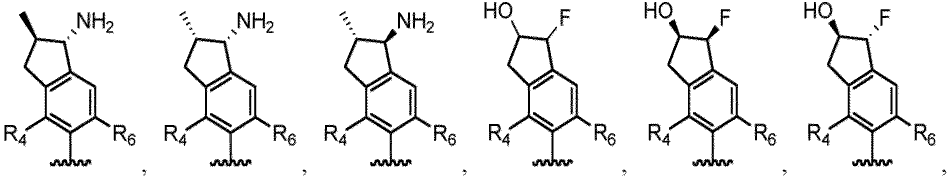 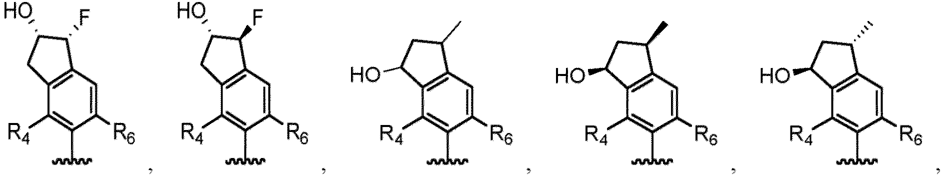 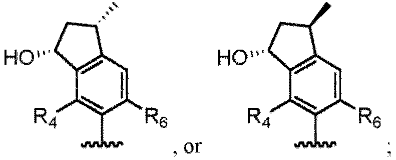
preferably 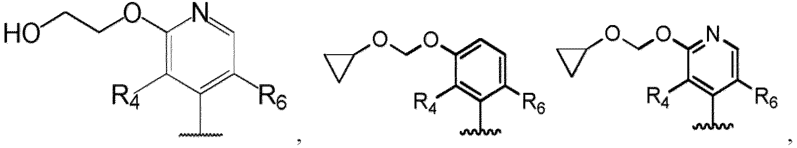 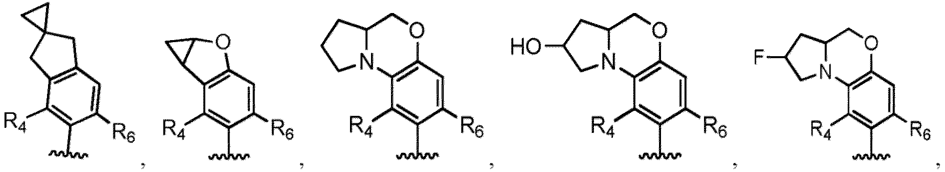 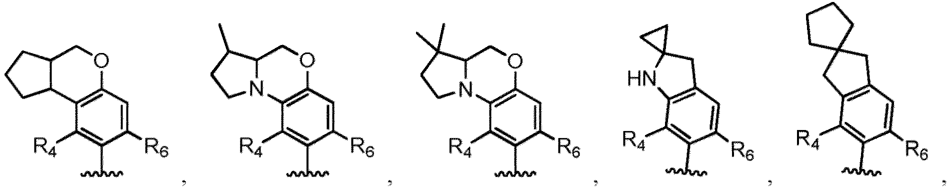 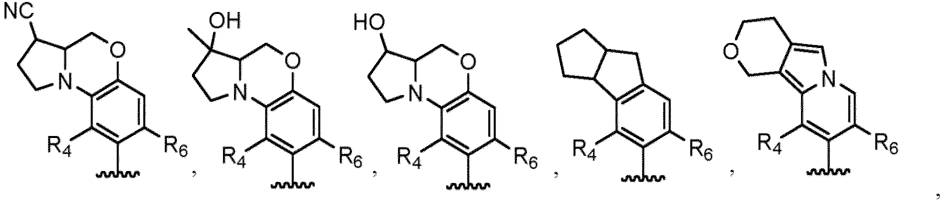 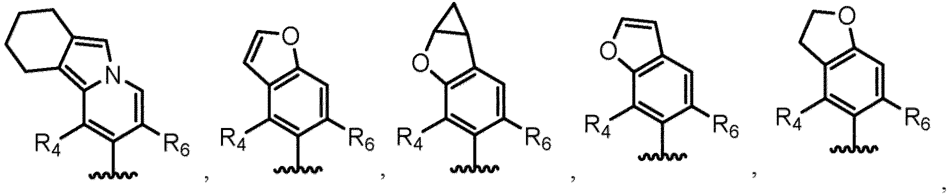 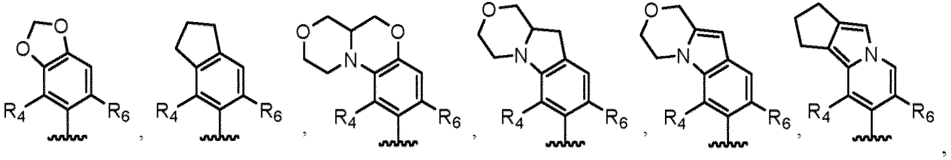 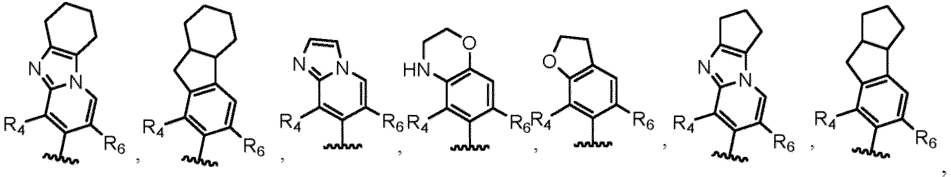 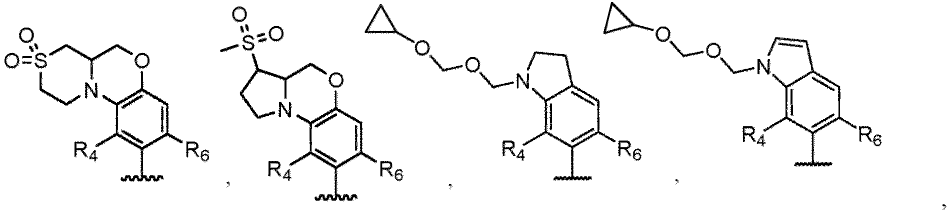 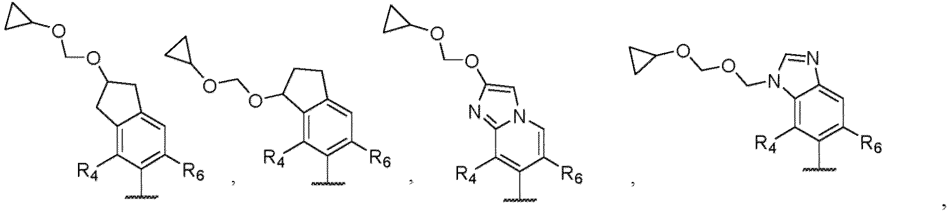 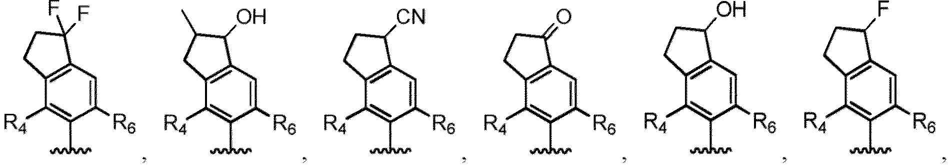 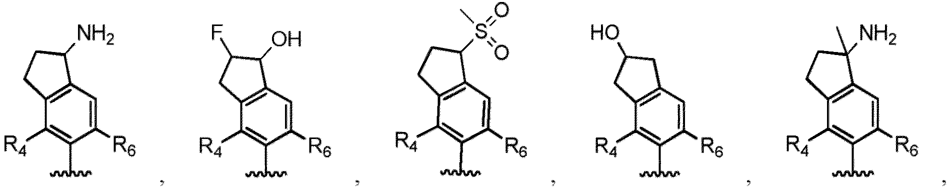 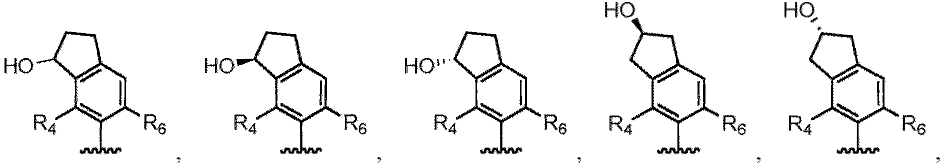 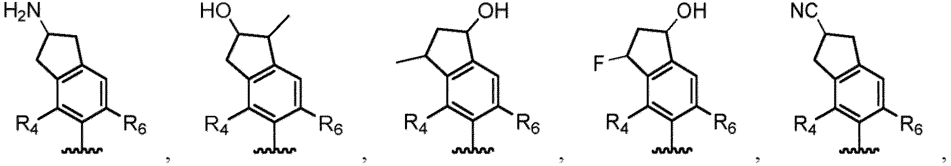 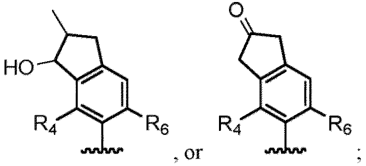
more preferably 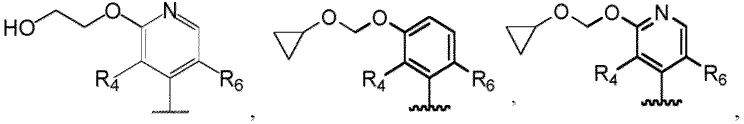 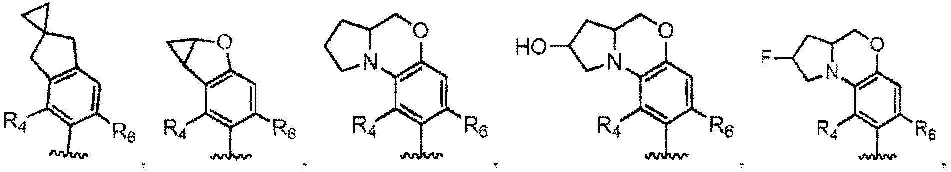 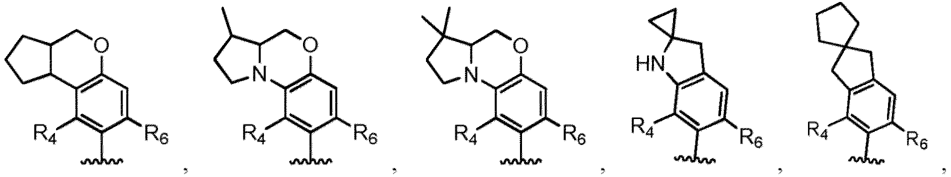 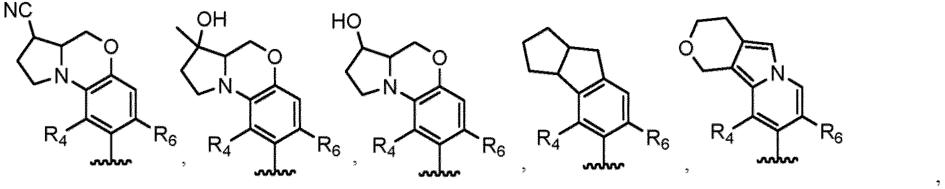 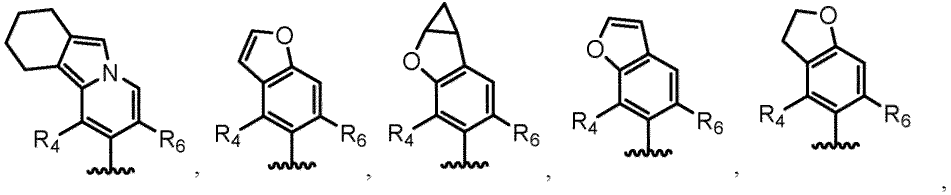 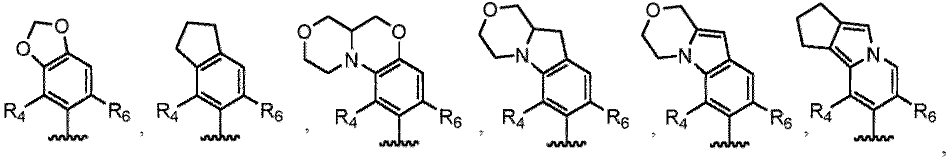 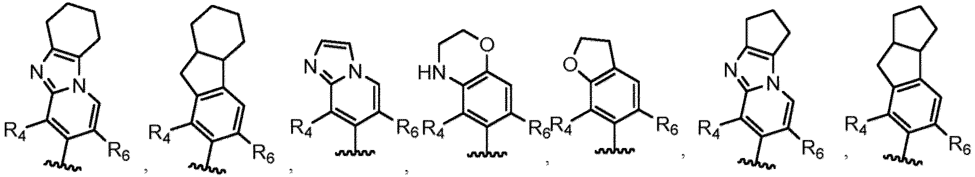 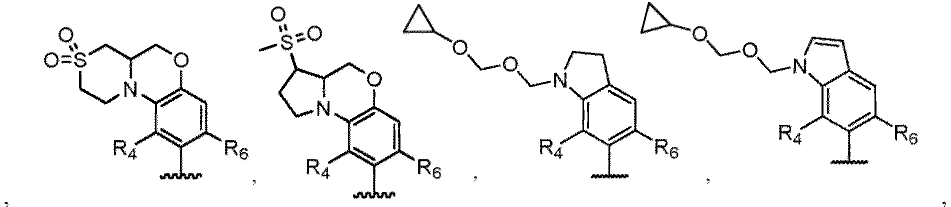 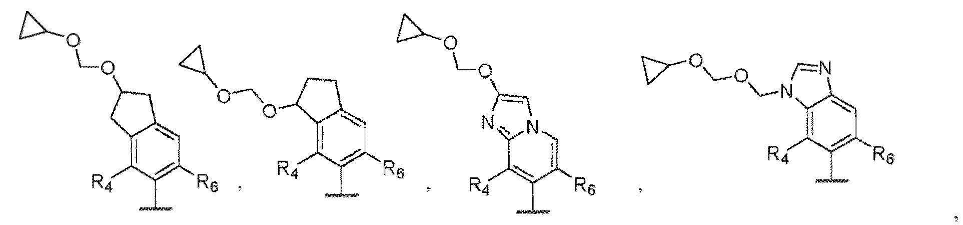 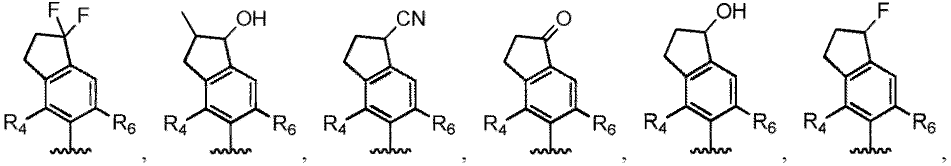 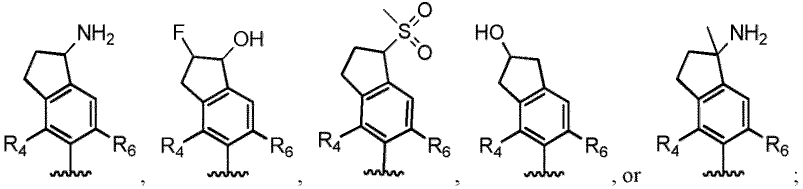
further more preferably 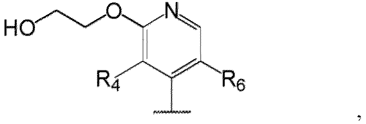 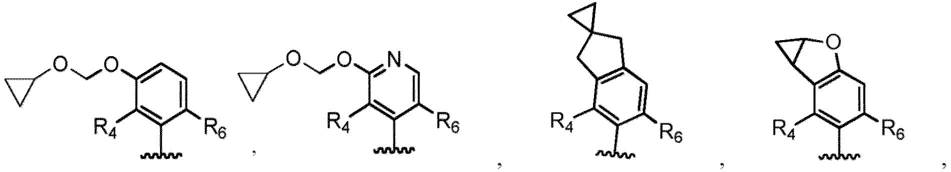 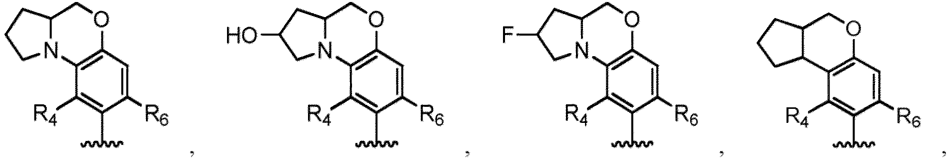 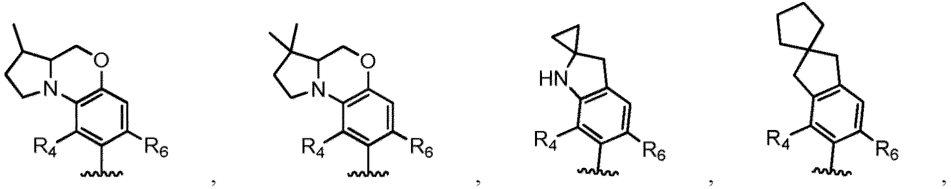 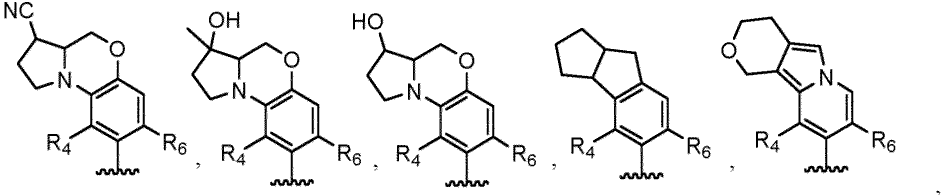 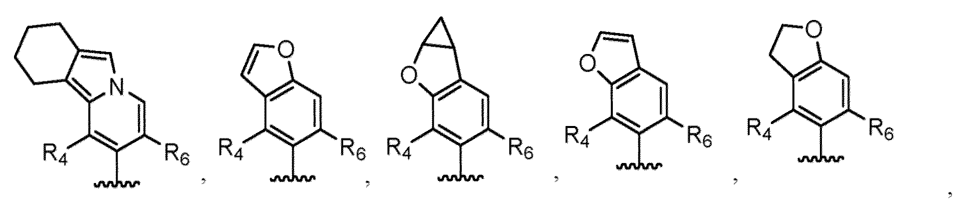 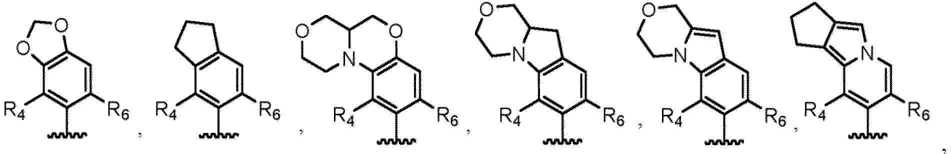 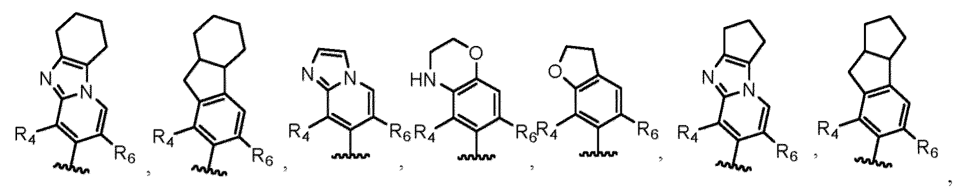 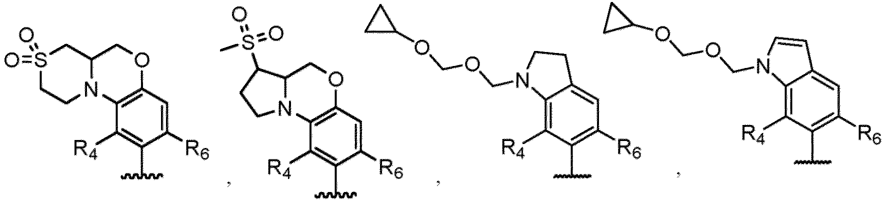 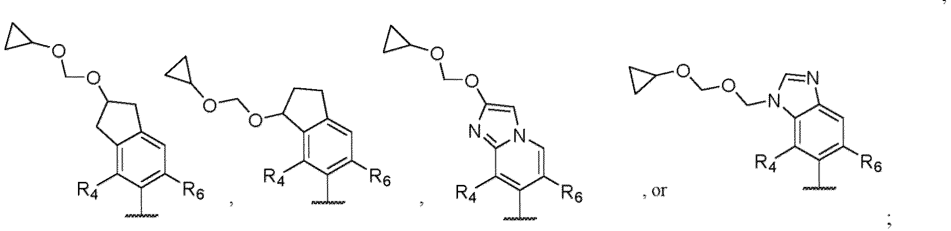
(3) in scheme 4, R₄ is 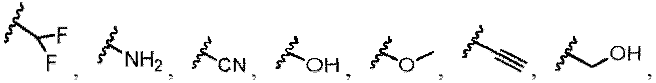 or 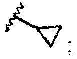
(4) in schemes 1 to 3 and 5, R₄ is independently 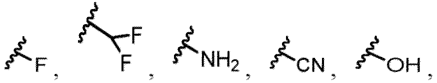 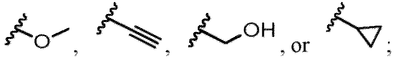
(5) in scheme 5, R₃ is 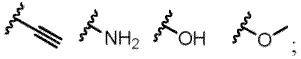
(6) in schemes 1 to 4, R₃ is independently 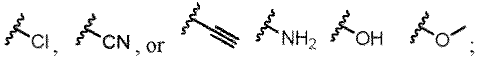
(7) in scheme 3, A is 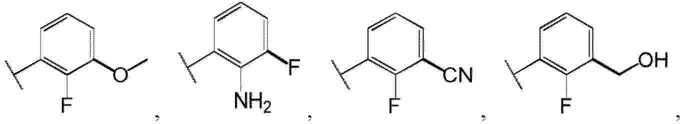 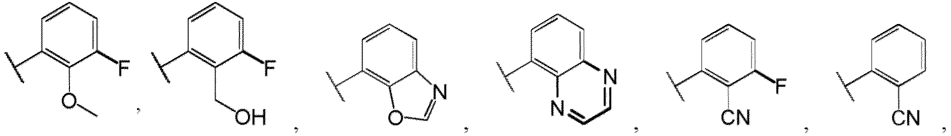 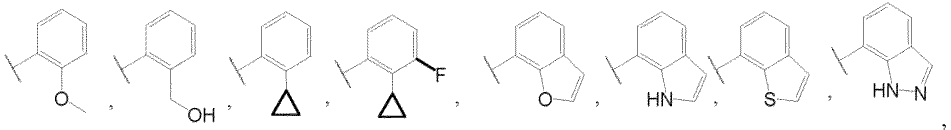 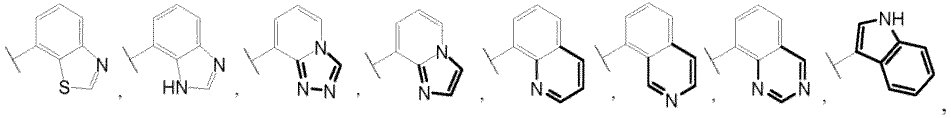 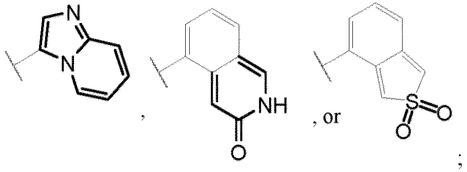
(8) in schemes 1 to 2 and 4 to 5, A is independently 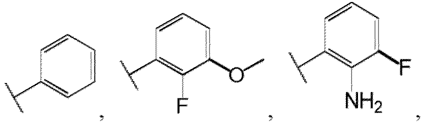 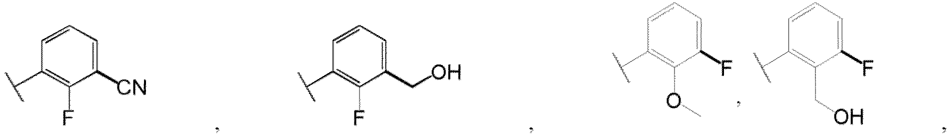 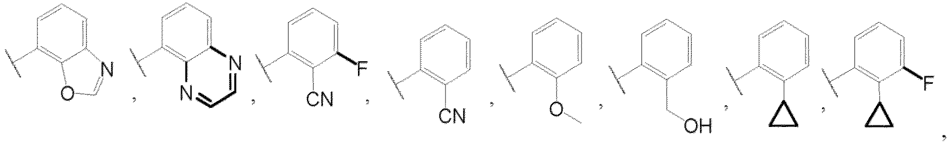 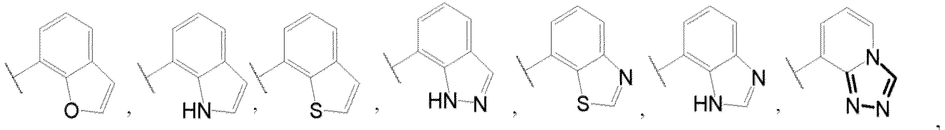 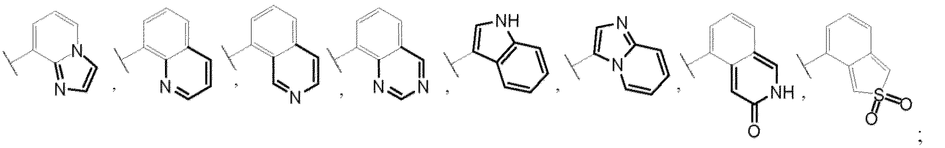
(9) in scheme 2, Q is 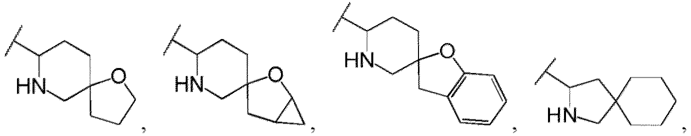 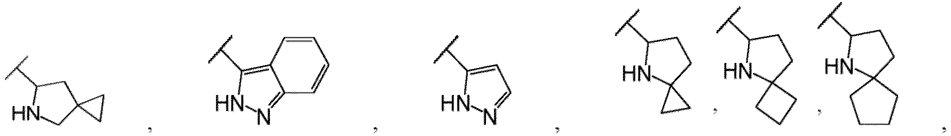 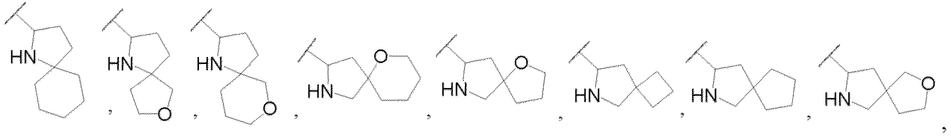 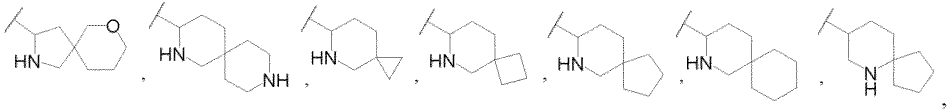 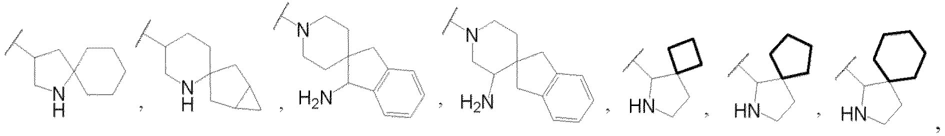 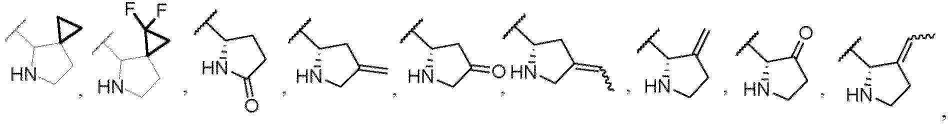 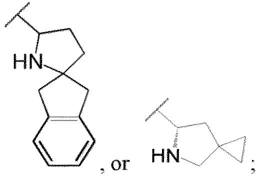
(10) in schemes 1 and 3 to 5, Q is

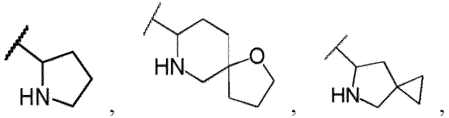

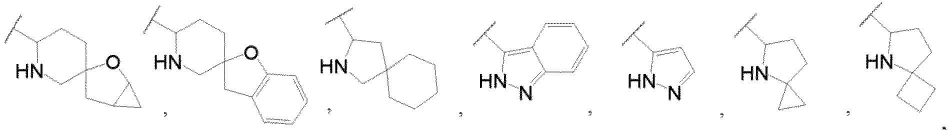

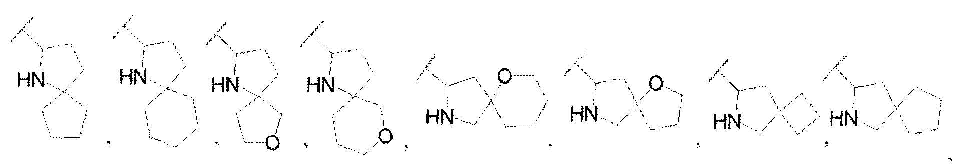

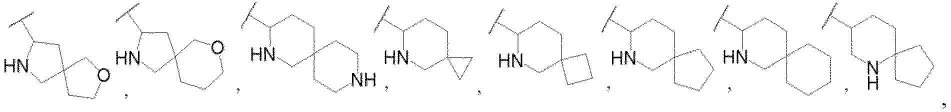

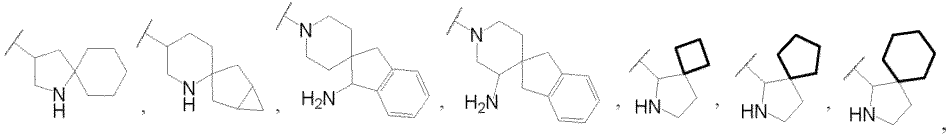

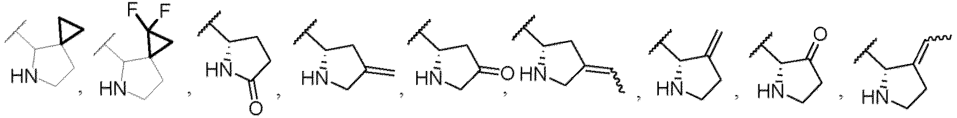

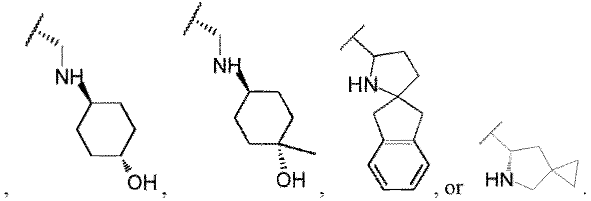

12. The compound of formula (I), the pharmaceutically acceptable salt thereof, or the solvate of the pharmaceutically acceptable salt thereof according to at least one of claims 1 to 3, wherein
the compound of formula (I) is as follows: 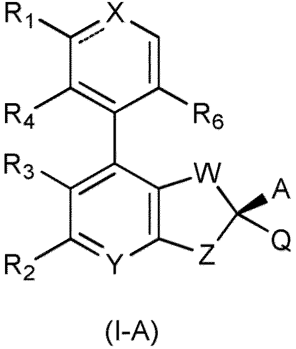
wherein R₁, R₂, R₃, R₄, R₆, X, Y, W, Z, A, and Q are as defined in at least one of claims 1 to 3.

13. The compound of formula (I), the pharmaceutically acceptable salt thereof, or the solvate of the pharmaceutically acceptable salt thereof according to at least one of claims 1 to 3, wherein the compound of formula (I) is as follows: 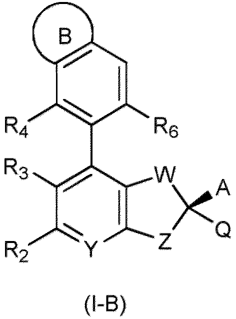 wherein ring B, R₂, R₃, R₄, R₆, Y, W, Z, A, and Q are as defined in at least one of claims 1 to 3.

14. The compound of formula (I), the pharmaceutically acceptable salt thereof, or the solvate of the pharmaceutically acceptable salt thereof according to at least one of claims 1 to 3, wherein the compound of formula (I) is as follows: 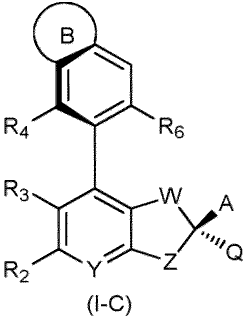 wherein ring B, R₂, R₃, R₄, R₆, Y, W, Z, A, and Q are as defined in at least one of claims 1 to 3.

15. The compound of formula (I), the pharmaceutically acceptable salt thereof, or the solvate of the pharmaceutically acceptable salt thereof according to claim 1 or 2, wherein
the compound of formula (I) is a compound of formula (I-D) or a compound of formula (I-E): 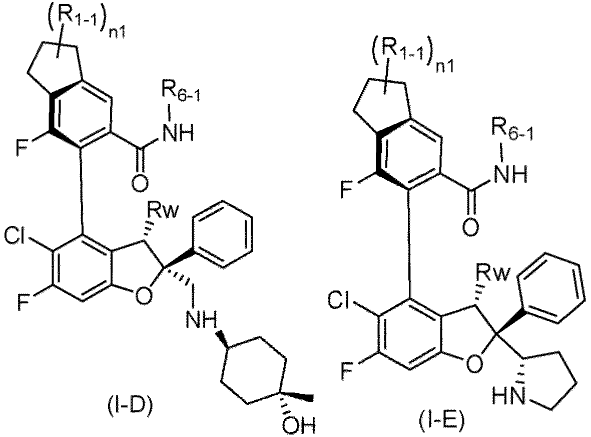
wherein n1 is 0, 1, 2, or 3; R₁₋₁, R₆₋₁, and R_{w} are as defined in at least one of claims 1 to 3.

16. The compound of formula (I), the pharmaceutically acceptable salt thereof, or the solvate of the pharmaceutically acceptable salt thereof according to claim 1, wherein the compound of formula (I) is any one of the following compounds:

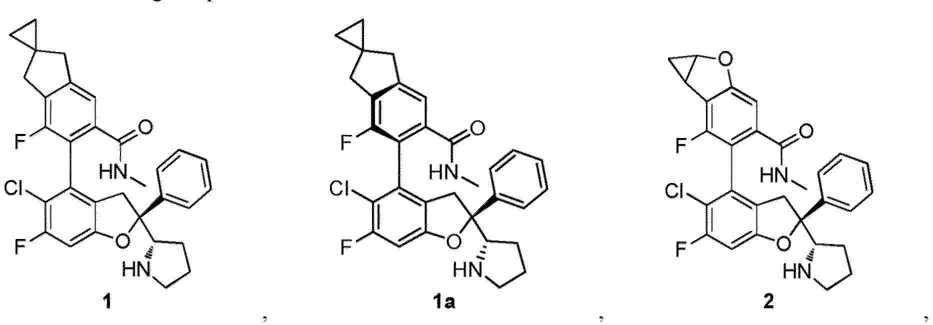

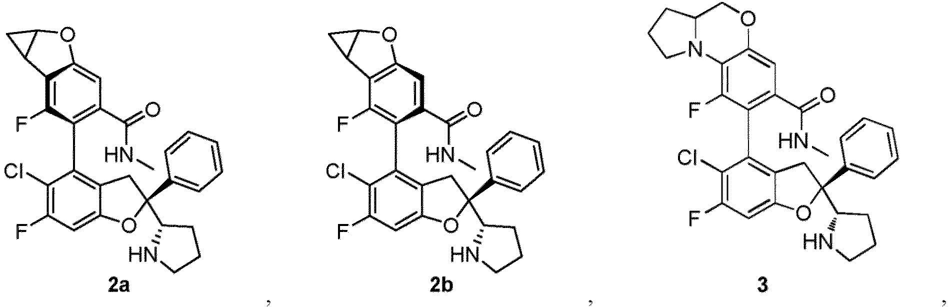

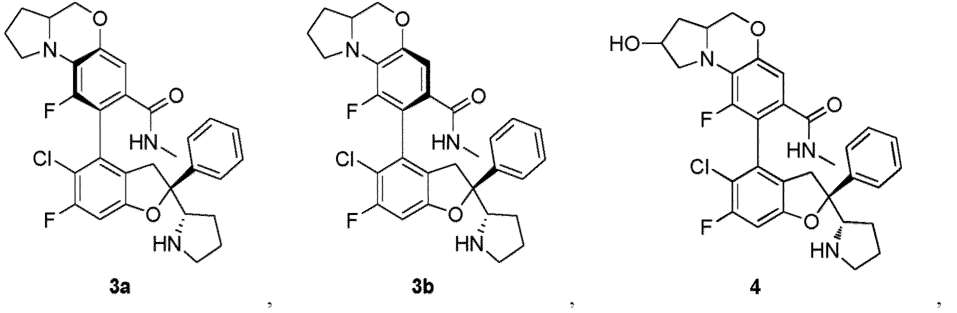

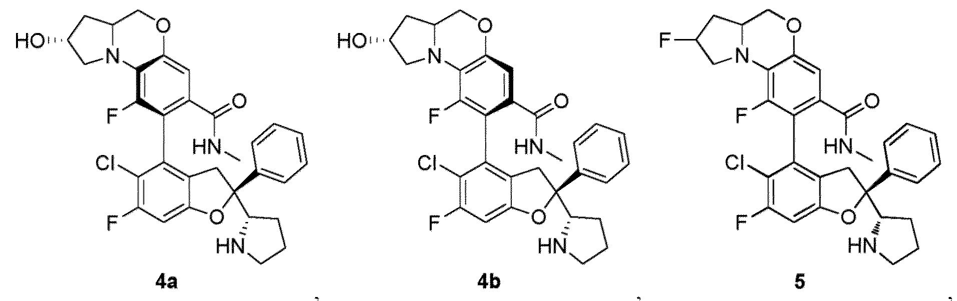

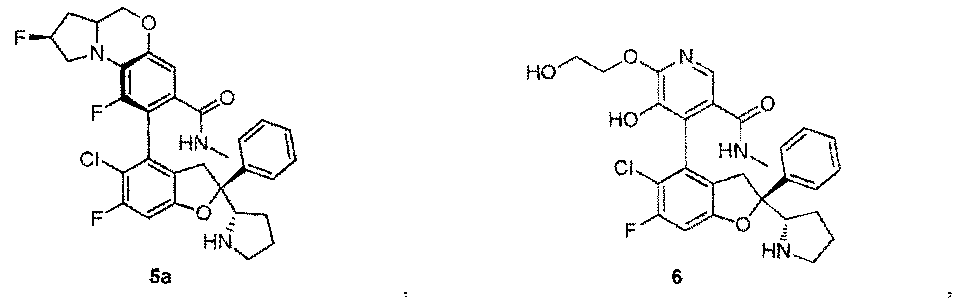

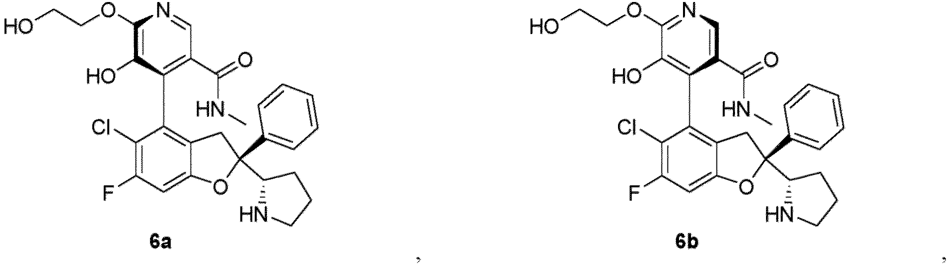

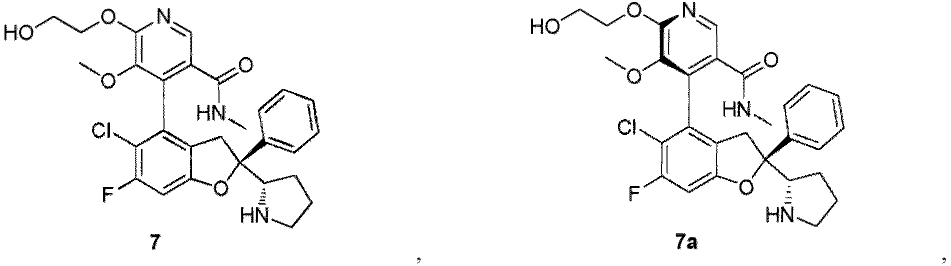

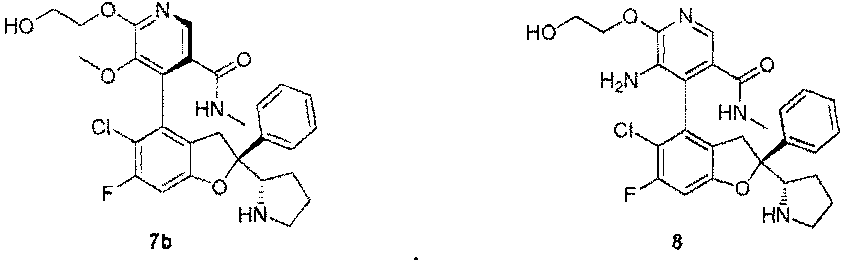

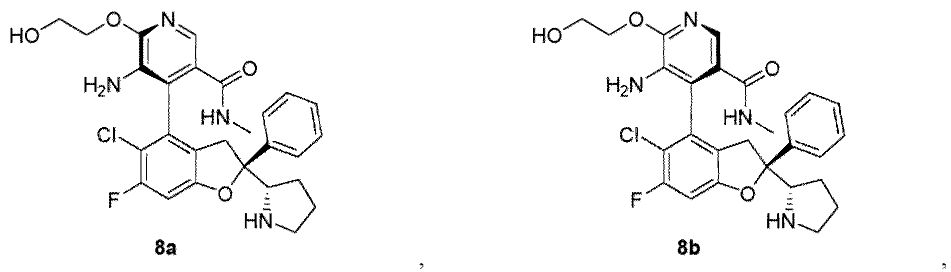

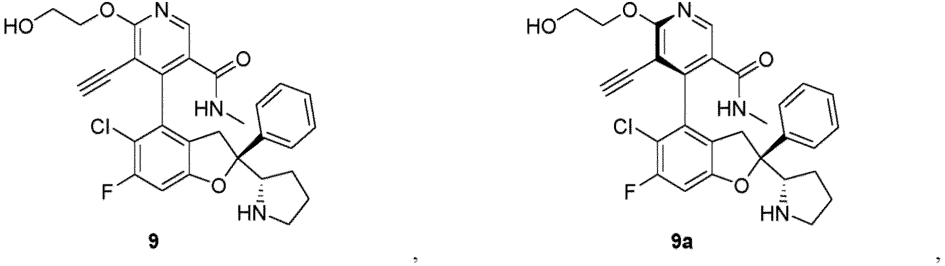

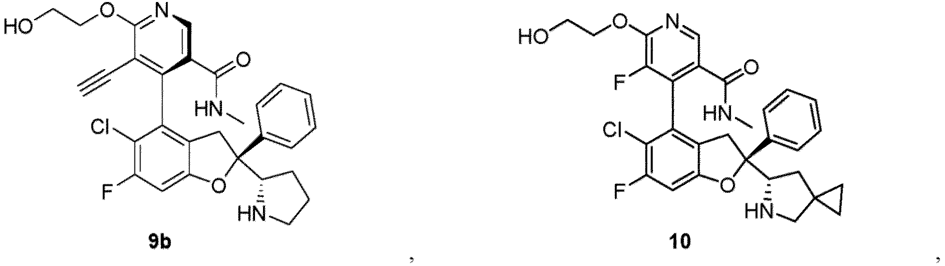

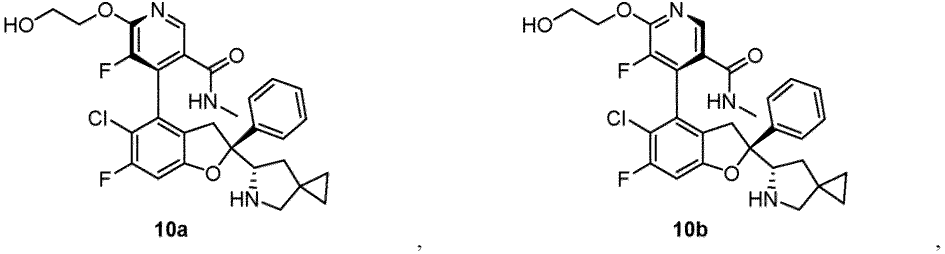

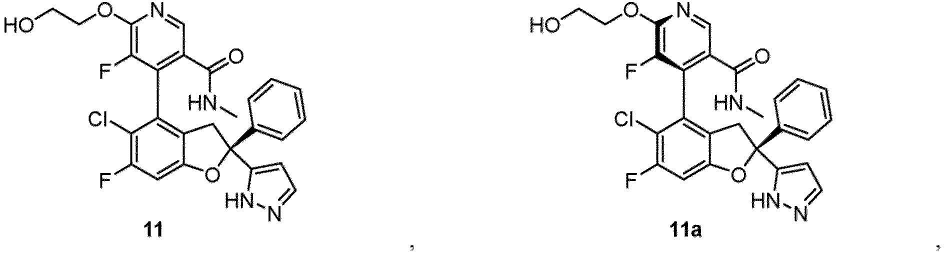

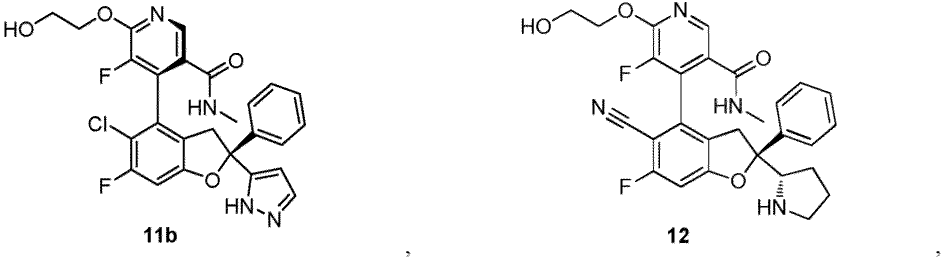

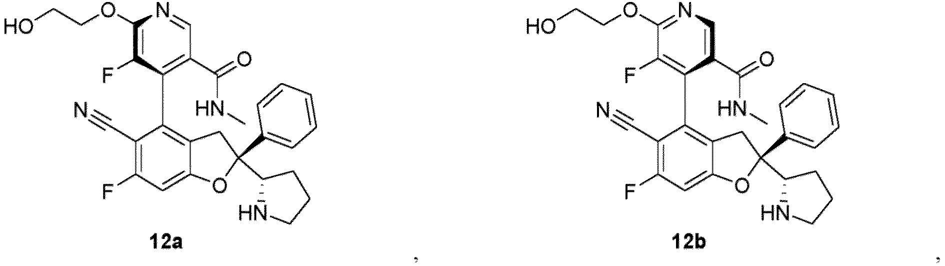

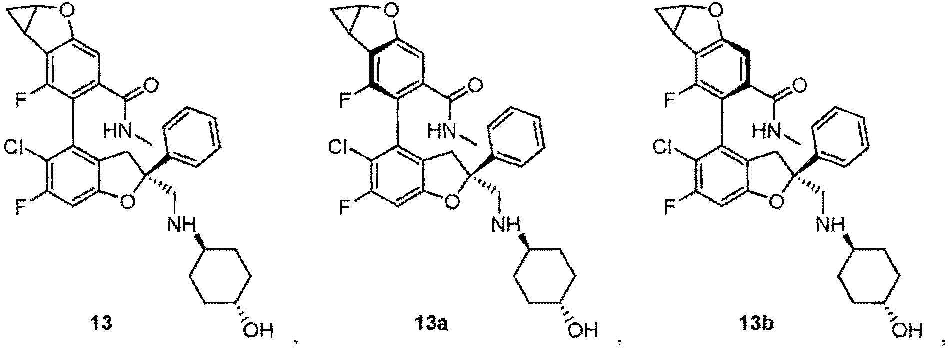

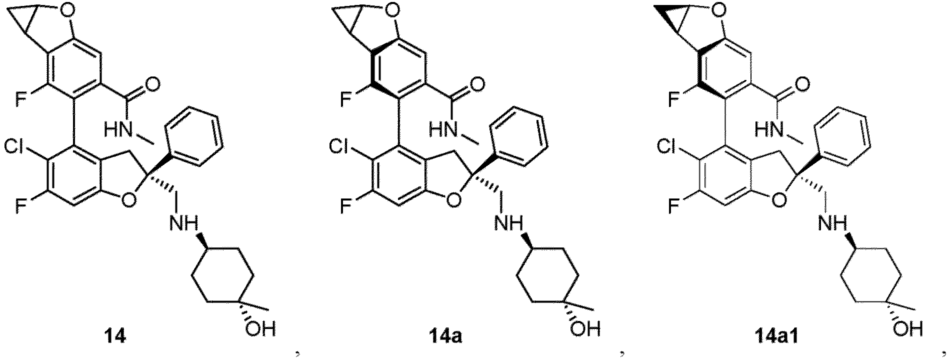

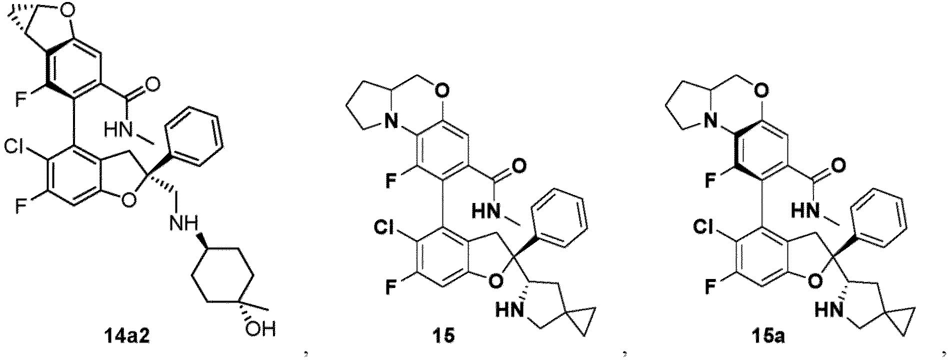

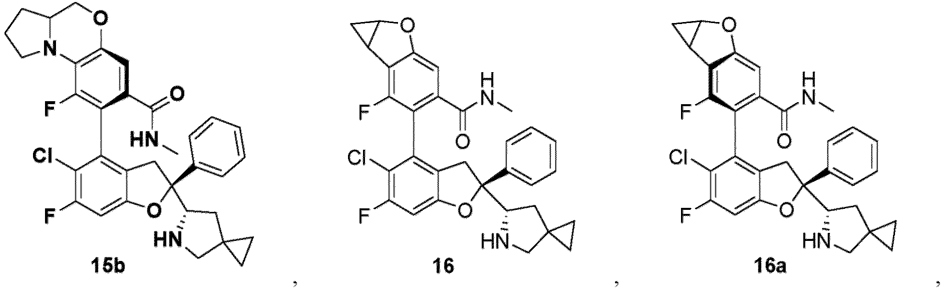

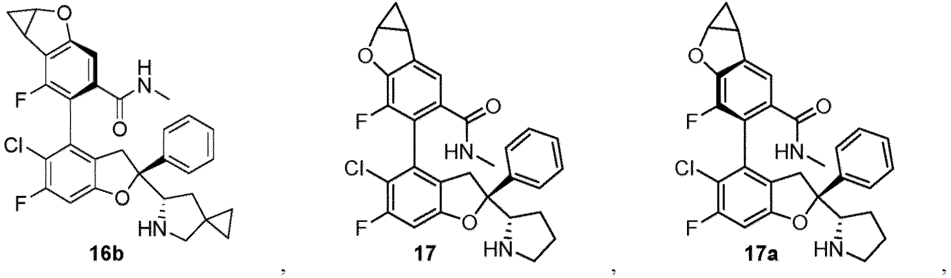

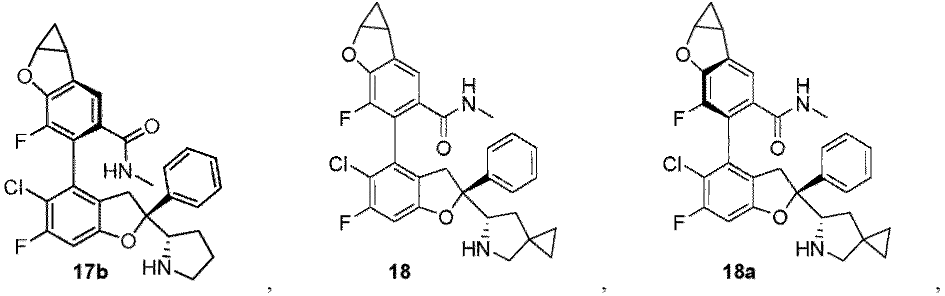

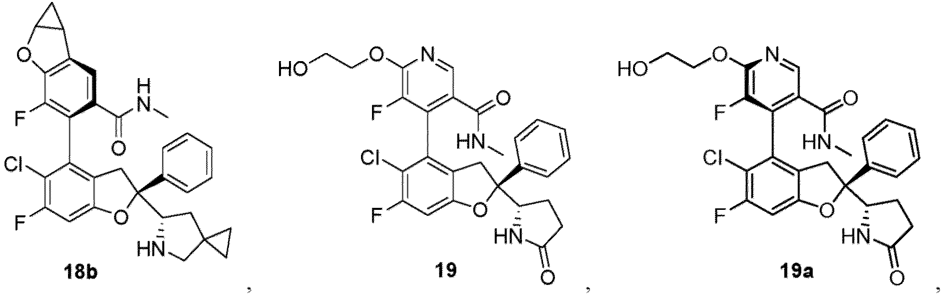

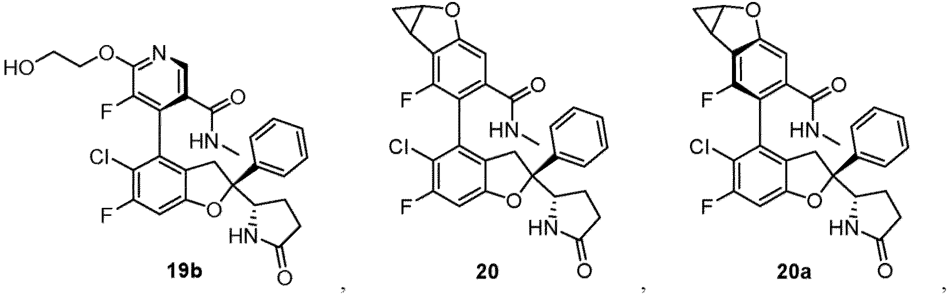

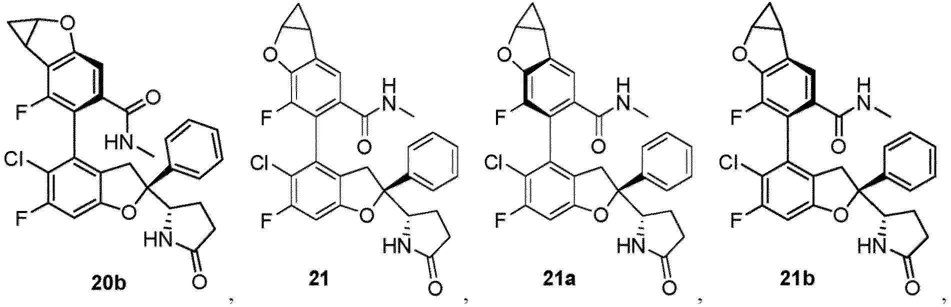

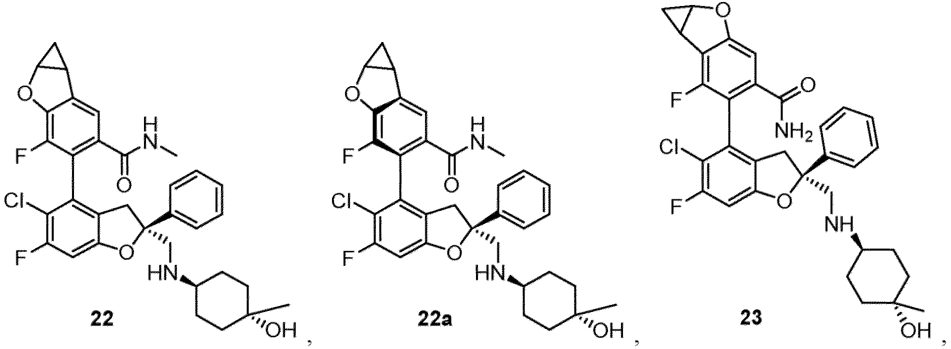

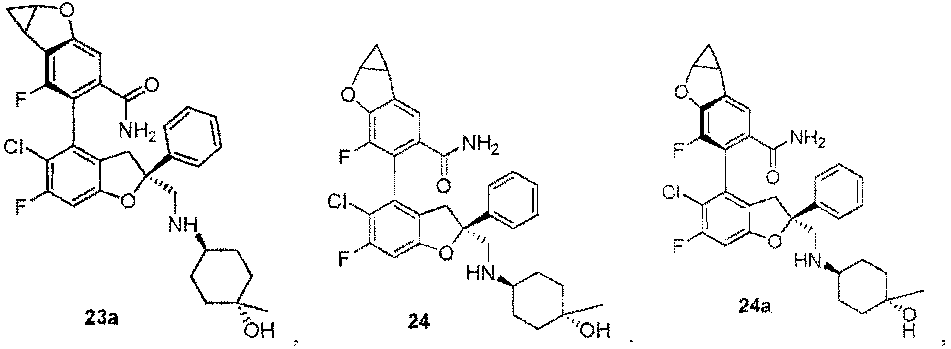

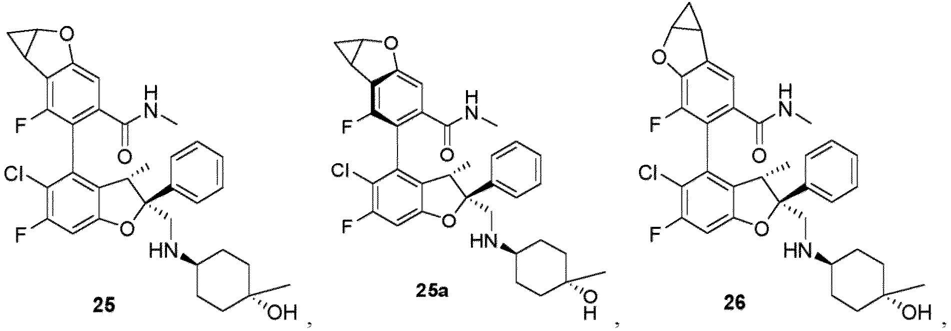

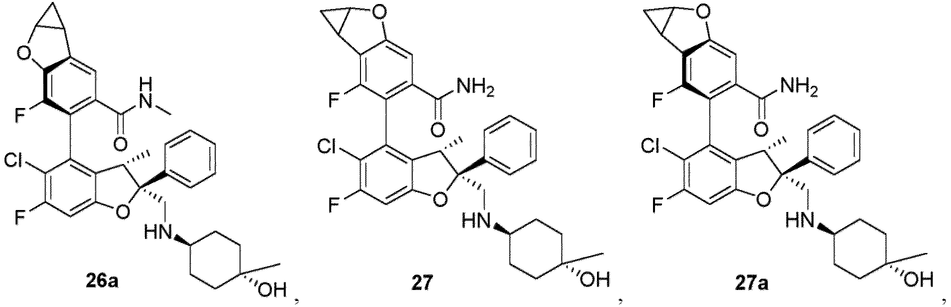

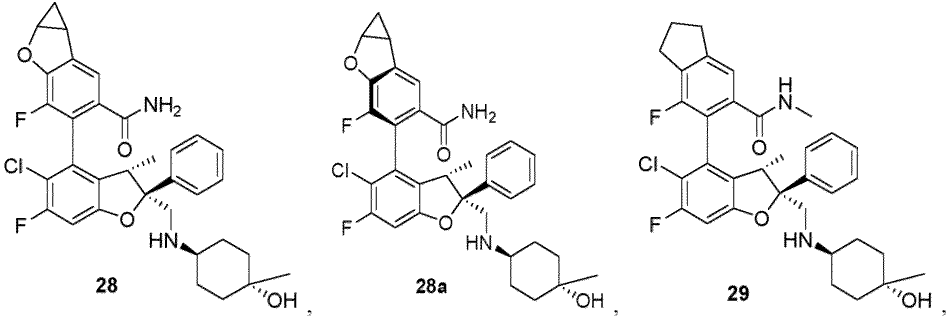

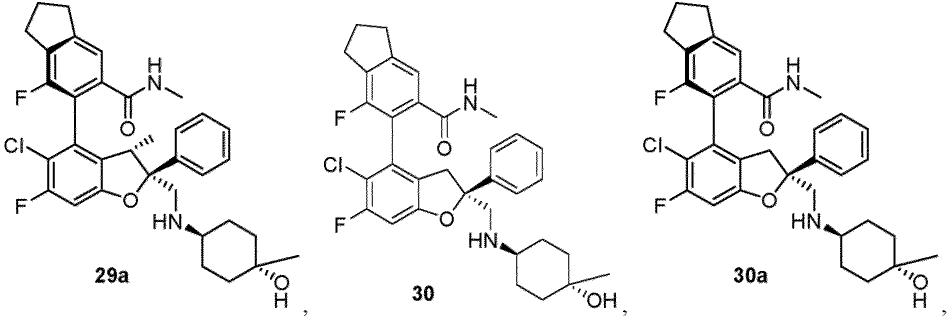

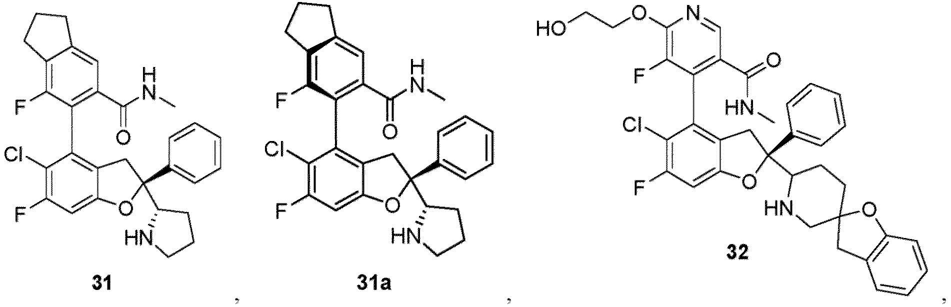

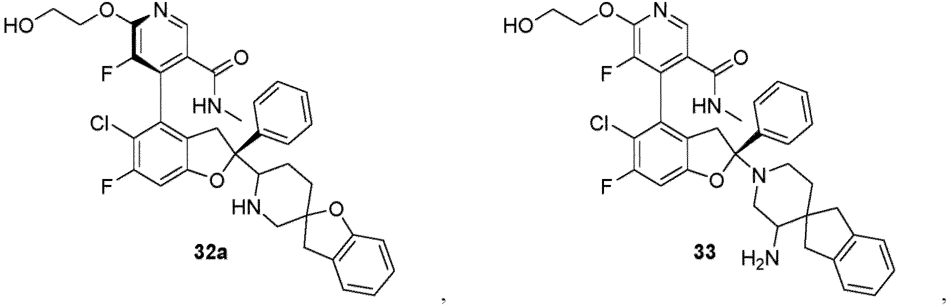

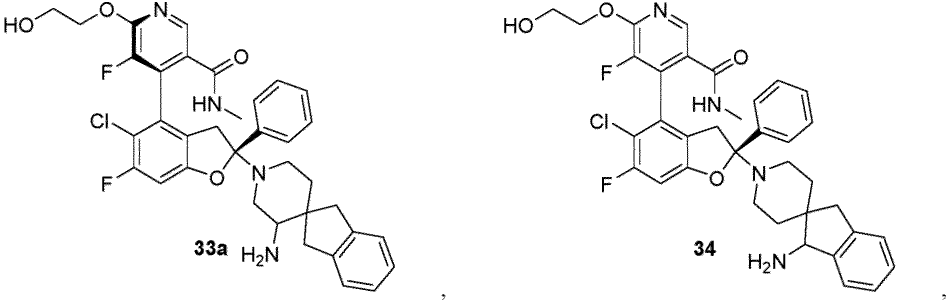

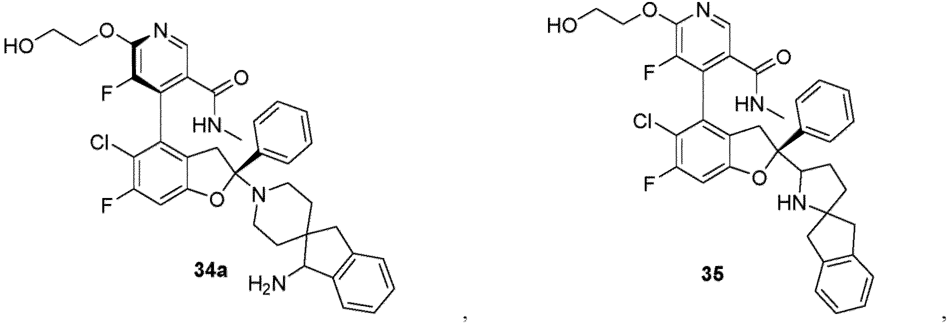

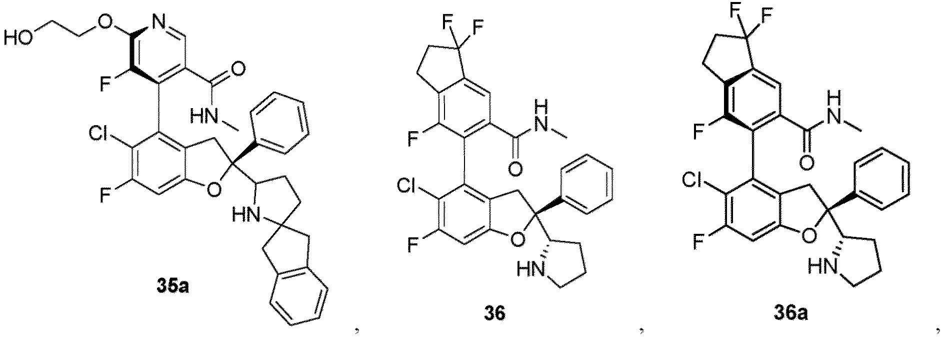

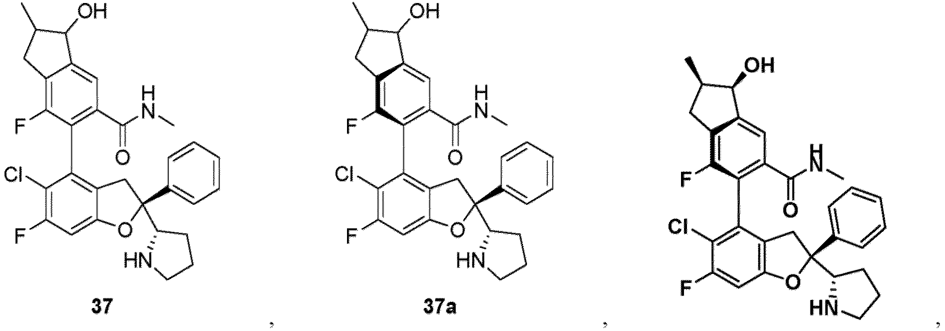

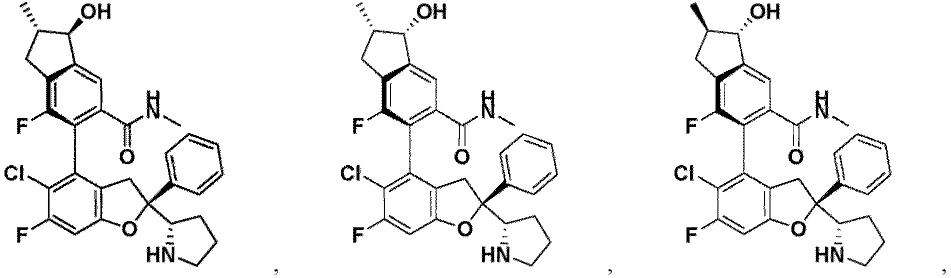

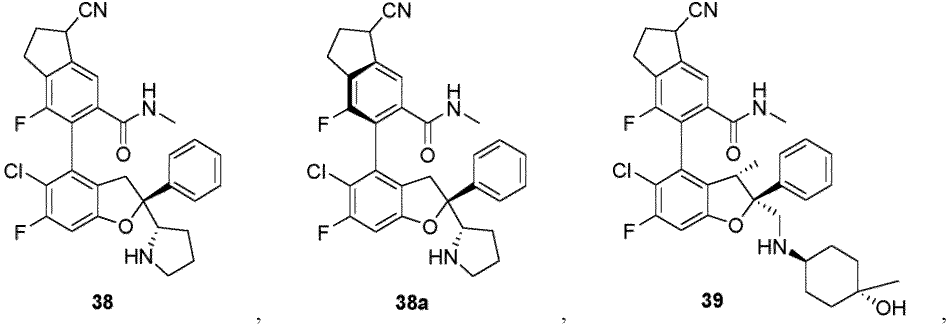

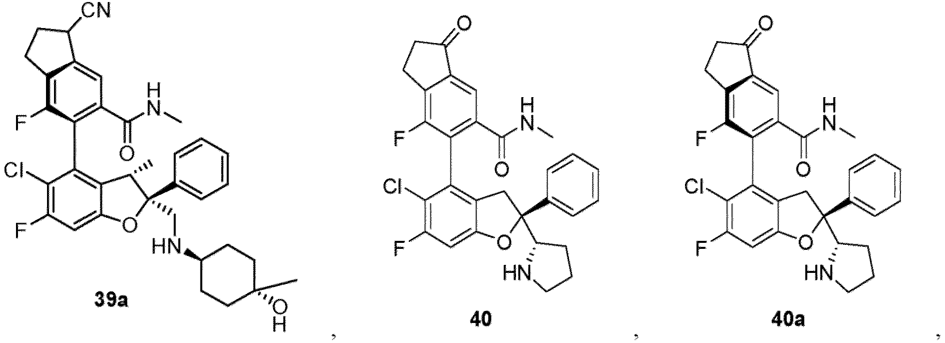

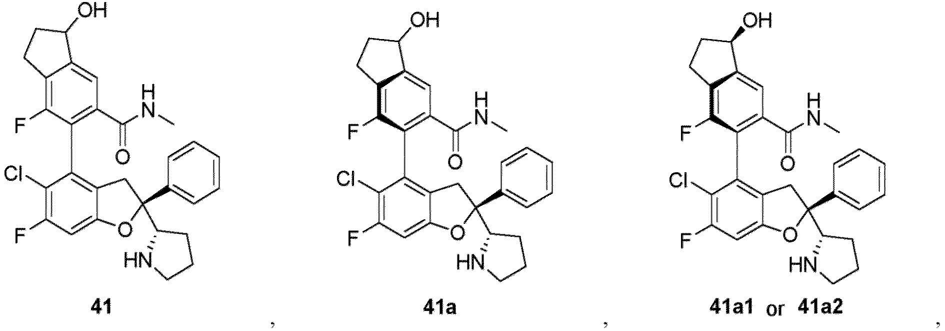

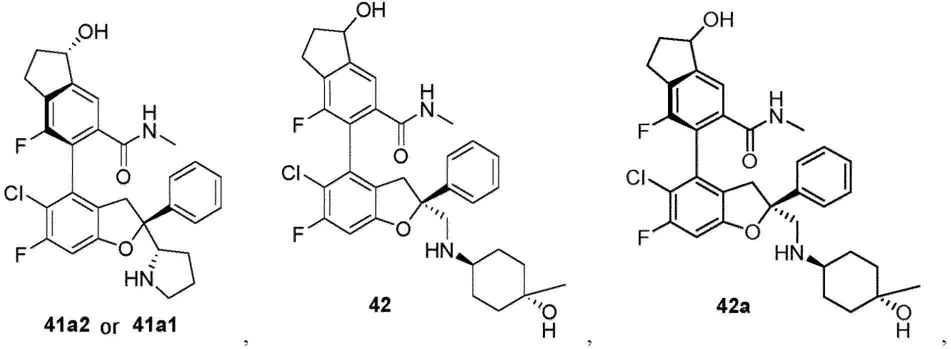

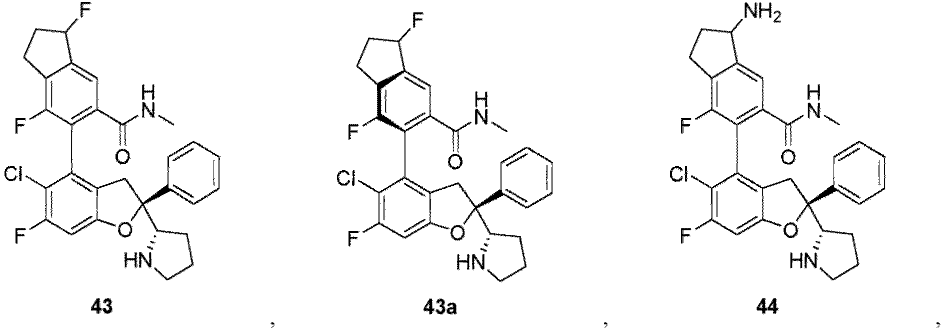

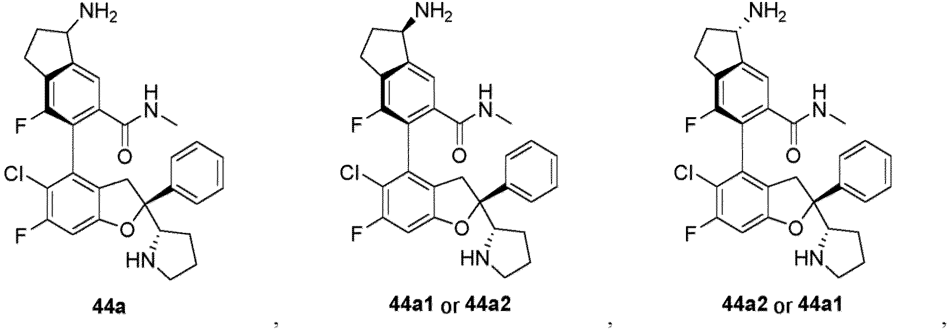

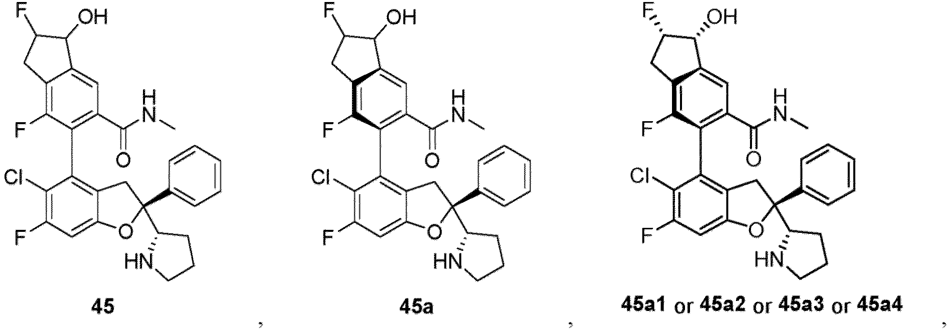

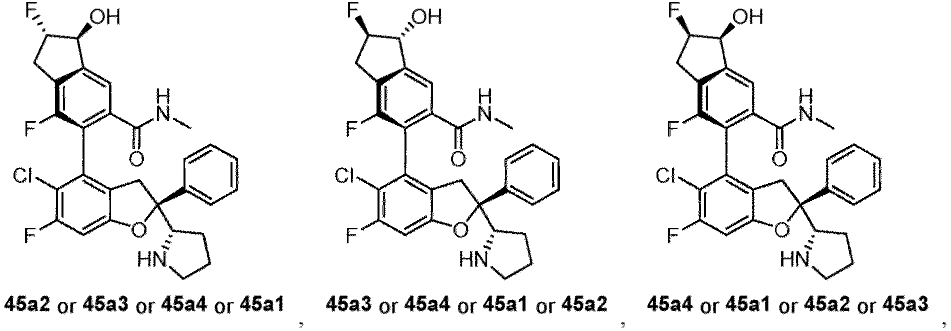

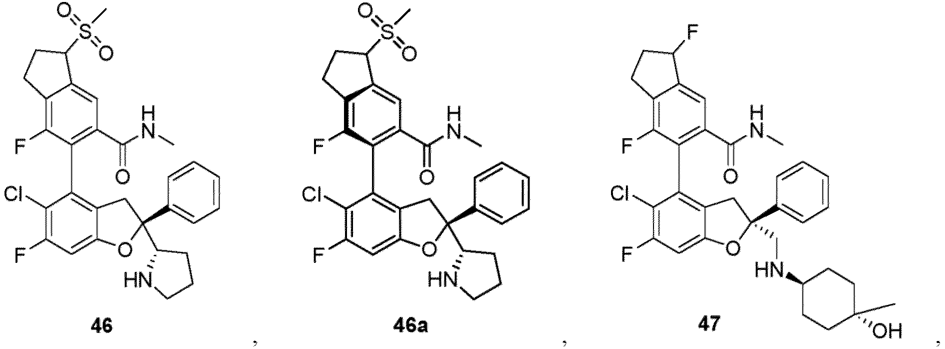

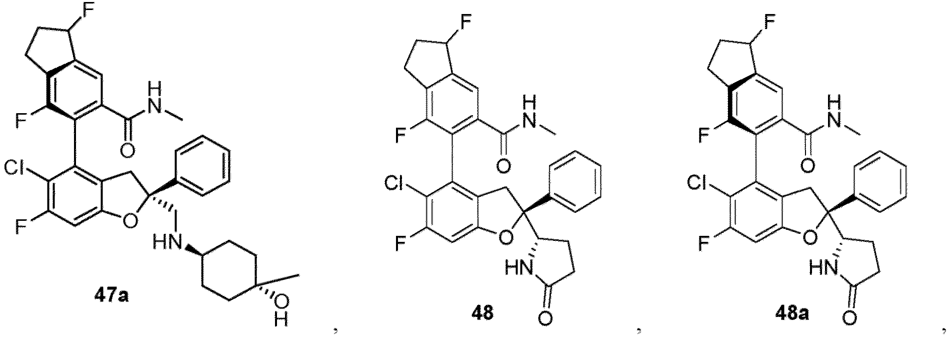

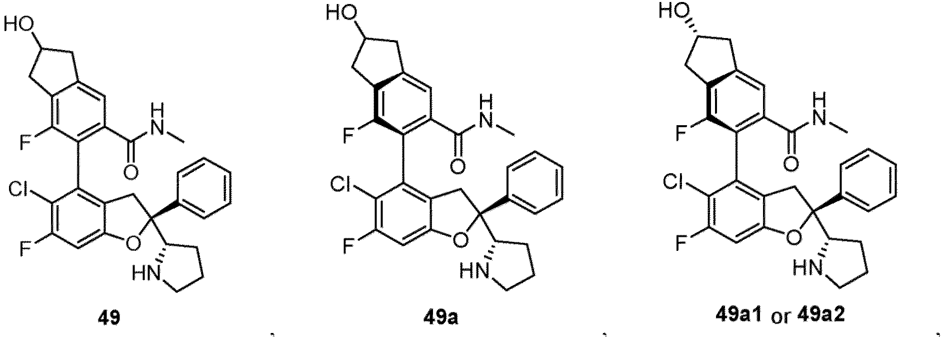

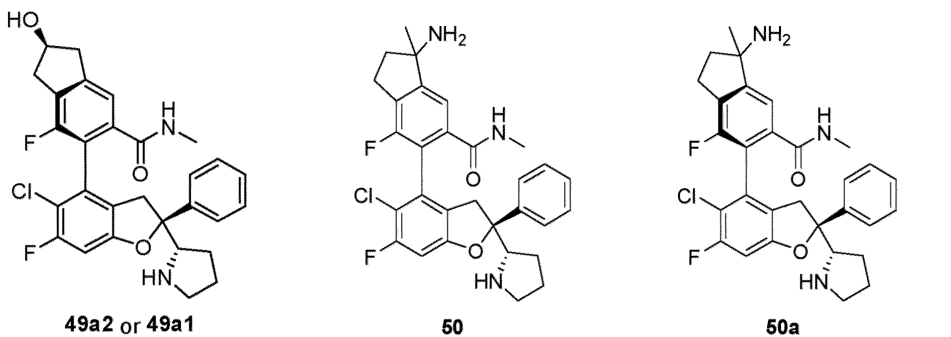

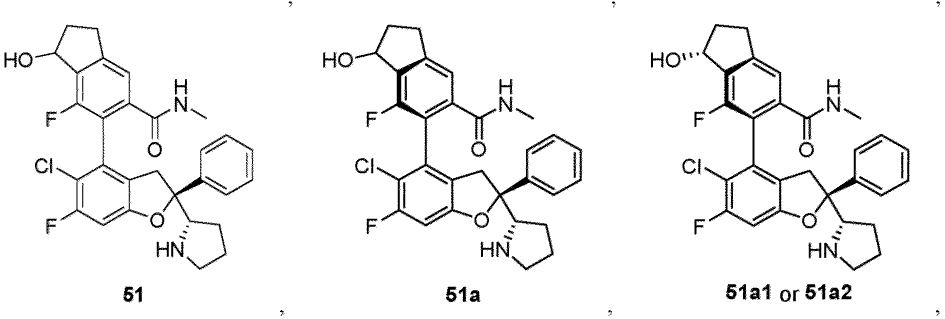

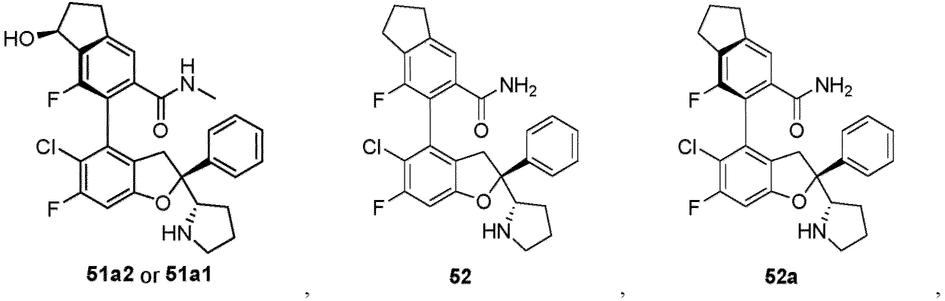

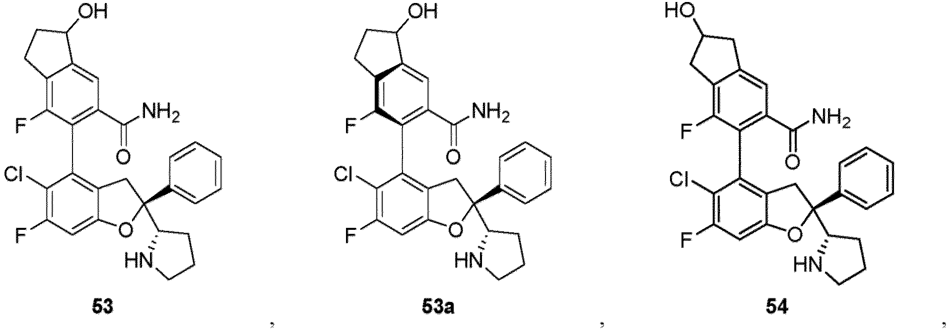

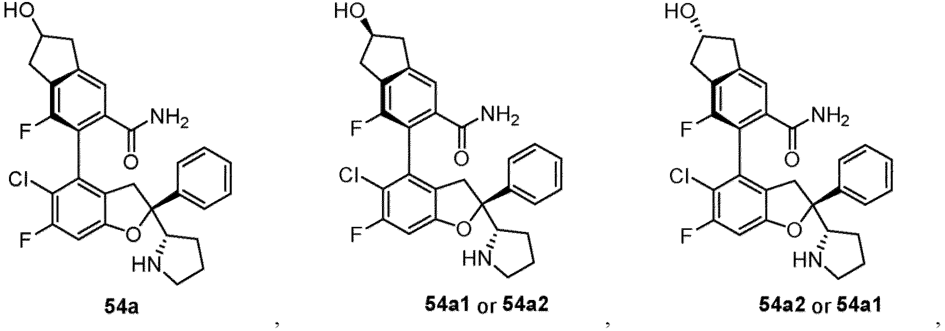

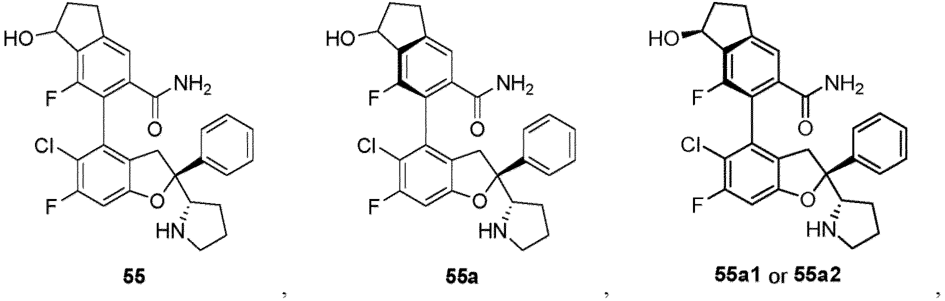

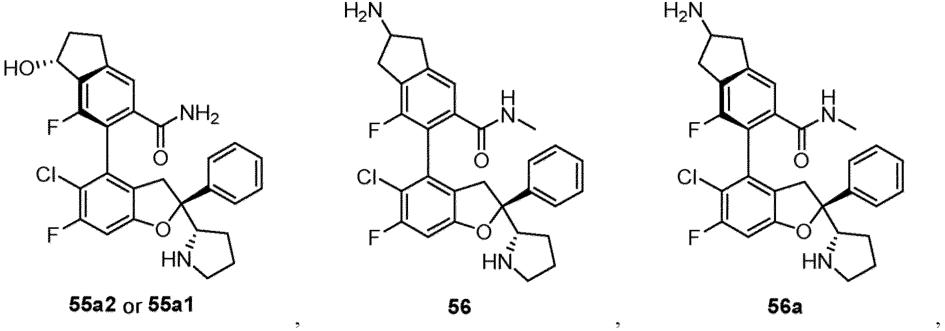

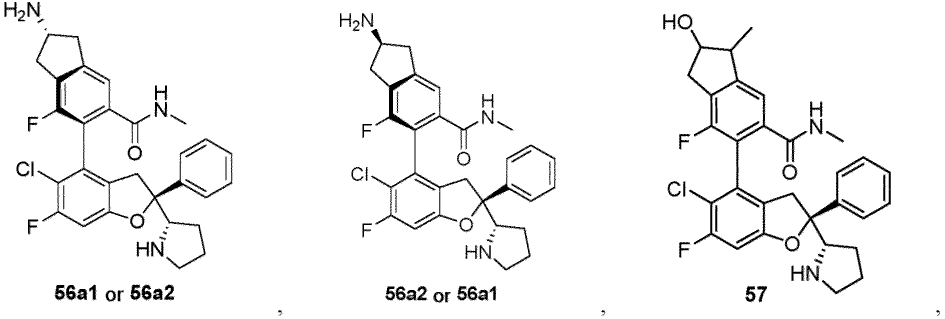

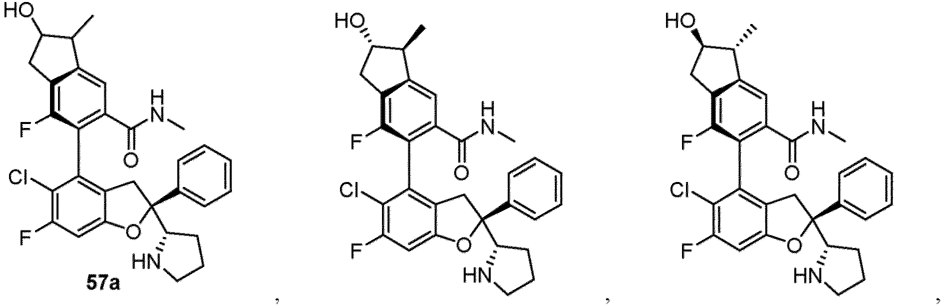

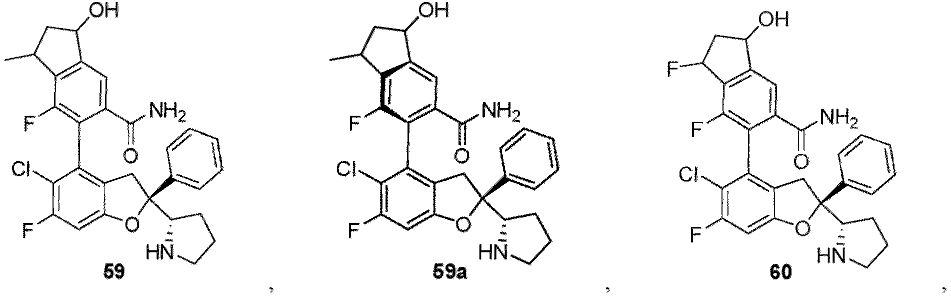

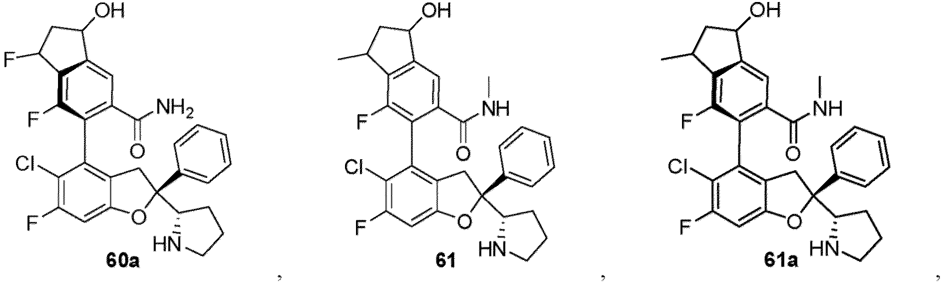

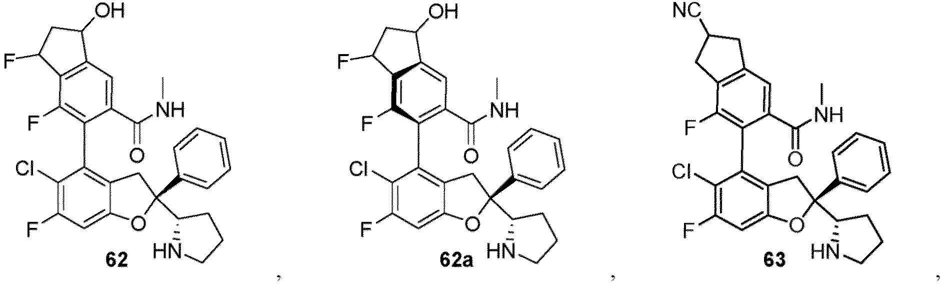

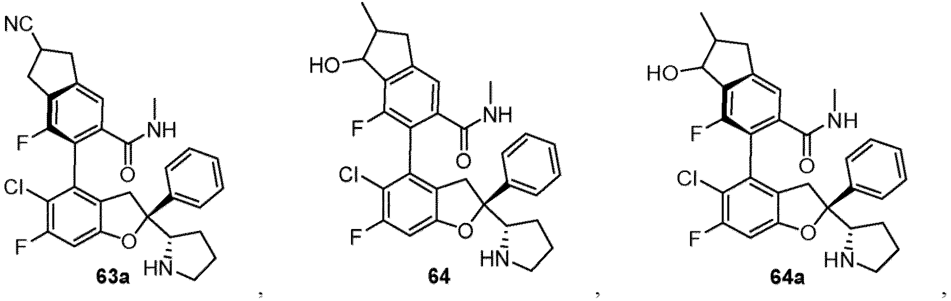

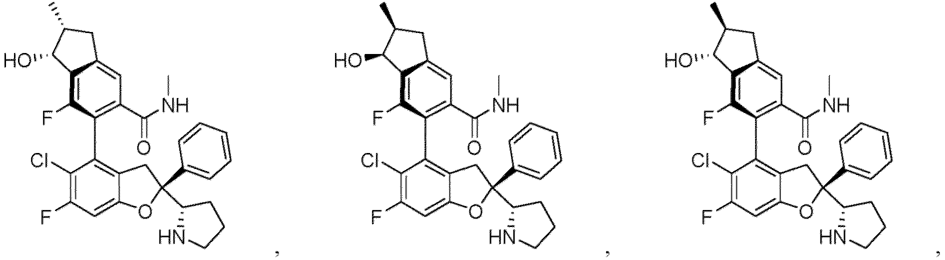

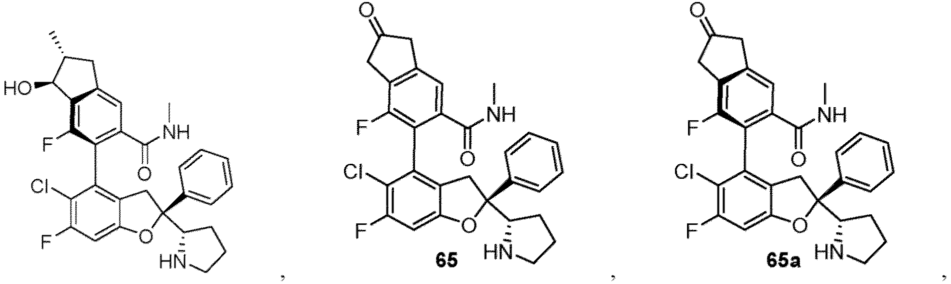

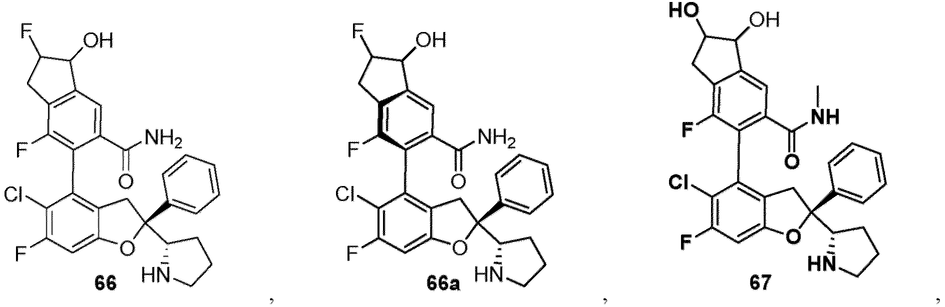

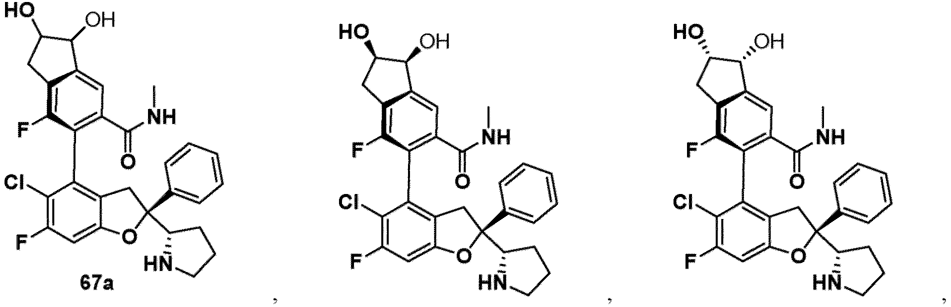

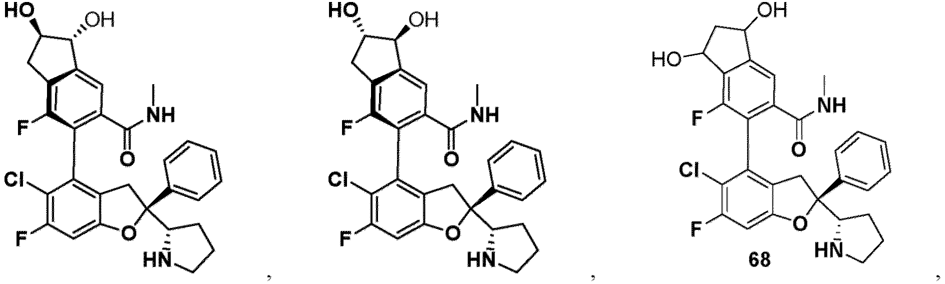

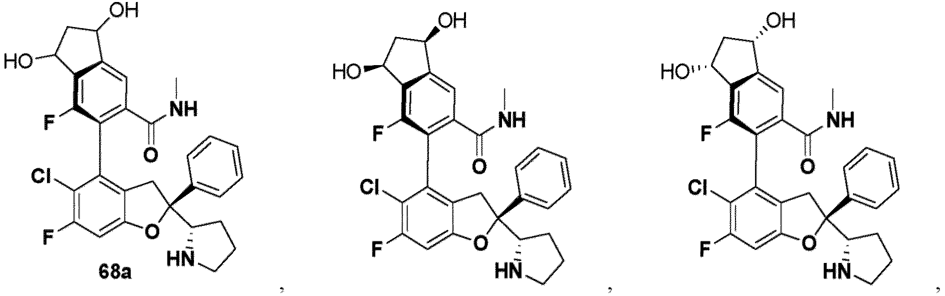

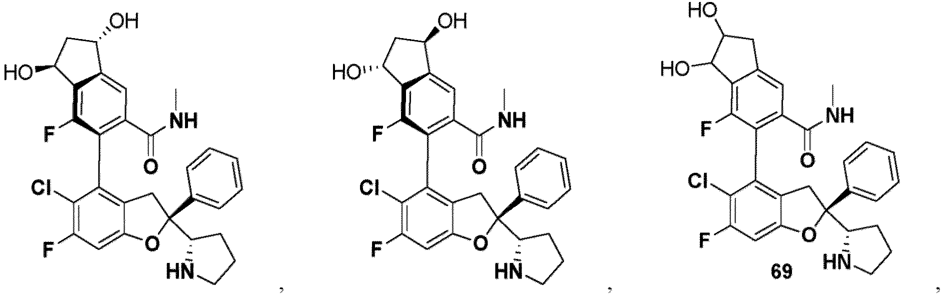

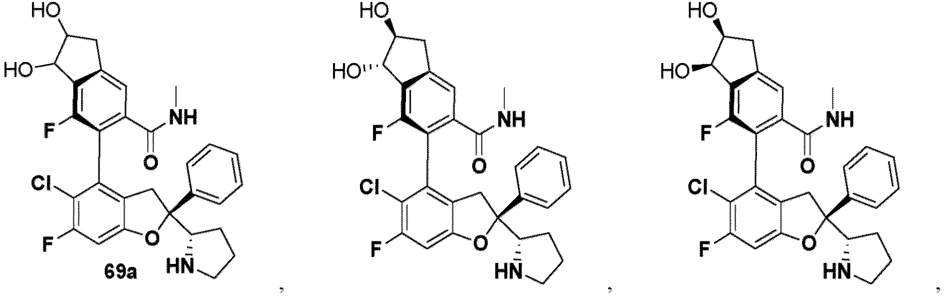

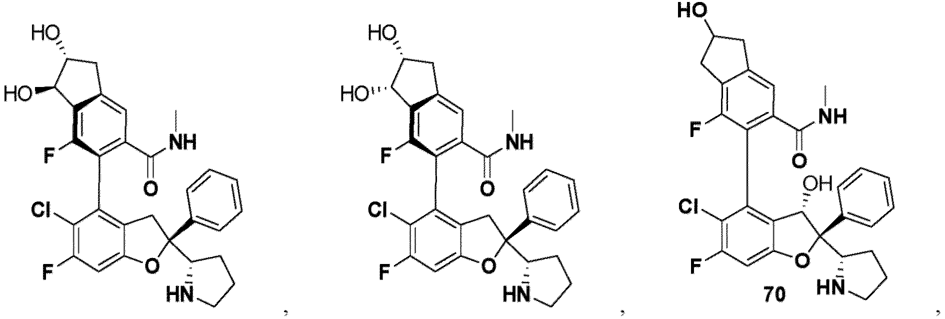

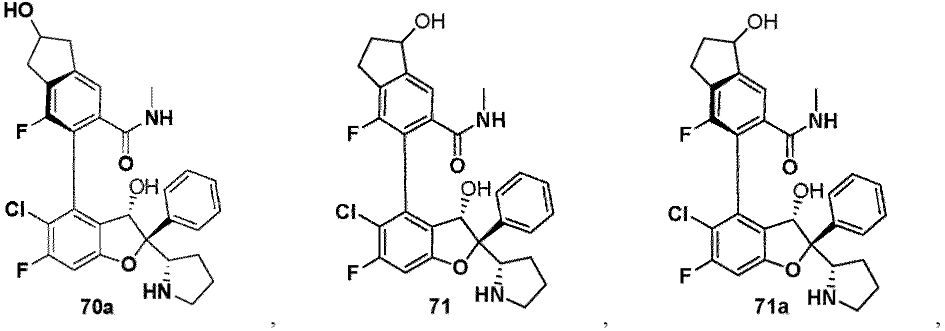

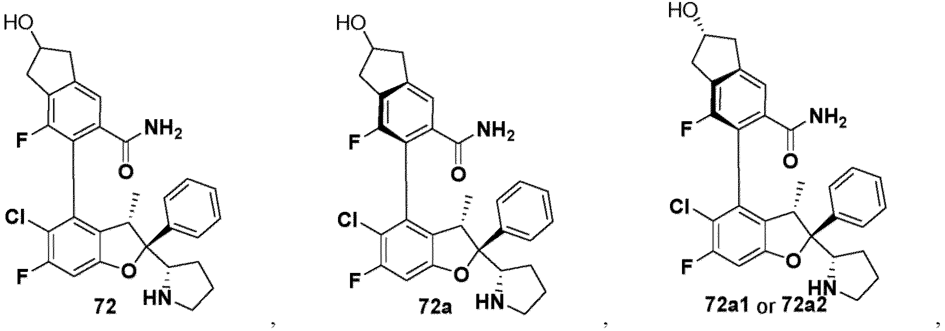

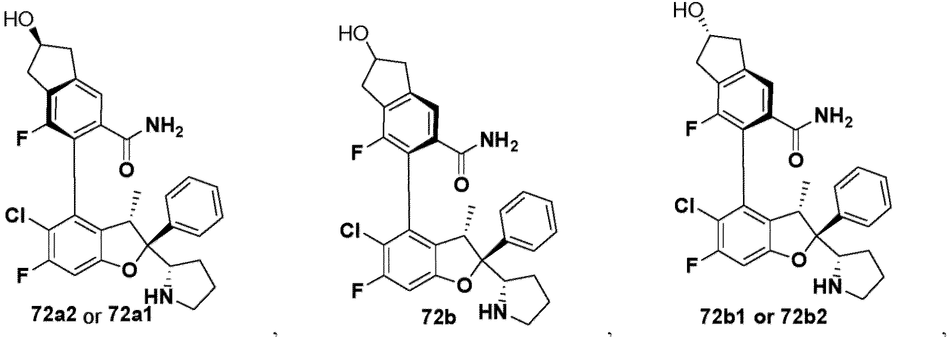

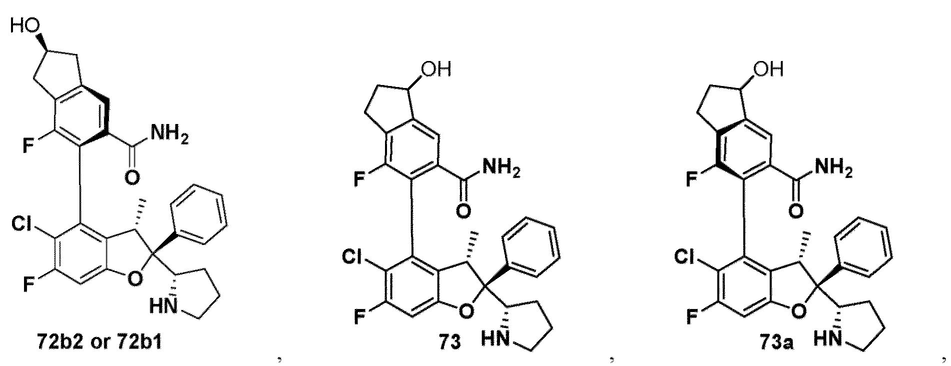

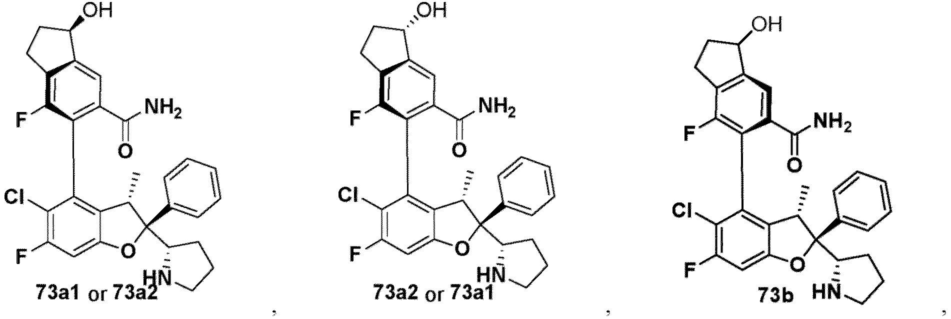

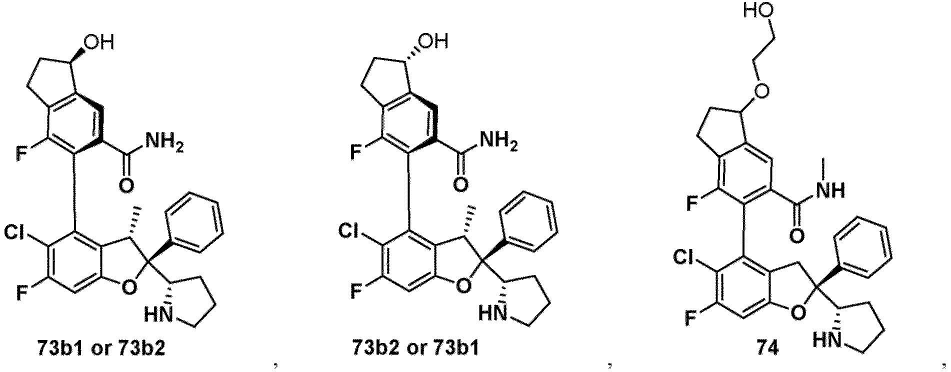

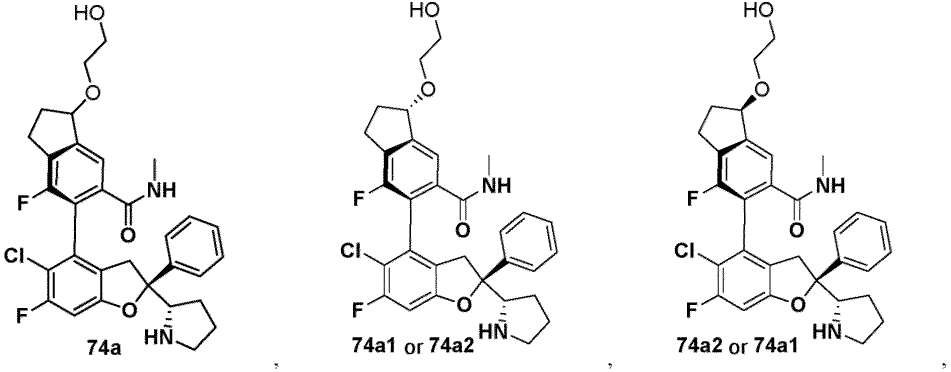

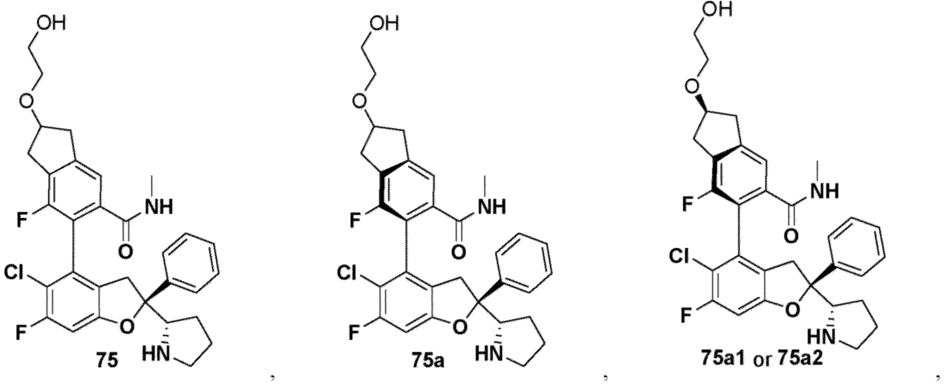

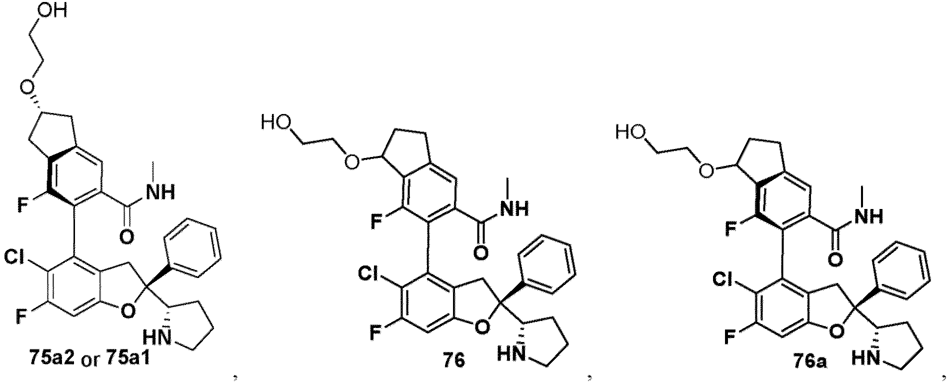

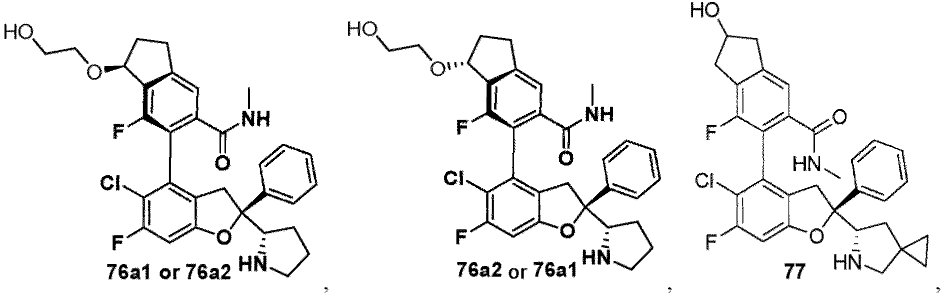

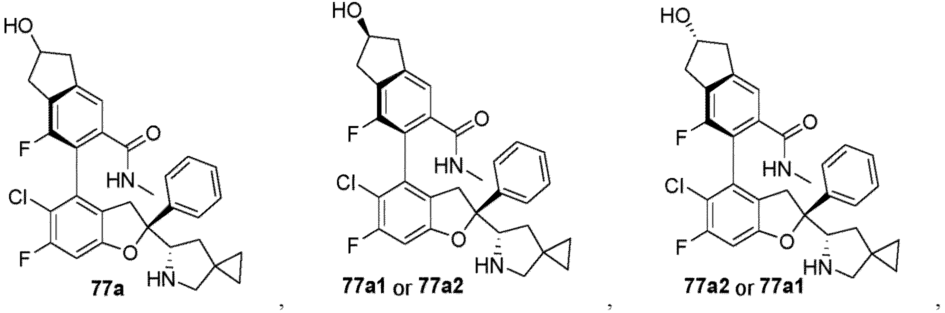

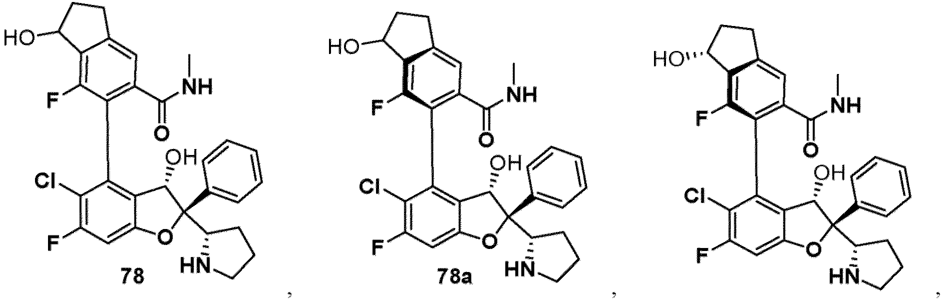

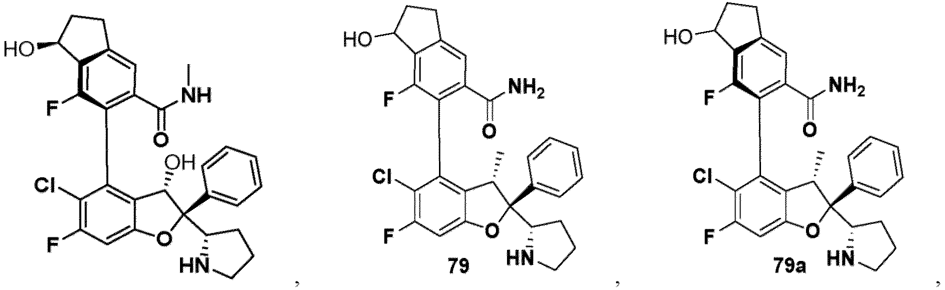

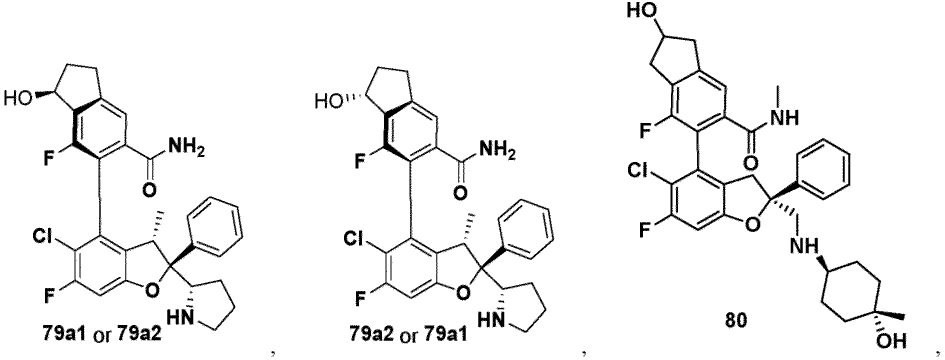

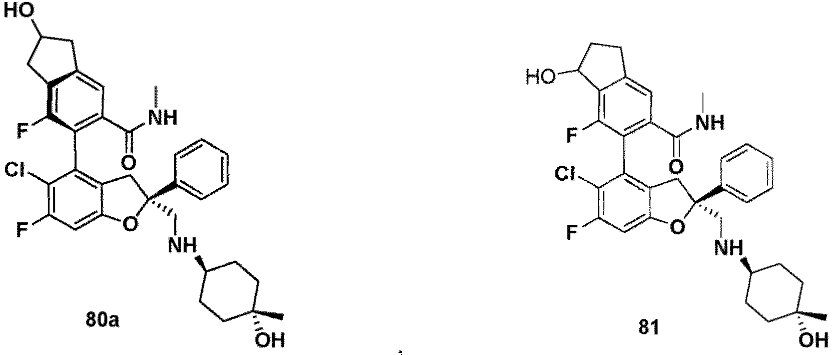

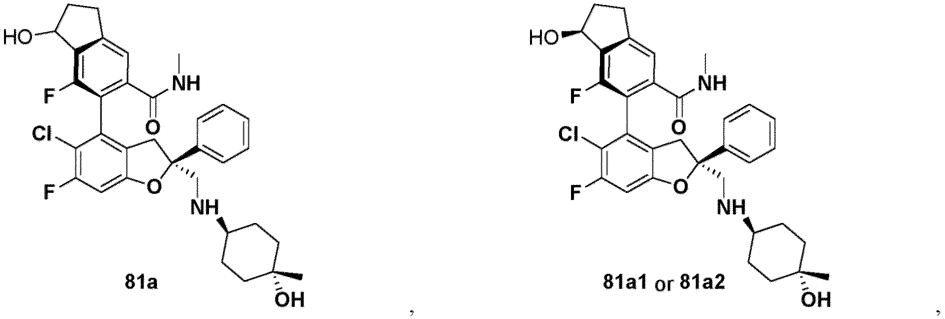

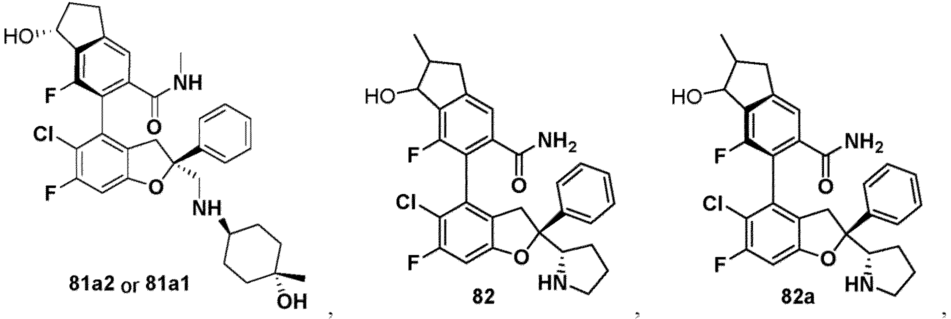

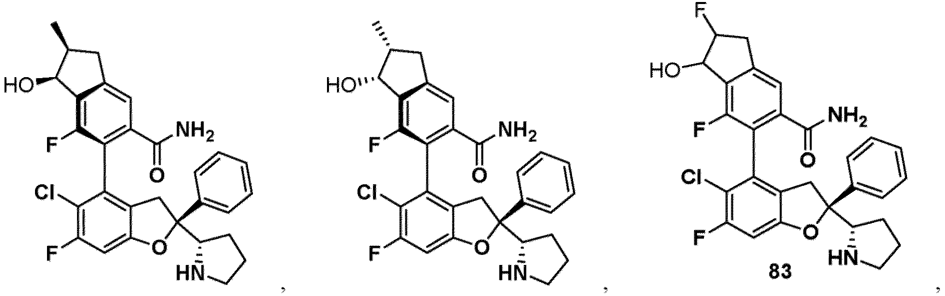

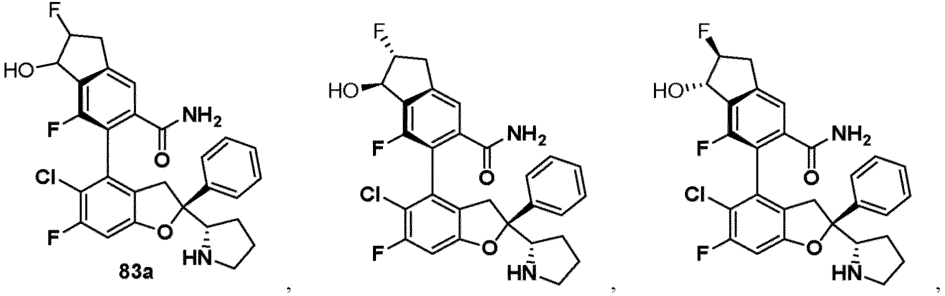

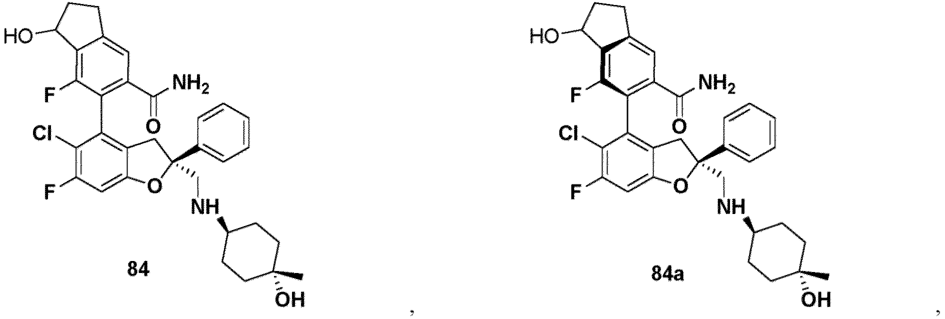

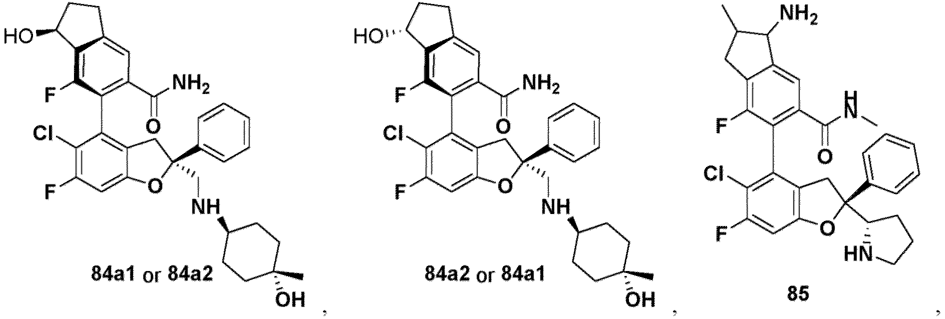

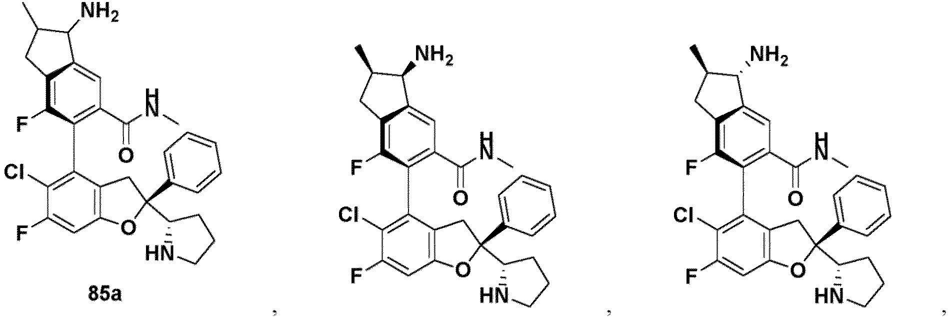

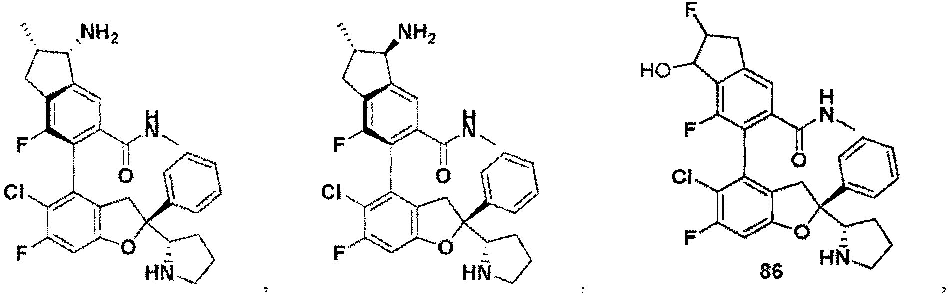

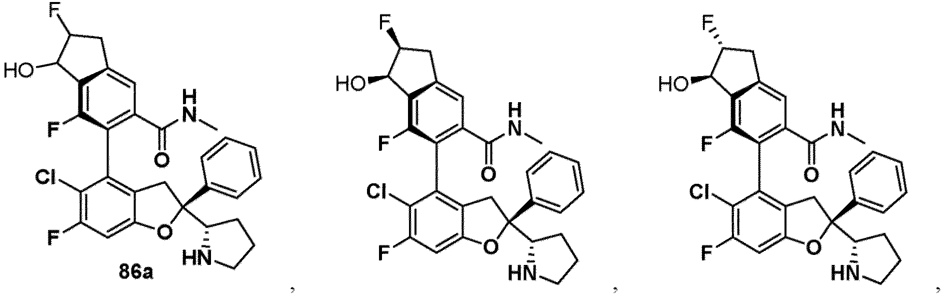

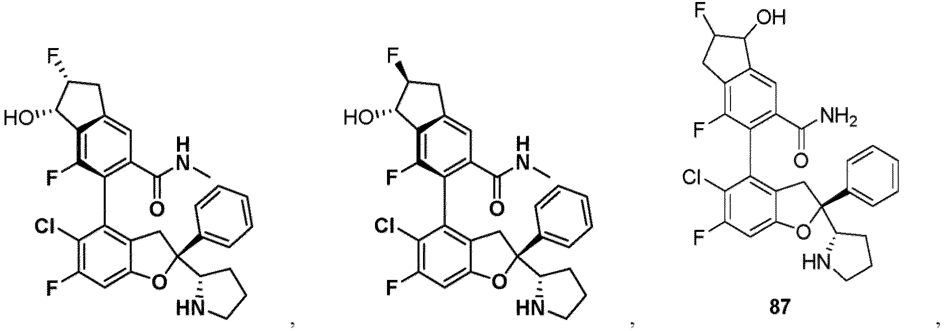

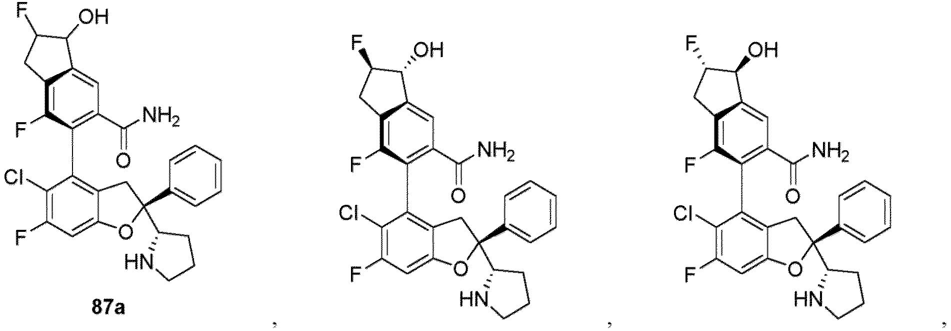

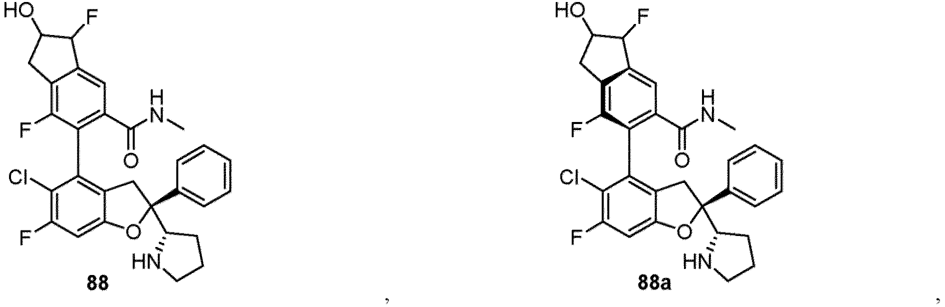

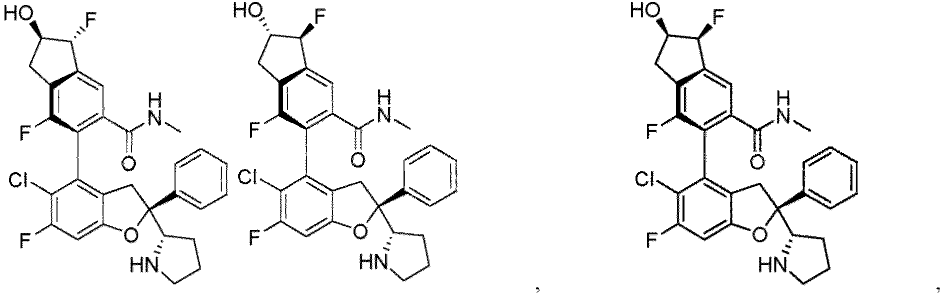

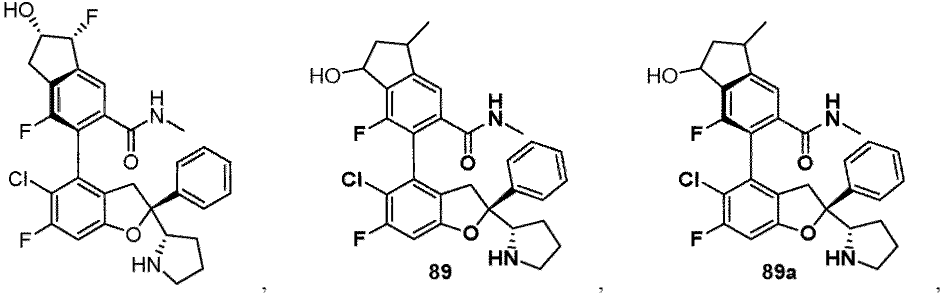

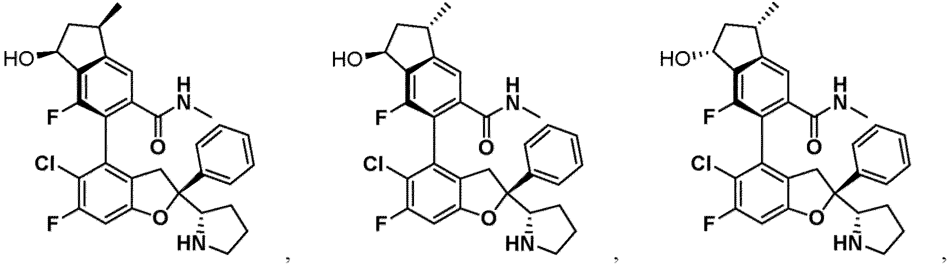

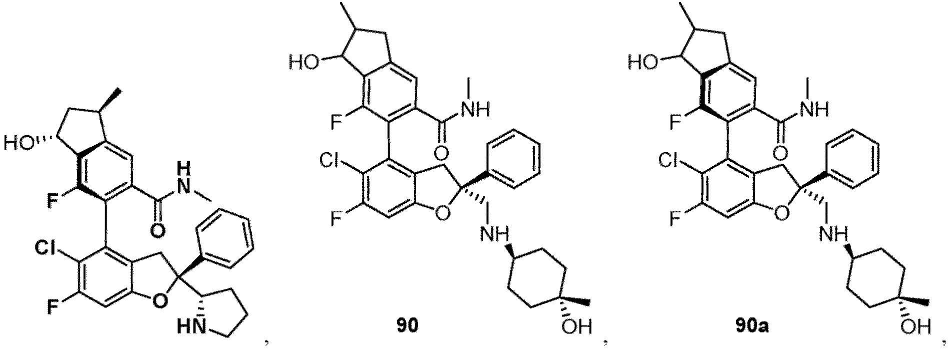

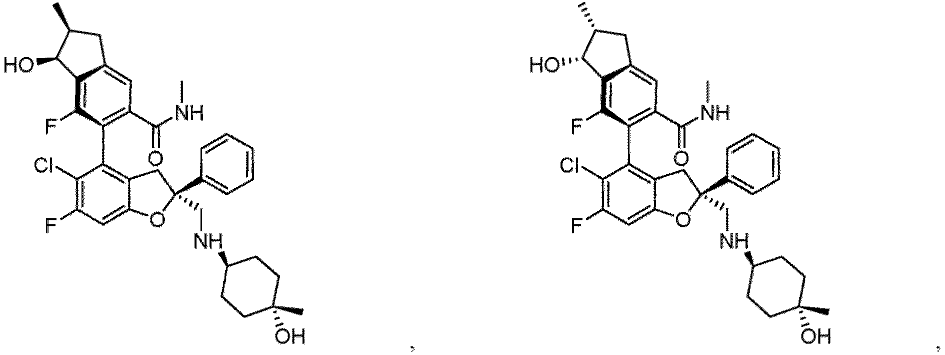

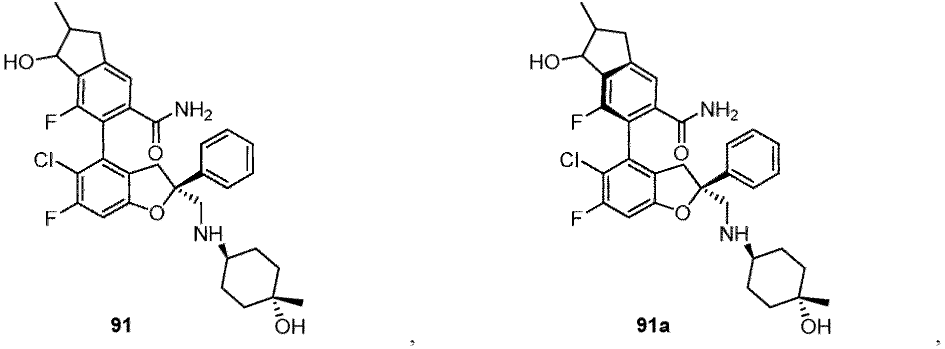

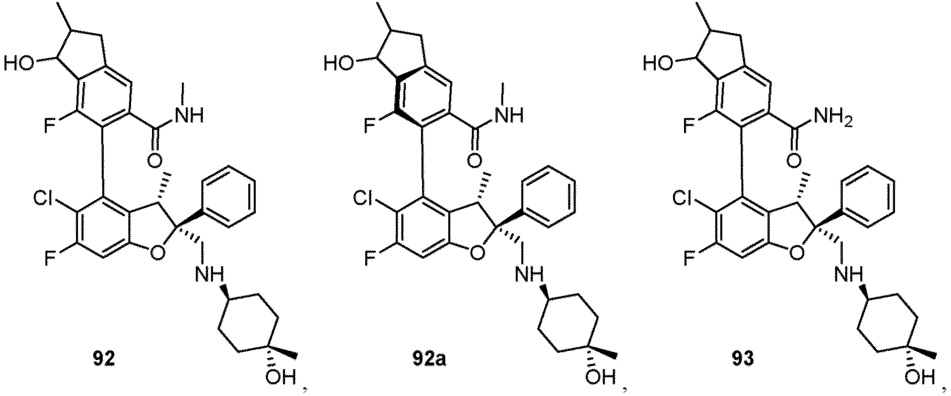

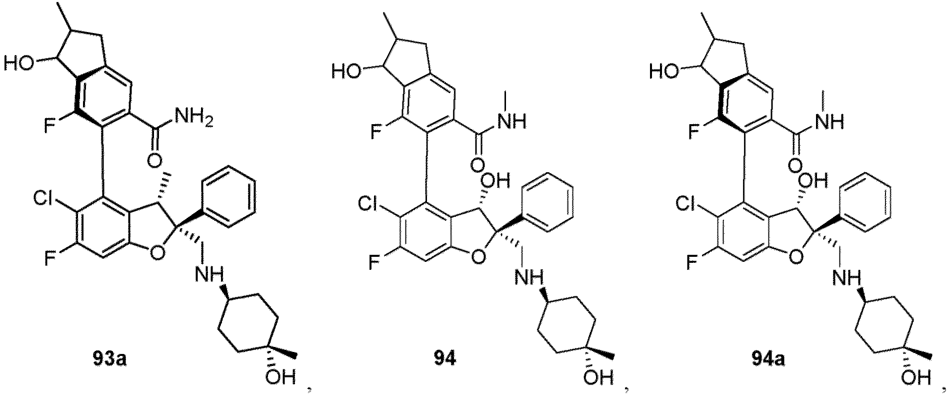

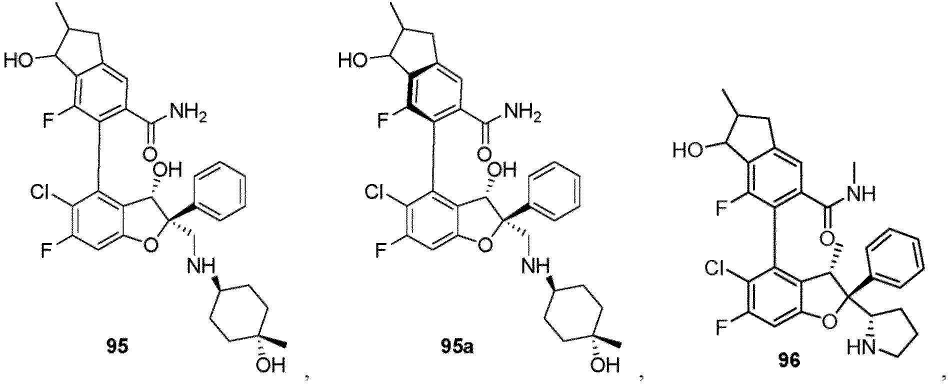

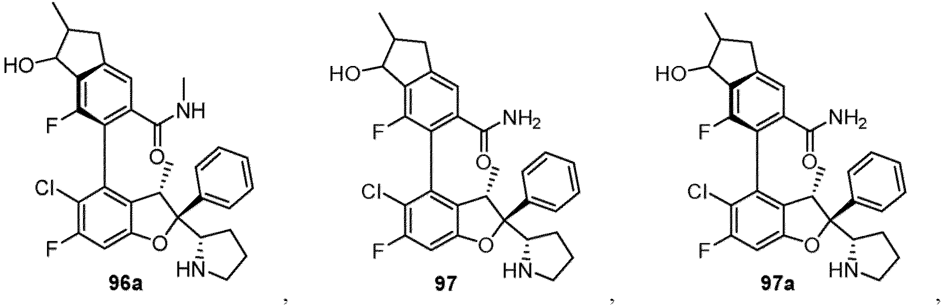

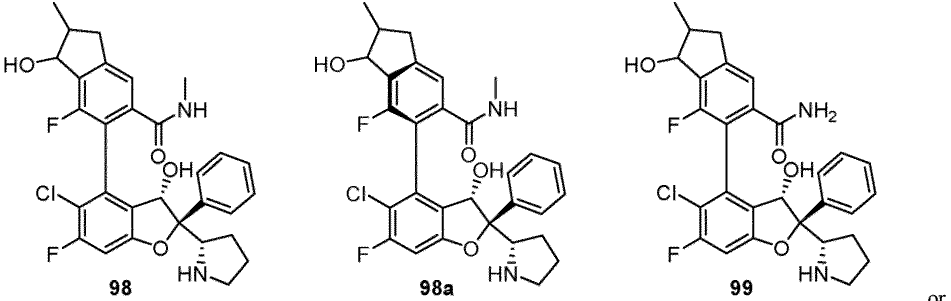

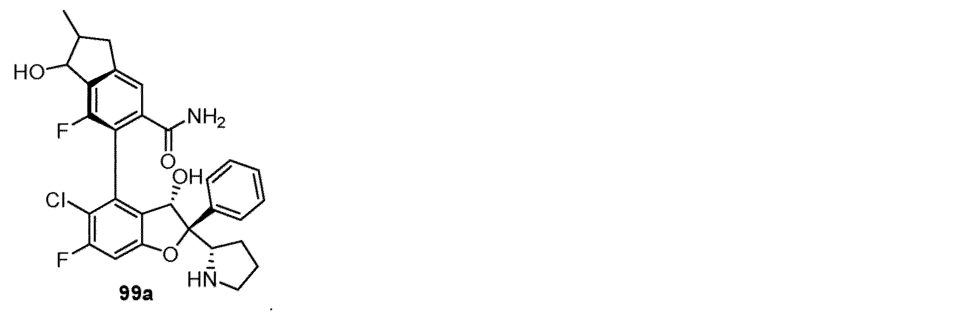

17. The compound of formula (I), the pharmaceutically acceptable salt thereof, or the solvate of the pharmaceutically acceptable salt thereof according to claim 1, wherein the compound of formula (I) is any one of the following compounds:
a compound with a retention time of 1.89 min under the following conditions, which is a stereoisomer in 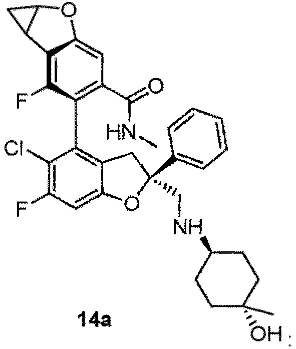 chiral column model: Cellulose-SB; eluent: carbon dioxide/(methanol + 20 mM NH₃); gradient: 10%; flow rate: 3.0 mL/min;
a compound with a retention time of 2.12 min under the following conditions, which is a stereoisomer in 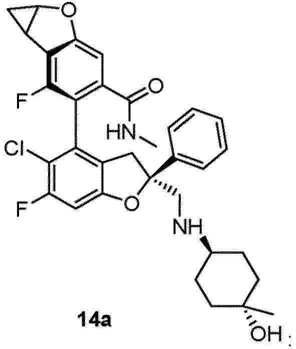 chiral column model: Cellulose-SB; eluent: carbon dioxide/(methanol + 20 mM NH₃); gradient: 10%; flow rate: 3.0 mL/min;
a compound with a retention time of 6.50 min under the following conditions, which is a stereoisomer in 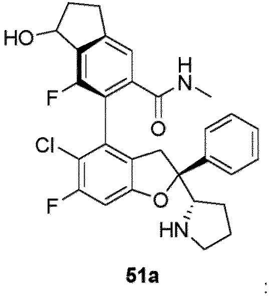 column model: Celluiose-SB, 100 * 4.6 mm, 5 µm; eluent: hexane (0.1% DEA)/IPA; gradient: isocratic 9/1, flow rate: 1 mL/min;
a compound with a retention time of 7.62 min under the following conditions, which is a stereoisomer in 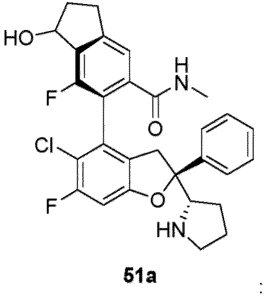 column model: Celluiose-SB, 100 * 4.6 mm, 5 µm; eluent: hexane (0.1% DEA)/IPA; gradient: isocratic 9/1, flow rate: 1 mL/min;
a compound with a retention time of 4.68 min under the following conditions, which is a stereoisomer in 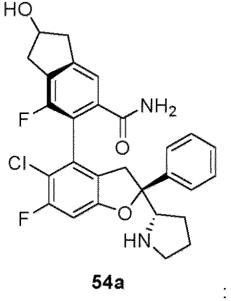 column model: Celluiose-SB, 100 * 4.6 mm, 5 µm; eluent: hexane (0.1% DEA)/IPA; gradient: isocratic 4/1, flow rate: 1 mL/min;
a compound with a retention time of 4.20 min under the following conditions, which is a stereoisomer in 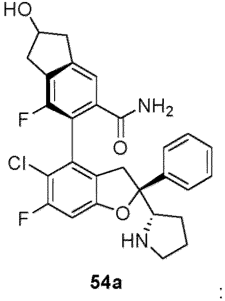 column model: Celluiose-SB, 100 * 4.6 mm, 5 µm; eluent: hexane (0.1% DEA)/IPA; gradient: isocratic 4/1, flow rate: 1 mL/min;
a mixture with retention times of 4.21 min and 4.71 min under the following conditions, which is a pair of diastereomers in 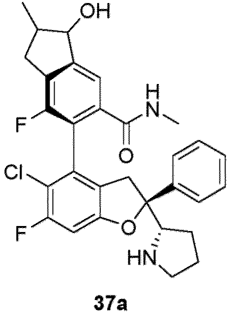 : column model: Amylose-SA, 100 * 4.6 mm, 5 µm; eluent: hexane (0.1% DEA)-IPA; gradient: isocratic 9/1, flow rate: 1 mL/min, dr = 54 (4.21 min):46 (4.71 min);
a mixture with retention times of 4.60 min and 6.10 min under the following conditions, which is a pair of diastereomers in 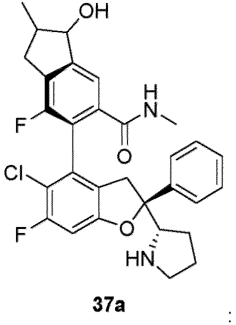 column model: Amylose-SA, 100 * 4.6 mm, 5 µm; eluent: hexane (0.1% DEA)-IPA; gradient: isocratic 9/1, flow rate: 1 mL/min, dr = 48 (4.60 min):52 (6.10 min);
a mixture with retention times of 620 min and 6.81 min under the following conditions, which is a pair of diastereomers in 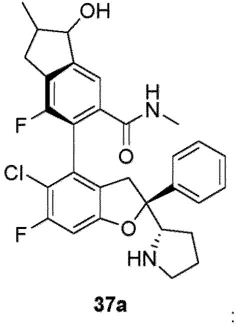 column model: Amylose-SA, 100 * 4.6 mm, 5 µm; eluent: hexane (0.1% DEA)-IPA; gradient: isocratic 9/1, flow rate: 1 mL/min, dr = 8 (6.20 min):92 (6.81 min);
a compound with a retention time of 2.43 min under the following conditions, which is a stereoisomer in 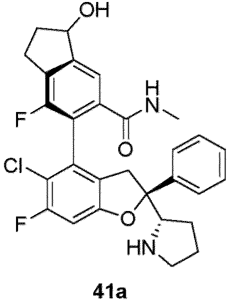 : column model: Celluiose-SB, 100 * 4.6 mm, 5 µm; eluent: hexane/IPA; gradient: isocratic 7/3, flow rate: 1 mL/min;
a compound with a retention time of 3.37 min under the following conditions, which is a stereoisomer in 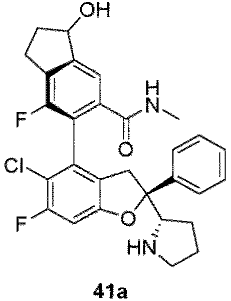 : column model: Celluiose-SB, 100 * 4.6 mm, 5 µm; eluent: hexane/IPA; gradient: isocratic 7/3, flow rate: 1 mL/min;
a mixture with retention times of 2.86 min and 3.75 min under the following conditions, which is a pair of diastereomers in 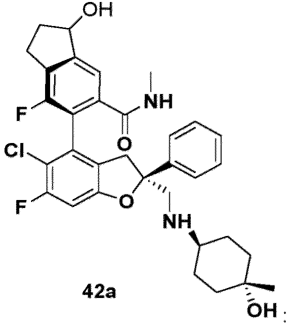 column model: Amylose-C 100 * 4.6mm 3 µm; eluent: hexane (0.1% DEA)/IPA; gradient: isocratic 8/2, flow rate: 1 mL/min, dr = 52 (2.86 min):48 (3.75 min);
a compound with a retention time of 8.87 min under the following conditions, which is a stereoisomer in 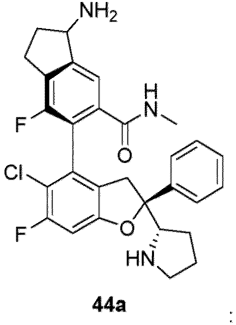 column model: Celluiose-SC, 100 * 4.6 mm, 5 µm; eluent: hexane (0.1% DEA)/IPA; gradient: isocratic 7/3, flow rate: 1 mL/min;
a compound with a retention time of 9.60 min under the following conditions, which is a stereoisomer in 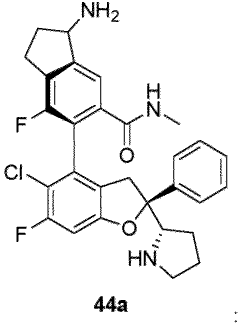 column model: Celluiose-SB, 100 * 4.6 mm, 5 µm; eluent: hexane/IPA; gradient: isocratic 7/3, flow rate: 1 mL/min;
a compound with a retention time of 4.16 min under the following conditions, which is a stereoisomer in 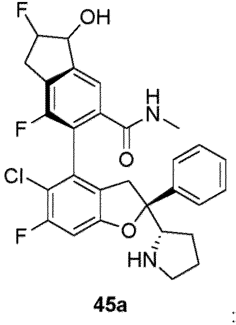 column model: Celluiose-C, 100 * 4.6 mm, 5 µm; eluent: hexane (0.1% DEA)-IPA; gradient: isocratic 4/1, flow rate: 1 mL/min;
a compound with a retention time of 2.68 min under the following conditions, which is a stereoisomer in 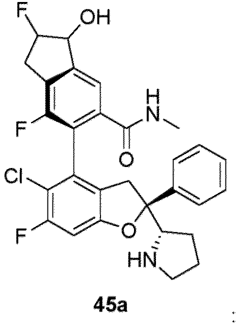 column model: Celluiose-C, 100 * 4.6 mm, 5 µm; eluent: hexane (0.1% DEA)-IPA; gradient: isocratic 4/1, flow rate: 1 mL/min;
a compound with a retention time of 4.92 min under the following conditions, which is a stereoisomer in 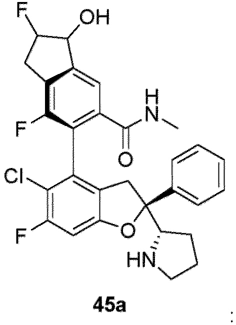 column model: Celluiose-C, 100 * 4.6 mm, 5 µm; eluent: hexane (0.1% DEA)-IPA; gradient: isocratic 4/1, flow rate: 1 mL/min;
a compound with a retention time of 4.87 min under the following conditions, which is a stereoisomer in 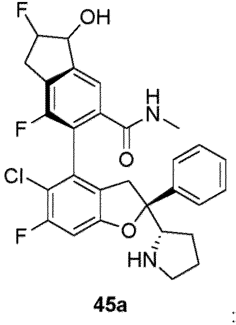 column model: Celluiose-C, 100 * 4.6 mm, 5 µm; eluent: hexane (0.1% DEA)-IPA; gradient: isocratic 4/1, flow rate: 1 mL/min;
a compound with a retention time of 2.197 min under the following conditions, which is a stereoisomer in 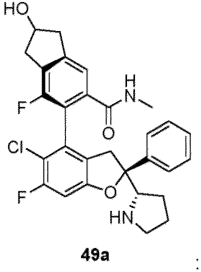 column model: Amylose-C, 100 * 4.6 mm, 5 µm; eluent: hexane-IPA; gradient: isocratic 7/3, flow rate: 1 mL/min;
a compound with a retention time of 2.646 min under the following conditions, which is a stereoisomer in 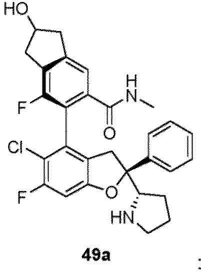 column model: Amylose-C, 100 * 4.6 mm, 5 µm; eluent: hexane-IPA; gradient: isocratic 7/3, flow rate: 1 mL/min;
a compound with a retention time of 3.14 min under the following conditions, which is a stereoisomer in 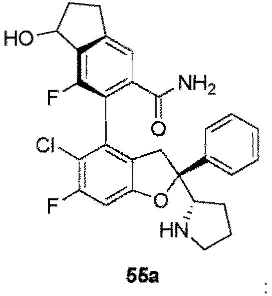 column model: Amylose-SA, 100 * 4.6 mm, 5 µm; eluent: hexane (0.1% DEA)-IPA; gradient: isocratic 4/1, flow rate: 1 mL/min;
a compound with a retention time of 3.04 min under the following conditions, which is a stereoisomer in 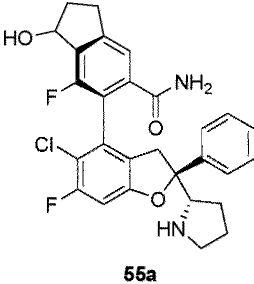 : column model: Amylose-SA, 100 * 4.6 mm, 5 µm; eluent: hexane (0.1% DEA)-IPA; gradient: isocratic 4/1, flow rate: 1 mL/min;
a compound with a retention time of 3.89 min under the following conditions, which is a stereoisomer in 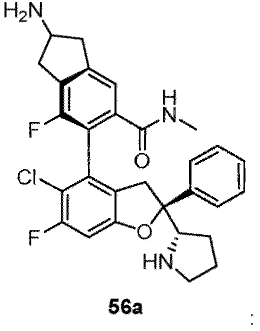 column model: Celluiose-SB, 100 * 4.6 mm, 5 µm; eluent: hexane (0.1% DEA)-IPA; gradient: isocratic 6/4, flow rate: 1 mL/min;
a compound with a retention time of 3.53 min under the following conditions, which is a stereoisomer in 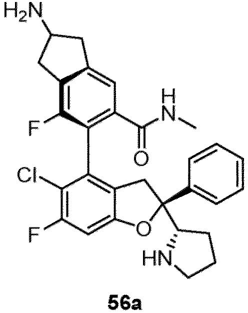 : column model: Celluiose-SB, 100 * 4.6 mm, 5 µm; eluent: hexane (0.1% DEA)-IPA; gradient: isocratic 6/4, flow rate: 1 mL/min;
a mixture with retention times of 1.95 min and 2.88 min under the following conditions, which is a pair of diastereomers in 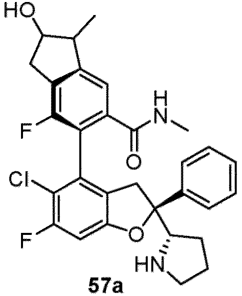 : column model: Amylose-C 100 * 4.6 mm, 5 µm; eluent: hexane (0.1% DEA)/IPA; gradient: isocratic 8/2, flow rate: 1 mL/min, dr = 65 (1.95 min):35 (2.88 min);
a compound with a retention time of 5.75 min under the following conditions, which is a stereoisomer in 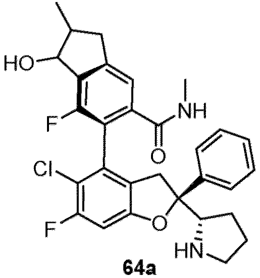 : column model: Celluiose-SB, 100 * 4.6 mm, 5 µm; eluent: hexane (0.1% DEA)-IPA; gradient: isocratic 9/1, flow rate: 1 mL/min;
a compound with a retention time of 4.76 min under the following conditions, which is a stereoisomer in 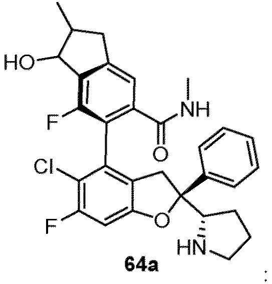 column model: Celluiose-SB, 100 * 4.6 mm, 5 µm; eluent: hexane (0.1% DEA)-IPA; gradient: isocratic 9/1, flow rate: 1 mL/min;
a compound with a retention time of 3.06 min under the following conditions, which is a stereoisomer in 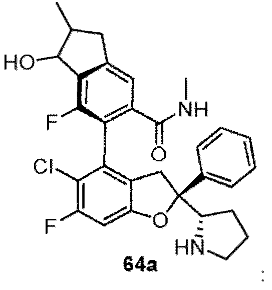 column model: Celluiose-SB, 100 * 4.6 mm, 5 µm; eluent: hexane (0.1% DEA)-IPA; gradient: isocratic 4/1, flow rate: 1 mL/min;
a compound with a retention time of 3.32 min under the following conditions, which is a stereoisomer in 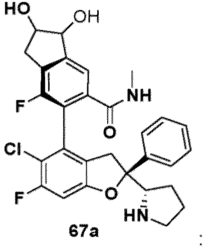 column model: Celluiose-SB, 100 * 4.6 mm, 3 µm; eluent: hexane (0.1% DEA)/EtOH; gradient: isocratic 8/2, flow rate: 1 mL/min;
a compound with a retention time of 3.12 min under the following conditions, which is a stereoisomer in 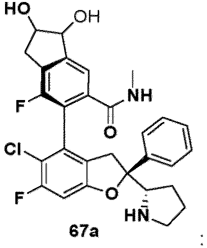 column model: Celluiose-SB, 100 * 4.6 mm, 3 µm; eluent: hexane (0.1% DEA)/EtOH; gradient: isocratic 8/2, flow rate: 1 mL/min;
a compound with a retention time of 2.67 min under the following conditions, which is a stereoisomer in 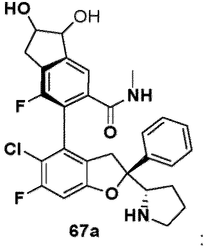 column model: Celluiose-SB, 100 * 4.6 mm, 3 µm; eluent: hexane (0.1% DEA)/EtOH; gradient: isocratic 8/2, flow rate: 1 mL/min;
a compound with a retention time of 4.10 min under the following conditions, which is a stereoisomer in 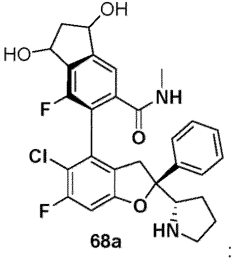 column model: Amylose-C, 100 * 4.6 mm, 3 µm; eluent: hexane/IPA; gradient: isocratic 85/15; flow rate: 1 mL/min;
a compound with a retention time of 4.47 min under the following conditions, which is a stereoisomer in 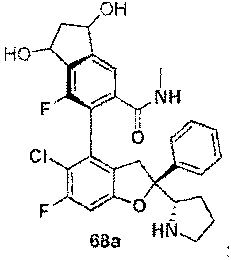 column model: Amylose-C, 100 * 4.6 mm, 3 µm; eluent: hexane/IPA; gradient: isocratic 85/15; flow rate: 1 mL/min;
a compound with a retention time of 5.59 min under the following conditions, which is a stereoisomer in 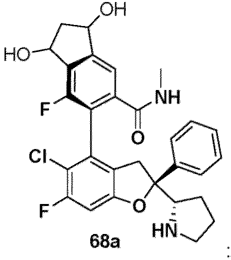 column model: Amylose-C, 100 * 4.6 mm, 3 µm; eluent: hexane/IPA; gradient: isocratic 85/15; flow rate: 1 mL/min;
a compound with a retention time of 4.29 min under the following conditions, which is a stereoisomer in 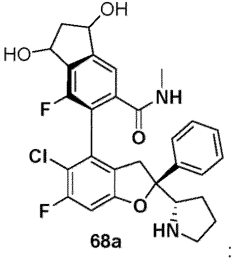 column model: Amylose-C, 100 * 4.6 mm, 3 µm; eluent: hexane/IPA; gradient: isocratic 85/15; flow rate: 1 mL/min;
a compound with a retention time of 6.65 min under the following conditions, which is a stereoisomer in 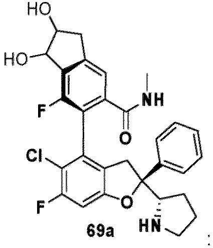 column model: Celluiose-SC, 100 * 4.6 mm, 3 µm; eluent: hexane (0.1% DEA)-IPA; gradient: isocratic 4/1, flow rate: 1 mL/min;
a compound with a retention time of 11.46 min under the following conditions, which is a stereoisomer in 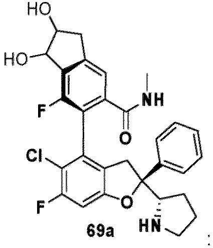 column model: Celluiose-SC, 100 * 4.6 mm, 3 µm; eluent: hexane (0.1% DEA)-IPA; gradient: isocratic 4/1, flow rate: 1 mL/min;
a compound with a retention time of 5.68 min under the following conditions, which is a stereoisomer in 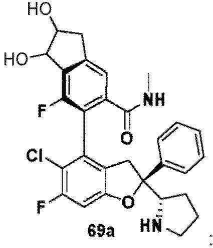 column model: Celluiose-SC, 100 * 4.6 mm, 3 µm; eluent: hexane (0.1% DEA)-IPA; gradient: isocratic 4/1, flow rate: 1 mL/min;
a compound with a retention time of 6.71 min under the following conditions, which is a stereoisomer in 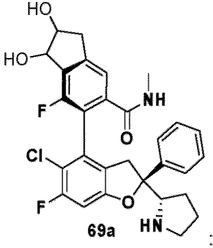 column model: Celluiose-SC, 100 * 4.6 mm, 3 µm; eluent: hexane (0.1% DEA)-IPA; gradient: isocratic 4/1, flow rate: 1 mL/min;
a compound with a retention time of 6.21 min under the following conditions, which is a stereoisomer in 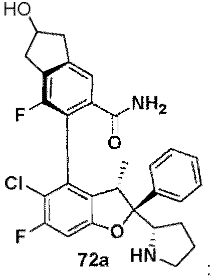 column model: Celluiose-SB, 100 * 4.6 mm, 3 µm; eluent: hexane/IPA; gradient: isocratic 8/2, flow rate: 1 mL/min;
a compound with a retention time of 4.71 min under the following conditions, which is a stereoisomer in 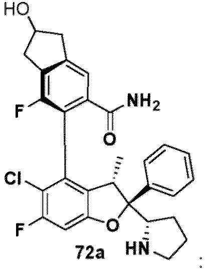 column model: Celluiose-SB, 100 * 4.6 mm, 3 µm; eluent: hexane/IPA; gradient: isocratic 8/2, flow rate: 1 mL/min;
a compound with a retention time of 8.1 min under the following conditions, which is a stereoisomer in 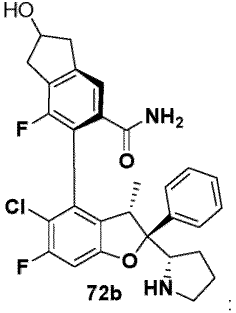 Xselect CSH Prep Fluoro-Phenyl, 150 * 19 mm, 5 µm; eluent: (0.1% FA) H₂O-ACN; gradient: 66% to 86%; flow rate: 20 mL/min;
a compound with a retention time of 6.5 min under the following conditions, which is a stereoisomer in 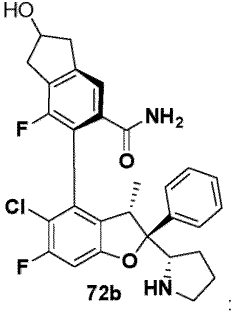 Xselect CSH Prep Fluoro-Phenyl, 150 * 19 mm, 5 µm; eluent: (0.1% FA) H₂O-ACN; gradient: 66% to 86%; flow rate: 20 mL/min;
a compound with a retention time of 3.90 min under the following conditions, which is a stereoisomer in 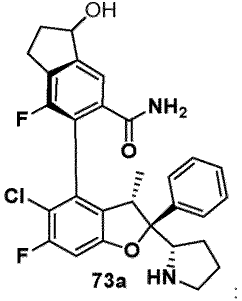 column model: Amylose-SA, 100 * 4.6 mm, 3 µm; eluent: hexane (0.1% DEA)/EtOH; gradient: isocratic 8/2, flow rate: 1 mL/min;
a compound with a retention time of 3.02 min under the following conditions, which is a stereoisomer in 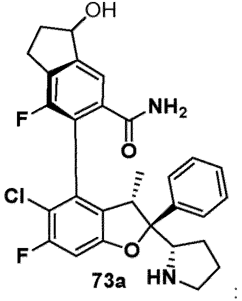 column model: Amylose-SA, 100 * 4.6 mm, 3 µm; eluent: hexane (0.1% DEA)/EtOH; gradient: isocratic 8/2, flow rate: 1 mL/min;
a compound with a retention time of 3.35 min under the following conditions, which is a stereoisomer in 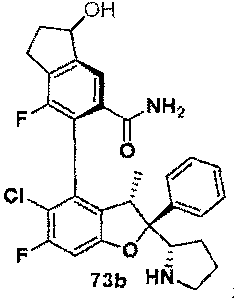 column model: Amylose-SA, 100 * 4.6 mm, 3 µm; eluent: hexane (0.1% DEA)/EtOH; gradient: isocratic 8/2, flow rate: 1 mL/min;
a compound with a retention time of 4.57 min under the following conditions, which is a stereoisomer in 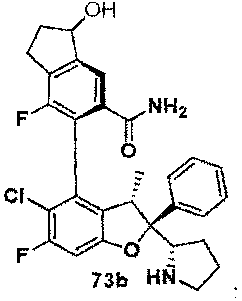 column model: Amylose-SA, 100 * 4.6 mm, 3 µm; eluent: hexane (0.1% DEA)/EtOH; gradient: isocratic 8/2, flow rate: 1 mL/min;
a compound with a retention time of 4.97 min under the following conditions, which is a stereoisomer in 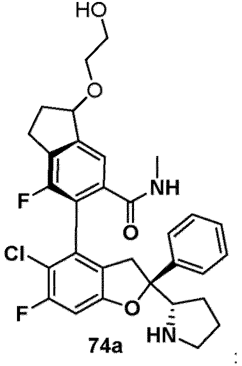 column model: Amylose-SA, 100 * 4.6 mm, 3 µm; eluent: hexane (0.1% DEA)/EtOH; gradient: isocratic 9/1, flow rate: 1 mL/min;
a compound with a retention time of 5.22 min under the following conditions, which is a stereoisomer in 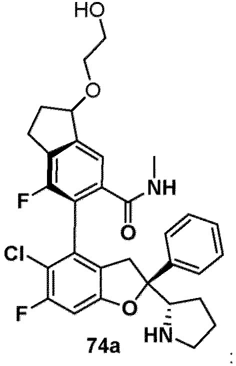 column model: Amylose-SA, 100 * 4.6 mm, 3 µm; eluent: hexane (0.1% DEA)/EtOH; gradient: isocratic 9/1, flow rate: 1 mL/min;
a compound with a retention time of 7.21 min under the following conditions, which is a stereoisomer in 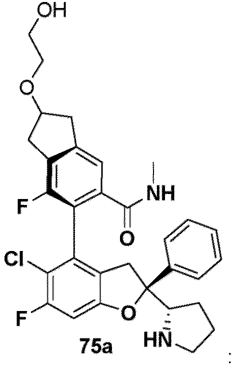 column model: Celluiose-SB, 100 * 4.6 mm, 5 µm; eluent: hexane (0.1% DEA)-IPA; gradient: isocratic 85/15, flow rate: 1 mL/min;
a compound with a retention time of 8.17 min under the following conditions, which is a stereoisomer in 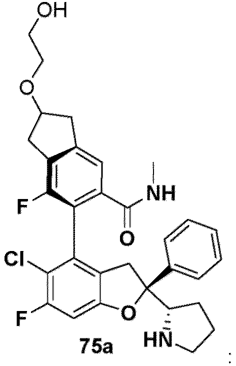 column model: Celluiose-SB, 100 * 4.6 mm, 5 µm; eluent: hexane (0.1% DEA)-IPA; gradient: isocratic 85/15, flow rate: 1 mL/min;
a compound with a retention time of 9.45 min under the following conditions, which is a stereoisomer in 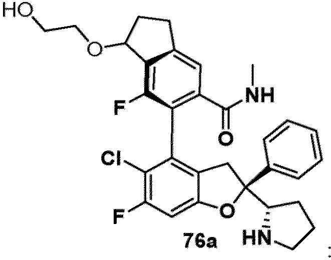 column model: Celluiose-SC, 100 * 4.6 mm, 3 µm; eluent: hexane (0.1% DEA)-IPA; gradient: isocratic 4/1, flow rate: 1 mL/min;
a compound with a retention time of 6.63 min under the following conditions, which is a stereoisomer in 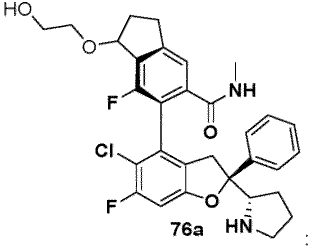 column model: Celluiose-SC, 100 * 4.6 mm, 3 µm; eluent: hexane (0.1% DEA)-IPA; gradient: isocratic 4/1, flow rate: 1 mL/min;
a compound with a retention time of 3.21 min under the following conditions, which is a stereoisomer in 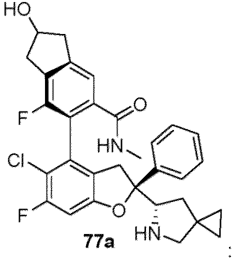 column model: Amylose-SA, 100 * 4.6 mm, 3 µm; eluent: hexane (0.1% DEA)-IPA; gradient: isocratic 85/15, flow rate: 1 mL/min;
a compound with a retention time of 7.29 min under the following conditions, which is a stereoisomer in 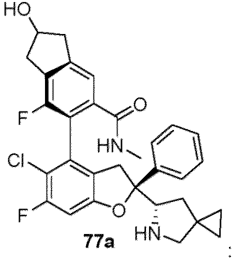 column model: Amylose-SA, 100 * 4.6 mm, 3 µm; eluent: hexane (0.1% DEA)-IPA; gradient: isocratic 85/15, flow rate: 1 mL/min;
a compound with a retention time of 5.46 min under the following conditions, which is a stereoisomer in 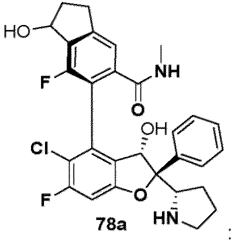 Celluiose-C, 100 * 4.6 mm, 3 µm; eluent: hexane (0.1% DEA)/IPA; gradient: isocratic 9/1, flow rate: 1 mL/min;
a compound with a retention time of 6.42 min under the following conditions, which is a stereoisomer in 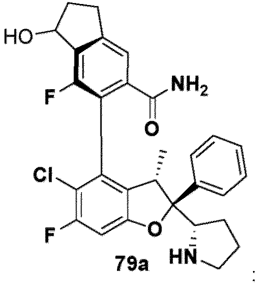 column model: Celluiose-SB, 100 * 4.6 mm, 3 µm; eluent: hexane (0.1% DEA)/IPA; gradient: isocratic 85/15, flow rate: 1 mL/min;
a compound with a retention time of 4.47 min under the following conditions, which is a stereoisomer in 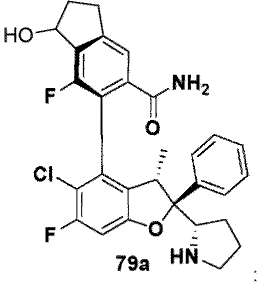 column model: Celluiose-SB, 100 * 4.6 mm, 3 µm; eluent: hexane (0.1% DEA)/IPA; gradient: isocratic 85/15, flow rate: 1 mL/min;
a mixture with retention times of 4.97 min and 6.09 min under the following conditions, which is a pair of diastereomers in 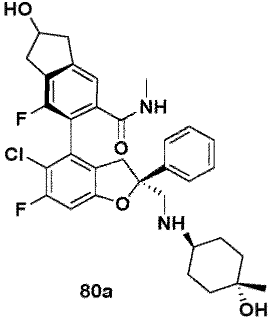 : column model: Celluiose-SC 100 * 4.6 mm 3 µm; eluent: hexane/IPA; gradient: isocratic 8/2, flow rate: 1 mL/min, dr = 49 (4.97 min):51 (6.09 min);
a compound with a retention time of 3.88 min under the following conditions, which is a stereoisomer in 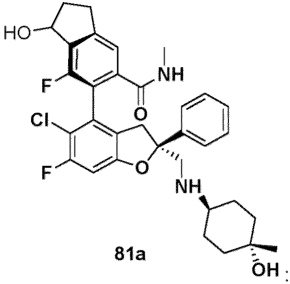 column model: Celluiose-C, 100 * 4.6 mm, 3 µm; eluent: hexane (0.1% DEA)/IPA; gradient: isocratic 4/1, flow rate: 1 mL/min;
a compound with a retention time of 3.52 min under the following conditions, which is a stereoisomer in 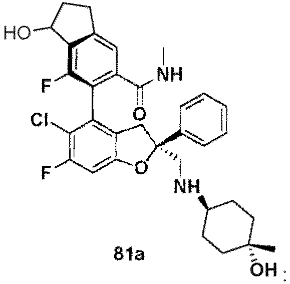 column model: Celluiose-C, 100 * 4.6 mm, 3 µm; eluent: hexane (0.1% DEA)/IPA; gradient: isocratic 4/1, flow rate: 1 mL/min;
a compound with a retention time of 4.91 min under the following conditions, which is a stereoisomer in 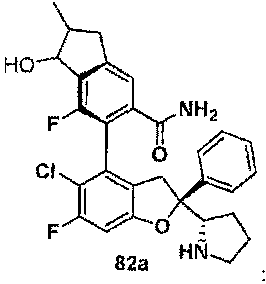 column model: Amylose-SA, 100 * 4.6 mm, 3 µm; eluent: hexane (0.1% DEA)-IPA; gradient: isocratic 9/1, flow rate: 1 mL/min;
a compound with a retention time of 5.05 min under the following conditions, which is a stereoisomer in 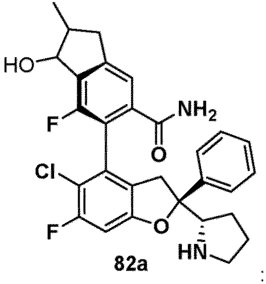 column model: Amylose-SA, 100 * 4.6 mm, 3 µm; eluent: hexane (0.1% DEA)-IPA; gradient: isocratic 9/1, flow rate: 1 mL/min;
a compound with a retention time of 7.00 min under the following conditions, which is a stereoisomer in 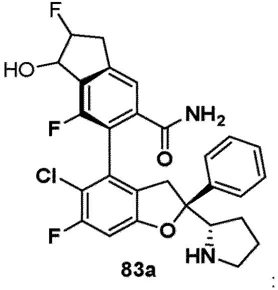 column model: Celluiose-SC, 100 * 4.6 mm, 5 µm; eluent: hexane (0.1% DEA)-IPA; gradient: isocratic 4/1, flow rate: 1 mL/min;
a compound with a retention time of 3.56 min under the following conditions, which is a stereoisomer in 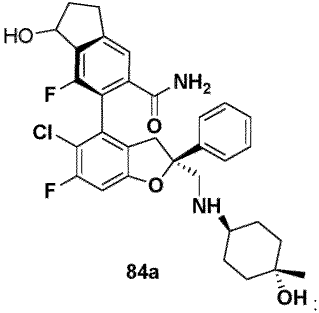 column model: Amylose-SA, 100 * 4.6 mm, 5 µm; eluent: hexane (0.1% DEA)-IPA; gradient: isocratic 4/1, flow rate: 1 mL/min;
a compound with a retention time of 3.40 min under the following conditions, which is a stereoisomer in 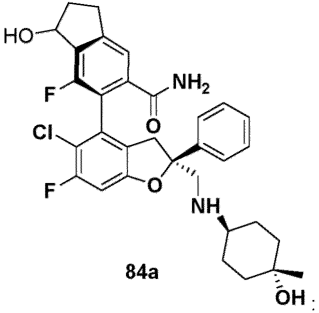 column model: Amylose-SA, 100 * 4.6 mm, 5 µm; eluent: hexane (0.1% DEA)-IPA; gradient: isocratic 4/1, flow rate: 1 mL/min;
a compound with a retention time of 3.45 min under the following conditions, which is a stereoisomer in 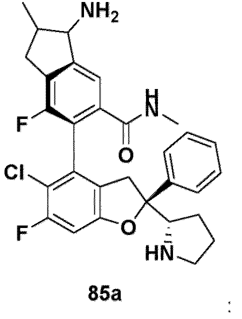 column model: Amylose-SA, 100 * 4.6 mm, 5 µm; eluent: hexane (0.1% DEA)-IPA; gradient: isocratic 4/1, flow rate: 1 mL/min;
a compound with a retention time of 3.53 min under the following conditions, which is a stereoisomer in 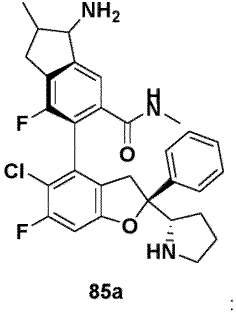 column model: Amylose-SA, 100 * 4.6 mm 5 µm; eluent: hexane (0.1% DEA)-IPA; gradient: isocratic 4/1, flow rate: 1 mL/min;
a compound with a retention time of 7.48 min under the following conditions, which is a stereoisomer in 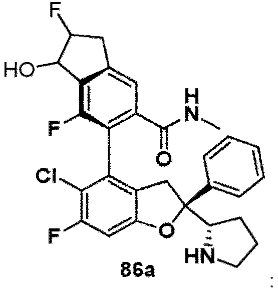 column model: Celluiose-SC, 100 * 4.6 mm, 5 µm; eluent: hexane (0.1% DEA)-IPA; gradient: isocratic 4/1, flow rate: 1 mL/min;
a compound with a retention time of 7.48 min under the following conditions, which is a stereoisomer in 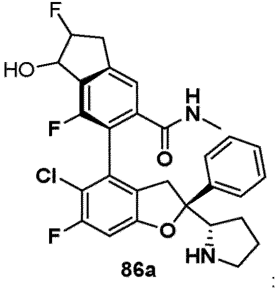 column model: Celluiose-SC, 100 * 4.6 mm, 5 µm; eluent: hexane (0.1% DEA)-IPA; gradient: isocratic 4/1, flow rate: 1 mL/min;
a compound with a retention time of 5.91 min under the following conditions, which is a stereoisomer in 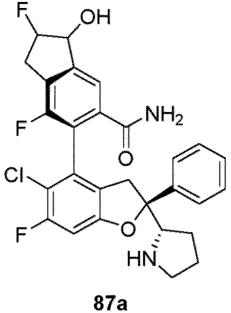 : column model: Amylose-C, 100 * 4.6 mm, 5 µm; eluent: hexane (0.1% DEA)-IPA; gradient: isocratic 4/1, flow rate: 1 mL/min;
a compound with a retention time of 4.72 min under the following conditions, which is a stereoisomer in 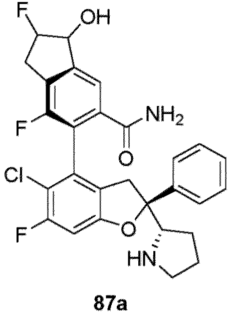 : column model: Amylose-C, 100 * 4.6 mm, 5 µm; eluent: hexane (0.1% DEA)-IPA; gradient: isocratic 4/1, flow rate: 1 mL/min;
a compound with a retention time of 2.09 min under the following conditions, which is a stereoisomer in 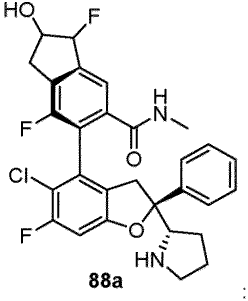 column model: Amylose-C, 100 * 4.6 mm, 3 µm; eluent: hexane (0.1% DEA)/IPA; gradient: isocratic 8/2, flow rate: 1 mL/min;
a compound with a retention time of 4.03 min under the following conditions, which is a stereoisomer in 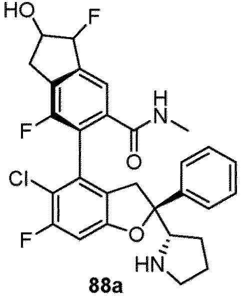 : column model: Amylose-C, 100 * 4.6 mm, 3 µm; eluent: hexane (0.1% DEA)/IPA; gradient: isocratic 8/2, flow rate: 1 mL/min;
a compound with a retention time of 2.92 min under the following conditions, which is a stereoisomer in 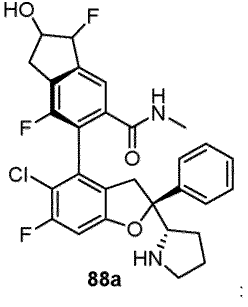 column model: Amylose-C, 100 * 4.6 mm, 3 µm; eluent: hexane (0.1% DEA)/IPA; gradient: isocratic 8/2, flow rate: 1 mL/min;
a compound with a retention time of 4.62 min under the following conditions, which is a stereoisomer in 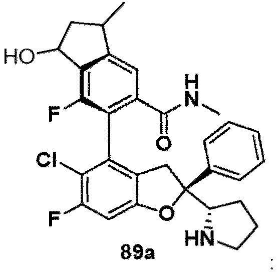 column model: Amylose-SA, 100 * 4.6 mm, 5 µm; eluent: hexane (0.1% DEA)-IPA; gradient: isocratic 9/1, flow rate: 1 mL/min;
a compound with a retention time of 5.32 min under the following conditions, which is a stereoisomer in 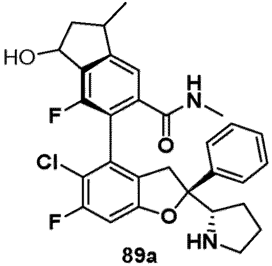 : column model: Amylose-SA, 100 * 4.6 mm, 3 µm; eluent: hexane (0.1% DEA)-IPA; gradient: isocratic 9/1, flow rate: 1 mL/min;
a compound with a retention time of 3.26 min under the following conditions, which is a stereoisomer in 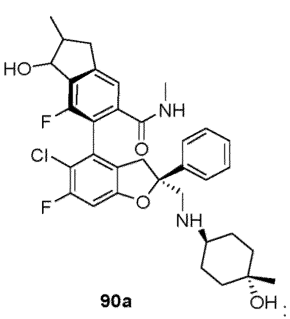 column model: Celluiose-C, 100 * 4.6 mm, 3 µm; eluent: hexane (0.1% DEA)-IPA; gradient: isocratic 4/1, flow rate: 1 mL/min;
a compound with a retention time of 2.90 min under the following conditions, which is a stereoisomer in 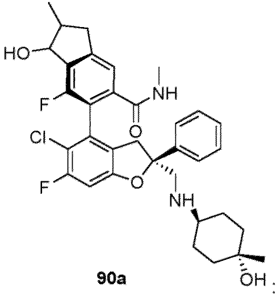 column model: Celluiose-C, 100 * 4.6 mm, 3 µm; eluent: hexane (0.1% DEA)-IPA; gradient: isocratic 4/1, flow rate: 1 mL/min.

18. A pharmaceutical composition, comprising:
(1) the compound of formula (I), the pharmaceutically acceptable salt thereof, or the solvate of the pharmaceutically acceptable salt thereof according to at least one of claims 1 to 17;
(2) a pharmaceutically acceptable excipient.

19. A use of the compound of formula (I), the pharmaceutically acceptable salt thereof, or the solvate of the pharmaceutically acceptable salt thereof according to at least one of claims 1 to 17, or the pharmaceutical composition according to claim 18 in the manufacture of a medicament for preventing and/or treating cancer.
